# EUROPEAN PATENT APPLICATION

(11) **EP 2 208 736 A2**
(43) Date of publication of application: **21.07.2010**
(21) Application number: 10153737.1
(22) Date of filing: 09.12.2002
(51) Int. Cl.: C07K 14/47, C12Q 1/68, C12Q 1/70, A61K 39/00, C07K 14/15, G01N 33/574

(54) **Endogenous retrovirus up-regulated in prostate cancer**

(30) Priority: 07.12.2001 US 16604; 07.12.2001 US 340640 P; 12.06.2002 US 388046 P; 07.12.2001 WO PCT/US01/47824
(62) Divisional of application: 02807929.1
(71) Applicant: Novartis Vaccines and Diagnostics, Inc., Emeryville, CA 94608 (US)
(72) Inventor: Hardy, Stephen F., San Francisco, CA 94121 (US); Garcia, Pablo, San Francisco, CA 94131 (US); Williams, Lewis T., Mill Valley, CA 94902 (US); Escobedo, Jaime, Alamo, CA 94507 (US)
(74) Representative: Marshall, Cameron John

(57) **Abstract**

A specific member of the HERV-K family located in chromosome 22 at 20.428 megabases (22q11.2) has been found to be preferentially and significantly up-regulated in prostate tumors. The invention provides methods for diagnosing prostate cancer, comprising the step of detecting in a patient sample the presence or absence of an expression product of the virus. The virus has five features not seen in other HERV-K members: (1) its own specific nucleotide sequence, and consequently amino acid sequences; (2) tandem 5'LTRs; (3) a fragmented 3'LTR; (4) an *env* gene interrupted by an alu insertion; and (5) unique gag sequences.

SEQUENCE LISTING

<110>   CHIRON CORPORATION
        GARCIA Pablo
        HARDY Stephen F
        WILLIAMS Lewis T
        ESCOBEDO Jaime

<120>   ENDOGENOUS RETROVIRUS UP-REGULATED IN PROSTATE CANCER

<130>   P037956EP

<150>   PCT/US01/47824
<151>   2001-12-07

<150>   USSN 10/016,604
<151>   2001-12-07

<150>   USSN 60/388,046
<151>   2002-06-12

<150>   USSN 60/340,064
<151>   2002-06-13

<160>   1208

<170>   SeqWin99, version 1.02

<210>   1
<211>   12366
<212>   DNA
<213>   HERV-K

<400>   1
tgtggggaaa agaaagagag atcagactgt tactgtgtct atgtagaaag aaatagacat   60
aagagactcc attttgttct gtactaagaa aaattcttct gctttgagat gctgttaatc   120
tgtaacccta gccccaaccc tgtgctcaca gaaacaggtg ctgtgttgac tcaaggttta   180
atggattcag ggctgtgcag gatgtgcttt gttaaacaaa tgcttgaagg cagcaagctt   240
gttaagagtc atcaccactc cctaatctca agtaagcagg gacacaaaca ctgcggaagg   300
ccgcagggac ctctgcctag gaaagccagg tgttgtccaa ggtttctccc catgtgacag   360
tctgaaatat ggcctcttgg gaagggaaag acctgactgt cccctggccc gacacccgta   420
aagggtctgt gctgaggatt agtaaaagag gaaggaaggc ctctttgcag ttgagataag   480
aggaaggcat ctgtctcctg ctcatccctg ggcaatggaa tgtcttggtg taaagcctga   540
ttgtatatgc catctactga gataggagaa aactgcctta gggctggagg tgggacatgc   600
tggcggcaat actgctcttt aaggcattga gatgtttatg tatatgcaca tcaaaagcac   660
agcacttttt tctttacctt gtttatgatg cagagacatt tgttcacatg ttttcctgct   720
ggccctctcc ccactattac cctattgtcc tgccacatcc ccctctccga gatggtagag   780
ataatgatca ataaatactg agggaactca gagaccggtg cggcgcgggt cctccatatg   840
ctgagcgccg gtcccctggg cccacttttc tttctctata ctttgtctct gttgtctttc   900
ttttctcaag tctctcgttc cacctgagga gaaatgccca cagctgtgga ggcgcaggcc   960
actccatctg gtgcccaacg tggatgcttt tctctagggt gaagggactc tcgagtgtgg   1020
tcattgagga caagtcaacg agagattccc gagtacgtct acagtgagcc ttgtggtaag   1080
cttgggcgct cggaagaagc cagggttaat ggggcaaact aaaagtaaag tctctcattc   1140
cacctgatga gaaacaccca gaggtgtgga ggggcaggcc accccttcag ggtagggtcc   1200
cctccatgca gaccatagag cacaggtgtg ccccaaagag gagcagagag aaggagggag   1260
agggcccacg agagacttgg aaatgaatgg caggatttta ggcgctggac ttgggttcgg   1320
ggcacctggc ctttccttgt gtatttctcc tactgtctgc ctaactattt aatacaataa   1380
aagaaaacca gcccctggtt cttgtggtgt ttccaccctc ccgggtcccc gctggctgcc   1440
tggcttcctc ccgcagctcc tgctgtgtgt gtatgtgtgt gtgtgtgcac atctgtgggg   1500
cgtatgtgtg ttcgtctttg taattgaggc tgcagagtgg agagagcagg ggttttctct   1560
ggggacccag agagaaggag gcgttttcac cacagccgaa cagggcagga ccccagcacc   1620

cgggacccag cgggactttg ccaaggggat ggacctggct gggccacgcg gctgtttgtg 1680
tagggaaaag aaagagagat cacactgtta ctgtgtctat gtagaaaagg aagacataaa 1740
ctccattttg agctgtacta agaaaaatta ttttgccttg acctgctgtt aacctgtaac 1800
tgtagcccca accctgtgct caaagaaaca tgtgctgtat ggaatcaagg tttaagggat 1860
caagggctgt acaggatgtg ccttgttaac aatgtgttta caggcagtat gcttggtaaa 1920
agtcatcgcc attctccatt ctccattaat caggggcacg atgcactgcg gaaagccaca 1980
gggacctctg cccgagaaag cctgggtatt gtccaaggct tccccccact gagacagcct 2040
gagatacggc ctcgtgggaa gggaaagacc tgaccgtccc ccagcccgac acccgtaaag 2100
ggtctgtgct gaggaggatt agtaaaaggg gaaggcctct tgcagttgag ataagaggaa 2160
ggcctccgtc tcctgcatgt ccttgggaat ggaatgtctt ggtgtaaaac ccgatagtac 2220
attccttcta ttctgagaga agaaaaccac cctgtggctg gaggtgagat atgctagcgg 2280
caatgctgct ctgttactct ttgctacact gagatgtttg ggtggagaga agcataaatc 2340
tggcctatgt gcacatctgg gcacagaacc tccccttgaa cttgtgacac agattccttt 2400
gttcacatgt tttcctgctg accttctccc cactatcgcc ctgttctccc accgcattcc 2460
ccttgctgag atagtgaaaa tagtaatctg tagataccaa gggaactcag agaccatggc 2520
cggtgcacat cctccgtacg ctgagcgctg gtcccctggg cccattgttc tttctctata 2580
ctttgtctct gtgtcttatt tctttcctca gtctctcatc cctcctgacg agaaataccc 2640
acaggtgtgg aggggctggc ccccttcatc tgatgcccaa tgtgggtgcc tttctctagg 2700
gtgaaggtac tctacagtgt ggtcattgag gacaagttga cgagagagtc ccaagtacgt 2760
ccacggtcag ccttgcggta agcttgtgtg cttagaggaa cccagggtaa cgatggggca 2820
aactgaaagt aaatatgcct cttatctcag ctttattaaa attcttttaa gaagaggggg 2880
agttagagct tctacagaaa atctaattac gctatttcaa acaatagaac aattctgccc 2940
atggtttcca gaacagggaa ctttagatct aaaagattgg gaaaaaattg gcaaagaatt 3000
aaaacaagca aatagggaag gtaaaatcat cccacttaca gtatggaatg attgggccat 3060
tattaaagca actttagaac catttcaaac aggagaagat attgtttcag tttctgatgc 3120
ccctaaaagc tgtgtaacag attgtgaaga agaggcaggg acagaatccc agcaaggaac 3180
ggaaagttca cattgtaaat atgtagcaga gtctgtaatg gctcagtcaa cgcaaaatgt 3240
tgactacagt caattacagg agataatata ccctgaatca tcaaaattgg gggaaggagg 3300
tccagaatca ttggggccat cagagcctaa accacgatcg ccatcaactc ctcctcccgt 3360
ggttcagatg cctgtaacat tacaacctca aacgcaggtt agacaagcac aaaccccaag 3420
agaaaatcaa gtagaaaggg acagagtctc tatcccggca atgccaactc agatacagta 3480
tccacaatat cagccggtag aaaataagac ccaaccgctg gtagtttatc aataccggct 3540
gccaaccgag cttcagtatc ggcctccttc agaggttcaa tacagacctc aagcggtgtg 3600
tcctgtgcca aatagcacgg caccatacca gcaacccaca gcgatggcgt ctaattcacc 3660
agcaacacag gacgcggcgc tgtatcctca gccgcccact gtgagactta atcctacagc 3720
atcacgtagt ggacagggtg gtgcactgca tgcagtcatt gatgaagcca gaaaacaggg 3780
cgatcttgag gcatggcggt tcctggtaat tttacaactg gtacaggccg gggaagagac 3840
tcaagtagga gcgcctgccc gagctgagac tagatgtgaa cctttcacca tgaaaatgtt 3900
aaaagatata aaggaaggag ttaaacaata tggatccaac tccccttata taagaacatt 3960
attagattcc attgctcatg gaaatagact tactccttat gactgggaaa ttttggccaa 4020
atcttccctt tcatcctctc agtatctaca gtttaaaacc tggtggattg atggagtaca 4080
agaacaggta cgaaaaaatc aggctactaa gcccactgtt aatatagacg cagaccaatt 4140
gttaggaaca ggtccaaatt ggagcaccat taaccaacaa tcagtgatgc agaatgaggc 4200
tattgaacaa gtaagggcta tttgcctcag ggcctgggga aaaattcagg acccaggaac 4260
agctttccct attaattcaa ttagacaagg ctctaaagag ccatatcctg actttgtggc 4320
aagattacaa gatgctgctc aaaagtctat tacagatgac aatgcccgaa aagttattgt 4380
agaattaatg gcctatgaaa atgcaaatcc agaatgtcag tcggccataa agccattaaa 4440
aggaaaagtt ccagcaggag ttgatgtaat tacagaatat gtgaaggctt gtgatgggat 4500
tggaggagct atgcataagg caatgctaat ggctcaagca atgagggggc tcactctagg 4560
aggacaagtt agaacatttg ggaaaaaatg ttataattgt ggtcaaatcg gtcatctgaa 4620
aaggagttgc ccaggcttaa ataaacagaa tataataaat caagctatta cagcaaaaaa 4680
taaaaagcca tctggcctgt gtccaaaatg tggaaaagca aaacattggg ccaatcaatg 4740
tcattctaaa tttgataaag atgggcaacc attgtctgga aacaggaaga ggggccagcc 4800
tcaggccccc caacaaactg gggcattccc agttaaactg tttgttcctc agggttttca 4860
aggacaacaa cccctacaga aaataccacc acttcaggga gtcagccaat tacaacaatc 4920
caacagctgt cccgcgccac agcaggcagc accgcagtag atttatgttc cacccaaatg 4980
gtctttttac tccctggaaa gcccccacaa aagattccta gaggggtata tggcccgctg 5040
ccagaaggga gggtaggcct ttgagggaga tcaagtctaa atttgaaggg agtccaaatt 5100
catactgggg taatttattc agattataaa gggggaattc agttagtgat cagctccact 5160
gttccccgga gtgccaatcc aggtgataga attgctcaat tactgctttt gccttatgtt 5220
aaaattgggg aaaacaaaaa ggaaagaaca ggagggtttg gaagtaccaa ccctgcagga 5280

aaagctgctt attgggctaa tcaggtctca gaggatagac ccgtgtgtac agtcactatt 5340
cagggaaaga gtttgaagga ttagtggata cccaggctga tgtttctgtc atcggcatag 5400
gtactgcctc agaagtgtat caaagtgcca tgattttaca ttgtccagga tctgataatc 5460
aagaaagtac ggttcagcct gtgatcactt cattccaatc aatttatggg gccgagactt 5520
gttacaacaa tggcatgcag agattactat cccagcctcc ctatacagcc ccaggaataa 5580
aaaaatcatg actaaaatgg gatagctccc taaaaaggga ctaggaaaga agtcccaatt 5640
gaggctgaaa aaaatcaaaa aagaaaagga atagggcatc ctttttagga gcggtcactg 5700
tagagcctcc aaaacccatt ccattaactt gggggaaaaa aaaacaactg tatggtaaat 5760
cagcagcgct tccaaaacaa aaactggagg ctttacattt attagcaaag aaacaattag 5820
aaaaaggaca ttgagccttc attttcgcct tggaattctg tttgtaattc agaaaaaatc 5880
cggcagatgg cgtataatgc cgtaattcaa cccatggggg ctctcccacc ccggttgccc 5940
tctccagcca tggtcccctt taattataat tgatctgaag gattgctttt ttaccattcc 6000
tctggcaaaa caggattttg aaaaatttgc ttttaccaca ccagcctaaa taataaagaa 6060
ccagccacca ggtttcagtg gaaagtattg cctcagggaa tgcttaatag ttcaactatt 6120
tgtcagctca agctctgcaa ccagttagag acaagttttc agactgttac atcgttcact 6180
atgttgatat tttgtgtgct gcagaaacga gagacaaatt aattgaccgt tacacatttc 6240
tgcagacaga ggttgccaac gcgggactga caataacatc tgataagatt caaacctcta 6300
ctcctttccg ttacttggga atgcaggtag aggaaaggaa aattaaacca caaaaaatag 6360
aaataagaaa agacacatta aaagcattaa atgagtttca aaagttgcta ggagatacta 6420
attggatttg gagatattaa ttggatttgg ccaactctag gcattcctac ttatgccatg 6480
tcaaatttgt tctctttctt aagaggggac tcggaattaa atagtgaaag aacgttaact 6540
ccagaggcaa ctaaagaaat taaattaatt gaagaaaaaa ttcggtcagc acaagtaaat 6600
agaatagatc acttggcccc actccaaatt ttgatttttg ctactgcaca ttccctaaca 6660
ggcatcattg ttcaaaatac agatcttgtg gagtggtcct tccttcctca cagtacaatt 6720
aagactttta cattgtactt ggatcaaatg gctacattaa ttggtcaggg aagattatga 6780
ataataacat tgtgtggaaa tgacccagat aaaatcactg ttcctttcaa caagcaacag 6840
gttagacaag cctttatcaa ttctggtgca tggcagattg gtcttgccga ttttgtggga 6900
attattgaca atcgttaccc caaaacaaaa atcttccagt ttttaaaatt gactacttgg 6960
atttacctta aagttaccaa acataagcct ttaaaaaatg ctctggcagt gtttactgat 7020
ggttccagca atggaaaagt ggcttacacc gggccaaaag aatgagtcat caaaactcag 7080
tatcacttga ctcaaagagc agagttggtt gccgtcatta cagtgttaac aagattttaa 7140
tcagtctatt aacattgtat cagattctgc atatgtagta caggctacaa aggatattga 7200
gagagcccta atcaaataca ttatggatga tcagttaaac ccgctgttta atttgttaca 7260
acaaaatgta agaaaaagaa atttcccatt ttatattact catattcgag cacacactaa 7320
tttaccaggg cctttaacta aagcaaatga acaagctgac ttgctagtat catctgcatt 7380
catggaagca caagaacttc atgccttgac tcatgtaaat gcaataggat taaaaaataa 7440
atttgatatc acatggaaac agacaaaaaa tattgtacaa cattgcaccc agtgtcagat 7500
tctacacctg gccactcagg aggcaagagt taatcccaga ggtctatgtc ctaatgtgtt 7560
atggcaaatg gatgtcatgc acgtaccttc atttggaaaa ttgtcatttg tccatgtgac 7620
agttgatact tattcacatt tcatatgggc aacctgccag acaggagaaa gtacttccca 7680
tgttaaaaga catttattat cttgttttcc tgtcatggga gttccagaaa aagttaaaac 7740
agacaatggg ccaggttact gtagtaaagc agttcaaaaa ttcttaaatc agtggaaaat 7800
tacacataca ataggaattc tctataattc ccaaggacag gccataattg aaagaactaa 7860
tagaacactc aaagctcaat tggttaaaca aaaaaaagga aaagacagga gtataacact 7920
ccccagatgc aacttaatct agcactctat actttaaatg ttttaaacat ttatagaaat 7980
cagaccacta cctctgcaga acaacatctt actggtaaaa ggaacagccc acatgaagga 8040
aaactgattt ggtggaaaga taataaaaat aaaacatggg aaatggggaa ggtgataacg 8100
tgggggagag gttttgcttg tgtttcacca ggagaaaatc agcttcctgt ttggataccc 8160
actagacatt taaagttcta caatgaactc actggagatg caaagaaaag tgtggagatg 8220
gagacacccc aatcgactcg ccaggtaaac aaaatggtga tatcagaaga acagaaaaag 8280
ttgccttcca tcaaggaagc agagttgcca atataggcac aattaaagaa gctgacacag 8340
ttagctaaaa aaaaaagcct agagaataca aaggtgacac caactccaga gaatatgctg 8400
cttgcagctc tgatgattgt atcaacggtg gtaagtcttc ccaagtctgc aggagcagct 8460
gcagctaatt atacttactg ggcctatgtg cctttcccac ccttaattcg ggcagttaca 8520
tagatggata atcctattga agtagatgtt aataatagtg catgggtgcc tggccccaca 8580
gatgactgtt gccctgccca acctgaagaa ggaatgatga tgaatatttc cattgggtat 8640
ccttatcctc ctgtttgcct agggaaggca ccaggatgct taatgcctac aacccaaaat 8700
tggttggtag aagtacctac agtcagtgct accagtagat ttacttatca catggtaagt 8760
ggaatgtcac agataaataa tttacaggac ccttcttatc aaagatcatt acaatgtagg 8820
cctaagggga aggcttgccc caaggaaatt cccaaagaat caaaaagccc agaagtctta 8880
gtctgcggag aatgtgtggc tgatactgca gtgtagtaca aaacaatgaa ttttgaacta 8940

tgatagactg ggtcccttga ggccaattat atcataactg tacaggccag actcattcat 9000
gttcacaggc cccatccatc tggcccatta atccagccta tgacggtgat gtaactgaaa 9060
ggctggacca ggtttataga aggttagaat cactctgtcc aaggaaatgg ggtgaaaagg 9120
gaatttcatc accttgacca aagttagtcc tgttactggt cctgaacatc cagaattagg 9180
aagcttactg tggcctcaca ccacattaga atttgttctg gaaatcaagc tataggaaca 9240
agagatcgta agtcatatta tactatcaac ctaaattcca gtctgacaat tcctttgcaa 9300
aattgtgtaa aactccctta tattgctagt tgtaggaaaa acatagttat taaacctgat 9360
tcccaaacca taatctgtga aaattgtgga atgtttactt gcattgattt gacttttaat 9420
tggcagcacc gtattctact aggaagagca agagagggtg tgtggatcct tgtgtccatg 9480
gaccgaccat gggaggcttc gctatccatc catattttaa cggaagtatt aaaaggaatt 9540
ctaactagat ccaaaagatt catttttact ttgatggcag tgattatggg cctcattgca 9600
gtcacagcta ctgctgcggc tgctggaatt gctttacact cctctgttca aactgcagaa 9660
tacgtaaatg attggcaaaa gaattcctca aaattgtgga attctcagat ccaaatagat 9720
caaaaattgg caaaccaaat taatgatctt agacaaactg tcatttggat gggagaggct 9780
catgagcttg gaatatcttt ttcagttacg atgtgactgg aatacatcag atttttgtgt 9840
tacaccacaa gcctataatg agtctgagca tcactgggac atggttagat gccatctgca 9900
aggaggagaa gataatctta ctttagacat ttcaaaatta aaagaatttt tttttctttt 9960
gagacagagt ctcgctctgt cgcccaggct ggagtgcagt ggcgtgatct cagctcactg 10020
caagttccgc ctcctgggtt tacaccattc tcctgcctca gcctcccaag tagttgggac 10080
tacaggagcc caccaccatg cctggctaat ttttttgggg tttttaatag agatggagtt 10140
tcaccgtgtt agccaggatg gtctcgatct cctgaccttg tgatctgccc accttggcct 10200
cccaaagtgc tgggattaca gtcgtgagcc accgtgccca gccaagaaaa aatttttgag 10260
gcatcaaaag cccatttaaa tttggtgcca ggaacggaga caatcgtgaa agctgctgat 10320
agcctcacaa atcttaagcc agtcacttgg gttaaaagca tcagaagttt cactattgta 10380
aatttcatat taatccttgt atgcctgttc tgtctgttgt tagtctacag gtgtatccag 10440
cagctccaaa gagacagcaa ccagcaagaa tgggccatag tgacgatggt ggttttgtca 10500
aaaagaaaag ggggggatat gtaaggaaaa gagagatcag actttcactg tgtctatgta 10560
gaaaaggaag acataagaaa ctccattttg atctgtacta agaaaaattg ttttgccttg 10620
agatgctgtt aatctgtaac tttagcccca accctgtgct cacggaaaca tgtgctgtaa 10680
ggtttaaggg atctagggct gtgcaggatg taccttgtta acaatatgtt tgcaggcagt 10740
atgtttggta aaagtcatcg ccattctcca ttctcgatta accaggggct caatgcactg 10800
tggaaagcca caggaacctc tgcccaagaa agcctggctg ttgtgggaag tcagggaccc 10860
cgaatggagg gaccagctgg tgctgcatca ggaaacataa attgtgaaga tttcttggac 10920
atttatcagt ttccaaaatt aatactttta taatttctta cacctgtctt actttaatct 10980
cttaatcctg ttatctttgt aagctgagga tatacgtcac ctcaggacca ctattgtaca 11040
aattgattgt aaaacatgtt cacatgtgtt tgaacaatat gaaatcagtg caccttgaaa 11100
atgaacagaa taacagtgat tttagggaac aaaggaagac aaccataagg tctgactgcc 11160
tgaggggtcg ggcaaaaagc catatttttc ttcttgcaga gagcctataa atggacgtgc 11220
aagtaggaga gatattgcta aattcttttc ctagcaagga atataatact aagaccctag 11280
ggaaagaatt gcattcctgg ggggaggtct ataaacggcc gctctgggag tgtctgtcct 11340
atgtggttga gataaggact gagatacgcc ctggtctcct gcagtaccct caggcttact 11400
aggattggga aaccccagtc ctggtaaatt tgaggtcagg ccggttcttt gctctgaacc 11460
ctgttttctg ttaagatgtt tatcaagaca atacatgcac cgctgaacat agacccttat 11520
caggagtttc tgattttgct ctggtcctgt ttcttcagaa gcatgtcatc tttgctctgc 11580
cttctgccct ttgaagcatg tgatctttgt gacctactcc ctgttcatac acccctcccc 11640
ttttaaaatc cctaataaaa acttgctggt tttgtggctc aggggggcat catggaccta 11700
ccaatacgtg atgtcacccc cggtggccca gctgtaaaat tcctttcttt atactcttat 11760
ttctcagacc agctgacact tagggaaaat agaaagaacc tatgttgaaa tattggaggc 11820
gggttccccc gatacctggg tattgtccaa ggtttccttt gctgaggagg attagtaaaa 11880
ggaatgcctc catctcctgc atgtccctgg gaacagaatg ttcccaccaa ccaccctgtg 11940
gctggaggcg ggatatgctg gcagcaatgc tgctctatta ctctttgcta cactgagatg 12000
tttgggtgga gagaagcata aatctggcct atgtgcacat ctgggcacag caccttcctt 12060
tgaacttatt tgtgacacag attcctttgc tcacgttttc ctgttgactt tctcaccact 12120
caccctattc tcctgtggca ttcgccttgc ggagatagtg aaaatagtaa taaatactga 12180
gggaactcag actgagggaa ctcagactgg gcagaccggg gccagtgtgg gtcctccata 12240
tgctgagcgc cggttccctg ggcccactgt tctttctcta tactttgtct ctgtgcctta 12300
ttttctcagt ctctcattcc acctgatgag aaatacccac aggtgtggag ggctggcccc 12360
ccttca 12366

<210> 2
<211> 517

<212>   DNA
<213>   HERV-K

<400>   2
gagataggag aaaactgcct tagggctgga ggtgggacat gctggcggca atactgctct   60
ttaaggcatt gagatgttta tgtatatgca catcaaaagc acagcacttt tttctttacc   120
ttgtttatga tgcagagaca tttgttcaca tgttttcctg ctggccctct ccccactatt   180
accctattgt cctgccacat ccccctctcc gagatggtag agataatgat caataaatac   240
tgagggaact cagagaccgg tgcggcgcgg gtcctccata tgctgagcgc cggtcccctg   300
ggcccacttt tctttctcta tactttgtct ctgttgtctt tcttttctca agtctctcgt   360
tccacctgag gagaaatgcc cacagctgtg gaggcgcagg ccactccatc tggtgcccaa   420
cgtggatgct tttctctagg gtgaagggac tctcgagtgt ggtcattgag gacaagtcaa   480
cgagagattc ccgagtacgt ctacagtgag ccttgtg                            517

<210>   3
<211>   10
<212>   DNA
<213>   HERV-K

<400>   3
tctctcatcc                                                          10

<210>   4
<211>   10
<212>   DNA
<213>   HERV-K

<400>   4
ggtgaaggta                                                          10

<210>   5
<211>   527
<212>   DNA
<213>   HERV-K

<400>   5
gagataggag aaaactgcct tagggctgga ggtgggacat gctggcggca atactgctct   60
ttaaggcatt gagatgttta tgtatatgca catcaaaagc acagcacttt tttctttacc   120
ttgtttatga tgcagagaca tttgttcaca tgttttcctg ctggccctct ccccactatt   180
accctattgt cctgccacat ccccctctcc gagatggtag agataatgat caataaatac   240
tgagggaact cagagaccgg tgcggcgcgg gtcctccata tgctgagcgc cggtcccctg   300
ggcccacttt tctttctcta tactttgtct ctgttgtctt tcttttctca agtctctcgt   360
tccacctgag gagaaatgcc cacagctgtg gaggcgcagg ccactccatc tggtgcccaa   420
cgtggatgct tttctctagg gtgaagggac tctcgagtgt ggtcattgag gacaagtcaa   480
cgagagattc ccgagtacgt ctacagtgag ccttgtgtct ctcatcc                527

<210>   6
<211>   527
<212>   DNA
<213>   HERV-K

<400>   6
gagataggag aaaactgcct tagggctgga ggtgggacat gctggcggca atactgctct   60
ttaaggcatt gagatgttta tgtatatgca catcaaaagc acagcacttt tttctttacc   120
ttgtttatga tgcagagaca tttgttcaca tgttttcctg ctggccctct ccccactatt   180
accctattgt cctgccacat ccccctctcc gagatggtag agataatgat caataaatac   240
tgagggaact cagagaccgg tgcggcgcgg gtcctccata tgctgagcgc cggtcccctg   300
ggcccacttt tctttctcta tactttgtct ctgttgtctt tcttttctca agtctctcgt   360
tccacctgag gagaaatgcc cacagctgtg gaggcgcagg ccactccatc tggtgcccaa   420
cgtggatgct tttctctagg gtgaagggac tctcgagtgt ggtcattgag gacaagtcaa   480
cgagagattc ccgagtacgt ctacagtgag ccttgtgggt gaaggta                527

<210>   7
<211>   319
<212>   DNA
<213>   HERV-K

<400>   7
tgtaaggaaa agagagatca gactttcact gtgtctatgt agaaaaggaa gacataagaa   60
actccatttt gatctgtact aagaaaaatt gttttgcctt gagatgctgt taatctgtaa   120
ctttagcccc aaccctgtgc tcacggaaac atgtgctgta aggtttaagg gatctagggc   180
tgtgcaggat gtaccttgtt aacaatatgt ttgcaggcag tatgtttggt aaaagtcatc   240
gccattctcc attctcgatt aaccaggggc tcaatgcact gtggaaagcc acaggaacct   300
ctgcccaaga aagcctggc                                                319

<210>   8
<211>   897
<212>   DNA
<213>   HERV-K

<400>   8
tgttgtggga agtcagggac cccgaatgga gggaccagct ggtgctgcat caggaaacat   60
aaattgtgaa gatttcttgg acatttatca gtttccaaaa ttaatacttt tataatttct   120
tacacctgtc ttactttaat ctcttaatcc tgttatcttt gtaagctgag gatatacgtc   180
acctcaggac cactattgta caaattgatt gtaaaacatg ttcacatgtg tttgaacaat   240
atgaaatcag tgcaccttga aaatgaacag aataacagtg attttaggga acaaaggaag   300
acaaccataa ggtctgactg cctgaggggt cgggcaaaaa gccatatttt tcttcttgca   360
gagagcctat aaatggacgt gcaagtagga gagatattgc taaattcttt tcctagcaag   420
gaatataata ctaagaccct agggaaagaa ttgcattcct ggggggaggt ctataaacgg   480
ccgctctggg agtgtctgtc ctatgtggtt gagataagga ctgagatacg ccctggtctc   540
ctgcagtacc ctcaggctta ctaggattgg gaaaccccag tcctggtaaa tttgaggtca   600
ggccggttct ttgctctgaa ccctgttttc tgttaagatg tttatcaaga caatacatgc   660
accgctgaac atagaccctt atcaggagtt tctgattttg ctctggtcct gtttcttcag   720
aagcatgtca tctttgctct gccttctgcc ctttgaagca tgtgatcttt gtgacctact   780
ccctgttcat acacccctcc ccttttaaaa tccctaataa aaacttgctg gttttgtggc   840
tcaggggggc atcatggacc taccaatacg tgatgtcacc cccggtggcc cagctgt      897

<210>   9
<211>   1216
<212>   DNA
<213>   HERV-K

<400>   9
tgtaaggaaa agagagatca gactttcact gtgtctatgt agaaaaggaa gacataagaa   60
actccatttt gatctgtact aagaaaaatt gttttgcctt gagatgctgt taatctgtaa   120
ctttagcccc aaccctgtgc tcacggaaac atgtgctgta aggtttaagg gatctagggc   180
tgtgcaggat gtaccttgtt aacaatatgt ttgcaggcag tatgtttggt aaaagtcatc   240
gccattctcc attctcgatt aaccaggggc tcaatgcact gtggaaagcc acaggaacct   300
ctgcccaaga aagcctggct gttgtgggaa gtcagggacc ccgaatggag ggaccagctg   360
gtgctgcatc aggaaacata aattgtgaag atttcttgga catttatcag tttccaaaat   420
taatactttt ataatttctt acacctgtct tactttaatc tcttaatcct gttatctttg   480
taagctgagg atatacgtca cctcaggacc actattgtac aaattgattg taaaacatgt   540
tcacatgtgt ttgaacaata tgaaatcagt gcaccttgaa aatgaacaga ataacagtga   600
ttttagggaa caaaggaaga caaccataag gtctgactgc ctgaggggtc gggcaaaaag   660
ccatattttt cttcttgcag agagcctata aatggacgtg caagtaggag agatattgct   720
aaattctttt cctagcaagg aatataatac taagacccta gggaaagaat tgcattcctg   780
gggggaggtc tataaacggc cgctctggga gtgtctgtcc tatgtggttg agataaggac   840
tgagatacgc cctggtctcc tgcagtaccc tcaggcttac taggattggg aaaccccagt   900
cctggtaaat ttgaggtcag gccggttctt tgctctgaac cctgttttct gttaagatgt   960
ttatcaagac aatacatgca ccgctgaaca tagaccctta tcaggagttt ctgattttgc   1020
tctggtcctg tttcttcaga agcatgtcat ctttgctctg ccttctgccc tttgaagcat   1080
gtgatctttg tgacctactc cctgttcata cacccctccc cttttaaaat ccctaataaa   1140

aacttgctgg ttttgtggct caggggggca tcatggacct accaatacgt gatgtcaccc 1200
ccggtggccc agctgt 1216

<210> 10
<211> 11177
<212> DNA
<213> HERV-K

<400> 10
gagataggag aaaactgcct tagggctgga ggtgggacat gctggcggca atactgctct 60
ttaaggcatt gagatgttta tgtatatgca catcaaaagc acagcacttt tttctttacc 120
ttgtttatga tgcagagaca tttgttcaca tgttttcctg ctggccctct ccccactatt 180
accctattgt cctgccacat ccccctctcc gagatggtag agataatgat caataaatac 240
tgagggaact cagagaccgg tgcggcgcgg gtcctccata tgctgagcgc cggtcccctg 300
ggcccacttt tctttctcta tactttgtct ctgttgtctt tcttttctca agtctctcgt 360
tccacctgag gagaaatgcc cacagctgtg gaggcgcagg ccactccatc tggtgcccaa 420
cgtggatgct tttctctagg gtgaagggac tctcgagtgt ggtcattgag gacaagtcaa 480
cgagagattc ccgagtacgt ctacagtgag ccttgtggta agcttgggcg ctcggaagaa 540
gccagggtta atggggcaaa ctaaaagtaa agtctctcat tccacctgat gagaaacacc 600
cagaggtgtg gaggggcagg ccaccccttc agggtagggt cccctccatg cagaccatag 660
agcacaggtg tgccccaaag aggagcagag agaaggaggg agagggccca cgagagactt 720
ggaaatgaat ggcaggattt taggcgctgg acttgggttc ggggcacctg gcctttcctt 780
gtgtatttct cctactgtct gcctaactat ttaatacaat aaaagaaaac cagcccctgg 840
ttcttgtggt gtttccaccc tcccgggtcc ccgctggctg cctggcttcc tcccgcagct 900
cctgctgtgt gtgtatgtgt gtgtgtgtgc acatctgtgg ggcgtatgtg tgttcgtctt 960
tgtaattgag gctgcagagt ggagagagca ggggttttct ctggggaccc agagagaagg 1020
aggcgttttc accacagccg aacagggcag gaccccagca cccgggaccc agcgggactt 1080
tgccaagggg atggacctgg ctgggccacg cggctgtttg tgtagggaaa agaaagagag 1140
atcacactgt tactgtgtct atgtagaaaa ggaagacata aactccattt tgagctgtac 1200
taagaaaaat tattttgcct tgacctgctg ttaacctgta actgtagccc caaccctgtg 1260
ctcaaagaaa catgtgctgt atggaatcaa ggtttaaggg atcaagggct gtacaggatg 1320
tgccttgtta acaatgtgtt tacaggcagt atgcttggta aaagtcatcg ccattctcca 1380
ttctccatta atcaggggca cgatgcactg cggaaagcca cagggacctc tgcccgagaa 1440
agcctgggta ttgtccaagg cttcccccca ctgagacagc ctgagatacg gcctcgtggg 1500
aagggaaaga cctgaccgtc ccccagcccg acacccgtaa agggtctgtg ctgaggagga 1560
ttagtaaaag gggaaggcct cttgcagttg agataagagg aaggcctccg tctcctgcat 1620
gtccttggga atggaatgtc ttggtgtaaa acccgatagt acattccttc tattctgaga 1680
gaagaaaacc accctgtggc tggaggtgag atatgctagc ggcaatgctg ctctgttact 1740
ctttgctaca ctgagatgtt tgggtggaga gaagcataaa tctggcctat gtgcacatct 1800
gggcacagaa cctccccttg aacttgtgac acagattcct ttgttcacat gttttcctgc 1860
tgaccttctc cccactatcg ccctgttctc ccaccgcatt ccccttgctg agatagtgaa 1920
aatagtaatc tgtagatacc aagggaactc agagaccatg gccggtgcac atcctccgta 1980
cgctgagcgc tggtcccctg ggcccattgt tctttctcta tactttgtct ctgtgtctta 2040
tttctttcct cagtctctca tccctcctga cgagaaatac ccacaggtgt ggaggggctg 2100
gcccccttca tctgatgccc aatgtgggtg cctttctcta gggtgaaggt actctacagt 2160
gtggtcattg aggacaagtt gacgagagag tcccaagtac gtccacggtc agccttgcgg 2220
taagcttgtg tgcttagagg aacccagggt aacgatgggg caaactgaaa gtaaatatgc 2280
ctcttatctc agctttatta aaattctttt aagaagaggg ggagttagag cttctacaga 2340
aaatctaatt acgctatttc aaacaataga acaattctgc ccatggtttc cagaacaggg 2400
aactttagat ctaaaagatt gggaaaaaat tggcaaagaa ttaaaacaag caaataggga 2460
aggtaaaatc atcccactta cagtatggaa tgattgggcc attattaaag caactttaga 2520
accatttcaa acaggagaag atattgtttc agtttctgat gcccctaaaa gctgtgtaac 2580
agattgtgaa gaagaggcag ggacagaatc ccagcaagga acggaaagtt cacattgtaa 2640
atatgtagca gagtctgtaa tggctcagtc aacgcaaaat gttgactaca gtcaattaca 2700
ggagataata taccctgaat catcaaaatt gggggaagga ggtccagaat cattggggcc 2760
atcagagcct aaaccacgat cgccatcaac tcctcctccc gtggttcaga tgcctgtaac 2820
attacaacct caaacgcagg ttagacaagc acaaacccca agagaaaatc aagtagaaag 2880
ggacagagtc tctatcccgg caatgccaac tcagatacag tatccacaat atcagccggt 2940
agaaaataag acccaaccgc tggtagttta tcaataccgg ctgccaaccg agcttcagta 3000
tcggcctcct tcagaggttc aatacagacc tcaagcggtg tgtcctgtgc caaatagcac 3060
ggcaccatac cagcaaccca cagcgatggc gtctaattca ccagcaacac aggacgcggc 3120

gctgtatcct cagccgccca ctgtgagact taatcctaca gcatcacgta gtggacaggg 3180
tggtgcactg catgcagtca ttgatgaagc cagaaaacag ggcgatcttg aggcatggcg 3240
gttcctggta attttacaac tggtacaggc cggggaagag actcaagtag gagcgcctgc 3300
ccgagctgag actagatgtg aacctttcac catgaaaatg ttaaaagata taaaggaagg 3360
agttaaacaa tatggatcca actcccctta tataagaaca ttattagatt ccattgctca 3420
tggaaataga cttactcctt atgactggga aattttggcc aaatcttccc tttcatcctc 3480
tcagtatcta cagtttaaaa cctggtggat tgatggagta caagaacagg tacgaaaaaa 3540
tcaggctact aagcccactg ttaatataga cgcagaccaa ttgttaggaa caggtccaaa 3600
ttggagcacc attaaccaac aatcagtgat gcagaatgag gctattgaac aagtaagggc 3660
tatttgcctc agggcctggg gaaaaattca ggacccagga acagctttcc ctattaattc 3720
aattagacaa ggctctaaag agccatatcc tgactttgtg gcaagattac aagatgctgc 3780
tcaaaagtct attacagatg acaatgcccg aaaagttatt gtagaattaa tggcctatga 3840
aaatgcaaat ccagaatgtc agtcggccat aaagccatta aaaggaaaag ttccagcagg 3900
agttgatgta attacagaat atgtgaaggc ttgtgatggg attggaggag ctatgcataa 3960
ggcaatgcta atggctcaag caatgagggg gctcactcta ggaggacaag ttagaacatt 4020
tgggaaaaaa tgttataatt gtggtcaaat cggtcatctg aaaaggagtt gcccaggctt 4080
aaataaacag aatataataa atcaagctat tacagcaaaa aataaaaagc catctggcct 4140
gtgtccaaaa tgtggaaaag caaaacattg ggccaatcaa tgtcattcta aatttgataa 4200
agatgggcaa ccattgtctg gaaacaggaa gaggggccag cctcaggccc cccaacaaac 4260
tggggcattc ccagttaaac tgtttgttcc tcagggtttt caaggacaac aacccctaca 4320
gaaaatacca ccacttcagg gagtcagcca attacaacaa tccaacagct gtcccgcgcc 4380
acagcaggca gcaccgcagt agatttatgt tccacccaaa tggtcttttt actccctgga 4440
aagcccccac aaaagattcc tagaggggta tatggcccgc tgccagaagg gagggtaggc 4500
ctttgaggga gatcaagtct aaatttgaag ggagtccaaa ttcatactgg ggtaatttat 4560
tcagattata aagggggaat tcagttagtg atcagctcca ctgttccccg gagtgccaat 4620
ccaggtgata gaattgctca attactgctt ttgccttatg ttaaaattgg ggaaaacaaa 4680
aaggaaagaa caggagggtt tggaagtacc aaccctgcag gaaaagctgc ttattgggct 4740
aatcaggtct cagaggatag acccgtgtgt acagtcacta ttcagggaaa gagtttgaag 4800
gattagtgga tacccaggct gatgtttctg tcatcggcat aggtactgcc tcagaagtgt 4860
atcaaagtgc catgatttta cattgtccag gatctgataa tcaagaaagt acggttcagc 4920
ctgtgatcac ttcattccaa tcaatttatg gggccgagac ttgttacaac aatggcatgc 4980
agagattact atcccagcct ccctatacag ccccaggaat aaaaaaatca tgactaaaat 5040
gggatagctc cctaaaaagg gactaggaaa gaagtcccaa ttgaggctga aaaaaatcaa 5100
aaaagaaaag gaatagggca tcctttttag gagcggtcac tgtagagcct ccaaaaccca 5160
ttccattaac ttgggggaaa aaaaaacaac tgtatggtaa atcagcagcg cttccaaaac 5220
aaaaactgga ggctttacat ttattagcaa agaaacaatt agaaaaagga cattgagcct 5280
tcattttcgc cttggaattc tgtttgtaat tcagaaaaaa tccggcagat ggcgtataat 5340
gccgtaattc aacccatggg ggctctccca ccccggttgc cctctccagc catggtcccc 5400
tttaattata attgatctga aggattgctt ttttaccatt cctctggcaa aacaggattt 5460
tgaaaaattt gcttttacca caccagccta aataataaag aaccagccac caggtttcag 5520
tggaaagtat tgcctcaggg aatgcttaat agttcaacta tttgtcagct caagctctgc 5580
aaccagttag agacaagttt tcagactgtt acatcgttca ctatgttgat attttgtgtg 5640
ctgcagaaac gagagacaaa ttaattgacc gttacacatt tctgcagaca gaggttgcca 5700
acgcgggact gacaataaca tctgataaga ttcaaacctc tactcctttc cgttacttgg 5760
gaatgcaggt agaggaaagg aaaattaaac cacaaaaaat agaaataaga aaagacacat 5820
taaaagcatt aaatgagttt caaaagttgc taggagatac taattggatt tggagatatt 5880
aattggattt ggccaactct aggcattcct acttatgcca tgtcaaattt gttctctttc 5940
ttaagagggg actcggaatt aaatagtgaa agaacgttaa ctccagaggc aactaaagaa 6000
attaaattaa ttgaagaaaa aattcggtca gcacaagtaa atagaataga tcacttggcc 6060
ccactccaaa ttttgatttt tgctactgca cattccctaa caggcatcat tgttcaaaat 6120
acagatcttg tggagtggtc cttccttcct cacagtacaa ttaagacttt tacattgtac 6180
ttggatcaaa tggctacatt aattggtcag ggaagattat gaataataac attgtgtgga 6240
aatgacccag ataaaatcac tgttcctttc aacaagcaac aggttagaca agcctttatc 6300
aattctggtg catggcagat tggtcttgcc gattttgtgg gaattattga caatcgttac 6360
cccaaaacaa aaatcttcca gtttttaaaa ttgactactt ggattttacc taaagttacc 6420
aaacataagc ctttaaaaaa tgctctggca gtgtttactg atggttccag caatggaaaa 6480
gtggcttaca ccgggccaaa agaatgagtc atcaaaactc agtatcactt gactcaaaga 6540
gcagagttgg ttgccgtcat tacagtgtta acaagatttt aatcagtcta ttaacattgt 6600
atcagattct gcatatgtag tacaggctac aaaggatatt gagagagccc taatcaaata 6660
cattatggat gatcagttaa acccgctgtt taatttgtta caacaaaatg taagaaaaag 6720
aaatttccca ttttatatta ctcatattcg agcacacact aatttaccag ggcctttaac 6780

taaagcaaat gaacaagctg acttgctagt atcatctgca ttcatggaag cacaagaact 6840
tcatgccttg actcatgtaa atgcaatagg attaaaaaat aaatttgata tcacatggaa 6900
acagacaaaa aatattgtac aacattgcac ccagtgtcag attctacacc tggccactca 6960
ggaggcaaga gttaatccca gaggtctatg tcctaatgtg ttatggcaaa tggatgtcat 7020
gcacgtacct tcatttggaa aattgtcatt tgtccatgtg acagttgata cttattcaca 7080
tttcatatgg gcaacctgcc agacaggaga aagtacttcc catgttaaaa gacatttatt 7140
atcttgtttt cctgtcatgg gagttccaga aaaagttaaa acagacaatg ggccaggtta 7200
ctgtagtaaa gcagttcaaa aattcttaaa tcagtggaaa attacacata caataggaat 7260
tctctataat tcccaaggac aggccataat tgaaagaact aatagaacac tcaaagctca 7320
attggttaaa caaaaaaaag gaaaagacag gagtataaca ctccccagat gcaacttaat 7380
ctagcactct atactttaaa tgttttaaac atttatagaa atcagaccac tacctctgca 7440
gaacaacatc ttactggtaa aaggaacagc ccacatgaag gaaaactgat ttggtggaaa 7500
gataataaaa ataaaacatg ggaaatgggg aaggtgataa cgtgggggag aggttttgct 7560
tgtgtttcac caggagaaaa tcagcttcct gtttggatac ccactagaca tttaaagttc 7620
tacaatgaac tcactggaga tgcaaagaaa agtgtggaga tggagacacc ccaatcgact 7680
cgccaggtaa acaaaatggt gatatcagaa gaacagaaaa agttgccttc catcaaggaa 7740
gcagagttgc caatataggc acaattaaag aagctgacac agttagctaa aaaaaaaagc 7800
ctagagaata caaaggtgac accaactcca gagaatatgc tgcttgcagc tctgatgatt 7860
gtatcaacgg tggtaagtct tcccaagtct gcaggagcag ctgcagctaa ttatacttac 7920
tgggcctatg tgcctttccc acccttaatt cgggcagtta catagatgga taatcctatt 7980
gaagtagatg ttaataatag tgcatgggtg cctggcccca cagatgactg ttgccctgcc 8040
caacctgaag aaggaatgat gatgaatatt tccattgggt atccttatcc tcctgtttgc 8100
ctagggaagg caccaggatg cttaatgcct acaacccaaa attggttggt agaagtacct 8160
acagtcagtg ctaccagtag atttacttat cacatggtaa gtggaatgtc acagataaat 8220
aatttacagg acccttctta tcaaagatca ttacaatgta ggcctaaggg gaaggcttgc 8280
cccaaggaaa ttcccaaaga atcaaaaagc ccagaagtct tagtctgcgg agaatgtgtg 8340
gctgatactg cagtgtagta caaaacaatg aattttgaac tatgatagac tgggtccctt 8400
gaggccaatt atatcataac tgtacaggcc agactcattc atgttcacag gccccatcca 8460
tctggcccat taatccagcc tatgacggtg atgtaactga aaggctggac caggtttata 8520
gaaggttaga atcactctgt ccaaggaaat ggggtgaaaa gggaatttca tcaccttgac 8580
caaagttagt cctgttactg gtcctgaaca tccagaatta ggaagcttac tgtggcctca 8640
caccacatta gaatttgttc tggaaatcaa gctataggaa caagagatcg taagtcatat 8700
tatactatca acctaaattc cagtctgaca attcctttgc aaaattgtgt aaaactccct 8760
tatattgcta gttgtaggaa aaacatagtt attaaacctg attcccaaac cataatctgt 8820
gaaaattgtg gaatgtttac ttgcattgat ttgactttta attggcagca ccgtattcta 8880
ctaggaagag caagagaggg tgtgtggatc cttgtgtcca tggaccgacc atgggaggct 8940
tcgctatcca tccatatttt aacggaagta ttaaaaggaa ttctaactag atccaaaaga 9000
ttcattttta ctttgatggc agtgattatg ggcctcattg cagtcacagc tactgctgcg 9060
gctgctggaa ttgctttaca ctcctctgtt caaactgcag aatacgtaaa tgattggcaa 9120
aagaattcct caaaattgtg gaattctcag atccaaatag atcaaaaatt ggcaaaccaa 9180
attaatgatc ttagacaaac tgtcatttgg atgggagagg ctcatgagct tggaatatct 9240
tttcagttta cgatgtgact ggaatacatc agatttttgt gttacaccac aagcctataa 9300
tgagtctgag catcactggg acatggttag atgccatctg caaggaggag aagataatct 9360
tactttagac atttcaaaat taaaagaatt ttttttttct ttgagacaga gtctcgctct 9420
gtcgcccagg ctggagtgca gtggcgtgat ctcagctcac tgcaagttcc gcctcctggg 9480
tttacaccat tctcctgcct cagcctccca agtagttggg actacaggag cccaccacca 9540
tgcctggcta attttttttg ggtttttaat agagatggag tttcaccgtg ttagccagga 9600
tggtctcgat ctcctgacct tgtgatctgc ccaccttggc ctcccaaagt gctgggatta 9660
cagtcgtgag ccaccgtgcc cagccaagaa aaaatttttg aggcatcaaa agcccattta 9720
aatttggtgc caggaacgga gacaatcgtg aaagctgctg atagcctcac aaatcttaag 9780
ccagtcactt gggttaaaag catcagaagt ttcactattg taaatttcat attaatcctt 9840
gtatgcctgt tctgtctgtt gttagtctac aggtgtatcc agcagctcca aagagacagc 9900
aaccagcaag aatgggccat agtgacgatg gtggttttgt caaaaagaaa agggggggat 9960
atgtaaggaa aagagagatc agactttcac tgtgtctatg tagaaaagga agacataaga 10020
aactccattt tgatctgtac taagaaaaat tgttttgcct tgagatgctg ttaatctgta 10080
actttagccc caaccctgtg ctcacggaaa catgtgctgt aaggtttaag ggatctaggg 10140
ctgtgcagga tgtaccttgt taacaatatg tttgcaggca gtatgtttgg taaaagtcat 10200
cgccattctc cattctcgat taaccagggg ctcaatgcac tgtggaaagc cacaggaacc 10260
tctgcccaag aaagcctggc tgttgtggga agtcagggac cccgaatgga gggaccagct 10320
ggtgctgcat caggaaacat aaattgtgaa gatttcttgg acatttatca gtttccaaaa 10380
ttaatacttt tataatttct tacacctgtc ttactttaat ctcttaatcc tgttatcttt 10440

gtaagctgag gatatacgtc acctcaggac cactattgta caaattgatt gtaaaacatg 10500
ttcacatgtg tttgaacaat atgaaatcag tgcaccttga aaatgaacag aataacagtg 10560
atttagggga acaaaggaag acaaccataa ggtctgactg cctgaggggt cgggcaaaaa 10620
gccatatttt tcttcttgca gagagcctat aaatggacgt gcaagtagga gagatattgc 10680
taaattcttt tcctagcaag gaatataata ctaagaccct agggaaagaa ttgcattcct 10740
ggggggaggt ctataaacgg ccgctctggg agtgtctgtc ctatgtggtt gagataagga 10800
ctgagatacg ccctggtctc ctgcagtacc ctcaggctta ctaggattgg gaaaccccag 10860
tcctggtaaa tttgaggtca ggccggttct ttgctctgaa ccctgttttc tgttaagatg 10920
tttatcaaga caatacatgc accgctgaac atagaccctt atcaggagtt tctgattttg 10980
ctctggtcct gtttcttcag aagcatgtca tctttgctct gccttctgcc ctttgaagca 11040
tgtgatcttt gtgacctact ccctgttcat acacccctcc ccttttaaaa tccctaataa 11100
aaacttgctg gttttgtggc tcaggggggc atcatggacc taccaatacg tgatgtcacc 11160
cccggtggcc cagctgt 11177

<210> 11
<211> 8
<212> DNA
<213> HERV-K

<400> 11
taggtctc 8

<210> 12
<211> 8
<212> DNA
<213> HERV-K

<400> 12
taggggtg 8

<210> 13
<211> 8
<212> DNA
<213> HERV-K

<400> 13
tggctgtt 8

<210> 14
<211> 204
<212> DNA
<213> HERV-K

<400> 14
aacgtggatg cttttctcta gggtgaaggg actctcgagt gtggtcattg aggacaagtc 60
aacgagagat tcccgagtac gtctacagtg agccttgtgg gtgaaggtac tctacagtgt 120
ggtcattgag gacaagttga cgagagagtc ccaagtacgt ccacggtcag ccttgcgaca 180
ttttaaagtt ctacaatgaa ctca 204

<210> 15
<211> 503
<212> DNA
<213> HERV-K

<400> 15
acagaagggt acatgaagga aaactgattt ggtggaaaga taataaaaat aaaacatggg 60
aaatggggaa ggtgataacg tgggggagag gttttgcttg tgtttcacca ggagaaaatc 120
agcttcctgt ttggataccc actagacatt taaagttcta caatgaactc actggagatg 180
caaagaaaag tgtggagatg gagacacccc aatcgactcg ccagtctaca ggtgtatcca 240
gcagctccaa agagacagca accagcaaga atgggccata gtgacgatgg tggttttgtc 300
aaaaagaaaa gggggggata tgtaaggaaa agagagatca gactttcact gtgtctatgt 360

agaaaaggaa gacataagaa actccatttt gatctgtact aagaaaaatt gttttgcctt 420
gagatgctgt taatctgtaa ctttagcccc agccctgtgc tcacggaaac atgtgctgta 480
aggtttaagg gatctagggc tgt 503

<210> 16
<211> 637
<212> DNA
<213> HERV-K

<400> 16
agtcatcaaa actcagtatc acttgactca aagagcagag ttggttgccg tcattacagt 60
gttaacaaga ttttaatcag tctattaaca ttgtatcaga ttctgcatat gtagtacagg 120
ctacaaagga tattgagaga gccctaatca aatacattat ggatgatcag ttaaacccgc 180
tgtttaattt gttacaacaa aatgtaagaa aaagaaattt cccattttat attactcata 240
ttcgagcaca cactaattta ccagggcctt taactaaagc aaatgaacaa gctgactcgc 300
tagtatcatc tgcattcatg gaagcacaag accttcatgc cttgactcat gtaaatgcaa 360
taggattaaa aaataaattt aatatcacat ggaaacagac aaaaaatatt gtacaacatt 420
gcacccagtg tcagattcta cacctggcca ctcaggaggc aagagttaat cccagaggtc 480
tatgtcctaa tgtgttatgg caaatggatg tcatgcacgt accttcattt ggaaaattgt 540
catttgtcca tgtgacagtt gatacttatt cacatttcat atgggcaacc tgccagacag 600
gagaaagtac ttcccatgtt aagagacatt tattatc 637

<210> 17
<211> 650
<212> DNA
<213> HERV-K

<400> 17
agtcatcaaa actcagtatc acttgactca aagagcagag ttggttgccg tcattacagt 60
gttaacaaga ttttaatcag tctattaaca ttgtatcaga ttctgcatat gtagtacagg 120
ctacaaagga tattgagaga gccctaatca aatacattat ggatgatcag ttaaacccgc 180
tgtttaattt gttacaacaa aatgtaagaa aaagaaattt cccattttat attactcata 240
ttcgagcaca cactaattta ccagggcctt taactaaagc aaatgaacaa gctgacttgc 300
tagtatcatc tgcattcatg gaagcacaag aacttcatgc cttgactcat gtaaatgcaa 360
taggattaaa aaataaattt gatatcacat ggaaacagac aaaaaatatt gtacaacatt 420
gcacccagtg tcagattcta cacctggcca ctcaggaggc aagagttaat cccagaggtc 480
tatgtcctaa tgtgttatgg caaatggatg tcatgcacgt accttcattt ggaaaattgt 540
catttgtcca tgtgacagtt gatacttatt cacatttcat atgggcaacc tgccagacag 600
gagaaagtac ttcccatgtt aaaagacatt tattatcttg ttttcctgtc 650

<210> 18
<211> 650
<212> DNA
<213> HERV-K

<400> 18
agtcatcaaa actcagtatc acttgactca aagagcagag ttggttgccg tcattacagt 60
gttaacaaga ttttaatcag tctattaaca ttgtatcaga ttctgcatat gtagtacagg 120
ctacaaagga tattgagaga gccctaatca aatacattat ggatgatcag ttaaacccgc 180
tgtttaattt gttacaacaa aatgtaagaa aaagaaattt cccattttat attactcata 240
ttcgagcaca cactaattta ccagggcctt taactaaagc aaatgaacaa gctgacttgc 300
tagtatcatc tgcattcatg gaagcacaag aacttcatgc cttgactcat gtaaatgcaa 360
taggattaaa aaataaattt gatatcacat ggaaacagac aaaaaatatt gtacaacatt 420
gcacccagtg tcagattcta cacctggcca ctcaggaggc aagagttaat cccagaggtc 480
tatgtcctaa tgtgttatgg caaatggatg tcatgcacgt accttcattt ggaaaattgt 540
catttgtcca tgtgacagtt gatacttatt cacatttcat atgggcaacc tgccagacag 600
gagaaagtac ttcccatgtt aaaagacatt tattatcttg ttttcctgtc 650

<210> 19
<211> 200
<212> DNA

<213>   HERV-K

<400>   19
agatctgatc atctggtgcc caacgtggag gcttttctct agggtgaagg gactctcgag   60
tgtggtcatt gaggacaagt caacgagaga ttcccgagta cgtctacagt gagccttgtg   120
ggtgaaggta ctctacagtg tggtcattga ggacaagttg acgagagagt cccaagtacg   180
tccacggtca gccttgcgac                                               200

<210>   20
<211>   53
<212>   DNA
<213>   Artificial Sequence

<220>
<223>   RACE primer

<400>   20
aactggaaga attcgcggcc gcattttttt tttttttttt tttttttttt ttt          53

<210>   21
<211>   20
<212>   DNA
<213>   Artificial Sequence

<220>
<223>   RACE primer

<400>   21
caggtgtayc carcagctcc                                               20

<210>   22
<211>   23
<212>   DNA
<213>   Artificial Sequence

<220>
<223>   RACE primer

<400>   22
aactggaaga attcgcggcc gca                                           23

<210>   23
<211>   11177
<212>   RNA
<213>   HERV-K

<400>   23
gagauaggag aaaacugccu uagggcugga ggugggacau gcuggcggca auacugcucu   60
uuaaggcauu gagauguuua uguauaugca caucaaaagc acagcacuuu uuucuuuacc   120
uuguuuauga ugcagagaca uuuguucaca uguuuuccug cuggcccucu ccccacuauu   180
acccuauugu ccugccacau cccccucucc gagaugguag agauaaugau caauaaauac   240
ugagggaacu cagagaccgg ugcggcgcgg guccuccaua ugcugagcgc cgguccccug   300
ggcccacuuu ucuuucucua uacuuugucu cuguugucuu ucuuuucuca agucucucgu   360
uccaccugag gagaaaugcc cacagcugug gaggcgcagg ccacuccauc uggugcccaa   420
cguggaugcu uuucucuagg gugaagggac ucucgagugu ggucauugag gacaagucaa   480
cgagagauuc ccgaguacgu cuacagugag ccuuguggua agcuugggcg cucggaagaa   540
gccaggguua auggggcaaa cuaaaaguaa agucucucau uccaccugau gagaaacacc   600
cagaggugug gaggggcagg ccaccccuuc aggguagggu ccccuccaug cagaccauag   660
agcacaggug ugccccaaag aggagcagag agaaggaggg agagggccca cgagagacuu   720
ggaaaugaau ggcaggauuu uaggcgcugg acuuggguuc ggggcaccug gccuuuccuu   780
guguauuucu ccuacugucu gccuaacuau uuaauacaau aaaagaaaac cagccccugg   840

uucuugugggu guuuccaccc ucccgggucc ccgcuggcug ccuggcuucc ucccgcagcu 900
ccugcugugu guguaugugu gugugugugc acaucugugg ggcguaugug uguucgucuu 960
uguaauugag gcugcagagu ggagagagca gggguuuucu cuggggaccc agagagaagg 1020
aggcguuuuc accacagccg aacagggcag gaccccagca cccgggaccc agcgggacuu 1080
ugccaagggg auggaccugg cugggccacg cggcuguuug uguagggaaa agaaagagag 1140
aucacacugu uacugugucu auguagaaaa ggaagacaua aacuccauuu ugagcuguac 1200
uaagaaaaau uauuuugccu ugaccugcug uuaaccugua acuguagccc caacccugug 1260
cucaaagaaa caugugcugu auggaaucaa gguuuaaggg aucaagggcu guacaggaug 1320
ugccuuguua acaauguguu uacaggcagu augcuuggua aaagucaucg ccauucucca 1380
uucuccauua aucaggggca cgaugcacug cggaaagcca cagggaccuc ugcccgagaa 1440
agccugggua uuguccaagg cuucccccca cugagacagc cugagauacg gccucguggg 1500
aagggaaaga ccugaccguc ccccagcccg acacccguaa agggucugug cugaggagga 1560
uuaguaaaag gggaaggccu cuugcaguug agauaagagg aaggccuccg ucuccugcau 1620
guccuuggga auggaauguc uugguguaaa acccgauagu acauuccuuc uauucugaga 1680
gaagaaaacc acccuguggc uggaggugag auaugcuagc ggcaaugcug cucuguuacu 1740
cuuugcuaca cugagauguu uggguggaga gaagcauaaa ucuggccuau gugcacaucu 1800
gggcacagaa ccuccccuug aacuugugac acagauuccu uuguucacau guuuuccugc 1860
ugaccuucuc cccacuaucg cccuguucuc ccaccgcauu ccccuugcug agauagugaa 1920
aauaguaauc uguagauacc aagggaacuc agagaccaug gccggugcac auccuccgua 1980
cgcugagcgc ugguccccug ggcccauugu ucuuucucua uacuuugucu cugugucuua 2040
uuucuuuccu cagucucuca ucccuccuga cgagaaauac ccacaggugu ggaggggcug 2100
gcccccuuca ucugaugccc aaugugggug ccuuucucua gggugaaggu acucuacagu 2160
guggucauug aggacaaguu gacgagagag ucccaaguac guccacgguc agccuugcgg 2220
uaagcuugug ugcuuagagg aacccagggu aacgaugggg caaacugaaa guaaauaugc 2280
cucuuaucuc agcuuuauua aaauucuuuu aagaagaggg ggaguuagag cuucuacaga 2340
aaaucuaauu acgcuauuuc aaacaauaga acaauucugc ccaugguuuc cagaacaggg 2400
aacuuuagau cuaaaagauu gggaaaaaau uggcaaagaa uuaaaacaag caaauaggga 2460
agguaaaauc aucccacuua caguauggaa ugauugggcc auuauuaaag caacuuuaga 2520
accauuucaa acaggagaag auauuguuuc aguuucugau gccccuaaaa gcuguguaac 2580
agauugugaa gaagaggcag ggacagaauc ccagcaagga acggaaaguu cacauuguaa 2640
auauguagca gagucuguaa uggcucaguc aacgcaaaau guugacuaca gucaauuaca 2700
ggagauaaua uacccugaau caucaaaauu gggggaagga gguccagaau cauuggggcc 2760
aucagagccu aaaccacgau cgccaucaac uccuccuccc gugguucaga ugccuguaac 2820
auuacaaccu caaacgcagg uuagacaagc acaaacccca agagaaaauc aaguagaaag 2880
ggacagaguc ucuaucccgg caaugccaac ucagauacag uauccacaau aucagccggu 2940
agaaaauaag acccaaccgc ugguaguuua ucaauaccgg cugccaaccg agcuucagua 3000
ucggccuccu ucagagguuc aauacagacc ucaagcggug uguccugugc caaauagcac 3060
ggcaccauac cagcaaccca cagcgauggc gucuaauuca ccagcaacac aggacgcggc 3120
gcuguauccu cagccgccca cugugagacu uaauccuaca gcaucacgua guggacaggg 3180
uggugcacug caugcaguca uugaugaagc cagaaaacag ggcgaucuug aggcauggcg 3240
guuccuggua auuuuacaac ugguacaggc cggggaagag acucaaguag gagcgccugc 3300
ccgagcugag acuagaugug aaccuuucac caugaaaaug uuaaaagaua uaaaggaagg 3360
aguuaaacaa uauggaucca acuccccuua uauaagaaca uuauuagauu ccauugcuca 3420
uggaaauaga cuuacuccuu augacuggga aauuuuggcc aaaucuuccc uuucauccuc 3480
ucaguaucua caguuuaaaa ccugguggau ugauggagua caagaacagg uacgaaaaaa 3540
ucaggcuacu aagcccacug uuaauauaga cgcagaccaa uuguuaggaa cagguccaaa 3600
uuggagcacc auuaaccaac aaucagugau gcagaaugag gcuauugaac aaguaagggc 3660
uauuugccuc agggccuggg gaaaaauuca ggacccagga acagcuuucc cuauuaauuc 3720
aauuagacaa ggcucuaaag agccauaucc ugacuuugug gcaagauuac aagaugcugc 3780
ucaaaagucu auuacagaug acaaugcccg aaaaguuauu guagaauuaa uggccuauga 3840
aaaugcaaau ccagaauguc agucggccau aaagccauua aaaggaaaag uuccagcagg 3900
aguugaugua auuacagaau augugaaggc uugugauggg auuggaggag cuaugcauaa 3960
ggcaaugcua auggcucaag caaugagggg gcucacucua ggaggacaag uuagaacauu 4020
ugggaaaaaa uguuauaauu guggucaaau cggucaucug aaaaggaguu gcccaggcuu 4080
aaauaaacag aauauaauaa aucaagcuau uacagcaaaa aauaaaaagc caucuggccu 4140
guguccaaaa uguggaaaag caaaacauug ggccaaucaa ugucauucua aauuugauaa 4200
agaugggcaa ccauugucug gaaacaggaa gaggggccag ccucaggccc cccaacaaac 4260
uggggcauuc ccaguuaaac uguuuguucc ucaggguuuu caaggacaac aaccccuaca 4320
gaaaauacca ccacuucagg gagucagcca auuacaacaa uccaacagcu gucccgcgcc 4380
acagcaggca gcaccgcagu agauuuaugu uccacccaaa uggucuuuuu acucccugga 4440
aagcccccac aaaagauucc uagaggggua uauggcccgc ugccagaagg gaggguaggc 4500

cuuugaggga gaucaagucu aaauuugaag ggaguccaaa uucauacugg gguaauuuau 4560
ucagauuaua aagggggaau ucaguuagug aucagcucca cuguuccccg gagugccaau 4620
ccaggugaua gaauugcuca auuacugcuu uugccuuaug uuaaaauugg ggaaaacaaa 4680
aaggaaagaa caggaggguu uggaaguacc aacccugcag gaaaagcugc uuauugggcu 4740
aaucaggucu cagaggauag acccgugugu acagucacua uucagggaaa gaguuugaag 4800
gauuagugga uacccaggcu gauguuucug ucaucggcau agguacugcc ucagaagugu 4860
aucaaagugc caugauuuua cauuguccag gaucugauaa ucaagaaagu acgguucagc 4920
cugugaucac uucauuccaa ucaauuuaug gggccgagac uuguuacaac aauggcaugc 4980
agagauuacu aucccagccu cccuauacag ccccaggaau aaaaaaauca ugacuaaaau 5040
gggauagcuc ccuaaaaagg gacuaggaaa gaagucccaa uugaggcuga aaaaaaucaa 5100
aaaagaaaag gaauagggca uccuuuuuag gagcggucac uguagagccu ccaaacccca 5160
uuccauuaac uugggggaaa aaaaaacaac uguaugguaa aucagcagcg cuuccaaaac 5220
aaaaacugga ggcuuuacau uuauuagcaa agaaacaauu agaaaaagga cauugagccu 5280
ucauuuucgc cuuggaauuc uguuuguaau ucagaaaaaa uccggcagau ggcguauaau 5340
gccguaauuc aacccauggg ggcucuccca ccccgguugc ccucuccagc cauggucccc 5400
uuuaauuaua auugaucuga aggauugcuu uuuuaccauu ccucuggcaa aacaggauuu 5460
ugaaaaauuu gcuuuuacca caccagccua aauaauaaag aaccagccac cagguuucag 5520
uggaaaguau ugccucaggg aaugcuuaau aguucaacua uuugucagcu caagcucugc 5580
aaccaguuag agacaaguuu ucagacuguu acaucguuca cuauguugau auuuugugug 5640
cugcagaaac gagagacaaa uuaauugacc guuacacauu ucugcagaca gagguugcca 5700
acgcgggacu gacaauaaca ucugauaaga uucaaaccuc uacuccuuuc cguuacuugg 5760
gaaugcaggu agaggaaagg aaaauuaaac cacaaaaaau agaaauaaga aaagacacau 5820
uaaaagcauu aaaugaguuu caaaaguugc uaggagauac uaauuggauu uggagauauu 5880
aauuggauuu ggccaacucu aggcauuccu acuuaugcca ugucaaauuu guucucuuuc 5940
uuaagagggg acucggaauu aaauagugaa agaacguuaa cuccagaggc aacuaaagaa 6000
auuaaauuaa uugaagaaaa aauucgguca gcacaaguaa auagaauaga ucacuuggcc 6060
ccacuccaaa uuuugauuuu ugcuacugca cauucccuaa caggcaucau uguucaaaau 6120
acagaucuug uggagugguc cuuccuuccu cacaguacaa uuaagacuuu uacauuguac 6180
uuggaucaaa uggcuacauu aauuggucag ggaagauuau gaauaauaac auugugugga 6240
aaugacccag auaaaaucac uguuccuuuc aacaagcaac agguuagaca agccuuuauc 6300
aauucuggug cauggcagau uggucuugcc gauuuugugg gaauuauuga caaucguuac 6360
cccaaaacaa aaaucuucca guuuuuaaaa uugacuacuu ggauuuuacc uaaaguuacc 6420
aaacauaagc cuuuaaaaaa ugcucuggca guguuuacug augguuccag caauggaaaa 6480
guggcuuaca ccgggccaaa agaaugaguc aucaaaacuc aguaucacuu gacucaaaga 6540
gcagaguugg uugccgucau uacaguguua acaagauuuu aaucagucua uuaacauugu 6600
aucagauucu gcauauguag uacaggcuac aaaggauauu gagagagccc uaaucaaaua 6660
cauuauggau gaucaguuaa acccgcuguu uaauuuguua caacaaaaug uaagaaaaag 6720
aaauuuccca uuuuauauua cucauauucg agcacacacu aauuuaccag ggccuuuaac 6780
uaaagcaaau gaacaagcug acuugcuagu aucaucugca uucauggaag cacaagaacu 6840
ucaugccuug acucauguaa augcaauagg auuaaaaaau aaauuugaua ucacauggaa 6900
acagacaaaa aauauuguac aacauugcac ccagugucag auucuacacc uggccacuca 6960
ggaggcaaga guuaauccca gaggucuaug uccuaaugug uuauggcaaa uggaugucau 7020
gcacguaccu ucauuuggaa aauugucauu uguccaugug acaguugaua cuuauucaca 7080
uuucauaugg gcaaccugcc agacaggaga aaguacuucc cauguuaaaa gacauuuauu 7140
aucuuguuuu ccugucaugg gaguuccaga aaaaguuaaa acagacaaug ggccagguua 7200
cuguaguaaa gcaguucaaa aauucuuaaa ucaguggaaa auuacacaua caauaggaau 7260
ucucuauaau ucccaaggac aggccauaau ugaaagaacu aauagaacac ucaaagcuca 7320
auugguuaaa caaaaaaaag gaaaagacag gaguauaaca cuccccagau gcaacuuaau 7380
cuagcacucu auacuuuaaa uguuuuaaac auuuauagaa aucagaccac uaccucugca 7440
gaacaacauc uuacugguaa aaggaacagc ccacaugaag gaaaacugau uugguggaaa 7500
gauaauaaaa auaaaacaug ggaaaugggg aaggugauaa cgugggggag agguuuugcu 7560
uguguuucac caggagaaaa ucagcuuccu guuuggauac ccacuagaca uuuaaaguuc 7620
uacaaugaac ucacuggaga ugcaaagaaa aguguggaga uggagacacc ccaaucgacu 7680
cgccagguaa acaaaauggu gauaucagaa gaacagaaaa aguugccuuc caucaaggaa 7740
gcagaguugc caauauaggc acaauuaaag aagcugacac aguuagcuaa aaaaaaaagc 7800
cuagagaaua caaaggugac accaacucca gagaauaugc ugcuugcagc ucugaugauu 7860
guaucaacgg ugguaagucu ucccaagucu gcaggagcag cugcagcuaa uuauacuuac 7920
ugggccuaug ugccuuuccc acccuuaauu cgggcaguua cauagaugga uaauccuauu 7980
gaaguagaug uuaauaauag ugcaugggug ccuggcccca cagaugacug uugcccugcc 8040
caaccugaag aaggaaugau gaugaauauu uccauugggu auccuuaucc uccuguuugc 8100
cuagggaagg caccaggaug cuuaaugccu acaacccaaa auugguuggu agaaguaccu 8160

acagucagug cuaccaguag auuuacuuau cacaugguaa guggaauguc acagauaaau 8220
aauuuacagg acccuucuua ucaaagauca uuacaaugua ggccuaaggg gaaggcuugc 8280
cccaaggaaa uucccaaaga aucaaaaagc ccagaagucu uagucugcgg agaaugugug 8340
gcugauacug caguguagua caaaacaaug aauuuugaac uaugauagac ugggucccuu 8400
gaggccaauu auaucauaac uguacaggcc agacucauuc auguucacag gccccaucca 8460
ucuggcccau uaauccagcc uaugacggug auguaacuga aaggcuggac cagguuuaua 8520
gaagguuaga aucacucugu ccaaggaaau ggggugaaaa gggaauuuca ucaccuugac 8580
caaaguuagu ccuguuacug guccugaaca uccagaauua ggaagcuuac uguggccuca 8640
caccacauua gaauuuguuc uggaaaucaa gcuauaggaa caagagaucg uaagucauau 8700
uauacuauca accuaaauuc cagucugaca auuccuuugc aaaauugugu aaaacucccu 8760
uauauugcua guuguaggaa aaacauaguu auuaaaccug auucccaaac cauaaucugu 8820
gaaaauugug gaauguuuac uugcauugau uugacuuuua auuggcagca ccguauucua 8880
cuaggaagag caagagaggg uguguggauc cuugugucca uggaccgacc augggaggcu 8940
ucgcuaucca uccauauuuu aacggaagua uuaaaaggaa uucuaacuag auccaaaaga 9000
uucauuuuua cuuugauggc agugauuaug ggccucauug cagucacagc uacugcugcg 9060
gcugcuggaa uugcuuuaca cuccucuguu caaacugcag aauacguaaa ugauuggcaa 9120
aagaauuccu caaaauugug gaauucucag auccaaauag aucaaaaauu ggcaaaccaa 9180
auuaaugauc uuagacaaac ugucauuugg augggagagg cucaugagcu uggaauaucu 9240
uuuucaguua cgaugugacu ggaauacauc agauuuuugu guuacaccac aagccuauaa 9300
ugagucugag caucacuggg acaugguuag augccaucug caaggaggag aagauaaucu 9360
uacuuuagac auuucaaaau uaaaagaauu uuuuuuuucu uugagacaga gucucgcucu 9420
gucgcccagg cuggagugca guggcgugau cucagcucac ugcaaguucc gccuccuggg 9480
uuuacaccau ucuccugccu cagccuccca aguaguuggg acuacaggag cccaccacca 9540
ugccuggcua auuuuuuuug gguuuuuaau agagauggag uuucaccgug uuagccagga 9600
uggucucgau cuccugaccu ugugaucugc ccaccuuggc cucccaaagu gcugggauua 9660
cagucgugag ccaccgugcc cagccaagaa aaaauuuuug aggcaucaaa agcccauuua 9720
aauuuggugc caggaacgga gacaaucgug aaagcugcug auagccucac aaaucuuaag 9780
ccagucacuu ggguuaaaag caucagaagu uucacuauug uaaauuucau auuaauccuu 9840
guaugccugu ucugucuguu guuagucuac agguguaucc agcagcucca aagagacagc 9900
aaccagcaag aaugggccau agugacgaug gugguuuugu caaaaagaaa agggggggau 9960
auguaaggaa aagagagauc agacuuucac ugugucuaug uagaaaagga agacauaaga 10020
aacuccauuu ugaucuguac uaagaaaaau uguuuugccu ugagaugcug uuaaucugua 10080
acuuuagccc caacccugug cucacggaaa caugugcugu aagguuuaag ggaucuaggg 10140
cugugcagga uguaccuugu uaacaauaug uuugcaggca guauguuugg uaaaagucau 10200
cgccauucuc cauucucgau uaaccagggg cucaaugcac uguggaaagc cacaggaacc 10260
ucugcccaag aaagccuggc uguuguggga agucagggac cccgaaugga gggaccagcu 10320
ggugcugcau caggaaacau aaauugugaa gauuucuugg acauuuauca guuuccaaaa 10380
uuaauacuuu uauaauuucu uacaccuguc uuacuuuaau cucuuaaucc uguuaucuuu 10440
guaagcugag gauauacguc accucaggac cacuauugua caaauugauu guaaaacaug 10500
uucacaugug uuugaacaau augaaaucag ugcaccuuga aaaugaacag aauaacagug 10560
auuuuaggga acaaaggaag acaaccauaa ggucugacug ccugaggggu cgggcaaaaa 10620
gccauauuuu ucuucuugca gagagccuau aaauggacgu gcaaguagga gagauauugc 10680
uaaauucuuu uccuagcaag gaauauaaua cuaagacccu agggaaagaa uugcauuccu 10740
ggggggaggu cuauaaacgg ccgcucuggg agugucuguc cuaugugguu gagauaagga 10800
cugagauacg cccuggucuc cugcaguacc cucaggcuua cuaggauugg gaaaccccag 10860
uccugguaaa uuugagguca ggccgguucu uugcucugaa cccuguuuuc uguuaagaug 10920
uuuaucaaga caauacaugc accgcugaac auagacccuu aucaggaguu ucugauuuug 10980
cucugguccu guuucuucag aagcauguca ucuuugcucu gccuucugcc cuuugaagca 11040
ugugaucuuu gugaccuacu cccuguucau acaccccucc ccuuuuaaaa ucccuaauaa 11100
aaacuugcug guuuuguggc ucaggggggc aucauggacc uaccaauacg ugaugucacc 11160
cccgguggcc cagcugu 11177

<210> 24
<211> 527
<212> RNA
<213> HERV-K

<400> 24
gagauaggag aaaacugccu uagggcugga ggugggacau gcuggcggca auacugcucu 60
uuaaggcauu gagauguuua uguauaugca caucaaaagc acagcacuuu uuucuuuacc 120
uuguuuauga ugcagagaca uuuguucaca uguuuuccug cuggcccucu ccccacuauu 180

acccuauugu ccugccacau cccccucucc gagaugguag agauaaugau caauaaauac 240
ugagggaacu cagagaccgg ugcggcgcgg guccuccaua ugcugagcgc cgguccccug 300
ggcccacuuu ucuuucucua uacuuugucu cuguugucuu ucuuuucuca agucucucgu 360
uccaccugag gagaaaugcc cacagcugug gaggcgcagg ccacuccauc uggugcccaa 420
cguggaugcu uuucucuagg gugaagggac ucucgagugu ggucauugag gacaagucaa 480
cgagagauuc ccgaguacgu cuacagugag ccuugugucu cucaucc 527

<210> 25
<211> 527
<212> RNA
<213> HERV-K

<400> 25
gagauaggag aaaacugccu uagggcugga ggugggacau gcuggcggca auacugcucu 60
uuaaggcauu gagauguuua uguauaugca caucaaaagc acagcacuuu uuucuuuacc 120
uuguuuauga ugcagagaca uuuguucaca uguuuuccug cuggcccucu ccccacuauu 180
acccuauugu ccugccacau cccccucucc gagaugguag agauaaugau caauaaauac 240
ugagggaacu cagagaccgg ugcggcgcgg guccuccaua ugcugagcgc cgguccccug 300
ggcccacuuu ucuuucucua uacuuugucu cuguugucuu ucuuuucuca agucucucgu 360
uccaccugag gagaaaugcc cacagcugug gaggcgcagg ccacuccauc uggugcccaa 420
cguggaugcu uuucucuagg gugaagggac ucucgagugu ggucauugag gacaagucaa 480
cgagagauuc ccgaguacgu cuacagugag ccuugugggu gaaggua 527

<210> 26
<211> 517
<212> RNA
<213> HERV-K

<400> 26
gagauaggag aaaacugccu uagggcugga ggugggacau gcuggcggca auacugcucu 60
uuaaggcauu gagauguuua uguauaugca caucaaaagc acagcacuuu uuucuuuacc 120
uuguuuauga ugcagagaca uuuguucaca uguuuuccug cuggcccucu ccccacuauu 180
acccuauugu ccugccacau cccccucucc gagaugguag agauaaugau caauaaauac 240
ugagggaacu cagagaccgg ugcggcgcgg guccuccaua ugcugagcgc cgguccccug 300
ggcccacuuu ucuuucucua uacuuugucu cuguugucuu ucuuuucuca agucucucgu 360
uccaccugag gagaaaugcc cacagcugug gaggcgcagg ccacuccauc uggugcccaa 420
cguggaugcu uuucucuagg gugaagggac ucucgagugu ggucauugag gacaagucaa 480
cgagagauuc ccgaguacgu cuacagugag ccuugug 517

<210> 27
<211> 10
<212> RNA
<213> HERV-K

<400> 27
ucucucaucc 10

<210> 28
<211> 10
<212> RNA
<213> HERV-K

<400> 28
ggugaaggua 10

<210> 29
<211> 1216
<212> RNA
<213> HERV-K

<400> 29

uguaaggaaa agagagauca gacuuucacu gugucuaugu agaaaaggaa gacauaagaa 60
acuccauuuu gaucuguacu aagaaaaauu guuuugccuu gagaugcugu uaaucuguaa 120
cuuuagcccc aacccugugc ucacggaaac augugcugua agguuuaagg gaucuagggc 180
ugugcaggau guaccuuguu aacaauaugu uugcaggcag uauguuuggu aaaagucauc 240
gccauucucc auucucgauu aaccaggggc ucaaugcacu guggaaagcc acaggaaccu 300
cugcccaaga aagccuggcu guugugggaa gucagggacc ccgaauggag ggaccagcug 360
gugcugcauc aggaaacaua aauugugaag auuucuugga cauuuaucag uuuccaaaau 420
uaauacuuuu auaauuucuu acaccugucu uacuuuaauc ucuuaauccu guuaucuuug 480
uaagcugagg auauacguca ccucaggacc acuauuguac aaauugauug uaaaacaugu 540
ucacaugugu uugaacaaua ugaaaucagu gcaccuugaa aaugaacaga auaacaguga 600
uuuuagggaa caaaggaaga caaccauaag gucugacugc cugagggguc gggcaaaaag 660
ccauauuuuu cuucuugcag agagccuaua aauggacgug caaguaggag agauauugcu 720
aaauucuuuu ccuagcaagg aauauaauac uaagacccua gggaaagaau ugcauuccug 780
gggggagguc uauaaacggc cgcucuggga gugucugucc uaugugguug agauaaggac 840
ugagauacgc ccuggucucc ugcaguaccc ucaggcuuac uaggauuggg aaaccccagu 900
ccugguaaau uugaggucag gccgguucuu ugcucugaac ccuguuuucu guuaagaugu 960
uuaucaagac aauacaugca ccgcugaaca uagacccuua ucaggaguuu cugauuuugc 1020
ucugguccug uuucuucaga agcaugucau cuuugcucug ccuucugccc uuugaagcau 1080
gugaucuuug ugaccuacuc ccuguucaua caccccuccc cuuuuaaaau cccuaauaaa 1140
aacuugcugg uuuuguggcu caggggggca ucauggaccu accaauacgu gaugucaccc 1200
ccgguggccc agcugu 1216

<210> 30
<211> 319
<212> RNA
<213> HERV-K

<400> 30
uguaaggaaa agagagauca gacuuucacu gugucuaugu agaaaaggaa gacauaagaa 60
acuccauuuu gaucuguacu aagaaaaauu guuuugccuu gagaugcugu uaaucuguaa 120
cuuuagcccc aacccugugc ucacggaaac augugcugua agguuuaagg gaucuagggc 180
ugugcaggau guaccuuguu aacaauaugu uugcaggcag uauguuuggu aaaagucauc 240
gccauucucc auucucgauu aaccaggggc ucaaugcacu guggaaagcc acaggaaccu 300
cugcccaaga aagccuggc 319

<210> 31
<211> 897
<212> RNA
<213> HERV-K

<400> 31
uguuguggga agucagggac cccgaaugga gggaccagcu ggugcugcau caggaaacau 60
aaauugugaa gauuucuugg acauuuauca guuuccaaaa uuaauacuuu uauaauuucu 120
uacaccuguc uuacuuuaau cucuuaaucc uguuaucuuu guaagcugag gauauacguc 180
accucaggac cacuauugua caaauugauu guaaaacaug uucacaugug uuugaacaau 240
augaaaucag ugcaccuuga aaaugaacag aauaacagug auuuuaggga acaaaggaag 300
acaaccauaa ggucugacug ccugaggggu cgggcaaaaa gccauauuuu ucuucuugca 360
gagagccuau aaauggacgu gcaaguagga gagauauugc uaaauucuuu uccuagcaag 420
gaauauaaua cuaagacccu agggaaagaa uugcauuccu ggggggaggu cuauaaacgg 480
ccgcucuggg agugucuguc cuaugugguu gagauaagga cugagauacg cccuggucuc 540
cugcaguacc cucaggcuua cuaggauugg gaaaccccag uccugguaaa uuugagguca 600
ggccgguucu uugcucugaa cccuguuuuc uguuaagaug uuuaucaaga caauacaugc 660
accgcugaac auagacccuu aucaggaguu ucugauuuug cucugguccu guuucuucag 720
aagcauguca ucuuugcucu gccuucugcc cuuugaagca ugugaucuuu gugaccuacu 780
cccuguucau acaccccucc ccuuuuaaaa ucccuaauaa aaacuugcug guuuuguggc 840
ucaggggggc aucauggacc uaccaauacg ugaugucacc cccgguggcc cagcugu 897

<210> 32
<211> 307
<212> DNA
<213> HERV-K

<400>   32
ttaaaagaat tttttttttc tttgagacag agtctcgctc tgtcgcccag gctggagtgc   60
agtggcgtga tctcagctca ctgcaagttc cgcctcctgg gtttacacca ttctcctgcc   120
tcagcctccc aagtagttgg gactacagga gcccaccacc atgcctggct aatttttttt   180
gggtttttaa tagagatgga gtttcaccgt gttagccagg atggtctcga tctcctgacc   240
ttgtgatctg cccaccttgg cctcccaaag tgctgggatt acagtcgtga gccaccgtgc   300
ccagcca                                                             307

<210>   33
<211>   10
<212>   DNA
<213>   HERV-K

<400>   33
catttcaaaa                                                          10

<210>   34
<211>   10
<212>   DNA
<213>   HERV-K

<400>   34
agaaaaaatt                                                          10

<210>   35
<211>   10
<212>   DNA
<213>   HERV-K

<400>   35
ttaaaagaat                                                          10

<210>   36
<211>   20
<212>   DNA
<213>   HERV-K

<400>   36
catttcaaaa ttaaaagaat                                               20

<210>   37
<211>   100
<212>   DNA
<213>   HERV-K

<400>   37
tgttacacca caagcctata atgagtctga gcatcactgg gacatggtta gatgccatct   60
gcaaggagga gaagataatc ttactttaga catttcaaaa                         100

<210>   38
<211>   407
<212>   DNA
<213>   HERV-K

<400>   38
tgttacacca caagcctata atgagtctga gcatcactgg gacatggtta gatgccatct   60
gcaaggagga gaagataatc ttactttaga catttcaaaa ttaaaagaat tttttttttc   120
tttgagacag agtctcgctc tgtcgcccag gctggagtgc agtggcgtga tctcagctca   180
ctgcaagttc cgcctcctgg gtttacacca ttctcctgcc tcagcctccc aagtagttgg   240
gactacagga gcccaccacc atgcctggct aatttttttt gggtttttaa tagagatgga   300

gtttcaccgt gttagccagg atggtctcga tctcctgacc ttgtgatctg cccaccttgg   360
cctcccaaag tgctgggatt acagtcgtga gccaccgtgc ccagcca                  407

<210>   39
<211>   8
<212>   DNA
<213>   HERV-K

<400>   39
aaaattaa                                                              8

<210>   40
<211>   100
<212>   DNA
<213>   HERV-K

<400>   40
agaaaaaatt tttgaggcat caaaagccca tttaaatttg gtgccaggaa cggagacaat   60
cgtgaaagct gctgatagcc tcacaaatct taagccagtc                         100

<210>   41
<211>   10
<212>   DNA
<213>   HERV-K

<400>   41
tgcccagcca                                                            10

<210>   42
<211>   110
<212>   DNA
<213>   HERV-K

<400>   42
tgcccagcca agaaaaaatt tttgaggcat caaaagccca tttaaatttg gtgccaggaa   60
cggagacaat cgtgaaagct gctgatagcc tcacaaatct taagccagtc              110

<210>   43
<211>   407
<212>   DNA
<213>   HERV-K

<400>   43
ttaaaagaat tttttttttc tttgagacag agtctcgctc tgtcgcccag gctggagtgc   60
agtggcgtga tctcagctca ctgcaagttc cgcctcctgg gtttacacca ttctcctgcc  120
tcagcctccc aagtagttgg gactacagga gcccaccacc atgcctggct aatttttttt  180
gggtttttaa tagagatgga gtttcaccgt gttagccagg atggtctcga tctcctgacc  240
ttgtgatctg cccaccttgg cctcccaaag tgctgggatt acagtcgtga gccaccgtgc  300
ccagccaaga aaaaattttt gaggcatcaa aagcccattt aaatttggtg ccaggaacgg  360
agacaatcgt gaaagctgct gatagcctca caaatcttaa gccagtc                 407

<210>   44
<211>   8
<212>   DNA
<213>   HERV-K

<400>   44
gccaagaa                                                              8

<210>   45
<211>   20

<212>   DNA
<213>   HERV-K

<400>   45
tgcccagcca agaaaaaatt                                                   20

<210>   46
<211>   100
<212>   DNA
<213>   HERV-K

<400>   46
tctctcatcc ctcctgacga gaaataccca caggtgtgga ggggctggcc cccttcatct      60
gatgcccaat gtgggtgcct ttctctaggg tgaaggtact                            100

<210>   47
<211>   617
<212>   DNA
<213>   HERV-K

<400>   47
gagataggag aaaactgcct tagggctgga ggtgggacat gctggcggca atactgctct      60
ttaaggcatt gagatgttta tgtatatgca catcaaaagc acagcacttt tttctttacc     120
ttgtttatga tgcagagaca tttgttcaca tgttttcctg ctggccctct ccccactatt     180
accctattgt cctgccacat ccccctctcc gagatggtag agataatgat caataaatac     240
tgagggaact cagagaccgg tgcggcgcgg gtcctccata tgctgagcgc cggtcccctg     300
ggcccacttt tctttctcta tactttgtct ctgttgtctt tcttttctca agtctctcgt     360
tccacctgag gagaaatgcc cacagctgtg gaggcgcagg ccactccatc tggtgcccaa     420
cgtggatgct ttctctaggg gtgaagggac tctcgagtgt ggtcattgag gacaagtcaa     480
cgagagattc ccgagtacgt ctacagtgag ccttgtgtct ctcatccctc ctgacgagaa     540
atacccacag gtgtggaggg gctggccccc ttcatctgat gcccaatgtg ggtgcctttc     600
tctagggtga aggtact                                                     617

<210>   48
<211>   100
<212>   DNA
<213>   HERV-K

<400>   48
ggtgaaggta ctctacagtg tggtcattga ggacaagttg acgagagagt cccaagtacg      60
tccacggtca gccttgcggt aagcttgtgt gcttagagga                            100

<210>   49
<211>   617
<212>   DNA
<213>   HERV-K

<400>   49
gagataggag aaaactgcct tagggctgga ggtgggacat gctggcggca atactgctct      60
ttaaggcatt gagatgttta tgtatatgca catcaaaagc acagcacttt tttctttacc     120
ttgtttatga tgcagagaca tttgttcaca tgttttcctg ctggccctct ccccactatt     180
accctattgt cctgccacat ccccctctcc gagatggtag agataatgat caataaatac     240
tgagggaact cagagaccgg tgcggcgcgg gtcctccata tgctgagcgc cggtcccctg     300
ggcccacttt tctttctcta tactttgtct ctgttgtctt tcttttctca agtctctcgt     360
tccacctgag gagaaatgcc cacagctgtg gaggcgcagg ccactccatc tggtgcccaa     420
cgtggatgct ttctctaggg gtgaagggac tctcgagtgt ggtcattgag gacaagtcaa     480
cgagagattc ccgagtacgt ctacagtgag ccttgtgggt gaaggtactc tacagtgtgg     540
tcattgagga caagttgacg agagagtccc aagtacgtcc acggtcagcc ttgcggtaag     600
cttgtgtgct tagagga                                                     617

<210>   50

<211>   20
<212>   DNA
<213>   HERV-K

<400>   50
gagccttgtg tctctcatcc                                                   20

<210>   51
<211>   20
<212>   DNA
<213>   HERV-K

<400>   51
gagccttgtg ggtgaaggta                                                   20

<210>   52
<211>   20
<212>   DNA
<213>   HERV-K

<400>   52
aaagcctggc tgttgtggga                                                   20

<210>   53
<211>   48
<212>   DNA
<213>   HERV-K

<400>   53
acagcgatgg cgtctaattc accagcaaca caggacgcgg cgctgtat                    48

<210>   54
<211>   715
<212>   PRT
<213>   HERV-K

<400>   54
Met Gly Gln Thr Glu Ser Lys Tyr Ala Ser Tyr Leu Ser Phe Ile Lys
1               5                   10                  15

Ile Leu Leu Arg Arg Gly Gly Val Arg Ala Ser Thr Glu Asn Leu Ile
            20                  25                  30

Thr Leu Phe Gln Thr Ile Glu Gln Phe Cys Pro Trp Phe Pro Glu Gln
        35                  40                  45

Gly Thr Leu Asp Leu Lys Asp Trp Glu Lys Ile Gly Lys Glu Leu Lys
    50                  55                  60

Gln Ala Asn Arg Glu Gly Lys Ile Ile Pro Leu Thr Val Trp Asn Asp
65                  70                  75                  80

Trp Ala Ile Ile Lys Ala Thr Leu Glu Pro Phe Gln Thr Gly Glu Asp
                85                  90                  95

Ile Val Ser Val Ser Asp Ala Pro Lys Ser Cys Val Thr Asp Cys Glu
            100                 105                 110

Glu Glu Ala Gly Thr Glu Ser Gln Gln Gly Thr Glu Ser Ser His Cys
        115                 120                 125

Lys Tyr Val Ala Glu Ser Val Met Ala Gln Ser Thr Gln Asn Val Asp
    130                 135                 140

Tyr Ser Gln Leu Gln Glu Ile Ile Tyr Pro Glu Ser Ser Lys Leu Gly
145                 150                 155                 160

Glu Gly Gly Pro Glu Ser Leu Gly Pro Ser Glu Pro Lys Pro Arg Ser
                165                 170                 175

Pro Ser Thr Pro Pro Pro Val Val Gln Met Pro Val Thr Leu Gln Pro
            180                 185                 190

Gln Thr Gln Val Arg Gln Ala Gln Thr Pro Arg Glu Asn Gln Val Glu
        195                 200                 205

Arg Asp Arg Val Ser Ile Pro Ala Met Pro Thr Gln Ile Gln Tyr Pro
    210                 215                 220

Gln Tyr Gln Pro Val Glu Asn Lys Thr Gln Pro Leu Val Val Tyr Gln
225                 230                 235                 240

Tyr Arg Leu Pro Thr Glu Leu Gln Tyr Arg Pro Pro Ser Glu Val Gln
                245                 250                 255

Tyr Arg Pro Gln Ala Val Cys Pro Val Pro Asn Ser Thr Ala Pro Tyr
            260                 265                 270

Gln Gln Pro Thr Ala Met Ala Ser Asn Ser Pro Ala Thr Gln Asp Ala
        275                 280                 285

Ala Leu Tyr Pro Gln Pro Pro Thr Val Arg Leu Asn Pro Thr Ala Ser
    290                 295                 300

Arg Ser Gly Gln Gly Gly Ala Leu His Ala Val Ile Asp Glu Ala Arg
305                 310                 315                 320

Lys Gln Gly Asp Leu Glu Ala Trp Arg Phe Leu Val Ile Leu Gln Leu
                325                 330                 335

Val Gln Ala Gly Glu Glu Thr Gln Val Gly Ala Pro Ala Arg Ala Glu
            340                 345                 350

Thr Arg Cys Glu Pro Phe Thr Met Lys Met Leu Lys Asp Ile Lys Glu
        355                 360                 365

Gly Val Lys Gln Tyr Gly Ser Asn Ser Pro Tyr Ile Arg Thr Leu Leu
    370                 375                 380

Asp Ser Ile Ala His Gly Asn Arg Leu Thr Pro Tyr Asp Trp Glu Ile
385                 390                 395                 400

Leu Ala Lys Ser Ser Leu Ser Ser Ser Gln Tyr Leu Gln Phe Lys Thr
                405                 410                 415

Trp Trp Ile Asp Gly Val Gln Glu Gln Val Arg Lys Asn Gln Ala Thr
            420                 425                 430

Lys Pro Thr Val Asn Ile Asp Ala Asp Gln Leu Leu Gly Thr Gly Pro
        435                 440                 445

Asn Trp Ser Thr Ile Asn Gln Gln Ser Val Met Gln Asn Glu Ala Ile

450                 455                 460

Glu Gln Val Arg Ala Ile Cys Leu Arg Ala Trp Gly Lys Ile Gln Asp
465                 470                 475                 480

Pro Gly Thr Ala Phe Pro Ile Asn Ser Ile Arg Gln Gly Ser Lys Glu
                485                 490                 495

Pro Tyr Pro Asp Phe Val Ala Arg Leu Gln Asp Ala Ala Gln Lys Ser
            500                 505                 510

Ile Thr Asp Asp Asn Ala Arg Lys Val Ile Val Glu Leu Met Ala Tyr
        515                 520                 525

Glu Asn Ala Asn Pro Glu Cys Gln Ser Ala Ile Lys Pro Leu Lys Gly
    530                 535                 540

Lys Val Pro Ala Gly Val Asp Val Ile Thr Glu Tyr Val Lys Ala Cys
545                 550                 555                 560

Asp Gly Ile Gly Gly Ala Met His Lys Ala Met Leu Met Ala Gln Ala
                565                 570                 575

Met Arg Gly Leu Thr Leu Gly Gly Gln Val Arg Thr Phe Gly Lys Lys
            580                 585                 590

Cys Tyr Asn Cys Gly Gln Ile Gly His Leu Lys Arg Ser Cys Pro Gly
        595                 600                 605

Leu Asn Lys Gln Asn Ile Ile Asn Gln Ala Ile Thr Ala Lys Asn Lys
    610                 615                 620

Lys Pro Ser Gly Leu Cys Pro Lys Cys Gly Lys Ala Lys His Trp Ala
625                 630                 635                 640

Asn Gln Cys His Ser Lys Phe Asp Lys Asp Gly Gln Pro Leu Ser Gly
                645                 650                 655

Asn Arg Lys Arg Gly Gln Pro Gln Ala Pro Gln Gln Thr Gly Ala Phe
            660                 665                 670

Pro Val Lys Leu Phe Val Pro Gln Gly Phe Gln Gly Gln Gln Pro Leu
        675                 680                 685

Gln Lys Ile Pro Pro Leu Gln Gly Val Ser Gln Leu Gln Gln Ser Asn
    690                 695                 700

Ser Cys Pro Ala Pro Gln Gln Ala Ala Pro Gln
705                 710                 715

<210>   55
<211>   23
<212>   PRT
<213>   HERV-K

<400>   55
Thr Gln Val Arg Gln Ala Gln Thr Pro Arg Glu Asn Gln Val Glu Arg
1               5                   10                  15

Asp Arg Val Ser Ile Pro Ala
            20

<210>   56
<211>   17
<212>   PRT
<213>   HERV-K

<400>   56
Pro Thr Ala Met Ala Ser Asn Ser Pro Ala Thr Gln Asp Ala Ala Leu
1               5                   10                  15

Tyr


<210>   57
<211>   2145
<212>   DNA
<213>   HERV-K

<400>   57
atggggcaaa ctgaaagtaa atatgcctct tatctcagct ttattaaaat tcttttaaga   60
agagggggag ttagagcttc tacagaaaat ctaattacgc tatttcaaac aatagaacaa   120
ttctgcccat ggtttccaga acagggaact ttagatctaa aagattggga aaaaattggc   180
aaagaattaa aacaagcaaa tagggaaggt aaaatcatcc cacttacagt atggaatgat   240
tgggccatta ttaaagcaac tttagaacca tttcaaacag gagaagatat tgtttcagtt   300
tctgatgccc ctaaaagctg tgtaacagat tgtgaagaag aggcagggac agaatcccag   360
caaggaacgg aaagttcaca ttgtaaatat gtagcagagt ctgtaatggc tcagtcaacg   420
caaaatgttg actacagtca attacaggag ataatatacc ctgaatcatc aaaattgggg   480
gaaggaggtc cagaatcatt ggggccatca gagcctaaac cacgatcgcc atcaactcct   540
cctcccgtgg ttcagatgcc tgtaacatta caacctcaaa cgcaggttag acaagcacaa   600
accccaagag aaaatcaagt agaaagggac agagtctcta tcccggcaat gccaactcag   660
atacagtatc cacaatatca gccggtagaa aataagaccc aaccgctggt agtttatcaa   720
taccggctgc caaccgagct tcagtatcgg cctccttcag aggttcaata cagacctcaa   780
gcggtgtgtc ctgtgccaaa tagcacggca ccataccagc aacccacagc gatggcgtct   840
aattcaccag caacacagga cgcggcgctg tatcctcagc cgcccactgt gagacttaat   900
cctacagcat cacgtagtgg acagggtggt gcactgcatg cagtcattga tgaagccaga   960
aaacagggcg atcttgaggc atggcggttc ctggtaattt tacaactggt acaggccggg   1020
gaagagactc aagtaggagc gcctgcccga gctgagacta gatgtgaacc tttcaccatg   1080
aaaatgttaa aagatataaa ggaaggagtt aaacaatatg gatccaactc cccttatata   1140
agaacattat tagattccat tgctcatgga aatagactta ctccttatga ctgggaaatt   1200
ttggccaaat cttccctttc atcctctcag tatctacagt ttaaaacctg gtggattgat   1260
ggagtacaag aacaggtacg aaaaaatcag gctactaagc ccactgttaa tatagacgca   1320
gaccaattgt taggaacagg tccaaattgg agcaccatta accaacaatc agtgatgcag   1380
aatgaggcta ttgaacaagt aagggctatt tgcctcaggg cctggggaaa aattcaggac   1440
ccaggaacag ctttccctat taattcaatt agacaaggct ctaaagagcc atatcctgac   1500
tttgtggcaa gattacaaga tgctgctcaa aagtctatta cagatgacaa tgcccgaaaa   1560
gttattgtag aattaatggc ctatgaaaat gcaaatccag aatgtcagtc ggccataaag   1620
ccattaaaag gaaaagttcc agcaggagtt gatgtaatta cagaatatgt gaaggcttgt   1680
gatgggattg gaggagctat gcataaggca atgctaatgg ctcaagcaat gagggggctc   1740
actctaggag gacaagttag aacatttggg aaaaaatgtt ataattgtgg tcaaatcggt   1800
catctgaaaa ggagttgccc aggcttaaat aaacagaata taataaatca agctattaca   1860
gcaaaaaata aaaagccatc tggcctgtgt ccaaaatgtg gaaaagcaaa acattgggcc   1920
aatcaatgtc attctaaatt tgataaagat gggcaaccat tgtctggaaa caggaagagg   1980
ggccagcctc aggcccccca acaaactggg gcattcccag ttaaactgtt tgttcctcag   2040
ggttttcaag gacaacaacc cctacagaaa ataccaccac ttcagggagt cagccaatta   2100
caacaatcca acagctgtcc cgcgccacag caggcagcac cgcag                   2145

<210>   58
<211>   927
<212>   DNA
<213>   HERV-K

<400>   58
tgggcaacca ttgtctggaa acaggaagag gggccagcct caggcccccc aacaaactgg   60
ggcattccca gttaaactgt ttgttcctca gggttttcaa ggacaacaac ccctacagaa   120
aataccacca cttcagggag tcagccaatt acaacaatcc aacagctgtc ccgcgccaca   180
gcaggcagca ccgcagtaga tttatgttcc acccaaatgg tctttttact ccctggaaag   240
cccccacaaa agattcctag aggggtatat ggcccgctgc cagaagggag ggtaggcctt   300
tgagggagat caagtctaaa tttgaaggga gtccaaattc atactggggt aatttattca   360
gattataaag ggggaattca gttagtgatc agctccactg ttccccggag tgccaatcca   420
ggtgatagaa ttgctcaatt actgcttttg ccttatgtta aaattgggga aaacaaaaag   480
gaaagaacag gagggtttgg aagtaccaac cctgcaggaa aagctgctta ttgggctaat   540
caggtctcag aggatagacc cgtgtgtaca gtcactattc agggaaagag tttgaaggat   600
tagtggatac ccaggctgat gtttctgtca tcggcatagg tactgcctca gaagtgtatc   660
aaagtgccat gattttacat tgtccaggat ctgataatca agaaagtacg gttcagcctg   720
tgatcacttc attccaatca atttatgggg ccgagacttg ttacaacaat ggcatgcaga   780
gattactatc ccagcctccc tatacagccc caggaataaa aaaatcatga ctaaaatggg   840
atagctccct aaaaagggac taggaaagaa gtcccaattg aggctgaaaa aaatcaaaaa   900
agaaaaggaa tagggcatcc tttttag                                       927

<210>   59
<211>   24
<212>   PRT
<213>   HERV-K

<400>   59
Trp Ala Thr Ile Val Trp Lys Gln Glu Glu Gly Pro Ala Ser Gly Pro
1               5                   10                  15

Pro Thr Asn Trp Gly Ile Pro Ser
            20

<210>   60
<211>   75
<212>   PRT
<213>   HERV-K

<400>   60
Thr Val Cys Ser Ser Gly Phe Ser Arg Thr Thr Thr Pro Thr Glu Asn
1               5                   10                  15

Thr Thr Thr Ser Gly Ser Gln Pro Ile Thr Thr Ile Gln Gln Leu Ser
            20                  25                  30

Arg Ala Thr Ala Gly Ser Thr Ala Val Asp Leu Cys Ser Thr Gln Met
        35                  40                  45

Val Phe Leu Leu Pro Gly Lys Pro Pro Gln Lys Ile Pro Arg Gly Val
    50                  55                  60

Tyr Gly Pro Leu Pro Glu Gly Arg Val Gly Leu
65                  70                  75

<210>   61
<211>   180
<212>   PRT
<213>   HERV-K

<400>   61
Gly Arg Ser Ser Leu Asn Leu Lys Gly Val Gln Ile His Thr Gly Val
1               5                   10                  15

Ile Tyr Ser Asp Tyr Lys Gly Gly Ile Gln Leu Val Ile Ser Ser Thr

20                  25                  30

Val Pro Arg Ser Ala Asn Pro Gly Asp Arg Ile Ala Gln Leu Leu Leu
        35                  40                  45

Leu Pro Tyr Val Lys Ile Gly Glu Asn Lys Lys Glu Arg Thr Gly Gly
    50                  55                  60

Phe Gly Ser Thr Asn Pro Ala Gly Lys Ala Ala Tyr Trp Ala Asn Gln
65                  70                  75                  80

Val Ser Glu Asp Arg Pro Val Cys Thr Val Thr Ile Gln Gly Lys Ser
                85                  90                  95

Leu Lys Asp Val Asp Thr Gln Ala Asp Val Ser Val Ile Gly Ile Gly
            100                 105                 110

Thr Ala Ser Glu Val Tyr Gln Ser Ala Met Ile Leu His Cys Pro Gly
        115                 120                 125

Ser Asp Asn Gln Glu Ser Thr Val Gln Pro Val Ile Thr Ser Phe Ile
    130                 135                 140

Pro Ile Asn Leu Trp Gly Arg Asp Leu Leu Gln Gln Trp His Ala Glu
145                 150                 155                 160

Ile Thr Ile Pro Ala Ser Leu Tyr Ser Pro Arg Asn Lys Lys Ile Met
                165                 170                 175

Thr Lys Met Gly
            180

<210>   62
<211>   28
<212>   PRT
<213>   HERV-K

<400>   62
Leu Pro Lys Lys Gly Leu Gly Lys Lys Glu Val Pro Ile Glu Ala Glu
1               5                   10                  15

Lys Asn Gln Lys Arg Lys Gly Ile Gly His Pro Phe
            20                  25

<210>   63
<211>   651
<212>   DNA
<213>   HERV-K

<400>   63
acatccagaa ttaggaagct tactgtggcc tcacaccaca ttagaatttg ttctggaaat    60
caagctatag gaacaagaga tcgtaagtca tattatacta tcaacctaaa ttccagtctg   120
acaattcctt tgcaaaattg tgtaaaactc ccttatattg ctagttgtag gaaaaacata   180
gttattaaac ctgattccca aaccataatc tgtgaaaatt gtggaatgtt tacttgcatt   240
gatttgactt ttaattggca gcaccgtatt ctactaggaa gagcaagaga gggtgtgtgg   300
atccttgtgt ccatggaccg accatgggag gcttcgctat ccatccatat tttaacggaa   360
gtattaaaag gaattctaac tagatccaaa agattcattt ttactttgat ggcagtgatt   420
atgggcctca ttgcagtcac agctactgct gcggctgctg gaattgcttt acactcctct   480
gttcaaactg cagaatacgt aaatgattgg caaaagaatt cctcaaaatt gtggaattct   540
cagatccaaa tagatcaaaa attggcaaac caaattaatg atcttagaca aactgtcatt   600
tggatgggag aggctcatga gcttggaata tctttttcag ttacgatgtg a            651

<210>   64
<211>   216
<212>   PRT
<213>   HERV-K

<400>   64
Thr Ser Arg Ile Arg Lys Leu Thr Val Ala Ser His His Ile Arg Ile
1               5                   10                  15

Cys Ser Gly Asn Gln Ala Ile Gly Thr Arg Asp Arg Lys Ser Tyr Tyr
            20                  25                  30

Thr Ile Asn Leu Asn Ser Ser Leu Thr Ile Pro Leu Gln Asn Cys Val
        35                  40                  45

Lys Leu Pro Tyr Ile Ala Ser Cys Arg Lys Asn Ile Val Ile Lys Pro
    50                  55                  60

Asp Ser Gln Thr Ile Ile Cys Glu Asn Cys Gly Met Phe Thr Cys Ile
65                  70                  75                  80

Asp Leu Thr Phe Asn Trp Gln His Arg Ile Leu Leu Gly Arg Ala Arg
                85                  90                  95

Glu Gly Val Trp Ile Leu Val Ser Met Asp Arg Pro Trp Glu Ala Ser
            100                 105                 110

Leu Ser Ile His Ile Leu Thr Glu Val Leu Lys Gly Ile Leu Thr Arg
        115                 120                 125

Ser Lys Arg Phe Ile Phe Thr Leu Met Ala Val Ile Met Gly Leu Ile
    130                 135                 140

Ala Val Thr Ala Thr Ala Ala Ala Ala Gly Ile Ala Leu His Ser Ser
145                 150                 155                 160

Val Gln Thr Ala Glu Tyr Val Asn Asp Trp Gln Lys Asn Ser Ser Lys
                165                 170                 175

Leu Trp Asn Ser Gln Ile Gln Ile Asp Gln Lys Leu Ala Asn Gln Ile
            180                 185                 190

Asn Asp Leu Arg Gln Thr Val Ile Trp Met Gly Glu Ala His Glu Leu
        195                 200                 205

Gly Ile Ser Phe Ser Val Thr Met
    210                 215

<210>   65
<211>   22
<212>   PRT
<213>   HERV-K

<400>   65
His Pro Glu Leu Gly Ser Leu Leu Trp Pro His Thr Thr Leu Glu Phe
1               5                   10                  15

Val Leu Glu Ile Lys Leu
            20

<210>   66
<211>   12
<212>   PRT
<213>   HERV-K

<400>   66
Glu Gln Glu Ile Val Ser His Ile Ile Leu Ser Thr
1               5                   10

<210>   67
<211>   3
<212>   PRT
<213>   HERV-K

<400>   67
Ile Pro Val
1

<210>   68
<211>   7
<212>   PRT
<213>   HERV-K

<400>   68
Gln Phe Leu Cys Lys Ile Val
1               5

<210>   69
<211>   11
<212>   PRT
<213>   HERV-K

<400>   69
Asn Ser Leu Ile Leu Leu Val Val Gly Lys Thr
1               5                   10

<210>   70
<211>   8
<212>   PRT
<213>   HERV-K

<400>   70
Leu Leu Asn Leu Ile Pro Lys Pro
1               5

<210>   71
<211>   12
<212>   PRT
<213>   HERV-K

<400>   71
Ser Val Lys Ile Val Glu Cys Leu Leu Ala Leu Ile
1               5                   10

<210>   72
<211>   9
<212>   PRT
<213>   HERV-K

<400>   72
Leu Leu Ile Gly Ser Thr Val Phe Tyr

1               5

<210>   73
<211>   25
<212>   PRT
<213>   HERV-K

<400>   73
Glu Glu Gln Glu Arg Val Cys Gly Ser Leu Cys Pro Trp Thr Asp His
1               5                   10                  15

Gly Arg Leu Arg Tyr Pro Ser Ile Phe
            20                  25

<210>   74
<211>   3
<212>   PRT
<213>   HERV-K

<400>   74
Arg Lys Tyr
1

<210>   75
<211>   3
<212>   PRT
<213>   HERV-K

<400>   75
Lys Glu Phe
1

<210>   76
<211>   9
<212>   PRT
<213>   HERV-K

<400>   76
Leu Asp Pro Lys Asp Ser Phe Leu Leu
1               5

<210>   77
<211>   2
<212>   PRT
<213>   HERV-K

<400>   77
Trp Gln
1

<210>   78
<211>   27
<212>   PRT
<213>   HERV-K

<400>   78
Leu Trp Ala Ser Leu Gln Ser Gln Leu Leu Leu Arg Leu Leu Glu Leu
1               5                   10                  15

Leu Tyr Thr Pro Leu Phe Lys Leu Gln Asn Thr
            20                  25

<210>   79
<211>   16
<212>   PRT
<213>   HERV-K

<400>   79
Met Ile Gly Lys Arg Ile Pro Gln Asn Cys Gly Ile Leu Arg Ser Lys
1               5                   10                  15


<210>   80
<211>   32
<212>   PRT
<213>   HERV-K

<400>   80
Ile Lys Asn Trp Gln Thr Lys Leu Met Ile Leu Asp Lys Leu Ser Phe
1               5                   10                  15

Gly Trp Glu Arg Leu Met Ser Leu Glu Tyr Leu Phe Gln Leu Arg Cys
            20                  25                  30


<210>   81
<211>   249
<212>   DNA
<213>   HERV-K

<400>   81
gtacaaaaca atgaattttg aactatgata gactgggtcc cttgaggcca attatatcat   60
aactgtacag gccagactca ttcatgttca caggccccat ccatctggcc cattaatcca   120
gcctatgacg gtgatgtaac tgaaaggctg gaccaggttt atagaaggtt agaatcactc   180
tgtccaagga aatggggtga aaagggaatt tcatcacctt gaccaaagtt agtcctgtta   240
ctggtcctg                                                           249

<210>   82
<211>   6
<212>   PRT
<213>   HERV-K

<400>   82
Val Gln Asn Asn Glu Phe
1               5

<210>   83
<211>   7
<212>   PRT
<213>   HERV-K

<400>   83
Thr Met Ile Asp Trp Val Pro
1               5

<210>   84
<211>   58
<212>   PRT
<213>   HERV-K

<400>   84
Gly Gln Leu Tyr His Asn Cys Thr Gly Gln Thr His Ser Cys Ser Gln

1               5                   10                  15

Ala Pro Ser Ile Trp Pro Ile Asn Pro Ala Tyr Asp Gly Asp Val Thr
            20                  25                  30

Glu Arg Leu Asp Gln Val Tyr Arg Arg Leu Glu Ser Leu Cys Pro Arg
        35                  40                  45

Lys Trp Gly Glu Lys Gly Ile Ser Ser Pro
    50                  55

<210>   85
<211>   9
<212>   PRT
<213>   HERV-K

<400>   85
Pro Lys Leu Val Leu Leu Leu Val Leu
1               5

<210>   86
<211>   1839
<212>   DNA
<213>   HERV-K

<400>   86
atgtcaaatt tgttctcttt cttaagaggg gactcggaat taaatagtga aagaacgtta   60
actccagagg caactaaaga aattaaatta attgaagaaa aaattcggtc agcacaagta   120
aatagaatag atcacttggc cccactccaa attttgattt ttgctactgc acattcccta   180
acaggcatca ttgttcaaaa tacagatctt gtggagtggt ccttccttcc tcacagtaca   240
attaagactt ttacattgta cttggatcaa atggctacat taattggtca gggaagatta   300
tgaataataa cattgtgtgg aaatgaccca gataaaatca ctgttccttt caacaagcaa   360
caggttagac aagcctttat caattctggt gcatggcaga ttggtcttgc cgattttgtg   420
ggaattattg acaatcgtta ccccaaaaca aaaatcttcc agtttttaaa attgactact   480
tggattttac ctaaagttac caaacataag cctttaaaaa atgctctggc agtgtttact   540
gatggttcca gcaatggaaa agtggcttac accgggccaa aagaatgagt catcaaaact   600
cagtatcact tgactcaaag agcagagttg gttgccgtca ttacagtgtt aacaagattt   660
taatcagtct attaacattg tatcagattc tgcatatgta gtacaggcta caaaggatat   720
tgagagagcc ctaatcaaat acattatgga tgatcagtta aacccgctgt ttaatttgtt   780
acaacaaaat gtaagaaaaa gaaatttccc attttatatt actcatattc gagcacacac   840
taatttacca gggcctttaa ctaaagcaaa tgaacaagct gacttgctag tatcatctgc   900
attcatggaa gcacaagaac ttcatgcctt gactcatgta aatgcaatag gattaaaaaa   960
taaatttgat atcacatgga aacagacaaa aaatattgta caacattgca cccagtgtca   1020
gattctacac ctggccactc aggaggcaag agttaatccc agaggtctat gtcctaatgt   1080
gttatggcaa atggatgtca tgcacgtacc ttcatttgga aaattgtcat ttgtccatgt   1140
gacagttgat acttattcac atttcatatg ggcaacctgc cagacaggag aaagtacttc   1200
ccatgttaaa agacatttat tatcttgttt tcctgtcatg ggagttccag aaaaagttaa   1260
aacagacaat gggccaggtt actgtagtaa agcagttcaa aaattcttaa atcagtggaa   1320
aattacacat acaataggaa ttctctataa ttcccaagga caggccataa ttgaaagaac   1380
taatagaaca ctcaaagctc aattggttaa acaaaaaaaa ggaaaagaca ggagtataac   1440
actccccaga tgcaacttaa tctagcactc tatactttaa atgttttaaa catttataga   1500
aatcagacca ctacctctgc agaacaacat cttactggta aaaggaacag cccacatgaa   1560
ggaaaactga tttggtggaa agataataaa aataaaacat gggaaatggg gaaggtgata   1620
acgtggggga gaggttttgc ttgtgtttca ccaggagaaa atcagcttcc tgtttggata   1680
cccactagac atttaaagtt ctacaatgaa ctcactggag atgcaaagaa aagtgtggag   1740
atggagacac cccaatcgac tcgccaggta aacaaaatgg tgatatcaga agaacagaaa   1800
aagttgcctt ccatcaagga agcagagttg ccaatatag                          1839

<210>   87
<211>   79
<212>   PRT

<213>   HERV-K

<400>   87
Met Asn Ser Leu Glu Met Gln Arg Lys Val Trp Arg Trp Arg His Pro
1               5                   10                  15

Asn Arg Leu Ala Ser Leu Gln Val Tyr Pro Ala Ala Pro Lys Arg Gln
            20                  25                  30

Gln Pro Ala Arg Met Gly His Ser Asp Asp Gly Gly Phe Val Lys Lys
        35                  40                  45

Lys Arg Gly Gly Tyr Val Arg Lys Arg Glu Ile Arg Leu Ser Leu Cys
    50                  55                  60

Leu Cys Arg Lys Gly Arg His Lys Lys Leu His Phe Asp Leu Tyr
65                  70                  75

<210>   88
<211>   237
<212>   DNA
<213>   HERV-K

<400>   88
atgaactcac tggagatgca aagaaaagtg tggagatgga gacaccccaa tcgactcgcc    60
agtctacagg tgtatccagc agctccaaag agacagcaac cagcaagaat gggccatagt   120
gacgatggtg gttttgtcaa aaagaaaagg gggggatatg taaggaaaag agagatcaga   180
ctttcactgt gtctatgtag aaaaggaaga cataagaaac tccattttga tctgtac      237

<210>   89
<211>   723
<212>   DNA
<213>   HERV-K

<400>   89
ggcccactat tgtacaaatt gattgtaaaa catgttcaca tgtgttgaac aatatnaaat    60
cagggcccnt tgaaaatgaa cagaataaca gtgattttag ggaacaaagg aagncancca   120
taaggtctgc ctgcctgagg ggtcgggcaa aaacccatat ttttnttntt gcagagagcc   180
tataaatgga cgtgcaagta gganaganat tgctaaattc ttttcctagc aaggaatata   240
atactaagnc cctagggaaa gaattgcatt cctgggggga ggtntataaa cggccgctnt   300
gggagtgtct gtcctatgtg gttgananaa ggactganan acgccctggt cgcctgcagt   360
accctcaggc ttactaggat tgggaaaccc cagtcctggt aaatttgagg tcaggccggt   420
tntttgctnt gaaccctgtt ttntgttaan atgtttatca agacaatacg tgcnccgctg   480
aacatagacc cttatcagga gtttntgatt ttgctctggt cctgtttntt canaagcatg   540
tcatctttgc tctgccttnt gccctttgaa gcatgtgatc tttgtgacct actccctgtt   600
catacacccc tcccctttta aaatccctaa taaaaacttg ctggttttgt ggctcagggg   660
ggcatcatgg acctaccaat acgtgatgtc acccccggtg gcccagctgt aaaaaaaaaa   720
aaa                                                                 723

<210>   90
<211>   765
<212>   DNA
<213>   HERV-K

<400>   90
cnngnnccaa aattttgatt ttgnaaaaaa aaattttccc cccattgtgg ttttggnccc    60
aaataatnaa aaacccggng gccccntttg aaaaaatggn cncanaaatt ncccgggtnt   120
ttttttaggg aacaaanggg aagccccccc aataagggtn tnncctcccn tgagggggtg   180
ggggaaaaaa acccaatttt tttttttttt gcnnnagagc cttaaaaatg gcggtgcaag   240
tagnaaaaan attgntaaat tnttttccca gcaaggaana taattntaag cccctaggga   300
aaaaattgcn ttnttggggg gaggtttata aacgnccnct ctgggagtgt ntgtcntatg   360

tggttganan aaggattnaa anacnccntg gtcgcctgna gnaccctcng gcttattagg 420
attgggaaac cccagtcntg gtaaatttna ggtcnggcng gttttttnnt ttnanccctg 480
ttttttgtta anatntttat caanacaana ngngccccgn tgaananana cccttatcag 540
gagtttntga ttttgctcgg gtcctgtttn ttcaaaagca tgtcattttt gntttgcctt 600
ntgccctttn aagnatgtga tntttgtgac ctactccctg ttcanacncc ccnccccttt 660
taaaatccct aataaaaact tgctggtttt gtggctcagg ggggcatcat ggacctacca 720
anacgtgatg tcncccccgg tggcccagct gtaaaaaaaa aaaaa 765

<210> 91
<211> 780
<212> DNA
<213> HERV-K

<400> 91
ancccanttt ttggtnncaa aatttgaatt gtaaaaaaca atggttncca ccattgttgt 60
tttggancca aatattgnaa antccagngg cncccttnga aaaatggaan cangaaataa 120
nccaggtgnt tttttagggg gaaccaaaag gaaagnccac cccataaagg gtnttgactt 180
gccttgaggg ggtcgggggc aaaaaaagcc aatatttttn tttntttgca gagagcctat 240
aaatggacgt gcaaagtagg aaaaatattg ctaaattctt ttcctagcaa ggaatataat 300
attaagnccc taggaaagaa ttgcattcct ggggggaggt ctataaacgg ccgctctggg 360
agtgtctgtc ctatgtggtt aaganaagga ttgaganacg cccctggtcg cctgcagtac 420
cctcaggctt antaggattg ggaaacccca gtcctggtaa atttgaggtc aggccggttn 480
tttgctttga accctgtttt ntgttaanat gtttatcaag acaanacgtg cnccgctgaa 540
cananaccct tatcaggagt ttctgatttt gctctggtcc tgtttnttca naagcatgtc 600
atctttgctn tgccttctgc cctttgaagc atgtgatntt tgtgacctac tccctgttca 660
tacacccctc cccttttaaa atccctaata aaaacttgct ggttttgtgg ctcagggggg 720
catcatggac ctaccaatac gtgatgtcac ccccggtggc ccagctgtaa aaaaaaaaaa 780

<210> 92
<211> 8
<212> PRT
<213> HERV-K

<400> 92
Glu Ser Ser Lys Leu Ser Ile Thr
1               5

<210> 93
<211> 12
<212> PRT
<213> HERV-K

<400> 93
Leu Lys Glu Gln Ser Trp Leu Pro Ser Leu Gln Cys
1               5                   10

<210> 94
<211> 270
<212> PRT
<213> HERV-K

<400> 94
Gln Asp Phe Asn Gln Ser Ile Asn Ile Val Ser Asp Ser Ala Tyr Val
1               5                   10                  15

Val Gln Ala Thr Lys Asp Ile Glu Arg Ala Leu Ile Lys Tyr Ile Met
            20                  25                  30

Asp Asp Gln Leu Asn Pro Leu Phe Asn Leu Leu Gln Gln Asn Val Arg
        35                  40                  45

Lys Arg Asn Phe Pro Phe Tyr Ile Thr His Ile Arg Ala His Thr Asn
    50                  55                  60

Leu Pro Gly Pro Leu Thr Lys Ala Asn Glu Gln Ala Asp Leu Leu Val
65                  70                  75                  80

Ser Ser Ala Phe Met Glu Ala Gln Glu Leu His Ala Leu Thr His Val
                85                  90                  95

Asn Ala Ile Gly Leu Lys Asn Lys Phe Asp Ile Thr Trp Lys Gln Thr
            100                 105                 110

Lys Asn Ile Val Gln His Cys Thr Gln Cys Gln Ile Leu His Leu Ala
        115                 120                 125

Thr Gln Glu Ala Arg Val Asn Pro Arg Gly Leu Cys Pro Asn Val Leu
    130                 135                 140

Trp Gln Met Asp Val Met His Val Pro Ser Phe Gly Lys Leu Ser Phe
145                 150                 155                 160

Val His Val Thr Val Asp Thr Tyr Ser His Phe Ile Trp Ala Thr Cys
                165                 170                 175

Gln Thr Gly Glu Ser Thr Ser His Val Lys Arg His Leu Leu Ser Cys
            180                 185                 190

Phe Pro Val Met Gly Val Pro Glu Lys Val Lys Thr Asp Asn Gly Pro
        195                 200                 205

Gly Tyr Cys Ser Lys Ala Val Gln Lys Phe Leu Asn Gln Trp Lys Ile
    210                 215                 220

Thr His Thr Ile Gly Ile Leu Tyr Asn Ser Gln Gly Gln Ala Ile Ile
225                 230                 235                 240

Glu Arg Thr Asn Arg Thr Leu Lys Ala Gln Leu Val Lys Gln Lys Lys
                245                 250                 255

Gly Lys Asp Arg Ser Ile Thr Leu Pro Arg Cys Asn Leu Ile
            260                 265                 270

<210>   95
<211>   100
<212>   PRT
<213>   HERV-K

<400>   95

<210>   96
<211>   94
<212>   PRT
<213>   HERV-K

<400>   96
Ile Ile Thr Leu Cys Gly Asn Asp Pro Asp Lys Ile Thr Val Pro Phe
1               5                   10                  15

Asn Lys Gln Gln Val Arg Gln Ala Phe Ile Asn Ser Gly Ala Trp Gln
            20                  25                  30

Ile Gly Leu Ala Asp Phe Val Gly Ile Ile Asp Asn Arg Tyr Pro Lys
        35                  40                  45

Thr Lys Ile Phe Gln Phe Leu Lys Leu Thr Thr Trp Ile Leu Pro Lys
    50                  55                  60

Val Thr Lys His Lys Pro Leu Lys Asn Ala Leu Ala Val Phe Thr Asp
65                  70                  75                  80

Gly Ser Ser Asn Gly Lys Val Ala Tyr Thr Gly Pro Lys Glu
                85                  90

<210>   97
<211>   142
<212>   PRT
<213>   HERV-K

<400>   97
Thr Lys Lys Arg Lys Arg Gln Glu Tyr Asn Thr Pro Gln Met Gln Leu
1               5                   10                  15

Asn Leu Ala Leu Tyr Thr Leu Asn Val Leu Asn Ile Tyr Arg Asn Gln
            20                  25                  30

Thr Thr Thr Ser Ala Glu Gln His Leu Thr Gly Lys Arg Asn Ser Pro
        35                  40                  45

His Glu Gly Lys Leu Ile Trp Trp Lys Asp Asn Lys Asn Lys Thr Trp
    50                  55                  60

Glu Met Gly Lys Val Ile Thr Trp Gly Arg Gly Phe Ala Cys Val Ser
65                  70                  75                  80

Pro Gly Glu Asn Gln Leu Pro Val Trp Ile Pro Thr Arg His Leu Lys
                85                  90                  95

Phe Tyr Asn Glu Leu Thr Gly Asp Ala Lys Lys Ser Val Glu Met Glu
            100                 105                 110

Thr Pro Gln Ser Thr Arg Gln Val Asn Lys Met Val Ile Ser Glu Glu
        115                 120                 125

Gln Lys Lys Leu Pro Ser Ile Lys Glu Ala Glu Leu Pro Ile
    130                 135                 140

<210>   98
<211>   79
<212>   PRT
<213>   HERV-K

<400>   98

<210>   99
<211>   2078
<212>   DNA
<213>   HERV-K

<400>   99
gagataggag aaaactgcct tagggctgga ggtgggacat gctggcggca atactgctct   60
ttaaggcatt gagatgttta tgtatatgca catcaaaagc acagcacttt tttctttacc   120
ttgtttatga tgcagagaca tttgttcaca tgttttcctg ctggccctct ccccactatt   180
accctattgt cctgccacat ccccctctcc gagatggtag agataatgat caataaatac   240
tgagggaact cagagaccgg tgcggcgcgg gtcctccata tgctgagcgc cggtcccctg   300
ggcccacttt tctttctcta tactttgtct ctgttgtctt tcttttctca agtctctcgt   360
tccacctgag gagaaatgcc cacagctgtg gaggcgcagg ccactccatc tggtgcccaa   420
cgtggatgct tttctctagg gtgaagggac tctcgagtgt ggtcattgag gacaagtcaa   480
cgagagattc ccgagtacgt ctacagtgag ccttgtgtct ctcatccctc ctgacgagaa   540
atacccacag gtgtggaggg gctggccccc ttcatctgat gcccaatgtg ggtgcctttc   600
tctagggtga aggtactcta cagtgtggtc attgaggaca agttgacgag agagtcccaa   660
gtacgtccac ggtcagcctt gcgacattta aagttctaca atgaactcac tggagatgca   720
aagaaaagtg tggagatgga gacaccccaa tcgactcgcc agtctacagg tgtatccagc   780
agctccaaag agacagcaac cagcaagaat gggccatagt gacgatggtg gttttgtcaa   840
aaagaaaagg gggggatatg taaggaaaag agagatcaga ctttcactgt gtctatgtag   900
aaaaggaaga cataagaaac tccattttga tctgtactaa gaaaaattgt tttgccttga   960
gatgctgtta atctgtaact ttagccccaa ccctgtgctc acggaaacat gtgctgtaag   1020
gtttaaggga tctagggctg tgcaggatgt accttgttaa caatatgttt gcaggcagta   1080
tgtttggtaa aagtcatcgc cattctccat tctcgattaa ccaggggctc aatgcactgt   1140
ggaaagccac aggaacctct gcccaagaaa gcctggctgt tgtgggaagt cagggacccc   1200
gaatggaggg accagctggt gctgcatcag gaaacataaa ttgtgaagat ttcttggaca   1260
tttatcagtt tccaaaatta atacttttat aatttcttac acctgtctta ctttaatctc   1320
ttaatcctgt tatctttgta agctgaggat atacgtcacc tcaggaccac tattgtacaa   1380
attgattgta aaacatgttc acatgtgttt gaacaatatg aaatcagtgc accttgaaaa   1440
tgaacagaat aacagtgatt ttagggaaca aaggaagaca accataaggt ctgactgcct   1500
gaggggtcgg gcaaaaagcc atatttttct tcttgcagag agcctataaa tggacgtgca   1560
agtaggagag atattgctaa attcttttcc tagcaaggaa tataatacta agaccctagg   1620
gaaagaattg cattcctggg gggaggtcta taaacggccg ctctgggagt gtctgtccta   1680
tgtggttgag ataaggactg agatacgccc tggtctcctg cagtaccctc aggcttacta   1740
ggattgggaa accccagtcc tggtaaattt gaggtcaggc cggttctttg ctctgaaccc   1800

tgttttctgt taagatgttt atcaagacaa tacatgcacc gctgaacata gacccttatc 1860
aggagtttct gattttgctc tggtcctgtt tcttcagaag catgtcatct ttgctctgcc 1920
ttctgccctt tgaagcatgt gatctttgtg acctactccc tgttcataca cccctcccct 1980
tttaaaatcc ctaataaaaa cttgctggtt ttgtggctca ggggggcatc atggacctac 2040
caatacgtga tgtcaccccc ggtggcccag ctgtaaaa 2078

<210> 100
<211> 2112
<212> DNA
<213> HERV-K

<400> 100
gagataggag aaaactgcct tagggctgga ggtgggacat gctggcggca atactgctct 60
ttaaggcatt gagatgttta tgtatatgca catcaaaagc acagcacttt tttctttacc 120
ttgtttatga tgcagagaca tttgttcaca tgttttcctg ctggccctct ccccactatt 180
accctattgt cctgccacat ccccctctcc gagatggtag agataatgat caataaatac 240
tgagggaact cagagaccgg tgcggcgcgg gtcctccata tgctgagcgc cggtcccctg 300
ggcccacttt tctttctcta tactttgtct ctgttgtctt tcttttctca agtctctcgt 360
tccacctgag gagaaatgcc cacagctgtg gaggcgcagg ccactccatc tggtgcccaa 420
cgtggatgct tttctctagg gtgaagggac tctcgagtgt ggtcattgag gacaagtcaa 480
cgagagattc ccgagtacgt ctacagtgag ccttgtgtct ctcatccctc ctgacgagaa 540
atacccacag gtgtggaggg gctggccccc ttcatctgat gcccaatgtg ggtgcctttc 600
tctagggtga aggtactcta cagtgtggtc attgaggaca agttgacgag agagtcccaa 660
gtacgtccac ggtcagcctt gcggagaaaa tcagcttcct gtttggatac ccactagaca 720
tttaaagttc tacaatgaac tcactggaga tgcaaagaaa agtgtggaga tggagacacc 780
ccaatcgact cgccagtcta caggtgtatc cagcagctcc aaagagacag caaccagcaa 840
gaatgggcca tagtgacgat ggtggttttg tcaaaaagaa aaggggggga tatgtaagga 900
aaagagagat cagactttca ctgtgtctat gtagaaaagg aagacataag aaactccatt 960
ttgatctgta ctaagaaaaa ttgttttgcc ttgagatgct gttaatctgt aactttagcc 1020
ccaaccctgt gctcacggaa acatgtgctg taaggtttaa gggatctagg gctgtgcagg 1080
atgtaccttg ttaacaatat gtttgcaggc agtatgtttg gtaaaagtca tcgccattct 1140
ccattctcga ttaaccaggg gctcaatgca ctgtggaaag ccacaggaac ctctgcccaa 1200
gaaagcctgg ctgttgtggg aagtcaggga ccccgaatgg agggaccagc tggtgctgca 1260
tcaggaaaca taaattgtga agatttcttg gacatttatc agtttccaaa attaatactt 1320
ttataatttc ttacacctgt cttactttaa tctcttaatc ctgttatctt tgtaagctga 1380
ggatatacgt cacctcagga ccactattgt acaaattgat tgtaaaacat gttcacatgt 1440
gtttgaacaa tatgaaatca gtgcaccttg aaaatgaaca gaataacagt gattttaggg 1500
aacaaaggaa gacaaccata aggtctgact gcctgagggg tcgggcaaaa agccatattt 1560
ttcttcttgc agagagccta taaatggacg tgcaagtagg agagatattg ctaaattctt 1620
ttcctagcaa ggaatataat actaagaccc tagggaaaga attgcattcc tggggggagg 1680
tctataaacg gccgctctgg gagtgtctgt cctatgtggt tgagataagg actgagatac 1740
gccctggtct cctgcagtac cctcaggctt actaggattg ggaaacccca gtcctggtaa 1800
atttgaggtc aggccggttc tttgctctga accctgtttt ctgttaagat gtttatcaag 1860
acaatacatg caccgctgaa catagaccct tatcaggagt ttctgatttt gctctggtcc 1920
tgtttcttca gaagcatgtc atctttgctc tgccttctgc cctttgaagc atgtgatctt 1980
tgtgacctac tccctgttca tacacccctc cccttttaaa atccctaata aaaacttgct 2040
ggttttgtgg ctcagggggg catcatggac ctaccaatac gtgatgtcac ccccggtggc 2100
ccagctgtaa aa 2112

<210> 101
<211> 1999
<212> DNA
<213> HERV-K

<400> 101
gagataggag aaaactgcct tagggctgga ggtgggacat gctggcggca atactgctct 60
ttaaggcatt gagatgttta tgtatatgca catcaaaagc acagcacttt tttctttacc 120
ttgtttatga tgcagagaca tttgttcaca tgttttcctg ctggccctct ccccactatt 180
accctattgt cctgccacat ccccctctcc gagatggtag agataatgat caataaatac 240
tgagggaact cagagaccgg tgcggcgcgg gtcctccata tgctgagcgc cggtcccctg 300
ggcccacttt tctttctcta tactttgtct ctgttgtctt tcttttctca agtctctcgt 360

tccacctgag gagaaatgcc cacagctgtg gaggcgcagg ccactccatc tggtgcccaa 420
cgtggatgct tttctctagg gtgaagggac tctcgagtgt ggtcattgag gacaagtcaa 480
cgagagattc ccgagtacgt ctacagtgag ccttgtgtct ctcatccctc ctgacgagaa 540
atacccacag gtgtggaggg gctggccccc ttcatctgat gcccaatgtg ggtgcctttc 600
tctagggtga aggtactcta cagtgtggtc attgaggaca agttgacgag agagtcccaa 660
gtacgtccac ggtcagcctt gcgtctacag gtgtatccag cagctccaaa gagacagcaa 720
ccagcaagaa tgggccatag tgacgatggt ggttttgtca aaaagaaaag ggggggatat 780
gtaaggaaaa gagagatcag actttcactg tgtctatgta gaaaaggaag acataagaaa 840
ctccattttg atctgtacta agaaaaattg ttttgccttg agatgctgtt aatctgtaac 900
tttagcccca accctgtgct cacggaaaca tgtgctgtaa ggtttaaggg atctagggct 960
gtgcaggatg taccttgtta acaatatgtt tgcaggcagt atgtttggta aaagtcatcg 1020
ccattctcca ttctcgatta accaggggct caatgcactg tggaaagcca caggaacctc 1080
tgcccaagaa agcctggctg ttgtgggaag tcagggaccc cgaatggagg gaccagctgg 1140
tgctgcatca ggaaacataa attgtgaaga tttcttggac atttatcagt ttccaaaatt 1200
aatactttta taatttctta cacctgtctt actttaatct cttaatcctg ttatctttgt 1260
aagctgagga tatacgtcac ctcaggacca ctattgtaca aattgattgt aaaacatgtt 1320
cacatgtgtt tgaacaatat gaaatcagtg caccttgaaa atgaacagaa taacagtgat 1380
tttagggaac aaaggaagac aaccataagg tctgactgcc tgaggggtcg ggcaaaaagc 1440
catatttttc ttcttgcaga gagcctataa atggacgtgc aagtaggaga gatattgcta 1500
aattcttttc ctagcaagga atataatact aagaccctag ggaaagaatt gcattcctgg 1560
ggggaggtct ataaacggcc gctctgggag tgtctgtcct atgtggttga gataaggact 1620
gagatacgcc ctggtctcct gcagtaccct caggcttact aggattggga aaccccagtc 1680
ctggtaaatt tgaggtcagg ccggttcttt gctctgaacc ctgttttctg ttaagatgtt 1740
tatcaagaca atacatgcac cgctgaacat agacccttat caggagtttc tgattttgct 1800
ctggtcctgt ttcttcagaa gcatgtcatc tttgctctgc cttctgccct ttgaagcatg 1860
tgatctttgt gacctactcc ctgttcatac acccctcccc ttttaaaatc cctaataaaa 1920
acttgctggt tttgtggctc aggggggcat catggaccta ccaatacgtg atgtcacccc 1980
cggtggccca gctgtaaaa 1999

<210> 102
<211> 1911
<212> DNA
<213> HERV-K

<400> 102
gagataggag aaaactgcct tagggctgga ggtgggacat gctggcggca atactgctct 60
ttaaggcatt gagatgttta tgtatatgca catcaaaagc acagcacttt tttctttacc 120
ttgtttatga tgcagagaca tttgttcaca tgttttcctg ctggccctct ccccactatt 180
accctattgt cctgccacat ccccctctcc gagatggtag agataatgat caataaatac 240
tgagggaact cagagaccgg tgcggcgcgg gtcctccata tgctgagcgc cggtcccctg 300
ggcccacttt tctttctcta tactttgtct ctgttgtctt tcttttctca agtctctcgt 360
tccacctgag gagaaatgcc cacagctgtg gaggcgcagg ccactccatc tggtgcccaa 420
cgtggatgct tttctctagg gtgaagggac tctcgagtgt ggtcattgag gacaagtcaa 480
cgagagattc ccgagtacgt ctacagtgag ccttgtgggt gaaggtactc tacagtgtgg 540
tcattgagga caagttgacg agagagtccc aagtacgtcc acggtcagcc ttgcgtctac 600
aggtgtatcc agcagctcca aagagacagc aaccagcaag aatgggccat agtgacgatg 660
gtggttttgt caaaaagaaa agggggggat atgtaaggaa aagagagatc agactttcac 720
tgtgtctatg tagaaaagga agacataaga aactccattt tgatctgtac taagaaaaat 780
tgttttgcct tgagatgctg ttaatctgta actttagccc caaccctgtg ctcacggaaa 840
catgtgctgt aaggtttaag ggatctaggg ctgtgcagga tgtaccttgt taacaatatg 900
tttgcaggca gtatgtttgg taaaagtcat cgccattctc cattctcgat taaccagggg 960
ctcaatgcac tgtggaaagc cacaggaacc tctgcccaag aaagcctggc tgttgtggga 1020
agtcagggac cccgaatgga gggaccagct ggtgctgcat caggaaacat aaattgtgaa 1080
gatttcttgg acatttatca gtttccaaaa ttaatacttt tataatttct tacacctgtc 1140
ttactttaat ctcttaatcc tgttatcttt gtaagctgag gatatacgtc acctcaggac 1200
cactattgta caaattgatt gtaaaacatg ttcacatgtg tttgaacaat atgaaatcag 1260
tgcaccttga aaatgaacag aataacagtg attttaggga acaaaggaag acaaccataa 1320
ggtctgactg cctgaggggt cggcaaaaaa gccatatttt tcttcttgca gagagcctat 1380
aaatggacgt gcaagtagga gagatattgc taaattcttt tcctagcaag gaatataata 1440
ctaagaccct agggaaagaa ttgcattcct ggggggaggt ctataaacgg ccgctctggg 1500
agtgtctgtc ctatgtggtt gagataagga ctgagatacg ccctggtctc ctgcagtacc 1560

ctcaggctta ctaggattgg gaaaccccag tcctggtaaa tttgaggtca ggccggttct 1620
ttgctctgaa ccctgttttc tgttaagatg tttatcaaga caatacatgc accgctgaac 1680
atagaccctt atcaggagtt tctgattttg ctctggtcct gtttcttcag aagcatgtca 1740
tctttgctct gccttctgcc ctttgaagca tgtgatcttt gtgacctact ccctgttcat 1800
acacccctcc ccttttaaaa tccctaataa aaacttgctg gttttgtggc tcaggggggc 1860
atcatggacc taccaatacg tgatgtcacc cccggtggcc cagctgtaaa a 1911

<210> 103
<211> 1990
<212> DNA
<213> HERV-K

<400> 103
   gagataggag aaaactgcct tagggctgga ggtgggacat gctggcggca atactgctct 60
   ttaaggcatt gagatgttta tgtatatgca catcaaaagc acagcacttt tttctttacc 120
   ttgtttatga tgcagagaca tttgttcaca tgttttcctg ctggccctct ccccactatt 180
   accctattgt cctgccacat ccccctctcc gagatggtag agataatgat caataaatac 240
   tgagggaact cagagaccgg tgcggcgcgg gtcctccata tgctgagcgc cggtcccctg 300
   ggcccacttt tctttctcta tactttgtct ctgttgtctt tcttttctca agtctctcgt 360
   tccacctgag gagaaatgcc cacagctgtg gaggcgcagg ccactccatc tggtgcccaa 420
   cgtggatgct ttctctaggg gtgaagggac tctcgagtgt ggtcattgag gacaagtcaa 480
   cgagagattc ccgagtacgt ctacagtgag ccttgtgggt gaaggtactc tacagtgtgg 540
   tcattgagga caagttgacg agagagtccc aagtacgtcc acggtcagcc ttgcgacatt 600
   taaagttcta caatgaactc actggagatg caaagaaaag tgtggagatg gagacacccc 660
   aatcgactcg ccagtctaca ggtgtatcca gcagctccaa agagacagca accagcaaga 720
   atgggccata gtgacgatgg tggttttgtc aaaaagaaaa gggggggata tgtaaggaaa 780
   agagagatca gactttcact gtgtctatgt agaaaaggaa gacataagaa actccatttt 840
   gatctgtact aagaaaaatt gttttgcctt gagatgctgt taatctgtaa ctttagcccc 900
   aaccctgtgc tcacggaaac atgtgctgta aggtttaagg gatctagggc tgtgcaggat 960
   gtaccttgtt aacaatatgt ttgcaggcag tatgtttggt aaaagtcatc gccattctcc 1020
   attctcgatt aaccaggggc tcaatgcact gtggaaagcc acaggaacct ctgcccaaga 1080
   aagcctggct gttgtgggaa gtcagggacc ccgaatggag ggaccagctg gtgctgcatc 1140
   aggaaacata aattgtgaag atttcttgga catttatcag tttccaaaat taatactttt 1200
   ataatttctt acacctgtct tactttaatc tcttaatcct gttatctttg taagctgagg 1260
   atatacgtca cctcaggacc actattgtac aaattgattg taaaacatgt tcacatgtgt 1320
   ttgaacaata tgaaatcagt gcaccttgaa aatgaacaga ataacagtga ttttagggaa 1380
   caaaggaaga caaccataag gtctgactgc ctgaggggtc gggcaaaaag ccatattttt 1440
   cttcttgcag agagcctata aatggacgtg caagtaggag agatattgct aaattctttt 1500
   cctagcaagg aatataatac taagacccta gggaaagaat tgcattcctg gggggaggtc 1560
   tataaacggc cgctctggga gtgtctgtcc tatgtggttg agataaggac tgagatacgc 1620
   cctggtctcc tgcagtaccc tcaggcttac taggattggg aaaccccagt cctggtaaat 1680
   ttgaggtcag gccggttctt tgctctgaac cctgttttct gttaagatgt ttatcaagac 1740
   aatacatgca ccgctgaaca tagaccctta tcaggagttt ctgattttgc tctggtcctg 1800
   tttcttcaga agcatgtcat ctttgctctg ccttctgccc tttgaagcat gtgatctttg 1860
   tgacctactc cctgttcata cacccctccc cttttaaaat ccctaataaa aacttgctgg 1920
   ttttgtggct caggggggca tcatggacct accaatacgt gatgtcaccc ccggtggccc 1980
   agctgtaaaa 1990

<210> 104
<211> 2024
<212> DNA
<213> HERV-K

<400> 104
gagataggag aaaactgcct tagggctgga ggtgggacat gctggcggca atactgctct 60
ttaaggcatt gagatgttta tgtatatgca catcaaaagc acagcacttt tttctttacc 120
ttgtttatga tgcagagaca tttgttcaca tgttttcctg ctggccctct ccccactatt 180
accctattgt cctgccacat ccccctctcc gagatggtag agataatgat caataaatac 240
tgagggaact cagagaccgg tgcggcgcgg gtcctccata tgctgagcgc cggtcccctg 300
ggcccacttt tctttctcta tactttgtct ctgttgtctt tcttttctca agtctctcgt 360
tccacctgag gagaaatgcc cacagctgtg gaggcgcagg ccactccatc tggtgcccaa 420

cgtggatgct tttctctagg gtgaagggac tctcgagtgt ggtcattgag gacaagtcaa 480
cgagagattc ccgagtacgt ctacagtgag ccttgtgggt gaaggtactc tacagtgtgg 540
tcattgagga caagttgacg agagagtccc aagtacgtcc acggtcagcc ttgcggagaa 600
aatcagcttc ctgtttggat acccactaga catttaaagt tctacaatga actcactgga 660
gatgcaaaga aaagtgtgga gatggagaca ccccaatcga ctcgccagtc tacaggtgta 720
tccagcagct ccaaagagac agcaaccagc aagaatgggc catagtgacg atggtggttt 780
tgtcaaaaag aaaagggggg gatatgtaag gaaaagagag atcagacttt cactgtgtct 840
atgtagaaaa ggaagacata agaaactcca ttttgatctg tactaagaaa aattgttttg 900
ccttgagatg ctgttaatct gtaactttag ccccaaccct gtgctcacgg aaacatgtgc 960
tgtaaggttt aagggatcta gggctgtgca ggatgtacct tgttaacaat atgtttgcag 1020
gcagtatgtt tggtaaaagt catcgccatt ctccattctc gattaaccag gggctcaatg 1080
cactgtggaa agccacagga acctctgccc aagaaagcct ggctgttgtg ggaagtcagg 1140
gaccccgaat ggagggacca gctggtgctg catcaggaaa cataaattgt gaagatttct 1200
tggacattta tcagtttcca aaattaatac ttttataatt tcttacacct gtcttacttt 1260
aatctcttaa tcctgttatc tttgtaagct gaggatatac gtcacctcag gaccactatt 1320
gtacaaattg attgtaaaac atgttcacat gtgtttgaac aatatgaaat cagtgcacct 1380
tgaaaatgaa cagaataaca gtgattttag ggaacaaagg aagacaacca taaggtctga 1440
ctgcctgagg ggtcgggcaa aaagccatat tttcttcttt gcagagagcc tataaatgga 1500
cgtgcaagta ggagagatat tgctaaattc ttttcctagc aaggaatata atactaagac 1560
cctagggaaa gaattgcatt cctgggggga ggtctataaa cggccgctct gggagtgtct 1620
gtcctatgtg gttgagataa ggactgagat acgccctggt ctcctgcagt accctcaggc 1680
ttactaggat tgggaaaccc cagtcctggt aaatttgagg tcaggccggt tctttgctct 1740
gaaccctgtt ttctgttaag atgtttatca agacaataca tgcaccgctg aacatagacc 1800
cttatcagga gtttctgatt ttgctctggt cctgtttctt cagaagcatg tcatctttgc 1860
tctgccttct gccctttgaa gcatgtgatc tttgtgacct actccctgtt catacacccc 1920
tcccctttta aaatccctaa taaaaacttg ctggttttgt ggctcagggg ggcatcatgg 1980
acctaccaat acgtgatgtc acccccggtg gcccagctgt aaaa 2024

<210> 105
<211> 2176
<212> DNA
<213> HERV-K

<400> 105
gagataggag aaaactgcct tagggctgga ggtgggacat gctggcggca atactgctct 60
ttaaggcatt gagatgttta tgtatatgca catcaaaagc acagcacttt tttctttacc 120
ttgtttatga tgcagagaca tttgttcaca tgttttcctg ctggccctct ccccactatt 180
accctattgt cctgccacat ccccctctcc gagatggtag agataatgat caataaatac 240
tgagggaact cagagaccgg tgcggcgcgg gtcctccata tgctgagcgc cggtcccctg 300
ggcccacttt tctttctcta tactttgtct ctgttgtctt tcttttctca agtctctcgt 360
tccacctgag gagaaatgcc cacagctgtg gaggcgcagg ccactccatc tggtgcccaa 420
cgtggatgct tttctctagg gtgaagggac tctcgagtgt ggtcattgag gacaagtcaa 480
cgagagattc ccgagtacgt ctacagtgag ccttgtgggt gaaggtactc tacagtgtgg 540
tcattgagga caagttgacg agagagtccc aagtacgtcc acggtcagcc ttgcgacatt 600
taaagttcta caatgaactc actggagatg caaagaaaag tgtggagatg gagacacccc 660
aatcgactcg ccaggtaaac aaaatggtga tatcagaaga acagaaaaag ttgccttcca 720
tcaaggaagc agagttgcca atataggcac aattaaagaa gctgacacag ttagctaaaa 780
aaaaaagcct agagaataca aaggtgacac caactccaga gaatatgctg cttgcagctc 840
tgatgattgt atcaacggtg tctacaggtg tatccagcag ctccaaagag acagcaacca 900
gcaagaatgg gccatagtga cgatggtggt tttgtcaaaa agaaaagggg gggatatgta 960
aggaaaagag agatcagact ttcactgtgt ctatgtagaa aaggaagaca taagaaactc 1020
cattttgatc tgtactaaga aaaattgttt tgccttgaga tgctgttaat ctgtaacttt 1080
agccccaacc ctgtgctcac ggaaacatgt gctgtaaggt ttaagggatc tagggctgtg 1140
caggatgtac cttgttaaca atatgtttgc aggcagtatg tttggtaaaa gtcatcgcca 1200
ttctccattc tcgattaacc aggggctcaa tgcactgtgg aaagccacag gaacctctgc 1260
ccaagaaagc ctggctgttg tgggaagtca gggaccccga atggagggac cagctggtgc 1320
tgcatcagga aacataaatt gtgaagattt cttggacatt tatcagtttc caaaattaat 1380
acttttataa tttcttacac ctgtcttact ttaatctctt aatcctgtta tctttgtaag 1440
ctgaggatat acgtcacctc aggaccacta ttgtacaaat tgattgtaaa acatgttcac 1500
atgtgtttga acaatatgaa atcagtgcac cttgaaaatg aacagaataa cagtgatttt 1560
agggaacaaa ggaagacaac cataaggtct gactgcctga ggggtcgggc aaaaagccat 1620

atttttcttc ttgcagagag cctataaatg gacgtgcaag taggagagat attgctaaat 1680
tcttttccta gcaaggaata taatactaag accctaggga aagaattgca ttcctggggg 1740
gaggtctata aacggccgct ctgggagtgt ctgtcctatg tggttgagat aaggactgag 1800
atacgccctg gtctcctgca gtaccctcag gcttactagg attgggaaac cccagtcctg 1860
gtaaatttga ggtcaggccg gttctttgct ctgaaccctg ttttctgtta agatgtttat 1920
caagacaata catgcaccgc tgaacataga cccttatcag gagtttctga ttttgctctg 1980
gtcctgtttc ttcagaagca tgtcatcttt gctctgcctt ctgccctttg aagcatgtga 2040
tctttgtgac ctactccctg ttcatacacc cctccccttt taaaatccct aataaaaact 2100
tgctggtttt gtggctcagg ggggcatcat ggacctacca atacgtgatg tcacccccgg 2160
tggcccagct gtaaaa 2176

<210> 106
<211> 2210
<212> DNA
<213> HERV-K

<400> 106
gagataggag aaaactgcct tagggctgga ggtgggacat gctggcggca atactgctct 60
ttaaggcatt gagatgttta tgtatatgca catcaaaagc acagcacttt tttctttacc 120
ttgtttatga tgcagagaca tttgttcaca tgttttcctg ctggccctct ccccactatt 180
accctattgt cctgccacat ccccctctcc gagatggtag agataatgat caataaatac 240
tgagggaact cagagaccgg tgcggcgcgg gtcctccata tgctgagcgc cggtcccctg 300
ggcccacttt tctttctcta tactttgtct ctgttgtctt tcttttctca agtctctcgt 360
tccacctgag gagaaatgcc cacagctgtg gaggcgcagg ccactccatc tggtgcccaa 420
cgtggatgct tttctctagg gtgaagggac tctcgagtgt ggtcattgag gacaagtcaa 480
cgagagattc ccgagtacgt ctacagtgag ccttgtgggt gaaggtactc tacagtgtgg 540
tcattgagga caagttgacg agagagtccc aagtacgtcc acggtcagcc ttgcggagaa 600
aatcagcttc ctgtttggat acccactaga catttaaagt tctacaatga actcactgga 660
gatgcaaaga aaagtgtgga gatggagaca ccccaatcga ctcgccaggt aaacaaaatg 720
gtgatatcag aagaacagaa aaagttgcct tccatcaagg aagcagagtt gccaatatag 780
gcacaattaa agaagctgac acagttagct aaaaaaaaaa gcctagagaa tacaaaggtg 840
acaccaactc cagagaatat gctgcttgca gctctgatga ttgtatcaac ggtgtctaca 900
ggtgtatcca gcagctccaa agagacagca accagcaaga atggccatag gtgacgatgg 960
tggttttgtc aaaaagaaaa gggggggata tgtaaggaaa agagagatca gactttcact 1020
gtgtctatgt agaaaaggaa gacataagaa actccatttt gatctgtact aagaaaaatt 1080
gttttgcctt gagatgctgt taatctgtaa ctttagcccc aaccctgtgc tcacggaaac 1140
atgtgctgta aggtttaagg gatctagggc tgtgcaggat gtaccttgtt aacaatatgt 1200
ttgcaggcag tatgtttggt aaaagtcatc gccattctcc attctcgatt aaccaggggc 1260
tcaatgcact gtggaaagcc acaggaacct ctgcccaaga aagcctggct gttgtgggaa 1320
gtcagggacc ccgaatggag ggaccagctg gtgctgcatc aggaaacata aattgtgaag 1380
atttcttgga catttatcag tttccaaaat taatactttt ataatttctt acacctgtct 1440
tactttaatc tcttaatcct gttatctttg taagctgagg atatacgtca cctcaggacc 1500
actattgtac aaattgattg taaaacatgt tcacatgtgt ttgaacaata tgaaatcagt 1560
gcaccttgaa aatgaacaga ataacagtga ttttagggaa caaaggaaga caaccataag 1620
gtctgactgc ctgaggggtc gggcaaaaag ccatattttt cttcttgcag agagcctata 1680
aatggacgtg caagtaggag agatattgct aaattctttt cctagcaagg aatataatac 1740
taagacccta gggaaagaat tgcattcctg gggggaggtc tataaacggc cgctctggga 1800
gtgtctgtcc tatgtggttg agataaggac tgagatacgc cctggtctcc tgcagtaccc 1860
tcaggcttac taggattggg aaaccccagt cctggtaaat ttgaggtcag gccggttctt 1920
tgctctgaac cctgttttct gttaagatgt ttatcaagac aatacatgca ccgctgaaca 1980
tagaccctta tcaggagttt ctgattttgc tctggtcctg tttcttcaga agcatgtcat 2040
ctttgctctg ccttctgccc tttgaagcat gtgatctttg tgacctactc cctgttcata 2100
cacccctccc cttttaaaat ccctaataaa aacttgctgg ttttgtggct caggggggca 2160
tcatggacct accaatacgt gatgtcaccc ccggtggccc agctgtaaaa 2210

<210> 107
<211> 1907
<212> DNA
<213> HERV-K

<400> 107

ttttgcttgt gtttcaccag gagaaaatca gcttcctgtt tggataccca ctagacattt 60
aaagttctac aatgaactca ctggagatgc aaagaaaagt gtggagatgg agacacccca 120
atcgactcgc caggtaaaca aaatggtgat atcagaagaa cagaaaaagt tgccttccat 180
caaggaagca gagttgccaa tataggcaca attaaagaag ctgacacagt tagctaaaaa 240
aaaaagccta gagaatacaa aggtgacacc aactccagag aatatgctgc ttgcagctct 300
gatgattgta tcaacggtgg taagtcttcc caagtctgca ggagcagctg cagctaatta 360
tacttactgg gcctatgtgc ctttcccacc cttaattcgg gcagttacat agatggataa 420
tcctattgaa gtagatgtta ataatagtgc atgggtgcct ggccccacag atgactgttg 480
ccctgcccaa cctgaagaag gaatgatgat gaatatttcc attgggtatc cttatcctcc 540
tgtttgccta gggaaggcac caggatgctt aatgcctaca acccaaaatt gtctacaggt 600
gtatccagca gctccaaaga gacagcaacc agcaagaatg ggccatagtg acgatggtgg 660
ttttgtcaaa aagaaaaggg ggggatatgt aaggaaaaga gagatcagac tttcactgtg 720
tctatgtaga aaaggaagac ataagaaact ccattttgat ctgtactaag aaaaattgtt 780
ttgccttgag atgctgttaa tctgtaactt tagccccaac cctgtgctca cggaaacatg 840
tgctgtaagg tttaagggat ctagggctgt gcaggatgta ccttgttaac aatatgtttg 900
caggcagtat gtttggtaaa agtcatcgcc attctccatt ctcgattaac caggggctca 960
atgcactgtg gaaagccaca ggaacctctg cccaagaaag cctggctgtt gtgggaagtc 1020
agggaccccg aatggaggga ccagctggtg ctgcatcagg aaacataaat tgtgaagatt 1080
tcttggacat ttatcagttt ccaaaattaa tacttttata atttcttaca cctgtcttac 1140
tttaatctct taatcctgtt atctttgtaa gctgaggata tacgtcacct caggaccact 1200
attgtacaaa ttgattgtaa aacatgttca catgtgtttg aacaatatga aatcagtgca 1260
ccttgaaaat gaacagaata acagtgattt tagggaacaa aggaagacaa ccataaggtc 1320
tgactgcctg aggggtcggg caaaaagcca tatttttctt cttgcagaga gcctataaat 1380
ggacgtgcaa gtaggagaga tattgctaaa ttcttttcct agcaaggaat ataatactaa 1440
gaccctaggg aaagaattgc attcctgggg ggaggtctat aaacggccgc tctgggagtg 1500
tctgtcctat gtggttgaga taaggactga gatacgccct ggtctcctgc agtaccctca 1560
ggcttactag gattgggaaa ccccagtcct ggtaaatttg aggtcaggcc ggttctttgc 1620
tctgaaccct gttttctgtt aagatgttta tcaagacaat acatgcaccg ctgaacatag 1680
acccttatca ggagtttctg attttgctct ggtcctgttt cttcagaagc atgtcatctt 1740
tgctctgcct tctgcccttt gaagcatgtg atctttgtga cctactccct gttcatacac 1800
ccctcccctt ttaaaatccc taataaaaac ttgctggttt tgtggctcag gggggcatca 1860
tggacctacc aatacgtgat gtcacccccg gtggcccagc tgtaaaa 1907

<210> 108
<211> 1959
<212> DNA
<213> HERV-K

<400> 108
ttttgcttgt gtttcaccag gagaaaatca gcttcctgtt tggataccca ctagacattt 60
aaagttctac aatgaactca ctggagatgc aaagaaaagt gtggagatgg agacacccca 120
atcgactcgc caggtaaaca aaatggtgat atcagaagaa cagaaaaagt tgccttccat 180
caaggaagca gagttgccaa tataggcaca attaaagaag ctgacacagt tagctaaaaa 240
aaaaagccta gagaatacaa aggtgacacc aactccagag aatatgctgc ttgcagctct 300
gatgattgta tcaacggtgg taagtcttcc caagtctgca ggagcagctg cagctaatta 360
tacttactgg gcctatgtgc ctttcccacc cttaattcgg gcagttacat agatggataa 420
tcctattgaa gtagatgtta ataatagtgc atgggtgcct ggccccacag atgactgttg 480
ccctgcccaa cctgaagaag gaatgatgat gaatatttcc attgggtatc cttatcctcc 540
tgtttgccta gggaaggcac caggatgctt aatgcctaca acccaaaatt ggttggtaga 600
agtacctaca gtcagtgcta ccagtagatt tacttatcac atgtctacag gtgtatccag 660
cagctccaaa gagacagcaa ccagcaagaa tgggccatag tgacgatggt ggttttgtca 720
aaaagaaaag ggggggatat gtaaggaaaa gagagatcag actttcactg tgtctatgta 780
gaaaaggaag acataagaaa ctccattttg atctgtacta agaaaaattg ttttgccttg 840
agatgctgtt aatctgtaac tttagcccca accctgtgct cacggaaaca tgtgctgtaa 900
ggtttaaggg atctagggct gtgcaggatg taccttgtta acaatatgtt tgcaggcagt 960
atgtttggta aaagtcatcg ccattctcca ttctcgatta accaggggct caatgcactg 1020
tggaaagcca caggaacctc tgcccaagaa agcctggctg ttgtgggaag tcagggaccc 1080
cgaatggagg gaccagctgg tgctgcatca ggaaacataa attgtgaaga tttcttggac 1140
atttatcagt ttccaaaatt aatactttta taatttctta cacctgtctt actttaatct 1200
cttaatcctg ttatctttgt aagctgagga tatacgtcac ctcaggacca ctattgtaca 1260
aattgattgt aaaacatgtt cacatgtgtt tgaacaatat gaaatcagtg caccttgaaa 1320

atgaacagaa taacagtgat tttagggaac aaaggaagac aaccataagg tctgactgcc 1380
tgaggggtcg ggcaaaaagc catatttttc ttcttgcaga gagcctataa atggacgtgc 1440
aagtaggaga gatattgcta aattcttttc ctagcaagga atataatact aagaccctag 1500
ggaaagaatt gcattcctgg ggggaggtct ataaacggcc gctctgggag tgtctgtcct 1560
atgtggttga gataaggact gagatacgcc ctggtctcct gcagtaccct caggcttact 1620
aggattggga aaccccagtc ctggtaaatt tgaggtcagg ccggttcttt gctctgaacc 1680
ctgttttctg ttaagatgtt tatcaagaca atacatgcac cgctgaacat agacccttat 1740
caggagtttc tgattttgct ctggtcctgt ttcttcagaa gcatgtcatc tttgctctgc 1800
cttctgccct ttgaagcatg tgatctttgt gacctactcc ctgttcatac acccctcccc 1860
ttttaaaatc cctaataaaa acttgctggt tttgtggctc aggggggcat catggaccta 1920
ccaatacgtg atgtcacccc cggtggccca gctgtaaaa 1959

<210> 109
<211> 1936
<212> DNA
<213> HERV-K

<400> 109
gagaagaaaa ccaccctgtg gctggaggtg agatatgcta gcggcaatgc tgctctgtta 60
ctctttgcta cactgagatg tttgggtgga gagaagcata aatctggcct atgtgcacat 120
ctgggcacag aacctcccct tgaacttgtg acacagattc ctttgttcac atgttttcct 180
gctgaccttc tccccactat cgccctgttc tcccaccgca ttccccttgc tgagatagtg 240
aaaatagtaa tctgtagata ccaagggaac tcagagacca tggccggtgc acatcctccg 300
tacgctgagc gctggtcccc tgggcccatt gttctttctc tatactttgt ctctgtgtct 360
tatttctttc ctcagtctct catccctcct gacgagaaat acccacaggt gtggaggggc 420
tggccccctt catctgatgc ccaatgtggg tgcctttctc tagggtgaag gtactctaca 480
gtgtggtcat tgaggacaag ttgacgagag agtcccaagt acgtccacgg tcagccttgc 540
gacatttaaa gttctacaat gaactcactg gagatgcaaa gaaaagtgtg gagatggaga 600
caccccaatc gactcgccag tctacaggtg tatccagcag ctccaaagag acagcaacca 660
gcaagaatgg gccatagtga cgatggtggt tttgtcaaaa agaaaagggg gggatatgta 720
aggaaaagag agatcagact ttcactgtgt ctatgtagaa aaggaagaca taagaaactc 780
cattttgatc tgtactaaga aaaattgttt tgccttgaga tgctgttaat ctgtaacttt 840
agccccaacc ctgtgctcac ggaaacatgt gctgtaaggt ttaagggatc tagggctgtg 900
caggatgtac cttgttaaca atatgtttgc aggcagtatg tttggtaaaa gtcatcgcca 960
ttctccattc tcgattaacc aggggctcaa tgcactgtgg aaagccacag gaacctctgc 1020
ccaagaaagc ctggctgttg tgggaagtca gggaccccga atggagggac cagctggtgc 1080
tgcatcagga aacataaatt gtgaagattt cttggacatt tatcagtttc caaaattaat 1140
acttttataa tttcttacac ctgtcttact ttaatctctt aatcctgtta tctttgtaag 1200
ctgaggatat acgtcacctc aggaccacta ttgtacaaat tgattgtaaa acatgttcac 1260
atgtgtttga acaatatgaa atcagtgcac cttgaaaatg aacagaataa cagtgatttt 1320
agggaacaaa ggaagacaac cataaggtct gactgcctga ggggtcgggc aaaaagccat 1380
atttttcttc ttgcagagag cctataaatg gacgtgcaag taggagagat attgctaaat 1440
tcttttccta gcaaggaata taatactaag accctaggga aagaattgca ttcctggggg 1500
gaggtctata aacggccgct ctgggagtgt ctgtcctatg tggttgagat aaggactgag 1560
atacgccctg gtctcctgca gtaccctcag gcttactagg attgggaaac cccagtcctg 1620
gtaaatttga ggtcaggccg gttctttgct ctgaaccctg ttttctgtta agatgtttat 1680
caagacaata catgcaccgc tgaacataga cccttatcag gagtttctga ttttgctctg 1740
gtcctgtttc ttcagaagca tgtcatcttt gctctgcctt ctgccctttg aagcatgtga 1800
tctttgtgac ctactccctg ttcatacacc cctccccttt taaaatccct aataaaaact 1860
tgctggtttt gtggctcagg ggggcatcat ggacctacca atacgtgatg tcacccccgg 1920
tggcccagct gtaaaa 1936

<210> 110
<211> 16
<212> PRT
<213> HERV-K

<400> 110
Thr Ala Met Ala Ser Asn Ser Pro Ala Thr Gln Asp Ala Ala Leu Tyr
1               5                   10                  15

<210>   111
<211>   69
<212>   DNA
<213>   HERV-K

<400>   111
acgcaggtta gacaagcaca aaccccaaga gaaaatcaag tagaaaggga cagagtctct   60
atcccggca                                                           69

<210>   112
<211>   51
<212>   DNA
<213>   HERV-K

<400>   112
cccacagcga tggcgtctaa ttcaccagca acacaggacg cggcgctgta t            51

<210>   113
<211>   780
<212>   DNA
<213>   HERV-K

<400>   113
ncggcctncg gctgcgnnta ntcgacagna nggngggtag gccttatttt agggagatca   60
agtctaaatt tgaagggagt ccaaattcat actggggtaa tttattcaga ttataaaggg   120
ggaattcagt tagtgtcagc tccactgttc cccggagtgc caatccaggt gatagaattg   180
ctcaattact gcttttgcct tatgttaaaa ttggggaaaa caaaacggaa agaacaggag   240
ggtttggaag taccaaccct gcaggaaaag ctgcttattg ggctaatcag gtctcagaag   300
atagacccgt gtgtacagtc actattcagg gaaagagttt gaaggattag tggataccca   360
ggctgattct atcatcggca taggtaccgc ctcagaagtg tatcaaagtg ccatgatttt   420
acattgtcta ggatctgata atcaagaaag tacggttcag cctgtgatca cttcattcca   480
atcaatttat ggggccgaga cttgttacaa caatggcatg cagagattac tatcccagcc   540
tccctataca gccccaggaa tcaaaaaatc atgactaaaa tgggatagct ccctaaaaag   600
ggactaggaa agaaagaagt cccaattgag gctgaaaaaa tcaaaaaaga aangaatagg   660
gcatcctttt taggagcgtc actgtanagc ctccaaaccc attcattaac ttgggaaaaa   720
aaactgnntg gtaaatcanc anccgcttcc aaaaaaaaaa aaaaaaaaaa cccnnnnccn   780

<210>   114
<211>   1058
<212>   DNA
<213>   HERV-K

<400>   114
atctttaccc tgtataaaca tctttctctt cccagtattt ctaagcatgt gacaatgaat   60
atgcaaagga agcgcagcag tccaccaggt gtgggatatg tgtggcacaa ttcaagacaa   120
tgattaaacc tccacttgat gttgcaaaag agattttgaa aaatttgctt tcaccacacc   180
agcctaaata ataaagaacc agccaccagg tttcagtgga aagtattgcc tcagggaatg   240
cttaatagtt caactatttg tcagctcaag ctctgcaacc agttagagac aagttttcag   300
actgttacat cgttcactat gttgatattt tgtgtgctgc agaaacgaga gacaaattaa   360
ttgaccgtta cacatttctg cagacagagg ttgccaacgc gggactgaca ataacatctg   420
ataagattca agcctctact cctttccgtt acttgggaat gcaggtagag gaaaggaaaa   480
ttaaaccaca aaaaaataga aataagaaaa gacacattaa aagcattaaa tgagtttcaa   540
aagttgctag gagatactaa ttggatttgg agatattaat tggatttggc caactctagg   600
cattcctact tatgccatgt caaatttgtt ctctttctta agaggggact cggaattaaa   660
tagtgaaaga acgttaactc cagaggcaac taaagaaatt aaattaattg aagaaaaaat   720
tcggtcagca caagtaaata gaatagatca cttggcccca ctccaaattt tgatttttac   780
tactgcacat tccctaacag gcatcattgt tcaaaacaca gatcttgtgg agtggtcctt   840
ccttcctcac agtacaatta agacttttac attgtacttg gatcaaatgg ctacattaat   900
tggtcaggga agattatgaa taataacatt gtgtggaaat gacccagata aaatcactgt   960
tcctttcaac aagcaacagg ttagacaagc ctttatcaat tctggtgcat ggcagattgg   1020

tcttgccgat tttgtgggaa ttattgacaa tcgttacc 1058

<210> 115
<211> 842
<212> DNA
<213> HERV-K

<400> 115
ccaaaagaat gagtcatcaa aactcagtat cacttgactc aaagagcaga gttggttgcc 60
gtcattacag tgttaacaag attttaatca gtctattaac attgtatcag attctgcata 120
tgtagtacag gctacaaagg atattgagag agccctaatc aaatacatta tggatgatca 180
gttaaacccg ctgtttaatt tgttacaaca aaatgtaaga aaaagaaatt tcccatttta 240
tattactcat attcgagcac acactaattt accagggcct ttaactaaag caaatgaaca 300
agctgactcg ctagtatcat ctgcattcat ggaagcacaa gaccttcatg ccttgactca 360
tgtaaatgca ataggattaa aaaataaatt taatatcaca tggaaacaga caaaaaatat 420
tgtacaacat tgcacccagt gtcagattct acacctggcc actcaggagg caagagttaa 480
tcccagaggt ctatgtccta atgtgttatg gcaaatggat gtcatgcacg taccttcatt 540
tggaaaattg tcatttgtcc atgtgacagt tgatacttat tcacatttca tatgggcaac 600
ctgccagaca ggagaaagta cttcccatgt taagagacat ttattatctt gttttcctgt 660
catgggagtt ccagaaaaag ttaaaacaga caatggccca ggttactgta gtaaagcagt 720
tcaaaaattc ttaaatcagt ggaaaattac acatacaata ggaattctct ataattccca 780
aggacaggcc ataattgaaa gaactaatag aacactcaaa gctcaattgg ttaaacaaaa 840
aa 842

<210> 116
<211> 661
<212> DNA
<213> HERV-K

<400> 116
ccttacggcc gggaagggcc ggaaagggag tcaagcagga gcgtctgtcc gaacggaggc 60
taggtaagaa tatttcacca tgaaaatgtt aaaagacata aaggaaggag ctaaacaata 120
tggacccaac tctccttata tgagaacgtt attagattcc attgctcatg gaaatagact 180
tattccttat gattgggaaa ttttacctaa atcttccctt tcaccctctc agtatctaca 240
gtttaaaacc tggtggattg atggagtaca agaacaggta cggaaaaatc aggctactta 300
tcctgttgtt aatatagatg cagaccaatt gctaggaaca cgtccaaatt ggagcactat 360
taaccaacaa tcagtaatgc aaaatgaggc tattgaacaa ctaggggcta tttgcctcag 420
ggcctgggaa aagattcagg acccaggaac cagttagaga cagttttcag actgttatat 480
cattcattat gttgatgata ttttgtgtgc tgcagaaaca agagacaaat taattgactt 540
ttacatgttt ctgcagacag aggttgcaaa cacaggcctg acaatagcat ctgataagat 600
tcagacctcc actcctttta attatttggg aatgcaggta gaggaaagaa aaattaaacc 660
a 661

<210> 117
<211> 711
<212> DNA
<213> HERV-K

<400> 117
ctgaaaaaaa tcaaaaaaga aaaggaatag ggcatccttt ttaggagcgg tcactgtaga 60
gcctccaaaa cccattccat taacttgggg gaaaaaaaaa caactgtatg gtaaatcagc 120
agcgcttcca aaacaaaaac tggaggcttt acatttatta gcaaagaaac aattagaaaa 180
aggacattga gccttcattt tcgccttgga attctgtttg taattcagaa aaaatccggc 240
agatggcgta taatgccgta attcaaccca tgggggctct cccaccccgg ttgccctctc 300
cagccatggt cccctttaat tataattgat ctgaaggatt gcttttttac cattcctctg 360
gcaaaacagg attttgagaa atttgctttt accacaccag cctaaataat aaagaaccag 420
ccaccaggtt tcagtggaaa gtattgcctc agggaatgct taatagttca actatttgtc 480
agctcaagct ctgcaaccag ttagagacaa gttttcagac tgttacatcg ttcactatgt 540
tgatattttg tgtgctgcag aaacgagaga caaattaatt gaccgttaca catttctgca 600
gacagaggtt gccaacgcgg gactgacaat aacatctgat aagattcaaa cctctactcc 660
tttccgttac ttgggaatgc aggtagagga aaggaaaatt aaaccacaaa a 711

<210>   118
<211>   838
<212>   DNA
<213>   HERV-K

<400>   118
acaacaatgg catgcagaga ttactatccc agcctcccta tacagcccca ggaatcaaaa   60
aatcatgact aaaatgggat agctccctaa aaagggacta ggaaagaaag aagtcccaat   120
tgaggctgaa aaaaattaaa aaagaaaagg aatagggcat cctttttagg agcggtcact   180
gtagagcctc caaaacccat tccattaact tgggaaaaaa aaaactgtat ggtaaatcag   240
cagccgcttc caaaacaaaa gctggaggcc ttacacttat tagcaaagaa accattagaa   300
aaaggacatt gagccttcat tttcgccttg gaattctgtt tgtgattcag aaaaaatccg   360
gcagatggcg tatgctaact gagccattaa tgccgtaatt caacccatgg gggctctccc   420
accccggttg ccctctccag ccatggtccc ctttaattat aattgatctg aaggattgct   480
tttttaccat tcctctggca aaacaggatt ttgaaaaatt tgcttttacc acaccagcct   540
aaataataaa gaaccagcca ccaggtttca gtggaaagta ttgcctcagg gaatgcttaa   600
tagttcaact atttgtcagc tcaagctctg caaccagtta gagacaagtt ttcagactgt   660
tacatcgttc actatgttga tattttgtgt gctgcagaaa cgagagacaa attaattgac   720
cgttacacat ttctgcagac agaggttgcc aacgcggggc tgacaataac atctgataag   780
attcaaacct ctactccttt ccgttacttg ggaatgcagg tagaggaaag gaaaatta   838

<210>   119
<211>   762
<212>   DNA
<213>   HERV-K

<400>   119
cattagaaaa aggacattga gccttcattt tcgccttgga attctgtttg taattcagaa   60
aaaatccggc agatggcgta tgctaactga gccattaatg ccgtaattca acccatgggg   120
gctctcccac cccggttgcc ctctccagcc atggtcccct ttaattataa ttgatctgaa   180
ggattgcttt tttaccattc ctctggcaaa acaggatttt gaaaaatttg cttttaccac   240
accagcctaa ataataaaga accagccacc aggtttcagt ggaaagtatt gcctcaggga   300
atgcttaata gttcaactat ttgtcagctc aagctctgca accagttaga gacaagtttt   360
cagactgtta catcgttcac tatgttgata ttttgtgtgc tgcagaaacg agagacaaat   420
taattgaccg ttacacattt ctgcagacag aggttgccaa cgcgggactg acaataacat   480
ctgataagat tcaaacctct actcctttcc gttacttggg aatgcaggta gaggaaagga   540
aaattaaacc acaaaaaata gaaataagaa aagacacatt aaaagcatta aatgagtttc   600
aaaagttgct aggagatact aattggattt ggagatatta attggatttg gccaactcta   660
ggcattccta cttatgccat gtcaaatttg tactctttct taagagggga ctcggaatta   720
aatagtgaaa gaacgttaac tccagaggca actaaagaaa aa   762

<210>   120
<211>   25
<212>   DNA
<213>   HERV-K

<400>   120
actgagatag gagaaaactg cctta   25

<210>   121
<211>   25
<212>   DNA
<213>   HERV-K

<400>   121
gataggagaa aactgcctta gggct   25

<210>   122
<211>   25
<212>   DNA

<213>   HERV-K

<400>   122
gagaaaactg ccttagggct ggagg                                          25

<210>   123
<211>   25
<212>   DNA
<213>   HERV-K

<400>   123
aactgcctta gggctggagg tggga                                          25

<210>   124
<211>   25
<212>   DNA
<213>   HERV-K

<400>   124
ccttagggct ggaggtggga catgc                                          25

<210>   125
<211>   25
<212>   DNA
<213>   HERV-K

<400>   125
gggctggagg tgggacatgc tggcg                                          25

<210>   126
<211>   25
<212>   DNA
<213>   HERV-K

<400>   126
ggaggtggga catgctggcg gcaat                                          25

<210>   127
<211>   25
<212>   DNA
<213>   HERV-K

<400>   127
tgggacatgc tggcggcaat actgc                                          25

<210>   128
<211>   25
<212>   DNA
<213>   HERV-K

<400>   128
catgctggcg gcaatactgc tcttt                                          25

<210>   129
<211>   25
<212>   DNA
<213>   HERV-K

<400>   129
tggcggcaat actgctcttt aaggc                                          25

<210>   130
<211>   25
<212>   DNA
<213>   HERV-K

<400>   130
gcaatactgc tctttaaggc attga                                          25

<210>   131
<211>   25
<212>   DNA
<213>   HERV-K

<400>   131
actgctcttt aaggcattga gatgt                                          25

<210>   132
<211>   25
<212>   DNA
<213>   HERV-K

<400>   132
tctttaaggc attgagatgt ttatg                                          25

<210>   133
<211>   25
<212>   DNA
<213>   HERV-K

<400>   133
aaggcattga gatgtttatg tatat                                          25

<210>   134
<211>   25
<212>   DNA
<213>   HERV-K

<400>   134
attgagatgt ttatgtatat gcaca                                          25

<210>   135
<211>   25
<212>   DNA
<213>   HERV-K

<400>   135
gatgtttatg tatatgcaca tcaaa                                          25

<210>   136
<211>   25
<212>   DNA
<213>   HERV-K

<400>   136
ttatgtatat gcacatcaaa agcac                                          25

<210>   137
<211>   25
<212>   DNA
<213>   HERV-K

<400>   137
tatatgcaca tcaaaagcac agcac                                          25

<210>   138
<211>   25
<212>   DNA
<213>   HERV-K

<400>   138
gcacatcaaa agcacagcac ttttt                                          25

<210>   139
<211>   25
<212>   DNA
<213>   HERV-K

<400>   139
tcaaaagcac agcacttttt tcttt                                          25

<210>   140
<211>   25
<212>   DNA
<213>   HERV-K

<400>   140
   agcacagcac ttttttcttt acctt                                          25

<210>   141
<211>   25
<212>   DNA
<213>   HERV-K

<400>   141
agcacttttt tctttacctt gttta                                          25

<210>   142
<211>   25
<212>   DNA
<213>   HERV-K

<400>   142
tttttctttt accttgttta tgatg                                          25

<210>   143
<211>   25
<212>   DNA
<213>   HERV-K

<400>   143
tctttacctt gtttatgatg cagag                                          25

<210>   144
<211>   25
<212>   DNA
<213>   HERV-K

<400>   144
accttgttta tgatgcagag acatt                                          25

<210>   145
<211>   25

<212>   DNA
<213>   HERV-K

<400>   145
gtttatgatg cagagacatt tgttc                                          25

<210>   146
<211>   25
<212>   DNA
<213>   HERV-K

<400>   146
tgatgcagag acatttgttc acatg                                          25

<210>   147
<211>   25
<212>   DNA
<213>   HERV-K

<400>   147
cagagacatt tgttcacatg ttttc                                          25

<210>   148
<211>   25
<212>   DNA
<213>   HERV-K

<400>   148
acatttgttc acatgttttc ctgct                                          25

<210>   149
<211>   25
<212>   DNA
<213>   HERV-K

<400>   149
tgttcacatg ttttcctgct ggccc                                          25

<210>   150
<211>   25
<212>   DNA
<213>   HERV-K

<400>   150
acatgttttc ctgctggccc tctcc                                          25

<210>   151
<211>   25
<212>   DNA
<213>   HERV-K

<400>   151
ttttcctgct ggccctctcc ccact                                          25

<210>   152
<211>   25
<212>   DNA
<213>   HERV-K

<400>   152
ctgctggccc tctccccact attac                                          25

<210> 153
<211> 25
<212> DNA
<213> HERV-K

<400> 153
ggccctctcc ccactattac cctat 25

<210> 154
<211> 25
<212> DNA
<213> HERV-K

<400> 154
tctccccact attaccctat tgtcc 25

<210> 155
<211> 25
<212> DNA
<213> HERV-K

<400> 155
ccactattac cctattgtcc tgcca 25

<210> 156
<211> 25
<212> DNA
<213> HERV-K

<400> 156
attaccctat tgtcctgcca catcc 25

<210> 157
<211> 25
<212> DNA
<213> HERV-K

<400> 157
cctattgtcc tgccacatcc ccctc 25

<210> 158
<211> 25
<212> DNA
<213> HERV-K

<400> 158
   tgtcctgcca catccccctc tccga 25

<210> 159
<211> 25
<212> DNA
<213> HERV-K

<400> 159
tgccacatcc ccctctccga gatgg 25

<210> 160
<211> 25
<212> DNA
<213> HERV-K

<400>   160
catccccctc tccgagatgg tagag                                            25

<210>   161
<211>   25
<212>   DNA
<213>   HERV-K

<400>   161
ccctctccga gatggtagag ataat                                            25

<210>   162
<211>   25
<212>   DNA
<213>   HERV-K

<400>   162
tccgagatgg tagagataat gatca                                            25

<210>   163
<211>   25
<212>   DNA
<213>   HERV-K

<400>   163
gatggtagag ataatgatca ataaa                                            25

<210>   164
<211>   25
<212>   DNA
<213>   HERV-K

<400>   164
tagagataat gatcaataaa tactg                                            25

<210>   165
<211>   25
<212>   DNA
<213>   HERV-K

<400>   165
ataatgatca ataaatactg aggga                                            25

<210>   166
<211>   25
<212>   DNA
<213>   HERV-K

<400>   166
gatcaataaa tactgaggga actca                                            25

<210>   167
<211>   25
<212>   DNA
<213>   HERV-K

<400>   167
ataaatactg agggaactca gagac                                            25

<210>   168

<211>   25
<212>   DNA
<213>   HERV-K

<400>   168
tactgaggga actcagagac cggtg                                             25

<210>   169
<211>   25
<212>   DNA
<213>   HERV-K

<400>   169
agggaactca gagaccggtg cggcg                                             25

<210>   170
<211>   25
<212>   DNA
<213>   HERV-K

<400>   170
actcagagac cggtgcggcg cgggt                                             25

<210>   171
<211>   25
<212>   DNA
<213>   HERV-K

<400>   171
gagaccggtg cggcgcgggt cctcc                                             25

<210>   172
<211>   25
<212>   DNA
<213>   HERV-K

<400>   172
cggtgcggcg cgggtcctcc atatg                                             25

<210>   173
<211>   25
<212>   DNA
<213>   HERV-K

<400>   173
cggcgcgggt cctccatatg ctgag                                             25

<210>   174
<211>   25
<212>   DNA
<213>   HERV-K

<400>   174
cgggtcctcc atatgctgag cgccg                                             25

<210>   175
<211>   25
<212>   DNA
<213>   HERV-K

<400>   175

cctccatatg ctgagcgccg gtccc 25

<210> 176
<211> 25
<212> DNA
<213> HERV-K

<400> 176
atatgctgag cgccggtccc ctggg 25

<210> 177
<211> 25
<212> DNA
<213> HERV-K

<400> 177
ctgagcgccg gtcccctggg cccac 25

<210> 178
<211> 25
<212> DNA
<213> HERV-K

<400> 178
cgccggtccc ctgggcccac ttttc 25

<210> 179
<211> 25
<212> DNA
<213> HERV-K

<400> 179
gtcccctggg cccacttttc tttct 25

<210> 180
<211> 25
<212> DNA
<213> HERV-K

<400> 180
ctgggcccac ttttctttct ctata 25

<210> 181
<211> 25
<212> DNA
<213> HERV-K

<400> 181
cccacttttc tttctctata ctttg 25

<210> 182
<211> 25
<212> DNA
<213> HERV-K

<400> 182
ttttctttct ctatactttg tctct 25

<210> 183
<211> 25
<212> DNA

<213>   HERV-K

<400>   183
tttctctata ctttgtctct gttgt                                          25

<210>   184
<211>   25
<212>   DNA
<213>   HERV-K

<400>   184
ctatactttg tctctgttgt ctttc                                          25

<210>   185
<211>   25
<212>   DNA
<213>   HERV-K

<400>   185
ctttgtctct gttgtctttc ttttc                                          25

<210>   186
<211>   25
<212>   DNA
<213>   HERV-K

<400>   186
tctctgttgt ctttcttttc tcaag                                          25

<210>   187
<211>   25
<212>   DNA
<213>   HERV-K

<400>   187
gttgtctttc ttttctcaag tctct                                          25

<210>   188
<211>   25
<212>   DNA
<213>   HERV-K

<400>   188
ctttcttttc tcaagtctct cgttc                                          25

<210>   189
<211>   25
<212>   DNA
<213>   HERV-K

<400>   189
ttttctcaag tctctcgttc cacct                                          25

<210>   190
<211>   25
<212>   DNA
<213>   HERV-K

<400>   190
tcaagtctct cgttccacct gagga                                          25

<210>   191
<211>   25
<212>   DNA
<213>   HERV-K

<400>   191
tctctcgttc cacctgagga gaaat                                             25

<210>   192
<211>   25
<212>   DNA
<213>   HERV-K

<400>   192
cgttccacct gaggagaaat gccca                                             25

<210>   193
<211>   25
<212>   DNA
<213>   HERV-K

<400>   193
cacctgagga gaaatgccca cagct                                             25

<210>   194
<211>   25
<212>   DNA
<213>   HERV-K

<400>   194
gaggagaaat gcccacagct gtgga                                             25

<210>   195
<211>   25
<212>   DNA
<213>   HERV-K

<400>   195
gaaatgccca cagctgtgga ggcgc                                             25

<210>   196
<211>   25
<212>   DNA
<213>   HERV-K

<400>   196
gcccacagct gtggaggcgc aggcc                                             25

<210>   197
<211>   25
<212>   DNA
<213>   HERV-K

<400>   197
cagctgtgga ggcgcaggcc actcc                                             25

<210>   198
<211>   25
<212>   DNA
<213>   HERV-K

<400>   198
gtggaggcgc aggccactcc atctg                                          25

<210>   199
<211>   25
<212>   DNA
<213>   HERV-K

<400>   199
ggcgcaggcc actccatctg gtgcc                                          25

<210>   200
<211>   25
<212>   DNA
<213>   HERV-K

<400>   200
aggccactcc atctggtgcc caacg                                          25

<210>   201
<211>   25
<212>   DNA
<213>   HERV-K

<400>   201
actccatctg gtgcccaacg tggat                                          25

<210>   202
<211>   25
<212>   DNA
<213>   HERV-K

<400>   202
atctggtgcc caacgtggat gcttt                                          25

<210>   203
<211>   25
<212>   DNA
<213>   HERV-K

<400>   203
gtgcccaacg tggatgcttt tctct                                          25

<210>   204
<211>   25
<212>   DNA
<213>   HERV-K

<400>   204
caacgtggat gcttttctct agggt                                          25

<210>   205
<211>   25
<212>   DNA
<213>   HERV-K

<400>   205
tggatgcttt tctctagggt gaagg                                          25

<210>   206
<211>   25

<212>   DNA
<213>   HERV-K

<400>   206
gcttttctct agggtgaagg gactc                                             25

<210>   207
<211>   25
<212>   DNA
<213>   HERV-K

<400>   207
tctctagggt gaagggactc tcgag                                             25

<210>   208
<211>   25
<212>   DNA
<213>   HERV-K

<400>   208
agggtgaagg gactctcgag tgtgg                                             25

<210>   209
<211>   25
<212>   DNA
<213>   HERV-K

<400>   209
gaagggactc tcgagtgtgg tcatt                                             25

<210>   210
<211>   25
<212>   DNA
<213>   HERV-K

<400>   210
gactctcgag tgtggtcatt gagga                                             25

<210>   211
<211>   25
<212>   DNA
<213>   HERV-K

<400>   211
tcgagtgtgg tcattgagga caagt                                             25

<210>   212
<211>   25
<212>   DNA
<213>   HERV-K

<400>   212
tgtggtcatt gaggacaagt caacg                                             25

<210>   213
<211>   25
<212>   DNA
<213>   HERV-K

<400>   213
tcattgagga caagtcaacg agaga                                             25

<210>   214
<211>   25
<212>   DNA
<213>   HERV-K

<400>   214
gaggacaagt caacgagaga ttccc                                             25

<210>   215
<211>   25
<212>   DNA
<213>   HERV-K

<400>   215
caagtcaacg agagattccc gagta                                             25

<210>   216
<211>   25
<212>   DNA
<213>   HERV-K

<400>   216
caacgagaga ttcccgagta cgtct                                             25

<210>   217
<211>   25
<212>   DNA
<213>   HERV-K

<400>   217
agagattccc gagtacgtct acagt                                             25

<210>   218
<211>   25
<212>   DNA
<213>   HERV-K

<400>   218
ttcccgagta cgtctacagt gagcc                                             25

<210>   219
<211>   25
<212>   DNA
<213>   HERV-K

<400>   219
gagtacgtct acagtgagcc ttgtg                                             25

<210>   220
<211>   25
<212>   DNA
<213>   HERV-K

<400>   220
gagaaaatca gcttcctgtt tggat                                             25

<210>   221
<211>   25
<212>   DNA
<213>   HERV-K

<400>   221
aatcagcttc ctgtttggat accca                                          25

<210>   222
<211>   25
<212>   DNA
<213>   HERV-K

<400>   222
gcttcctgtt tggataccca ctaga                                          25

<210>   223
<211>   25
<212>   DNA
<213>   HERV-K

<400>   223
ctgtttggat acccactaga cattt                                          25

<210>   224
<211>   25
<212>   DNA
<213>   HERV-K

<400>   224
acccactaga catttaaagt tctac                                          25

<210>   225
<211>   25
<212>   DNA
<213>   HERV-K

<400>   225
ctagacattt aaagttctac aatga                                          25

<210>   226
<211>   25
<212>   DNA
<213>   HERV-K

<400>   226
ggtgaaggta ctctacagtg tggtc                                          25

<210>   227
<211>   25
<212>   DNA
<213>   HERV-K

<400>   227
aggtactcta cagtgtggtc attga                                          25

<210>   228
<211>   25
<212>   DNA
<213>   HERV-K

<400>   228
ctctacagtg tggtcattga ggaca                                          25

<210>   229

<211>   25
<212>   DNA
<213>   HERV-K

<400>   229
cagtgtggtc attgaggaca agttg                                          25

<210>   230
<211>   25
<212>   DNA
<213>   HERV-K

<400>   230
tggtcattga ggacaagttg acgag                                          25

<210>   231
<211>   25
<212>   DNA
<213>   HERV-K

<400>   231
attgaggaca agttgacgag agagt                                          25

<210>   232
<211>   25
<212>   DNA
<213>   HERV-K

<400>   232
ggacaagttg acgagagagt cccaa                                          25

<210>   233
<211>   25
<212>   DNA
<213>   HERV-K

<400>   233
agttgacgag agagtcccaa gtacg                                          25

<210>   234
<211>   25
<212>   DNA
<213>   HERV-K

<400>   234
acgagagagt cccaagtacg tccac                                          25

<210>   235
<211>   25
<212>   DNA
<213>   HERV-K

<400>   235
agagtcccaa gtacgtccac ggtca                                          25

<210>   236
<211>   25
<212>   DNA
<213>   HERV-K

<400>   236

cccaagtacg tccacggtca gcctt                                          25

<210>  237
<211>  25
<212>  DNA
<213>  HERV-K

<400>  237
gtacgtccac ggtcagcctt gcgac                                          25

<210>  238
<211>  25
<212>  DNA
<213>  HERV-K

<400>  238
tccacggtca gccttgcgac attta                                          25

<210>  239
<211>  25
<212>  DNA
<213>  HERV-K

<400>  239
ggtcagcctt gcgacattta aagtt                                          25

<210>  240
<211>  25
<212>  DNA
<213>  HERV-K

<400>  240
gccttgcgac atttaaagtt ctaca                                          25

<210>  241
<211>  25
<212>  DNA
<213>  HERV-K

<400>  241
gcgacattta aagttctaca atgaa                                          25

<210>  242
<211>  25
<212>  DNA
<213>  HERV-K

<400>  242
atttaaagtt ctacaatgaa ctcac                                          25

<210>  243
<211>  25
<212>  DNA
<213>  HERV-K

<400>  243
aagttctaca atgaactcac tggag                                          25

<210>  244
<211>  25
<212>  DNA

<213>   HERV-K

<400>   244
ctacaatgaa ctcactggag atgca                                             25

<210>   245
<211>   25
<212>   DNA
<213>   HERV-K

<400>   245
atgaactcac tggagatgca aagaa                                             25

<210>   246
<211>   25
<212>   DNA
<213>   HERV-K

<400>   246
ctcactggag atgcaaagaa aagtg                                             25

<210>   247
<211>   25
<212>   DNA
<213>   HERV-K

<400>   247
tggagatgca aagaaaagtg tggag                                             25

<210>   248
<211>   25
<212>   DNA
<213>   HERV-K

<400>   248
atgcaaagaa aagtgtggag atgga                                             25

<210>   249
<211>   25
<212>   DNA
<213>   HERV-K

<400>   249
aagaaaagtg tggagatgga gacac                                             25

<210>   250
<211>   25
<212>   DNA
<213>   HERV-K

<400>   250
aagtgtggag atggagacac cccaa                                             25

<210>   251
<211>   25
<212>   DNA
<213>   HERV-K

<400>   251
tggagatgga gacaccccaa tcgac                                             25

<210>   252
<211>   25
<212>   DNA
<213>   HERV-K

<400>   252
atggagacac cccaatcgac tcgcc                                          25

<210>   253
<211>   25
<212>   DNA
<213>   HERV-K

<400>   253
gacaccccaa tcgactcgcc agtct                                          25

<210>   254
<211>   25
<212>   DNA
<213>   HERV-K

<400>   254
cccaatcgac tcgccagtct acagg                                          25

<210>   255
<211>   25
<212>   DNA
<213>   HERV-K

<400>   255
tcgactcgcc agtctacagg tgtat                                          25

<210>   256
<211>   25
<212>   DNA
<213>   HERV-K

<400>   256
tcgccagtct acaggtgtat ccagc                                          25

<210>   257
<211>   25
<212>   DNA
<213>   HERV-K

<400>   257
agtctacagg tgtatccagc agctc                                          25

<210>   258
<211>   25
<212>   DNA
<213>   HERV-K

<400>   258
acaggtgtat ccagcagctc caaag                                          25

<210>   259
<211>   25
<212>   DNA
<213>   HERV-K

<400>   259
tgtatccagc agctccaaag agaca                                          25

<210>   260
<211>   25
<212>   DNA
<213>   HERV-K

<400>   260
ccagcagctc caaagagaca gcaac                                          25

<210>   261
<211>   25
<212>   DNA
<213>   HERV-K

<400>   261
agctccaaag agacagcaac cagca                                          25

<210>   262
<211>   25
<212>   DNA
<213>   HERV-K

<400>   262
caaagagaca gcaaccagca agaat                                          25

<210>   263
<211>   25
<212>   DNA
<213>   HERV-K

<400>   263
agacagcaac cagcaagaat gggcc                                          25

<210>   264
<211>   25
<212>   DNA
<213>   HERV-K

<400>   264
gcaaccagca agaatgggcc atagt                                          25

<210>   265
<211>   25
<212>   DNA
<213>   HERV-K

<400>   265
cagcaagaat gggccatagt gacga                                          25

<210>   266
<211>   25
<212>   DNA
<213>   HERV-K

<400>   266
agaatgggcc atagtgacga tggtg                                          25

<210>   267
<211>   25

<212>   DNA
<213>   HERV-K

<400>   267
gggccatagt gacgatggtg gtttt                                             25

<210>   268
<211>   25
<212>   DNA
<213>   HERV-K

<400>   268
atagtgacga tggtggtttt gtcaa                                             25

<210>   269
<211>   25
<212>   DNA
<213>   HERV-K

<400>   269
gacgatggtg gttttgtcaa aaaga                                             25

<210>   270
<211>   25
<212>   DNA
<213>   HERV-K

<400>   270
tggtggtttt gtcaaaaaga aaagg                                             25

<210>   271
<211>   25
<212>   DNA
<213>   HERV-K

<400>   271
gttttgtcaa aaagaaaagg ggggg                                             25

<210>   272
<211>   25
<212>   DNA
<213>   HERV-K

<400>   272
gtcaaaaaga aaaggggggg atatg                                             25

<210>   273
<211>   25
<212>   DNA
<213>   HERV-K

<400>   273
aaagaaaagg gggggatatg taagg                                             25

<210>   274
<211>   25
<212>   DNA
<213>   HERV-K

<400>   274
aaaggggggg atatgtaagg aaaag                                             25

<210>   275
<211>   25
<212>   DNA
<213>   HERV-K

<400>   275
gggggatatg taaggaaaag agaga                                          25

<210>   276
<211>   25
<212>   DNA
<213>   HERV-K

<400>   276
atatgtaagg aaaagagaga tcaga                                          25

<210>   277
<211>   25
<212>   DNA
<213>   HERV-K

<400>   277
taaggaaaag agagatcaga ctttc                                          25

<210>   278
<211>   25
<212>   DNA
<213>   HERV-K

<400>   278
aaaagagaga tcagactttc actgt                                          25

<210>   279
<211>   25
<212>   DNA
<213>   HERV-K

<400>   279
agagatcaga ctttcactgt gtcta                                          25

<210>   280
<211>   25
<212>   DNA
<213>   HERV-K

<400>   280
tcagactttc actgtgtcta tgtag                                          25

<210>   281
<211>   25
<212>   DNA
<213>   HERV-K

<400>   281
ctttcactgt gtctatgtag aaaag                                          25

<210>   282
<211>   25
<212>   DNA
<213>   HERV-K

<400>   282
actgtgtcta tgtagaaaag gaaga                                          25

<210>   283
<211>   25
<212>   DNA
<213>   HERV-K

<400>   283
gtctatgtag aaaaggaaga cataa                                          25

<210>   284
<211>   25
<212>   DNA
<213>   HERV-K

<400>   284
tgtagaaaag gaagacataa gaaac                                          25

<210>   285
<211>   25
<212>   DNA
<213>   HERV-K

<400>   285
aaaaggaaga cataagaaac tccat                                          25

<210>   286
<211>   25
<212>   DNA
<213>   HERV-K

<400>   286
gaagacataa gaaactccat tttga                                          25

<210>   287
<211>   25
<212>   DNA
<213>   HERV-K

<400>   287
cataagaaac tccattttga tctgt                                          25

<210>   288
<211>   25
<212>   DNA
<213>   HERV-K

<400>   288
gaaactccat tttgatctgt actaa                                          25

<210>   289
<211>   25
<212>   DNA
<213>   HERV-K

<400>   289
tccattttga tctgtactaa gaaaa                                          25

<210>   290

<211>   25
<212>   DNA
<213>   HERV-K

<400>   290
tttgatctgt actaagaaaa attgt                                          25

<210>   291
<211>   25
<212>   DNA
<213>   HERV-K

<400>   291
tctgtactaa gaaaaattgt tttgc                                          25

<210>   292
<211>   25
<212>   DNA
<213>   HERV-K

<400>   292
actaagaaaa attgttttgc cttga                                          25

<210>   293
<211>   25
<212>   DNA
<213>   HERV-K

<400>   293
gaaaaattgt tttgccttga gatgc                                          25

<210>   294
<211>   25
<212>   DNA
<213>   HERV-K

<400>   294
attgttttgc cttgagatgc tgtta                                          25

<210>   295
<211>   25
<212>   DNA
<213>   HERV-K

<400>   295
tttgccttga gatgctgtta atctg                                          25

<210>   296
<211>   25
<212>   DNA
<213>   HERV-K

<400>   296
cttgagatgc tgttaatctg taact                                          25

<210>   297
<211>   25
<212>   DNA
<213>   HERV-K

<400>   297

gatgctgtta atctgtaact ttagc                                          25

<210>  298
<211>  25
<212>  DNA
<213>  HERV-K

<400>  298
tgttaatctg taactttagc cccaa                                          25

<210>  299
<211>  25
<212>  DNA
<213>  HERV-K

<400>  299
atctgtaact ttagccccaa ccctg                                          25

<210>  300
<211>  25
<212>  DNA
<213>  HERV-K

<400>  300
taactttagc cccaaccctg tgctc                                          25

<210>  301
<211>  25
<212>  DNA
<213>  HERV-K

<400>  301
ttagccccaa ccctgtgctc acgga                                          25

<210>  302
<211>  25
<212>  DNA
<213>  HERV-K

<400>  302
cccaaccctg tgctcacgga aacat                                          25

<210>  303
<211>  25
<212>  DNA
<213>  HERV-K

<400>  303
ccctgtgctc acggaaacat gtgct                                          25

<210>  304
<211>  25
<212>  DNA
<213>  HERV-K

<400>  304
tgctcacgga aacatgtgct gtaag                                          25

<210>  305
<211>  25
<212>  DNA

<213>   HERV-K

<400>   305
acggaaacat gtgctgtaag gttta                                          25

<210>   306
<211>   25
<212>   DNA
<213>   HERV-K

<400>   306
aacatgtgct gtaaggttta aggga                                          25

<210>   307
<211>   25
<212>   DNA
<213>   HERV-K

<400>   307
gtgctgtaag gtttaaggga tctag                                          25

<210>   308
<211>   25
<212>   DNA
<213>   HERV-K

<400>   308
gtaaggttta agggatctag ggctg                                          25

<210>   309
<211>   25
<212>   DNA
<213>   HERV-K

<400>   309
gtttaaggga tctagggctg tgcag                                          25

<210>   310
<211>   25
<212>   DNA
<213>   HERV-K

<400>   310
agggatctag ggctgtgcag gatgt                                          25

<210>   311
<211>   25
<212>   DNA
<213>   HERV-K

<400>   311
tctagggctg tgcaggatgt acctt                                          25

<210>   312
<211>   25
<212>   DNA
<213>   HERV-K

<400>   312
ggctgtgcag gatgtacctt gttaa                                          25

<210>   313
<211>   25
<212>   DNA
<213>   HERV-K

<400>   313
tgcaggatgt accttgttaa caata                                          25

<210>   314
<211>   25
<212>   DNA
<213>   HERV-K

<400>   314
gatgtacctt gttaacaata tgttt                                          25

<210>   315
<211>   25
<212>   DNA
<213>   HERV-K

<400>   315
accttgttaa caatatgttt gcagg                                          25

<210>   316
<211>   25
<212>   DNA
<213>   HERV-K

<400>   316
gttaacaata tgtttgcagg cagta                                          25

<210>   317
<211>   25
<212>   DNA
<213>   HERV-K

<400>   317
caatatgttt gcaggcagta tgttt                                          25

<210>   318
<211>   25
<212>   DNA
<213>   HERV-K

<400>   318
tgtttgcagg cagtatgttt ggtaa                                          25

<210>   319
<211>   25
<212>   DNA
<213>   HERV-K

<400>   319
gcaggcagta tgtttggtaa aagtc                                          25

<210>   320
<211>   25
<212>   DNA
<213>   HERV-K

<400>   320
cagtatgttt ggtaaaagtc atcgc                                          25

<210>   321
<211>   25
<212>   DNA
<213>   HERV-K

<400>   321
tgtttggtaa aagtcatcgc cattc                                          25

<210>   322
<211>   25
<212>   DNA
<213>   HERV-K

<400>   322
ggtaaaagtc atcgccattc tccat                                          25

<210>   323
<211>   25
<212>   DNA
<213>   HERV-K

<400>   323
aagtcatcgc cattctccat tctcg                                          25

<210>   324
<211>   25
<212>   DNA
<213>   HERV-K

<400>   324
atcgccattc tccattctcg attaa                                          25

<210>   325
<211>   25
<212>   DNA
<213>   HERV-K

<400>   325
cattctccat tctcgattaa ccagg                                          25

<210>   326
<211>   25
<212>   DNA
<213>   HERV-K

<400>   326
tccattctcg attaaccagg ggctc                                          25

<210>   327
<211>   25
<212>   DNA
<213>   HERV-K

<400>   327
tctcgattaa ccaggggctc aatgc                                          25

<210>   328
<211>   25

<212>   DNA
<213>   HERV-K

<400>   328
attaaccagg ggctcaatgc actgt                                          25

<210>   329
<211>   25
<212>   DNA
<213>   HERV-K

<400>   329
ccaggggctc aatgcactgt ggaaa                                          25

<210>   330
<211>   25
<212>   DNA
<213>   HERV-K

<400>   330
ggctcaatgc actgtggaaa gccac                                          25

<210>   331
<211>   25
<212>   DNA
<213>   HERV-K

<400>   331
aatgcactgt ggaaagccac aggaa                                          25

<210>   332
<211>   25
<212>   DNA
<213>   HERV-K

<400>   332
actgtggaaa gccacaggaa cctct                                          25

<210>   333
<211>   25
<212>   DNA
<213>   HERV-K

<400>   333
ggaaagccac aggaacctct gccca                                          25

<210>   334
<211>   25
<212>   DNA
<213>   HERV-K

<400>   334
gccacaggaa cctctgccca agaaa                                          25

<210>   335
<211>   25
<212>   DNA
<213>   HERV-K

<400>   335
aggaacctct gcccaagaaa gcctg                                          25

<210>   336
<211>   25
<212>   DNA
<213>   HERV-K

<400>   336
cctctgccca agaaagcctg gctgt                                             25

<210>   337
<211>   25
<212>   DNA
<213>   HERV-K

<400>   337
tgtggggaaa agaaagagag atcag                                             25

<210>   338
<211>   25
<212>   DNA
<213>   HERV-K

<400>   338
ggaaaagaaa gagagatcag actgt                                             25

<210>   339
<211>   25
<212>   DNA
<213>   HERV-K

<400>   339
agaaagagag atcagactgt tactg                                             25

<210>   340
<211>   25
<212>   DNA
<213>   HERV-K

<400>   340
gagagatcag actgttactg tgtct                                             25

<210>   341
<211>   25
<212>   DNA
<213>   HERV-K

<400>   341
atcagactgt tactgtgtct atgta                                             25

<210>   342
<211>   25
<212>   DNA
<213>   HERV-K

<400>   342
actgttactg tgtctatgta gaaag                                             25

<210>   343
<211>   25
<212>   DNA
<213>   HERV-K

<400>   343
tactgtgtct atgtagaaag aaata                                          25

<210>   344
<211>   25
<212>   DNA
<213>   HERV-K

<400>   344
tgtctatgta gaaagaaata gacat                                          25

<210>   345
<211>   25
<212>   DNA
<213>   HERV-K

<400>   345
atgtagaaag aaatagacat aagag                                          25

<210>   346
<211>   25
<212>   DNA
<213>   HERV-K

<400>   346
gaaagaaata gacataagag actcc                                          25

<210>   347
<211>   25
<212>   DNA
<213>   HERV-K

<400>   347
aaatagacat aagagactcc atttt                                          25

<210>   348
<211>   25
<212>   DNA
<213>   HERV-K

<400>   348
gacataagag actccatttt gttct                                          25

<210>   349
<211>   25
<212>   DNA
<213>   HERV-K

<400>   349
aagagactcc attttgttct gtact                                          25

<210>   350
<211>   25
<212>   DNA
<213>   HERV-K

<400>   350
actccatttt gttctgtact aagaa                                          25

<210>   351

<211>   25
<212>   DNA
<213>   HERV-K

<400>   351
attttgttct gtactaagaa aaatt                                             25

<210>   352
<211>   25
<212>   DNA
<213>   HERV-K

<400>   352
gttctgtact aagaaaaatt cttct                                             25

<210>   353
<211>   25
<212>   DNA
<213>   HERV-K

<400>   353
gtactaagaa aaattcttct gcttt                                             25

<210>   354
<211>   25
<212>   DNA
<213>   HERV-K

<400>   354
aagaaaaatt cttctgcttt gagat                                             25

<210>   355
<211>   25
<212>   DNA
<213>   HERV-K

<400>   355
aaattcttct gctttgagat gctgt                                             25

<210>   356
<211>   25
<212>   DNA
<213>   HERV-K

<400>   356
cttctgcttt gagatgctgt taatc                                             25

<210>   357
<211>   25
<212>   DNA
<213>   HERV-K

<400>   357
gctttgagat gctgttaatc tgtaa                                             25

<210>   358
<211>   25
<212>   DNA
<213>   HERV-K

<400>   358

gagatgctgt taatctgtaa cccta                                          25

<210>  359
<211>  25
<212>  DNA
<213>  HERV-K

<400>  359
gctgttaatc tgtaacccta gcccc                                          25

<210>  360
<211>  25
<212>  DNA
<213>  HERV-K

<400>  360
taatctgtaa ccctagcccc aaccc                                          25

<210>  361
<211>  25
<212>  DNA
<213>  HERV-K

<400>  361
tgtaacccta gccccaaccc tgtgc                                          25

<210>  362
<211>  25
<212>  DNA
<213>  HERV-K

<400>  362
ccctagcccc aaccctgtgc tcaca                                          25

<210>  363
<211>  25
<212>  DNA
<213>  HERV-K

<400>  363
gccccaaccc tgtgctcaca gaaac                                          25

<210>  364
<211>  25
<212>  DNA
<213>  HERV-K

<400>  364
aaccctgtgc tcacagaaac aggtg                                          25

<210>  365
<211>  25
<212>  DNA
<213>  HERV-K

<400>  365
gaaacaggtg ctgtgttgac tcaag                                          25

<210>  366
<211>  25
<212>  DNA

<213>   HERV-K

<400>   366
aggtgctgtg ttgactcaag gttta                                             25

<210>   367
<211>   25
<212>   DNA
<213>   HERV-K

<400>   367
ctgtgttgac tcaaggttta atgga                                             25

<210>   368
<211>   25
<212>   DNA
<213>   HERV-K

<400>   368
ttgactcaag gtttaatgga ttcag                                             25

<210>   369
<211>   25
<212>   DNA
<213>   HERV-K

<400>   369
atggattcag ggctgtgcag gatgt                                             25

<210>   370
<211>   25
<212>   DNA
<213>   HERV-K

<400>   370
ttcagggctg tgcaggatgt gcttt                                             25

<210>   371
<211>   25
<212>   DNA
<213>   HERV-K

<400>   371
ggctgtgcag gatgtgcttt gttaa                                             25

<210>   372
<211>   25
<212>   DNA
<213>   HERV-K

<400>   372
tgcaggatgt gctttgttaa acaaa                                             25

<210>   373
<211>   25
<212>   DNA
<213>   HERV-K

<400>   373
gatgtgcttt gttaaacaaa tgctt                                             25

<210>   374
<211>   25
<212>   DNA
<213>   HERV-K

<400>   374
gctttgttaa acaaatgctt gaagg                                          25

<210>   375
<211>   25
<212>   DNA
<213>   HERV-K

<400>   375
gttaaacaaa tgcttgaagg cagca                                          25

<210>   376
<211>   25
<212>   DNA
<213>   HERV-K

<400>   376
gttaagagtc atcaccactc cctaa                                          25

<210>   377
<211>   25
<212>   DNA
<213>   HERV-K

<400>   377
gagtcatcac cactccctaa tctca                                          25

<210>   378
<211>   25
<212>   DNA
<213>   HERV-K

<400>   378
atcaccactc cctaatctca agtaa                                          25

<210>   379
<211>   25
<212>   DNA
<213>   HERV-K

<400>   379
gcagggacac aaacactgcg gaagg                                          25

<210>   380
<211>   25
<212>   DNA
<213>   HERV-K

<400>   380
gacacaaaca ctgcggaagg ccgca                                          25

<210>   381
<211>   25
<212>   DNA
<213>   HERV-K

<400>   381
aaacactgcg gaaggccgca gggac                                             25

<210>   382
<211>   25
<212>   DNA
<213>   HERV-K

<400>   382
ctgcggaagg ccgcagggac ctctg                                             25

<210>   383
<211>   25
<212>   DNA
<213>   HERV-K

<400>   383
gaaggccgca gggacctctg cctag                                             25

<210>   384
<211>   25
<212>   DNA
<213>   HERV-K

<400>   384
ccgcagggac ctctgcctag gaaag                                             25

<210>   385
<211>   25
<212>   DNA
<213>   HERV-K

<400>   385
gggacctctg cctaggaaag ccagg                                             25

<210>   386
<211>   25
<212>   DNA
<213>   HERV-K

<400>   386
ctctgcctag gaaagccagg tgttg                                             25

<210>   387
<211>   25
<212>   DNA
<213>   HERV-K

<400>   387
cctaggaaag ccaggtgttg tccaa                                             25

<210>   388
<211>   25
<212>   DNA
<213>   HERV-K

<400>   388
gaaagccagg tgttgtccaa ggttt                                             25

<210>   389
<211>   25

<212>   DNA
<213>   HERV-K

<400>   389
ccaggtgttg tccaaggttt ctccc                                             25

<210>   390
<211>   25
<212>   DNA
<213>   HERV-K

<400>   390
tgttgtccaa ggtttctccc catgt                                             25

<210>   391
<211>   25
<212>   DNA
<213>   HERV-K

<400>   391
tccaaggttt ctccccatgt gacag                                             25

<210>   392
<211>   25
<212>   DNA
<213>   HERV-K

<400>   392
ggtttctccc catgtgacag tctga                                             25

<210>   393
<211>   25
<212>   DNA
<213>   HERV-K

<400>   393
ctccccatgt gacagtctga aatat                                             25

<210>   394
<211>   25
<212>   DNA
<213>   HERV-K

<400>   394
catgtgacag tctgaaatat ggcct                                             25

<210>   395
<211>   25
<212>   DNA
<213>   HERV-K

<400>   395
tctgaaatat ggcctcttgg gaagg                                             25

<210>   396
<211>   25
<212>   DNA
<213>   HERV-K

<400>   396
aatatggcct cttgggaagg gaaag                                             25

<210>   397
<211>   25
<212>   DNA
<213>   HERV-K

<400>   397
ggcctcttgg gaagggaaag acctg                                             25

<210>   398
<211>   25
<212>   DNA
<213>   HERV-K

<400>   398
cttgggaagg gaaagacctg actgt                                             25

<210>   399
<211>   25
<212>   DNA
<213>   HERV-K

<400>   399
gaagggaaag acctgactgt cccct                                             25

<210>   400
<211>   25
<212>   DNA
<213>   HERV-K

<400>   400
ggcccgacac ccgtaaaggg tctgt                                             25

<210>   401
<211>   25
<212>   DNA
<213>   HERV-K

<400>   401
gacacccgta aagggtctgt gctga                                             25

<210>   402
<211>   25
<212>   DNA
<213>   HERV-K

<400>   402
ccgtaaaggg tctgtgctga ggatt                                             25

<210>   403
<211>   25
<212>   DNA
<213>   HERV-K

<400>   403
gctgaggatt agtaaaagag gaagg                                             25

<210>   404
<211>   25
<212>   DNA
<213>   HERV-K

<400>   404
ggattagtaa aagaggaagg aaggc                                          25

<210>   405
<211>   25
<212>   DNA
<213>   HERV-K

<400>   405
agtaaaagag gaaggaaggc ctctt                                          25

<210>   406
<211>   25
<212>   DNA
<213>   HERV-K

<400>   406
aaggcctctt tgcagttgag ataag                                          25

<210>   407
<211>   25
<212>   DNA
<213>   HERV-K

<400>   407
ctctttgcag ttgagataag aggaa                                          25

<210>   408
<211>   25
<212>   DNA
<213>   HERV-K

<400>   408
tgcagttgag ataagaggaa ggcat                                          25

<210>   409
<211>   25
<212>   DNA
<213>   HERV-K

<400>   409
ttgagataag aggaaggcat ctgtc                                          25

<210>   410
<211>   25
<212>   DNA
<213>   HERV-K

<400>   410
ataagaggaa ggcatctgtc tcctg                                          25

<210>   411
<211>   25
<212>   DNA
<213>   HERV-K

<400>   411
aggaaggcat ctgtctcctg ctcat                                          25

<210>   412

<211>   25
<212>   DNA
<213>   HERV-K

<400>   412
ggcatctgtc tcctgctcat ccctg                                             25

<210>   413
<211>   25
<212>   DNA
<213>   HERV-K

<400>   413
ctgtctcctg ctcatccctg ggcaa                                             25

<210>   414
<211>   25
<212>   DNA
<213>   HERV-K

<400>   414
tcctgctcat ccctgggcaa tggaa                                             25

<210>   415
<211>   25
<212>   DNA
<213>   HERV-K

<400>   415
ctcatccctg ggcaatggaa tgtct                                             25

<210>   416
<211>   25
<212>   DNA
<213>   HERV-K

<400>   416
ttgtatatgc catctactga gatag                                             25

<210>   417
<211>   25
<212>   DNA
<213>   HERV-K

<400>   417
cgtctacagt gagccttgtg ggtga                                             25

<210>   418
<211>   25
<212>   DNA
<213>   HERV-K

<400>   418
acagtgagcc ttgtgggtga aggta                                             25

<210>   419
<211>   25
<212>   DNA
<213>   HERV-K

<400>   419

gagccttgtg ggtgaaggta ctcta 25

<210> 420
<211> 25
<212> DNA
<213> HERV-K

<400> 420
ttgtgggtga aggtactcta cagtg 25

<210> 421
<211> 25
<212> DNA
<213> HERV-K

<400> 421
gcccaagaaa gcctggctgt tgtgg 25

<210> 422
<211> 25
<212> DNA
<213> HERV-K

<400> 422
agaaagcctg gctgttgtgg gaagt 25

<210> 423
<211> 25
<212> DNA
<213> HERV-K

<400> 423
gcctggctgt tgtgggaagt caggg 25

<210> 424
<211> 25
<212> DNA
<213> HERV-K

<400> 424
gctgttgtgg gaagtcaggg acccc 25

<210> 425
<211> 25
<212> DNA
<213> HERV-K

<400> 425
tgtgggaagt cagggacccc gaatg 25

<210> 426
<211> 25
<212> DNA
<213> HERV-K

<400> 426
gaagtcaggg accccgaatg gaggg 25

<210> 427
<211> 25
<212> DNA

<213>   HERV-K

<400>   427
cagggacccc gaatggaggg accag                                          25

<210>   428
<211>   25
<212>   DNA
<213>   HERV-K

<400>   428
accccgaatg gagggaccag ctggt                                          25

<210>   429
<211>   25
<212>   DNA
<213>   HERV-K

<400>   429
gaatggaggg accagctggt gctgc                                          25

<210>   430
<211>   25
<212>   DNA
<213>   HERV-K

<400>   430
gagggaccag ctggtgctgc atcag                                          25

<210>   431
<211>   25
<212>   DNA
<213>   HERV-K

<400>   431
accagctggt gctgcatcag gaaac                                          25

<210>   432
<211>   25
<212>   DNA
<213>   HERV-K

<400>   432
ctggtgctgc atcaggaaac ataaa                                          25

<210>   433
<211>   25
<212>   DNA
<213>   HERV-K

<400>   433
gctgcatcag gaaacataaa ttgtg                                          25

<210>   434
<211>   25
<212>   DNA
<213>   HERV-K

<400>   434
atcaggaaac ataaattgtg aagat                                          25

<210>   435
<211>   25
<212>   DNA
<213>   HERV-K

<400>   435
gaaacataaa ttgtgaagat ttctt                                             25

<210>   436
<211>   25
<212>   DNA
<213>   HERV-K

<400>   436
ataaattgtg aagatttctt ggaca                                             25

<210>   437
<211>   25
<212>   DNA
<213>   HERV-K

<400>   437
ttgtgaagat ttcttggaca tttat                                             25

<210>   438
<211>   25
<212>   DNA
<213>   HERV-K

<400>   438
aagatttctt ggacatttat cagtt                                             25

<210>   439
<211>   25
<212>   DNA
<213>   HERV-K

<400>   439
ttcttggaca tttatcagtt tccaa                                             25

<210>   440
<211>   25
<212>   DNA
<213>   HERV-K

<400>   440
ggacatttat cagtttccaa aatta                                             25

<210>   441
<211>   25
<212>   DNA
<213>   HERV-K

<400>   441
tttatcagtt tccaaaatta atact                                             25

<210>   442
<211>   25
<212>   DNA
<213>   HERV-K

<400>   442
cagtttccaa aattaatact tttat                                           25

<210>   443
<211>   25
<212>   DNA
<213>   HERV-K

<400>   443
tccaaaatta atacttttat aattt                                           25

<210>   444
<211>   25
<212>   DNA
<213>   HERV-K

<400>   444
aattaatact tttataattt cttac                                           25

<210>   445
<211>   25
<212>   DNA
<213>   HERV-K

<400>   445
atacttttat aatttcttac acctg                                           25

<210>   446
<211>   25
<212>   DNA
<213>   HERV-K

<400>   446
tttataattt cttacacctg tctta                                           25

<210>   447
<211>   25
<212>   DNA
<213>   HERV-K

<400>   447
aatttcttac acctgtctta cttta                                           25

<210>   448
<211>   25
<212>   DNA
<213>   HERV-K

<400>   448
cttacacctg tcttacttta atctc                                           25

<210>   449
<211>   25
<212>   DNA
<213>   HERV-K

<400>   449
acctgtctta ctttaatctc ttaat                                           25

<210>   450
<211>   25

<212>   DNA
<213>   HERV-K

<400>   450
tcttacttta atctcttaat cctgt                                          25

<210>   451
<211>   25
<212>   DNA
<213>   HERV-K

<400>   451
ctttaatctc ttaatcctgt tatct                                          25

<210>   452
<211>   25
<212>   DNA
<213>   HERV-K

<400>   452
atctcttaat cctgttatct ttgta                                          25

<210>   453
<211>   25
<212>   DNA
<213>   HERV-K

<400>   453
ttaatcctgt tatctttgta agctg                                          25

<210>   454
<211>   25
<212>   DNA
<213>   HERV-K

<400>   454
cctgttatct ttgtaagctg aggat                                          25

<210>   455
<211>   25
<212>   DNA
<213>   HERV-K

<400>   455
tatctttgta agctgaggat atacg                                          25

<210>   456
<211>   25
<212>   DNA
<213>   HERV-K

<400>   456
ttgtaagctg aggatatacg tcacc                                          25

<210>   457
<211>   25
<212>   DNA
<213>   HERV-K

<400>   457
agctgaggat atacgtcacc tcagg                                          25

<210>   458
<211>   25
<212>   DNA
<213>   HERV-K

<400>   458
aggatatacg tcacctcagg accac                                          25

<210>   459
<211>   25
<212>   DNA
<213>   HERV-K

<400>   459
atacgtcacc tcaggaccac tattg                                          25

<210>   460
<211>   25
<212>   DNA
<213>   HERV-K

<400>   460
tcacctcagg accactattg tacaa                                          25

<210>   461
<211>   25
<212>   DNA
<213>   HERV-K

<400>   461
tcaggaccac tattgtacaa attga                                          25

<210>   462
<211>   25
<212>   DNA
<213>   HERV-K

<400>   462
accactattg tacaaattga ttgta                                          25

<210>   463
<211>   25
<212>   DNA
<213>   HERV-K

<400>   463
tattgtacaa attgattgta aaaca                                          25

<210>   464
<211>   25
<212>   DNA
<213>   HERV-K

<400>   464
tacaaattga ttgtaaaaca tgttc                                          25

<210>   465
<211>   25
<212>   DNA
<213>   HERV-K

<400>   465
attgattgta aaacatgttc acatg                                          25

<210>   466
<211>   25
<212>   DNA
<213>   HERV-K

<400>   466
ttgtaaaaca tgttcacatg tgttt                                          25

<210>   467
<211>   25
<212>   DNA
<213>   HERV-K

<400>   467
aaacatgttc acatgtgttt gaaca                                          25

<210>   468
<211>   25
<212>   DNA
<213>   HERV-K

<400>   468
tgttcacatg tgtttgaaca atatg                                          25

<210>   469
<211>   25
<212>   DNA
<213>   HERV-K

<400>   469
acatgtgttt gaacaatatg aaatc                                          25

<210>   470
<211>   25
<212>   DNA
<213>   HERV-K

<400>   470
tgtttgaaca atatgaaatc agtgc                                          25

<210>   471
<211>   25
<212>   DNA
<213>   HERV-K

<400>   471
gaacaatatg aaatcagtgc acctt                                          25

<210>   472
<211>   25
<212>   DNA
<213>   HERV-K

<400>   472
atatgaaatc agtgcacctt gaaaa                                          25

<210>   473

<211>   25
<212>   DNA
<213>   HERV-K

<400>   473
aaatcagtgc accttgaaaa tgaac                                          25

<210>   474
<211>   25
<212>   DNA
<213>   HERV-K

<400>   474
agtgcacctt gaaaatgaac agaat                                          25

<210>   475
<211>   25
<212>   DNA
<213>   HERV-K

<400>   475
accttgaaaa tgaacagaat aacag                                          25

<210>   476
<211>   25
<212>   DNA
<213>   HERV-K

<400>   476
gaaaatgaac agaataacag tgatt                                          25

<210>   477
<211>   25
<212>   DNA
<213>   HERV-K

<400>   477
tgaacagaat aacagtgatt ttagg                                          25

<210>   478
<211>   25
<212>   DNA
<213>   HERV-K

<400>   478
agaataacag tgattttagg gaaca                                          25

<210>   479
<211>   25
<212>   DNA
<213>   HERV-K

<400>   479
aacagtgatt ttagggaaca aagga                                          25

<210>   480
<211>   25
<212>   DNA
<213>   HERV-K

<400>   480
   tgattttagg gaacaaagga agaca                                          25

<210>  481
<211>  25
<212>  DNA
<213>  HERV-K

<400>  481
ttagggaaca aaggaagaca accat                                          25

<210>  482
<211>  25
<212>  DNA
<213>  HERV-K

<400>  482
gaacaaagga agacaaccat aaggt                                          25

<210>  483
<211>  25
<212>  DNA
<213>  HERV-K

<400>  483
aaggaagaca accataaggt ctgac                                          25

<210>  484
<211>  25
<212>  DNA
<213>  HERV-K

<400>  484
agacaaccat aaggtctgac tgcct                                          25

<210>  485
<211>  25
<212>  DNA
<213>  HERV-K

<400>  485
accataaggt ctgactgcct gaggg                                          25

<210>  486
<211>  25
<212>  DNA
<213>  HERV-K

<400>  486
aaggtctgac tgcctgaggg gtcgg                                          25

<210>  487
<211>  25
<212>  DNA
<213>  HERV-K

<400>  487
ctgactgcct gaggggtcgg gcaaa                                          25

<210>  488
<211>  25
<212>  DNA

<213>   HERV-K

<400>   488
tgcctgaggg gtcgggcaaa aagcc                                             25

<210>   489
<211>   25
<212>   DNA
<213>   HERV-K

<400>   489
gaggggtcgg gcaaaaagcc atatt                                             25

<210>   490
<211>   25
<212>   DNA
<213>   HERV-K

<400>   490
gtcgggcaaa aagccatatt tttct                                             25

<210>   491
<211>   25
<212>   DNA
<213>   HERV-K

<400>   491
gcaaaaagcc atatttttct tcttg                                             25

<210>   492
<211>   25
<212>   DNA
<213>   HERV-K

<400>   492
aagccatatt tttcttcttg cagag                                             25

<210>   493
<211>   25
<212>   DNA
<213>   HERV-K

<400>   493
atatttttct tcttgcagag agcct                                             25

<210>   494
<211>   25
<212>   DNA
<213>   HERV-K

<400>   494
tttcttcttg cagagagcct ataaa                                             25

<210>   495
<211>   25
<212>   DNA
<213>   HERV-K

<400>   495
tcttgcagag agcctataaa tggac                                             25

<210>   496
<211>   25
<212>   DNA
<213>   HERV-K

<400>   496
cagagagcct ataaatggac gtgca                                          25

<210>   497
<211>   25
<212>   DNA
<213>   HERV-K

<400>   497
agcctataaa tggacgtgca agtag                                          25

<210>   498
<211>   25
<212>   DNA
<213>   HERV-K

<400>   498
ataaatggac gtgcaagtag gagag                                          25

<210>   499
<211>   25
<212>   DNA
<213>   HERV-K

<400>   499
tggacgtgca agtaggagag atatt                                          25

<210>   500
<211>   25
<212>   DNA
<213>   HERV-K

<400>   500
gtgcaagtag gagagatatt gctaa                                          25

<210>   501
<211>   25
<212>   DNA
<213>   HERV-K

<400>   501
agtaggagag atattgctaa attct                                          25

<210>   502
<211>   25
<212>   DNA
<213>   HERV-K

<400>   502
gagagatatt gctaaattct tttcc                                          25

<210>   503
<211>   25
<212>   DNA
<213>   HERV-K

<400>   503
atattgctaa attcttttcc tagca                                          25

<210>   504
<211>   25
<212>   DNA
<213>   HERV-K

<400>   504
gctaaattct tttcctagca aggaa                                          25

<210>   505
<211>   25
<212>   DNA
<213>   HERV-K

<400>   505
attcttttcc tagcaaggaa tataa                                          25

<210>   506
<211>   25
<212>   DNA
<213>   HERV-K

<400>   506
tttcctagca aggaatataa tacta                                          25

<210>   507
<211>   25
<212>   DNA
<213>   HERV-K

<400>   507
tagcaaggaa tataatacta agacc                                          25

<210>   508
<211>   25
<212>   DNA
<213>   HERV-K

<400>   508
aggaatataa tactaagacc ctagg                                          25

<210>   509
<211>   25
<212>   DNA
<213>   HERV-K

<400>   509
tataatacta agaccctagg gaaag                                          25

<210>   510
<211>   25
<212>   DNA
<213>   HERV-K

<400>   510
tactaagacc ctagggaaag aattg                                          25

<210>   511
<211>   25

<212>   DNA
<213>   HERV-K

<400>   511
agaccctagg gaaagaattg cattc                                          25

<210>   512
<211>   25
<212>   DNA
<213>   HERV-K

<400>   512
ctagggaaag aattgcattc ctggg                                          25

<210>   513
<211>   25
<212>   DNA
<213>   HERV-K

<400>   513
gaaagaattg cattcctggg gggag                                          25

<210>   514
<211>   25
<212>   DNA
<213>   HERV-K

<400>   514
   aattgcattc ctggggggag gtcta                                           25

<210>   515
<211>   25
<212>   DNA
<213>   HERV-K

<400>   515
cattcctggg gggaggtcta taaac                                          25

<210>   516
<211>   25
<212>   DNA
<213>   HERV-K

<400>   516
   ctggggggag gtctataaac ggccg                                           25

<210>   517
<211>   25
<212>   DNA
<213>   HERV-K

<400>   517
gggaggtcta taaacggccg ctctg                                          25

<210>   518
<211>   25
<212>   DNA
<213>   HERV-K

<400>   518
gtctataaac ggccgctctg ggagt                                          25

<210>   519
<211>   25
<212>   DNA
<213>   HERV-K

<400>   519
taaacggccg ctctgggagt gtctg                                             25

<210>   520
<211>   25
<212>   DNA
<213>   HERV-K

<400>   520
ggccgctctg ggagtgtctg tccta                                             25

<210>   521
<211>   25
<212>   DNA
<213>   HERV-K

<400>   521
ctctgggagt gtctgtccta tgtgg                                             25

<210>   522
<211>   25
<212>   DNA
<213>   HERV-K

<400>   522
ggagtgtctg tcctatgtgg ttgag                                             25

<210>   523
<211>   25
<212>   DNA
<213>   HERV-K

<400>   523
gtctgtccta tgtggttgag ataag                                             25

<210>   524
<211>   25
<212>   DNA
<213>   HERV-K

<400>   524
tcctatgtgg ttgagataag gactg                                             25

<210>   525
<211>   25
<212>   DNA
<213>   HERV-K

<400>   525
tgtggttgag ataaggactg agata                                             25

<210>   526
<211>   25
<212>   DNA
<213>   HERV-K

<400>   526
ttgagataag gactgagata cgccc                                          25

<210>   527
<211>   25
<212>   DNA
<213>   HERV-K

<400>   527
ataaggactg agatacgccc tggtc                                          25

<210>   528
<211>   25
<212>   DNA
<213>   HERV-K

<400>   528
gactgagata cgccctggtc tcctg                                          25

<210>   529
<211>   25
<212>   DNA
<213>   HERV-K

<400>   529
agatacgccc tggtctcctg cagta                                          25

<210>   530
<211>   25
<212>   DNA
<213>   HERV-K

<400>   530
cgccctggtc tcctgcagta ccctc                                          25

<210>   531
<211>   25
<212>   DNA
<213>   HERV-K

<400>   531
tggtctcctg cagtaccctc aggct                                          25

<210>   532
<211>   25
<212>   DNA
<213>   HERV-K

<400>   532
tcctgcagta ccctcaggct tacta                                          25

<210>   533
<211>   25
<212>   DNA
<213>   HERV-K

<400>   533
cagtaccctc aggcttacta ggatt                                          25

<210>   534

<211>   25
<212>   DNA
<213>   HERV-K

<400>   534
ccctcaggct tactaggatt gggaa                                          25

<210>   535
<211>   25
<212>   DNA
<213>   HERV-K

<400>   535
aggcttacta ggattgggaa acccc                                          25

<210>   536
<211>   25
<212>   DNA
<213>   HERV-K

<400>   536
tactaggatt gggaaacccc agtcc                                          25

<210>   537
<211>   25
<212>   DNA
<213>   HERV-K

<400>   537
ggattgggaa accccagtcc tggta                                          25

<210>   538
<211>   25
<212>   DNA
<213>   HERV-K

<400>   538
gggaaacccc agtcctggta aattt                                          25

<210>   539
<211>   25
<212>   DNA
<213>   HERV-K

<400>   539
accccagtcc tggtaaattt gaggt                                          25

<210>   540
<211>   25
<212>   DNA
<213>   HERV-K

<400>   540
agtcctggta aatttgaggt caggc                                          25

<210>   541
<211>   25
<212>   DNA
<213>   HERV-K

<400>   541

tggtaaattt gaggtcaggc cggtt                                          25

<210>  542
<211>  25
<212>  DNA
<213>  HERV-K

<400>  542
aatttgaggt caggccggtt ctttg                                          25

<210>  543
<211>  25
<212>  DNA
<213>  HERV-K

<400>  543
gaggtcaggc cggttctttg ctctg                                          25

<210>  544
<211>  25
<212>  DNA
<213>  HERV-K

<400>  544
caggccggtt ctttgctctg aaccc                                          25

<210>  545
<211>  25
<212>  DNA
<213>  HERV-K

<400>  545
cggttctttg ctctgaaccc tgttt                                          25

<210>  546
<211>  25
<212>  DNA
<213>  HERV-K

<400>  546
ctttgctctg aaccctgttt tctgt                                          25

<210>  547
<211>  25
<212>  DNA
<213>  HERV-K

<400>  547
ctctgaaccc tgttttctgt taaga                                          25

<210>  548
<211>  25
<212>  DNA
<213>  HERV-K

<400>  548
aaccctgttt tctgttaaga tgttt                                          25

<210>  549
<211>  25
<212>  DNA

<213>   HERV-K

<400>   549
tgttttctgt taagatgttt atcaa                                             25

<210>   550
<211>   25
<212>   DNA
<213>   HERV-K

<400>   550
tctgttaaga tgtttatcaa gacaa                                             25

<210>   551
<211>   25
<212>   DNA
<213>   HERV-K

<400>   551
taagatgttt atcaagacaa tacat                                             25

<210>   552
<211>   25
<212>   DNA
<213>   HERV-K

<400>   552
tgtttatcaa gacaatacat gcacc                                             25

<210>   553
<211>   25
<212>   DNA
<213>   HERV-K

<400>   553
atcaagacaa tacatgcacc gctga                                             25

<210>   554
<211>   25
<212>   DNA
<213>   HERV-K

<400>   554
gacaatacat gcaccgctga acata                                             25

<210>   555
<211>   25
<212>   DNA
<213>   HERV-K

<400>   555
tacatgcacc gctgaacata gaccc                                             25

<210>   556
<211>   25
<212>   DNA
<213>   HERV-K

<400>   556
gcaccgctga acatagaccc ttatc                                             25

<210>   557
<211>   25
<212>   DNA
<213>   HERV-K

<400>   557
gctgaacata gacccttatc aggag                                          25

<210>   558
<211>   25
<212>   DNA
<213>   HERV-K

<400>   558
acatagaccc ttatcaggag tttct                                          25

<210>   559
<211>   25
<212>   DNA
<213>   HERV-K

<400>   559
gacccttatc aggagtttct gattt                                          25

<210>   560
<211>   25
<212>   DNA
<213>   HERV-K

<400>   560
ttatcaggag tttctgattt tgctc                                          25

<210>   561
<211>   25
<212>   DNA
<213>   HERV-K

<400>   561
aggagtttct gattttgctc tggtc                                          25

<210>   562
<211>   25
<212>   DNA
<213>   HERV-K

<400>   562
tttctgattt tgctctggtc ctgtt                                          25

<210>   563
<211>   25
<212>   DNA
<213>   HERV-K

<400>   563
gattttgctc tggtcctgtt tcttc                                          25

<210>   564
<211>   25
<212>   DNA
<213>   HERV-K

<400>   564
tgctctggtc ctgtttcttc agaag                                          25

<210>   565
<211>   25
<212>   DNA
<213>   HERV-K

<400>   565
tggtcctgtt tcttcagaag catgt                                          25

<210>   566
<211>   25
<212>   DNA
<213>   HERV-K

<400>   566
ctgtttcttc agaagcatgt catct                                          25

<210>   567
<211>   25
<212>   DNA
<213>   HERV-K

<400>   567
tcttcagaag catgtcatct ttgct                                          25

<210>   568
<211>   25
<212>   DNA
<213>   HERV-K

<400>   568
agaagcatgt catctttgct ctgcc                                          25

<210>   569
<211>   25
<212>   DNA
<213>   HERV-K

<400>   569
catgtcatct ttgctctgcc ttctg                                          25

<210>   570
<211>   25
<212>   DNA
<213>   HERV-K

<400>   570
catctttgct ctgccttctg ccctt                                          25

<210>   571
<211>   25
<212>   DNA
<213>   HERV-K

<400>   571
ttgctctgcc ttctgccctt tgaag                                          25

<210>   572
<211>   25

<212>   DNA
<213>   HERV-K

<400>   572
ctgccttctg ccctttgaag catgt                                             25

<210>   573
<211>   25
<212>   DNA
<213>   HERV-K

<400>   573
ttctgccctt tgaagcatgt gatct                                             25

<210>   574
<211>   25
<212>   DNA
<213>   HERV-K

<400>   574
ccctttgaag catgtgatct ttgtg                                             25

<210>   575
<211>   25
<212>   DNA
<213>   HERV-K

<400>   575
tgaagcatgt gatctttgtg accta                                             25

<210>   576
<211>   25
<212>   DNA
<213>   HERV-K

<400>   576
catgtgatct ttgtgaccta ctccc                                             25

<210>   577
<211>   25
<212>   DNA
<213>   HERV-K

<400>   577
gatctttgtg acctactccc tgttc                                             25

<210>   578
<211>   25
<212>   DNA
<213>   HERV-K

<400>   578
ttgtgaccta ctccctgttc ataca                                             25

<210>   579
<211>   25
<212>   DNA
<213>   HERV-K

<400>   579
acctactccc tgttcataca cccct                                             25

<210>   580
<211>   25
<212>   DNA
<213>   HERV-K

<400>   580
ctccctgttc atacacccct cccct                                          25

<210>   581
<211>   25
<212>   DNA
<213>   HERV-K

<400>   581
tgttcataca cccctcccct tttaa                                          25

<210>   582
<211>   25
<212>   DNA
<213>   HERV-K

<400>   582
atacacccct ccccttttaa aatcc                                          25

<210>   583
<211>   25
<212>   DNA
<213>   HERV-K

<400>   583
cccctcccct tttaaaatcc ctaat                                          25

<210>   584
<211>   25
<212>   DNA
<213>   HERV-K

<400>   584
ccccttttaa aatccctaat aaaaa                                          25

<210>   585
<211>   25
<212>   DNA
<213>   HERV-K

<400>   585
tttaaaatcc ctaataaaaa cttgc                                          25

<210>   586
<211>   25
<212>   DNA
<213>   HERV-K

<400>   586
aatccctaat aaaaacttgc tggtt                                          25

<210>   587
<211>   25
<212>   DNA
<213>   HERV-K

<400>   587
ctaataaaaa cttgctggtt ttgtg                                          25

<210>   588
<211>   25
<212>   DNA
<213>   HERV-K

<400>   588
aaaaacttgc tggttttgtg gctca                                          25

<210>   589
<211>   25
<212>   DNA
<213>   HERV-K

<400>   589
cttgctggtt ttgtggctca ggggg                                          25

<210>   590
<211>   25
<212>   DNA
<213>   HERV-K

<400>   590
tggttttgtg gctcaggggg gcatc                                          25

<210>   591
<211>   25
<212>   DNA
<213>   HERV-K

<400>   591
ttgtggctca gggggcatc atgga                                           25

<210>   592
<211>   25
<212>   DNA
<213>   HERV-K

<400>   592
gctcaggggg gcatcatgga cctac                                          25

<210>   593
<211>   25
<212>   DNA
<213>   HERV-K

<400>   593
gggggcatc atggacctac caata                                           25

<210>   594
<211>   25
<212>   DNA
<213>   HERV-K

<400>   594
gcatcatgga cctaccaata cgtga                                          25

<210>   595

<211>   25
<212>   DNA
<213>   HERV-K

<400>   595
atggacctac caatacgtga tgtca                                          25

<210>   596
<211>   25
<212>   DNA
<213>   HERV-K

<400>   596
cctaccaata cgtgatgtca ccccc                                          25

<210>   597
<211>   25
<212>   DNA
<213>   HERV-K

<400>   597
caatacgtga tgtcaccccc ggtgg                                          25

<210>   598
<211>   25
<212>   DNA
<213>   HERV-K

<400>   598
cgtgatgtca cccccggtgg cccag                                          25

<210>   599
<211>   25
<212>   DNA
<213>   HERV-K

<400>   599
tgtcaccccc ggtggcccag ctgta                                          25

<210>   600
<211>   25
<212>   DNA
<213>   HERV-K

<400>   600
tgtgctcaca gaaacaggtg ctgtg                                          25

<210>   601
<211>   25
<212>   DNA
<213>   HERV-K

<400>   601
tcacagaaac aggtgctgtg ttgac                                          25

<210>   602
<211>   25
<212>   DNA
<213>   HERV-K

<400>   602

tcaaggttta atggattcag ggctg 25

<210> 603
<211> 25
<212> DNA
<213> HERV-K

<400> 603
gtttaatgga ttcagggctg tgcag 25

<210> 604
<211> 25
<212> DNA
<213> HERV-K

<400> 604
acaaatgctt gaaggcagca agctt 25

<210> 605
<211> 25
<212> DNA
<213> HERV-K

<400> 605
tgcttgaagg cagcaagctt gttaa 25

<210> 606
<211> 25
<212> DNA
<213> HERV-K

<400> 606
gaaggcagca agcttgttaa gagtc 25

<210> 607
<211> 25
<212> DNA
<213> HERV-K

<400> 607
cagcaagctt gttaagagtc atcac 25

<210> 608
<211> 25
<212> DNA
<213> HERV-K

<400> 608
agcttgttaa gagtcatcac cactc 25

<210> 609
<211> 25
<212> DNA
<213> HERV-K

<400> 609
cactccctaa tctcaagtaa gcagg 25

<210> 610
<211> 25
<212> DNA

<213>   HERV-K

<400>   610
cctaatctca agtaagcagg gacac                                          25

<210>   611
<211>   25
<212>   DNA
<213>   HERV-K

<400>   611
tctcaagtaa gcagggacac aaaca                                          25

<210>   612
<211>   25
<212>   DNA
<213>   HERV-K

<400>   612
agtaagcagg gacacaaaca ctgcg                                          25

<210>   613
<211>   25
<212>   DNA
<213>   HERV-K

<400>   613
gacagtctga aatatggcct cttgg                                          25

<210>   614
<211>   25
<212>   DNA
<213>   HERV-K

<400>   614
gaaagacctg actgtcccct ggccc                                          25

<210>   615
<211>   25
<212>   DNA
<213>   HERV-K

<400>   615
acctgactgt cccctggccc gacac                                          25

<210>   616
<211>   25
<212>   DNA
<213>   HERV-K

<400>   616
actgtcccct ggcccgacac ccgta                                          25

<210>   617
<211>   25
<212>   DNA
<213>   HERV-K

<400>   617
cccctggccc gacacccgta aaggg                                          25

<210>   618
<211>   25
<212>   DNA
<213>   HERV-K

<400>   618
aagggtctgt gctgaggatt agtaa                                          25

<210>   619
<211>   25
<212>   DNA
<213>   HERV-K

<400>   619
tctgtgctga ggattagtaa aagag                                          25

<210>   620
<211>   25
<212>   DNA
<213>   HERV-K

<400>   620
aagaggaagg aaggcctctt tgcag                                          25

<210>   621
<211>   25
<212>   DNA
<213>   HERV-K

<400>   621
gaaggaaggc ctctttgcag ttgag                                          25

<210>   622
<211>   25
<212>   DNA
<213>   HERV-K

<400>   622
ccctgggcaa tggaatgtct tggtg                                          25

<210>   623
<211>   25
<212>   DNA
<213>   HERV-K

<400>   623
ggcaatggaa tgtcttggtg taaag                                          25

<210>   624
<211>   25
<212>   DNA
<213>   HERV-K

<400>   624
tggaatgtct tggtgtaaag cctga                                          25

<210>   625
<211>   25
<212>   DNA
<213>   HERV-K

<400>   625
tgtcttggtg taaagcctga ttgta                                          25

<210>   626
<211>   25
<212>   DNA
<213>   HERV-K

<400>   626
tggtgtaaag cctgattgta tatgc                                          25

<210>   627
<211>   25
<212>   DNA
<213>   HERV-K

<400>   627
taaagcctga ttgtatatgc catct                                          25

<210>   628
<211>   25
<212>   DNA
<213>   HERV-K

<400>   628
cctgattgta tatgccatct actga                                          25

<210>   629
<211>   25
<212>   DNA
<213>   HERV-K

<400>   629
tatgccatct actgagatag gagaa                                          25

<210>   630
<211>   25
<212>   DNA
<213>   HERV-K

<400>   630
catctactga gataggagaa aactg                                          25

<210>   631
<211>   25
<212>   DNA
<213>   HERV-K

<400>   631
gggctggagg tgggacatgc tggcg                                          25

<210>   632
<211>   25
<212>   DNA
<213>   HERV-K

<400>   632
ggaggtggga catgctggcg gcaat                                          25

<210>   633
<211>   25

<212>   DNA
<213>   HERV-K

<400>   633
tgggacatgc tggcggcaat actgc                                          25

<210>   634
<211>   25
<212>   DNA
<213>   HERV-K

<400>   634
catgctggcg gcaatactgc tcttt                                          25

<210>   635
<211>   25
<212>   DNA
<213>   HERV-K

<400>   635
tggcggcaat actgctcttt aaggc                                          25

<210>   636
<211>   25
<212>   DNA
<213>   HERV-K

<400>   636
gcaatactgc tctttaaggc attga                                          25

<210>   637
<211>   25
<212>   DNA
<213>   HERV-K

<400>   637
ttatgtatat gcacatcaaa agcac                                          25

<210>   638
<211>   25
<212>   DNA
<213>   HERV-K

<400>   638
tatatgcaca tcaaaagcac agcac                                          25

<210>   639
<211>   25
<212>   DNA
<213>   HERV-K

<400>   639
gcacatcaaa agcacagcac ttttt                                          25

<210>   640
<211>   25
<212>   DNA
<213>   HERV-K

<400>   640
tcaaaagcac agcacttttt tcttt                                          25

<210>   641
<211>   25
<212>   DNA
<213>   HERV-K

<400>   641
agcacagcac ttttttcttt acctt                                          25

<210>   642
<211>   25
<212>   DNA
<213>   HERV-K

<400>   642
agcacttttt tctttacctt gttta                                          25

<210>   643
<211>   25
<212>   DNA
<213>   HERV-K

<400>   643
accttgttta tgatgcagag acatt                                          25

<210>   644
<211>   25
<212>   DNA
<213>   HERV-K

<400>   644
gtttatgatg cagagacatt tgttc                                          25

<210>   645
<211>   25
<212>   DNA
<213>   HERV-K

<400>   645
tgatgcagag acatttgttc acatg                                          25

<210>   646
<211>   25
<212>   DNA
<213>   HERV-K

<400>   646
actcagagac cggtgcggcg cgggt                                          25

<210>   647
<211>   25
<212>   DNA
<213>   HERV-K

<400>   647
gagaccggtg cggcgcgggt cctcc                                          25

<210>   648
<211>   25
<212>   DNA
<213>   HERV-K

<400>   648
cggtgcggcg cgggtcctcc atatg                                          25

<210>   649
<211>   25
<212>   DNA
<213>   HERV-K

<400>   649
ctttgtctct gttgtctttc ttttc                                          25

<210>   650
<211>   25
<212>   DNA
<213>   HERV-K

<400>   650
tctctgttgt ctttcttttc tcaag                                          25

<210>   651
<211>   25
<212>   DNA
<213>   HERV-K

<400>   651
tcaagtctct cgttccacct gagga                                          25

<210>   652
<211>   25
<212>   DNA
<213>   HERV-K

<400>   652
tctctcgttc cacctgagga gaaat                                          25

<210>   653
<211>   25
<212>   DNA
<213>   HERV-K

<400>   653
cgttccacct gaggagaaat gccca                                          25

<210>   654
<211>   25
<212>   DNA
<213>   HERV-K

<400>   654
cacctgagga gaaatgccca cagct                                          25

<210>   655
<211>   25
<212>   DNA
<213>   HERV-K

<400>   655
gaggagaaat gcccacagct gtgga                                          25

<210>   656

<211>   25
<212>   DNA
<213>   HERV-K

<400>   656
gaaatgccca cagctgtgga ggcgc                                          25

<210>   657
<211>   25
<212>   DNA
<213>   HERV-K

<400>   657
gcccacagct gtggaggcgc aggcc                                          25

<210>   658
<211>   25
<212>   DNA
<213>   HERV-K

<400>   658
cagctgtgga ggcgcaggcc actcc                                          25

<210>   659
<211>   25
<212>   DNA
<213>   HERV-K

<400>   659
gtggaggcgc aggccactcc atctg                                          25

<210>   660
<211>   25
<212>   DNA
<213>   HERV-K

<400>   660
ggcgcaggcc actccatctg gtgcc                                          25

<210>   661
<211>   25
<212>   DNA
<213>   HERV-K

<400>   661
aggccactcc atctggtgcc caacg                                          25

<210>   662
<211>   25
<212>   DNA
<213>   HERV-K

<400>   662
actccatctg gtgcccaacg tggat                                          25

<210>   663
<211>   25
<212>   DNA
<213>   HERV-K

<400>   663

gcttttctct agggtgaagg gactc                                          25

<210>  664
<211>  25
<212>  DNA
<213>  HERV-K

<400>  664
tctctagggt gaagggactc tcgag                                          25

<210>  665
<211>  25
<212>  DNA
<213>  HERV-K

<400>  665
agggtgaagg gactctcgag tgtgg                                          25

<210>  666
<211>  25
<212>  DNA
<213>  HERV-K

<400>  666
gaagggactc tcgagtgtgg tcatt                                          25

<210>  667
<211>  25
<212>  DNA
<213>  HERV-K

<400>  667
gactctcgag tgtggtcatt gagga                                          25

<210>  668
<211>  25
<212>  DNA
<213>  HERV-K

<400>  668
tgtggtcatt gaggacaagt caacg                                          25

<210>  669
<211>  25
<212>  DNA
<213>  HERV-K

<400>  669
gagtacgtct acagtgagcc ttgtg                                          25

<210>  670
<211>  25
<212>  DNA
<213>  HERV-K

<400>  670
cgtctacagt gagccttgtg gtaag                                          25

<210>  671
<211>  25
<212>  DNA

<213>   HERV-K

<400>   671
acagtgagcc ttgtggtaag cttgg                                             25

<210>   672
<211>   25
<212>   DNA
<213>   HERV-K

<400>   672
gagccttgtg gtaagcttgg gcgct                                             25

<210>   673
<211>   25
<212>   DNA
<213>   HERV-K

<400>   673
ttgtggtaag cttgggcgct cggaa                                             25

<210>   674
<211>   25
<212>   DNA
<213>   HERV-K

<400>   674
cttgggcgct cggaagaagc caggg                                             25

<210>   675
<211>   25
<212>   DNA
<213>   HERV-K

<400>   675
gcgctcggaa gaagccaggg ttaat                                             25

<210>   676
<211>   25
<212>   DNA
<213>   HERV-K

<400>   676
cggaagaagc cagggttaat ggggc                                             25

<210>   677
<211>   25
<212>   DNA
<213>   HERV-K

<400>   677
gaagccaggg ttaatggggc aaact                                             25

<210>   678
<211>   25
<212>   DNA
<213>   HERV-K

<400>   678
cagggttaat ggggcaaact aaaag                                             25

<210>   679
<211>   25
<212>   DNA
<213>   HERV-K

<400>   679
ggggcaaact aaaagtaaag tctct                                          25

<210>   680
<211>   25
<212>   DNA
<213>   HERV-K

<400>   680
aaaagtaaag tctctcattc cacct                                          25

<210>   681
<211>   25
<212>   DNA
<213>   HERV-K

<400>   681
taaagtctct cattccacct gatga                                          25

<210>   682
<211>   25
<212>   DNA
<213>   HERV-K

<400>   682
ggggcaggcc accccttcag ggtag                                          25

<210>   683
<211>   25
<212>   DNA
<213>   HERV-K

<400>   683
aggccacccc ttcagggtag ggtcc                                          25

<210>   684
<211>   25
<212>   DNA
<213>   HERV-K

<400>   684
accccttcag ggtagggtcc cctcc                                          25

<210>   685
<211>   25
<212>   DNA
<213>   HERV-K

<400>   685
ttcagggtag ggtcccctcc atgca                                          25

<210>   686
<211>   25
<212>   DNA
<213>   HERV-K

<400>   686
ggtagggtcc cctccatgca gacca                                          25

<210>   687
<211>   25
<212>   DNA
<213>   HERV-K

<400>   687
ggtcccctcc atgcagacca tagag                                          25

<210>   688
<211>   25
<212>   DNA
<213>   HERV-K

<400>   688
cctccatgca gaccatagag cacag                                          25

<210>   689
<211>   25
<212>   DNA
<213>   HERV-K

<400>   689
atgcagacca tagagcacag gtgtg                                          25

<210>   690
<211>   25
<212>   DNA
<213>   HERV-K

<400>   690
gaccatagag cacaggtgtg cccca                                          25

<210>   691
<211>   25
<212>   DNA
<213>   HERV-K

<400>   691
tagagcacag gtgtgcccca aagag                                          25

<210>   692
<211>   25
<212>   DNA
<213>   HERV-K

<400>   692
cacaggtgtg ccccaaagag gagca                                          25

<210>   693
<211>   25
<212>   DNA
<213>   HERV-K

<400>   693
gtgtgcccca aagaggagca gagag                                          25

<210>   694
<211>   25

<212>   DNA
<213>   HERV-K

<400>   694
ccccaaagag gagcagagag aagga                                          25

<210>   695
<211>   25
<212>   DNA
<213>   HERV-K

<400>   695
aagaggagca gagagaagga gggag                                          25

<210>   696
<211>   25
<212>   DNA
<213>   HERV-K

<400>   696
gagcagagag aaggagggag agggc                                          25

<210>   697
<211>   25
<212>   DNA
<213>   HERV-K

<400>   697
gagagaagga gggagagggc ccacg                                          25

<210>   698
<211>   25
<212>   DNA
<213>   HERV-K

<400>   698
aaggagggag agggcccacg agaga                                          25

<210>   699
<211>   25
<212>   DNA
<213>   HERV-K

<400>   699
gggagagggc ccacgagaga cttgg                                          25

<210>   700
<211>   25
<212>   DNA
<213>   HERV-K

<400>   700
agggcccacg agagacttgg aaatg                                          25

<210>   701
<211>   25
<212>   DNA
<213>   HERV-K

<400>   701
ccacgagaga cttggaaatg aatgg                                          25

<210>   702
<211>   25
<212>   DNA
<213>   HERV-K

<400>   702
agagacttgg aaatgaatgg cagga                                             25

<210>   703
<211>   25
<212>   DNA
<213>   HERV-K

<400>   703
cttggaaatg aatggcagga tttta                                             25

<210>   704
<211>   25
<212>   DNA
<213>   HERV-K

<400>   704
aaatgaatgg caggatttta ggcgc                                             25

<210>   705
<211>   25
<212>   DNA
<213>   HERV-K

<400>   705
aatggcagga ttttaggcgc tggac                                             25

<210>   706
<211>   25
<212>   DNA
<213>   HERV-K

<400>   706
caggatttta ggcgctggac ttggg                                             25

<210>   707
<211>   25
<212>   DNA
<213>   HERV-K

<400>   707
ttttaggcgc tggacttggg ttcgg                                             25

<210>   708
<211>   25
<212>   DNA
<213>   HERV-K

<400>   708
ggcgctggac ttgggttcgg ggcac                                             25

<210>   709
<211>   25
<212>   DNA
<213>   HERV-K

<400>   709
tggacttggg ttcggggcac ctggc                                          25

<210>   710
<211>   25
<212>   DNA
<213>   HERV-K

<400>   710
ttgggttcgg ggcacctggc ctttc                                          25

<210>   711
<211>   25
<212>   DNA
<213>   HERV-K

<400>   711
ttcggggcac ctggcctttc cttgt                                          25

<210>   712
<211>   25
<212>   DNA
<213>   HERV-K

<400>   712
ggcacctggc ctttccttgt gtatt                                          25

<210>   713
<211>   25
<212>   DNA
<213>   HERV-K

<400>   713
ctggcctttc cttgtgtatt tctcc                                          25

<210>   714
<211>   25
<212>   DNA
<213>   HERV-K

<400>   714
ctttccttgt gtatttctcc tactg                                          25

<210>   715
<211>   25
<212>   DNA
<213>   HERV-K

<400>   715
cttgtgtatt tctcctactg tctgc                                          25

<210>   716
<211>   25
<212>   DNA
<213>   HERV-K

<400>   716
gtatttctcc tactgtctgc ctaac                                          25

<210>   717

<211>   25
<212>   DNA
<213>   HERV-K

<400>   717
tctcctactg tctgcctaac tattt                                             25

<210>   718
<211>   25
<212>   DNA
<213>   HERV-K

<400>   718
aatacaataa aagaaaacca gcccc                                             25

<210>   719
<211>   25
<212>   DNA
<213>   HERV-K

<400>   719
aataaaagaa aaccagcccc tggtt                                             25

<210>   720
<211>   25
<212>   DNA
<213>   HERV-K

<400>   720
aagaaaacca gcccctggtt cttgt                                             25

<210>   721
<211>   25
<212>   DNA
<213>   HERV-K

<400>   721
aaccagcccc tggttcttgt ggtgt                                             25

<210>   722
<211>   25
<212>   DNA
<213>   HERV-K

<400>   722
gcccctggtt cttgtggtgt ttcca                                             25

<210>   723
<211>   25
<212>   DNA
<213>   HERV-K

<400>   723
tggttcttgt ggtgtttcca ccctc                                             25

<210>   724
<211>   25
<212>   DNA
<213>   HERV-K

<400>   724

cttgtggtgt ttccaccctc ccggg                                          25

<210>   725
<211>   25
<212>   DNA
<213>   HERV-K

<400>   725
ggtgtttcca ccctcccggg tcccc                                          25

<210>   726
<211>   25
<212>   DNA
<213>   HERV-K

<400>   726
ttccaccctc ccgggtcccc gctgg                                          25

<210>   727
<211>   25
<212>   DNA
<213>   HERV-K

<400>   727
ccctcccggg tccccgctgg ctgcc                                          25

<210>   728
<211>   25
<212>   DNA
<213>   HERV-K

<400>   728
ccgggtcccc gctggctgcc tggct                                          25

<210>   729
<211>   25
<212>   DNA
<213>   HERV-K

<400>   729
tccccgctgg ctgcctggct tcctc                                          25

<210>   730
<211>   25
<212>   DNA
<213>   HERV-K

<400>   730
gctggctgcc tggcttcctc ccgca                                          25

<210>   731
<211>   25
<212>   DNA
<213>   HERV-K

<400>   731
ctgcctggct tcctcccgca gctcc                                          25

<210>   732
<211>   25
<212>   DNA

<213>   HERV-K

<400>   732
tggcttcctc ccgcagctcc tgctg                                          25

<210>   733
<211>   25
<212>   DNA
<213>   HERV-K

<400>   733
tcctcccgca gctcctgctg tgtgt                                          25

<210>   734
<211>   25
<212>   DNA
<213>   HERV-K

<400>   734
ccgcagctcc tgctgtgtgt gtatg                                          25

<210>   735
<211>   25
<212>   DNA
<213>   HERV-K

<400>   735
gctcctgctg tgtgtgtatg tgtgt                                          25

<210>   736
<211>   25
<212>   DNA
<213>   HERV-K

<400>   736
tgctgtgtgt gtatgtgtgt gtgtg                                          25

<210>   737
<211>   25
<212>   DNA
<213>   HERV-K

<400>   737
tgtgtgtatg tgtgtgtgtg tgcac                                          25

<210>   738
<211>   25
<212>   DNA
<213>   HERV-K

<400>   738
gtatgtgtgt gtgtgtgcac atctg                                          25

<210>   739
<211>   25
<212>   DNA
<213>   HERV-K

<400>   739
tgtgtgtgtg tgcacatctg tgggg                                          25

<210>   740
<211>   25
<212>   DNA
<213>   HERV-K

<400>   740
gtgtgtgcac atctgtgggg cgtat                                          25

<210>   741
<211>   25
<212>   DNA
<213>   HERV-K

<400>   741
tgcacatctg tggggcgtat gtgtg                                          25

<210>   742
<211>   25
<212>   DNA
<213>   HERV-K

<400>   742
atctgtgggg cgtatgtgtg ttcgt                                          25

<210>   743
<211>   25
<212>   DNA
<213>   HERV-K

<400>   743
tggggcgtat gtgtgttcgt ctttg                                          25

<210>   744
<211>   25
<212>   DNA
<213>   HERV-K

<400>   744
cgtatgtgtg ttcgtctttg taatt                                          25

<210>   745
<211>   25
<212>   DNA
<213>   HERV-K

<400>   745
gtgtgttcgt ctttgtaatt gaggc                                          25

<210>   746
<211>   25
<212>   DNA
<213>   HERV-K

<400>   746
ttcgtctttg taattgaggc tgcag                                          25

<210>   747
<211>   25
<212>   DNA
<213>   HERV-K

<400>   747
ctttgtaatt gaggctgcag agtgg                                          25

<210>   748
<211>   25
<212>   DNA
<213>   HERV-K

<400>   748
taattgaggc tgcagagtgg agaga                                          25

<210>   749
<211>   25
<212>   DNA
<213>   HERV-K

<400>   749
gaggctgcag agtggagaga gcagg                                          25

<210>   750
<211>   25
<212>   DNA
<213>   HERV-K

<400>   750
tgcagagtgg agagagcagg ggttt                                          25

<210>   751
<211>   25
<212>   DNA
<213>   HERV-K

<400>   751
agtggagaga gcaggggttt tctct                                          25

<210>   752
<211>   25
<212>   DNA
<213>   HERV-K

<400>   752
agagagcagg ggttttctct gggga                                          25

<210>   753
<211>   25
<212>   DNA
<213>   HERV-K

<400>   753
gcaggggttt tctctgggga cccag                                          25

<210>   754
<211>   25
<212>   DNA
<213>   HERV-K

<400>   754
ggttttctct ggggacccag agaga                                          25

<210>   755
<211>   25

<212>   DNA
<213>   HERV-K

<400>   755
tctctgggga cccagagaga aggag                                          25

<210>   756
<211>   25
<212>   DNA
<213>   HERV-K

<400>   756
ggggacccag agagaaggag gcgtt                                          25

<210>   757
<211>   25
<212>   DNA
<213>   HERV-K

<400>   757
cccagagaga aggaggcgtt ttcac                                          25

<210>   758
<211>   25
<212>   DNA
<213>   HERV-K

<400>   758
agagaaggag gcgttttcac cacag                                          25

<210>   759
<211>   25
<212>   DNA
<213>   HERV-K

<400>   759
aggaggcgtt ttcaccacag ccgaa                                          25

<210>   760
<211>   25
<212>   DNA
<213>   HERV-K

<400>   760
gcgttttcac cacagccgaa caggg                                          25

<210>   761
<211>   25
<212>   DNA
<213>   HERV-K

<400>   761
ttcaccacag ccgaacaggg cagga                                          25

<210>   762
<211>   25
<212>   DNA
<213>   HERV-K

<400>   762
cacagccgaa cagggcagga cccca                                          25

<210>   763
<211>   25
<212>   DNA
<213>   HERV-K

<400>   763
ccgaacaggg caggacccca gcacc                                             25

<210>   764
<211>   25
<212>   DNA
<213>   HERV-K

<400>   764
cagggcagga ccccagcacc cggga                                             25

<210>   765
<211>   25
<212>   DNA
<213>   HERV-K

<400>   765
caggacccca gcacccggga cccag                                             25

<210>   766
<211>   25
<212>   DNA
<213>   HERV-K

<400>   766
ccccagcacc cgggacccag cggga                                             25

<210>   767
<211>   25
<212>   DNA
<213>   HERV-K

<400>   767
gcacccggga cccagcggga ctttg                                             25

<210>   768
<211>   25
<212>   DNA
<213>   HERV-K

<400>   768
cgggacccag cgggactttg ccaag                                             25

<210>   769
<211>   25
<212>   DNA
<213>   HERV-K

<400>   769
   cccagcggga ctttgccaag gggat                                              25

<210>   770
<211>   25
<212>   DNA
<213>   HERV-K

<400>   770
cgggactttg ccaaggggat ggacc                                          25

<210>   771
<211>   25
<212>   DNA
<213>   HERV-K

<400>   771
ctttgccaag gggatggacc tggct                                          25

<210>   772
<211>   25
<212>   DNA
<213>   HERV-K

<400>   772
ccaaggggat ggacctggct gggcc                                          25

<210>   773
<211>   25
<212>   DNA
<213>   HERV-K

<400>   773
gggatggacc tggctgggcc acgcg                                          25

<210>   774
<211>   25
<212>   DNA
<213>   HERV-K

<400>   774
ggacctggct gggccacgcg gctgt                                          25

<210>   775
<211>   25
<212>   DNA
<213>   HERV-K

<400>   775
tggctgggcc acgcggctgt ttgtg                                          25

<210>   776
<211>   25
<212>   DNA
<213>   HERV-K

<400>   776
gggccacgcg gctgtttgtg taggg                                          25

<210>   777
<211>   25
<212>   DNA
<213>   HERV-K

<400>   777
acgcggctgt ttgtgtaggg aaaag                                          25

<210>   778

<211>   25
<212>   DNA
<213>   HERV-K

<400>   778
gtagaaaagg aagacataaa ctcca                                             25

<210>   779
<211>   25
<212>   DNA
<213>   HERV-K

<400>   779
aaaggaagac ataaactcca ttttg                                             25

<210>   780
<211>   25
<212>   DNA
<213>   HERV-K

<400>   780
aagacataaa ctccattttg agctg                                             25

<210>   781
<211>   25
<212>   DNA
<213>   HERV-K

<400>   781
ataaactcca ttttgagctg tacta                                             25

<210>   782
<211>   25
<212>   DNA
<213>   HERV-K

<400>   782
agaaaaatta ttttgccttg acctg                                             25

<210>   783
<211>   25
<212>   DNA
<213>   HERV-K

<400>   783
aattattttg ccttgacctg ctgtt                                             25

<210>   784
<211>   25
<212>   DNA
<213>   HERV-K

<400>   784
ttttgccttg acctgctgtt aacct                                             25

<210>   785
<211>   25
<212>   DNA
<213>   HERV-K

<400>   785

ccttgacctg ctgttaacct gtaac                                          25

<210>   786
<211>   25
<212>   DNA
<213>   HERV-K

<400>   786
acctgctgtt aacctgtaac tgtag                                          25

<210>   787
<211>   25
<212>   DNA
<213>   HERV-K

<400>   787
ctgttaacct gtaactgtag cccca                                          25

<210>   788
<211>   25
<212>   DNA
<213>   HERV-K

<400>   788
aacctgtaac tgtagcccca accct                                          25

<210>   789
<211>   25
<212>   DNA
<213>   HERV-K

<400>   789
tgtagcccca accctgtgct caaag                                          25

<210>   790
<211>   25
<212>   DNA
<213>   HERV-K

<400>   790
tttaagggat caagggctgt acagg                                          25

<210>   791
<211>   25
<212>   DNA
<213>   HERV-K

<400>   791
gggatcaagg gctgtacagg atgtg                                          25

<210>   792
<211>   25
<212>   DNA
<213>   HERV-K

<400>   792
caagggctgt acaggatgtg ccttg                                          25

<210>   793
<211>   25
<212>   DNA

<213>   HERV-K

<400>   793
atgtgccttg ttaacaatgt gttta                                             25

<210>   794
<211>   25
<212>   DNA
<213>   HERV-K

<400>   794
ccattctcca ttaatcaggg gcacg                                             25

<210>   795
<211>   25
<212>   DNA
<213>   HERV-K

<400>   795
ctccattaat caggggcacg atgca                                             25

<210>   796
<211>   25
<212>   DNA
<213>   HERV-K

<400>   796
ttaatcaggg gcacgatgca ctgcg                                             25

<210>   797
<211>   25
<212>   DNA
<213>   HERV-K

<400>   797
gcacgatgca ctgcggaaag ccaca                                             25

<210>   798
<211>   25
<212>   DNA
<213>   HERV-K

<400>   798
ccacagggac ctctgcccga gaaag                                             25

<210>   799
<211>   25
<212>   DNA
<213>   HERV-K

<400>   799
gggacctctg cccgagaaag cctgg                                             25

<210>   800
<211>   25
<212>   DNA
<213>   HERV-K

<400>   800
ctctgcccga gaaagcctgg gtatt                                             25

<210>   801
<211>   25
<212>   DNA
<213>   HERV-K

<400>   801
gaaagcctgg gtattgtcca aggct                                             25

<210>   802
<211>   25
<212>   DNA
<213>   HERV-K

<400>   802
cctgggtatt gtccaaggct tcccc                                             25

<210>   803
<211>   25
<212>   DNA
<213>   HERV-K

<400>   803
gtattgtcca aggcttcccc ccact                                             25

<210>   804
<211>   25
<212>   DNA
<213>   HERV-K

<400>   804
gtccaaggct tccccccact gagac                                             25

<210>   805
<211>   25
<212>   DNA
<213>   HERV-K

<400>   805
aggcttcccc ccactgagac agcct                                             25

<210>   806
<211>   25
<212>   DNA
<213>   HERV-K

<400>   806
ccactgagac agcctgagat acggc                                             25

<210>   807
<211>   25
<212>   DNA
<213>   HERV-K

<400>   807
aggaaggcct ccgtctcctg catgt                                             25

<210>   808
<211>   25
<212>   DNA
<213>   HERV-K

<400>   808
ggcctccgtc tcctgcatgt ccttg                                          25

<210>   809
<211>   25
<212>   DNA
<213>   HERV-K

<400>   809
ccgtctcctg catgtccttg ggaat                                          25

<210>   810
<211>   25
<212>   DNA
<213>   HERV-K

<400>   810
tcctgcatgt ccttgggaat ggaat                                          25

<210>   811
<211>   25
<212>   DNA
<213>   HERV-K

<400>   811
catgtccttg ggaatggaat gtctt                                          25

<210>   812
<211>   25
<212>   DNA
<213>   HERV-K

<400>   812
ccttgggaat ggaatgtctt ggtgt                                          25

<210>   813
<211>   25
<212>   DNA
<213>   HERV-K

<400>   813
ggaatgtctt ggtgtaaaac ccgat                                          25

<210>   814
<211>   25
<212>   DNA
<213>   HERV-K

<400>   814
gtcttggtgt aaaacccgat agtac                                          25

<210>   815
<211>   25
<212>   DNA
<213>   HERV-K

<400>   815
ggtgtaaaac ccgatagtac attcc                                          25

<210>   816
<211>   25

<212>   DNA
<213>   HERV-K

<400>   816
aaaacccgat agtacattcc ttcta                                             25

<210>   817
<211>   25
<212>   DNA
<213>   HERV-K

<400>   817
ccgatagtac attccttcta ttctg                                             25

<210>   818
<211>   25
<212>   DNA
<213>   HERV-K

<400>   818
agtacattcc ttctattctg agaga                                             25

<210>   819
<211>   25
<212>   DNA
<213>   HERV-K

<400>   819
ttctattctg agagaagaaa accac                                             25

<210>   820
<211>   25
<212>   DNA
<213>   HERV-K

<400>   820
ttctgagaga agaaaaccac cctgt                                             25

<210>   821
<211>   25
<212>   DNA
<213>   HERV-K

<400>   821
agagaagaaa accaccctgt ggctg                                             25

<210>   822
<211>   25
<212>   DNA
<213>   HERV-K

<400>   822
gaggtgagat atgctagcgg caatg                                             25

<210>   823
<211>   25
<212>   DNA
<213>   HERV-K

<400>   823
gagatatgct agcggcaatg ctgct                                             25

<210>   824
<211>   25
<212>   DNA
<213>   HERV-K

<400>   824
atgctagcgg caatgctgct ctgtt                                             25

<210>   825
<211>   25
<212>   DNA
<213>   HERV-K

<400>   825
tggcctatgt gcacatctgg gcaca                                             25

<210>   826
<211>   25
<212>   DNA
<213>   HERV-K

<400>   826
tatgtgcaca tctgggcaca gaacc                                             25

<210>   827
<211>   25
<212>   DNA
<213>   HERV-K

<400>   827
gcacatctgg gcacagaacc tcccc                                             25

<210>   828
<211>   25
<212>   DNA
<213>   HERV-K

<400>   828
tctgggcaca gaacctcccc ttgaa                                             25

<210>   829
<211>   25
<212>   DNA
<213>   HERV-K

<400>   829
gcacagaacc tccccttgaa cttgt                                             25

<210>   830
<211>   25
<212>   DNA
<213>   HERV-K

<400>   830
gaacctcccc ttgaacttgt gacac                                             25

<210>   831
<211>   25
<212>   DNA
<213>   HERV-K

<400>   831
tccccttgaa cttgtgacac agatt                                          25

<210>   832
<211>   25
<212>   DNA
<213>   HERV-K

<400>   832
ttgaacttgt gacacagatt ccttt                                          25

<210>   833
<211>   25
<212>   DNA
<213>   HERV-K

<400>   833
cttgtgacac agattccttt gttca                                          25

<210>   834
<211>   25
<212>   DNA
<213>   HERV-K

<400>   834
agattccttt gttcacatgt tttcc                                          25

<210>   835
<211>   25
<212>   DNA
<213>   HERV-K

<400>   835
ctccccacta tcgccctgtt ctccc                                          25

<210>   836
<211>   25
<212>   DNA
<213>   HERV-K

<400>   836
cactatcgcc ctgttctccc accgc                                          25

<210>   837
<211>   25
<212>   DNA
<213>   HERV-K

<400>   837
tcgccctgtt ctcccaccgc attcc                                          25

<210>   838
<211>   25
<212>   DNA
<213>   HERV-K

<400>   838
ctgttctccc accgcattcc ccttg                                          25

<210>   839

<211>   25
<212>   DNA
<213>   HERV-K

<400>   839
   ctcccaccgc attccccttg ctgag                                              25

<210>   840
<211>   25
<212>   DNA
<213>   HERV-K

<400>   840
tagtaatctg tagataccaa gggaa                                             25

<210>   841
<211>   25
<212>   DNA
<213>   HERV-K

<400>   841
atctgtagat accaagggaa ctcag                                             25

<210>   842
<211>   25
<212>   DNA
<213>   HERV-K

<400>   842
tagataccaa gggaactcag agacc                                             25

<210>   843
<211>   25
<212>   DNA
<213>   HERV-K

<400>   843
accaagggaa ctcagagacc atggc                                             25

<210>   844
<211>   25
<212>   DNA
<213>   HERV-K

<400>   844
gggaactcag agaccatggc cggtg                                             25

<210>   845
<211>   25
<212>   DNA
<213>   HERV-K

<400>   845
ctcagagacc atggccggtg cacat                                             25

<210>   846
<211>   25
<212>   DNA
<213>   HERV-K

<400>   846

agaccatggc cggtgcacat cctcc                                                25

<210>  847
<211>  25
<212>  DNA
<213>  HERV-K

<400>  847
atggccggtg cacatcctcc gtacg                                                25

<210>  848
<211>  25
<212>  DNA
<213>  HERV-K

<400>  848
cggtgcacat cctccgtacg ctgag                                                25

<210>  849
<211>  25
<212>  DNA
<213>  HERV-K

<400>  849
ctgagcgctg gtcccctggg cccat                                                25

<210>  850
<211>  25
<212>  DNA
<213>  HERV-K

<400>  850
cgctggtccc ctgggcccat tgttc                                                25

<210>  851
<211>  25
<212>  DNA
<213>  HERV-K

<400>  851
cctcagtctc tcatccctcc tgacg                                                25

<210>  852
<211>  25
<212>  DNA
<213>  HERV-K

<400>  852
gtctctcatc cctcctgacg agaaa                                                25

<210>  853
<211>  25
<212>  DNA
<213>  HERV-K

<400>  853
tcatccctcc tgacgagaaa taccc                                                25

<210>  854
<211>  25
<212>  DNA

<213>   HERV-K

<400>   854
aggggctggc cccttcatc tgatg                                          25

<210>   855
<211>   25
<212>   DNA
<213>   HERV-K

<400>   855
ctggccccct tcatctgatg cccaa                                         25

<210>   856
<211>   25
<212>   DNA
<213>   HERV-K

<400>   856
tcatctgatg cccaatgtgg gtgcc                                         25

<210>   857
<211>   25
<212>   DNA
<213>   HERV-K

<400>   857
tgatgcccaa tgtgggtgcc tttct                                         25

<210>   858
<211>   25
<212>   DNA
<213>   HERV-K

<400>   858
tttctctagg gtgaaggtac tctac                                         25

<210>   859
<211>   25
<212>   DNA
<213>   HERV-K

<400>   859
ctagggtgaa ggtactctac agtgt                                         25

<210>   860
<211>   25
<212>   DNA
<213>   HERV-K

<400>   860
gtgaaggtac tctacagtgt ggtca                                         25

<210>   861
<211>   25
<212>   DNA
<213>   HERV-K

<400>   861
ggtactctac agtgtggtca ttgag                                         25

<210>   862
<211>   25
<212>   DNA
<213>   HERV-K

<400>   862
tctacagtgt ggtcattgag gacaa                                             25

<210>   863
<211>   25
<212>   DNA
<213>   HERV-K

<400>   863
gacaagttga cgagagagtc ccaag                                             25

<210>   864
<211>   25
<212>   DNA
<213>   HERV-K

<400>   864
gttgacgaga gagtcccaag tacgt                                             25

<210>   865
<211>   25
<212>   DNA
<213>   HERV-K

<400>   865
cgagagagtc ccaagtacgt ccacg                                             25

<210>   866
<211>   25
<212>   DNA
<213>   HERV-K

<400>   866
cttagaggaa cccagggtaa cgatg                                             25

<210>   867
<211>   25
<212>   DNA
<213>   HERV-K

<400>   867
caaacaggag aagatattgt ttcag                                             25

<210>   868
<211>   25
<212>   DNA
<213>   HERV-K

<400>   868
aggagaagat attgtttcag tttct                                             25

<210>   869
<211>   25
<212>   DNA
<213>   HERV-K

<400>   869
gatgccccta aaagctgtgt aacag                                          25

<210>   870
<211>   25
<212>   DNA
<213>   HERV-K

<400>   870
ccctaaaagc tgtgtaacag attgt                                          25

<210>   871
<211>   25
<212>   DNA
<213>   HERV-K

<400>   871
gaagaagagg cagggacaga atccc                                          25

<210>   872
<211>   25
<212>   DNA
<213>   HERV-K

<400>   872
agaggcaggg acagaatccc agcaa                                          25

<210>   873
<211>   25
<212>   DNA
<213>   HERV-K

<400>   873
cagggacaga atcccagcaa ggaac                                          25

<210>   874
<211>   25
<212>   DNA
<213>   HERV-K

<400>   874
acagaatccc agcaaggaac ggaaa                                          25

<210>   875
<211>   25
<212>   DNA
<213>   HERV-K

<400>   875
atcccagcaa ggaacggaaa gttca                                          25

<210>   876
<211>   25
<212>   DNA
<213>   HERV-K

<400>   876
tgactacagt caattacagg agata                                          25

<210>   877
<211>   25

<212>   DNA
<213>   HERV-K

<400>   877
acaggagata atataccctg aatca                                          25

<210>   878
<211>   25
<212>   DNA
<213>   HERV-K

<400>   878
accacgatcg ccatcaactc ctcct                                          25

<210>   879
<211>   25
<212>   DNA
<213>   HERV-K

<400>   879
gatcgccatc aactcctcct cccgt                                          25

<210>   880
<211>   25
<212>   DNA
<213>   HERV-K

<400>   880
ccatcaactc ctcctcccgt ggttc                                          25

<210>   881
<211>   25
<212>   DNA
<213>   HERV-K

<400>   881
aactcctcct cccgtggttc agatg                                          25

<210>   882
<211>   25
<212>   DNA
<213>   HERV-K

<400>   882
cctcaaacgc aggttagaca agcac                                          25

<210>   883
<211>   25
<212>   DNA
<213>   HERV-K

<400>   883
aacgcaggtt agacaagcac aaacc                                          25

<210>   884
<211>   25
<212>   DNA
<213>   HERV-K

<400>   884
agacaagcac aaaccccaag agaaa                                          25

<210>   885
<211>   25
<212>   DNA
<213>   HERV-K

<400>   885
agcacaaacc ccaagagaaa atcaa                                             25

<210>   886
<211>   25
<212>   DNA
<213>   HERV-K

<400>   886
ccaagagaaa atcaagtaga aaggg                                             25

<210>   887
<211>   25
<212>   DNA
<213>   HERV-K

<400>   887
agaaaatcaa gtagaaaggg acaga                                             25

<210>   888
<211>   25
<212>   DNA
<213>   HERV-K

<400>   888
atcaagtaga aagggacaga gtctc                                             25

<210>   889
<211>   25
<212>   DNA
<213>   HERV-K

<400>   889
gtagaaaggg acagagtctc tatcc                                             25

<210>   890
<211>   25
<212>   DNA
<213>   HERV-K

<400>   890
aagggacaga gtctctatcc cggca                                             25

<210>   891
<211>   25
<212>   DNA
<213>   HERV-K

<400>   891
tatcccggca atgccaactc agata                                             25

<210>   892
<211>   25
<212>   DNA
<213>   HERV-K

<400>   892
cggcaatgcc aactcagata cagta                                          25

<210>   893
<211>   25
<212>   DNA
<213>   HERV-K

<400>   893
aaaataagac ccaaccgctg gtagt                                          25

<210>   894
<211>   25
<212>   DNA
<213>   HERV-K

<400>   894
aagacccaac cgctggtagt ttatc                                          25

<210>   895
<211>   25
<212>   DNA
<213>   HERV-K

<400>   895
cgctggtagt ttatcaatac cggct                                          25

<210>   896
<211>   25
<212>   DNA
<213>   HERV-K

<400>   896
gtagtttatc aataccggct gccaa                                          25

<210>   897
<211>   25
<212>   DNA
<213>   HERV-K

<400>   897
ttatcaatac cggctgccaa ccgag                                          25

<210>   898
<211>   25
<212>   DNA
<213>   HERV-K

<400>   898
aataccggct gccaaccgag cttca                                          25

<210>   899
<211>   25
<212>   DNA
<213>   HERV-K

<400>   899
cggctgccaa ccgagcttca gtatc                                          25

<210>   900

<211>   25
<212>   DNA
<213>   HERV-K

<400>   900
gccaaccgag cttcagtatc ggcct                                          25

<210>   901
<211>   25
<212>   DNA
<213>   HERV-K

<400>   901
ccgagcttca gtatcggcct ccttc                                          25

<210>   902
<211>   25
<212>   DNA
<213>   HERV-K

<400>   902
cttcagtatc ggcctccttc agagg                                          25

<210>   903
<211>   25
<212>   DNA
<213>   HERV-K

<400>   903
gtatcggcct ccttcagagg ttcaa                                          25

<210>   904
<211>   25
<212>   DNA
<213>   HERV-K

<400>   904
ggcctccttc agaggttcaa tacag                                          25

<210>   905
<211>   25
<212>   DNA
<213>   HERV-K

<400>   905
ccttcagagg ttcaatacag acctc                                          25

<210>   906
<211>   25
<212>   DNA
<213>   HERV-K

<400>   906
caccatacca gcaacccaca gcgat                                          25

<210>   907
<211>   25
<212>   DNA
<213>   HERV-K

<400>   907

gcaacccaca gcgatggcgt ctaat                                          25

<210>  908
<211>  25
<212>  DNA
<213>  HERV-K

<400>  908
ccacagcgat ggcgtctaat tcacc                                          25

<210>  909
<211>  25
<212>  DNA
<213>  HERV-K

<400>  909
gcgatggcgt ctaattcacc agcaa                                          25

<210>  910
<211>  25
<212>  DNA
<213>  HERV-K

<400>  910
ggcgtctaat tcaccagcaa cacag                                          25

<210>  911
<211>  25
<212>  DNA
<213>  HERV-K

<400>  911
ctaattcacc agcaacacag gacgc                                          25

<210>  912
<211>  25
<212>  DNA
<213>  HERV-K

<400>  912
tcaccagcaa cacaggacgc ggcgc                                          25

<210>  913
<211>  25
<212>  DNA
<213>  HERV-K

<400>  913
agcaacacag gacgcggcgc tgtat                                          25

<210>  914
<211>  25
<212>  DNA
<213>  HERV-K

<400>  914
cacaggacgc ggcgctgtat cctca                                          25

<210>  915
<211>  25
<212>  DNA

<213>   HERV-K

<400>   915
gacgcggcgc tgtatcctca gccgc                                          25

<210>   916
<211>   25
<212>   DNA
<213>   HERV-K

<400>   916
ggcgctgtat cctcagccgc ccact                                          25

<210>   917
<211>   25
<212>   DNA
<213>   HERV-K

<400>   917
atcacgtagt ggacagggtg gtgca                                          25

<210>   918
<211>   25
<212>   DNA
<213>   HERV-K

<400>   918
gtagtggaca gggtggtgca ctgca                                          25

<210>   919
<211>   25
<212>   DNA
<213>   HERV-K

<400>   919
ggacagggtg gtgcactgca tgcag                                          25

<210>   920
<211>   25
<212>   DNA
<213>   HERV-K

<400>   920
gggtggtgca ctgcatgcag tcatt                                          25

<210>   921
<211>   25
<212>   DNA
<213>   HERV-K

<400>   921
gtgcactgca tgcagtcatt gatga                                          25

<210>   922
<211>   25
<212>   DNA
<213>   HERV-K

<400>   922
aaatggtctt tttactccct ggaaa                                          25

<210>   923
<211>   25
<212>   DNA
<213>   HERV-K

<400>   923
gtctttttac tccctggaaa gcccc                                             25

<210>   924
<211>   25
<212>   DNA
<213>   HERV-K

<400>   924
agggagggta ggcctttgag ggaga                                             25

<210>   925
<211>   25
<212>   DNA
<213>   HERV-K

<400>   925
gggtaggcct ttgagggaga tcaag                                             25

<210>   926
<211>   25
<212>   DNA
<213>   HERV-K

<400>   926
ggcctttgag ggagatcaag tctaa                                             25

<210>   927
<211>   25
<212>   DNA
<213>   HERV-K

<400>   927
caggaaaagc tgcttattgg gctaa                                             25

<210>   928
<211>   25
<212>   DNA
<213>   HERV-K

<400>   928
aaagctgctt attgggctaa tcagg                                             25

<210>   929
<211>   25
<212>   DNA
<213>   HERV-K

<400>   929
tgtttctgtc atcggcatag gtact                                             25

<210>   930
<211>   25
<212>   DNA
<213>   HERV-K

<400>   930
ctgtcatcgg cataggtact gcctc                                          25

<210>   931
<211>   25
<212>   DNA
<213>   HERV-K

<400>   931
atcggcatag gtactgcctc agaag                                          25

<210>   932
<211>   25
<212>   DNA
<213>   HERV-K

<400>   932
ggttcagcct gtgatcactt cattc                                          25

<210>   933
<211>   25
<212>   DNA
<213>   HERV-K

<400>   933
agcctgtgat cacttcattc caatc                                          25

<210>   934
<211>   25
<212>   DNA
<213>   HERV-K

<400>   934
gtgatcactt cattccaatc aattt                                          25

<210>   935
<211>   25
<212>   DNA
<213>   HERV-K

<400>   935
cacttcattc caatcaattt atggg                                          25

<210>   936
<211>   25
<212>   DNA
<213>   HERV-K

<400>   936
agggactagg aaagaagtcc caatt                                          25

<210>   937
<211>   25
<212>   DNA
<213>   HERV-K

<400>   937
ctaggaaaga agtcccaatt gaggc                                          25

<210>   938
<211>   25

<212>   DNA
<213>   HERV-K

<400>   938
ccattccatt aacttggggg aaaaa                                             25

<210>   939
<211>   25
<212>   DNA
<213>   HERV-K

<400>   939
ccattaactt gggggaaaaa aaaac                                             25

<210>   940
<211>   25
<212>   DNA
<213>   HERV-K

<400>   940
aacttggggg aaaaaaaaac aactg                                             25

<210>   941
<211>   25
<212>   DNA
<213>   HERV-K

<400>   941
gggggaaaaa aaaacaactg tatgg                                             25

<210>   942
<211>   25
<212>   DNA
<213>   HERV-K

<400>   942
aaaacaactg tatggtaaat cagca                                             25

<210>   943
<211>   25
<212>   DNA
<213>   HERV-K

<400>   943
aactgtatgg taaatcagca gcgct                                             25

<210>   944
<211>   25
<212>   DNA
<213>   HERV-K

<400>   944
tatggtaaat cagcagcgct tccaa                                             25

<210>   945
<211>   25
<212>   DNA
<213>   HERV-K

<400>   945
taaatcagca gcgcttccaa aacaa                                             25

<210>   946
<211>   25
<212>   DNA
<213>   HERV-K

<400>   946
cagcagcgct tccaaaacaa aaact                                             25

<210>   947
<211>   25
<212>   DNA
<213>   HERV-K

<400>   947
attagaaaaa ggacattgag ccttc                                             25

<210>   948
<211>   25
<212>   DNA
<213>   HERV-K

<400>   948
aaaaaggaca ttgagccttc atttt                                             25

<210>   949
<211>   25
<212>   DNA
<213>   HERV-K

<400>   949
attttcgcct tggaattctg tttgt                                             25

<210>   950
<211>   25
<212>   DNA
<213>   HERV-K

<400>   950
cgccttggaa ttctgtttgt aattc                                             25

<210>   951
<211>   25
<212>   DNA
<213>   HERV-K

<400>   951
aaatccggca gatggcgtat aatgc                                             25

<210>   952
<211>   25
<212>   DNA
<213>   HERV-K

<400>   952
cggcagatgg cgtataatgc cgtaa                                             25

<210>   953
<211>   25
<212>   DNA
<213>   HERV-K

<400>   953
gatggcgtat aatgccgtaa ttcaa                                          25

<210>   954
<211>   25
<212>   DNA
<213>   HERV-K

<400>   954
tttgctttta ccacaccagc ctaaa                                          25

<210>   955
<211>   25
<212>   DNA
<213>   HERV-K

<400>   955
ttttaccaca ccagcctaaa taata                                          25

<210>   956
<211>   25
<212>   DNA
<213>   HERV-K

<400>   956
aatagttcaa ctatttgtca gctca                                          25

<210>   957
<211>   25
<212>   DNA
<213>   HERV-K

<400>   957
ttcaactatt tgtcagctca agctc                                          25

<210>   958
<211>   25
<212>   DNA
<213>   HERV-K

<400>   958
ctatttgtca gctcaagctc tgcaa                                          25

<210>   959
<211>   25
<212>   DNA
<213>   HERV-K

<400>   959
ttttcagact gttacatcgt tcact                                          25

<210>   960
<211>   25
<212>   DNA
<213>   HERV-K

<400>   960
agactgttac atcgttcact atgtt                                          25

<210>   961

<211>   25
<212>   DNA
<213>   HERV-K

<400>   961
ctctactcct ttccgttact tggga                                             25

<210>   962
<211>   25
<212>   DNA
<213>   HERV-K

<400>   962
tcggaattaa atagtgaaag aacgt                                             25

<210>   963
<211>   25
<212>   DNA
<213>   HERV-K

<400>   963
atagtgaaag aacgttaact ccaga                                             25

<210>   964
<211>   25
<212>   DNA
<213>   HERV-K

<400>   964
gaaagaacgt taactccaga ggcaa                                             25

<210>   965
<211>   25
<212>   DNA
<213>   HERV-K

<400>   965
ttttgctact gcacattccc taaca                                             25

<210>   966
<211>   25
<212>   DNA
<213>   HERV-K

<400>   966
ctactgcaca ttccctaaca ggcat                                             25

<210>   967
<211>   25
<212>   DNA
<213>   HERV-K

<400>   967
gcacattccc taacaggcat cattg                                             25

<210>   968
<211>   25
<212>   DNA
<213>   HERV-K

<400>   968

ttccctaaca ggcatcattg ttcaa 25

<210> 969
<211> 25
<212> DNA
<213> HERV-K

<400> 969
attattgaca atcgttaccc caaaa 25

<210> 970
<211> 25
<212> DNA
<213> HERV-K

<400> 970
tgacaatcgt taccccaaaa caaaa 25

<210> 971
<211> 25
<212> DNA
<213> HERV-K

<400> 971
atcgttaccc caaaacaaaa atctt 25

<210> 972
<211> 25
<212> DNA
<213> HERV-K

<400> 972
acctaaagtt accaaacata agcct 25

<210> 973
<211> 25
<212> DNA
<213> HERV-K

<400> 973
acataagcct ttaaaaaatg ctctg 25

<210> 974
<211> 25
<212> DNA
<213> HERV-K

<400> 974
agcctttaaa aaatgctctg gcagt 25

<210> 975
<211> 25
<212> DNA
<213> HERV-K

<400> 975
gggccaaaag aatgagtcat caaaa 25

<210> 976
<211> 25
<212> DNA

<213>   HERV-K

<400>   976
aaaagaatga gtcatcaaaa ctcag                                          25

<210>   977
<211>   25
<212>   DNA
<213>   HERV-K

<400>   977
aatgagtcat caaaactcag tatca                                          25

<210>   978
<211>   25
<212>   DNA
<213>   HERV-K

<400>   978
gtcatcaaaa ctcagtatca cttga                                          25

<210>   979
<211>   25
<212>   DNA
<213>   HERV-K

<400>   979
caaaactcag tatcacttga ctcaa                                          25

<210>   980
<211>   25
<212>   DNA
<213>   HERV-K

<400>   980
ctcagtatca cttgactcaa agagc                                          25

<210>   981
<211>   25
<212>   DNA
<213>   HERV-K

<400>   981
tatcacttga ctcaaagagc agagt                                          25

<210>   982
<211>   25
<212>   DNA
<213>   HERV-K

<400>   982
cttgactcaa agagcagagt tggtt                                          25

<210>   983
<211>   25
<212>   DNA
<213>   HERV-K

<400>   983
tggttgccgt cattacagtg ttaac                                          25

<210>   984
<211>   25
<212>   DNA
<213>   HERV-K

<400>   984
gccgtcatta cagtgttaac aagat                                          25

<210>   985
<211>   25
<212>   DNA
<213>   HERV-K

<400>   985
caggctacaa aggatattga gagag                                          25

<210>   986
<211>   25
<212>   DNA
<213>   HERV-K

<400>   986
tacaaaggat attgagagag cccta                                          25

<210>   987
<211>   25
<212>   DNA
<213>   HERV-K

<400>   987
aggatattga gagagcccta atcaa                                          25

<210>   988
<211>   25
<212>   DNA
<213>   HERV-K

<400>   988
attgagagag ccctaatcaa ataca                                          25

<210>   989
<211>   25
<212>   DNA
<213>   HERV-K

<400>   989
ttatggatga tcagttaaac ccgct                                          25

<210>   990
<211>   25
<212>   DNA
<213>   HERV-K

<400>   990
tcagttaaac ccgctgttta atttg                                          25

<210>   991
<211>   25
<212>   DNA
<213>   HERV-K

<400>   991
taaacccgct gtttaatttg ttaca                                          25

<210>   992
<211>   25
<212>   DNA
<213>   HERV-K

<400>   992
ttgactcatg taaatgcaat aggat                                          25

<210>   993
<211>   25
<212>   DNA
<213>   HERV-K

<400>   993
cattgcaccc agtgtcagat tctac                                          25

<210>   994
<211>   25
<212>   DNA
<213>   HERV-K

<400>   994
agtgtcagat tctacacctg gccac                                          25

<210>   995
<211>   25
<212>   DNA
<213>   HERV-K

<400>   995
cagattctac acctggccac tcagg                                          25

<210>   996
<211>   25
<212>   DNA
<213>   HERV-K

<400>   996
tctacacctg gccactcagg aggca                                          25

<210>   997
<211>   25
<212>   DNA
<213>   HERV-K

<400>   997
acctggccac tcaggaggca agagt                                          25

<210>   998
<211>   25
<212>   DNA
<213>   HERV-K

<400>   998
gccactcagg aggcaagagt taatc                                          25

<210>   999
<211>   25

<212>   DNA
<213>   HERV-K

<400>   999
atggcaaatg gatgtcatgc acgta                                             25

<210>   1000
<211>   25
<212>   DNA
<213>   HERV-K

<400>   1000
aaatggatgt catgcacgta ccttc                                             25

<210>   1001
<211>   25
<212>   DNA
<213>   HERV-K

<400>   1001
gatgtcatgc acgtaccttc atttg                                             25

<210>   1002
<211>   25
<212>   DNA
<213>   HERV-K

<400>   1002
catgcacgta ccttcatttg gaaaa                                             25

<210>   1003
<211>   25
<212>   DNA
<213>   HERV-K

<400>   1003
gttccagaaa aagttaaaac agaca                                             25

<210>   1004
<211>   25
<212>   DNA
<213>   HERV-K

<400>   1004
agaaaaagtt aaaacagaca atggg                                             25

<210>   1005
<211>   25
<212>   DNA
<213>   HERV-K

<400>   1005
aagttaaaac agacaatggg ccagg                                             25

<210>   1006
<211>   25
<212>   DNA
<213>   HERV-K

<400>   1006
aaaacagaca atgggccagg ttact                                             25

<210>   1007
<211>   25
<212>   DNA
<213>   HERV-K

<400>   1007
agacaatggg ccaggttact gtagt                                          25

<210>   1008
<211>   25
<212>   DNA
<213>   HERV-K

<400>   1008
ccaggttact gtagtaaagc agttc                                          25

<210>   1009
<211>   25
<212>   DNA
<213>   HERV-K

<400>   1009
aaacaaaaaa aaggaaaaga cagga                                          25

<210>   1010
<211>   25
<212>   DNA
<213>   HERV-K

<400>   1010
aaggaaaaga caggagtata acact                                          25

<210>   1011
<211>   25
<212>   DNA
<213>   HERV-K

<400>   1011
aaagacagga gtataacact cccca                                          25

<210>   1012
<211>   25
<212>   DNA
<213>   HERV-K

<400>   1012
cactacctct gcagaacaac atctt                                          25

<210>   1013
<211>   25
<212>   DNA
<213>   HERV-K

<400>   1013
aaaataaaac atgggaaatg gggaa                                          25

<210>   1014
<211>   25
<212>   DNA
<213>   HERV-K

<400>   1014
aaaacatggg aaatggggaa ggtga                                          25

<210>   1015
<211>   25
<212>   DNA
<213>   HERV-K

<400>   1015
taaagttcta caatgaactc actgg                                          25

<210>   1016
<211>   25
<212>   DNA
<213>   HERV-K

<400>   1016
ttctacaatg aactcactgg agatg                                          25

<210>   1017
<211>   25
<212>   DNA
<213>   HERV-K

<400>   1017
caatgaactc actggagatg caaag                                          25

<210>   1018
<211>   25
<212>   DNA
<213>   HERV-K

<400>   1018
aactcactgg agatgcaaag aaaag                                          25

<210>   1019
<211>   25
<212>   DNA
<213>   HERV-K

<400>   1019
actggagatg caaagaaaag tgtgg                                          25

<210>   1020
<211>   25
<212>   DNA
<213>   HERV-K

<400>   1020
agatgcaaag aaaagtgtgg agatg                                          25

<210>   1021
<211>   25
<212>   DNA
<213>   HERV-K

<400>   1021
caaagaaaag tgtggagatg gagac                                          25

<210>   1022

<211>   25
<212>   DNA
<213>   HERV-K

<400>   1022
aaaagtgtgg agatggagac acccc                                          25

<210>   1023
<211>   25
<212>   DNA
<213>   HERV-K

<400>   1023
tgtggagatg gagacacccc aatcg                                          25

<210>   1024
<211>   25
<212>   DNA
<213>   HERV-K

<400>   1024
agatggagac accccaatcg actcg                                          25

<210>   1025
<211>   25
<212>   DNA
<213>   HERV-K

<400>   1025
gagacacccc aatcgactcg ccagg                                          25

<210>   1026
<211>   25
<212>   DNA
<213>   HERV-K

<400>   1026
accccaatcg actcgccagg taaac                                          25

<210>   1027
<211>   25
<212>   DNA
<213>   HERV-K

<400>   1027
aatcgactcg ccaggtaaac aaaat                                          25

<210>   1028
<211>   25
<212>   DNA
<213>   HERV-K

<400>   1028
ggtgatatca gaagaacaga aaaag                                          25

<210>   1029
<211>   25
<212>   DNA
<213>   HERV-K

<400>   1029

tatcagaaga acagaaaaag ttgcc                                          25

<210>  1030
<211>  25
<212>  DNA
<213>  HERV-K

<400>  1030
gaagaacaga aaaagttgcc ttcca                                          25

<210>  1031
<211>  25
<212>  DNA
<213>  HERV-K

<400>  1031
acagaaaaag ttgccttcca tcaag                                          25

<210>  1032
<211>  25
<212>  DNA
<213>  HERV-K

<400>  1032
aaaagttgcc ttccatcaag gaagc                                          25

<210>  1033
<211>  25
<212>  DNA
<213>  HERV-K

<400>  1033
ttgccttcca tcaaggaagc agagt                                          25

<210>  1034
<211>  25
<212>  DNA
<213>  HERV-K

<400>  1034
ttccatcaag gaagcagagt tgcca                                          25

<210>  1035
<211>  25
<212>  DNA
<213>  HERV-K

<400>  1035
tcaaggaagc agagttgcca atata                                          25

<210>  1036
<211>  25
<212>  DNA
<213>  HERV-K

<400>  1036
gaagcagagt tgccaatata ggcac                                          25

<210>  1037
<211>  25
<212>  DNA

<213>   HERV-K

<400>   1037
agagttgcca atataggcac aatta                                          25

<210>   1038
<211>   25
<212>   DNA
<213>   HERV-K

<400>   1038
tgccaatata ggcacaatta aagaa                                          25

<210>   1039
<211>   25
<212>   DNA
<213>   HERV-K

<400>   1039
atataggcac aattaaagaa gctga                                          25

<210>   1040
<211>   25
<212>   DNA
<213>   HERV-K

<400>   1040
cacagttagc taaaaaaaaa agcct                                          25

<210>   1041
<211>   25
<212>   DNA
<213>   HERV-K

<400>   1041
aaaaaagcct agagaataca aaggt                                          25

<210>   1042
<211>   25
<212>   DNA
<213>   HERV-K

<400>   1042
agcctagaga atacaaaggt gacac                                          25

<210>   1043
<211>   25
<212>   DNA
<213>   HERV-K

<400>   1043
agagaataca aaggtgacac caact                                          25

<210>   1044
<211>   25
<212>   DNA
<213>   HERV-K

<400>   1044
atacaaaggt gacaccaact ccaga                                          25

<210>   1045
<211>   25
<212>   DNA
<213>   HERV-K

<400>   1045
aaggtgacac caactccaga gaata                                          25

<210>   1046
<211>   25
<212>   DNA
<213>   HERV-K

<400>   1046
gacaccaact ccagagaata tgctg                                          25

<210>   1047
<211>   25
<212>   DNA
<213>   HERV-K

<400>   1047
gaatatgctg cttgcagctc tgatg                                          25

<210>   1048
<211>   25
<212>   DNA
<213>   HERV-K

<400>   1048
atcaacggtg gtaagtcttc ccaag                                          25

<210>   1049
<211>   25
<212>   DNA
<213>   HERV-K

<400>   1049
cggtggtaag tcttcccaag tctgc                                          25

<210>   1050
<211>   25
<212>   DNA
<213>   HERV-K

<400>   1050
gtaagtcttc ccaagtctgc aggag                                          25

<210>   1051
<211>   25
<212>   DNA
<213>   HERV-K

<400>   1051
tcttcccaag tctgcaggag cagct                                          25

<210>   1052
<211>   25
<212>   DNA
<213>   HERV-K

<400>   1052
ccttaattcg ggcagttaca tagat                                          25

<210>   1053
<211>   25
<212>   DNA
<213>   HERV-K

<400>   1053
attcgggcag ttacatagat ggata                                          25

<210>   1054
<211>   25
<212>   DNA
<213>   HERV-K

<400>   1054
ggcagttaca tagatggata atcct                                          25

<210>   1055
<211>   25
<212>   DNA
<213>   HERV-K

<400>   1055
tagtgcatgg gtgcctggcc ccaca                                          25

<210>   1056
<211>   25
<212>   DNA
<213>   HERV-K

<400>   1056
catgggtgcc tggccccaca gatga                                          25

<210>   1057
<211>   25
<212>   DNA
<213>   HERV-K

<400>   1057
gtgcctggcc ccacagatga ctgtt                                          25

<210>   1058
<211>   25
<212>   DNA
<213>   HERV-K

<400>   1058
tggccccaca gatgactgtt gccct                                          25

<210>   1059
<211>   25
<212>   DNA
<213>   HERV-K

<400>   1059
gcccaacctg aagaaggaat gatga                                          25

<210>   1060
<211>   25

<212>   DNA
<213>   HERV-K

<400>   1060
cattgggtat ccttatcctc ctgtt                                          25

<210>   1061
<211>   25
<212>   DNA
<213>   HERV-K

<400>   1061
ggtatcctta tcctcctgtt tgcct                                          25

<210>   1062
<211>   25
<212>   DNA
<213>   HERV-K

<400>   1062
ccttatcctc ctgtttgcct aggga                                          25

<210>   1063
<211>   25
<212>   DNA
<213>   HERV-K

<400>   1063
cctacagtca gtgctaccag tagat                                          25

<210>   1064
<211>   25
<212>   DNA
<213>   HERV-K

<400>   1064
agtcagtgct accagtagat ttact                                          25

<210>   1065
<211>   25
<212>   DNA
<213>   HERV-K

<400>   1065
catggtaagt ggaatgtcac agata                                          25

<210>   1066
<211>   25
<212>   DNA
<213>   HERV-K

<400>   1066
tcattacaat gtaggcctaa gggga                                          25

<210>   1067
<211>   25
<212>   DNA
<213>   HERV-K

<400>   1067
acaatgtagg cctaagggga aggct                                          25

<210>   1068
<211>   25
<212>   DNA
<213>   HERV-K

<400>   1068
gtaggcctaa ggggaaggct tgccc                                             25

<210>   1069
<211>   25
<212>   DNA
<213>   HERV-K

<400>   1069
caaaaagccc agaagtctta gtctg                                             25

<210>   1070
<211>   25
<212>   DNA
<213>   HERV-K

<400>   1070
agcccagaag tcttagtctg cggag                                             25

<210>   1071
<211>   25
<212>   DNA
<213>   HERV-K

<400>   1071
agaagtctta gtctgcggag aatgt                                             25

<210>   1072
<211>   25
<212>   DNA
<213>   HERV-K

<400>   1072
tcttagtctg cggagaatgt gtggc                                             25

<210>   1073
<211>   25
<212>   DNA
<213>   HERV-K

<400>   1073
gtctgcggag aatgtgtggc tgata                                             25

<210>   1074
<211>   25
<212>   DNA
<213>   HERV-K

<400>   1074
gtggctgata ctgcagtgta gtaca                                             25

<210>   1075
<211>   25
<212>   DNA
<213>   HERV-K

<400>   1075
tgatactgca gtgtagtaca aaaca                                          25

<210>   1076
<211>   25
<212>   DNA
<213>   HERV-K

<400>   1076
ctgcagtgta gtacaaaaca atgaa                                          25

<210>   1077
<211>   25
<212>   DNA
<213>   HERV-K

<400>   1077
ttttgaacta tgatagactg ggtcc                                          25

<210>   1078
<211>   25
<212>   DNA
<213>   HERV-K

<400>   1078
aactatgata gactgggtcc cttga                                          25

<210>   1079
<211>   25
<212>   DNA
<213>   HERV-K

<400>   1079
tgatagactg ggtcccttga ggcca                                          25

<210>   1080
<211>   25
<212>   DNA
<213>   HERV-K

<400>   1080
ggtcccttga ggccaattat atcat                                          25

<210>   1081
<211>   25
<212>   DNA
<213>   HERV-K

<400>   1081
cttgaggcca attatatcat aactg                                          25

<210>   1082
<211>   25
<212>   DNA
<213>   HERV-K

<400>   1082
attatatcat aactgtacag gccag                                          25

<210>   1083

<211>   25
<212>   DNA
<213>   HERV-K

<400>   1083
atcataactg tacaggccag actca                                          25

<210>   1084
<211>   25
<212>   DNA
<213>   HERV-K

<400>   1084
aactgtacag gccagactca ttcat                                          25

<210>   1085
<211>   25
<212>   DNA
<213>   HERV-K

<400>   1085
tacaggccag actcattcat gttca                                          25

<210>   1086
<211>   25
<212>   DNA
<213>   HERV-K

<400>   1086
tggcccatta atccagccta tgacg                                          25

<210>   1087
<211>   25
<212>   DNA
<213>   HERV-K

<400>   1087
cattaatcca gcctatgacg gtgat                                          25

<210>   1088
<211>   25
<212>   DNA
<213>   HERV-K

<400>   1088
atccagccta tgacggtgat gtaac                                          25

<210>   1089
<211>   25
<212>   DNA
<213>   HERV-K

<400>   1089
gcctatgacg gtgatgtaac tgaaa                                          25

<210>   1090
<211>   25
<212>   DNA
<213>   HERV-K

<400>   1090

tgacggtgat gtaactgaaa ggctg                                          25

<210>  1091
<211>  25
<212>  DNA
<213>  HERV-K

<400>  1091
atagaaggtt agaatcactc tgtcc                                          25

<210>  1092
<211>  25
<212>  DNA
<213>  HERV-K

<400>  1092
aggttagaat cactctgtcc aagga                                          25

<210>  1093
<211>  25
<212>  DNA
<213>  HERV-K

<400>  1093
agaatcactc tgtccaagga aatgg                                          25

<210>  1094
<211>  25
<212>  DNA
<213>  HERV-K

<400>  1094
cactctgtcc aaggaaatgg ggtga                                          25

<210>  1095
<211>  25
<212>  DNA
<213>  HERV-K

<400>  1095
tgtccaagga aatggggtga aaagg                                          25

<210>  1096
<211>  25
<212>  DNA
<213>  HERV-K

<400>  1096
tcatcacctt gaccaaagtt agtcc                                          25

<210>  1097
<211>  25
<212>  DNA
<213>  HERV-K

<400>  1097
accttgacca aagttagtcc tgtta                                          25

<210>  1098
<211>  25
<212>  DNA

<213>   HERV-K

<400>   1098
gaccaaagtt agtcctgtta ctggt                                          25

<210>   1099
<211>   25
<212>   DNA
<213>   HERV-K

<400>   1099
cctgaacatc cagaattagg aagct                                          25

<210>   1100
<211>   25
<212>   DNA
<213>   HERV-K

<400>   1100
acatccagaa ttaggaagct tactg                                          25

<210>   1101
<211>   25
<212>   DNA
<213>   HERV-K

<400>   1101
cagaattagg aagcttactg tggcc                                          25

<210>   1102
<211>   25
<212>   DNA
<213>   HERV-K

<400>   1102
ttaggaagct tactgtggcc tcaca                                          25

<210>   1103
<211>   25
<212>   DNA
<213>   HERV-K

<400>   1103
ttctggaaat caagctatag gaaca                                          25

<210>   1104
<211>   25
<212>   DNA
<213>   HERV-K

<400>   1104
tcctttgcaa aattgtgtaa aactc                                          25

<210>   1105
<211>   25
<212>   DNA
<213>   HERV-K

<400>   1105
tgcaaaattg tgtaaaactc cctta                                          25

<210>   1106
<211>   25
<212>   DNA
<213>   HERV-K

<400>   1106
aactccctta tattgctagt tgtag                                             25

<210>   1107
<211>   25
<212>   DNA
<213>   HERV-K

<400>   1107
gttattaaac ctgattccca aacca                                             25

<210>   1108
<211>   25
<212>   DNA
<213>   HERV-K

<400>   1108
taaacctgat tcccaaacca taatc                                             25

<210>   1109
<211>   25
<212>   DNA
<213>   HERV-K

<400>   1109
ctgattccca aaccataatc tgtga                                             25

<210>   1110
<211>   25
<212>   DNA
<213>   HERV-K

<400>   1110
tcccaaacca taatctgtga aaatt                                             25

<210>   1111
<211>   25
<212>   DNA
<213>   HERV-K

<400>   1111
aaccataatc tgtgaaaatt gtgga                                             25

<210>   1112
<211>   25
<212>   DNA
<213>   HERV-K

<400>   1112
taatctgtga aaattgtgga atgtt                                             25

<210>   1113
<211>   25
<212>   DNA
<213>   HERV-K

<400>   1113
tgtgaaaatt gtggaatgtt tactt                                          25

<210>   1114
<211>   25
<212>   DNA
<213>   HERV-K

<400>   1114
aaattgtgga atgtttactt gcatt                                          25

<210>   1115
<211>   25
<212>   DNA
<213>   HERV-K

<400>   1115
gtggaatgtt tacttgcatt gattt                                          25

<210>   1116
<211>   25
<212>   DNA
<213>   HERV-K

<400>   1116
gcaccgtatt ctactaggaa gagca                                          25

<210>   1117
<211>   25
<212>   DNA
<213>   HERV-K

<400>   1117
gtattctact aggaagagca agaga                                          25

<210>   1118
<211>   25
<212>   DNA
<213>   HERV-K

<400>   1118
ctactaggaa gagcaagaga gggtg                                          25

<210>   1119
<211>   25
<212>   DNA
<213>   HERV-K

<400>   1119
aggaagagca agagagggtg tgtgg                                          25

<210>   1120
<211>   25
<212>   DNA
<213>   HERV-K

<400>   1120
atccttgtgt ccatggaccg accat                                          25

<210>   1121
<211>   25

<212>   DNA
<213>   HERV-K

<400>   1121
gaccgaccat gggaggcttc gctat                                             25

<210>   1122
<211>   25
<212>   DNA
<213>   HERV-K

<400>   1122
accatgggag gcttcgctat ccatc                                             25

<210>   1123
<211>   25
<212>   DNA
<213>   HERV-K

<400>   1123
gggaggcttc gctatccatc catat                                             25

<210>   1124
<211>   25
<212>   DNA
<213>   HERV-K

<400>   1124
gcttcgctat ccatccatat tttaa                                             25

<210>   1125
<211>   25
<212>   DNA
<213>   HERV-K

<400>   1125
gctatccatc catattttaa cggaa                                             25

<210>   1126
<211>   25
<212>   DNA
<213>   HERV-K

<400>   1126
ttgatggcag tgattatggg cctca                                             25

<210>   1127
<211>   25
<212>   DNA
<213>   HERV-K

<400>   1127
ggcagtgatt atgggcctca ttgca                                             25

<210>   1128
<211>   25
<212>   DNA
<213>   HERV-K

<400>   1128
cagaatacgt aaatgattgg caaaa                                             25

<210>   1129
<211>   25
<212>   DNA
<213>   HERV-K

<400>   1129
tacgtaaatg attggcaaaa gaatt                                             25

<210>   1130
<211>   25
<212>   DNA
<213>   HERV-K

<400>   1130
tcatttggat gggagaggct catga                                             25

<210>   1131
<211>   25
<212>   DNA
<213>   HERV-K

<400>   1131
tggatgggag aggctcatga gcttg                                             25

<210>   1132
<211>   25
<212>   DNA
<213>   HERV-K

<400>   1132
gggagaggct catgagcttg gaata                                             25

<210>   1133
<211>   25
<212>   DNA
<213>   HERV-K

<400>   1133
tctttttcag ttacgatgtg actgg                                             25

<210>   1134
<211>   25
<212>   DNA
<213>   HERV-K

<400>   1134
ttcagttacg atgtgactgg aatac                                             25

<210>   1135
<211>   25
<212>   DNA
<213>   HERV-K

<400>   1135
ttacgatgtg actggaatac atcag                                             25

<210>   1136
<211>   25
<212>   DNA
<213>   HERV-K

<400>   1136
atcagatttt tgtgttacac cacaa                                          25

<210>   1137
<211>   25
<212>   DNA
<213>   HERV-K

<400>   1137
atttttgtgt tacaccacaa gccta                                          25

<210>   1138
<211>   25
<212>   DNA
<213>   HERV-K

<400>   1138
tgtgttacac cacaagccta taatg                                          25

<210>   1139
<211>   25
<212>   DNA
<213>   HERV-K

<400>   1139
atggttagat gccatctgca aggag                                          25

<210>   1140
<211>   25
<212>   DNA
<213>   HERV-K

<400>   1140
tagatgccat ctgcaaggag gagaa                                          25

<210>   1141
<211>   25
<212>   DNA
<213>   HERV-K

<400>   1141
gccatctgca aggaggagaa gataa                                          25

<210>   1142
<211>   25
<212>   DNA
<213>   HERV-K

<400>   1142
ctgcaaggag gagaagataa tctta                                          25

<210>   1143
<211>   25
<212>   DNA
<213>   HERV-K

<400>   1143
taaatttggt gccaggaacg gagac                                          25

<210>   1144

<211>   25
<212>   DNA
<213>   HERV-K

<400>   1144
ttggtgccag gaacggagac aatcg                                             25

<210>   1145
<211>   25
<212>   DNA
<213>   HERV-K

<400>   1145
gccaggaacg gagacaatcg tgaaa                                             25

<210>   1146
<211>   25
<212>   DNA
<213>   HERV-K

<400>   1146
gaacggagac aatcgtgaaa gctgc                                             25

<210>   1147
<211>   25
<212>   DNA
<213>   HERV-K

<400>   1147
gagacaatcg tgaaagctgc tgata                                             25

<210>   1148
<211>   25
<212>   DNA
<213>   HERV-K

<400>   1148
gcctcacaaa tcttaagcca gtcac                                             25

<210>   1149
<211>   25
<212>   DNA
<213>   HERV-K

<400>   1149
acaaatctta agccagtcac ttggg                                             25

<210>   1150
<211>   25
<212>   DNA
<213>   HERV-K

<400>   1150
tcttaagcca gtcacttggg ttaaa                                             25

<210>   1151
<211>   25
<212>   DNA
<213>   HERV-K

<400>   1151

agccagtcac ttgggttaaa agcat                                          25

<210>  1152
<211>  25
<212>  DNA
<213>  HERV-K

<400>  1152
ttgggttaaa agcatcagaa gtttc                                          25

<210>  1153
<211>  25
<212>  DNA
<213>  HERV-K

<400>  1153
agcatcagaa gtttcactat tgtaa                                          25

<210>  1154
<211>  25
<212>  DNA
<213>  HERV-K

<400>  1154
agctccaaag agacagcaac cagca                                          25

<210>  1155
<211>  25
<212>  DNA
<213>  HERV-K

<400>  1155
caaagagaca gcaaccagca agaat                                          25

<210>  1156
<211>  25
<212>  DNA
<213>  HERV-K

<400>  1156
agacagcaac cagcaagaat gggcc                                          25

<210>  1157
<211>  25
<212>  DNA
<213>  HERV-K

<400>  1157
gcaaccagca agaatgggcc atagt                                          25

<210>  1158
<211>  25
<212>  DNA
<213>  HERV-K

<400>  1158
cagcaagaat gggccatagt gacga                                          25

<210>  1159
<211>  25
<212>  DNA

<213>   HERV-K

<400>   1159
agaatgggcc atagtgacga tggtg                                          25

<210>   1160
<211>   25
<212>   DNA
<213>   HERV-K

<400>   1160
gggccatagt gacgatggtg gtttt                                          25

<210>   1161
<211>   25
<212>   DNA
<213>   HERV-K

<400>   1161
atagtgacga tggtggtttt gtcaa                                          25

<210>   1162
<211>   25
<212>   DNA
<213>   HERV-K

<400>   1162
gacgatggtg gttttgtcaa aaaga                                          25

<210>   1163
<211>   25
<212>   DNA
<213>   HERV-K

<400>   1163
gtcaaaaaga aaaggggggg atatg                                          25

<210>   1164
<211>   25
<212>   DNA
<213>   HERV-K

<400>   1164
aaagaaaagg gggggatatg taagg                                          25

<210>   1165
<211>   25
<212>   DNA
<213>   HERV-K

<400>   1165
aaaggggggg atatgtaagg aaaag                                          25

<210>   1166
<211>   25
<212>   DNA
<213>   HERV-K

<400>   1166
taaggaaaag agagatcaga ctttc                                          25

<210>   1167
<211>   25
<212>   DNA
<213>   HERV-K

<400>   1167
aaaagagaga tcagactttc actgt                                             25

<210>   1168
<211>   25
<212>   DNA
<213>   HERV-K

<400>   1168
ctttcactgt gtctatgtag aaaag                                             25

<210>   1169
<211>   25
<212>   DNA
<213>   HERV-K

<400>   1169
actaagaaaa attgttttgc cttga                                             25

<210>   1170
<211>   25
<212>   DNA
<213>   HERV-K

<400>   1170
tgctcacgga aacatgtgct gtaag                                             25

<210>   1171
<211>   25
<212>   DNA
<213>   HERV-K

<400>   1171
acggaaacat gtgctgtaag gttta                                             25

<210>   1172
<211>   25
<212>   DNA
<213>   HERV-K

<400>   1172
aacatgtgct gtaaggttta aggga                                             25

<210>   1173
<211>   25
<212>   DNA
<213>   HERV-K

<400>   1173
gtgctgtaag gtttaaggga tctag                                             25

<210>   1174
<211>   25
<212>   DNA
<213>   HERV-K

<400>   1174
tgcaggatgt accttgttaa caata                                          25

<210>   1175
<211>   25
<212>   DNA
<213>   HERV-K

<400>   1175
accttgttaa caatatgttt gcagg                                          25

<210>   1176
<211>   25
<212>   DNA
<213>   HERV-K

<400>   1176
caatatgttt gcaggcagta tgttt                                          25

<210>   1177
<211>   25
<212>   DNA
<213>   HERV-K

<400>   1177
tgtttgcagg cagtatgttt ggtaa                                          25

<210>   1178
<211>   25
<212>   DNA
<213>   HERV-K

<400>   1178
gcaggcagta tgtttggtaa aagtc                                          25

<210>   1179
<211>   25
<212>   DNA
<213>   HERV-K

<400>   1179
attaaccagg ggctcaatgc actgt                                          25

<210>   1180
<211>   25
<212>   DNA
<213>   HERV-K

<400>   1180
ggctcaatgc actgtggaaa gccac                                          25

<210>   1181
<211>   25
<212>   DNA
<213>   HERV-K

<400>   1181
aatgcactgt ggaaagccac aggaa                                          25

<210>   1182
<211>   25

<212>   DNA
<213>   HERV-K

<400>   1182
ggaaagccac aggaacctct gccca                                          25

<210>   1183
<211>   25
<212>   DNA
<213>   HERV-K

<400>   1183
gccacaggaa cctctgccca agaaa                                          25

<210>   1184
<211>   25
<212>   DNA
<213>   HERV-K

<400>   1184
aggaacctct gcccaagaaa gcctg                                          25

<210>   1185
<211>   2016
<212>   DNA
<213>   HERV-K

<400>   1185
agcttacaaa acaaaatggg gcaaactgaa agtaaatatg cctcttatct cagctttatt   60
aaaattcttt taaaaagagg gggagttaga gtatctacaa aaaatctaat caagctattt   120
caaataatag aacaattttg cccatggttt ccagaacaag gaactttaga tctaaaagat   180
tggaaaagaa ttggcgagga actaaaacaa gcaggtagaa agggtaatat cattccactt   240
acagtatgga atgattgggc cattattaaa gcagctttag aaccatttca aacaaaagaa   300
gatagcgttt cagtttctga tgcccctgga agctgtgtaa tagattgtaa tgaaaagaca   360
gggagaaaat cccagaaaga aacagaaagt ttacattgcg aatatgtaac agagccagta   420
atggctcagt caacgcaaaa tgttgactat aatcaattac agggggtgat atatcctgaa   480
acgttaaaat tagaaggaaa aggtccagaa ttagtggggc catcagagtc taaaccacga   540
gggccaagtc ctcttccagc aggtcaggtg cccgtaacat tacaacctca aacgcaggtt   600
aaagaaaata agacccaacc gccagtagct tatcaatact ggccgccggc tgaacttcag   660
tatctgccac ccccagaaag tcagtatgga tatccaggaa tgcccccagc actacagggc   720
agggcgccat atcctcagcc gcccactgtg agacttaatc ctacagcatc acgtagtgga   780
caaggtggta cactgcacgc agtcattgat gaagccagaa aacagggaga tcttgaggca   840
tggcggttcc tggtaatttt acaactggta caggccgggg aagagactca agtaggagcg   900
cctgcccgag ctgagactag atgtgaacct ttcaccatga aaatgttaaa agatataaag   960
gaaggagtta aacaatatgg atccaactcc ccttatataa gaacattatt agattccatt   1020
gctcatggaa atagacttac tccttatgac tgggaaagtt tggccaaatc ttccctttca   1080
tcctctcagt atctacagtt taaaacctgg tggattgatg gagtacaaga acaggtacga   1140
aaaaatcagg ctactaagcc cactgttaat atagacgcag accaattgtt aggaacaggt   1200
ccaaattgga gcaccattaa ccaacaatca gtgatgcaga atgaggctat tgaacaagta   1260
agggctattt gcctcagggc ctggggaaaa attcaggacc caggaacagc tttccctatt   1320
aattcaatta gacaaggctc taaagagcca tatcctgact ttgtggcaag attacaagat   1380
gctgctcaaa agtctattac agatgacaat gcccgaaaag ttattgtaga attaatggcc   1440
tatgaaaatg caaatccaga atgtcagtcg gccataaagc cattaaaagg aaaagttcca   1500
gcaggagttg atgtaattac agaatatgtg aaggcttgtg atgggattgg aggagctatg   1560
cataaggcaa tgctaatggc tcaagcaatg agggggctca ctctaggagg acaagttaga   1620
acatttggga aaaaatgtta taattgtggt caaatcggtc atctgaaaag gagttgccca   1680
gtcttaaata aacagaatat aataaatcaa gctattacag caaaaaataa aaagccatct   1740
ggcctgtgtc caaaatgtgg aaaaggaaaa cattgggcca atcaatgtca ttctaaattt   1800
gataaggatg ggcaaccatt gtcgggaaac aggaagaggg gccagcctca ggccccccaa   1860
caaactgggg cattcccagt tcaactgttt gttcctcagg gttttcaagg acaacaaccc   1920
ctacagaaaa taccaccact tcagggagtc agccaattac aacaatccaa cagctgtccc   1980

gcgccacagc aggcagcacc gcagtagtaa gtcgac                                    2016

<210>   1186
<211>   663
<212>   PRT
<213>   HERV-K

<400>   1186
Met Gly Gln Thr Glu Ser Lys Tyr Ala Ser Tyr Leu Ser Phe Ile Lys
1               5                   10                  15

Ile Leu Leu Lys Arg Gly Gly Val Arg Val Ser Thr Lys Asn Leu Ile
            20                  25                  30

Lys Leu Phe Gln Ile Ile Glu Gln Phe Cys Pro Trp Phe Pro Glu Gln
        35                  40                  45

Gly Thr Leu Asp Leu Lys Asp Trp Lys Arg Ile Gly Glu Glu Leu Lys
    50                  55                  60

Gln Ala Gly Arg Lys Gly Asn Ile Ile Pro Leu Thr Val Trp Asn Asp
65                  70                  75                  80

Trp Ala Ile Ile Lys Ala Ala Leu Glu Pro Phe Gln Thr Lys Glu Asp
                85                  90                  95

Ser Val Ser Val Ser Asp Ala Pro Gly Ser Cys Val Ile Asp Cys Asn
            100                 105                 110

Glu Lys Thr Gly Arg Lys Ser Gln Lys Glu Thr Glu Ser Leu His Cys
        115                 120                 125

Glu Tyr Val Thr Glu Pro Val Met Ala Gln Ser Thr Gln Asn Val Asp
    130                 135                 140

Tyr Asn Gln Leu Gln Gly Val Ile Tyr Pro Glu Thr Leu Lys Leu Glu
145                 150                 155                 160

Gly Lys Gly Pro Glu Leu Val Gly Pro Ser Glu Ser Lys Pro Arg Gly
                165                 170                 175

Pro Ser Pro Leu Pro Ala Gly Gln Val Pro Val Thr Leu Gln Pro Gln
            180                 185                 190

Thr Gln Val Lys Glu Asn Lys Thr Gln Pro Pro Val Ala Tyr Gln Tyr
        195                 200                 205

Trp Pro Pro Ala Glu Leu Gln Tyr Leu Pro Pro Pro Glu Ser Gln Tyr
    210                 215                 220

Gly Tyr Pro Gly Met Pro Pro Ala Leu Gln Gly Arg Ala Pro Tyr Pro
225                 230                 235                 240

Gln Pro Pro Thr Val Arg Leu Asn Pro Thr Ala Ser Arg Ser Gly Gln
                245                 250                 255

Gly Gly Thr Leu His Ala Val Ile Asp Glu Ala Arg Lys Gln Gly Asp
            260                 265                 270

Leu Glu Ala Trp Arg Phe Leu Val Ile Leu Gln Leu Val Gln Ala Gly
        275                 280                 285

Glu Glu Thr Gln Val Gly Ala Pro Ala Arg Ala Glu Thr Arg Cys Glu
    290                 295                 300

Pro Phe Thr Met Lys Met Leu Lys Asp Ile Lys Glu Gly Val Lys Gln
305                 310                 315                 320

Tyr Gly Ser Asn Ser Pro Tyr Ile Arg Thr Leu Leu Asp Ser Ile Ala
                325                 330                 335

His Gly Asn Arg Leu Thr Pro Tyr Asp Trp Glu Ser Leu Ala Lys Ser
            340                 345                 350

Ser Leu Ser Ser Ser Gln Tyr Leu Gln Phe Lys Thr Trp Trp Ile Asp
        355                 360                 365

Gly Val Gln Glu Gln Val Arg Lys Asn Gln Ala Thr Lys Pro Thr Val
    370                 375                 380

Asn Ile Asp Ala Asp Gln Leu Leu Gly Thr Gly Pro Asn Trp Ser Thr
385                 390                 395                 400

Ile Asn Gln Gln Ser Val Met Gln Asn Glu Ala Ile Glu Gln Val Arg
                405                 410                 415

Ala Ile Cys Leu Arg Ala Trp Gly Lys Ile Gln Asp Pro Gly Thr Ala
            420                 425                 430

Phe Pro Ile Asn Ser Ile Arg Gln Gly Ser Lys Glu Pro Tyr Pro Asp
        435                 440                 445

Phe Val Ala Arg Leu Gln Asp Ala Ala Gln Lys Ser Ile Thr Asp Asp
    450                 455                 460

Asn Ala Arg Lys Val Ile Val Glu Leu Met Ala Tyr Glu Asn Ala Asn
465                 470                 475                 480

Pro Glu Cys Gln Ser Ala Ile Lys Pro Leu Lys Gly Lys Val Pro Ala
                485                 490                 495

Gly Val Asp Val Ile Thr Glu Tyr Val Lys Ala Cys Asp Gly Ile Gly
            500                 505                 510

Gly Ala Met His Lys Ala Met Leu Met Ala Gln Ala Met Arg Gly Leu
        515                 520                 525

Thr Leu Gly Gly Gln Val Arg Thr Phe Gly Lys Lys Cys Tyr Asn Cys
    530                 535                 540

Gly Gln Ile Gly His Leu Lys Arg Ser Cys Pro Val Leu Asn Lys Gln
545                 550                 555                 560

Asn Ile Ile Asn Gln Ala Ile Thr Ala Lys Asn Lys Lys Pro Ser Gly
                565                 570                 575

Leu Cys Pro Lys Cys Gly Lys Gly Lys His Trp Ala Asn Gln Cys His
            580                 585                 590

Ser Lys Phe Asp Lys Asp Gly Gln Pro Leu Ser Gly Asn Arg Lys Arg
        595                 600                 605

Gly Gln Pro Gln Ala Pro Gln Gln Thr Gly Ala Phe Pro Val Gln Leu
    610                 615                 620

Phe Val Pro Gln Gly Phe Gln Gly Gln Gln Pro Leu Gln Lys Ile Pro
625                 630                 635                 640

Pro Leu Gln Gly Val Ser Gln Leu Gln Gln Ser Asn Ser Cys Pro Ala
                645                 650                 655

Pro Gln Gln Ala Ala Pro Gln
            660

<210>   1187
<211>   2172
<212>   DNA
<213>   HERV-K

<400>   1187
agcttacaaa acaaaatggg gcaaactgaa agtaaatatg cctcttatct cagctttatt    60
aaaattcttt taagaagagg gggagttaga gcttctacag aaaatctaat tacgctattt   120
caaacaatag aacaattctg cccatggttt ccagaacagg gaactttaga tctaaaagat   180
tgggaaaaaa ttggcaaaga attaaaacaa gcaaataggg aaggtaaaat catcccactt   240
acagtatgga atgattgggc cattattaaa gcaactttag aaccatttca aacaggagaa   300
gatattgttt cagtttctga tgcccctaaa agctgtgtaa cagattgtga agaagaggca   360
gggacagaat cccagcaagg aacggaaagt tcacattgta aatatgtagc agagtctgta   420
atggctcagt caacgcaaaa tgttgactac agtcaattac aggagataat ataccctgaa   480
tcatcaaaat tgggggaagg aggtccagaa tcattggggc catcagagcc taaaccacga   540
tcgccatcaa ctcctcctcc cgtggttcag atgcctgtaa cattacaacc tcaaacgcag   600
gttagacaag cacaaacccc aagagaaaat caagtagaaa gggacagagt ctctatcccg   660
gcaatgccaa ctcagataca gtatccacaa tatcagccgg tagaaaataa gacccaaccg   720
ctggtagttt atcaataccg gctgccaacc gagcttcagt atcggcctcc ttcagaggtt   780
caatacagac ctcaagcggt gtgtcctgtg ccaaatagca cggcaccata ccagcaaccc   840
acagcgatgg cgtctaattc accagcaaca caggacgcgg cgctgtatcc tcagccgccc   900
actgtgagac ttaatcctac agcatcacgt agtggacagg gtggtgcact gcatgcagtc   960
attgatgaag ccagaaaaca gggcgatctt gaggcatggc ggttcctggt aattttacaa  1020
ctggtacagg ccggggaaga gactcaagta ggagcgcctg cccgagctga gactagatgt  1080
gaacctttca ccatgaaaat gttaaaagat ataaaggaag gagttaaaca atatggatcc  1140
aactcccctt atataagaac attattagat tccattgctc atggaaatag acttactcct  1200
tatgactggg aaattttggc caaatcttcc ctttcatcct ctcagtatct acagtttaaa  1260
acctggtgga ttgatggagt acaagaacag gtacgaaaaa atcaggctac taagcccact  1320
gttaatatag acgcagacca attgttagga acaggtccaa attggagcac cattaaccaa  1380
caatcagtga tgcagaatga ggctattgaa caagtaaggg ctatttgcct cagggcctgg  1440
ggaaaaattc aggacccagg aacagctttc cctattaatt caattagaca aggctctaaa  1500
gagccatatc ctgactttgt ggcaagatta caagatgctg ctcaaaagtc tattacagat  1560
gacaatgccc gaaaagttat tgtagaatta atggcctatg aaaatgcaaa tccagaatgt  1620
cagtcggcca taaagccatt aaaaggaaaa gttccagcag gagttgatgt aattacagaa  1680
tatgtgaagg cttgtgatgg gattggagga gctatgcata aggcaatgct aatggctcaa  1740
gcaatgaggg ggctcactct aggaggacaa gttagaacat ttgggaaaaa atgttataat  1800
tgtggtcaaa tcggtcatct gaaaaggagt tgcccagtct taaataaaca gaatataata  1860
aatcaagcta ttacagcaaa aaataaaaag ccatctggcc tgtgtccaaa atgtggaaaa  1920
ggaaaacatt gggccaatca atgtcattct aaatttgata aggatgggca accattgtcg  1980
ggaaacagga agaggggcca gcctcaggcc ccccaacaaa ctggggcatt cccagttcaa  2040
ctgtttgttc ctcagggttt tcaaggacaa caacccctac agaaaatacc accacttcag  2100
ggagtcagcc aattacaaca atccaacagc tgtcccgcgc cacagcaggc agcaccgcag  2160
tagtaagtcg ac                                                      2172

<210>   1188
<211>   715
<212>   PRT
<213>   HERV-K

<400>   1188
Met Gly Gln Thr Glu Ser Lys Tyr Ala Ser Tyr Leu Ser Phe Ile Lys
1               5                   10                  15

Ile Leu Leu Arg Arg Gly Gly Val Arg Ala Ser Thr Glu Asn Leu Ile
            20                  25                  30

Thr Leu Phe Gln Thr Ile Glu Gln Phe Cys Pro Trp Phe Pro Glu Gln
        35                  40                  45

Gly Thr Leu Asp Leu Lys Asp Trp Glu Lys Ile Gly Lys Glu Leu Lys
    50                  55                  60

Gln Ala Asn Arg Glu Gly Lys Ile Ile Pro Leu Thr Val Trp Asn Asp
65                  70                  75                  80

Trp Ala Ile Ile Lys Ala Thr Leu Glu Pro Phe Gln Thr Gly Glu Asp
                85                  90                  95

Ile Val Ser Val Ser Asp Ala Pro Lys Ser Cys Val Thr Asp Cys Glu
            100                 105                 110

Glu Glu Ala Gly Thr Glu Ser Gln Gln Gly Thr Glu Ser Ser His Cys
        115                 120                 125

Lys Tyr Val Ala Glu Ser Val Met Ala Gln Ser Thr Gln Asn Val Asp
    130                 135                 140

Tyr Ser Gln Leu Gln Glu Ile Ile Tyr Pro Glu Ser Ser Lys Leu Gly
145                 150                 155                 160

Glu Gly Gly Pro Glu Ser Leu Gly Pro Ser Glu Pro Lys Pro Arg Ser
                165                 170                 175

Pro Ser Thr Pro Pro Pro Val Val Gln Met Pro Val Thr Leu Gln Pro
            180                 185                 190

Gln Thr Gln Val Arg Gln Ala Gln Thr Pro Arg Glu Asn Gln Val Glu
        195                 200                 205

Arg Asp Arg Val Ser Ile Pro Ala Met Pro Thr Gln Ile Gln Tyr Pro
    210                 215                 220

Gln Tyr Gln Pro Val Glu Asn Lys Thr Gln Pro Leu Val Val Tyr Gln
225                 230                 235                 240

Tyr Arg Leu Pro Thr Glu Leu Gln Tyr Arg Pro Pro Ser Glu Val Gln
                245                 250                 255

Tyr Arg Pro Gln Ala Val Cys Pro Val Pro Asn Ser Thr Ala Pro Tyr
            260                 265                 270

Gln Gln Pro Thr Ala Met Ala Ser Asn Ser Pro Ala Thr Gln Asp Ala
        275                 280                 285

Ala Leu Tyr Pro Gln Pro Pro Thr Val Arg Leu Asn Pro Thr Ala Ser
    290                 295                 300

Arg Ser Gly Gln Gly Gly Ala Leu His Ala Val Ile Asp Glu Ala Arg
305                 310                 315                 320

Lys Gln Gly Asp Leu Glu Ala Trp Arg Phe Leu Val Ile Leu Gln Leu
                325                 330                 335

Val Gln Ala Gly Glu Glu Thr Gln Val Gly Ala Pro Ala Arg Ala Glu
            340                 345                 350

Thr Arg Cys Glu Pro Phe Thr Met Lys Met Leu Lys Asp Ile Lys Glu
        355                 360                 365

Gly Val Lys Gln Tyr Gly Ser Asn Ser Pro Tyr Ile Arg Thr Leu Leu
    370                 375                 380

Asp Ser Ile Ala His Gly Asn Arg Leu Thr Pro Tyr Asp Trp Glu Ile
385                 390                 395                 400

Leu Ala Lys Ser Ser Leu Ser Ser Ser Gln Tyr Leu Gln Phe Lys Thr
                405                 410                 415

Trp Trp Ile Asp Gly Val Gln Glu Gln Val Arg Lys Asn Gln Ala Thr
            420                 425                 430

Lys Pro Thr Val Asn Ile Asp Ala Asp Gln Leu Leu Gly Thr Gly Pro
        435                 440                 445

Asn Trp Ser Thr Ile Asn Gln Gln Ser Val Met Gln Asn Glu Ala Ile
    450                 455                 460

Glu Gln Val Arg Ala Ile Cys Leu Arg Ala Trp Gly Lys Ile Gln Asp
465                 470                 475                 480

Pro Gly Thr Ala Phe Pro Ile Asn Ser Ile Arg Gln Gly Ser Lys Glu
                485                 490                 495

Pro Tyr Pro Asp Phe Val Ala Arg Leu Gln Asp Ala Ala Gln Lys Ser
            500                 505                 510

Ile Thr Asp Asp Asn Ala Arg Lys Val Ile Val Glu Leu Met Ala Tyr
        515                 520                 525

Glu Asn Ala Asn Pro Glu Cys Gln Ser Ala Ile Lys Pro Leu Lys Gly
    530                 535                 540

Lys Val Pro Ala Gly Val Asp Val Ile Thr Glu Tyr Val Lys Ala Cys
545                 550                 555                 560

Asp Gly Ile Gly Gly Ala Met His Lys Ala Met Leu Met Ala Gln Ala
                565                 570                 575

Met Arg Gly Leu Thr Leu Gly Gly Gln Val Arg Thr Phe Gly Lys Lys
            580                 585                 590

Cys Tyr Asn Cys Gly Gln Ile Gly His Leu Lys Arg Ser Cys Pro Val
        595                 600                 605

Leu Asn Lys Gln Asn Ile Ile Asn Gln Ala Ile Thr Ala Lys Asn Lys
    610                 615                 620

Lys Pro Ser Gly Leu Cys Pro Lys Cys Gly Lys Gly Lys His Trp Ala
625                 630                 635                 640

Asn Gln Cys His Ser Lys Phe Asp Lys Asp Gly Gln Pro Leu Ser Gly

645                 650                 655

Asn Arg Lys Arg Gly Gln Pro Gln Ala Pro Gln Gln Thr Gly Ala Phe
        660                 665                 670

Pro Val Gln Leu Phe Val Pro Gln Gly Phe Gln Gly Gln Gln Pro Leu
        675                 680                 685

Gln Lys Ile Pro Pro Leu Gln Gly Val Ser Gln Leu Gln Gln Ser Asn
    690                 695                 700

Ser Cys Pro Ala Pro Gln Gln Ala Ala Pro Gln
705                 710                 715

<210>   1189
<211>   14
<212>   PRT
<213>   Artificial Sequence

<220>
<223>   V5 tag

<400>   1189
Gly Lys Pro Ile Pro Asn Pro Leu Leu Gly Leu Asp Ser Thr
1               5                   10

<210>   1190
<211>   962
<212>   DNA
<213>   HERV-K

<400>   1190
tgtggggaaa agcaagagag atcagattgt cactgtatct gtgtagaaag aagtagacat    60
gggagactcc attttgttat gtactaagaa aaattcttct gccttgagat tctgtgacct   120
tacccccaac cccgtgctct ctgaaacatg tgctgtgtca aactcagggt taaatggatt   180
aagggcggtg caggatgtgc tttgttaaac agatgcttga aggcagcatg ctccttaaga   240
gtcatcacca ctccctaatc tcaagtaccc agggacacaa acactgcgga aggccgcagg   300
gacctctgcc taggaaagcc aggtattgtc caaggtttct ccccatgtga tagtctgaaa   360
tatggcctcg tgggaaggga aagacctgac cgtcccccag cccgacaccc gtaaagggtc   420
tgtgctgagg aggattagta aaagaggaag gcatgcctct tgcagttgag acaagaggaa   480
ggcatctgtc tcctgcccgt ccctgggcaa tggaatgtct cggtataaaa ccggattgta   540
cgttccatct actgagatag ggaaaaaccg ccttagggct ggaggtggga cctgcgggca   600
gcaatactgc tttttaaagc attgagatgt ttatgtgtat gcatatctaa aagcacagca   660
cttaatcctt taccttgtct atgatgcaaa gatctttgtt cacgtgtttg tctgctgacc   720
ctctccccac tattgtcttg tgaccctgac acatccccct ctcggagaaa cacccacgaa   780
tgaccaataa atactaaagg gaactcagag gctggcggga tcctccatat gctgaacgct   840
ggttccccgg gcccccttat ttctttctct acactttgtc tctgtgtctt tttctttcct   900
aagtctctcg ttccacctta cgagaaacac ccacaggtgt ggaggggcaa cccaccccta   960
ca                                                                  962

<210>   1191
<211>   364
<212>   DNA
<213>   HERV-K

<400>   1191
gggtgaaggt actctacagt gtggtcattg aggacaagtt gacgagagag tcccaagtac    60
gtccacggtc agccttgcga catttaaagt tctacaatga actcactgga gatgcaaaga   120
aaagtgtgga gatggagaca ccccaatcga ctcgccagtc tacaggtgta tccagcagct   180
ccaaagagac agcaaccagc aagaatgggc catagtgacg atggtggttt tgtcaaaaag   240
aaaagggggg gatatgtaag gaaaagagag atcagacttt cactgtgtct atgtagaaaa   300

ggaagacata agaaactcca ttttgttctg tactaagaaa aattgttttg ccttgagatg 360
ctgt 364

<210> 1192
<211> 25
<212> DNA
<213> HERV-K

<400> 1192
ctcgttccac ctgaggagaa atgcc 25

<210> 1193
<211> 20
<212> DNA
<213> HERV-K

<400> 1193
gaggcgcagg ccactccatc 20

<210> 1194
<211> 27
<212> DNA
<213> HERV-K

<400> 1194
cttgtcctca atgaccacac tgtagag 27

<210> 1195
<211> 24
<212> DNA
<213> HERV-K

<400> 1195
agagtacctt cacccacaag gctc 24

<210> 1196
<211> 1010
<212> DNA
<213> HERV-K

<400> 1196
tgtagggaaa agaaagagag atcacactgt tactgtgtct atgtagaaaa aggaagacat 60
aagaaactcc attttgatct gtactaagaa aaattcttct gctttgaaat gctattaatc 120
tgtaacccta gccccaaccc tgtgctcaca gaaacatgcg ctgtattgac tcaaggttaa 180
tggatttagg gctgtgcagg atgtgctttg ttaacaatgt gtttgaaggc agtatgcttg 240
gtaaaggtca tcgccattct ccagtcttga gtacccaggg acacaatgca ctgtggaaag 300
ccatggggac ctctgcccaa gaaagcctgg gtgttgtcca ggcttcccca cactgagaca 360
gcctgagatg tggcctcgtt ggaagggaaa gaccttacat tatagtcccc cagccggaca 420
cccataaaag gtctgtgctg aggaggatta ctgaaagagg aaggcctctt tgcagttaag 480
aggaaagcat ctgtctcatg atcccctggg aatggaatgt cttggtgtaa aacctgatcg 540
tacattctat ttactgagat aggagaaaac cgccctatgg ctggaggtga gacatgctgg 600
tggcaatacc gatctttact gcacggcaat actgatcttt actgcactga gatgtttatg 660
taaagttaaa cataaatcta gcctacgtgc acattcaggc atagcacctt tccttaaact 720
tatttatgac acagagtctt ttgttcacgt gttttcctgt tgaccctctc tccaccatta 780
ccctatagtc ctgccacatc cccctcactg agatagtaga gataatgatc aataaatact 840
gagggaattc agaaaccagt gccggtgcag gtcctcactt gctgagtgcc ggtcccctgg 900
gcccactttt cttcctctat gctttacctc tgtgtcttat ttcttttctc agtctctcgt 960
ctccaccttg cgagaaatac ccacaggtgt ggaggggctg gcccccttca 1010

<210> 1197
<211> 11100

<212>   RNA
<213>   HERV-K

<400>   1197
uuauguauau gcacaucaaa agcacagcac uuuuuucuuu accuuguuua ugaugcagag   60
acauuuguuc acauguuuuc cugcuggccc ucuccccacu auuacccuau uguccugcca   120
caucccccuc uccgagaugg uagagauaau gaucaauaaa uacugaggga acucagagac   180
cggugcggcg cggguccucc auaugcugag cgccgguccc cugggcccac uuuucuuucu   240
cuauacuuug ucucuguugu cuuucuuuuc ucaagucucu cguuccaccu gaggagaaau   300
gcccacagcu guggaggcgc aggccacucc aucuggugcc caacguggau gcuuuucucu   360
agggugaagg gacucucgag uguggucauu gaggacaagu caacgagaga uucccgagua   420
cgucuacagu gagccuugug guaagcuugg gcgcucggaa gaagccaggg uuaauggggc   480
aaacuaaaag uaaagucucu cauuccaccu gaugagaaac acccagaggu guggaggggc   540
aggccacccc uucaggguag gguccccucc augcagacca uagagcacag gugugcccca   600
aagaggagca gagagaagga gggagagggc ccacgagaga cuuggaaaug aauggcagga   660
uuuuaggcgc uggacuuggg uucggggcac cuggccuuuc cuuguguauu ucuccuacug   720
ucugccuaac uauuuaauac aauaaaagaa aaccagcccc ugguucuugu gguguuucca   780
cccucccggg uccccgcugg cugccuggcu uccucccgca gcuccugcug uguguguaug   840
ugugugugug ugcacaucug uggggcguau guguguucgu cuuuguaauu gaggcugcag   900
aguggagaga gcagggguuu ucucugggga cccagagaga aggaggcguu uucaccacag   960
ccgaacaggg caggacccca gcacccggga cccagcggga cuuugccaag gggauggacc   1020
uggcugggcc acgcggcugu uuguguaggg aaaagaaaga gagaucacac uguuacugug   1080
ucuauguaga aaaggaagac auaaacucca uuuugagcug uacuaagaaa aauuauuuug   1140
ccuugaccug cuguuaaccu guaacuguag ccccaacccu gugcucaaag aaacaugugc   1200
uguauggaau caagguuuaa gggaucaagg gcuguacagg augugccuug uuaacaaugu   1260
guuuacaggc aguaugcuug guaaaaguca ucgccauucu ccauucucca uuaaucaggg   1320
gcacgaugca cugcggaaag ccacagggac cucugcccga gaaagccugg guauugucca   1380
aggcuucccc ccacugagac agccugagau acggccucgu gggaagggaa agaccugacc   1440
gucccccagc ccgacacccg uaaagggucu gugcugagga ggauuaguaa aaggggaagg   1500
ccucuugcag uugagauaag aggaaggccu ccgucuccug cauguccuug ggaauggaau   1560
gucuuggugu aaaacccgau aguacauucc uucuauucug agagaagaaa accacccugu   1620
ggcuggaggu gagauaugcu agcggcaaug cugcucuguu acucuuugcu acacugagau   1680
guuugggugg agagaagcau aaaucuggcc uaugugcaca ucugggcaca gaaccucccc   1740
uugaacuugu gacacagauu ccuuuguuca cauguuuucc ugcugaccuu cuccccacua   1800
ucgcccuguu cucccaccgc auuccccuug cugagauagu gaaaauagua aucuguagau   1860
accaagggaa cucagagacc auggccggug cacauccucc guacgcugag cgcugguccc   1920
cugggcccau uguucuuucu cuauacuuug ucucuguguc uuauuucuuu ccucagucuc   1980
ucaucccucc ugacgagaaa uacccacagg uguggagggg cuggcccccu ucaucugaug   2040
cccaaugugg gugccuuucu cuagggugaa gguacucuac agugugguca uugaggacaa   2100
guugacgaga gagucccaag uacguccacg gucagccuug cgguaagcuu gugugcuuag   2160
aggaacccag gguaacgaug gggcaaacug aaaguaaaua ugccucuuau cucagcuuua   2220
uuaaaauucu uuuaagaaga gggggaguua gagcuucuac agaaaaucua auuacgcuau   2280
uucaaacaau agaacaauuc ugcccauggu uuccagaaca gggaacuuua gaucuaaaag   2340
auuggaaaaa aauuggcaaa gaauuaaaac aagcaaauag ggaagguaaa aucaucccac   2400
uuacaguaug gaaugauugg gccauuauua aagcaacuuu agaaccauuu caaacaggag   2460
aagauauugu uucaguuucu gaugccccua aaagcugugu aacagauugu gaagaagagg   2520
cagggacaga aucccagcaa ggaacggaaa guucacauug uaaauaugua gcagagucug   2580
uaauggcuca gucaacgcaa aauguugacu acagucaauu acaggagaua auauacccug   2640
aaucaucaaa auugggggaa ggagguccag aaucauuggg gccaucagag ccuaaaccac   2700
gaucgccauc aacuccuccu cccgugguuc agaugccugu aacauuacaa ccucaaacgc   2760
agguuagaca agcacaaacc ccaagagaaa aucaaguaga aagggacaga gucucuaucc   2820
cggcaaugcc aacucagaua caguauccac aauaucagcc gguagaaaau aagacccaac   2880
cgcugguagu uuaucaauac cggcugccaa ccgagcuuca guaucggccu ccuucagagg   2940
uucaauacag accucaagcg guguguccug ugccaaauag cacggcacca uaccagcaac   3000
ccacagcgau ggcgucuaau ucaccagcaa cacaggacgc ggcgcuguau ccucagccgc   3060
ccacugugag acuuaauccu acagcaucac guaguggaca ggguggugca cugcaugcag   3120
ucauugauga agccagaaaa cagggcgauc uugaggcaug gcgguuccug guaauuuuac   3180
aacugguaca ggccggggaa gagacucaag uaggagcgcc ugcccgagcu gagacuagau   3240
gugaaccuuu caccaugaaa auguuaaaag auauaaagga aggaguuaaa caauauggau   3300
ccaacucccc uuauauaaga acauuauuag auuccauugc ucauggaaau agacuuacuc   3360
cuuaugacug ggaaauuuug gccaaaucuu cccuuucauc cucucaguau cuacaguuua   3420

aaaccuggug gauugaugga guacaagaac agguacgaaa aaaucaggcu acuaagccca 3480
cuguuaauau agacgcagac caauuguuag gaacaggucc aaauuggagc accauuaacc 3540
aacaaucagu gaugcagaau gaggcuauug aacaaguaag ggcuauuugc cucagggccu 3600
ggggaaaaau ucaggaccca ggaacagcuu ucccuauuaa uucaauuaga caaggcucua 3660
aagagccaua uccugacuuu guggcaagau uacaagaugc ugcucaaaag ucuauuacag 3720
augacaaugc ccgaaaaguu auuguagaau uaauggccua ugaaaaugca aauccagaau 3780
gucagucggc cauaaagcca uuaaaaggaa aaguuccagc aggaguugau guaauuacag 3840
aauaugugaa ggcuugugau gggauuggag gagcuaugca uaaggcaaug cuaauggcuc 3900
aagcaaugag ggggcucacu cuaggaggac aaguuagaac auuugggaaa aaauguuaua 3960
auugugguca aaucggucau cugaaaagga guugcccagg cuuaaauaaa cagaauauaa 4020
uaaaucaagc uauuacagca aaaaauaaaa agccaucugg ccugugucca aaauguggaa 4080
aagcaaaaca uugggccaau caaugucauu cuaaauuuga uaaagauggg caaccauugu 4140
cuggaaacag gaagaggggc cagccucagg ccccccaaca aacuggggca uucccaguua 4200
aacuguuugu uccucagggu uuucaaggac aacaaccccu acagaaaaua ccaccacuuc 4260
agggagucag ccaauuacaa caauccaaca gcugucccgc gccacagcag gcagcaccgc 4320
aguagauuua uguuccaccc aaauggucuu uuuacucccu ggaaagcccc cacaaaagau 4380
uccuagaggg guauauggcc cgcugccaga agggagggua ggccuuugag ggagaucaag 4440
ucuaaauuug aagggagucc aaauucauac ugggguaauu uauucagauu auaaaggggg 4500
aauucaguua gugaucagcu ccacuguucc ccggagugcc aauccaggug auagaauugc 4560
ucaauuacug cuuuugccuu auguuaaaau uggggaaaac aaaaaggaaa gaacaggagg 4620
guuuggaagu accaacccug caggaaaagc ugcuuauugg gcuaaucagg ucucagagga 4680
uagacccgug uguacaguca cuauucaggg aaagaguuug aaggauuagu ggauacccag 4740
gcugauguuu cugucaucgg cauagguacu gccucagaag uguaucaaag ugccaugauu 4800
uuacauuguc caggaucuga uaaucaagaa aguacgguuc agccugugau cacuucauuc 4860
caaucaauuu auggggccga gacuuguuac aacaauggca ugcagagauu acuaucccag 4920
ccucccuaua cagccccagg aauaaaaaaa ucaugacuaa aaugggauag cucccuaaaa 4980
agggacuagg aaagaagucc caauugaggc ugaaaaaaau caaaaaagaa aaggaauagg 5040
gcauccuuuu uaggagcggu cacuguagag ccuccaaaac ccauuccauu aacuuggggg 5100
aaaaaaaaac aacuguaugg uaaaucagca gcgcuuccaa aacaaaaacu ggaggcuuua 5160
cauuuauuag caaagaaaca auuagaaaaa ggacauugag ccuucauuuu cgccuuggaa 5220
uucuguuugu aauucagaaa aaauccggca gauggcguau aaugccguaa uucaacccau 5280
gggggcucuc ccaccccggu ugcccucucc agccaugguc cccuuuaauu auaauugauc 5340
ugaaggauug cuuuuuuacc auuccucugg caaaacagga uuuugaaaaa uuugcuuuua 5400
ccacaccagc cuaaauaaua aagaaccagc caccagguuu caguggaaag uauugccuca 5460
gggaaugcuu aauaguucaa cuauuuguca gcucaagcuc ugcaaccagu uagagacaag 5520
uuuucagacu guuacaucgu ucacuauguu gauauuuugu gugcugcaga aacgagagac 5580
aaauuaauug accguuacac auuucugcag acagagguug ccaacgcggg acugacaaua 5640
acaucugaua agauucaaac cucuacuccu uuccguuacu ugggaaugca gguagaggaa 5700
aggaaaauua aaccacaaaa aauagaaaua agaaaagaca cauuaaaagc auuaaaugag 5760
uuucaaaagu ugcuaggaga uacuaauugg auuuggagau auuaauugga uuuggccaac 5820
ucuaggcauu ccuacuuaug ccaugucaaa uuuguucucu uucuuaagag gggacucgga 5880
auuaaauagu gaaagaacgu uaacuccaga ggcaacuaaa gaaauuaaau uaauugaaga 5940
aaaaauucgg ucagcacaag uaaauagaau agaucacuug gccccacucc aaauuuugau 6000
uuuugcuacu gcacauuccc uaacaggcau cauuguucaa aauacagauc uuguggagug 6060
guccuucccu ccucacagua caauuaagac uuuuacauug uacuuggauc aaauggcuac 6120
auuaauuggu cagggaagau uaugaauaau aacauugugu ggaaaugacc cagauaaaau 6180
cacuguuccu uucaacaagc aacagguuag acaagccuuu aucaauucug gugcauggca 6240
gauuggucuu gccgauuuug ugggaauuau ugacaaucgu uaccccaaaa caaaaaucuu 6300
ccaguuuuua aaauugacua cuuggauuuu accuaaaguu accaaacaua agccuuuaaa 6360
aaaugcucug gcaguguuua cugaugguuc cagcaaugga aaaguggcuu acaccgggcc 6420
aaaagaauga gucaucaaaa cucaguauca cuugacucaa agagcagagu ugguugccgu 6480
cauuacagug uuaacaagau uuuaaucagu cuauuaacau uguaucagau ucugcauaug 6540
uaguacaggc uacaaaggau auugagagag cccuaaucaa auacauuaug gaugaucagu 6600
uaaacccgcu guuuaauuug uuacaacaaa auguaagaaa aagaaauuuc ccauuuuaua 6660
uuacucauau ucgagcacac acuaauuuac cagggccuuu aacuaaagca aaugaacaag 6720
cugacuugcu aguaucaucu gcauucaugg aagcacaaga acuucaugcc uugacucaug 6780
uaaaugcaau aggauuaaaa aauaaauuug auaucacaug gaaacagaca aaaaauauug 6840
uacaacauug cacccagugu cagauucuac accuggccac ucaggaggca agaguuaauc 6900
ccagaggucu auguccuaau guguuauggc aaauggaugu caugcacgua ccuucauuug 6960
gaaaauuguc auuuguccau gugacaguug auacuuauuc acauuucaua ugggcaaccu 7020
gccagacagg agaaaguacu ucccauguua aaagacauuu auuaucuugu uuuccuguca 7080

ugggaguucc agaaaaaguu aaaacagaca augggccagg uuacuguagu aaagcaguuc 7140
aaaaauucuu aaaucagugg aaaauuacac auacaauagg aauucucuau aauucccaag 7200
gacaggccau aauugaaaga acuaauagaa cacucaaagc ucaauugguu aaacaaaaaa 7260
aaggaaaaga caggaguaua acacucccca gaugcaacuu aaucuagcac ucuauacuuu 7320
aaauguuuua aacauuuaua gaaaucagac cacuaccucu gcagaacaac aucuuacugg 7380
uaaaaggaac agcccacaug aaggaaaacu gauuuggugg aaagauaaua aaaauaaaac 7440
augggaaaug gggaagguga uaacgugggg gagagguuuu gcuuguguuu caccaggaga 7500
aaaucagcuu ccuguuugga uacccacuag acauuuaaag uucuacaaug aacucacugg 7560
agaugcaaag aaaagugugg agauggagac accccaaucg acucgccagg uaaacaaaau 7620
ggugauauca gaagaacaga aaaaguugcc uuccaucaag gaagcagagu ugccaauaua 7680
ggcacaauua aagaagcuga cacaguuagc uaaaaaaaaa agccuagaga auacaaaggu 7740
gacaccaacu ccagagaaua ugcugcuugc agcucugaug auuguaucaa cggugguaag 7800
ucuucccaag ucugcaggag cagcugcagc uaauuauacu uacugggccu augugccuuu 7860
cccacccuua auucgggcag uuacauagau ggauaauccu auugaaguag auguuaauaa 7920
uagugcaugg gugccuggcc ccacagauga cuguugcccu gcccaaccug aagaaggaau 7980
gaugaugaau auuuccauug gguauccuua uccuccuguu ugccuaggga aggcaccagg 8040
augcuuaaug ccuacaaccc aaaauugguu gguagaagua ccuacaguca gugcuaccag 8100
uagauuuacu uaucacaugg uaaguggaau gucacagaua aauaauuuac aggacccuuc 8160
uuaucaaaga ucauuacaau guaggccuaa ggggaaggcu ugccccaagg aaauucccaa 8220
agaaucaaaa agcccagaag ucuuagucug cggagaaugu guggcugaua cugcagugua 8280
guacaaaaca augaauuuug aacuaugaua gacugggucc cuugaggcca auuauaucau 8340
aacuguacag gccagacuca uucauguuca caggccccau ccaucuggcc cauuaaucca 8400
gccuaugacg gugauguaac ugaaaggcug gaccagguuu auagaagguu agaaucacuc 8460
uguccaagga aaugggguga aaagggaauu ucaucaccuu gaccaaaguu aguccuguua 8520
cugguccuga acauccagaa uuaggaagcu uacuguggcc ucacaccaca uuagaauuug 8580
uucuggaaau caagcuauag gaacaagaga ucguaaguca uauuauacua ucaaccuaaa 8640
uuccagucug acaauuccuu ugcaaaauug uguaaaacuc ccuuauauug cuaguuguag 8700
gaaaaacaua guuauuaaac cugauuccca aaccauaauc ugugaaaauu guggaauguu 8760
uacuugcauu gauuugacuu uuaauuggca gcaccguauu cuacuaggaa gagcaagaga 8820
gggugugugg auccuugugu ccauggaccg accaugggag gcuucgcuau ccauccauau 8880
uuuaacggaa guauuaaaag gaauucuaac uagauccaaa agauucauuu uuacuuugau 8940
ggcagugauu augggccuca uugcagucac agcuacugcu gcggcugcug gaauugcuuu 9000
acacuccucu guucaaacug cagaauacgu aaaugauugg caaaagaauu ccucaaaauu 9060
guggaauucu cagauccaaa uagaucaaaa auuggcaaac caaauuaaug aucuuagaca 9120
aacugucauu uggaugggag aggcucauga gcuuggaaua ucuuuuucag uuacgaugug 9180
acuggaauac aucagauuuu uguguuacac cacaagccua uaaugagucu gagcaucacu 9240
gggacauggu uagaugccau cugcaaggag gagaagauaa ucuuacuuua gacauuucaa 9300
aauuaaaaga auuuuuuuuu ucuuugagac agagucucgc ucugucgccc aggcuggagu 9360
gcaguggcgu gaucucagcu cacugcaagu uccgccuccu ggguuuacac cauucuccug 9420
ccucagccuc ccaaguaguu gggacuacag gagcccacca ccaugccugg cuaauuuuuu 9480
uuggguuuuu aauagagaug gaguuucacc guguuagcca ggauggucuc gaucuccuga 9540
ccuugugauc ugcccaccuu ggccucccaa agugcuggga uuacagucgu gagccaccgu 9600
gcccagccaa gaaaaaauuu uugaggcauc aaaagcccau uuaaauuugg ugccaggaac 9660
ggagacaauc gugaaagcug cugauagccu cacaaaucuu aagccaguca cuuggguuaa 9720
aagcaucaga aguuucacua uuguaaauuu cauauuaauc cuuguaugcc uguucugucu 9780
guuguuaguc uacaggugua uccagcagcu ccaaagagac agcaaccagc aagaaugggc 9840
cauagugacg augguguuuu ugucaaaaag aaaagggggg gauauguaag gaaaagagag 9900
aucagacuuu cacugugucu auguagaaaa ggaagacaua agaaacucca uuuugaucug 9960
uacuaagaaa aauuguuuug ccuugagaug cuguuaaucu guaacuuuag ccccaacccu 10020
gugcucacgg aaacaugugc uguaagguuu aagggaucua gggcugugca ggauguaccu 10080
uguuaacaau auguuugcag gcaguauguu ugguaaaagu caucgccauu cuccauucuc 10140
gauuaaccag gggcucaaug cacuguggaa agccacagga accucugccc aagaaagccu 10200
ggcuguugug ggaagucagg gaccccgaau ggagggacca gcuggugcug caucaggaaa 10260
cauaaauugu gaagauuucu uggacauuua ucaguuucca aaauuaauac uuuuauaauu 10320
ucuuacaccu gucuuacuuu aaucucuuaa uccuguuauc uuuguaagcu gaggauauac 10380
gucaccucag gaccacuauu guacaaauug auuguaaaac auguucacau guguuugaac 10440
aauaugaaau cagugcaccu ugaaaaugaa cagaauaaca gugauuuuag ggaacaaagg 10500
aagacaacca uaaggucuga cugccugagg ggucgggcaa aaagccauau uuuucuucuu 10560
gcagagagcc uauaaaugga cgugcaagua ggagagauau ugcuaaauuc uuuuccuagc 10620
aaggaauaua auacuaagac ccuagggaaa gaauugcauu ccugggggga ggucuauaaa 10680
cggccgcucu gggagugucu guccuaugug guugagauaa ggacugagau acgcccuggu 10740

cuccugcagu acccucaggc uuacuaggau ugggaaaccc caguccuggu aaauuugagg 10800
ucaggccggu ucuuugcucu gaacccuguu uucuguuaag auguuuauca agacaauaca 10860
ugcaccgcug aacauagacc cuuaucagga guuucugauu uugcucuggu ccuguuucuu 10920
cagaagcaug ucaucuuugc ucugccuucu gcccuuugaa gcaugugauc uuugugaccu 10980
acucccuguu cauacacccc uccccuuuua aaaucccuaa uaaaaacuug cugguuuugu 11040
ggcucagggg ggcaucaugg accuaccaau acgugauguc acccccggug gcccagcugu 11100

<210> 1198
<211> 11077
<212> RNA
<213> HERV-K

<400> 1198
acagcacuuu uuucuuuacc uuguuuauga ugcagagaca uuuguucaca uguuuuccug 60
cuggcccucu ccccacuauu acccuauugu ccugccacau cccccucucc gagaugguag 120
agauaaugau caauaaauac ugagggaacu cagagaccgg ugcggcgcgg guccuccaua 180
ugcugagcgc cgguccccug ggcccacuuu ucuuucucua uacuuugucu cuguugucuu 240
ucuuuucuca agucucucgu uccaccugag gagaaaugcc cacagcugug gaggcgcagg 300
ccacuccauc uggugcccaa cguggaugcu uuucucuagg gugaagggac ucucgagugu 360
ggucauugag gacaagucaa cgagagauuc ccgaguacgu cuacagugag ccuuguggua 420
agcuugggcg cucggaagaa gccaggguua auggggcaaa cuaaaaguaa agucucucau 480
uccaccugau gagaaacacc cagaggugug gaggggcagg ccaccccuuc aggguagggu 540
ccccuccaug cagaccauag agcacaggug ugccccaaag aggagcagag agaaggaggg 600
agagggccca cgagagacuu ggaaaugaau ggcaggauuu uaggcgcugg acuuggguuc 660
ggggcaccug gccuuuccuu guguauuucu ccuacugucu gccuaacuau uuaauacaau 720
aaaagaaaac cagccccugg uucuuguggu guuuccaccc ucccgggucc ccgcuggcug 780
ccuggcuucc ucccgcagcu ccugcugugu guguaugugu gugugugugc acaucugugg 840
ggcguaugug uguucgucuu uguaauugag gcugcagagu ggagagagca gggguuuucu 900
cuggggaccc agagagaagg aggcguuuuc accacagccg aacagggcag gaccccagca 960
cccgggaccc agcgggacuu ugccaagggg auggaccugg cugggccacg cggcuguuug 1020
uguagggaaa agaaagagag aucacacugu uacugugucu auguagaaaa ggaagacaua 1080
aacuccauuu ugagcuguac uaagaaaaau uauuuugccu ugaccugcug uuaaccugua 1140
acuguagccc caacccugug cucaaagaaa caugugcugu auggaaucaa gguuuaaggg 1200
aucaagggcu guacaggaug ugccuuguua acaauguguu uacaggcagu augcuuggua 1260
aaagucaucg ccauucucca uucuccauua aucaggggca cgaugcacug cggaaagcca 1320
cagggaccuc ugcccgagaa agccugggua uuguccaagg cuucccccca cugagacagc 1380
cugagauacg gccucguggg aagggaaaga ccugaccguc ccccagcccg acacccguaa 1440
agggucugug cugaggagga uuaguaaaag gggaaggccu cuugcaguug agauaagagg 1500
aaggccuccg ucuccugcau guccuuggga auggaauguc uugguguaaa acccgauagu 1560
acauuccuuc uauucugaga gaagaaaacc acccuguggc uggaggugag auaugcuagc 1620
ggcaaugcug cucuguuacu cuuugcuaca cugagauguu uggguggaga gaagcauaaa 1680
ucuggccuau gugcacaucu gggcacagaa ccuccccuug aacuugugac acagauuccu 1740
uuguucacau guuuuccugc ugaccuucuc cccacuaucg cccuguucuc ccaccgcauu 1800
ccccuugcug agauagugaa aauaguaauc uguagauacc aagggaacuc agagaccaug 1860
gccggugcac auccuccgua cgcugagcgc ugguccccug ggcccauugu ucuuucucua 1920
uacuuugucu cugugucuua uuucuuuccu cagucucuca ucccuccuga cgagaaauac 1980
ccacaggugu ggaggggcug gcccccuuca ucugaugccc aaugugggug ccuuucucua 2040
gggugaaggu acucuacagu guggucauug aggacaaguu gacgagagag ucccaaguac 2100
guccacgguc agccuugcgg uaagcuugug ugcuuagagg aacccagggu aacgaugggg 2160
caaacugaaa guaaauaugc cucuuaucuc agcuuuauua aaauucuuuu aagaagaggg 2220
ggaguuagag cuucuacaga aaaucuaauu acgcuauuuc aaacaauaga acaauucugc 2280
ccaugguuuc cagaacaggg aacuuuagau cuaaaagauu gggaaaaaau uggcaaagaa 2340
uuaaaacaag caaauaggga agguaaaauc aucccacuua caguauggaa ugauugggcc 2400
auuauuaaag caacuuuaga accauuucaa acaggagaag auauuguuuc aguuucugau 2460
gccccuaaaa gcuguguaac agauugugaa gaagaggcag ggacagaauc ccagcaagga 2520
acggaaaguu cacauuguaa auauguagca gagucuguaa uggcucaguc aacgcaaaau 2580
guugacuaca gucaauuaca ggagauaaua uacccugaau caucaaaauu gggggaagga 2640
gguccagaau cauuggggcc aucagagccu aaaccacgau cgccaucaac uccucucccc 2700
gugguucaga ugccuguaac auuacaaccu caaacgcagg uuagacaagc acaaacccca 2760
agagaaaauc aaguagaaag ggacagaguc ucuaucccgg caaugccaac ucagauacag 2820
uauccacaau aucagccggu agaaaauaag acccaaccgc ugguaguuua ucaauaccgg 2880

cugccaaccg agcuucagua ucggccuccu ucagagguuc aauacagacc ucaagcggug 2940
uguccugugc caaauagcac ggcaccauac cagcaaccca cagcgauggc gucuaauuca 3000
ccagcaacac aggacgcggc gcuguauccu cagccgccca cugugagacu uaauccuaca 3060
gcaucacgua guggacaggg uggugcacug caugcaguca uugaugaagc cagaaaacag 3120
ggcgaucuug aggcauggcg guuccuggua auuuuacaac ugguacaggc cggggaagag 3180
acucaaguag gagcgccugc ccgagcugag acuagaugug aaccuuucac caugaaaaug 3240
uuaaaagaua uaaaggaagg aguuaaacaa uauggaucca acuccccuua uauaagaaca 3300
uuauuagauu ccauugcuca uggaaauaga cuuacuccuu augacuggga aauuuuggcc 3360
aaaucuuccc uuucauccuc ucaguaucua caguuuaaaa ccugguggau ugauggagua 3420
caagaacagg uacgaaaaaa ucaggcuacu aagcccacug uuaauauaga cgcagaccaa 3480
uuguuaggaa cagguccaaa uuggagcacc auuaaccaac aaucagugau gcagaaugag 3540
gcuauugaac aaguaagggc uauuugccuc agggccuggg gaaaaauuca ggacccagga 3600
acagcuuucc cuauuaauuc aauuagacaa ggcucuaaag agccauaucc ugacuuugug 3660
gcaagauuac aagaugcugc ucaaaagucu auuacagaug acaaugcccg aaaaguuauu 3720
guagaauuaa uggccuauga aaaugcaaau ccagaauguc agucggccau aaagccauua 3780
aaaggaaaag uuccagcagg aguugaugua auuacagaau augugaaggc uugugauggg 3840
auuggaggag cuaugcauaa ggcaaugcua auggcucaag caaugagggg gcucacucua 3900
ggaggacaag uuagaacauu ugggaaaaaa uguuauaauu guggucaaau cggucaucug 3960
aaaaggaguu gcccaggcuu aaauaaacag aauauaauaa aucaagcuau uacagcaaaa 4020
aauaaaaagc caucuggccu guguccaaaa uguggaaaag caaaacauug ggccaaucaa 4080
ugucauucua aauuugauaa agaugggcaa ccauugucug gaaacaggaa gaggggccag 4140
ccucaggccc cccaacaaac uggggcauuc ccaguuaaac uguuuguucc ucaggguuuu 4200
caaggacaac aaccccuaca gaaaauacca ccacuucagg gagucagcca auuacaacaa 4260
uccaacagcu gucccgcgcc acagcaggca gcaccgcagu agauuuaugu uccacccaaa 4320
uggucuuuuu acucccugga aagcccccac aaaagauucc uagaggggua uauggcccgc 4380
ugccagaagg gaggguaggc cuuugaggga gaucaagucu aaauuugaag ggaguccaaa 4440
uucauacugg gguaauuuau ucagauuaua aagggggaau ucaguuagug aucagcucca 4500
cuguuccccg gagugccaau ccaggugaua gaauugcuca auuacugcuu uugccuuaug 4560
uuaaaauugg ggaaaacaaa aaggaaagaa caggaggguu uggaaguacc aacccugcag 4620
gaaaagcugc uuauugggcu aaucaggucu cagaggauag acccgugugu acagucacua 4680
uucagggaaa gaguuugaag gauuagugga uacccaggcu gauguuucug ucaucggcau 4740
agguacugcc ucagaagugu aucaaagugc caugauuuua cauuguccag gaucugauaa 4800
ucaagaaagu acgguucagc cugugaucac uucauuccaa ucaauuuaug gggccgagac 4860
uuguuacaac aauggcaugc agagauuacu aucccagccu cccuauacag ccccaggaau 4920
aaaaaaauca ugacuaaaau gggauagcuc ccuaaaaagg gacuaggaaa gaagucccaa 4980
uugaggcuga aaaaaaucaa aaaagaaaag gaauagggca uccuuuuuag gagcggucac 5040
uguagagccu ccaaaaccca uuccauuaac uugggggaaa aaaaaacaac uguaugguaa 5100
aucagcagcg cuuccaaaac aaaaacugga ggcuuuacau uuauuagcaa agaaacaauu 5160
agaaaaagga cauugagccu ucauuuucgc cuuggaauuc uguuuguaau ucagaaaaaa 5220
uccggcagau ggcguauaau gccguaauuc aacccauggg ggcucuccca ccccgguugc 5280
ccucuccagc cauggucccc uuuaauuaua auugaucuga aggauugcuu uuuuaccauu 5340
ccucuggcaa aacaggauuu ugaaaaauuu gcuuuuacca caccagccua aauaauaaag 5400
aaccagccac cagguuucag uggaaaguau ugccucaggg aaugcuuaau aguucaacua 5460
uuugucagcu caagcucugc aaccaguuag agacaaguuu ucagacuguu acaucguuca 5520
cuauguugau auuuugugug cugcagaaac gagagacaaa uuaauugacc guuacacauu 5580
ucugcagaca gagguugcca acgcgggacu gacaauaaca ucugauaaga uucaaaccuc 5640
uacuccuuuc cguuacuugg gaaugcaggu agaggaaagg aaaauuaaac cacaaaaaau 5700
agaaauaaga aaagacacau uaaaagcauu aaaugaguuu caaaaguugc uaggagauac 5760
uaauuggauu uggagauauu aauuggauuu ggccaacucu aggcauuccu acuuaugcca 5820
ugucaaauuu guucucuuuc uuaagagggg acucggaauu aaauagugaa agaacguuaa 5880
cuccagaggc aacuaaagaa auuaaauuaa uugaagaaaa aauucgguca gcacaaguaa 5940
auagaauaga ucacuuggcc ccacuccaaa uuuugauuuu ugcuacugca cauucccuaa 6000
caggcaucau uguucaaaau acagaucuug uggagugguc cuuccuuccu cacaguacaa 6060
uuaagacuuu uacauuguac uuggaucaaa uggcuacauu aauuggucag ggaagauuau 6120
gaauaauaac auugugugga aaugacccag auaaaaucac uguuccuuuc aacaagcaac 6180
agguuagaca agccuuuauc aauucuggug cauggcagau uggucuugcc gauuuugugg 6240
gaauuauuga caaucguuac cccaaaacaa aaaucuucca guuuuuaaaa uugacuacuu 6300
ggauuuuacc uaaaguuacc aaacauaagc cuuuaaaaaa ugcucuggca guguuuacug 6360
augguuccag caauggaaaa guggcuuaca ccgggccaaa agaaugaguc aucaaaacuc 6420
aguaucacuu gacucaaaga gcagaguugg uugccgucau uacaguguua acaagauuuu 6480
aaucagucua uuaacauugu aucagauucu gcauauguag uacaggcuac aaaggauauu 6540

gagagagccc uaaucaaaua cauuauggau gaucaguuaa acccgcuguu uaauuuguua 6600
caacaaaaug uaagaaaaag aaauuuccca uuuuauauua cucauauucg agcacacacu 6660
aauuuaccag ggccuuuaac uaaagcaaau gaacaagcug acuugcuagu aucaucugca 6720
uucauggaag cacaagaacu ucaugccuug acucauguaa augcaauagg auuaaaaaau 6780
aaauuugaua ucacauggaa acagacaaaa aauauuguac aacauugcac ccagugucag 6840
auucuacacc uggccacuca ggaggcaaga guuaauccca gaggucuaug uccuaaugug 6900
uuauggcaaa uggaugucau gcacguaccu ucauuuggaa aauugucauu uguccaugug 6960
acaguugaua cuuauucaca uuucauaugg gcaaccugcc agacaggaga aaguacuucc 7020
cauguuaaaa gacauuuauu aucuuguuuu ccugucaugg gaguuccaga aaaaguuaaa 7080
acagacaaug ggccagguua cuguaguaaa gcaguucaaa aauucuuaaa ucaguggaaa 7140
auuacacaua caauaggaau ucucuauaau ucccaaggac aggccauaau ugaaagaacu 7200
aauagaacac ucaaagcuca auugguuaaa caaaaaaaag gaaaagacag gaguauaaca 7260
cuccccagau gcaacuuaau cuagcacucu auacuuuaaa uguuuuaaac auuuauagaa 7320
aucagaccac uaccucugca gaacaacauc uuacugguaa aaggaacagc ccacaugaag 7380
gaaaacugau uugguggaaa gauaauaaaa auaaaacaug ggaaaugggg aaggugauaa 7440
cgugggggag agguuuugcu uguguuucac caggagaaaa ucagcuuccu guuuggauac 7500
ccacuagaca uuuaaaguuc uacaaugaac ucacuggaga ugcaaagaaa aguguggaga 7560
uggagacacc ccaaucgacu cgccagguaa acaaaauggu gauaucagaa gaacagaaaa 7620
aguugccuuc caucaaggaa gcagaguugc caauauaggc acaauuaaag aagcugacac 7680
aguuagcuaa aaaaaaaagc cuagagaaua caaaggugac accaacucca gagaauaugc 7740
ugcuugcagc ucugaugauu guaucaacgg ugguaagucu ucccaagucu gcaggagcag 7800
cugcagcuaa uuauacuuac ugggccuaug ugccuuuccc acccuuaauu cgggcaguua 7860
cauagaugga uaauccuauu gaaguagaug uuaauaauag ugcaugggug ccuggcccca 7920
cagaugacug uugcccugcc caaccugaag aaggaaugau gaugaauauu uccauugggu 7980
auccuuaucc uccuguuugc cuagggaagg caccaggaug cuuaaugccu acaacccaaa 8040
auugguuggu agaaguaccu acagucagug cuaccaguag auuuacuuau cacaugguaa 8100
guggaauguc acagauaaau aauuuacagg acccuucuua ucaaagauca uuacaaugua 8160
ggccuaaggg gaaggcuugc cccaaggaaa uucccaaaga aucaaaaagc ccagaagucu 8220
uagucugcgg agaaugugug gcugauacug caguguagua caaaacaaug aauuuugaac 8280
uaugauagac ugggucccuu gaggccaauu auaucauaac uguacaggcc agacucauuc 8340
auguucacag gccccaucca ucuggcccau uaauccagcc uaugacggug auguaacuga 8400
aaggcuggac cagguuuaua gaagguuaga aucacucugu ccaaggaaau ggggugaaaa 8460
gggaauuuca ucaccuugac caaaguuagu ccuguuacug guccugaaca uccagaauua 8520
ggaagcuuac uguggccuca caccacauua gaauuuguuc uggaaaucaa gcuauaggaa 8580
caagagaucg uaagucauau uauacuauca accuaaauuc cagucugaca auuccuuugc 8640
aaaauugugu aaaacucccu uauauugcua guuguaggaa aaacauaguu auuaaaccug 8700
auucccaaac cauaaucugu gaaaauugug gaauguuuac uugcauugau uugacuuuua 8760
auuggcagca ccguauucua cuaggaagag caagagaggg uguguggauc cuugugucca 8820
uggaccgacc augggaggcu ucgcuaucca uccauauuuu aacggaagua uuaaaaggaa 8880
uucuaacuag auccaaaaga uucauuuuua cuuugauggc agugauuaug ggccucauug 8940
cagucacagc uacugcugcg gcugcuggaa uugcuuuaca cuccucuguu caaacugcag 9000
aauacguaaa ugauuggcaa aagaauuccu caaaauugug gaauucucag auccaaauag 9060
aucaaaaauu ggcaaaccaa auuaaugauc uuagacaaac ugucauuugg augggagagg 9120
cucaugagcu uggaauaucu uuuucaguua cgaugugacu ggaauacauc agauuuuugu 9180
guuacaccac aagccuauaa ugagucugag caucacuggg acaugguuag augccaucug 9240
caaggaggag aagauaaucu uacuuuagac auuucaaaau uaaaagaauu uuuuuuuucu 9300
uugagacaga gucucgcucu gucgcccagg cuggagugca guggcgugau cucagcucac 9360
ugcaaguucc gccuccuggg uuuacaccau ucuccugccu cagccuccca aguaguuggg 9420
acuacaggag cccaccacca ugccuggcua auuuuuuuug gguuuuuaau agagauggag 9480
uuucaccgug uuagccagga uggucucgau cuccugaccu ugugaucugc ccaccuuggc 9540
cucccaaagu gcugggauua cagucgugag ccaccgugcc cagccaagaa aaaauuuuug 9600
aggcaucaaa agcccauuua aauuuggugc caggaacgga gacaaucgug aaagcugcug 9660
auagccucac aaaucuuaag ccagucacuu ggguuaaaag caucagaagu uucacuauug 9720
uaaauuucau auuaauccuu guaugccugu ucugucuguu guuagucuac agguguaucc 9780
agcagcucca aagagacagc aaccagcaag aaugggccau agugacgaug gugguuuugu 9840
caaaaagaaa agggggggau auguaaggaa aagagagauc agacuuucac ugugucuaug 9900
uagaaaagga agacauaaga aacuccauuu ugaucuguac uaagaaaaau uguuuugccu 9960
ugagaugcug uuaaucugua acuuuagccc caacccugug cucacggaaa caugugcugu 10020
aagguuuaag ggaucuaggg cugugcagga uguaccuugu uaacaauaug uuugcaggca 10080
guauguuugg uaaaagucau cgccauucuc cauucucgau uaaccagggg cucaaugcac 10140
uguggaaagc cacaggaacc ucugcccaag aaagccuggc uguuguggga agucagggac 10200

cccgaaugga gggaccagcu ggugcugcau caggaaacau aaauugugaa gauuucuugg 10260
acauuuauca guuuccaaaa uuaauacuuu uauaauuucu uacaccuguc uuacuuuaau 10320
cucuuaaucc uguuaucuuu guaagcugag gauauacguc accucaggac cacuauugua 10380
caaauugauu guaaaacaug uucacaugug uuugaacaau augaaaucag ugcaccuuga 10440
aaaugaacag aauaacagug auuuuaggga acaaaggaag acaaccauaa ggucugacug 10500
ccugaggggu cgggcaaaaa gccauauuuu ucuucuugca gagagccuau aaauggacgu 10560
gcaaguagga gagauauugc uaaauucuuu uccuagcaag gaauauaaua cuaagacccu 10620
agggaaagaa uugcauuccu ggggggaggu cuauaaacgg ccgcucuggg agugucuguc 10680
cuaugugguu gagauaagga cugagauacg cccuggucuc cugcaguacc cucaggcuua 10740
cuaggauugg gaaaccccag uccugguaaa uuugagguca ggccgguucu uugcucugaa 10800
cccuguuuuc uguuaagaug uuuaucaaga caauacaugc accgcugaac auagacccuu 10860
aucaggaguu ucugauuuug cucugguccu guuucuucag aagcauguca ucuuugcucu 10920
gccuucugcc cuuugaagca ugugaucuuu gugaccuacu cccuguucau acaccccucc 10980
ccuuuuaaaa ucccuaauaa aaacuugcug guuuuguggc ucaggggggc aucauggacc 11040
uaccaauacg ugaugucacc cccgguggcc cagcugu 11077

<210> 1199
<211> 450
<212> RNA
<213> HERV-K

<400> 1199
uuauguauau gcacaucaaa agcacagcac uuuuuucuuu accuuguuua ugaugcagag 60
acauuuguuc acauguuuuc cugcuggccc ucuccccacu auuacccuau uguccugcca 120
caucccccuc uccgagaugg uagagauaau gaucaauaaa uacugaggga acucagagac 180
cggugcggcg cggguccucc auaugcugag cgccgguccc cugggcccac uuuucuuucu 240
cuauacuuug ucucuguugu cuuucuuuuc ucaagucucu cguuccaccu gaggagaaau 300
gcccacagcu guggaggcgc aggccacucc aucuggugcc caacguggau gcuuuucucu 360
agggugaagg gacucucgag uguggucauu gaggacaagu caacgagaga uucccgagua 420
cgucuacagu gagccuugug ucucucaucc 450

<210> 1200
<211> 450
<212> RNA
<213> HERV-K

<400> 1200
uuauguauau gcacaucaaa agcacagcac uuuuuucuuu accuuguuua ugaugcagag 60
acauuuguuc acauguuuuc cugcuggccc ucuccccacu auuacccuau uguccugcca 120
caucccccuc uccgagaugg uagagauaau gaucaauaaa uacugaggga acucagagac 180
cggugcggcg cggguccucc auaugcugag cgccgguccc cugggcccac uuuucuuucu 240
cuauacuuug ucucuguugu cuuucuuuuc ucaagucucu cguuccaccu gaggagaaau 300
gcccacagcu guggaggcgc aggccacucc aucuggugcc caacguggau gcuuuucucu 360
agggugaagg gacucucgag uguggucauu gaggacaagu caacgagaga uucccgagua 420
cgucuacagu gagccuugug ggugaaggua 450

<210> 1201
<211> 440
<212> RNA
<213> HERV-K

<400> 1201
uuauguauau gcacaucaaa agcacagcac uuuuuucuuu accuuguuua ugaugcagag 60
acauuuguuc acauguuuuc cugcuggccc ucuccccacu auuacccuau uguccugcca 120
caucccccuc uccgagaugg uagagauaau gaucaauaaa uacugaggga acucagagac 180
cggugcggcg cggguccucc auaugcugag cgccgguccc cugggcccac uuuucuuucu 240
cuauacuuug ucucuguugu cuuucuuuuc ucaagucucu cguuccaccu gaggagaaau 300
gcccacagcu guggaggcgc aggccacucc aucuggugcc caacguggau gcuuuucucu 360
agggugaagg gacucucgag uguggucauu gaggacaagu caacgagaga uucccgagua 420
cgucuacagu gagccuugug 440

<210>   1202
<211>   26
<212>   DNA
<213>   Artificial Sequence

<220>
<223>   RT-PCR oligonucleotide

<400>   1202
agagaaaagc ctccacgttg ggcacc                                            26

<210>   1203
<211>   18
<212>   DNA
<213>   Artificial Sequence

<220>
<223>   RT-PCR oligonucleotide

<400>   1203
gtaggggtgg gttgcccc                                                     18

<210>   1204
<211>   27
<212>   DNA
<213>   Artificial Sequence

<220>
<223>   RT-PCR oligonucleotide

<400>   1204
aaaccgcctt agggctggag gtgggac                                           27

<210>   1205
<211>   18
<212>   DNA
<213>   Artificial Sequence

<220>
<223>   RT-PCR oligonucleotide

<400>   1205
tgcgggcagc aatactgc                                                     18

<210>   1206
<211>   28
<212>   DNA
<213>   Artificial Sequence

<220>
<223>   RT-PCR oligonucleotide

<400>   1206
taaagcactg agatgtttat gtgtatgc                                          28

<210>   1207
<211>   24
<212>   DNA
<213>   Artificial Sequence

<220>

<223>   RT-PCR oligonucleotide

<400>   1207
gcacagcact taatccttta catt                                              24

<210>   1208
<211>   22
<212>   DNA
<213>   Artificial Sequence

<220>
<223>   RT-PCR oligonucleotide

<400>   1208
gtttgtctgc tgaccctctc cc                                                22

198

## Description

This application claims the benefit of: international patent application PCT/US01/47824 (published in English on June 13, 2002, as WO02/46477), filed December 7th 2001; US patent application 10/016,604, filed December 7th 2001; US provisional patent application 60/340,064, filed December 7, 2001; and US provisional patent application 60/388,046, filed June 12th 2002.

All publications and patent applications mentioned in this specification are incorporated herein by reference to the same extent as if each individual document were specifically and individually indicated to be incorporated by reference.

### TECHNICAL FIELD

The present invention relates to the diagnosis of cancer, particularly prostate cancer. In particular, it relates to a human endogenous retrovirus (HERV) located on chromosome 22 which shows up-regulated expression in tumors, particularly prostate tumors.

### BACKGROUND ART

Prostate cancer is the most common type of cancer in men in the USA. Benign prostatic hyperplasia (BPH) is the abnormal growth of benign prostate cells in which the prostate grows and pushes against the urethra and bladder, blocking the normal flow of urine. More than half of the men in the USA aged 60-70 and as many as 90% percent aged 70-90 have symptoms of BPH. Although BPH is seldom a threat to life, it may require treatment to relieve symptoms.

Cancer that begins in the prostate is called primary prostate cancer (or prostatic cancer). Prostate cancer may remain in the prostate gland, or it may spread to nearby lymph nodes and may also spread to the bones, bladder, rectum, and other organs. Prostate cancer is currently diagnosed by measuring levels of prostate-specific antigen (PSA) and prostatic acid phosphatase (PAP) in the blood. The level of PSA in blood may rise in men who have prostate cancer, BPH, or an infection in the prostate. The level of PAP rises above normal in many prostate cancer patients, especially if the cancer has spread beyond the prostate. However, prostate cancer cannot be diagnosed using these tests alone because elevated PSA or PAP levels may also indicate other, non-cancerous problems.

In order to help determine whether conditions of the prostate are benign or malignant further tests such as transrectal ultrasonography, intravenous pyelogram, and cystoscopy are usually performed. If these test results suggest that cancer may be present, the patient must undergo a biopsy as the only sure way of diagnosis. Consequently, it is desirable to provide a simple and direct test for the early detection and diagnosis of prostate cancer without having to undergo multiple rounds of cumbersome testing procedures. It is also desirable and necessary to provide compositions and methods for the prevention and/or treatment of prostate cancer.

References 1 and 2 disclose that human endogenous retroviruses (HERVs) of the HML-2 subgroup of the HERV-K family show up-regulated expression in prostate tumors. This finding is disclosed as being useful in prostate cancer screening, diagnosis and therapy. In particular, higher levels of an HML-2 expression product relative to normal tissue are said to indicate that the patient from whom the sample was taken has cancer.

It is an object of the invention to provide additional and improved materials and methods that can be used in the diagnosis, prevention and treatment of prostate cancer.

### DISCLOSURE OF THE INVENTION

A specific member of the HERV-K family located in chromosome 22 at 20.428 megabases (22q11.2) has been found to be preferentially and significantly up-regulated in prostate tumors. This endogenous retrovirus (named 'PCAV' herein) has several features not found in other members of the HERV-K family and these features can be exploited in prostate cancer screening, diagnosis and therapy (*e.g*. adjuvant therapy).

The invention provides a method for diagnosing cancer, especially prostate cancer, the method comprising the step of detecting in a patient sample the presence or absence of an expression product of a human endogenous retrovirus located at megabase 20.428 on chromosome 22. Higher levels of expression product relative to normal tissue indicate that the patient from whom the sample was taken has cancer.

The expression product which is detected is preferably a mRNA transcript, but may alternatively be a polypeptide translated from such a transcript. These expression products may be detected directly or indirectly. A direct test uses an assay which detects PCAV RNA or polypeptide in a patient sample. An indirect test uses an assay which detects biomolecules which are not directly expressed *in vivo* from PCAV *e.g*. an assay to detect cDNA which has been reverse-transcribed from PCAV mRNA, or an assay to detect an antibody which has been raised in response to a PCAV polypeptide.

### A - THE HUMAN CHROMOSOME 22 ENDOGENOUS RETROVIRUS

Many regions within the published human genome sequence are annotated as endogenous retroviruses and, even before its sequence was determined, it was known that the human genome contained multiple HERVs. One of the many HERVs is a HERV-K located at megabase 20.428 of chromosome 22, referred to herein as 'PCAV'. Expression of this HERV has been found to be up-regulated in cancer tissue. Furthermore, PCAV has five specific features not found in other HERVs. These five features are manifested in PCAV mRNA transcripts and can be exploited in screening, diagnosis and therapy: (1) it has a specific nucleotide sequence which distinguishes it from other HERVs within the genome, although the sequence shares significant identity with the other HERVs; (2) it has tandem 5' LTRs; (3) it has a fragmented 3' LTR; (4) its *env* gene is interrupted by an alu insertion; and (5) its gag contains a unique insertion.

### A,1 - Nucleotide sequence

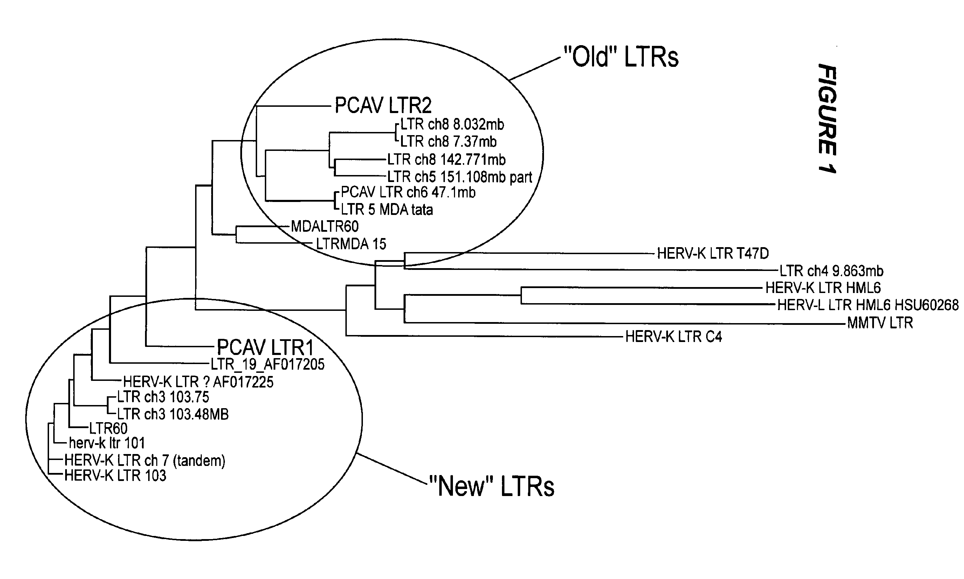

PCAV is a member of the HERV-K sub-family HML2.0. There are roughly 30 to 50 copies of HML2.0 viruses per haploid human genome. HML2 viruses appear to have inserted at least twice in human ancestry: 30 million years ago, before the ape lineage (including humans) split off from monkeys; and 20 million years ago, after the split. The viruses from the 30 million year insertion are sometimes referred to as "old type" viruses and the 20 million insertion as "new type" {3}. Old and new virus proteins are very highly related at the amino acid sequence level, but there are some distinguishing epitopes. DNA sequence identity is high at some regions of the genome but in others, particularly the LTRs, conservation is only about 70%. Most of the differences between old and new LTRs are clustered near the start of transcription, where old viruses have oen or two insertions relative to the new viruses. Old and new LTRs cluster as two separate groups in phylogenetic analyses (figure 1). In keeping with their relative genetic ages, old viruses also contain more interruptions and deletions than new viruses.

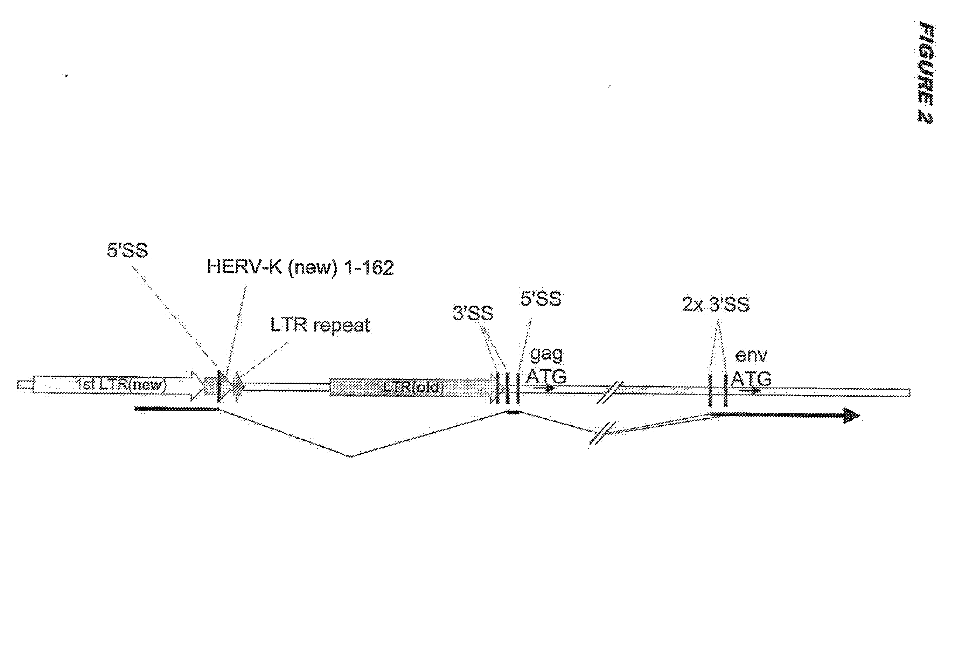

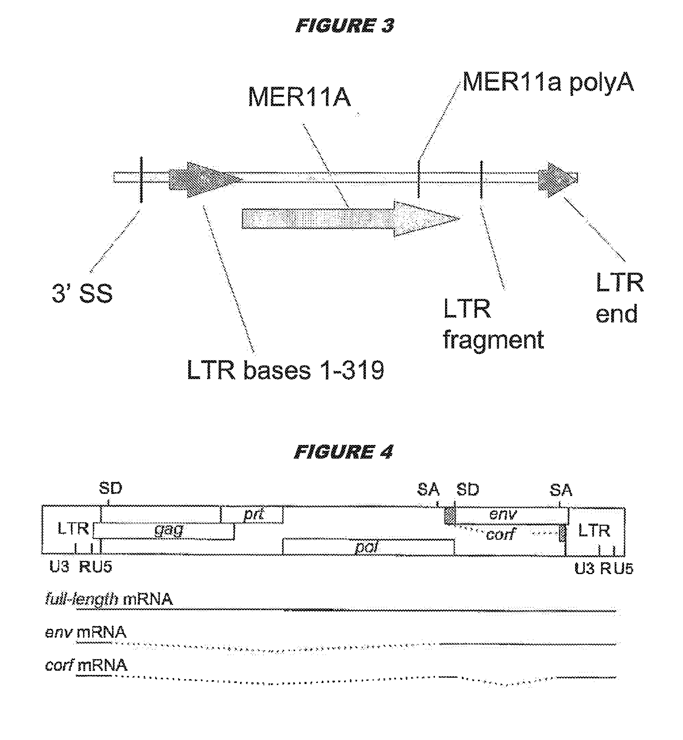

PCAV appears to have arisen from a rearrangement between a new and an old virus. The 5' region of the virus (figure 2) starts with a new LTR allowed by 162 bp from a new virus. The rest of the new virus seems to be missing, as the 162 bp is followed by a 552 bp of non-viral sequence and then an almost-complete old virus, The 3' LTR of the old virus (figure 3) is fragmented and includes a MER11a insertion.

SEQ ID 1 is the 12366bp sequence of PCAV, based on available human chromosome 22 sequence {4}, from the beginning of its first 5' LTR to the end of its fragmented 3' LTR. It is the sense strand of the double-stranded genomic DNA. SEQ ID 10 is the 11101bp sequence of PCAV from nucleotide 559 in SEQ ID 1 (a possible transcription start site) to its polyadenylation site (up to nucleotide 11735 in SEQ ID 1), although a more downstream transcription start site (*e.g.* nucleotide 635±5) is more likely.

The specific sequence of PCAV is manifested at both the mRNA and amino acid levels, and can be used to distinguish it from other HERVs within, the genome.

### A.2 - Tandem 5'LTRs

Downstream of the 5' LTR of a HERV-K, before the start of the gag open reading frame, there is a conserved splice donor site (5'SS). This splice donor can join to splice acceptor sites (3'SS) at the start of the env open reading frame (Figure 4).

HERV-K genomes also include two splice acceptor sequences near the 3' end of the LTR, but these are not ordinarily used because they have no upstream viral splice donor partner. However, PCAV has two LTRs at its 5' end: the first is from a new HERV-K and the second is from an old HERV-K. The normally-unused splice acceptors in the old LTR can thus co-operate with the splice donor in the new LTR (Figure 2), and transcripts resulting from these splice donor/acceptor pairings are specific to PCAV.

Transcripts formed by using a splice acceptor site near the 3' end of the second 5' LTR comprise (i) a sequence transcribed from the transcription start site in the first 5' LTR, continuing to a splice donor site closely downstream of the first 5' LTR, joined to (ii) a sequence transcribed from one of the splice acceptor sites near the 3' end of the second 5' LTR. Detection of such transcripts indicates that PCAV is being transcribed.

In SEQ ID 1: the transcription start site in the first 5' LTR would be at nucleotide 559 by homology to other viruses, but seems to be further downstream (*e.g,* at around 635±2) empirically; the conserved splice donor site downstream of the first 5' LTR is at nucleotides 1076-1081; the two splice acceptor sites near the 3' end of the second 5' LTR are at nucleotides 2593-2611 and 2680-2699. SEQ ID 2 is the sequence between the predicted transcription start site and the splice donor site. SEQ ID 3 is the first 10 nucleotides following the first splice acceptor site. SEQ ID 4 is the first 10 nucleotides following the second splice acceptor site. SEQ ID 5 is SEQ ID 2 fused to SEQ ID 3. SEQ ID 6 is SEQ ID 2 fused to SEQ ID 4.

### A.3 - Fragmented 3'LTR

The 3' LTR of PCAV is fragmented, including insertion of a MER11a repetitive element (figure 3). PCAV mRNAs terminate using a polyadenylation signal within the MER11a insertion, rather than using the signal within the viral LTR. Transcripts which terminate with a partial copy of a 3'HERV-K LTR followed by a MER11a sequence are specific to PCAV.

The 3' ends of transcripts from PCAV include copies of a partial LTR and a partial MER11a (figure 3). Detection of such transcripts indicates that PCAV is being transcribed.

In SEQ ID 1: the 3' LTR begins at nucleotide 10520 and continues until nucleotide 10838, where it is interrupted by a MER11a insertion; the MER11a insertion starts at nucleotide 10839 and continues to nucleotide 11834; after nucleotides 11835-11928, the 3' LTR continues from nucleotide 11929 to 12366. Within the MER11a insertion is its polyadenylation signal (located between nucleotides 11654 to 11659). SEQ ID 7 is the sequence of the first 319nt fragment of the 3' LTR. SEQ ID 8 is the sequence of the MER11a insertion up to its polyA site. SEQ ID 9 is SEQ ID 7 fused to SEQ ID 8.

### A.4 - Alu in env

As well as being disrupted by mutations due to genetic age, the env gene of PCAV is interrupted by an alu sequence. Detection of transcripts containing both env and alu sequence indicates that PCAV is being transcribed.

In SEQ ID 1, the alu is at nucleotides 9938 to 10244 (SEQ ID 32). The 100 nucleotides immediately preceding the alu sequence (9838-9937) are SEQ ID 37, the last 10mer of which (9928-9937) is SEQ ID 33. The 100 nucleotides immediately following the alu sequence are SEQ ID 40, the first 10mer of which (10244-10253) is SEQ ID 34. The first 10 nucleotides of the alu sequence are SEQ ID 35 and the last 10 are SEQ ID 41. SEQ ID 36 is the 20mer bridging the alu/env boundary and SEQ ID 45 is the 20mer bridging the end of the alu sequence, SEQ ID 39 is the 8mer bridging the alu/env boundary, and SEQ ID 44 is the 8mer bridging the end of the alu sequence. SEQ ID 38 is SEQ ID 37 + SEQ ID 32, SEQ ID 42 is SEQ ID 41 + SEQ ID 40, and SEQ ID 43 is SEQ ID 32 + SEQ ID 40.

### A.5 - Unique gag sequences

The PCAV gag gene contains a 48 nucleotide sequence (SEQ ID 53) which is not found in other HERV-Ks. The 48mer encodes 16mer SEQ ID 110, which is not found in gag proteins from new or in other old HERV-Ks. Detection of transcripts containing SEQ ID 53, or of polypeptides containing SEQ ID 110, or antibodies which recognize epitope within or including SEQ ID 110 thus indicates that PCAV is being transcribed.

The PCAV gag gene also contains a 69 nucleotide sequence (SEQ ID 111) which is not found in new HERV-Ks. The 69mer encodes 23mer SEQ ID 55. Detection of transcripts containing SEQ ID 111, or of polypeptides containing SEQ ID 55, or antibodies which recognize epitope within or including SEQ ID 55 thus indicates that an old HERV-K, typically PCAV, is being transcribed.

### B - DETECTING mRNA EXPRESSION PRODUCTS

The diagnostic method of the invention may be based on mRNA detection. PCAV mRNA may be detected directly or indirectly. It is preferred to detect a mRNA directly, thereby avoiding the need for separate preparation of mRNA-derived material (*e.g.* cDNA).

### B.1 - PCAV mRNA transcripts of the invention

mRNA transcripts for use according to the present invention are transcribed from PCAV. Three preferred types of transcript are: (1) transcripts spliced using a splice acceptor site near the 3' end of the second 5' LTR; (2) transcripts comprising both 3' LTR and MER11a sequences; (3) transcripts comprising the alu-interrupted *env* gene; and (4) transcripts comprising a PCAV-specific gag sequence.

The invention provides a mRNA transcript transcribed from a human endogenous retrovirus located at megabase 20.428 on chromosome 22.

The invention also provides a mRNA transcript comprising a nucleotide sequence with n% or more sequence identity to SEQ ID 23, or to a nucleotide sequence lacking up to 100 nucleotides (*e.g.* 10, 20, 30, 40, 50, 60, 70, 71, 72, 73, 74, 75, 76, 77, 78, 79, 80, 81, 82, 83, 84, 85, 86, 87, 88, 89, 90 or 100) from the 5' end of SEQ ID 23 *e.g. n%* or more sequence identity to SEQ ID 1197 or 1198. The nucleotide sequence is preferably at the 5' end of the RNA, although upstream sequences may be present. The nucleotide sequence may be at the 3' end of the RNA, but there will typically be further downstream elements such as a poly-A tail. These mRNA transcripts include allelic variants, SNP variants, homologs, orthologs, paralogs, mutants, etc. of SEQ ID 23, SEQ ID 1197 and SEQ ID 1198.

The invention provides a mRNA transcript formed by splicing involving a splice acceptor site near the 3' end of the second 5' LTR. Thus the invention provides a mRNA transcript comprising the sequence -N₁-N₂- (*e.g.* SEQ ID 24, SEQ YD 25, SEQ ID 1199 or SEQ ID 1200), where: N₁ is a nucleotide sequence (*e.g.* SEQ ID 26, SEQ ID 1201) from (i) the 5' end of a RNA transcribed from the first 5' LTR of a human endogenous retrovirus located at megabase 20.428 on chromosome 22, to (ii) a first splice donor site downstream of the U5 region of said mRNA transcribed from the first 5' LTR; and N₂ is a nucleotide sequence (*e.g.* SEQ ID 27 or SEQ ID 28) immediately downstream of a splice acceptor site located (i) downstream of said first splice donor site and (ii) upstream of a second splice donor site, the second splice donor site being downstream of the second 5' LTR of said endogenous retrovirus. The first splice donor site is preferably the site conserved in the HML2 sub-family, located about 100 nucleotides downstream of the first 5' LTR (after nucleotide 1075 in SEQ ID 1). The second splice donor site is preferably the site conserved in the HML2 sub-family, located about 100 nucleotides downstream of the second 5' LTR (after SEQ ID 1 nucleotide 2778). The splice acceptor is preferably downstream of the second 5' LTR.

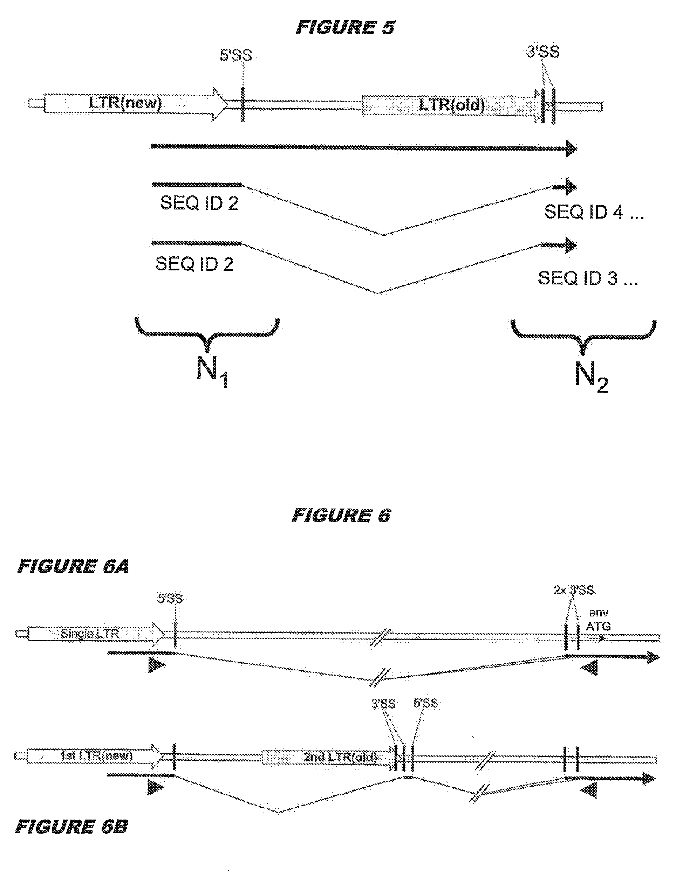

The invention also provides a mRNA transcript comprising the sequence -N₁-N₂-, where: N₁ is a nucleotide sequence with *a%* or more sequence identity to SEQ ID 26 and/or SEQ ID 1201 and N₂ is a nucleotide sequence with b% or more sequence identity to SEQ ID 27 or SEQ ID 28. These mRNA transcripts of the invention are illustrated in figure 5. Transcripts which use the second splice site (*i.e.* N₂ is SEQ ID 28) are preferred.

In both cases, N₁ is preferably at the 5' end of the RNA, although upstream sequences may be present. N₂ may be at the 3' end of the RNA, but downstream sequences will usually be present.

The invention also provides a mRNA transcript comprising a nucleotide sequence with *c%* or more sequence identity to SEQ ID 24, SEQ ID 25, SEQ ID 1199 or SEQ ID 1200.

The invention provides a mRNA transcript comprising the sequence -N₃-N₄- (*e.g.* SEQ ID 29), where: N₃ is a nucleotide sequence (*e.g.* SEQ ID 30) from the 3' end of the 5' fragment of the 3' LTR of a human endogenous retrovirus located at megabase 20.428 on chromosome 22, and N₄ is a nucleotide sequence (*e.g.* SEQ ID 31) from 5' end of the MER11a insertion in a human endogenous retrovirus located at megabase 20.428 on chromosome 22.

The invention also provides a mRNA transcript comprising the sequence -N₃-N₄-, where: N₃ is a nucleotide sequence with *d%* or more sequence identity to SEQ ID 30 and N₄ is a nucleotide sequence with e% or more sequence identity to SEQ ID 31. The RNA may comprise the sequence -N₃-N₄-N₅-N₆-, wherein: N₅ is a nucleotide sequence between the polyA signal and the polyA site of a MER11a sequence; and N₆ is a polyA tail.

In both cases, the transcript will generally include sequence upstream of N₃. The transcript will generally include sequence downstream of N₄, such as a polyA tail.

The invention also provides a mRNA transcript comprising a nucleotide sequence with *f%* or more sequence identity to SEQ ID 29.

The invention provides a mRNA transcript comprising the sequence -N₇-N₈- (*e.g.* SEQ ID 38), where: N₇ is a nucleotide sequence (*e.g.* SEQ ID 37) preceding the alu insertion within the *env* gene of a human endogenous retrovirus located at megabase 20.428 on chromosome 22, and N₈ is a nucleotide sequence (*e.g.* SEQ ID 32) beginning at the 5' end of said alu insertion.

The invention also provides a mRNA transcript comprising the sequence -N₇-N₈-, where: N₇ is a nudeotide sequence with *mm%* or more sequence identity to SEQ ID 37 and N₈ is a nucleotide sequence with *nn%* or more sequence identity to SEQ ID 32.

The transcript will generally include sequence upstream of _{N}7 and downstream of N₈.

The invention also provides a mRNA transcript comprising a nucleotide sequence with *pp%* or more sequence identity to SEQ ID 38.

The invention provides a mRNA transcript comprising the sequence "N₉-N₁₀- (*e.g.* SEQ ID 43), where: N₉ is a nucleotide sequence (*e.g.* SEQ ID 32) at the end of the alu insertion within the *env* gene of a human endogenous retrovirus located at megabase 20.428 on chromosome 22, and N₁₀ is a nucleotide sequence (*e.g.* SEQ ID 40) immediately downstream of said alu insertion.

The invention also provides a mRNA transcript comprising the sequence -N₉-N₁₀-, where: N₉ is a nucleotide sequence with *uu%* or more sequence identity to SEQ ID 41 and N₁₀ is a nucleotide sequence with *vv%* or more sequence identity to SEQ ID 40.

The transcript will generally include sequence upstream of N₉ and downstream of N₁₀.

The invention also provides a mRNA transcript comprising a nucleotide sequence with *ww%* or more sequence identity to SEQ ID 42.

The invention provides a mRNA transcript comprising a nucleotide sequence with *uu%* or more sequence identity to SEQ ID 41.

The transcript will generally include sequence upstream of N₉ and downstream of N₁₀.

The invention also provides a mRNA transcript comprising a nucleotide sequence with *ii%* or more sequence identity to SEQ ID 53.

The invention also provides a mRNA transcript comprising a nucleotide sequence with *ii%* or more sequence identity to SEQ ID 111.

The invention also provides a mRNA transcript comprising a nucleotide sequence with *ii%* or more sequence identity to SEQ ID 1191. The invention also provides a mRNA transcript which encodes a polypeptide having at least *ii%* sequence identity to SEQ ID 98.

### B.2 - Direct and indirect detection of mRNA

PCAV mRNA transcripts of the invention may be detected directly, for example by sequencing of the mRNA or by hybridization to mRNA transcripts (*e.g.* by Northern blot). Various techniques are available for detecting the presence or absence of a particular RNA sequence in a sample {*e.g*. refs. 20 & 21}.

Indirect detection of mRNA transcripts is also possible and is performed on nucleic acid derived from a PCAV mRNA transcript *e.g.* detection of a cDNA copy of PCAV mRNA, detection of nucleic acids amplified from a PCAV mRNA template, etc.

A preferred method for detecting RNA is RT-PCR (reverse transcriptase polymerase chain reaction) {*e.g*. refs. 5 to 13}. RT-PCR of mRNA from prostate cells is reported in, for example, references 14 to 19. It is preferred to use PCAV-specific probes in RT-PCR.

Whether direct or indirect detection is used, the method of the invention involves detection of a single-stranded or double-stranded PCAV nucleic acid target, either (a) in the form of PCAV mRNA or (b) in the form of nucleic acid comprising a copy of at least a portion of a PCAV mRNA and/or a sequence complementary to at least a portion of a PCAV mRNA.

The method of the invention does not involve the detection of PCAV genomic DNA, as this is present in all human cells and its presence is therefore not characteristic of tumors. If a sample contains PCAV DNA, it is preferred to use a RNA-specific detection technique or to focus on sequences present in PCAV mRNA transcripts but not in PCAV genomic DNA (*e.g.* splice junctions, polyA tail *etc.*)*,* The method of the invention may therefore comprise an initial step of: (a) extracting mRNA from a patient sample; (b) removing DNA from a patient sample without removing mRNA; and/or (c) removing or disrupting PCAV DNA, but not PCAV mRNA, in a patient sample. As an alternative, a RNA-specific assay can be used which is not affected by the presence of homologous DNA. For RT-PCR, genomic DNA should be removed,

Methods for selectively extracting RNA from biological samples are well known {*e.g*. refs. 20 & 21} and include methods based on guanidinium buffers, lithium chloride, acid phenol:chloroform extraction, SDS/potassium acetate etc. After total cellular RNA has been extracted, mRNA may be enriched *e.g.* using oligo-dT techniques.

Methods for removing DNA from biological samples without removing mRNA are well known {*e.g.* appendix C of ref. 20} and include DNase digestion. If DNase is used then it must be removed or inactivated (*e.g.* by chelation with EDTA, by heating, or by proteinase K treatment followed by phcnol/chloroform extraction and NH₄OAc/EtOH precipitation) prior to subsequent DNA synthesis or amplification, in order to avoid digestion of the newly-synthesized DNA.

Methods for removing PCAV DNA, but not PCAV RNA, will use a reagent which is specific to a sequence within a PCAV DNA *e.g.* a restriction enzyme which recognizes a DNA sequence within the PCAV genome, but which does not cleave the corresponding RNA sequence.

Methods for specifically purifying PCAV mRNAs from a sample may also be used. One such method uses an affinity support which binds to PCAV mRNAs. The affinity support may include a polypeptide sequence which binds to the PCAV mRNA *e.g.* the cORF polypeptide, which binds to the LTR of HERV-K mRNAs in a sequence-specific manner, or HIV Rev protein, which has been shown to recognize the HERV-K LTR in RNA transcripts {22}.

PCAV mRNA need not be maintained in a wild-type form for detection. It may, for example, be fragmented, provided that the fragmentation maintains PCAV-specific sequences within the mRNA.

### B.3 - PCAV nucleic acid targets for detection

The invention provides nucleic acid comprising (a) the nucleotide sequence of a mRNA transcript transcribed from a human endogenous retrovirus located at megabase 20.428 on chromosome 22, and/or (b) the complement of (a). The invention also provides nucleic acid comprising a nucleotide sequence with *qq%* or more sequence identity to SEQ ID 10, SEQ ID 1197 and/or SEQ ID 1198. PCAV is approximately 87.5% identical to the HBRV-K found at megabase 47.1 on chromosome 6 and approximately 86% identical to the HERV-K found at megabase 103.75 on chromosome 3.

The invention provides nucleic acid comprising (a) nucleotide sequence -N₁-N₂- as defined above, and/or (b) the complement of (a). The invention also provides nucleic acid comprising (a) a nucleotide sequence with c% or more sequence identity to SEQ ID 5, SEQ ID 6, SEQ ID 1199 or SEQ ID 1200, and/or (b) the complement of (a).

The invention provides nucleic acid comprising (a) nucleotide sequence -N₃-N₄- as defined above, and/or (b) the complement of (a). The invention also provides nucleic acid comprising (a) a nucleotide sequence with *f%* or more sequence identity to SEQ ID 9, and/or (b) the complement of (a).

The invention also provides nucleic acid comprising (a) nucleotide sequence -N₃-N₄-N₅-N₆- as defined above, and/or (b) the complement of (a).

The invention provides nucleic acid comprising (a) nucleotide sequence -N₇-N₈- as defined above, and/or (b) the complement of (a). The invention also provides nucleic acid comprising (a) a nucleotide sequence with *aa%* or more sequence identity to SEQ ID 38, and/or (b) the complement of (a).

The invention provides nucleic acid comprising (a) nucleotide sequence -N₉-N₁₀- as defined above, and/or (b) the complement of (a). The invention also provides nucleic acid comprising (a) a nucleotide sequence with *hh%* or more sequence identity to SEQ ID 42, and/or (b) the complement of (a).

The invention provides nucleic acid comprising a nucleotide sequence with *bbb%* or more sequence identity to SEQ ID 53, and/or (b) the complement of (a).

The invention provides nucleic acid comprising a nucleotide sequence with *fff%* or more sequence identity to SEQ ID 111, and/or (b) the complement of (a).

Specific nucleic acid targets include SEQ IDs 99 to 109, which are splice variant cDNA sequences assuming a transcription start site in SEQ ID 1 at 559 and including four A residues at the 3' end. Assuming a more downstream transcription start site (*e.g.* nucleotide 635 of SEQ ID 1), these nucleic targets would not include a stretch ofnucleotides at the 5' end of SEQ IDs 99 to 109 *e.g.* they would not include 10, 20, 30, 40, 50, 60, 70, 71, 72, 73, 74, 75, 76, 77, 78, 79, 80, 81, 82, 83, 84, 85, 86, 87, 88, 89, 90, 100 or more of the 5' nucleotides. 25mer sequences based on cDNA sequences are given as SEQ IDs 337 to 599.

### B.4 - Nucleic acid materials for direct or indirect mRNA detection

The invention provides nucleic acid which can hybridize to a PCAV nucleic acid target.

Hybridization reactions can be performed under conditions of different "stringency". Conditions that increase stringency of a hybridization reaction of widely known and published in the art {*e.g.* page 7.52 of reference 21}. Examples of relevant conditions include (in order of increasing stringency): incubation temperatures of 25°C, 37°C, 50°C, 55°C and 68°C; buffer concentrations of 10 x SSC, 6 x SSC, 1 x SSC, 0.1 x SSC (where SSC is 0.15 M NaCl and 15 mM citrate buffer) and their equivalents using other buffer systems; formamide concentrations of 0%, 25%, 50%, and 75%; incubation times from 5 minutes to 24 hours; 1, 2, or more washing steps; wash incubation times of 1, 2, or 15 minutes; and wash solutions of 6 x SSC, 1 x SSC, 0.1 x SSC, or de-ionized water, Hybridization techniques and their optimization are well known in the art {*e.g*. see references 20, 21, 23, 24, 28 *etc*.}.

In some embodiments, nucleic acid of the invention hybridizes to a target of the invention under low stringency conditions; in other embodiments it hybridizes under intermediate stringency conditions; in preferred embodiments, it hybridizes under high stringency conditions. An exemplary set of low stringency hybridization conditions is 50°C and 10 x SSC. An exemplary set of intermediate stringency hybridization conditions is 55°C and 1 x SSC. An exemplary set of high stringency hybridization conditions is 68°C and 0.1 x SSC.

Preferred nucleic acids of the invention hybridize to PCAV nucleic acid targets but not to nucleic acid targets from other HERV-Ks. PCAV-specific hybridization is favored by exploiting features found within PCAV transcripts but not in other HERV-K transcripts *e.g.* specific nucleotide sequences, features arising from the tandem 5' LTRs, features arising from the MER11a insertion within the 3' LTR, or features arising from the alu interruption of env. Sequence alignments can be used to locate regions of PCAV which are most divergent from other HERV-K genomes and in which PCAV-specific hybridization can occur. Specificity for PCAV is desirable in order to detect its up-regulation above the low-level of natural background expression of other new HERV-Ks seen in most cells.

One group of preferred nucleic acids of the invention can specifically detect PCAV products in which a splice acceptor site near the 3' end of the second 5' LTR has been used. As described above, such splicing brings together sequences N₁ and N₂, which are not juxtaposed in PCAV genomic DNA. Thus the invention provides a nucleic acid which hybridizes to sequence -N₁-N₂- (or the complement thereof) within a PCAV nucleic acid target, but which does not hybridize to sequences N₁ or N₂ alone (or to their complements alone). The nucleic acid comprises a first sequence which can hybridize to N₁ (or to its complement) and a second sequence which can hybridize to N₂ (or to its complement), such that it will hybridize to a target in which N₁ and N₂ are adjacent, but will not hybridize to targets in which splicing has not brought N₁ and N₂ together. Such nucleic acids can identify PCAV transcripts in the presence of PCAV genomic DNA because of the difference in relative locations of N₁ and N₂.

Another group of preferred nucleic acids of the invention can specifically detect mRNAs containing 3' LTR and MER11a sequences. Thus the invention provides a nucleic acid which hybridizes to sequence -N₃-N₄- (or the complement thereof) within a PCAV nucleic acid target, but which does not hybridize to sequences N₃ or N₄ alone (or to their complements alone). The nucleic acid comprises a first sequence which can hybridize to N₃ (or to its complement) and a second sequence which can hybridize to N₄ (or to its complement), such that it will hybridize to targets which include both (i) a 3' LTR sequence and (ii) a MER11a sequence, but not to targets which include only one of (i) and (ii). The nucleic acid may inherently be able to hybridize to gnomic DNA, although this property is not useful for detecting transcripts.

Another group of preferred nucleic acids of the invention can specifically detect mRNAs containing the alu-inlerrupted env gene. Thus the invention provides a nucleic acid which hybridizes to sequence -N₇-N₈- (or the complement thereof) within a PCAV nucleic acid target, but which does not hybridize to sequences N₇ or N₈ alone (or to their complements alone). The nucleic acid comprises a first sequence which can hybridize to N₇ (or to its complement) and a second sequence which can hybridize to N₈ (or to its complement), such that it will hybridize to targets which include both (i) the env sequence immediately preceding the alu interruption and (ii) an alu interruption, but not to targets which include only one of (i) and (ii). The nucleic acid may inherently be able to hybridize to genomic DNA, although this property is not useful for detecting transcripts.

The invention also provides a nucleic acid which hybridizes to sequence -N₉-N₁₀- (or the complement thereof) within a PCAV nucleic acid target, but which does not hybridize to sequences N₉ or N₁₀ alone (or to their complements alone). The nucleic acid comprises a first sequence which can hybridize to N₉ (or to its complement) and a second sequence which can hybridize to N₁₀ (or to its complement), such that it will hybridize to targets which include both (i) the 3' region of the alu interruption within env and (ii) the sequence immediately downstream of the alu interruption, but not to targets which include only one of (i) and (ii). The nucleic acid may inherently be able to hybridize to genomic DNA, although this property is not useful for detecting transcripts.

The ability of a nucleic acid to hybridize to a PCAV nucleic acid target is related to its intrinsic features (*e.g.* the degree of sequence identity to the target) as well as extrinsic features (*e.g.* temperature, salt concentration *etc.*). A group of preferred nucleic acids of the invention have a good intrinsic ability to hybridize to PCAV nucleic acid targets.

Thus the invention provides a nucleic acid comprising a nucleotide sequence with *s*% or more sequence identity to a fragment of a PCAV nucleic acid target or to the complement of a fragment of a PCAV nucleic acid target. The invention provides a nucleic acid comprising a nucleotide sequence with *g*% or more sequence identity to a fragment of SEQ ID 10 or to the complement of a fragment of SEQ ID 10. The invention also provides a nucleic acid comprising a nucleotide sequence with *h*% or more sequence identity to a fragment of SEQ ID 5 or to the complement of a fragment of SEQ ID 5. The invention also provides a nucleic acid comprising a nucleotide sequence with *i*% or more sequence identity to a fragment of SEQ ID 6 or to the complement of a fragment of SEQ ID 6. The invention also provides a nucleic acid comprising a nucleotide sequence with *j*% or more sequence identity to a fragment of SEQ ID 9 or to the complement of a fragment of SEQ ID 9. The invention also provides a nucleic acid comprising a nucleotide sequence with *ccc*% or more sequence identity to a fragment of SEQ ID 53 or to the complement of a fragment of SEQ ID 53. The invention also provides a nucleic acid comprising a nucleotide sequence with *kkk*% or more sequence identity to SEQ ID 1191. It also provides a nucleic acid comprising a nucleotide sequence which encodes a polypeptide having at least *mmm*% sequence identity to SEQ ID 98. The invention also provides a nucleic acid comprising a nucleotide sequence with *nnn*% or more sequence identity to SEQ ID 1198. It also provides a nucleic acid comprising a nucleotide sequence which encodes a polypeptide having at least *qqq*% sequence identity to SEQ ID 1199. It also provides a nucleic acid comprising a nucleotide sequence which encodes a polypeptide having at least *rrr*% sequence identity to SEQ ID 1200.

The invention provides a nucleic acid comprising a fragment of at least *k* contiguous nucleotides of SEQ ID 10 or of the complement of SEQ ID 10. The fragment is preferably located within SEQ ID 1197 and/or 1198.

The invention also provides a nucleic acid comprising a fragment of at least *l* contiguous nucleotides of SEQ ID 47 or of the complement of SEQ ID 47. The fragment preferably comprises nucleotide sequence B₁ₐ-B₂ₐ (or its complement), wherein B₁ₐ comprises *m* or more nucleotides from the 3' end of SEQ ID 2 and B₂ₐ comprises p or more nucleotides from the 5' end of SEQ ID 46. These nucleic acids thus span a splice junction which brings sequences N₁ and N₂ together and are thus able to identify PCAV transcripts in the presence of PCAV genomic DNA because of the difference in the relative locations of B₁ₐ and B₂ₐ. B₁ₐ-B₂ₐ preferably comprises SEQ ID 11 (or its complement), where m=p=4, and more preferably comprises SEQ ID 50 (or its complement), where m=p=10.

The invention also provides a nucleic acid comprising a fragment of at least *q* contiguous nucleotides of SEQ ID 49 or of the complement of SEQ ID 49. The fragment preferably comprises nucleotide sequence B_{1b}-B_{2b} (or its complement), wherein B_{1b} comprises *r* or more nucleotides from the 3' end of SEQ ID 2 and B_{2b} comprises *t* or more nucleotides from the 5' end of SEQ ID 48. These nucleic acids thus span the splice junction which brings sequences N₁ and N₂ together and are thus able to identify PCAV transcripts in the presence of PCAV genomic DNA because of the difference in the relative locations of B_{1b} and B_{2b}. B_{1b}∼B_{2b} preferably comprises SEQ ID 12 (or its complement), where r=t=4, and more preferably comprises SEQ ID 51 (or its complement), where r=t=10.

The invention also provides a nucleic acid comprising a fragment of at least *u* contiguous nucleotides of SEQ ID 9 or of the complement of SEQ ID 9. The fragment preferably comprises nucleotide sequence B₃-B₄ (or its complement), wherein B₃ comprises *v* or more nucleotides from the 3' end of SEQ ID 7 and B₄ comprises w or more nucleotides from the 5' end of SEQ ID 8. These nucleic acids thus include part of both of N₃ and N₄. B₃∼B₄ preferably comprises SEQ ID 13 (or its complement), where v=w=4, and more preferably comprises SEQ ID 52 (or its complement), where v=w=10.

The invention also provides a nucleic acid comprising a fragment of at least *rr* contiguous nucleotides of SEQ ID 38 or of the complement of SEQ ID 38. The fragment preferably comprises nucleotide sequence B₇-B₈ (or its complement), wherein B₇ comprises *ss* or more nucleotides from the 3' end of SEQ ID 37 and B₄ comprises *tt* or more nucleotides from the 5' end of SEQ ID 32. These nucleic acids thus include part of both of N₇ and N₈. B₇-B₈ preferably comprises SEQ ID 39 (or its complement), where ss=tt=4, and more preferably comprises SEQ ID 36 (or its complement), where ss=tt=10.

The invention also provides a nucleic acid comprising a fragment of at least *jj* contiguous nucleotides of SEQ ID 43 or of the complement of SEQ ID 43. The fragment preferably comprises nucleotide sequence B₉-B₁₀, or its complement, and wherein B₉ comprises kk or more nucleotides from the 3' end of SEQ ID 32 and B₁₀ comprises *ll* or more nucleotides from the 5' end of SEQ TD 40. These nucleic acids thus include part of both of N₉ and N₁₀. B₉-B₁₀ preferably comprises SEQ ID 44 (or its complement), where kk=ll=4, and more preferably comprises SEQ ID 45 (or its complement), where kk=ll=10.

The invention also provides a nucleic acid comprising a fragment of at least *ddd* contiguous nucleotides of SEQ ID 53 or of the complement of SEQ ID 53. The invention also provides a nucleic acid comprising a fragment of at least *ggg* contiguous nucleotides of SEQ ID 111 or of the complement of SEQ ID 111. The invention also provides a nucleic acid comprising a fragment of at least *hhh* contiguous nucleotides of SEQ ID 112 or of the complement of SEQ ID 112. The invention also provides a nucleic acid comprising a fragment of at least *jjj* contiguous nucleotides of SEQ ID 1191 or of the complement of SEQ ID 1191.

The invention provides a nucleic acid of formula 5'-X-Y-Z-3', wherein: -X- is a nucleotide sequence consisting of *x* nucleotides; -Z- is a nucleotide sequence consisting of z nucleotides; -Y- is a nucleotide sequence consisting of either (a) a fragment of *y* nucleotides of any of SEQ IDs 1-13, 20-53, 57, 58, 63, 81, 86, 88-91, 99-109, 111, 112, 1191, 1197 or 1198, or (b) the complement of (a); and said nucleic acid 5'-X-Y-Z-3' is neither (i) a fragment of SEQ IDs 1-13, 20-53, 57, 58, 63, 81, 86, 88-91, 99-109, 111, 112, 1191, 1197 or 1198 or (ii) the complement of (i).

Where -Y- is (a), the nucleotide sequence of -X- preferably shares less than *bb*% sequence identity to the *x* nucleotides which are 5' of sequence -Y- in SEQ IDs 1-13, 20-53, 57, 58, 63, 81, 86, 88-91, 99-109, 111, 112, 1191, 1197 or 1198 and/or the nucleotide sequence of -Z-preferably shares less than *cc*% sequence identity to the z nucleotides which are 3' of sequence - Z- in SEQ IDs 1-13, 20-53, 57, 58, 63, 81, 86, 88-91, 99-109, 111, 112, 1191, 1197 or 1198.

Where -Y- is (b), the nucleotide sequence of -X- preferably shares less than *bb*% sequence identity to the complement of the *x* nucleotides which are 5' of the complement of sequence -Yin SEQ IDs 1-1,3, 20-53, 57, 58, 63, 81, 86, 88-91, 99-109, 111, 112, 1191, 1197 or 1198 and/or the nucleotide sequence of -Z- preferably shares less than *cc*% sequence identity to the complement of the z nucleotides which are 3' of the complement of sequence -Y- in SEQ IDs 1-13, 20-53, 57, 58, 63, 81, 86, 88-91, 99-109,111, 112, 1191, 1197 or 1198.

The -X- and/or -Z- moieties may comprise a promoter sequence (or its complement).

The invention provides nucleic acid comprising nucleotide sequence SEQ ID 53. This sequence is specific within the human genome to PCAV. The invention also provides nucleic acid comprising nucleotide sequence SEQ ID 111.

The invention also provides nucleic acid comprising nucleotide sequence SEQ ID 1191.

Various PCAV nucleic acids are provided by the invention. 25mer fragments of PCAV sequences are given as SEQ IDs 120 to 1184. The invention provides these sequences as 25mers, as well as fragments thereof (*e.g*. the 2 x 24mers, the 3 x 23mers, the 4 x 22mers ... the 19 x 7mers in each) and as longer PCAV fragments comprising these 25mers.

Preferred nucleic acids of the invention comprise one or more of SEQ IDs 53 and 842-1184,

Nucleic acids of the invention are particularly useful as probes and/or as primers for use in hybridization and/or amplification reactions.

More than one nucleic acid of the invention can hybridize to the same target (*e.g.* more than one can hybridize to a single mRNA or cDNA).

### B.5 - Nucleic acid amplification

Nucleic acid in a sample can conveniently and sensitively be detected by nucleic acid amplification techniques such as PCR, SDA, SSSR, LCR, TMA, NASBA, T7 amplification *etc.* The technique preferably gives exponential amplification. A preferred technique for use with RNA is RT-PCR (*e.g.* see chapter 15 of ref. 20). The technique may be quantitative and/or real-time.

Amplification techniques generally involve the use of two primers. Where a target sequence is single-stranded, the techniques generally involve a preliminary step in which a complementary strand is made in order to give a double-stranded target. The two primers hybridize to different strands of the double-stranded target and are then extended. The extended products can serve as targets for further rounds of hybridization/extension. The net effect is to amplify a template sequence within the target, the 5' and 3' termini of the template being defined by the locations of the two primers in the target.

The invention provides a kit comprising primers for amplifying a template sequence contained within a PCAV nucleic acid target, the kit comprising a first primer and a second primer, wherein the first primer comprises a sequence substantially complementary to a portion of said template sequence and the second primer comprises a sequence substantially complementary to a portion of the complement of said template sequence, wherein the sequences within said primers which have substantial complementarity define the termini of the template sequence to be amplified.

Kits of the invention may further comprise a probe which is substantially complementary to the template sequence and/or to its complement and which can hybridize thereto. This probe can be used in a hybridization technique to detect amplified template.

Kits of the invention may further comprise primers and/or probes for generating and detecting an internal standard, in order to aid quantitative measurements {*e.g.* 15, 25}.

Kits of the invention may comprise more than one pair of primers (*e.g.* for nested amplification), and one primer may be common to more than one primer pair. The kit may also comprise more than one probe.

The template sequence is preferably located within a transcript of a HERV-K located at megabase 20.428 of chromosome 22, and is more preferably a fragment of SEQ ID 10 (or SEQ ID 23). The template sequence is preferably at least 50 nucleotides long (*e.g.* 60, 70, 80, 90, 100, 125, 150, 175, 200, 250, 300, 400, 500, 600, 700, 800, 900, 1000, 1250, 1500, 2000, 3000 nucleotides or longer). The length of the template is inherently limited by the length of the target within which it is located, but the template sequence is preferably shorter than 500 nucleotides (*e.g.* 450, 400, 350, 300, 250, 200, 175, 150, 125, 100, 90, 80, 70 or shorter).

A preferred template comprises SEQ ID 53 and/or SEQ ID 111.

Primers and probes used in kits of the invention are preferably nucleic acids as described in section B.4 above. Particularly preferred primers are those based on SEQ IDs 600-1184 (or their complements) *e.g.* comprising primers comprising SEQ IDs 600-1184, or primers comprising fragments of *ppp* or more nucleotides from one of SEQ IDs 600-1184.

Further features of primers and probes are described in section B.6 below.

Preferred kits comprise (i) a first primer comprising a sequence which is substantially identical to a portion of SEQ ID 10 and (ii) a second primer comprising a sequence which is substantially complementary to a portion of SEQ ID 10, such that the primer pair (i) and (ii) defines a template sequence within SEQ ID 10. Other preferred kits comprise (i) a first primer comprising a sequence which is substantially identical to a portion of the complement of SEQ ID 10 and (ii) a second primer comprising a sequence which is substantially complementary to a portion of the complement of SEQ ID 10, such that the primer pair defines a template sequence within SEQ ID 10. The portion and template sequence preferably fall within SEQ ID 1197 or SEQ ID 1198.

It is preferred that one or both of the primers is not substantially complementary to a portion of a HERV-K other than PCAV (or its complement) such that the primer pair is specific for PCAV.

SEQ ID 10 may be divided into four exons: (1) nucleotides 1-517, containing sequences up to the conserved splice donor downstream of the first 5' LTR; (2) nucleotides 2142-2209, containing sequences between the splice acceptor near the 3' end of the second 5' LTR and the conserved splice donor; (3) nucleotides 7608-7686; and (4) nucleotides 9866-11181 (assuming transcription start at nucleotide 559 of SEQ ID 1). Exon (2) arises because of the unique PCAV feature of tandem 5' LTRs, but the other three exons exist in other HERV-Ks.

In preferred kits of the invention, the first and second primers are located in different exons. This arrangement means that the amplified template sequence is shorter than would be obtained from genomic DNA, because of the absence ofintrons. For example:

| | | | | | | |
|---|---|---|---|---|---|---|
| **First primer in exon** | 1 | 1 | 1 | 2 | 2 | 3 |
| **Second primer in exon** | 2 | 3 | 4 | 3 | 4 | 4 |

With reference to SEQ ID 10, therefore, the primers may comprise a fragment of SEQ ID 10 (or its complement) located between the following coordinates:

| | | | | | | |
|---|---|---|---|---|---|---|
| **First primer** | 1-517 | 1-517 | 1-517 | 2142-2219 | 2142-2219 | 7608-7686 |
| **Second primer** | 2142-2219 | 7608-7686 | 9866-11181 | 7608-7686 | 9866-11181 | 9866-11181 |

With reference to SEQ ID 1, these coordinates are:

| | | | | | | |
|---|---|---|---|---|---|---|
| **First primer** | 559-1075 | 559-1075 | 559-1075 | 2700-2777 | 2700-2777 | 8166-8244 |
| **Second primer** | 2700-2777 | 8166-8244 | 10424-11739 | 8166-8244 | 10424-11739 | 10424-11739 |

With a more-downstream transcription start site, however, the first exon may begin downstream of nucleotide 559 *e.g.* at around nucleotide 633, 635 or 637.

Example primers within exon 1 are SEQ IDs 120 to 219. Example primers within exons 2 to 4 are SEQ IDs 220 to 336.

In other preferred kits, one or both of the first and second primers comprise a first sequence from a first exon and a second sequence from a second exon, such that the primer bridges an exon-exon boundary after splicing. For example, a primer may comprise sequences from exons 1 & 2, exons 1 & 3, exons 1 & 4, exons 2 & 3, exons 2 & 4, or exons 3 & 4. These primers hybridize to transcripts where splicing has taken place.

With reference to SEQ ID 10, therefore, the primers may comprise a first sequence from the 3' end of the following coordinates and second sequence from the 5' end of the following coordinates (or complements thereof):

| | | | | | | |
|---|---|---|---|---|---|---|
| **First sequence** | 1-517 | 1-517 | 1.517 | 2142-2209 | 2142-2209 | 7608-7686 |
| **Second sequence** 2142-2209 | 2142-2209 | 7608-7686 | 9866-11181 | 7608-7686 | 9866-11181 | 9866-11181 |

Taking a more-downstream transcription start site, however, the range '1-517' for selecting the first sequence should be replaced with around '77-517' *e.g.* 75-517 or 80-517.

In preferred kits for detecting PCAV nucleic acid targets in which a splice acceptor site near the 3' end of the second 5' LTR has been used, either (i) the first primer comprises a sequence which is substantially identical to a portion of N₁ and the second primer comprises a sequence which is substantially complementary to a portion of N₂, or (ii) the first primer comprises a sequence which is substantially identical to a portion of the complement of N₁ and the second primer comprises a sequence which is substantially complementary to a portion of the complement of N₂. This primer pair defines a template sequence which bridges the PCAV-specific splice junction. The amplified sequence will be shorter for targets where the splice junction has been used than for unspliced targets (figure 5) or for genomic DNA. For targets where transcription may start in the LTR immediately upstream of the splice acceptor sites (*e.g.* in the second 5' LTR of PCAV, or in the single 5' LTR of other HERVs), the amplified sequence will be shorter than for P CAV targets where transcription started in a more upstream 5' LTR.

In other preferred kits for detecting PCAV products in which a splice acceptor site near the 3' end of the second 5' LTR has been used, either (i) the first primer comprises a sequence which is substantially identical to a portion of N₁ and the second primer comprises a sequence which is substantially complementary to a portion of PCAV sequence downstream of a splice donor which is itself downstream of the splice acceptors near the 3' end of the second PCAV 5' LTR, or (ii) the first primer comprises a sequence which is substantially identical to a portion of the complement of N₁ and the second primer comprises a sequence which is substantially complementary to a portion of the complement of a PCAV sequence downstream of a splice donor which is itself downstream of the splice acceptors near the 3' end of the second PCAV 5' LTR. The primers are located either side of exon 2 and thus define a template sequence which bridges exon 2. The amplified sequence will be longer in targets where the exon is present than in targets where the exon absent (figure 6A *vs*. 6B) and only PCAV targets can give the longer amplification product. All splice products, whether or not including the exon, will give shorter amplification products than unspliced RNA or genomic DNA targets.

In other preferred kits for detecting PCAV products in which a splice acceptor site near the 3' end of the second 5' LTR has been used, either (i) the first primer comprises a sequence which is substantially identical to the splice junction site in N₁-N₂ and the second primer comprises a sequence which is substantially complementary to a portion of a PCAV sequence upstream or downstream of the splice junction site, or (ii) the first primer comprises a sequence which is substantially identical to the complement of the splice junction site in N₁-N₂ and the second primer comprises a sequence which is substantially complementary to a portion of a PCAV upstream or sequence downstream of the splice junction site. The first primer comprises a first sequence which is substantially complementary to a portion of N₁ and a second sequence which is substantially complementary to a portion of N₂ and can hybridize to targets where the splice junction has been used but not to targets where the splice junction has not been used. Amplification from such primer pairs will only occur where the target sequence has been formed by use of the splice junction, and will not occur with unspliced targets or genomic DNA.

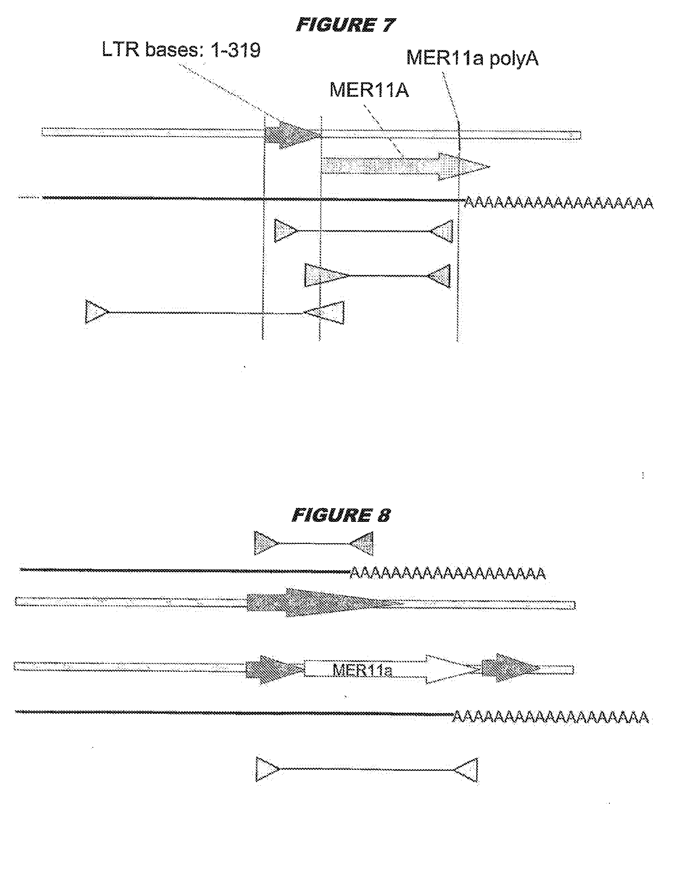

In preferred kits for detecting the 3' region of PCAV products, either (i) the first primer comprises a sequence which is substantially identical to a portion of N₃ and the second primer comprises a sequence which is substantially complementary to a portion of N₄, or (ii) the first primer comprises a sequence which is substantially identical to a portion of the complement of N₃ and the second primer comprises a sequence which is substantially complementary to a portion of the complement of N₄. The primer pair amplifies a template sequence which bridges the 3' LTR/MER11a junction and amplification will occur only where the target sequence contains both a 3' LTR sequence and a MER11a sequence (figure 7).

In other preferred kits for detecting the 3' region of PCAV products, either (i) the first primer comprises a first sequence which is substantially identical to a portion of N₃ and a second sequence which is substantially identical to a portion of N₄, and the second primer comprises a sequence which is substantially complementary to a portion of an upstream or downstream PCAV sequence, or (ii) the first primer comprises a first sequence which is substantially identical to a portion of the complement of N₃ and a second sequence which is substantially identical to a portion of the complement of N₄, and the second primer comprises a sequence which is substantially complementary to a portion of the complement of an upstream or downstream PCAV sequence. The first primer hybridizes only to targets which contain both a 3' LTR sequence and a MER11a sequence, such that amplification occurs only where the target sequence contains both a 3' LTR sequence and a MER11a sequence (figure 7). The second primer is preferably located in exon 3, so the amplification product is shorter than in the genome.

In other preferred kits for detecting the 3' region of PCAV products, either (i) the first primer comprises a sequence which is substantially identical to a portion of N₃ and the second primer comprises a sequence which is substantially complementary to a portion of a polyA tail, or (ii) the first primer comprises a sequence which is substantially identical to a portion of the complement of N₃ and the second primer comprises a sequence which is substantially complementary to a portion of the complement of polyA tail. The template sequence defined by this primer pair is longer in targets where the 3' LTR contains a MER11a insertion than in targets (*e.g.* other HERVs) where the 3' LTR is intact (figure 8). PolyA-specificity means that genomic DNA is not amplified.

In preferred kits for detecting PCAV products containing alu-interrupted env, either (i) the first primer comprises a sequence which is substantially identical to a portion of N₇ and the second primer comprises a sequence which is substantially complementary to a portion of N₈, or (ii) the first primer comprises a sequence which is substantially identical to a portion of the complement of N₇ and the second primer comprises a sequence which is substantially complementary to a portion of the complement of N₈. The primer pair amplifies a template sequence which bridges the env/alu junction and amplification will occur only where the target sequence contains both an env sequence and an alu sequence.

In other preferred kits for detecting PCAV products containing alu-interrupted env, either (i) the first primer comprises a first sequence which is substantially identical to a portion of N₇ and a second sequence which is substantially identical to a portion of N₈, and the second primer comprises a sequence which is substantially complementary to a portion of an upstream or downstream PCAV sequence, or (ii) the first primer comprises a first sequence which is substantially identical to a portion of the complement of N₇ and a second sequence which is substantially identical to a portion of the complement of N₈, and the second primer comprises a sequence which is substantially complementary to a portion of the complement of an upstream or downstream PCAV sequence. The first primer hybridizes only to targets which contain both an alu sequence and an env sequence, such that amplification occurs only where the target sequence contains both an alu sequence and an env sequence.

In further preferred kits for detecting PCAV products containing alu-interrupted env, either (i) the first primer comprises a sequence which is substantially identical to a portion of N₉ and the second primer comprises a sequence which is substantially complementary to a portion of N₁₀, or (ii) the first primer comprises a sequence which is substantially identical to a portion of the complement of N₉ and the second primer comprises a sequence which is substantially complementary to a portion of the complement of N_{10.} The primer pair amplifies a template sequence which bridges the end of the alu interruption.

In other preferred kits for detecting PCAV products containing alu-interrupted env, either (i) the first primer comprises a first sequence which is substantially identical to a portion of N₉ and a second sequence which is substantially identical to a portion of N₁₀. and the second primer comprises a sequence which is substantially complementary to a portion of an upstream or downstream PCAV sequence, or (ii) the first primer comprises a first sequence which is substantially identical to a portion of the complement of N₉ and a second sequence which is substantially identical to a portion of the complement of N₁₀, and the second primer comprises a sequence which is substantially complementary to the complement of an upstream or downstream PCAV sequence. The first primer hybridizes only to targets which contain the alu-interrupted env.

Another preferred kit comprises either (i) a first primer comprising a sequence which is substantially identical to a first portion of SEQ ID 111, 112 or 53 and a second primer comprising a sequence which is substantially complementary to a second portion of SEQ ID 111, 112 or 53, or (ii) a first primer comprising a sequence which is substantially identical to a first portion of the complement of SEQ ID 111, 112 or 53 and a second primer comprising a sequence which is substantially complementary to a second portion of the complement of SEQ ID 111, 112 or 53, such that the primer pair defines a template sequence within, consisting of or comprising SEQ ID 111, 112 or 53.

### B.6 - General features of nucleic acids of the invention

Nucleic acids and transcripts of the invention are preferably provided in isolated or substantially isolated form *i.e*. substantially free from other nucleic acids (*e.g*. free from naturally-occurring nucleic acids), generally being at least about 50% pure (by weight), and usually at least about 90% pure.

Nucleic acids of the invention can take various forms.

Nucleic acids of the invention may be single-stranded or double-stranded. Unless otherwise specified or required, any embodiment of the invention that utilizes a nucleic acid may utilize both the double-stranded form and each of two complementary single-stranded forms which make up the double-stranded form. Primers and probes are generally single-stranded, as are antisense nucleic acids.

Nucleic acids of the invention may be circular or branched, but will generally be linear.

Nucleic acid of the invention may be attached to a solid support (*e.g*. a bead, plate, filter, film, slide, microarray support, resin, *etc*.)

For certain embodiments of the invention, nucleic acids are preferably at least 7 nucleotides in length (*e.g*. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 90, 100, 110, 120, 130, 140, 150, 160, 170, 180, 190, 200, 225, 250, 275, 300 nucleotides or longer).

For certain embodiments of the invention, nucleic acids are preferably at most 500 nucleotides in length (*e.g*. 450, 400, 350, 300, 250, 200,150, 140, 130, 120, 110, 100, 90, 80, 75, 70, 65, 60, 55, 50, 45, 40, 39, 38, 37, 36, 35, 34, 33, 32, 31, 30, 29, 28, 27, 26, 25, 24, 23, 22, 21, 20, 19, 18, 17, 16, 15 nucleotides or shorter).

Primers and probes of the invention, and other nucleic acids used for hybridization, are preferably between 10 and 30 nucleotides in length (*e.g*. 10, 11, 12, 13, 14, 15, 16, 17, 18, 19, 20, 21, 22, 23, 24, 25, 26, 27, 28, 29, or 30 nucleotides).

Nucleic acids of the invention may be carry a detectable label *e.g*. a radioactive or fluorescent label, or a biotin label. This is particularly useful where the nucleic acid is to be used in nucleic acid detection techniques *e.g*. where the nucleic acid is a probe or a primer.

Nucleic acids of the invention comprise PCAV sequences, but they may also comprise non-PCAV sequences (*e.g*. in nucleic acids of formula 5'-X-Y-Z-3', as defined above). This is particularly useful for primers, which may thus comprise a first sequence complementary to a PCAV nucleic acid target and a second sequence which is not complementary to the nucleic acid target. Any such non-complementary sequences in the primer are preferably 5' to the complementary sequences. Typical non-complementary sequences comprise restriction sites {26} or promoter sequences {27}.

Nucleic acids of the invention can be prepared in many ways *e.g.* by chemical synthesis (at least in part), by digesting longer nucleic acids using nucleases (*e.g*. restriction enzymes), by joining shorter nucleic acids (*e.g*. using ligases or polymerases), from genomic or cDNA libraries, *etc*.

Nucleic acids of the invention may be part of a vector *i.e*. part of a nucleic acid construct designed for transduction/transfection of one or more cell types. Vectors may be, for example, "cloning vectors" which are designed for isolation, propagation and replication of inserted nucleotides, "expression vectors" which are designed for expression of a nucleotide sequence in a host cell, "viral vectors" which is designed to result in the production of a recombinant virus or virus-like particle, or "shuttle vectors", which comprise the attributes of more than one type of vector. A "host cell" includes an individual cell or cell culture which can be or has been a recipient of exogenous nucleic acid. Host cells include progeny of a single host cell, and the progeny may not necessarily be completely identical (in morphology or in total DNA complement) to the original parent cell due to natural, accidental, or deliberate mutation and/or change. Host cells include cells transfected or infected *in vivo* or *in vitro* with nucleic acid of the invention.

The term "nucleic acid" includes in general means a polymeric form of nucleotides of any length, which contain deoxyribonucleotides, ribonucleotides, and/or their analogs. It includes DNA, RNA, DNA/RNA hybrids. It also includes DNA or RNA analogs, such as those containing modified backbones (*e.g*. peptide nucleic acids (PNAs) or phosphorothioates) or modified bases. The term "nucleic acid" is not intended to be limiting as to the length or structure of a nucleic acid unless specifically indicated, and the following are non-limiting examples of nucleic acids: a gene or gene fragment, mRNA, tRNA, rRNA, ribozymes, cDNA, recombinant nucleic acids, branched nucleic acids, plasmids, vectors, DNA from any source, RNA from any source, probes, and primers. Where nucleic acid of the invention takes the form of RNA, it may have a 5' cap.

Where a nucleic acid is DNA, it will be appreciated that "U" in a RNA sequence will be replaced by "T" in the DNA. Similarly, where a nucleic acid is RNA, it will be appreciated that "T" in a DNA sequence will be replaced by "U" in the RNA.

The term "complement" or "complementary" when used in relation to nucleic acids refers to Watson-Crick base pairing. Thus the complement of C is G, the complement of G is C, the complement of A is T (or U), and the complement of T (or U) is A. It is also possible to use bases such as I (the purine inosine) *e.g*. to complement pyrimidines (C or T). The terms also imply a direction - the complement of 5'-ACAGT-3' is 5'-ACTGT-3' rather than 5'-TGTCA-3'.

Nucleic acids of the invention can be used, for example: to produce polypeptides; as hybridization probes for the detection of nucleic acid in biological samples; to generate additional copies of the nucleic acids; to generate ribozymes or antisense oligonucleotides; as single-stranded DNA primers or probes; or as triple-strand forming oligonucleotides. The nucleic acids are preferably uses to detect PCAV nucleic acid targets such as PCAV mRNAs.

References to a percentage sequence identity between two nucleic acid sequences mean that, when aligned, that percentage of bases are the same in comparing the two sequences. This alignment and the percent homology or sequence identity can be determined using software programs known in the art, for example those described in section 7.7.18 of reference 28. A preferred alignment program is GCG Gap (Genetics Computer Group, Wisconsin, Suite Version 10.1), preferably using default parameters, which are as follows: open gap = 3; extend gap = 1.

The percentage values of *a, aa, b, bbb, c, ccc, d, e, eee, f, fff, g, h, hh, i, ii, j, kkk, mm, mmm, n, nn, nnn, pp, qq, qqq, rrr, s, uu, vv* and *ww* as used above may each independently be 50, 55, 60, 65, 70, 75, 80, 85, 90, 91, 92, 93, 94, 95, 96, 97, 98, 99, 99.5, 99.9 or 100. The values of each of *a*, *aa, b, bbb, c, ccc, d, e, eee, f, fff, g, h, hh, i, ii, j, mm, n, nn, pp,* qq, s, *uu, vv* and *ww* may be the same or different as each other. Nucleic acid sequences which include 'silent' changes (*i.e.* which do not affect the encoded amino acid for a codon) are examples of these nucleic acids.

The values of *ddd, ggg, hhh, jj, jjj, k, kk, l, ll, m, p, ppp, q, r, rr, ss, t, tt, u, v, w* and y as used above may each independently be 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50, 60, 70, 80, 90, 100 or more. The values of each of *ddd, ggg, jj, k, kk, l, ll, m, p, q, r, rr, ss, t, tt, u, v, w* and *y* may be the same or different as each other.

The value of *x*+*z* is at least 1 (*e.g*. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 60, 70, 80, 90, 100 *etc*.). It is preferred that the value of *x*+*y*+*z* is at least 8 (*e.g*. at least 9, 10, 11, 12, 13, 14, 15, 16, 17, 18, 19, 20, 21, 22, 23, 24, 25, 30, 35, 40, 45, 50, 60, 70, 80, 90, 100 *etc*.). It is preferred that the value of x+y+z is at most 500 (*e.g*. at most 450, 400, 350, 300, 250, 200, 190, 180, 170, 160, 150, 140, 130, 120, 110, 100, 90, 80, 70, 60, 50, 40, 30, 25, 20, 19, 18, 17, 16, 15, 14, 13, 12, 11, 10, 9, 8).

The percentage values of *bb* and *cc* as used above are independently each preferably less than 60 (*e.g.* 50, 40, 30, 20, 10), or may even be 0. The values of *bb* and *cc* may be the same or different as each other.

Preferred nucleic acids of the invention comprise nucleotide sequences which remain unmasked following application of a masking program for masking low complexity (*e.g*. XBLAST).

Where a nucleic acid is said to "encode" a polypeptide, it is not necessarily implied that the polynucleotide is translated, but it will include a series of codons which encode the amino acids of the polypeptide.

It is preferred that the invention does not encompass: (i) nucleic acid comprising a nucleotide sequence disclosed in reference 1; (ii) nucleic acid comprising a nucleotide sequence within SEQ IDs 1 to 225 in reference 1; (iii) a known nucleic acid; (iv) nucleic acid comprising SEQ ID 505, 506, 507, 508 or 509 from reference 29; (v) nucleic acid comprising SEQ ID 407 from references 30, 31 or 32; (vi) nucleic acid comprising SEQ ID 591 from references 30, 31 or 32; (vii) nucleic acid comprising SEQ ID 2192 from reference 33; (viii) nucleic acid comprising diagnostic protein #19115 from reference 34; (ix) nucleic acid comprising SEQ ID 37169 from reference 35; (x) nucleic acid comprising probe nos. 11882, 12335, 12181, 11701 or 24114 from reference 36; (xi) nucleic acid comprising probe nos. 9239 or 9663 from reference 37; (xii) nucleic acid comprising SEQ ID 12094 or 12516 from reference 38; (xiii) nucleic acid comprising SEQ ID 12377 or 12795 from reference 39; (xiv) nucleic acid comprising probe nos. 8509, 8960 or 17545 from reference 40; (xv) nucleic acid comprising probe nos. 12376, 12685, 12194, 25151 or 25457 from reference 41; (xvi) nucleic acid comprising nucleic acid 4609 from reference 42; (xvii) nucleic acid comprising SEQ ID 3685, 12135 or 13658 from reference 43; (xviii) a nucleic acid known as of 7th December 2001 (*e.g*. a nucleic acid whose sequence is available in a public database such as GenBank or GeneSeq before 7th December 2001); or (xix) a nucleic acid known as of 10th June 2002 (*e.g*. a nucleic acid whose sequence is available in a public database such as GenBank or GeneSeq before 10th June 2002).

### C - DETECTING POLYPEPTIDE EXPRESSION PRODUCTS

Where the method is based on polypeptide detection, it will involve detecting expression of a polypeptide encoded by a PCAV RNA transcript. This will typically involve detecting one or more of the following polypeptides: gag (*e.g*. SEQ ID 57) or PCAP3/mORF (*e.g*. SEQ ID 87). Although some PCAV mRNAs encode all of these polypeptides (*e.g*, ERVK6 {44}), PCAV is an old virus and its prt, pol and env genes are highly fragmented.

The transcripts which encode HML-2 polypeptides are generated by alternative splicing of the full-length mRNA copy of the endogenous genome {*e.g*. Figure 4 of ref. 45, Figure 1 of ref. 54}. PCAV_gag polypeptide is encoded by the first long ORF in the genome (nucleotides 2813-4683 of SEQ ID 1; SEQ ID 54). Full-length gag polypeptide is proteolytically cleaved. PCAV prt polypeptide is encoded by the second long ORF in the genome and is translated as a gag-prt fusion polypeptide which is proteolytically cleaved to give the protease. PCAV ppl polypeptide is encoded by the third long ORF in the genome and is translated as a gag-prt-pol fusion polypeptide which is proteolytically cleaved to give three pol products - reverse transcriptase, endonuclease and integrase {46}. PCAV env polypeptide is encoded by the fourth long ORF in the genome. The translated polypeptide is proteolytically cleaved. PCAY__cORF polypeptide is encoded by an ORF which shares the same 5' region and start codon as env, but in which a splicing event removes env-coding sequences and shifts to a reading frame +1 relative to that of env {47, 48}. PCAP3 polypeptide is encoded by an ORF which shares the same 5' region and start codon as env, but in which a splicing event removes env-coding sequences and shifts to a reading frame +2 relative to that of env (the third reading frame).

### C.1 - Direct detection of HML-2 polypeptides

Various techniques are available for detecting the presence or absence of a particular polypeptides in a sample. These are generally immunoassay techniques which are based on the specific interaction between an antibody and an antigenic amino acid sequence in the polypeptide. Suitable techniques include standard immunohistological methods, ELISA, RIA, FIA, immunoprecipitation, immunofluorescence, *etc.*

Polypeptides of the invention can also be detected by functional assays *e.g.* assays to detect binding activity or enzymatic activity. For instance, functional assays for cORF are disclosed in references 48 to 50, and a functional assay for the protease is disclosed in reference 51. PCAP3 has been found to cause apoptosis in primary prostate epithelial cells and, when apoptosis is suppressed, to enable cells to expand beyond their normal senescence point.

Another way of detecting polypeptides of the invention is to use standard proteomics techniques *e.g.* purify or separate polypeptides and then use peptide sequencing. For example, polypeptides can be separated using 2D-PAGE and polypeptide spots can be sequenced (*e.g.* by mass spectroscopy) in order to identify if a sequence is present in a target polypeptide.

Techniques may require the enrichment of target polypeptides prior to detection. However, immunofluorescence assays can be easily performed on cells without the need for such enrichment. Cells may first be fixed onto a solid support, such as a microscope slide or microtiter well. The membranes of the cells can then be permeablized in order to permit entry of antibody (NB: fixing and permeabilization can be achieved together). Next, the fixed cells can be exposed to fluorescently-labeled antibody which is specific for the polypeptide. The presence of this label identifies cells which express the target PCAV polypeptide. To increase the sensitivity of the assay, it is possible to use a second antibody to bind to the anti-PCAV antibody, with the label being earned by the second antibody. {52}

### C.2 - Indirect detection of HML-2 polypeptides

Rather than detect polypeptides directly, it may be preferred to detect molecules which are produced by the body in response to a polypeptide (*i.e*. indirect detection of a polypeptide). This will typically involve the detection of antibodies, so the patient sample will generally be a blood sample. Antibodies can be detected by conventional immunoassay techniques *e.g.* using PCAV polypeptides of the invention, which will typically be immobilized.

Antibodies against HERV-K polypeptides have been detected in humans {*e.g*. 45, 53, 54} *e.g*. in seminoma or teratocareinoma tissue.

### C.3 - Polypeptide materials

The invention provides polypeptides which can be used in detection methods of the invention, wherein the polypeptides are encoded by a human endogenous retrovirus located at megabase 20.428 on chromosome 22.

The invention provides a polypeptide comprising an amino acid sequence selected from the group consisting of SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188. SEQ IDs 54, 55, 56, 87, 98 and 110 are preferred members of this group.

The invention also provides (a) a polypeptide comprising a fragment of at least *dd* amino acids of one or more of SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188, and (b) a polypeptide comprising an amino acid sequence having at least *ee*% identity to one or more of SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188. These polypeptides include variants (*e.g*. allelic variants, homologs, orthologs, mutants, *etc*.).

The fragment of (a) may comprise a T-cell or, preferably, a B-cell epitope of SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188. T- and B-cell epitopes can be identified empirically (*e.g*. using PEPSCAN {55, 56} or similar methods), or they can be predicted (*e.g.* using the Jameson-Wolf antigenic index {57}, matrix-based approaches {58}, TEPTROPE {59}, neural networks {60}, OptiMer & EpiMer {61, 62}, ADEPT {63}, Tsites {64}, hydrophilicity {65}, antigenic index {66} or the methods disclosed in reference 67 *etc..*

Preferred fragments of (a) are SEQ IDs 55, 56 and 110, or are fragments of SEQ IDs 55, 56 or 110. SEQ IDs 55, 56 & 110 are found within the PCAV gag protein and are particularly useful for detecting PCAV expression above background expression of other HERV-Ks.

Within (b), the polypeptide may, compared to SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188, comprise one or more conservative amino acid replacements *i.e*. replacements of one amino acid with another which has a related side chain. Genetically-encoded amino acids are generally divided into four families: (1) acidic *i.e*. aspartate, glutamate; (2) basic *i.e.* lysine, arginine, histidine; (3) non-polar *i.e.* alanine, valine, leucine, isoleucine, proline, phenylalanine, methionine, tryptophan; and (4) uncharged polar *i.e*. glycine, asparagine, glutamine, cystine, serine, threonine, tyrosine. Phenylalanine, tryptophan, and tyrosine are sometimes classified jointly as aromatic amino acids. In general, substitution of single amino acids within these families does not have a major effect on the biological activity.

The invention also provides a polypeptide having formula NH₂-XX-YY-ZZ-COOH, wherein: XX is a polypeptide sequence consisting of *xx* amino acids; ZZ is a polypeptide sequence consisting of *zz* amino acids; YY is a polypeptide sequence consisting of a fragment of *yy* amino acids of an amino acid sequence selected from the group consisting of SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188; and said polypeptide NH₂-XX-NY-ZZ-COOH is not a fragment of a polypeptide sequence selected from SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94,95,96,97,98,110,1186 and 1188.

The sequence of -XX- preferably shares less than *ff*% sequence identity to the *xx* amino acids which are N-terminus to sequence -YY- in SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188. The sequence of -ZZ- preferably shares less than *gg*% sequence identity to the *zz* amino acids which are C-terminus to sequence -YY- in SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188.

Polypeptides of the invention can be prepared in various forms (*e.g*. native, fusions, glycosylated, non-glycosylated, myristoylated, non-myristoylated, lipdated, non-lipidated, monomeric, multimeric, particulate, denatured, *etc*.).

Polypeptides of the invention maybe attached to a solid support.

Polypeptides of the invention may comprise a detectable label (*e.g.* a radioactive or fluorescent label, or a biotin label).

Polypeptides of the invention can be prepared in many ways *e.g*. by chemical synthesis (at least in part), by digesting longer polypeptides using proteases, by translation from RNA, by purification from cell culture (*e.g*. from recombinant expression), from the organism itself (*e.g*. isolation from prostate tissue), from a cell line source *etc*.

The term "polypeptides" refers to amino acid polymers of any length. The polymer may be linear or branched, it may comprise modified amino acids, and it may be interrupted by non-ammo acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc*.), as well as other modifications known in the art. Polypeptides can occur as single chains or associated chains. Polypeptides of the invention can be naturally or non-naturally glycosylated (*i.e.* the polypeptide has a glycosylation pattern that differs from the glycosylation pattern found in the corresponding naturally occurring polypeptide).

In general, the polypeptides of the invention are provided in a non-naturally occurring environment *e.g*. they are separated from their naturally-occurring environment. In certain embodiments, the polypeptide is present in a composition that is enriched for the polypeptide as compared to a control. Polypeptides of the invention are thus preferably provided in isolated or substantially isolated form *i.e*. the polypeptide is present in a composition that is substantially free of other expressed polypeptides, where by substantially free is meant that less than 75% (by weight), preferably less than 50%, and more preferably less than 10% (*e.g*. 5%) of the composition is made up of other expressed polypeptides.

Mutants can include amino acid substitutions, additions or deletions. The amino acid substitutions can be conservative amino acid substitutions or substitutions to eliminate non-essential amino acids, such as to alter a glycosylation site, a phosphorylation site or an acetylation site, or to minimize misfolding by substitution or deletion of one or more cysteine residues that are not necessary for function. Conservative amino acid substitutions are those that preserve the general charge, hydrophobicity/hydrophilicity, and/or steric bulk of the amino acid substituted. Variants can be designed so as to retain or have enhanced biological activity of a particular region of the polypeptide (*e.g*. a functional domain and/or, where the polypeptide is a member of a polypeptide family, a region associated with a consensus sequence). Selection of amino acid alterations for production of variants can be based upon the accessibility (interior *vs*, exterior) of the amino acid (*e.g*. ref. 68), the thermostability of the variant polypeptide (*e.g*. ref. 69), desired glycosylation sites (*e.g*. ref. 70), desired disulfide bridges (*e.g.* refs. 71 & 72), desired metal binding sites (*e.g*. refs. 73 & 74), and desired substitutions with in proline loops (*e.g.* ref. 75). Cysteine-depleted muteins can be produced as disclosed in reference 76.

The percentage value of *ee* as used above may be 50, 60, 65, 70, 75, 80, 85, 90, 91, 92, 93, 94, 95, 96, 97, 98, 99, 99.5, 99.9 or 100.

The percentage values of *ff* and *gg* as used above are independently each preferably less than 60 (*e.g*. 50, 40, 30, 20, 10), or may even be 0. The values of *ff* and *gg* may be the same or different as each other.

The values of *dd, xx*, *yy* and *zz* as used above may each independently be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50, 60, 70, 75, 80, 90, 100 or more. The values of each of *dd,* xx, *yy* and *zz* may be the same or different as each other. The value of *dd* may be less than 2000 (*e.g,* less than 1000, 500, 100, or 50).

The value of *xx*+*zz* is at least 1 (*e.g*. at least 2, 3, 4, 5, 6, 7, 8, 9,10, 11, 12, 13, 14, 15, 16, 17, 18, 19, 20, 21, 22, 23, 24, 25, 26, 27, 28, 29, 30, 35, 40, 45, 50, 60, 70, 80, 90, 100 *etc*.). It is preferred that the value of *xx*+*yy*+*zz* is at least 8 (*e.g*. at least 9, 10, 11, 12, 13, 14, 15, 16, 17, 18, 19, 20, 21, 22, 23, 24, 25, 30, 35, 40, 45, 50, 60, 70, 80, 90, 100 *etc*.). It is preferred that the value of *xx*+*yy*+*zz* is at most 500 (*e.g.* at most 450, 400, 350, 300, 250, 200, 190, 180, 170, 160, 150, 140, 130, 120, 110, 100, 90, 80, 70, 60, 50, 40, 30, 25, 20, 19, 18, 17, 16, 15, 14, 13, 12, 11, 10, 9, 8).

Polypeptides of the invention are generally at least 7 amino acids in length (*e.g*. 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 90, 100, 110, 120, 130, 140, 150, 160, 170, 180, 190, 200, 225, 250, 275, 300 amino acids or longer).

For certain embodiments of the invention, polypeptides are preferably at most 500 amino acids in length (*e.g.* 450, 400, 350, 300, 250, 200, 150, 140, 130, 120, 110, 100, 90, 80, 75, 70, 65, 60, 55, 50, 45, 40, 39, 38, 37, 36, 35, 34, 33, 32, 31, 30, 29, 28, 27, 26, 25, 24, 23, 22, 21, 20, 19, 18, 17, 16, 15 ammo acids or shorter).

References to a percentage sequence identity between two amino acid sequences means that, when aligned, that percentage of amino acids are the same in comparing the two sequences. This alignment and the percent homology or sequence identity can be determined using software programs known in the art, for example those described in section 7.7.18 of reference 28. A preferred alignment is determined by the Smith-Waterman homology search algorithm using an affine gap search with a gap open penalty of 12 and a gap extension penalty of 2, BLOSUM matrix of 62. The Smith-Waterman homology search algorithm is taught in reference 77.

Preferred polypeptides of the invention comprise amino acid sequences which remain unmasked following application of a masking program for masking low complexity (*e.g*. XBLAST).

It is preferred that the invention does not encompass: (i) polypeptides comprising an amino acid sequence disclosed in reference 1; (ii) polypeptides comprising an amino acid sequence within SEQ IDs 1 to 225 in reference 1; (iii) a polypeptide comprising SEQ ID 592 from references 30, 30 or 32; (iv) a known polypeptide; (v) a polypeptide known as of 7th December 2001 (*e.g*. a polypeptide whose sequence is available in a public database such as GenBank or GeneSeq before 7th December 2001); or (vi) a polypeptide known as of 10th June 2002 (*e.g*. a polypeptide whose sequence is available in a public database such as GenBank or GeneSeq before 10th June 2002),

### C.4 - Antibody materials

The invention provides antibody that binds to a polypeptide of the invention. The invention also provides antibody that binds to a polypeptide encoded by a nucleic acid of the invention.

Preferred antibodies of the invention recognize epitopes within SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188. More preferred antibodies of the invention recognize epitopes within SEQ IDs 54, 55, 56 or 110.

Other preferred antibodies of the invention recognize a HERV-K. gag protein. The antibody may (a) recognize gag from PCAV and also from one or more further HERV-Ks, (b) recognize gag from PCAV but not from any other HERV-Ks, (c) recognize gag from PCAV and also from one or more old HERV-Ks, but not from new HERV-Ks, or (d) recognize gag from one or more HBRV-Ks but not from PCAV. A preferred antibody in group (a) is 5G2; a preferred antibody in group (c) is 5A5.

Antibodies of the invention may be polyclonal or monoclonal.

Antibodies of the invention may be produced by any suitable means *e.g*. by recombinant expression, or by administering (*e.g*. injecting) a polypeptide of the invention to an appropriate animal (*e.g*. a rabbit, hamster, mouse or other rodent).

Antibodies of the invention may include a label. The label may be detectable directly, such as a radioactive or fluorescent label. Alternatively, the label may be detectable indirectly, such as an enzyme whose products are detectable (*e.g*. luciferase, β-galactosidase, peroxidase *etc.*).

Antibodies of the invention may be attached to a solid support.

In general, antibodies of the invention are provided in a non-naturally occurring environment *e.g.* they are separated from their naturally-occurring environment. In certain embodiments, the antibodies are present in a composition that is enriched for them as compared to a control. Antibodies of the invention are thus preferably provided in isolated or substantially isolated form *i.e.* the antibody is present in a composition that is substantially free of other antibodies, where by substantially free is meant that less than 75% (by weight), preferably less than 50%, and more preferably less than 10% (*e.g*. 5%) of the composition is made up of other antibodies.

The term "antibody" includes any suitable natural or artificial immunoglobulin or derivative thereof. In general, the antibody will comprise a Fv region which possesses specific antigen-binding activity. This includes, but is not limited to: whole immunoglobulins, antigen-binding immunoglobulin fragments (*e.g*. Fv, Fab, F(ab')₂ *etc.*), single-chain antibodies (*e.g*. scFv), oligobodies, chimeric antibodies, humanized antibodies, veneered antibodies, *etc*.

To increase compatibility with the human immune system, the antibodies may be chimeric or humanized {*e.g*. refs. 78 & 79}, or fully human antibodies may be used. Because humanized antibodies are far less immunogenic in humans than the original non-human monoclonal antibodies, they can be used for the treatment of humans with far less risk of anaphylaxis. Thus, these antibodies may be preferred in therapeutic applications that involve *in vivo* administration to a human such as, use as radiation sensitizers for the treatment of neoplastic disease or use in methods to reduce the side effects of cancer therapy.

Humanized antibodies may be achieved by a variety of methods including, for example: (1) grafting non-human complementarity determining regions (CDRs) onto a human framework and constant region ("humanizing"), with the optional transfer of one or more framework residues from the non-human antibody; (2) transplanting entire non-human variable domains, but "cloaking" them with a human-like surface by replacement of surface residues ("veneering"). In the present invention, humanized antibodies will include both "humanized" and "veneered" antibodies. {refs. 80 to 86}. CDRs are amino acid sequences which together define the binding affinity and specificity of a Fv region of a native immunoglobulin binding site {*e.g*. 87 & 88}.

The phrase "constant region" refers to the portion of the antibody molecule that confers effector functions. In chimeric antibodies, mouse constant regions are substituted by human constant regions. The constant regions of humanized antibodies are derived from human immunoglobulins. The heavy chain constant region can be selected from any of the 5 isotypes: alpha, delta, epsilon, gamma or mu, and thus antibody can be of any isotype (*e.g.* IgG, IgA, IgM, IgD, IgE). IgG is preferred, which may be of any subclass (*e.g.* IgG₁, IgG₂).

Humanized or fully-human antibodies can also be produced using transgenic animals that are engineered to contain human immunoglobulin loci. For example, ref. 89 discloses transgenic animals having a human Ig locus wherein the animals do not produce functional endogenous immunoglobulins due to the inactivation of endogenous heavy and light chain loci. Ref. 90 also discloses transgenic non-primate mammalian hosts capable of mounting an immune response to an immunogen, wherein the antibodies have primate constant and/or variable regions, and wherein the endogenous immunoglobulin-encoding loci are substituted or inactivated. Ref. 91 discloses the use of the Cre/Lox system to modify the immunoglobulin locus in a mammal, such as to replace all or a portion of the constant or variable region to form a modified antibody molecule. Ref. 92 discloses non-human mammalian hosts having inactivated endogenous Ig loci and functional human Ig loci. Ref. 93 discloses methods of making transgenic mice in which the mice lack endogenous heavy chains, and express an exogenous immunoglobulin locus comprising one or more xenogeneic constant regions.

Using a transgenic animal described above, an immune response can be produced to a PCAV polypeptide, and antibody-producing cells can be removed from the animal and used to produce hybridomas that secrete human monoclonal antibodies. Immunization protocols, adjuvants, and the like are known in the art, and are used in immunization of, for example, a transgenic mouse as described in ref. 94. The monoclonal antibodies can be tested for the ability to inhibit or neutralize the biological activity or physiological effect of the corresponding polypeptide.

It is preferred that the invention does not encompass: (i) antibodies which recognize a polypeptide disclosed in reference 1; (ii) antibodies which recognize a polypeptide comprising an amino acid sequence within SEQ IDs 1 to 225 in reference 1; (iii) known antibodies; (iv) an antibody known as of 7th December 2001 (*e.g.* a polypeptide whose sequence is available in a public database such as GenBank or GeneSeq before 7th December 2001); or (v) an antibody known as of 10th June 2002 (*e.g.* a polypeptide whose sequence is available in a public database such as GenBank or GeneSeq before 10th June 2002).

### D - PATIENT SAMPLES AND NORMAL SAMPLES

### D.1 - The patient sample

Where the diagnostic method of the invention is based on detecting mRNA expression, the patient sample will generally comprise cells (*e.g.* prostate cells, particularly those from the luminal epithelium). These may be present in a sample of tissue (*e.g.* prostate tissue), or may be cells which have escaped into circulation (*e.g.* during metastasis). Instead of or as well as comprising prostate cells, the sample may comprise virions which contain PCAV mRNA.

Where the diagnostic method of the invention is based on detecting polypeptide expression, the patient sample may comprise cells, preferably, prostate cells and/or virions (as described above for mRNA), or may comprise antibodies which recognize PCAV polypeptides. Such antibodies will typically be present in circulation.

In general, therefore, the patient sample is tissue sample, preferably, a prostate sample (*e.g.* a biopsy) or a blood sample. Other possible sources of patient samples include isolated cells, whole tissues, or bodily fluids (*e.g.* blood, plasma, serum, urine, pleural effusions, cerebro-spinal fluid, *etc*.). Another preferred patient sample is a semen sample.

The patient is generally a human, preferably a human male, and more preferably an adult human male.

Expression products may be detected in the patient sample itself, or may be detected in material derived from the sample (*e.g.* the supernatant of a cell lysate, a RNA extract, cDNA generated from a RNA extract, polypeptides translated from a RNA extract, cells derived from culturing cells extracted from a patient *etc*.). These are still considered to be "patient samples" within the meaning of the invention.

Detection methods of the invention can be conducted *in vitro* or *in vivo.*

### D.2 - Controls

PCAV transcripts are up-regulated in prostate tumors. To detect such up-regulation, a reference point is typically needed *i.e.* a control. Analysis of the control sample gives a standard level of mRNA and/or protein expression against which a patient sample can be compared. As PCAV transcription is negligible in normal cells and highly up-regulated in tumor cells, however, a reference point may not always be necessary - significant expression indicates disease. Even so, the use of controls is preferable, particularly for standardization or for quantitative assays.

A negative control gives a background or basal level of expression against which a patient sample can be compared. Higher levels of expression product relative to a negative control indicate that the patient from whom the sample was taken has a prostate tumor. Conversely, equivalent levels of expression product indicate that the patient does not have a PCAV-related cancer.

A negative control will generally comprise material from cells which are not tumor cells. The negative control could be a sample from the same patient as the patient sample, but from a tissue in which PCAV expression is not up-regulated *e.g.* a non-tumor non-prostate cell. The negative control could be a prostate cell from the same patient as the patient sample, but taken at an earlier stage in the patient's life (*e.g.* before the development of cancer, or from a BPH patient).. The negative control could be a cell from a patient without a prostate tumor, and this cell may or may not be a prostate cell. The negative control could be a suitable cell line. Typically, the negative control will be the same tissue or cell type as the patient sample being tested (*e.g.* a prostate cell or a blood sample).

A positive control gives a level of expression against which a patient sample can be compared. Equivalent or higher levels of expression product relative to a positive control indicate that the patient from whom the sample was taken has a prostate tumor. Conversely, lower levels of expression product indicate that the patient does not have a PCAV-related tumor.

A positive control will generally comprise material from tumor cells or from a blood sample taken from a patient known to have a tumor. The positive control could be a prostate tumor cell from the same patient as the patient sample, but taken at an earlier stage in the patient's life (*e.g* to monitor remission). The positive control could be a cell from another patient with a prostate tumor. The positive control could be a suitable prostate cell line.

Other suitable positive and negative controls will be apparent to the skilled person.

PCAV expression in the control can be assessed at the same time as expression in the patient sample. Alternatively, PCAV expression in the control can be assessed separately (earlier or later). Rather than actually compare two samples, however, the control may be an absolute value *i.e.* a level of expression which has been empirically determined from samples taken from prostate tumor patients (*e.g.* under standard conditions). Examples of such negative controls for prostate tumors include lifetime baseline levels of expression or the expression level *e.g.* as observed in pooled normals.

### D.3 - Degree of up-regulation

The up-regulation relative to the control (100%) will usually be at least 150% (*e.g* 200%, 250%, 300%, 400%, 500%, 600% or more). A twenty- to forty-fold up-regulation is not uncommon.

### E - DIAGNOSTIC METHODS AND DIAGNOSIS

The invention provides a method for diagnosing prostate cancer, comprising the step of detecting in a patient sample the presence or absence of an expression product of a human endogenous retrovirus located at megabase 20.428 on chromosome 22.

### E.1 - Products for use in diagnosis

Preferred expression products for detection in diagnostic methods of the invention are described in sections B.1, B.3 and C.3 above.

Preferred reagents for use in diagnostic methods of the invention are described in sections B.4, C.3 and C.4 above.

Preferred kits for use in diagnostic methods of the invention are described in section B.5 above.

The invention provides nucleic acids, polypeptides and antibodies of the invention for use in diagnosis.

The invention also provides the use of nucleic acids, polypeptides and antibodies of the invention in the manufacture of diagnostic assays.

### E.2 - mRNA-based methods of the invention

The invention provides a method for analyzing a patient sample, comprising the steps of: (a) contacting the patient sample with nucleic acid of the invention under hybridizing conditions; and (b) detecting the presence or absence of hybridization of nucleic acid of the invention to nucleic acid present in the patient sample. The presence of hybridization in step (b) indicates that the patient from whom the sample was taken has a prostate tumor.

The invention also provides a method for analyzing a patient sample, comprising the steps of: (a) enriching mRNA in the sample relative to DNA to give a mRN-A-enriched sample; (b) contacting the mRNA-enriched sample with nucleic acid of the invention under hybridizing conditions; and (c) detecting the presence or absence of hybridization of nucleic acid of the invention to mRNA present in the mRNA-enriched sample. The presence of hybridization in step (c) indicates that the patient from whom the sample was taken has a prostate tumor. The enrichment in step (a) may take the form of extracting mRNA without extracting DNA, removing DNA without removing mRNA, or disrupting PCAV DNA without disrupting PCAV mRNA *etc.* (see section B.2 above).

The invention also provides a method for analyzing a patient sample, comprising the steps of: (a) preparing DNA copies of RNA in the sample; (b) contacting the DNA copies with nucleic acid of the invention under hybridizing conditions; and (c) detecting the presence or absence of hybridization of nucleic acid of the invention to said DNA copies. The presence of hybridization in step (c) indicates that the patient from whom the sample was taken has a prostate tumor. Preparation of DNA in step (a) may be specific to PCAV (*e.g.* by using RT-PCR with appropriate primers) or may be non-specific (*e.g.* preparation of cellular cDNA).

In the above methods for analyzing a patient sample, the nucleic acid of the invention contacted with the sample may be a probe of the invention. As an alternative, it may comprise primers of the invention, in which case the relevant step of the method will generally involve two or more (*e.g.* 3, 4, 5, 6, 7, 8, 9,10 or more) cycles of amplification. Where primers are used, the method may involve the use of a probe for detecting hybridization to amplified DNA.

The invention also provides a method for analyzing a patient sample, comprising the steps of: (a) amplifying any PCAV nucleic acid targets in the sample; and (b) detecting the presence or absence of amplified targets. The presence of amplified targets in step (b) indicates that the patient from whom the sample was taken has a prostate tumor.

These methods of the invention may be qualitative, quantitative, or semi-quantitative.

### E.3 - Polypeptide-based methods of the invention

The invention provides an immunoassay method for diagnosing prostate cancer, comprising the step of contacting a patient sample with a polypeptide or antibody of the invention.

The invention also provides a method for analyzing a patient blood sample, comprising the steps of: (a) contacting the blood sample with a polypeptide of the invention; and (b) detecting the presence or absence of interaction between said polypeptide and antibodies in said sample. The presence of an interaction in step (b) indicates that the patient from whom the blood sample was taken has raised anti-PCAV antibodies, and thus that they have a prostate tumor. Step (a) may be preceded by a step wherein antibodies in the blood sample are enriched.

The invention also provides a method for analyzing a patient sample, comprising the steps of: (a) contacting the sample with antibody of the invention; and (b) detecting the presence or absence of interaction between said antibody and said sample. The presence of an interaction in step (b) indicates that the patient from whom the sample was taken is expressing PCAV polypeptides, and thus that they have a prostate tumor. Step (a) may be preceded by a step wherein cells in the sample are lysed or permeabilized and/or wherein polypeptides in the sample are enriched.

These methods of the invention maybe qualitative, quantitative, or semi-quantitative.

The above methods may be adapted for *use in vivo* (*e.g.* to locate or identify sites where tumor cells are present). In these embodiments, an antibody specific for a target PCAV polypeptide is administered to an individual (*e.g.* by injection) and the antibody is located using standard imaging techniques (*e.g.* magnetic resonance imaging, computerized tomography scanning, *etc.*). Appropriate labels (*e.g.* spin labels *etc.*) will be used. Using these techniques, cancer cells are differentially labeled.

Other *in vivo* methods may detect PCAV polypeptides functionally. For instance, a construct comprising a PCAV LTR operatively linked to a reporter gene (*e.g.* a fluorescent protein such as GFP) will be expressed in parallel to native PCAV polypeptides.

To increase the sensitivity of immunoassays, it is possible to use a second antibody to bind to the anti-PCAV antibody, with a label being carried by the second antibody.

### E.4 - The meaning of "diagnosis"

The invention provides a method for diagnosing prostate cancer. It will be appreciated that "diagnosis" according to the invention can range from a definite clinical diagnosis of disease to an indication that the patient should undergo further testing which may lead to a definite diagnosis. For example, the method of the invention can be used as part of a screening process, with positive samples being subjected to further analysis.

Furthermore, diagnosis includes monitoring the progress of cancer in a patient already known to have the cancer. Cancer can also be staged by the methods of the invention. Preferably, the cancer is prostate cancer.

The efficacy of a treatment regimen (therametries) of a cancer associated can also monitored by the method of the invention *e.g.* to determine its efficacy.

Susceptibility to a cancer can also be detected *e.g.* where up-regulation of expression has occurred, but before cancer has developed. Prognostic methods are also encompassed.

All of these techniques fall within the general meaning of "diagnosis" in the present invention.

### F - PHARMACEUTICAL COMPOSITIONS

The invention provides a pharmaceutical composition comprising nucleic acid, polypeptide, or antibody of the invention. The invention also provides their use as medicaments, and their use in the manufacture of medicaments for treating prostate cancer. The invention also provides a method for raising an immune response, comprising administering an immunogenic dose of nucleic acid or polypeptide of the invention to an animal (*e.g.* to a patient).

Pharmaceutical compositions encompassed by the present invention include as active agent, the nucleic acids, polypeptides, or antibodies of the invention disclosed herein in a therapeutically effective amount. An "effective amount" is an amount sufficient to effect beneficial or desired results, including clinical results. An effective amount can be administered in one or more administrations. For purposes of this invention, an effective amount is an amount that is sufficient to palliate, ameliorate, stabilize, reverse, slow or delay the symptoms and/or progression of prostate cancer.

The compositions can be used to treat cancer as well as metastases of primary cancer. In addition, the pharmaceutical compositions can be used in conjunction with conventional methods of cancer treatment, e.g. to sensitize tumors to radiation or conventional chemotherapy. The terms "treatment", "treating", "treat" and the like are used herein to generally refer to obtaining a desired pharmacologic and/or physiologic effect. The effect may be prophylactic in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject which may be predisposed to the disease or symptom but has not yet been diagnosed as having it; (b) inhibiting the disease symptom, *i.e.* arresting its development; or (c) relieving the disease symptom, *i.e.* causing regression of the disease or symptom.

Where the pharmaceutical composition comprises an antibody that specifically binds to a gene product encoded by a differentially expressed nucleic acid, the antibody can be coupled to a drug for delivery to a treatment site or coupled to a detectable label to facilitate imaging of a site comprising cancer cells, such as prostate cancer cells. Methods for coupling antibodies to drugs and detectable labels are well known in the art, as are methods for imaging using detectable labels.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms. The precise effective amount for a subject will depend upon the subject's size and health, the nature and extent of the condition, and the therapeutics or combination of therapeutics selected for administration. The effective amount for a given situation is determined by routine experimentation and is within the judgment of the clinician. For purposes of the present invention, an effective dose will generally be from about 0.01mg/kg to about 5 mg/kg, or about 0.01 mg/kg to about 50 mg/kg or about 0.05 mg/kg to about 10 mg/kg of the compositions of the present invention in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which can be administered without undue toxicity. Suitable carriers can be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art. Pharmaceutically acceptable carriers in therapeutic compositions can include liquids such as water, saline, glycerol and ethanol. Auxiliary substances, such as wetting or emulsifying agents, pH buffering substances, and the like, can also be present in such vehicles, Typically, the therapeutic compositions are prepared as injectables, either as liquid solutions or suspensions; solid forms suitable for solution in, or suspension in, liquid vehicles prior to injection can also be prepared. Liposomes are included within the definition of a pharmaceutically acceptable carrier. Pharmaceutically acceptable salts can also be present in the pharmaceutical composition, e.g. mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in reference 95.

The composition is preferably sterile and/or pyrogen-free. It will typically be buffered at about pH 7.

Once formulated, the compositions contemplated by the invention can be (1) administered directly to the subject (*e.g.* as nucleic acid, polypeptides, small molecule agonists or antagonists, and the like); or (2) delivered *ex vivo,* to cells derived from the subject (*e.g.* as in *ex vivo* gene therapy). Direct delivery of the compositions will generally be accomplished by parenteral injection, e.g. subcutaneously, intraperitoneally, intravenously or intramuscularly, intratumoral or to the interstitial space of a tissue. Other modes of administration include oral and pulmonary administration, suppositories, and transdermal applications, needles, and gene guns or hyposprays. Dosage treatment can be a single dose schedule or a multiple dose schedule.

Methods for the *ex vivo* delivery and reimplantation of transformed cells into a subject are known in the art {*e.g.* ref. 96}. Examples of cells useful in *ex vivo* applications include, for example, stem cells, particularly hematopoetic, lymph cells, macrophages, dendritic cells, or tumor cells. Generally, delivery of nucleic acids for both ex vivo and in vitro applications can be accomplished by, for example, dextran-mediated transfection, calcium phosphate precipitation, polybrene mediated transfection, protoplast fusion, electroporation, encapsulation of the nucleic acid(s) in liposomes, and direct microinjection of the DNA into nuclei, all well known in the art.

Differential expression of PCAV nucleic acids has been found to correlate with prostate tumors. The tumor can be amenable to treatment by administration of a therapeutic agent based on the provided nucleic acid, corresponding polypeptide or other corresponding molecule (*e.g.* antisense, ribozyme, *etc.*). In other embodiments, the disorder can be amenable to treatment by administration of a small molecule drug that, for example, serves as an inhibitor (antagonist) of the function of the encoded gene product of a gene having increased expression in cancerous cells relative to normal cells or as an agonist for gene products that are decreased in expression in cancerous cells (*e.g.* to promote the activity of gene products that act as tumor suppressors).

The dose and the means of administration of the inventive pharmaceutical compositions are determined based on the specific qualities of the therapeutic composition, the condition, age, and weight of the patient, the progression of the disease, and other relevant factors. For example, administration of nucleic acid therapeutic compositions agents includes local or systemic administration, including injection, oral administration, particle gun or catheterized administration, and topical administration. Preferably, the therapeutic nucleic acid composition contains an expression construct comprising a promoter operably linked to a nucleic acid of the invention. Various methods can be used to administer the therapeutic composition directly to a specific site in the body. For example, a small metastatic lesion is located and the therapeutic composition injected several times in several different locations within the body of tumor. Alternatively, arteries which serve a tumor are identified, and the therapeutic composition injected into such an artery, in order to deliver the composition directly into the tumor. A tumor that has a necrotic center is aspirated and the composition injected directly into the now empty center of the tumor. An antisense composition is directly administered to the surface of the tumor, for example, by topical application of the composition. X-ray imaging may be used to assist in certain of the above delivery methods.

Targeted delivery of therapeutic compositions containing an antisense nucleic acid, subgenomic nucleic acids, or antibodies to specific tissues can also be used. Receptor-mediated DNA delivery techniques are described in, for example, references 97 to 102. Therapeutic compositions containing a nucleic acid are administered in a range of about 100 ng to about 200 mg of DNA for local administration in a gene therapy protocol. Concentration ranges of about 500 ng to about 50 mg, about 1 µg to about 2 mg, about 5 µg to about 500 µg, and about 20 µg to about 100 µg of DNA can also be used during a gene therapy protocol. Factors such as method of action (*e.g.* for enhancing or inhibiting levels of the encoded gene product) and efficacy of transformation and expression are considerations which will affect the dosage required for ultimate efficacy of the antisense subgenomic nucleic acids. Where greater expression is desired over a larger area of tissue, larger amounts of antisense subgenomic nucleic acids or the same amounts re-administered in a successive protocol of administrations, or several administrations to different adjacent or close tissue portions of, for example, a tumor site, may be required to effect a positive therapeutic outcome. In all cases, routine experimentation in clinical trials will determine specific ranges for optimal therapeutic effect.

The therapeutic nucleic acids and polypeptides of the present invention can be delivered using gene delivery vehicles. The gene delivery vehicle can be of viral or non-viral origin (see generally references 103, 104, 105 and 106). Expression of such coding sequences can be induced using endogenous mammalian or heterologous promoters. Expression of the coding sequence can be either constitutive or regulated.

Viral-based vectors for delivery of a desired nucleic acid and expression in a desired cell are well known in the art. Exemplary viral-based vehicles include, but are not limited to, recombinant retroviruses (*e.g.* references 107 to 117), alphavirus-based vectors (*e.g.* Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532)), adenovirus vectors, and adeno-associated virus (AAV) vectors (*e.g.* see refs, 118 to 123). Administration of DNA linked to killed adenovirus {124} can also be employed.

Non-viral delivery vehicles and methods can also be employed, including, but not limited to, polycationic condensed DNA linked or unlinked to killed adenovirus alone {*e.g.* 124}, ligand-linked DNA {125}, eukaryotic cell delivery vehicles cells {*e.g.* refs. 126 to 130} and nucleic charge neutralization or fusion with cell membranes. Naked DNA can also be employed. Exemplary naked DNA introduction methods are described in refs. 131 and 132. Liposomes that can act as gene delivery vehicles are described in refs. 133 to 137. Additional approaches are described in refs. 138 & 139.

Further non-viral delivery suitable for use includes mechanical delivery systems such as the approach described in ref. 139. Moreover, the coding sequence and the product of expression of such can be delivered through deposition of photopolymerized hydrogel materials or use of ionizing radiation {*e.g.* refs. 140 & 141}. Other conventional methods for gene delivery that can be used for delivery of the coding sequence include, for example, use of hand-held gene transfer particle gun {142} or use of ionizing radiation for activating transferred genes {140 & 141}.

### Vaccine compositions

The pharmaceutical composition is preferably an immunogenic composition and is more preferably a vaccine composition. Such compositions can be used to raise antibodies in a mammal (*e.g.* a human).

The composition may additionally comprise an adjuvant. For example, the composition may comprise one or more of the following adjuvants: (1) oil-in-water emulsion formulations (with or without other specific immunostimulating agents such as muramyl peptides (see below) or bacterial cell wall components), such as for example (a) MF59™ {143; Chapter 10 in ref. 144}, containing 5% Squalene, 0.5% Tween 80, and 0.5% Span 85 (optionally containing MTP-PE) formulated into submicron particles using a microfluidizer, (b) SAF, containing 10% Squalane, 0.4% Tween 80, 5% pluronic-blocked polymer L121, and thr-MDP either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CAWS (Detox™): (2) saponin adjuvants, such as QS21 or Stimulon™ (Cambridge Bioscience, Worcester, MA) may be used or particles generated therefrom such as ISCOMs (immunostimulating complexes), which ISCOMS may be devoid of additional detergent {145}; (3) Complete Freund's Adjuvant (CFA) and Incomplete Freund's Adjuvant (IFA); (4) cytokines, such as interleukins (*e.g.* IL-1, IL-2, IL-4, IL-5, IL-6, IL-7, IL-12 *etc.*), interferons (*e.g.* gamma interferon), macrophage colony stimulating factor (M-CSF), tumor necrosis factor (TNF), etc.; (5) monophosphoryl lipid A (MPL) or 3-O-deacylated MPL (3dMPL) {*e.g.* 146, 147}; (6) combinations of 3dMPL with, for example, QS21 and/or oil-in-water emulsions {*e.g.* 148, 149, 150}; (7) oligonucleotides comprising CpG motifs *i.e.* containing at least one CG dinucleotide, with 5-methylcytosine optionally being used in place of cytosine; (8) a polyoxyethylene ether or a polyoxyethylene ester {151}; (9) a polyoxyethylene sorbitan ester surfactant in combination with an octoxynol {152} or a polyoxyethylene alkyl ether or ester surfactant in combination with at least one additional non-ionic surfactant such as an octoxynol {153}; (10) an immunostimulatory oligonucleotide (*e.g.* a CpG oligonucleotide) and a saponin {154}; (11) an immunostimulant and a particle of metal salt {155}; (12) a saponin and an oil-in-water emulsion {156}; (13) a saponin (*e.g.* QS21) + 3dMPL + IL-12 (optionally + a sterol) {157}; (14) aluminium salts, preferably hydroxide or phosphate, but any other suitable salt may also be used (*e.g.* hydroxyphosphate, oxyhydroxide, orthophosphate, sulphate etc. {chapters 8 & 9 of ref. 144}). Mixtures of different aluminium salts may also be used. The salt may take any suitable form (*e.g.* gel, crystalline, amorphous etc.); (15) chitosan; (16) cholera toxin or *E.coli* heat labile toxin, or detoxified mutants thereof {158}; (17) microparticles of poly(a-hydroxy)acids, such as PLG; (18) other substances that act as immunostimulating agents to enhance the efficacy of the composition. Aluminium salts and/or MF59™ are preferred.

Vaccines of the invention may be prophylactic (*i.e.* to prevent disease) or therapeutic (*i.e.* to reduce or eliminate the symptoms of a disease).

Efficacy can be tested by monitoring expression of nucleic acids and/or polypeptides of the invention after administration of the composition of the invention.

### G - SCREENING METHODS AND DRUG DESIGN

The invention provides methods of screening for compounds with activity against cancer, comprising: contacting a test compound with a tissue sample derived from a cell in which PCAV expression is up-regulated, or a cell line; and monitoring PCAV expression in the sample. A decrease in expression indicates potential anti-cancer efficacy of the test compound.

The invention also provides methods of screening for compounds with activity against prostate cancer, comprising: contacting a test compound with a nucleic acid or polypeptide of the invention; and detecting a binding interaction between the test compound and the nucleic acid/polypeptide. A binding interaction indicates potential anti-cancer efficacy of the test compound.

The invention also provides methods of screening for compounds with activity against prostate cancer, comprising: contacting a test compound with a polypeptide of the invention; and assaying the function of the polypeptide. Inhibition of the polypeptide's function (*e*.*g*. loss of protease activity, loss of RNA export, loss of reverse transcriptase activity, loss of endonuclease activity, loss of integrase activity *etc*.) indicates potential anti-cancer efficacy of the test compound.

Typical test compounds include, but are not restricted to, peptides, peptoids, proteins, lipids, metals, nucleotides, nucleosides, small organic molecules, antibiotics, polyamines, and combinations and derivatives thereof. Small organic molecules have a molecular weight of more than 50 and less than about 2,500 daltons, and most preferably between about 300 and about 800 daltons. Complex mixtures of substances, such as extracts containing natural products, or the products of mixed combinatorial syntheses, can also be tested and the component that binds to the target RNA can be purified from the mixture in a subsequent step.

Test compounds may be derived from large libraries of synthetic or natural compounds. For instance, synthetic compound libraries are commercially available from Maybridge Chemical Co. (Trevillet, Cornwall, UK) or Aldrich (Milwaukee, WI). Alternatively, libraries of natural compounds in the form of bacterial, fungal, plant and animal extracts may be used. Additionally, test compounds may be synthetically produced using combinatorial chemistry either as individual compounds or as mixtures.

Agonists or antagonists of the polypeptides of the invention can be screened using any available method known in the art, such as signal transduction, antibody binding, receptor binding, mitogenic assays, chemotaxis assays, *etc*., The assay conditions ideally should resemble the conditions under which the native activity is exhibited *in vivo,* that is, under physiologic pH, temperature, and ionic strength. Suitable agonists or antagonists will exhibit strong inhibition or enhancement of the native activity at concentrations that do not cause toxic side effects in the subject. Agonists or antagonists that compete for binding to the native polypeptide can require concentrations equal to or greater than the native concentration, while inhibitors capable of binding irreversibly to the polypeptide can be added in concentrations on the order of the native concentration.

Such screening and experimentation can lead to identification of an agonist or antagonist of a PCAV polypeptide. Such agonists and antagonists can be used to modulate, enhance, or inhibit PCAV expression and/or function. {159}

The present invention relates to methods of using the polypeptides of the invention to screen compounds for their ability to bind or otherwise modulate, such as, inhibit, the activity of PCAV polypeptides, and thus to identify compounds that can serve, for example, as agonists or antagonists of the PCAV polypeptides. In one screening assay, the PCAV polypeptide is incubated with cells susceptible to the growth stimulatory activity of PCAV, in the presence and absence of a test compound. The PCAV activity altering or binding potential of the test compound is measured. Growth of the cells is then determined. A reduction in cell growth in the test sample indicates that the test compound binds to and thereby inactivates the PCAV polypeptide, or otherwise inhibits the PCAV polypeptide activity.

Transgenic animals (*e*.*g*. rodents) that have been transformed to over-express PCAV genes can be used to screen compounds *in vivo* for the ability to inhibit development of tumors resulting from PCAV over-expression or to treat such tumors once developed. Transgenic animals that have prostate tumors of increased invasive or malignant potential can be used to screen compounds, including antibodies or peptides, for their ability to inhibit the effect of PCAV polypeptide. Such animals can be produced, for example, as described in the examples herein.

Screening procedures such as those described above are useful for identifying agents for their potential use in pharmacological intervention strategies in prostate cancer treatment. Additionally, nucleic acid sequences corresponding to PCAV, including LTRs, may be used to assay for inhibitors of elevated gene expression.

Antisense oligonucleotides complementary to PCAV mRNA can be used to selectively diminish or oblate the expression of the polypeptide. More specifically, antisense constructs or antisense oligonucleotides can be used to inhibit the production of PCAV polypeptide(s) in prostate tumor cells. Antisense mRNA can be produced by transfecting into target cancer cells an expression vector with a PCAV nucleic acid of the invention oriented in an antisense direction relative to the direction of PCAV-mRNA transcription. Appropriate vectors include viral vectors, including retroviral vectors, as well as non-viral vectors. Alternately, antisense oligonucleotides can be introduced directly into target cells to achieve the same goal. Oligonucleotides can be selected/designed to achieve the highest level of specificity and, for example, to bind to a PCAV-mRNA at the initiator ATG.

Monoclonal antibodies to PCAV polypeptides can be used to block the action of the polypeptides and thereby control growth of cancer cells. This can be accomplished by infusion of antibodies that bind to PCAV polypeptides and block their action.

The invention also provides high-throughput screening methods for identifying compounds that bind to a nucleic acid or polypeptide of the invention. Preferably, all the biochemical steps for this assay are performed in a single solution in, for instance, a test tube or microtitre plate, and the test compounds are analyzed initially at a single compound concentration. for the purposes of high throughput screening, the experimental conditions are adjusted to achieve a proportion of test compounds identified as "positive" compounds from amongst the total compounds screened. The assay is preferably set to identify compounds with an appreciable affinity towards the target *e*.*g*. when 0.1% to 1% of the total test compounds from a large compound library are shown to bind to a given target with a Kᵢ of 10µM or less (e,g. 1µM, 100nM, 10nM, or less).

### H - DEFINITIONS

The term "comprising" means "including" as well as "consisting" *e*.*g*. a composition "comprising" X may consist exclusively of X or may include something additional *e*.*g*. X + Y.

The term "about" in relation to a numerical value *x* means, for example, *x*±10%.

The terms "neoplastic cells", "neoplasia", "tumor", "tumor cells", "cancer" and "cancer cells" (used interchangeably) refer to cells which exhibit relatively autonomous growth, so that they exhibit an aberrant growth phenotype characterized by a significant loss of control of cell proliferation (*i*.*e*. de-regulated cell division). Neoplastic cells can be malignant or benign and include prostate cancer derived tissue.

The word "substantially'' does not exclude "completely" *e*.*g*. a composition which is "substantially free" from Y may be completely free from Y. Where necessary, the word "substantially" may be omitted from the definition of the invention.

### BRIEF DESCRIPTION OF DRAWINGS

Figure 1 is a phylogenetic tree showing the relationship between various endogenous retroviral LTRs. "Old" and "new" HERV-K LTRs are highlighted.
Figure 2 illustrates the arrangement the PCAV genome at its 5' end.
Figure 3 illustrates the arrangement the PCAV genome at its 3' end.
Figure 4 shows splicing events which take place in a prior art HERV-K ('HTDV' {45}) to produce env and cORF proteins,
Figure 5 illustrates splicing events at the 5' LTRs of PCAV.
Figure 6 illustrates how splicing events at the tandem 5' LTRs of PCAV (Figure 6B) can be distinguished from those in other HERV-Ks (Figure 6A).
Figure 7 illustrates how primers can be used to specifically detect PCAV mRNA.
Figure 8 illustrates how insertions at the 3' end of PCAV can be exploited to distinguish it from other HERV-Ks.
Figure 9 maps the location of positive array features to the PCAV genome.
Figure 10 shows the results ofRT-PCR analysis of the exon 1-2 splicing event in various tissues. Lanes are: (1) markers; (2) placenta; (3) & (4) brain; (5) testis; (6) prostate; (7) breast; (8) uterus; (9) thyroid; (10) cervix; and (11) lung.
Figure 11 shows the results ofRT-PCR analysis of the exon 1-2 splicing event in cell lines. Lanes are: (1) and (12) markers; (2) Tera1; (3) colo360; (4) PC3; (5) DU145; (6) 22RV1; (7) PCA 2B; (8) LNCaP; (9) RWPE1; (10) RWPE2; and (11) PrEC.
Figure 12 shows fluorescence results obtained using 5G2 monoclonal antibody against: (12B) MDA PCA 2b cells; (12C) PC3 cells; and (12D) NIH3T3 cells. Figure 12A shows MDA PCA 2b cells without 5G2 antibody.

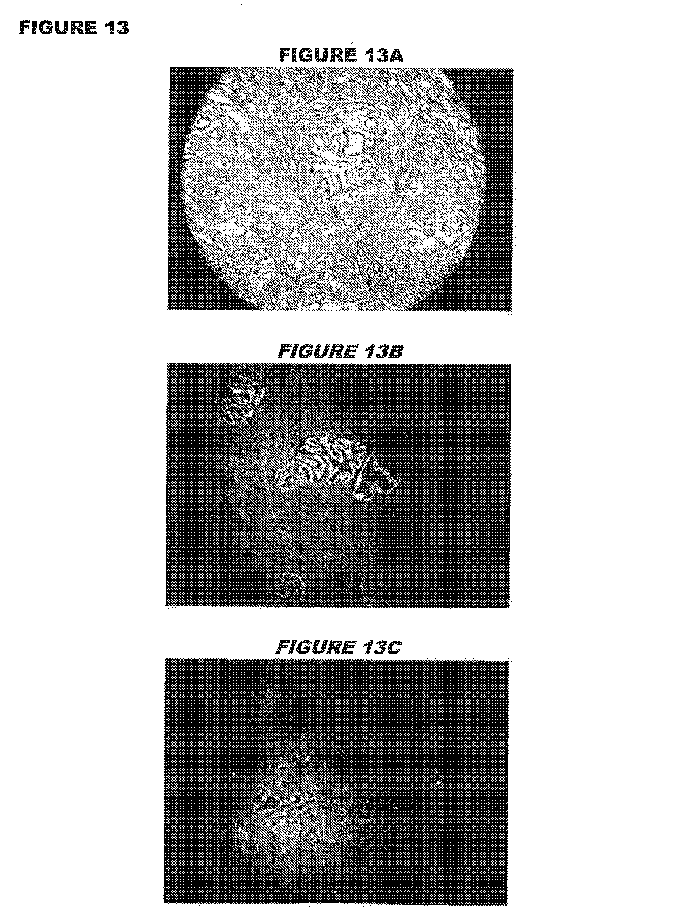

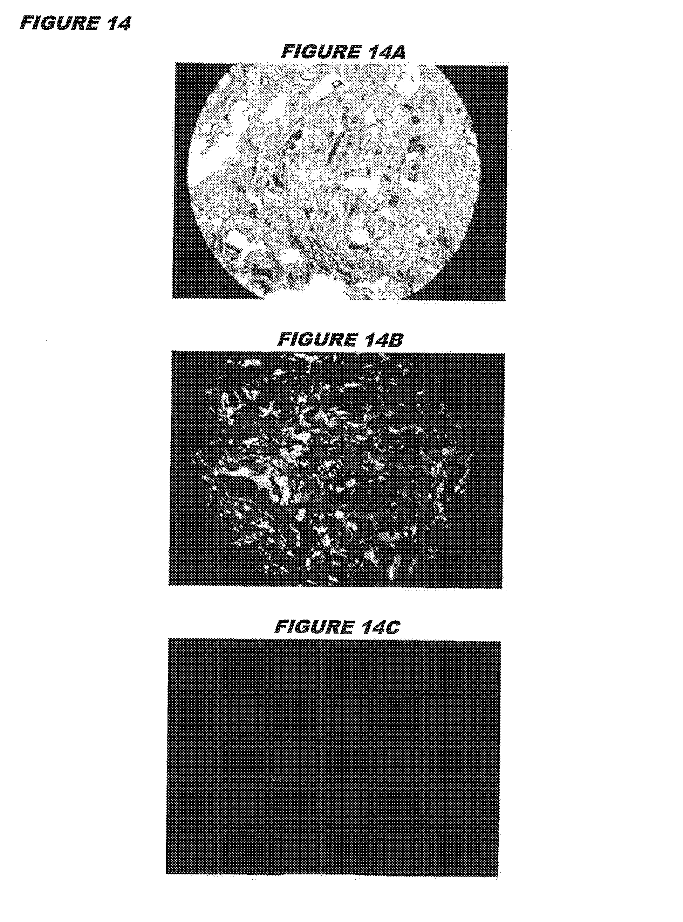

Figures 13 and 14 show staining of prostate tumor samples with (A) hematoxylin & eosin stained, (B) mAb 5G2 plus fluorescein-anti-mouse, or (C) fluorescein-anti-mouse only.
Figure 15 shows expression of HERV-K gag proteins in yeast, with 15A being a stained protein gel and 15B being a western blot.
Figure 16 shows western blots of gag proteins using eight monoclonal antibodies.

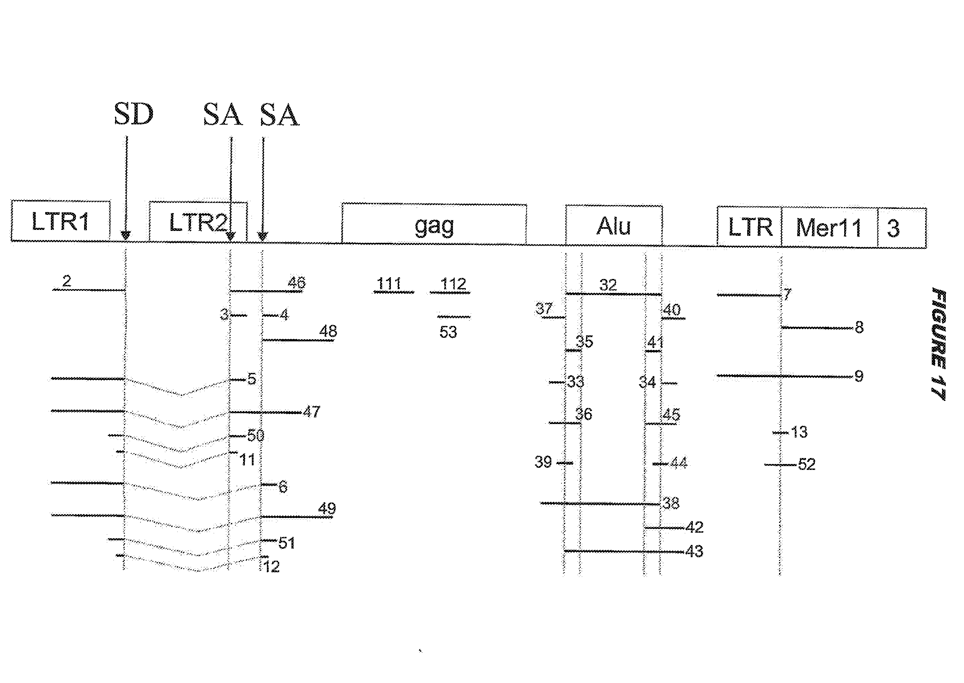

Figure 17 is a not-to-scale schematic of certain SEQ IDs mapped against the genome.
Figure 18 shows microarray analysis of PCAV expression in patient samples. In the expanded portion on the right, the headings indicate Gleason grades of the samples. Red identifies sequences up-regulated in cancer, green identifies those depressed in cancer, and black denotes unchanged spots. Individual sequences are arrayed vertically and patients are presented horizontally. The panel on the left shows all 6000 sequences assayed with RNA from 103 patients, and the region showing almost uniform up-regulation is expanded on the right.
Figure 19 shows the sub-cellular localization of PCAP3 using immuno-staining.
Figure 20 shows PIN staining using anti-gag immunofluorescence. A fresh frozen section of PIN tissue was used, and the assessment of PIN was made by a certified pathologist in an hemotoxylin and eosin stained serial section.
Figure 21 shows TUNEL for cells transfected with PCAP3-encoding adenovirus at moi 100 (top left), 50 (top right), 25 (bottom left), or an untransfected control (bottom right).
Figure 22 shows results from a cell division assay using bromo-deoxyuridine labeling.
Figure 23 shows splicing within the PCAV genome, particularly for env, cORF & PCAP3.
Figure 24 shows the adenovirus vector used in an expression assay to test for LTR activity, and Figure 25 shows the results of GFP expression driven from this vector.
Figure 26 shows the vector used to test the ability of PCAP3 to activate the PCAV LTR.
Figure 27 shows immunofluorescence experiments using an anti-gag monoclonal antibody 5G2 to stain sections of tissue taken from a prostate cancer patient. Figure 27A shows a normal prostate gland, 27B shows atrophied tissue, 27C shows a Gleason grade 3 cancer, and 27D shows a Gleason grade 4 cancer.
Figure 28 shows the position of PCAV-specific primers (*cf.* 5' region of Figure 2), and Figure 29 shows the results of PCR using these primers. 'P' is prostate tissue and 'B' is breast tissue. Figure 30 shows RT-PCR results using the primers. Pairs of matched normal ('N') or cancer ('C') prostate tissue was used, and the signal ratio is given above each pair.
Figure 31 shows quantitative PCR results for various tissues. The y-axis shows PCAV levels normalized to HPRT. The tissues are, from left to right: placenta, fetal brain, fetal heart, fetal liver, brain, heart, liver, pancreas, stomach, small intestine, colon, rectum, testicle, prostate (47 year old man), ovary, adrenal, thyroid, kidney, bladder, breast, uterus, cervix, skeletal muscle, lung, spleen, thymus, skin.
Figure 32 shows the age-related increase in PCAV mRNA expression in prostate tissue.
Figure 33 shows the results of a RT-PCR scanning assay used to map the 5' end of PCAV mRNAs.
Figure 34 gives details of a RNase protection assay. Two antisense probes were used - a long probe (24B) and a short probe (24C). Both probes protected the region shown in 24A. In 24B, the position of the band expected based on the 'usual' 5' end based on the position of the TATA signal is shown, plus the actual band achieved. The three lanes in 24B are: (1) Teral; (2) no RNA; (3) probe, no RNase. The two lanes in 24C are: (1) Teral; (2) probe, no RNase.

### MODES FOR CARRYING OUT THE INVENTION

Certain aspects of the present invention are described in greater detail in the non-limiting examples that follow. The examples are put forth so as to provide those of ordinary skill in the art with a disclosure and description of how to make and use the present invention, and are not intended to limit the scope of what the inventors regard as their invention nor are they intended to represent that the experiments below are all and only experiments performed. Efforts have been made to ensure accuracy with respect to numbers used (*e.g*. amounts, temperature, *etc*.) but some experimental errors and deviations should be accounted for. Unless indicated otherwise, parts are parts by weight, molecular weight is weight average molecular weight, temperature is in degrees Celsius, and pressure is at or near atmospheric.

### Source of human prostate cell samples and isolation of nucleic acids expressed by them

Candidate nucleic acids that may represent genes differentially expressed in cancer were obtained from both publicly-available sources and from cDNA libraries generated from selected cell lines and patient tissues. A normalized cDNA library was prepared from one patient tumor tissue and cloned nucleic acids for spotting on microarrays were isolated from the library. Normal and tumor tissues from 100 patients were processed to generate T7 RNA polymerase transcribed nucleic acids, which were, in turn, assessed for expression in the microarrays.

Normalization: The objective of normalization is to generate a cDNA library in which all transcripts expressed in a particular cell type or tissue are equally represented {refs. 160 & 161}, and therefore isolation of as few as 30,000 recombinant clones in an optimally normalized library may represent the entire gene expression repertoire of a cell, estimated to number 10,000 per cell. The source materials for generating the normalized prostate libraries were cryopreserved prostate tumor tissue from a patient with Gleason grade 3+3 adenocarcinoma and normal prostate biopsies from a pool of at-risk subjects under medical surveillance. Prostate epithelia were harvested directly from frozen sections of tissue by laser capture microdissection (LCM, Arcturus Engineering Inc., Mountain View, CA), carried out according to methods well known in the art (*e*.*g*. ref. 162), to provide substantially homogenous cell samples.

Total RNA was extracted from LCM-harvested cells using RNeasy™ Protect Kit (Qiagen, Valencia, CA), following manufacturer's recommended procedures. RNA was quantified using RiboGreen™ RNA quantification kit (Molecular Probes, Inc. Eugene, OR). One µg of total RNA was reverse transcribed and PCR amplified using SMART™ PCR cDNA synthesis kit (ClonTech, Palo Alto, CA). The cDNA products were size-selected by agarose gel electrophoresis using standard procedures (ref. **21**). The cDNA was extracted using Bio 101Geneclean® II kit (Qbiogene, Carlsbad, CA). Normalization of the cDNA was carried out using kinetics of hybridization principles: 1.0 µg of cDNA was denatured by heat at 100° C for 10 minutes, then incubated at 42°C for 42 hours in the presence of 120 mM NaCl, 10 mM Tris.HCl (pH=8.0), 5 mM EDTA.Na⁺ and 50% formamide. Single-stranded cDNA ("normalized" cDNA) was purified by bydroxyapatite chromatography (#130-0520, BioRad, Hercules, CA) following the manufacturer's recommended procedures, amplified and converted to double-stranded cDNA by three cycles of PCR amplification, and cloned into plasmid vectors using standard procedures (ref. 21). All primers/adaptors used in the normalization and cloning process are provided by the manufacturer in the SMART™ PCR cDNA synthesis kit (ClonTech, Palo Alto, CA). Supercompetent cells (XL-2 Blue Ultracompetent Cells, Stratagene, California) were transfected with the normalized cDNA libraries, plated on plated on solid media and grown overnight at 36°C.

Characterization of normalized libraries: The sequences of 10,000 recombinants per library were analyzed by capillary sequencing using the ABI PRISM 3700 DNA Analyzer (Applied Biosystems, California). To determine the representation of transcripts in a library, BLAST analysis was performed on the clone sequences to assign transcript identity to each isolated clone, i.e. the sequences of the isolated nucleic acids were first masked to eliminate low complexity sequences using the XBLAST masking program (refs. 163, 164 and 165). Generally, masking does not influence the final search results, except to eliminate sequences of relative little interest due to their low complexity, and to eliminate multiple "hits" based on similarity to repetitive regions common to multiple sequences *e.g*. Alu repeats. The remaining sequences were then used in a BLASTN *vs.* GenBank search. The sequences were also used as query sequence in a BLASTX *vs.* NRP (non-redundant proteins) database search.

Automated sequencing reactions were performed using a Perkin-Elmer PRISM Dye Terminator Cycle Sequencing Ready Reaction Kit containing AmpliTaq DNA Polymerase, FS, according to the manufacturer's directions. The reactions were cycled on a GeneAmp PCR System. 9600 as per manufacturer's instructions, except that they were annealed at 20° C. or 30° C. for one minute. Sequencing reactions were ethanol precipitated, pellets were resuspended in 8 microliters of loading buffer, 1.5 microliters was loaded on a sequencing gel, and the data was collected by an ABI PRISM 3700 DNA Sequencer. (Applied Biosystems, Foster City, CA).

The number of times a sequence is represented in a library is determined by performing sequence identity analysis on cloned cDNA sequences and assigning transcript identity to each isolated clone. First, each sequence was checked to see if it was a mitochondrial, bacterial or ribosomal contaminant. Such sequences were excluded from the subsequent analysis. Second, sequence artifacts (*e.g.* vector and repetitive elements) were masked and/or removed from each sequence.

The remaining sequences were compared via BLAST {166} to GenBank and EST databases for gene identification and were compared with each other via FastA {167} to calculate the frequency of cDNA appearance in the normalized DNA library. The sequences were also searched against the GenBank and GeneScq nucleotide databases using the BLASTN program (BLASTN 1.3MP {166}). Fourth, the sequences were analyzed against a non-redundant protein (NRP) database with the BLASTX program (BLASTX 1.3MP {166}). This protein database is a combination of the Swiss-Prot, PIR, and NCBI GenPept protein databases. The BLASTX program was run using the default BLOSUM-62 substitution matrix with the filter parameter: "xnu+seg". The score cutoff utilized was 75.

Assembly of overlapping clones into contigs was done using the program Sequencher (Gene Codes Corp.; Ann Arbor, Mich.). The assembled contigs were analyzed using the programs in the GCG package (Genetic Computer Group, University Research Park, 575 Science Drive, Madison, Wis. 53711) Suite Version 10.1.

### Detection of elevated levels of cDNA associated with prostate cancer using arrays

cDNA sequences representing a variety of candidate genes to be screened for differential expression in prostate cancer were assayed by hybridization on nucleic acid arrays. The cDNA sequences included cDNA clones isolated from cell lines or tissues as described above. The cDNA sequences analyzed also included nucleic acids comprising sequence overlap with sequences in the Unigene database, and which encode a variety gene products of various origins, functionality, and levels of characterization. cDNAs were spotted onto reflective slides (Amersham) according to methods well known in the art at a density of 9,216 spots per slide representing 4608 sequences (including controls) spotted in duplicate, with approximately 0.8 µl of an approximately 200ng/µl solution of cDNA.

PCR products of selected cDNA clones corresponding to the gene products of interest were prepared in a 50% DMSO solution. These PCR products were spotted onto Amersham aluminum microarray slides at a density of 9216 clones per array using a Molecular Dynamics Generation III spotting robot. Clones were spotted in duplicate, giving 4608 different sequences per array.

cDNA probes were prepared from total RNA obtained by laser capture microdissection (LCM, Arcturus Enginering Inc., Mountain View, CA) of tumor tissue samples and normal tissue samples isolated from the patients described above.

Total RNA was first reverse transcribed into cDNA using a primer containing a T7 RNA polymerase promoter, followed by second strand DNA synthesis. cDNA was then transcribed *in vitro* to produce antisense RNA using the T7 promoter-mediated expression (*e*.*g*. ref. 168), and the antisense RNA was then converted into cDNA. The second set of cDNAs were again transcribed *in vitro*, using the T7 promoter, to provide antisense RNA. This antisense RNA was then fluorescently labeled, or the RNA was again converted into cDNA, allowing for third round of T7-mediated amplification to produce more antisense RNA. Thus the procedure provided for two or three rounds of *in vitro* transcription to produce the final RNA used for fluorescent labeling. Probes were labeled by making fluorescently labeled cDNA from the RNA starting material. Fluorescently-labeled cDNAs prepared from the tumor RNA sample were compared to fluorescently labeled cDNAs prepared from normal cell RNA sample. For example, the cDNA probes from the normal cells were labeled with Cy3 fluorescent dye (green) and cDNA probes prepared from the tumor cells were labeled with Cy5 fluorescent dye (red).

The differential expression assay was performed by mixing equal amounts of probes from tumor cells and normal cells of the same patient. The arrays were pre-hybridized by incubation for about 2 hrs at 60°C in 5X SSC/0.2% SDS/1 mM EDTA, and then washed three times in water and twice in isopropanol. Following pre-hybridization of the array, the probe mixture was then hybridized to the array under conditions of high stringency (overnight at 42°C in 50% formamide, 5X SSC, and 0.2% SDS. After hybridization, the array was washed at 55°C three times as follows: 1) first wash in 1X SSC/0.2% SDS; 2) second wash in 0.1X SSC/0.2% SDS; and 3) third wash in 0.1X SSC.

The arrays were then scanned for green and red fluorescence using a Molecular Dynamics Generation III dual color laser-scanner/detector. The images were processed using BioDiscovery Autogene software, and the data from each scan set normalized. The experiment was repeated, this time labeling the two probes with the opposite color in order to perform the assay in both "color directions." Each experiment was sometimes repeated with two more slides (one in each color direction). The data from each scan was normalized, and the level fluorescence for each sequence on the array expressed as a ratio of the geometric mean of 8 replicate spots/genes from the four arrays or 4 replicate spots/gene from 2 arrays or some other permutation.

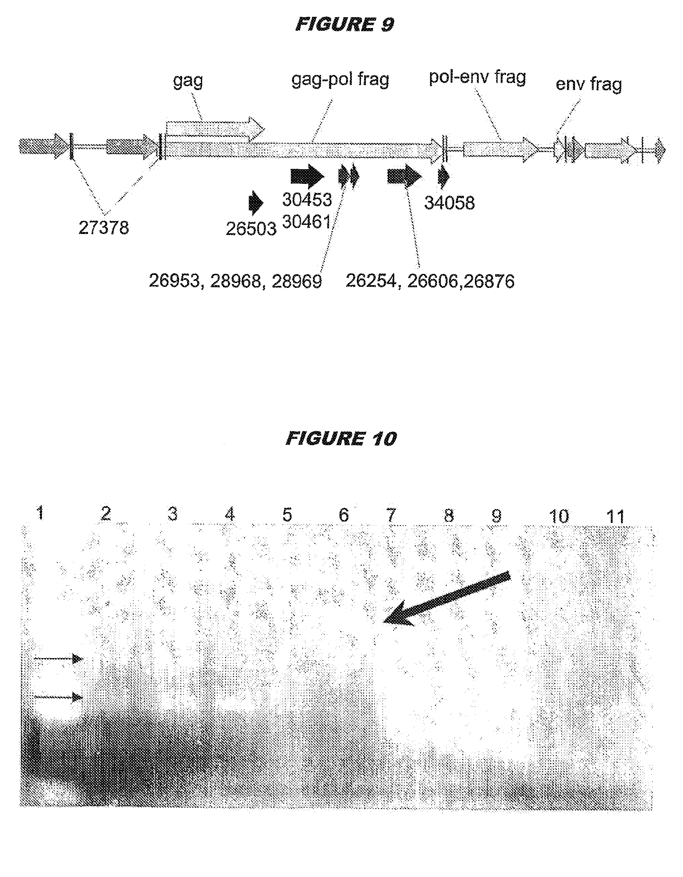

Array features which were found to give elevated signals using prostate tumor tissue were sequenced and mapped to the human genome sequence. The elevated array spots features span about 90% of PCAV and the locations of 11 such sequences on the PCAV genome are shown in figure 9, with five-digit numbers being the codes for individual array features.

Although some of the 11 elevated sequences come from regions in the genome which are highly conserved among the HERV-K HML2.0 family, and will thus not be specific for the virus at megabase 20.428 of chromosome 22, other spots are not.

### Sequence 27378

27378 (SEQ ID 14) is present at elevated levels in prostate tumors. It aligns to two separate regions of the genomic DNA sequence on chromosome 22 (nucleotides 977-1075 & 2700-2777 of SEQ ID 1):

957 1006
PCAV ch22 20.428mb + LTRs (957) GGCCACTCCATCTGGTGCCCAACGTGGATGCTTTTCTCTAGGGTGAAGGG
27378 (1) --------------------AACGTGGATGCTTTTCTCTAGGGTGAAGGG

1007 1056
PCAV ch22 20.428mb + LTRs (1007) ACTCTCGAGTGTGGTCATTGAGGACAAGTCAACGAGAGATTCCCGAGTAC
27378 (31) ACTCTCGAGTGTGGTCATTGAGGACAAGTCAACGAGAGATTCCCGAGTAC

1057 1093
PCAV ch22 20.428mb + LTRs (1057) GTCTACAGTGAGCCTTGTGGTAAGCTTGGGCGCTCGG
27378 (81) GTCTACAGTGAGCCTTGTG------------------

--INTRON 1—

2684 2733
PCAV ch22 20.428mb + LTRs (2684) GGGTGCCTTTCTCTAGGGTGAAGGTACTCTACAGTGTGGTCATTGAGGAC
27378 (100) ----------------GGTGAAGGTACTCTACAGTGTGGTCATTGAGGAC

2734 2783
PCAV ch22 20.428mb + LTRs (2734) AAGTTGACGAGAGAGTCCCAAGTACGTCCACGGTCAGCCTTGCGGTAAGC
27378 (134) AAGTTGACGAGAGAGTCCCAAGTACGTCCACGGTCAGCCTTGCG------

--INTRON 2--

8134 8183
PCAV ch22 20.428mb + LTRs (8134) GAAAATCAGCTTCCTGTTTGGATACCCACTAGACATTT-AAAGTTCTACA
27378 (178) --------------------------------ACATTTTAAAGTTCTACA

8184 8211
PCAV ch22 20.428mb + LTRs (8183) ATGAACTCACTGGAGATGCAAAGAAAAG

27378 (196) ATGAACTCA-------------------

Within SEQ ID 1, nucleotides 1076-1077 are GT and nucleotides 2698-2699 are AG, these being consensus splice donor and acceptor sequences, respectively. Hybridization to 27378 thus verifies splicing in which the first 5' LTR is joined to the splice acceptor site near the 3' end of the second 5' LTR (joins nucleotide 1075 of SEQ ID 1 to nucleotide 2700). Because the sequences in the two exons are from two different viruses (old and new), and these are significantly different from other family new and old family members, it is unlikely that the 27378 product was transcribed from a HERV-K other than PCAV.

### Sequence 34058

Spot 34058 (SEQ ID 15) is highly elevated in prostate tumor tissue. Its sequence spans an alternative splice site that occurs in some "old" genomes and that connects the envelope ATG to a splice acceptor site near the 3' LTR. The sequence matches PCAV more closely (single mismatch at 2443) than the related HERV-Ks found on chromosomes 3 and 6:

1                                                  50
34058 (1) -ACAGAAGGGTACATGAAGGAAAACTGATTTGGTGGAAAGATAATAAAAA
env gemonic PCAV ch 22 20.4 mb (1) -ACAGAAGGGTACATGAAGGAAAACTGATTTGGTGGAAAGATAATAAAAA
env genomic PCAV ch 6 47.1 mb (1) -AGAATAGCCCACATGAAGGAAAACTGATTTGGTGGAAAGACAAGAAAAA
env genomic PCAV ch3 103 mb (1) AAGCACAGCCCACATGAAGGAAAACTAATTTGGTGGAAAGATAATAAAAA

51                                                 100
34058 (50) TAAAACATGGGAAATGGGGAAGGTGATAACGTGGGGGAGAGGTTTTGCTT
env gemonic PCAV ch 22 20.4 mb (50) TAAAACATGGGAAATGGGGAAGGTGATAACGTGGGGGAGAGGTTTTGCTT
env genomic PCAV ch 6 47.1 mb (50) TAAGACATGGGAAATAGGGAAGGTGATAACATGGGAGAGAGGCTTTGCTT
env genomic PCAV ch3 103 mb (51) TAAGACATGGGAAATAGGGAAGGTGATAACGTGGGGGAGAGGTTTTGCTT

101                                                150
34058 (100) GTGTTTCACCAGGAGAAAATCAGCTTCCTGTTTGGATACCCACTAGACAT
env gemonic PCAV ch 22 20.4 mb (100) GTGTTTCACCAGGAGAAAATCAGCTTCCTGTTTGGATACCCACTAGACAT
env genomic PCAV ch 6 47.1 mb (100) GTGTTTCACAAGGAGAAAATCAGCTTCCTGGTTGGATACCCACTAGAAAT
env genomic PCAV ch3 103 mb (101) GTGTTTCACCAGGAGAAAATCAGCTTCCTGTTTGGATACCCACTAGACAT

151                                                200
34058 (150) TTAAAGTTCTACAATGAACTCACTGGAGATGCAAAGAAAAGTGTGGAGAT
env gemonic PCAV ch 22 20.4 mb (150) TTAAAGTTCTACAATGAACTCACTGGAGATGCAAAGAAAAGTGTGGAGAT
env genomic PCAV ch 6 47.1 mb (150) TTAAAGTTCTACAATGAACCCCTTGGAGATGCGAAGAAAAGCGCCTCTGC
env genomic PCAV ch3 103 mb (151) TTGAAGTTCTACAATGAACCCATCGGAGATGCAAAGAAAAGGGCCTCCAC

201                          5' splice junction:   250
34058 (200) GGAGAC------ACCCCA----------ATCGACTCGCCAG:--------
env gemonic PCAV ch 22 20.4 mb (200) GGAGAC------ACCCCA----------ATCGACTCGCCAGGTAAACAAA
env genomic PCAV ch 6 47.1 mb (200) GGAGACAAAAAACCCTCAATTGAGCATCATCGACTCGCCAGGTAAATAAA
env genomic PCAV ch3 103 mb (201) AGAGATGGTAACCC------------------------------------

<intron>

3' splice site:
2201                                               2250
34058 (225) ---------:TCTACAGGTGTATCCAGCAGCTCCAAAGAGACAGCAACCA
env gemonic PCAV ch 22 20.4 mb (2106) CTGTTGTTAGTCTACAGGTGTATCCAGCAGCTCCAAAGAGACAGCAACCA
env genomic PCAV ch 6 47.1 mb (2135) CTGTTGTTAGTCCACAGGTGTATCCACCAGCTCCGAAGAGACAGCGACCA
env genomic PCAV ch3 103 mb (1835) --------------------------------------------------
Consensus (2201) CTGTTGTTAGTCTACAGGTGTATCCAGCAGCTCCAAAGAGACAGCAACCA

2251                                               2300
34058 (265) GCAAGAATGGGCCATAGTGACGATGGTGGTTTTGTCAAAAAGAAAAGGGG
env gemonic PCAV ch 22 20.4 mb (2156) GCAAGAATGGGCCATAGTGACGATGGTGGTTTTGTCAAAAAGAAAAGGGG

env genomic PCAV ch 6 47.1 mb (2185) GCAAGAACGGGCCATAACGACGATGGCAGTTTTGTCAAAAAG-------G
env genomic PCAV ch3 103 mb (1835) -------------------TG-TGCCTGTTTTGTCT----G--------
Consensus (2251) GCAAGAATGGGCCATAGTGACGATGGTGGTTTTGTCAAAAAGAAAAGGGG

2301 2350
34058 (315) GGGATATGTAAGGAAAAG----AGAGATCAGACTTTCACTGTGTCTATGT
env gemonic PCAV ch 22 20.4 mb (2206) GGGATATGTAAGGAAAAG----AGAGATCAGACTTTCACTGTGTCTATGT
env genomic PCAV ch 6 47.1 mb (2228) GGGATATGTAGGGAAAAG----AGAGATCCGACTGTTACTGTGTCTACAT
env genomic PCAV ch3 103 mb (1852) TTGTTATGTGGGGAAAAGCAAGAGAGATCAGATTGTTACTGTGTCTGTGT
Consensus (2301) GGGATATGTAGGGAAAAG    AGAGATCAGACTTTTACTGTGTCTATGT

2351 2400
34058 (361) AGAAA-AGGAAGACATAAGAAACTCCATTTTGATCTGTACTAAGAAAAAT
env gemonic PCAV ch 22 20.4 mb (2252) AGAAA-AGGAAGACATAAGAAACTCCATTTTGATCTGTACTAAGAAAAAT
env genomic PCAV ch 6 47.1 mb (2274) AGAAA-AGGAAGACATAAGAAACTCCATTTTGATCTGTACCCTGAACAAT
env genomic PCAV ch3 103 mb (1902) AGAAAGAAGTAGACATAGGAGACTCCATTTTGTTCTGTACTAAGAAAAAT
Consensus (2351) AGAAA AGGAAGACATAAGAAACTCCATTTTGATCTGTACTAAGAAAAAT

2401 2450
34058 (410) TGTTTTGCCTTGAGATGCTGTTAATCTGTAACTTTAGCCCCAGCCCTGTG
env gemonic PCAV ch 22 20.4 mb (2301) TGTTTTGCCTTGAGATGCTGTTAATCTGTAACTTTAGCCCCAACCCTGTG
env genomic PCAV ch 6 47.1 mb (2323) TGTTTTGCCTTGAGATGCTGTTAATATGTAACTTTAGCCCCAACCTTGTG
env genomic PCAV ch3 103 mb (1952) TCTTCTGCCTTGAGATTCTGTTAATCTATGACCTTACCCCCAACCCCGTG
Consensus (2401) TGTTTTGCCTTGAGATGCTGTTAATCTGTAACTTTAGCCCCAACCCTGTG

2451 2500
34058 (460) CTCACGGAAACATGTGCTGTA---------AGGTTTAAGGATCTAGGGC
env gemonic PCAV ch 22 20.4 mb (2351) CTCACGGAAACATGTGCTGTA---------AGGTTTAAGGATCTAGGGC
env genomic PCAV ch 6 47.1 mb (2373) CTCACGGAAATATGTGTTGTATGGAA-TCAAGGTTTAAGGATCTAGGGC
env genomic PCAV ch3 103 mb (2002) CTCTCTGAAACAGGTGCTGTGTCAAACTCAGGGTTAAATGGATTAAGGGT
Consensus (2451) CTCACGGAAACATGTGCTGTAT  AA TCAAGGTTTAAGGATCTAGGGC

2501 2550
34058 (501) TGT-----------------------------------------------
env gemonic PCAV ch 22 20.4 mb (2392) TGT-----------------------------------------------
env genomic PCAV ch 6 47.1 mb (2422) TGTGCAGGATGTGCCTTGTTAACAAAATGTTTACAGGCAGTATGCTTGGT
env genomic PCAV ch3 103 mb (2052) TGTGCAAGATGTGCTTTGTTAAACAAATGCTTGAAGGCAGCATGCTCCTT
Consensus (2501) TGTGCA GATGTGC TTGTTAA  AAATG TT  AGGCAG ATGCT   T

### Sequence 26254

Signal from sequence 26254 on the array was elevated in prostate tumor tissue compared to normal tissue. The 26254 sequence (SEQ ID 16) aligns almost perfectly to chromosome 22 contigs AP000345 (SEQ ID 17 = nucleotides 63683-64332 of AP000345) and AP000346 (SEQ ID 18 = nucleotides 26271-26920 of AP000346) (nucleotides 7065-7701 of SEQ ID 1):

26254 (1) AGTCATCAAAACTCAGTATCACTTGACTCAAAGAGCAGAGTTGGTTGCCG
AP000346 (1) AGTCATCAAAACTCAGTATCACTTGACTCAAAGAGCAGAGTTGGTTGCCG
AP000345 (1) AGTCATCAAAACTCAGTATCACTTGACTCAAAGAGCAGAGTTGGTTGCCG

26254 (51) TCATTACAGTGTTAACAAGATTTTAATCAGTCTATTAACATTGTATCAGA
AP000346 (51) TCATTACAGTGTTAACAAGATTTTAATCAGTCTATTAACATTGTATCAGA
AP000345 (51) TCATTACAGTGTTAACAAGATTTTAATCAGTCTATTAACATTGTATCAGA

26254 (101) TTCTGCATATGTAGTACAGGCTACAAAGGATATTGAGAGAGCCCTAATCA
AP000346 (101) TTCTGCATATGTAGTACAGGCTACAAAGGATATTGAGAGAGCCCTAATCA
AP000345 (101) TTCTGCATATGTAGTACAGGCTACAAAGGATATTGAGAGAGCCCTAATCA

26254 (151) AATACATTATGGATGATCAGTTAAACCCGCTGTTTAATTTGTTACAACAA
AP000346 (151) AATACATTATGGATGATCAGTTAAACCCGCTGTTTAATTTGTTACAACAA
AP000345 (151) AATACATTATGGATGATCAGTTAAACCCGCTGTTTAATTTGTTACAACAA

26254 (201) AATGTAAGAAAAAGAAATTTCCCATTTTATATTACTCATATTCGAGCACA

AP000346 (201) AATGTAAGAAAAAGAAATTTCCCATTTTATATTACTCATATTCGAGCACA
AP000345 (201) AATGTAAGAAAAAGAAATTTCCCATTTTATATTACTCATATTCGAGCACA

26254 (251) CACTAATTTACCAGGGCCTTTAACTAAAGCAAATGAACAAGCTGACTCGC
AP000346 (251) CACTAATTTACCAGGGCCTTTAACTAAAGCAAATGAACAAGCTGACTTGC
AP000345 (251) CACTAATTTACCAGGGCCTTTAACTAAAGCAAATGAACAAGCTGACTTGC

26254 (301) TAGTATCATCTGCATTCATGGAAGCACAAGACCTTCATGCCTTGACTCAT
AP000346 (301) TAGTATCATCTGCATTCATGGAAGCACAAGAACTTCATGCCTTGACTCAT
AP000345 (301) TAGTATCATCTGCATTCATGGAAGCACAAGAACTTCATGCCTTGACTCAT

26254 (351) GTAAATGCAATAGGATTAAAAAATAAATTTAATATCACATGGAAACAGAC
AP000346 (351) GTAAATGCAATAGGATTAAAAAATAAATTTGATATCACATGGAAACAGAC
AP000345 (351) GTAAATGCAATAGGATTAAAAAATAAATTTGATATCACATGGAAACAGAC

26254 (401) AAAAAATATTGTACAACATTGCACCCAGTGTCAGATTCTACACCTGGCCA
AP000346 (401) AAAAAATATTGTACAACATTGCACCCAGTGTCAGATTCTACACCTGGCCA
AP000345 (401) AAAAAATATTGTACAACATTGCACCCAGTGTCAGATTCTACACCTGGCCA

26254 (451) CTCAGGAGGCAAGAGTTAATCCCAGAGGTCTATGTCCTAATGTGTTATGG
AP000346 (451) CTCAGGAGGCAAGAGTTAATCCCAGAGGTCTATGTCCTAATGTGTTATGG
AP000345 (451) CTCAGGAGGCAAGAGTTAATCCCAGAGGTCTATGTCCTAATGTGTTATGG

26254 (501) CAAATGGATGTCATGCACGTACCTTCATTTGGAAAATTGTCATTTGTCCA
AP000346 (501) CAAATGGATGTCATGCACGTACCTTCATTTGGAAAATTGTCATTTGTCCA
AP000345 (501) CAAATGGATGTCATGCACGTACCTTCATTTGGAAAATTGTCATTTGTCCA

26254 (551) TGTGACAGTTGATACTTATTCACATTTCATATGGGCAACCTGCCAGACAG
AP000346 (551) TGTGACAGTTGATACTTATTCACATTTCATATGGGCAACCTGCCAGACAG
AP000345 (551) TGTGACAGTTGATACTTATTCACATTTCATATGGGCAACCTGCCAGACAG

26254 (601) GAGAAAGTACTTCCCATGTTAAGAGACATTTATTATC
AP000346 (601) GAGAAAGTACTTCCCATGTTAAAAGACATTTATTATCTTGTTTTCCTGTC
AP000345 (601) GAGAAAGTACTTCCCATGTTAAAAGACATTTATTATCTTGTTTTCCTGTC

The four point mutations relative to the chromosome 22 sequence could represent sequencing errors (either for the chromosome or for 26254) or could, alternatively, be SNPs within the human genome.

PCAV is most closely related to HERV-Ks found on chromosomes 3 and 6. Alignment of the chromosome 3, 6 and 22 viruses in the region of 26254 shows that it is unlikely that 26254 is derived from chromosome 3 or 6 and that it is most likely derived from a chromosome 22 PCAV transcript:

ch22 AP000346 (1) AGTCATCAAAACTCAGTATCACTTGACTCAAAGAGCAGAGTTGGTTGCCG
ch22 AP000345 (1) AGTCATCAAAACTCAGTATCACTTGACTCAAAGAGCAGAGTTGGTTGCCG
ch3 103.75 (1) AGTAATCAAAACTCCGTATCAATCAGCTCAAAGAGCAGAGTTGGTTGCAG
ch6 47.1mb (1) ACTCATTGAAACTCAATATCACTGGGCTCAAAGAGCAGAGTTGGTTGCTG

ch22 AP000346 (51) TCATTACAGTGTTAACAAGATTTTAATCAGTCTATTAACATTGTATCAGA
ch22 AP000345 (51) TCATTACAGTGTTAACAAGATTTTAATCAGTCTATTAACATTGTATCAGA
ch3 103.75 (51) TCATTACAGTGTTA-CAAGATTTTGACCAACCTATCAATATTAATATCAGA
ch6 47.1mb (51) TCATTACAGTGTTA-CAAGATTTTAATCAGCCTATTAACATTGTATCAGA

ch22 AP000346 (101) TTCTGCATATGTAGTACAGGCTACAAAGGATATTGAGAGAGCCCTAATCA
ch22 AP000345 (101) TTCTGCATATGTAGTACAGGCTACAAAGGATATTGAGAGAGCCCTAATCA
ch3 103.75 (100) TTCTGCATATGTAGTACAGGCTACAAGGGATGTTGAGACAGCTCTAATTA
ch6 47.1mb (100) TTCTGCATATGTAGTACAGGCTACAAAGGATGTTGAGACAGCCCTAATCA

ch22 AP000346 (151) AATACATTATGGATGATCAGTTAAACCCGCTGTTTAATTTGTTACAACAA
ch22 AP000345 (151) AATACATTATGGATGATCAGTTAAACCCGCTGTTTAATTTGTTACAACAA
ch3 103.75 (150) AATATAGCACGGACGATCATTTAAACCAGCTATTCAATTTATTACAACAA
ch6 47.1mb (150) AATATAGTATAGATGATCAGTTAAACCAGCTGTTTAATATGTTACAACAA

ch22 AP000346 (201) AATGTAAGAAAAAGAAATTTCCCATTTTATATTACTCATATTCGAGCACA
ch22 AP000345 (201) AATGTAAGAAAAAGAAATTTCCCATTTTATATTACTCATATTCGAGCACA
ch3 103.75 (200) ACTGTAAGAAAAAGAAATTTCCCATTTTATATTACTCATATTCGAGCACA
ch6 47.1mb (200) ACTGTAAGAAAAAGAAATTTCCCATTTTATGTTACTCATATTCGAGCACA

ch22 AP000346 (251) CACTAATTTACCAGGGCCTTTAACTAAAGCAAATGAACAAGCTGACTTGC
ch22 AP000345 (251) CACTAATTTACCAGGGCCTTTAACTAAAGCAAATGAACAAGCTGACTTGC
ch3 103.75 (250) CACTAATTTACCAGGGCCTTTGACTAAAGCAAATGAACAAGCTGACTTAC
ch6 47.1mb (250) TACTAATTTACCAGAGCCTTTAACTAAAGCAAATGAACAAGTTGACTTGC

ch22 AP000346 (301) TAGTATCATCTGCATTCATGGAAGCACAAGAACTTCATGCCTTGACTCAT
ch22 AP000345 (301) TAGTATCATCTGCATTCATGGAAGCACAAGAACTTCATGCCTTGACTCAT
ch3 103.75 (300) TGGTATCATCTGCATTCATAAAAGCACAAGAACTTCTTGCTTTGACTCAT
ch6 47.1mb (300) TAGTATCATCTGCATTCCTGGAAGCACAAGAACTTCATGCCTTGACTCAT

ch22 AP000346 (351) GTAAATGCAATAGGATTAAAAAATAAATTTGATATCACATGGAAACAGAC
ch22 AP000345 (351) GTAAATGCAATAGGATTAAAAAATAAATTTGATATCACATGGAAACAGAC
ch3 103.75 (350) GTAAATGCAGCAGGATTAAAAAACAAATTTGATGTCACATGGAAACAGGC
ch6 47.1mb (350) GTAAATGCAACAGGACTAAAAAATAAATTTGACATCACATGGAAACAGGC

ch22 AP000346 (401) AAAAAATATTGTACAACATTGCACCCAGTGTCAGATTCTACACCTGGCCA
ch22 AP000345 (401) AAAAAATATTGTACAACATTGCACCCAGTGTCAGATTCTACACCTGGCCA
ch3 103.75 (400) AAAAGATATTGTACAACATTGCACCCAGTGTCAAGTCTTACACCTGTCCA
ch6 47.1mb (400) AAAAAATATTGTACAACATTACACCCAGTGTCAGATTCCACACCTGCCCA

ch22 AP000346 (451) CTCAGGAGGCAAGAGTTAATCCCAGAGGTCTATGTCCTAATGTGTTATGG
ch22 AP000345 (451) CTCAGGAGGCAAGAGTTAATCCCAGAGGTCTATGTCCTAATGTGTTATGG
ch3 103.75 (450) CTCAAGAGGCAGGAGTTAATCCCAGAGGTCTGTGTCCTAATGCGTTATGG
ch6 47.1mb (450) CTCAGGAGGCAGGAGTTAATCCCAGAGGTCTGTGTCCTAATGCGTTATGG

ch22 AP000346 (501) CAAATGGATGTCATGCACGTACCTTCATTTGGAAAATTGTCATTTGTCCA
ch22 AP000345 (501) CAAATGGATGTCATGCACGTACCTTCATTTGGAAAATTGTCATTTGTCCA
ch3 103.75 (500) CAAATGGATGGCACGCATGTTCCTTCATTTGGAAGATTATCATATGTTCA
ch6 47.1mb (500) CAAATGGATGTCACACATGTACCTTCAGTTGGAAAATTGTCATTTCTCCA

ch22 AP000346 (551) TGTGACAGTTGATACTTATTCACATTTCATATGGGCAACCTGCCAGACAG
ch22 AP000345 (551) TGTGACAGTTGATACTTATTCACATTTCATATGGGCAACCTGCCAGACAG
ch3 103.75 (550) TGTAACAGTTGATACTTATTCACATTTCATATGGGCAACTTGCCAAACAG
ch6 47.1mb (550) TGTGACAGTTGATGCTTATTCACATTTTGTAAGGGCAAACTGCCAGACAG

ch22 AP000346 (601) GAGAAAGTACTTCCCATGTTAAAAGACATTTATTATCTTGTTTTCCTGTC
ch22 AP000345 (601) GAGAAAGTACTTCCCATGTTAAAAGACATTTATTATCTTGTTTTCCTGTC
ch3 103.75 (600) GAGAAAGTACTTCCCATGTTAAAAAACATTTATTATC-------------
ch6 47.1mb (600) GAGAAAATACTTCCCATGTTAAAAGACATTTATTATCT--------

Although the HERVs on chromosomes 3, 6 and 22 are closely-related, therefore, they can be distinguished by hybridization.

### Sequence 30453

Signal from sequence 30453 on the array was elevated in prostate tumor tissue compared to normal tissue. The 30453 sequence (SEQ ID 113) aligns with chromosome 22:

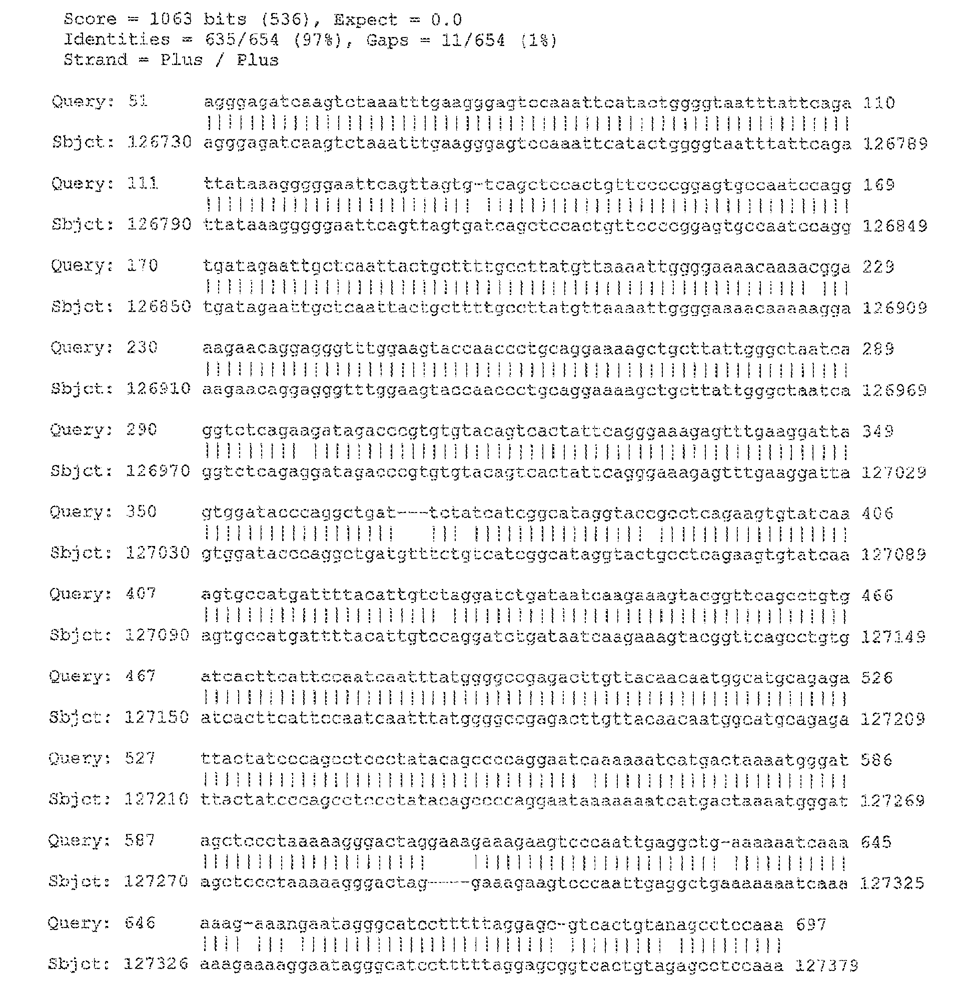

### Sequence 26503

Signal from sequence 26503 on the array was elevated in prostate tumor tissue compared to normal tissue. The 26503 sequence (SEQ ID 116) aligns with chromosome 22:

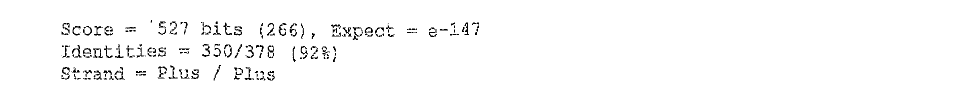

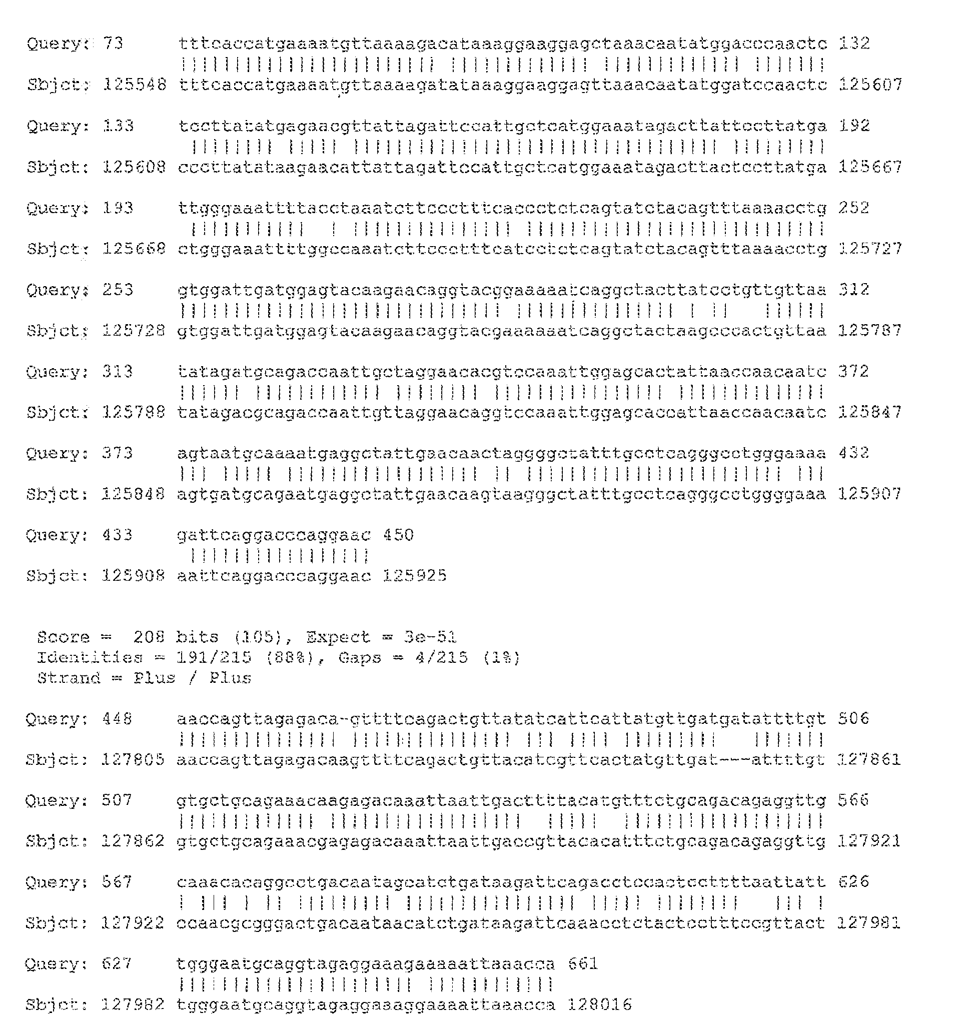

### Patient libraries

HERV-K IL-NIL2.0 cDNAs cloned from patient libraries align with PCAV. Clones from libraries derived from four patients align with >95% identity to PCAV.

SEQ ID 19 is from a cDNA which is present at elevated levels in prostate tumors. The first 463 of its 470 nucleotides align to four separate regions of the genomic DNA sequence on chromosome 22 (nucleotides 956-1075,2700-2777, 8166-8244 & 10424-10609 of SEQ ID 1):

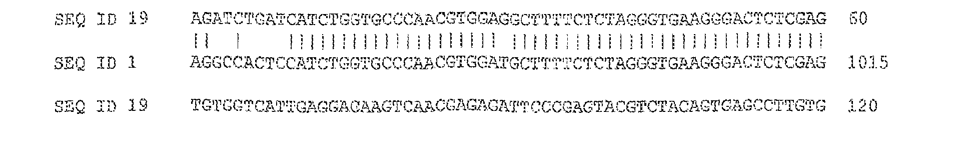

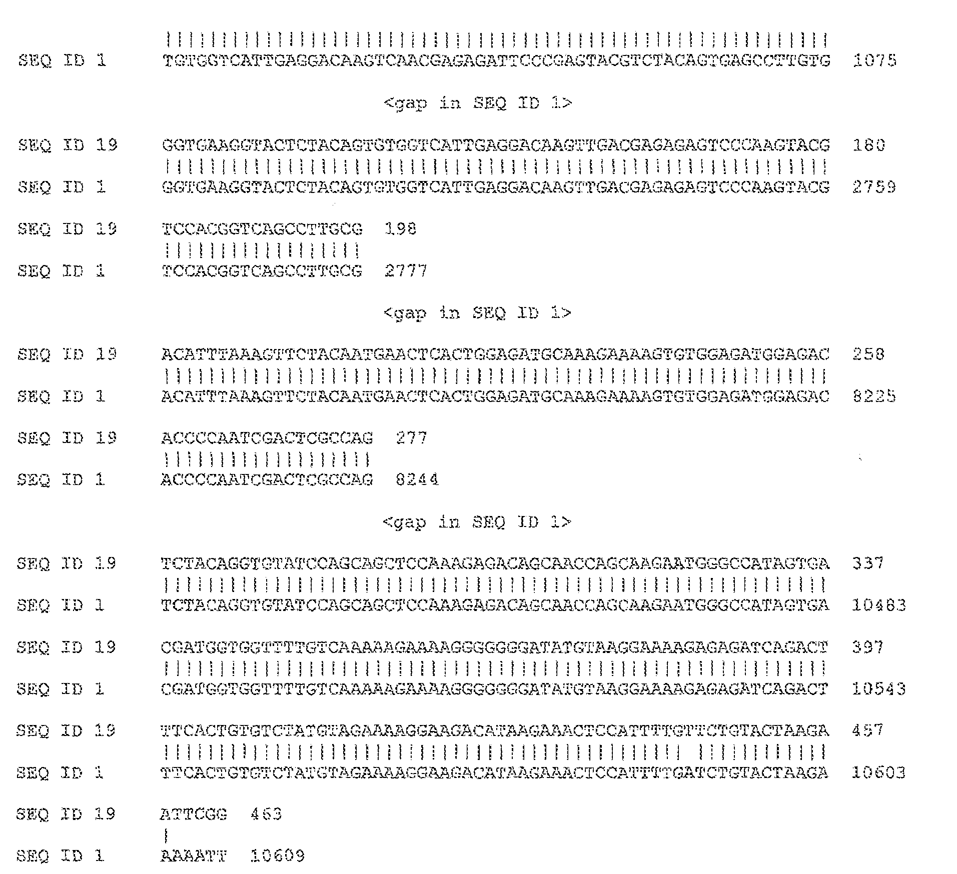

The dinucleotide sequences before and after the "gaps" in SEQ ID 1 are as follows:

| **SEQ ID 19** | **Exon** | **SEQ ID 1** | **Preceding and following dinucleotide in SEQ ID 1** | |
|---|---|---|---|---|
| 1-120 | 1 | 956-1075 | - | 1076-1077: GT |
| 121-198 | 2 | 2700-2777 | 2698-2699: AG | 2778-2779: GT |
| 199-277 | 3 | 8166-8244 | 8164-8165: AG | 8245-8246: GT |
| 278-463 | 4 | 10424-10609 | 10422-10423:AG | - |

The "gaps" in SEQ ID 1 thus begin and end with consensus splice donor and acceptor sequences. The presence of SEQ ID 19 in a cDNA thus verifies splicing in which the first 5' LTR is joined to the splice acceptor site near the 3' end of the second 5' LTR (nucleotide 1075 of SEQ ID 1 joined to nucleotide 2700), as well as other splicing events. Because the sequences in exons 1 and 2 are from two different viruses (old and new), and these are significantly different from other family new and old family members, it is unlikely that the SEQ ID 19 product was transcribed from a HERV-K other than PCAV.

SEQ ID 114 (035JN013.F03-FIS) aligns with available chromosome 22 sequence:

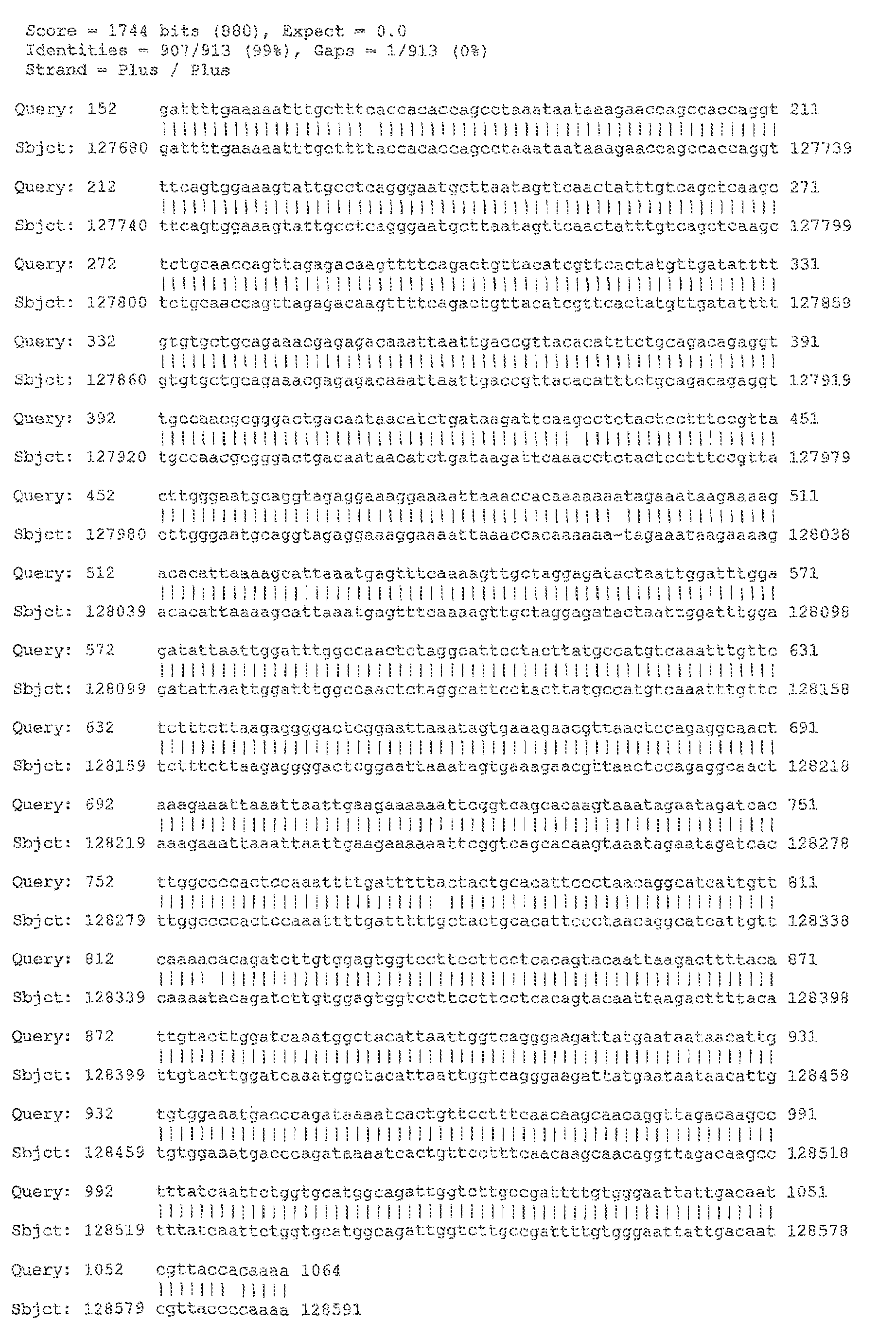

SEQ ID 115 (035JN015.H02-FIS) aligns with available chromosome 22 sequence:

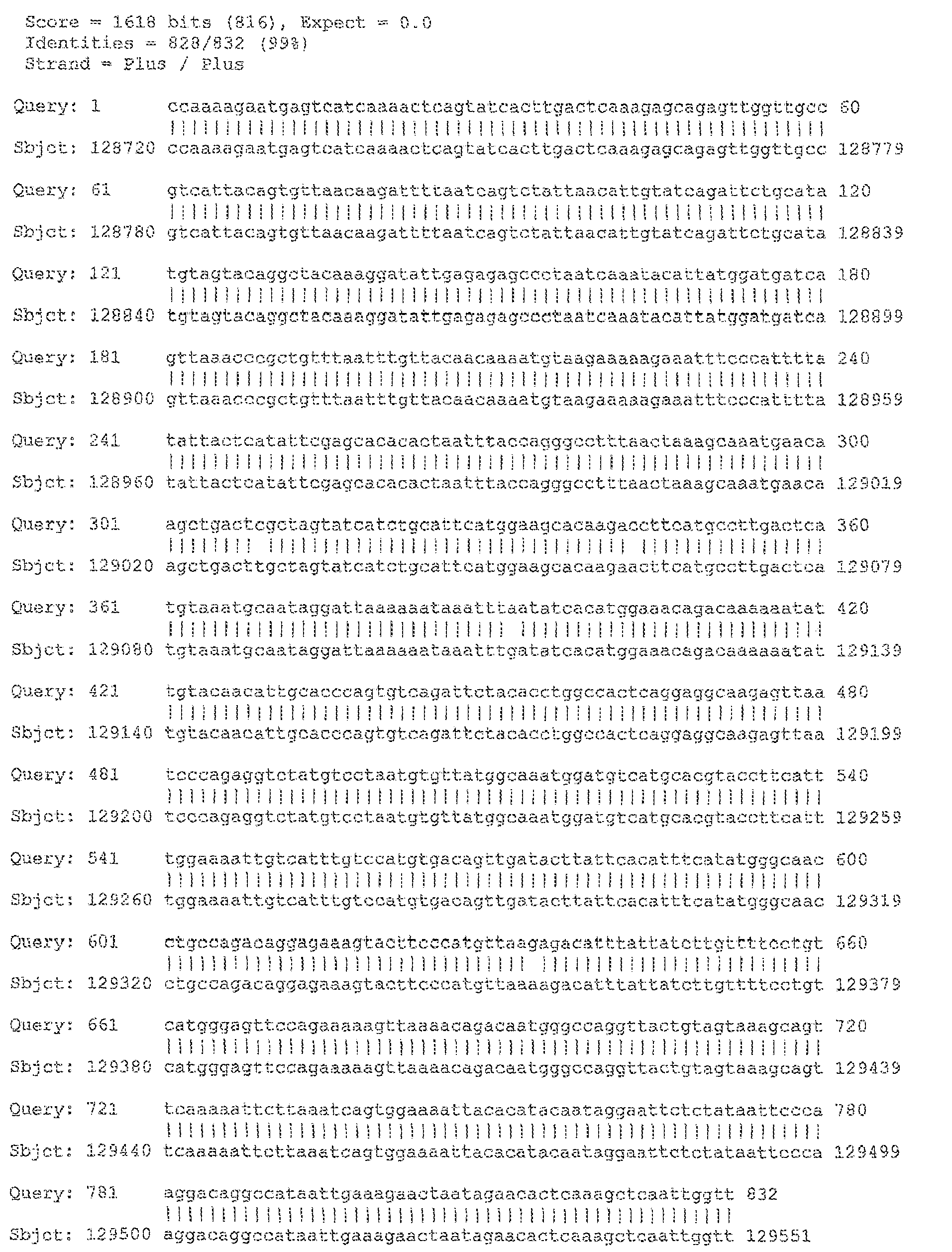

SEQ ID 117 (035JN003.E06-FIS) aligns with available chromosome 22 sequence:

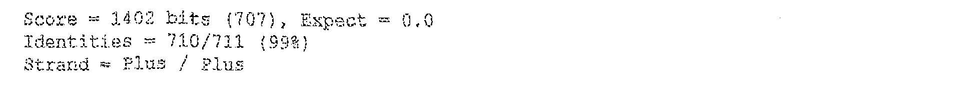

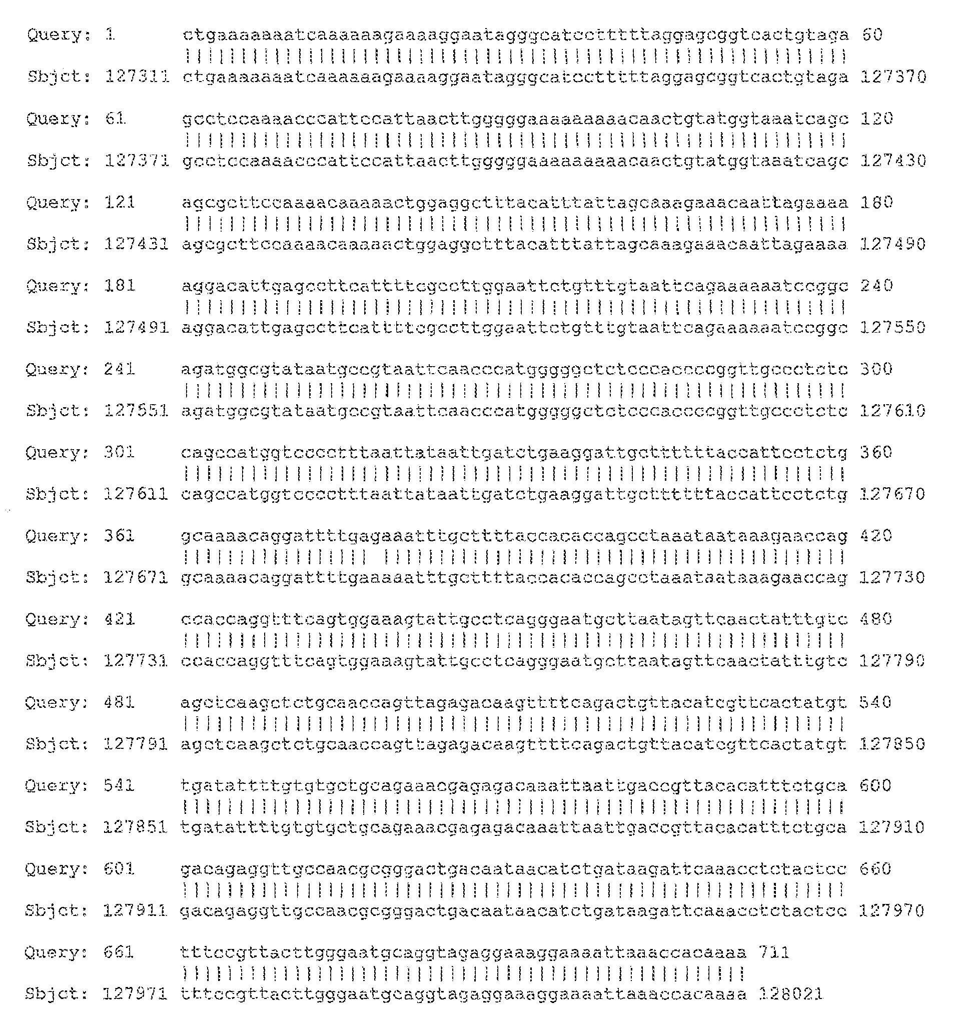

SEQ ID 118 (035JN013.C11) aligns with available chromosome 22 sequence:

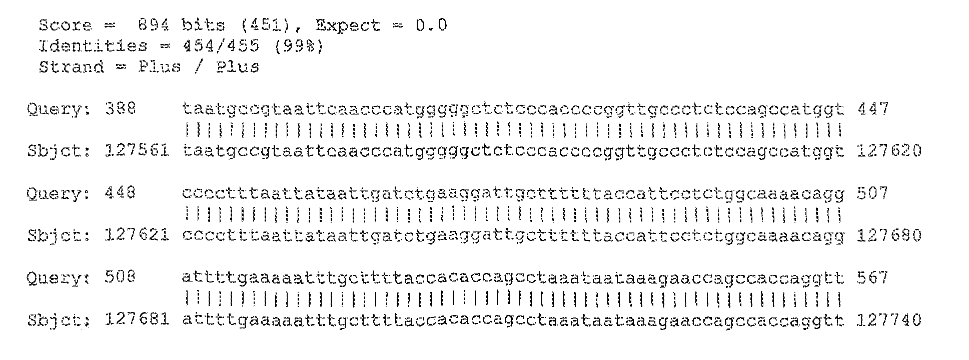

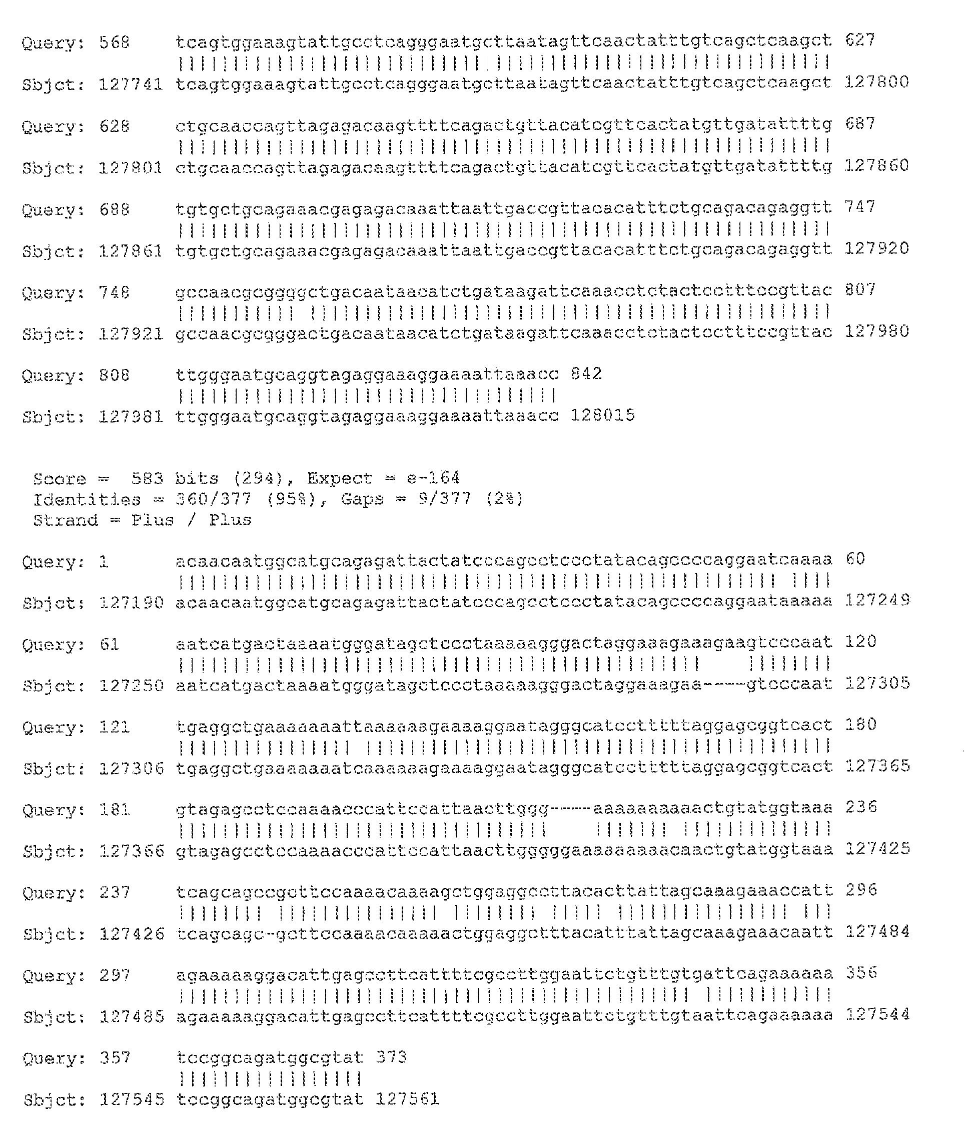

SEQ ID 119 (035JN001.F06) aligns with available chromosome 22 sequence:

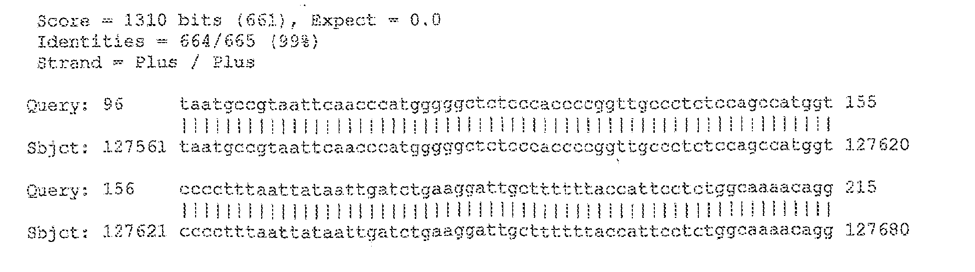

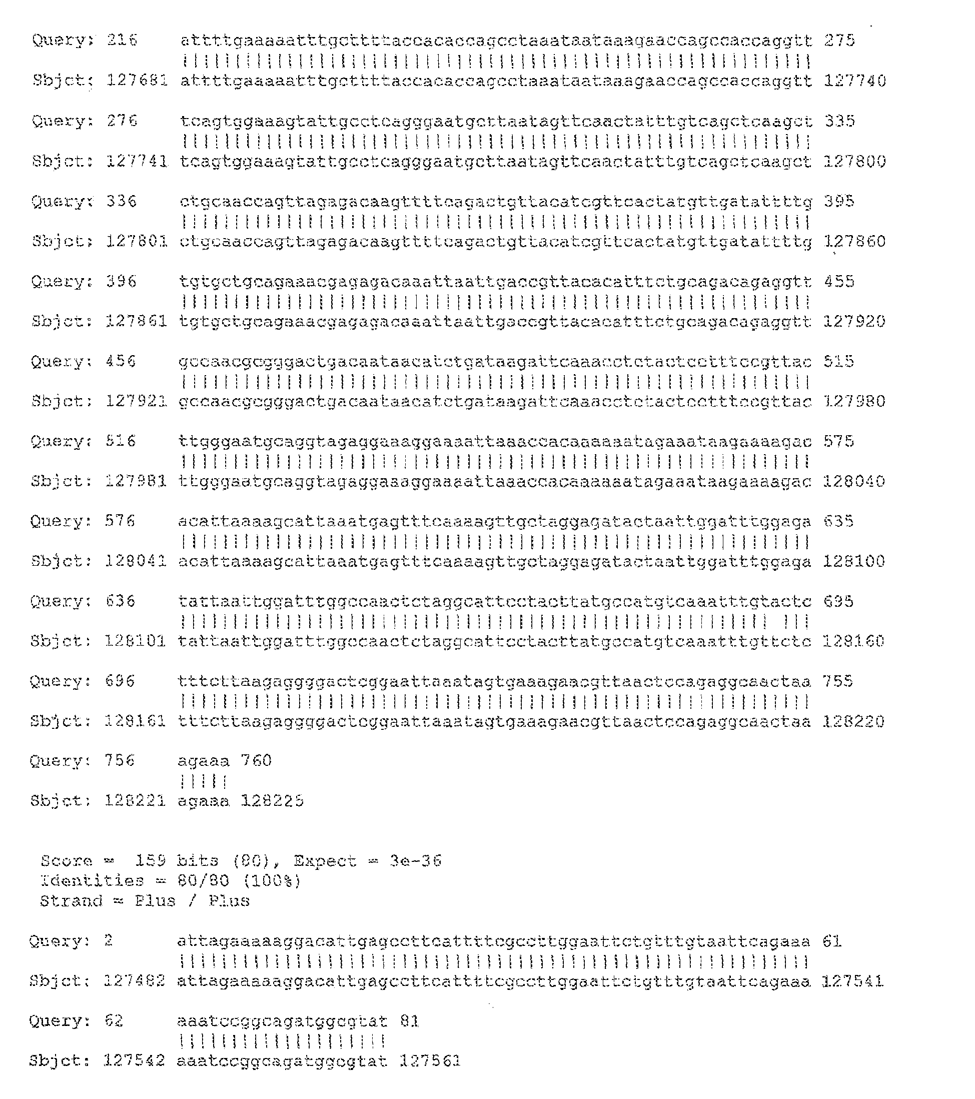

### Patient tumor samples

Fresh frozen prostate cancer tissue from two patients was cut in 10 micron sections, mounted on glass slides, and stained with murine monoclonal antibody 5G2. The staining was visualized with a second antibody (fluorescein-coupled goat anti-mouse). Staining was found to be specific for cancerous tissue. The samples were also analyzed by hybridization to 26254 and signal was 35-40 times stronger than in control samples from the same patient:

| **patient ID#** | **Gleason grade** | **5G2 stainig** | **spot 26254 ratio** |
|---|---|---|---|
| 101 | 3+3 | + (Figure 13) | 35 |
| 153 | 3+3 | +(Figure 14) | 40 |

### RT-PCR

RNA extracts from various tissues were analyzed by RT-PCR. In particular, the splicing event between exons 1 and 2 was investigated using primers as shown in figure 6. Results are shown in figure 10. All lanes show background levels of HERV-K HML2.0 (*i.e.* new virus) expression (thin lines) but prostate tissue (lane 6) shows a longer product (thick line), indicating expression of a HERV-K with a longer sequence between the 5'LTR and the start ofENV. The difference in length between the long lane 6 product and the background product seen in other tissues (∼80 bp) corresponds in length to the length of exon 2 illustrated in figure 6B.

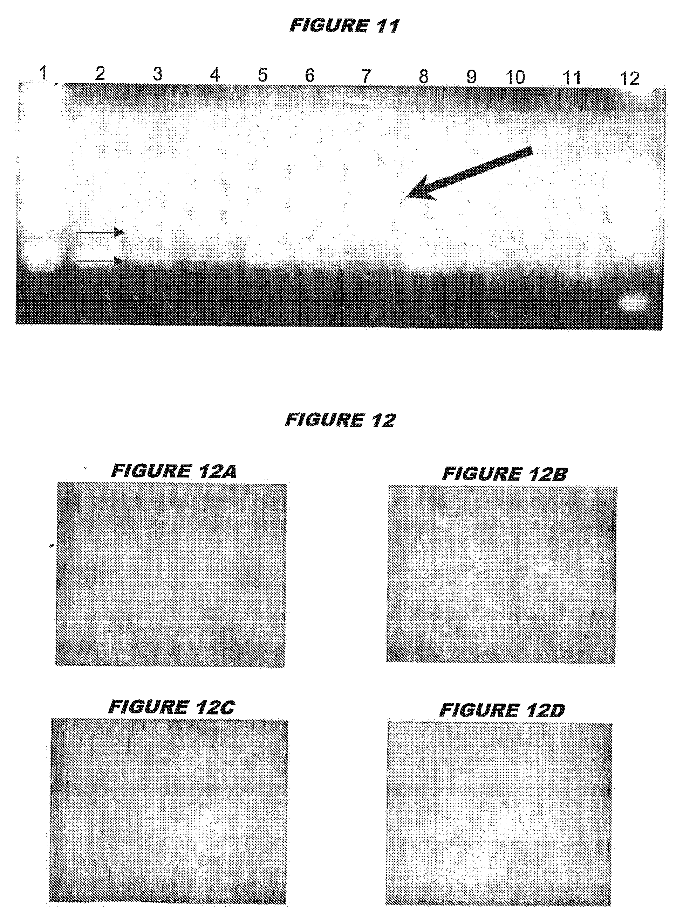

Extracts from cell lines were also tested (figure 11). Again, background levels of "ubiquitous" HERV-K expression were evident in most cell lines. Prostate cell lines MDA PCA 2b (lane 7) and, to a lesser extent, 22RV1 (lane 6), clearly showed longer RT-PCR products,

### MDA PCA 2b cell line

RNA was extracted from MDA PCA 2b cell lines. Spliced mRNAs were cloned and sequenced which confirm that splice acceptor sites near the 3' end of the second 5' LTR are used. These mRNAs have four exons with sequences exactly matching PCAV. They have exons adjacent to LTRs 1 and 2 followed by an exon containing the envelope ATG and a very short open reading frame and finally terminating in the final fragmentary 3' LTR.

The use of a splice acceptor site near the 3' end of the second 5' LTR was also seen in a cDNA present in a private prostate cancer library (Chiron clone ID 035JN024.B09).

The 3' end of MDA PCA 2b RNA was mapped by RACE. The forward PCR primer was SEQ ID 21, which matches PCAV and new HERV-Ks. The reverse PCR primer was SEQ ID 22. The primer for reverse transcription was SEQ ID 20. Using mRNA targets from MDA PCA 2b gave a major band at 1.3 kb. The bands were cloned and sequenced (using either T7 or SP6 sequencing primers) and an alignment is shown below:

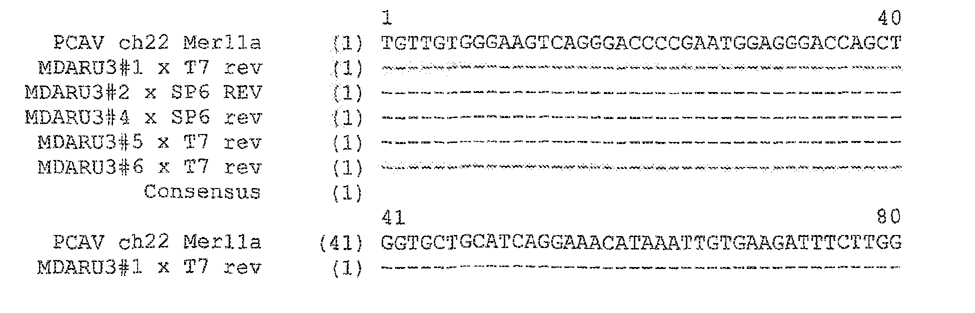

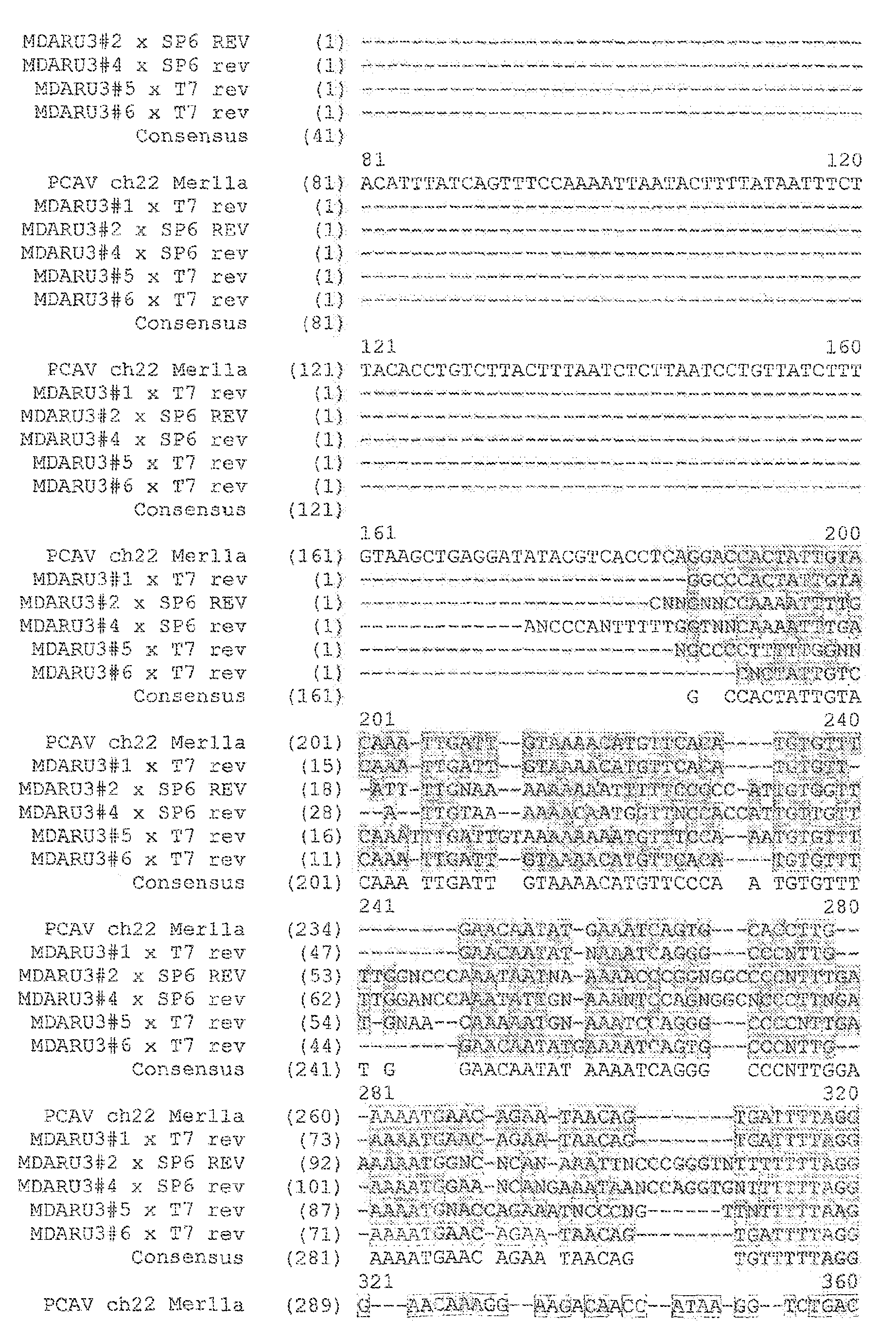

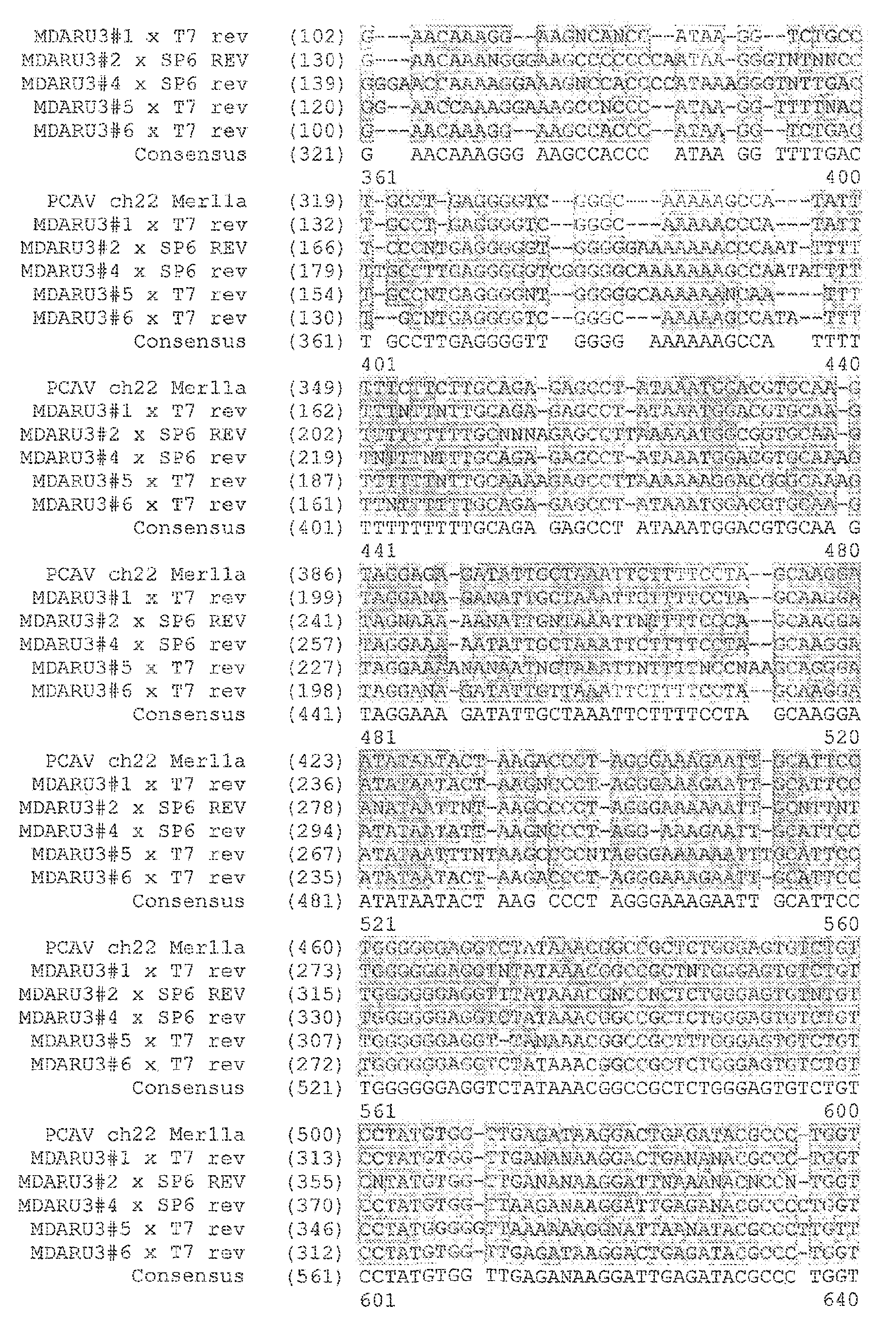

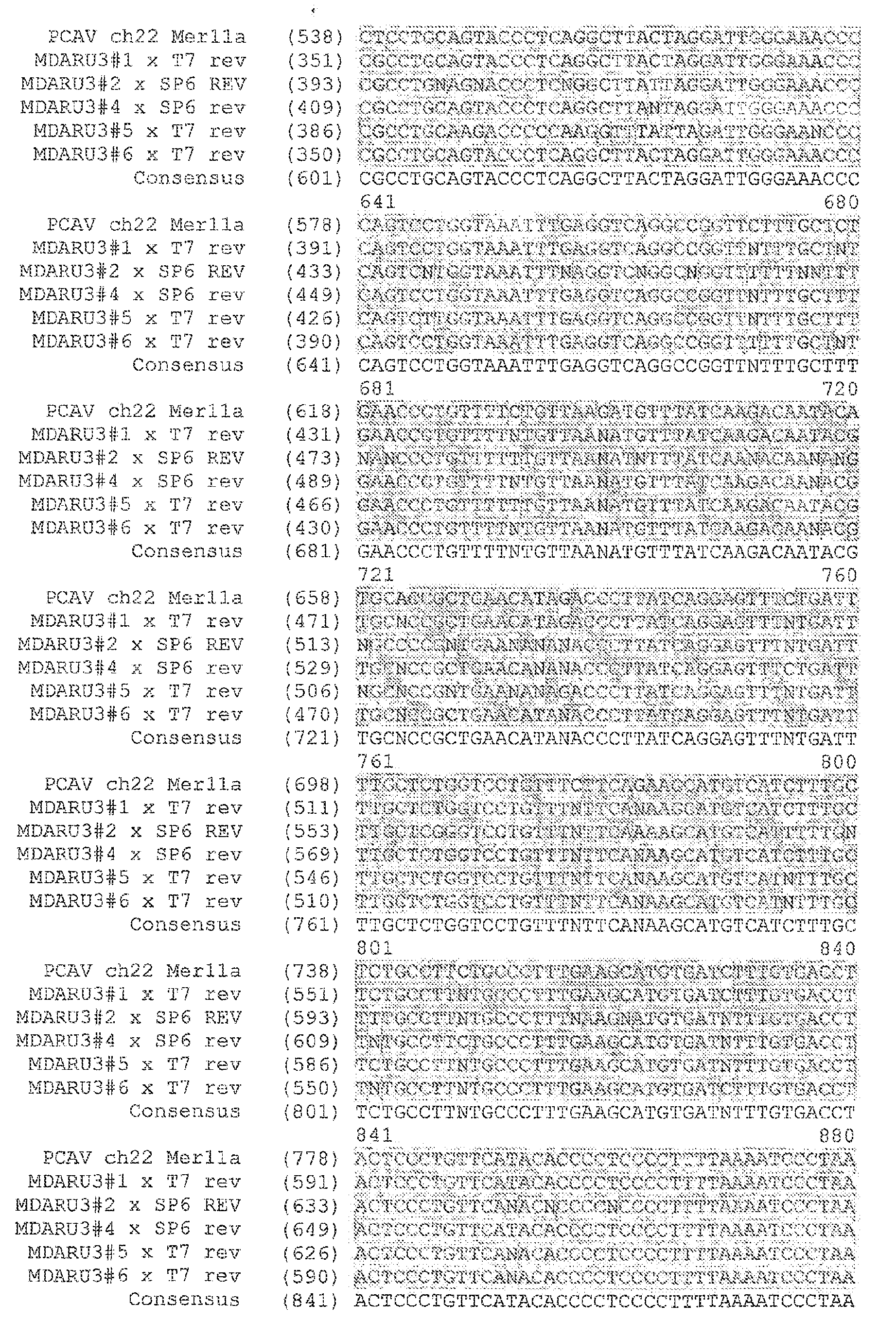

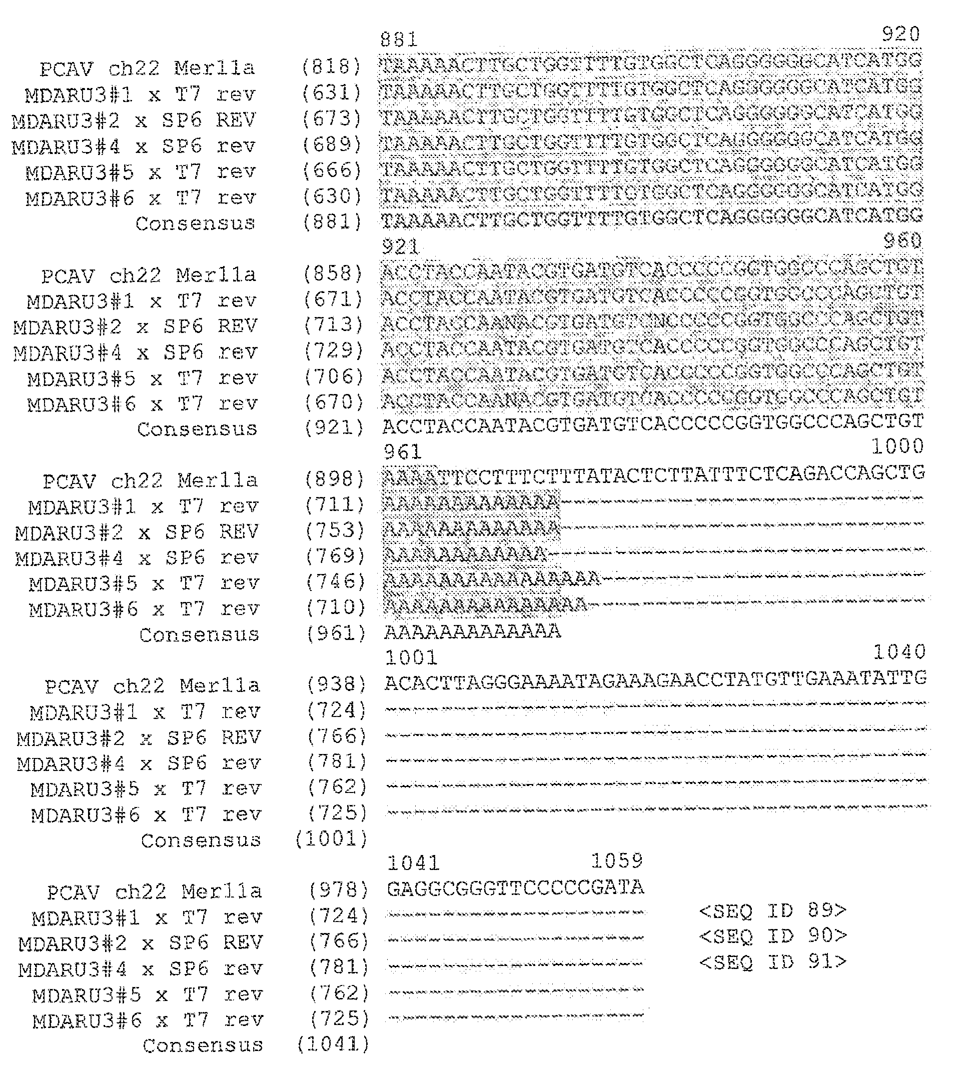

Sequencing of these amplification products shows that transcripts terminate using a polyA signal within a MER11a insertion (see row beginning with nucleotide 961). Again, this is a perfect match for PCAV.

### Anti-gag monoclonal antibodies

PCAV is an "old" HERV-K. Low-level expression of "new" HERV-Ks can also be detected. The gag open reading frames from PCAV and the "new" HERV-Ks are homologous at the primary sequence level, but with significant divergence. Gag protein was expressed in yeast and purified for both PCAV and "new" HERV-K, and mouse monoclonal antibodies were raised.

The "new" HERV-K gag sequence used for expression was isolated from the prostate cancer cell line LnCap and the PCAV gag sequence was isolated from the prostate cancer cell line MDA PCA 2b. These sequences were genetically engineered for expression in *Saccharomyces cerevisiae* AD3 strain, using the yeast expression vector pBS24.1 This vector contains the 2µ sequence for autonomous replication in yeast and the yeast genes leu2d and URA3 as selectable markers. The β-lactamase gene and the ColEl origin of replication, required for plasmid replication in bacteria, are also present in this expression vector, as well as the a-factor terminator. Expression of the recombinant proteins is under the control of the hybrid ADH2/GAPDH promoter.

The coding sequences for "new" HERV-K and PCAV gag were cloned as *Hind*III-*Sal*I fragments of 2012bp and 2168bp respectively. Each gag was subcloned in two parts:
1. The "new" HERV-K gag was subcloned into pSP72. A 143bp synthetic oligonucleotide from the *Hind*III site adjoined the ADH/GAPDH promoter to a *Nco*I site within the gag coding sequence. The remaining 1869bp of "new" HERV-K gag sequence, from *Nco*I to *Sal*I, was derived by PCR using a cDNA clone obtained from LnCaP cells named orf-99 as the template.
2. PCR. was used to create a 1715bp *Hind*III-*Ava*3 fragment PCAV gag, using a cDNA clone obtained from MDA PCa 2b cells named 2B11.12-44 as the template. The resulting PCR product was subcloned into pGEM7-Z. The *AVa*3-*Sal*I fragment encoding the 3' end of this construct was isolated from the "new" HERV-K gag clone above, since the 3' end of the gag protein was missing in the 2B11.12-44 clone.

After sequence confirmation the respective fragments were ligated with the ADH2/GAPDH promoter into the yeast expression vector to create pd.LnCap.gag (encoding the "new" HERV-K gag) and pd.MDA.gag (encoding the hybrid PCAV/ "new" HERV-K gag) yeast expression plasmids.

The "new" expression construct is SEQ ID 1185 and encodes SEQ ID 1186:

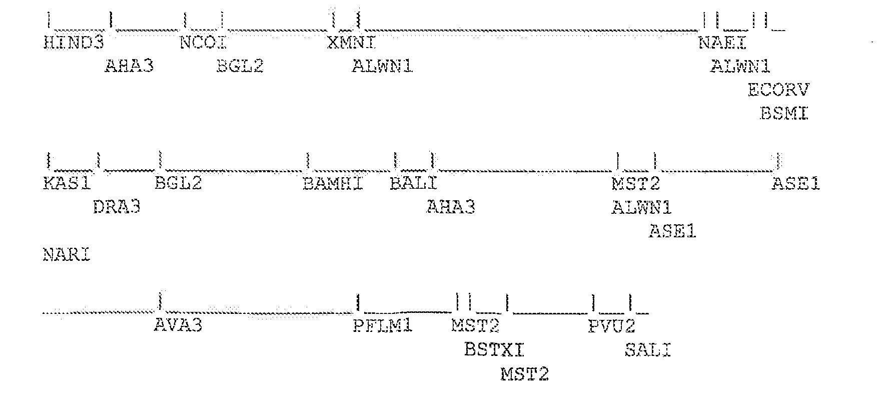

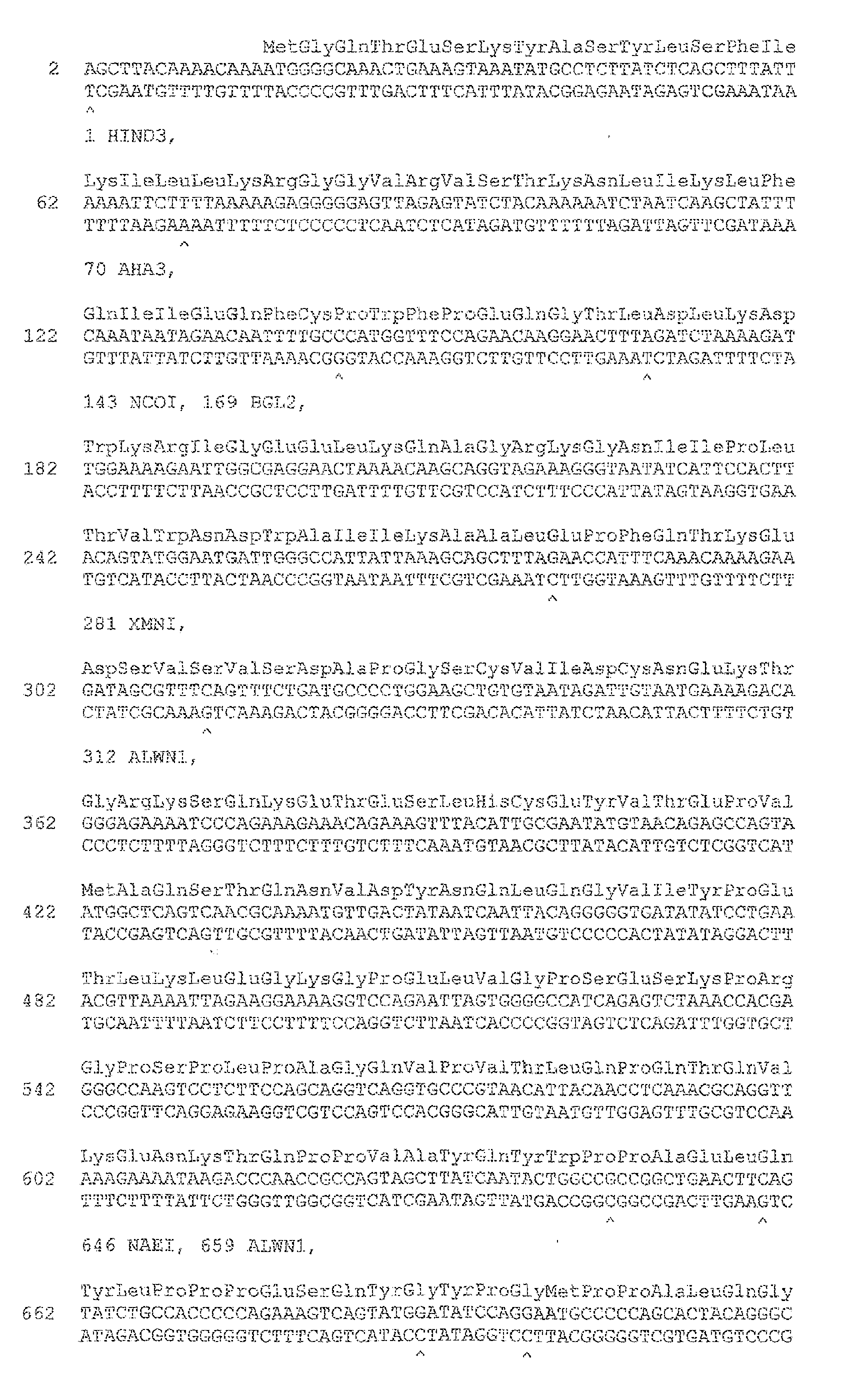

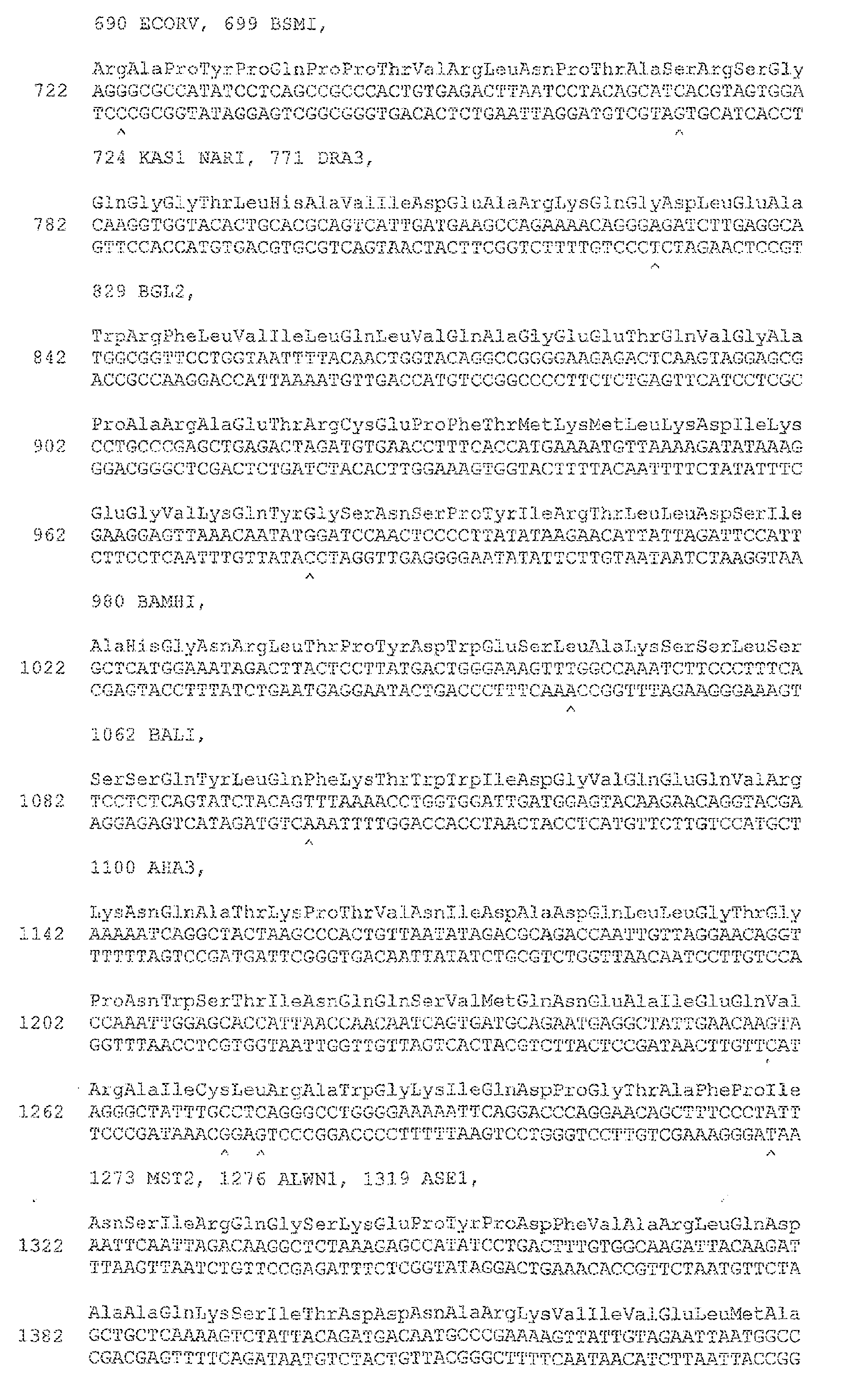

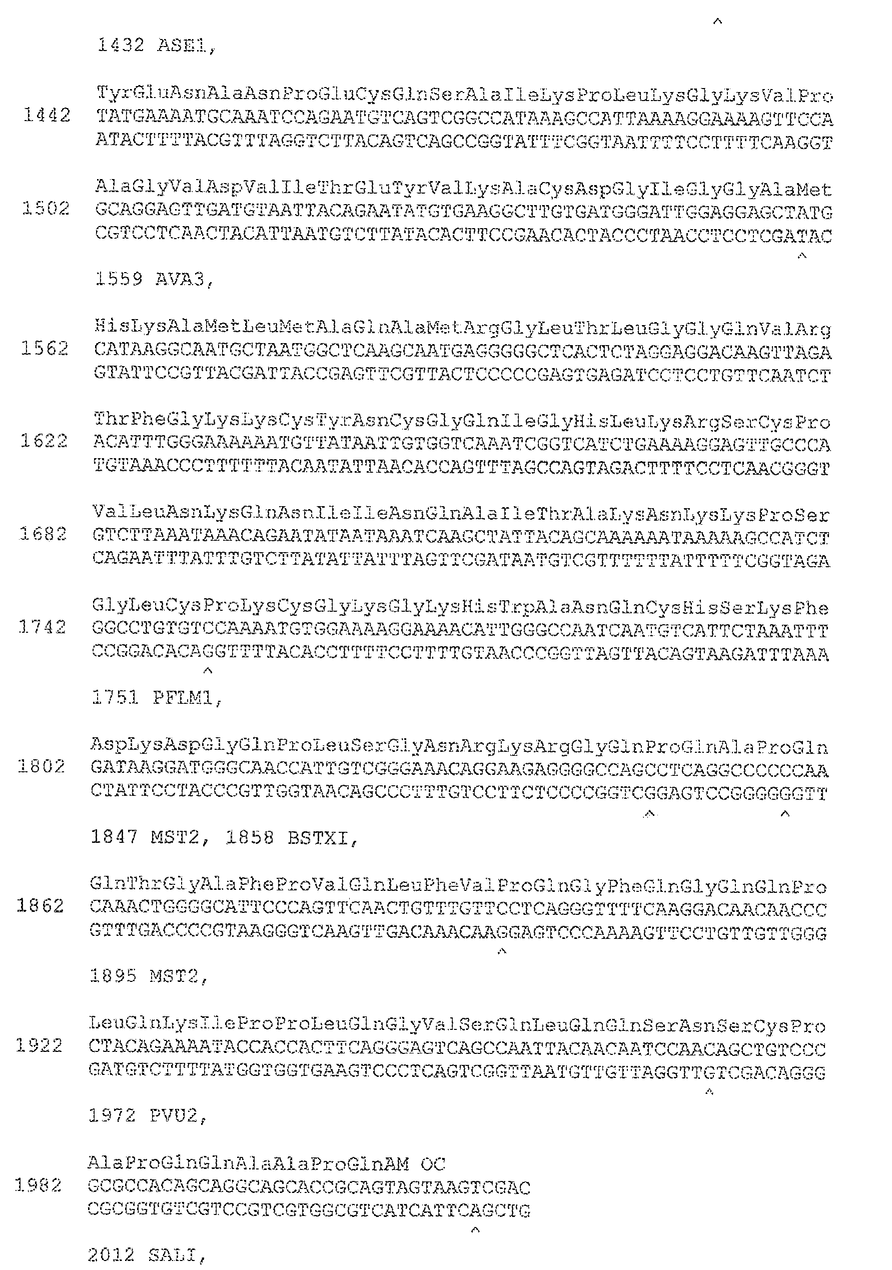

The hybrid construct is SEQ TO 1187 and encodes SEQ ID 1188:

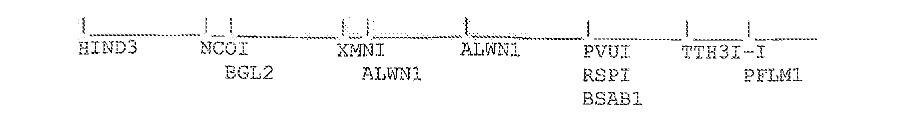

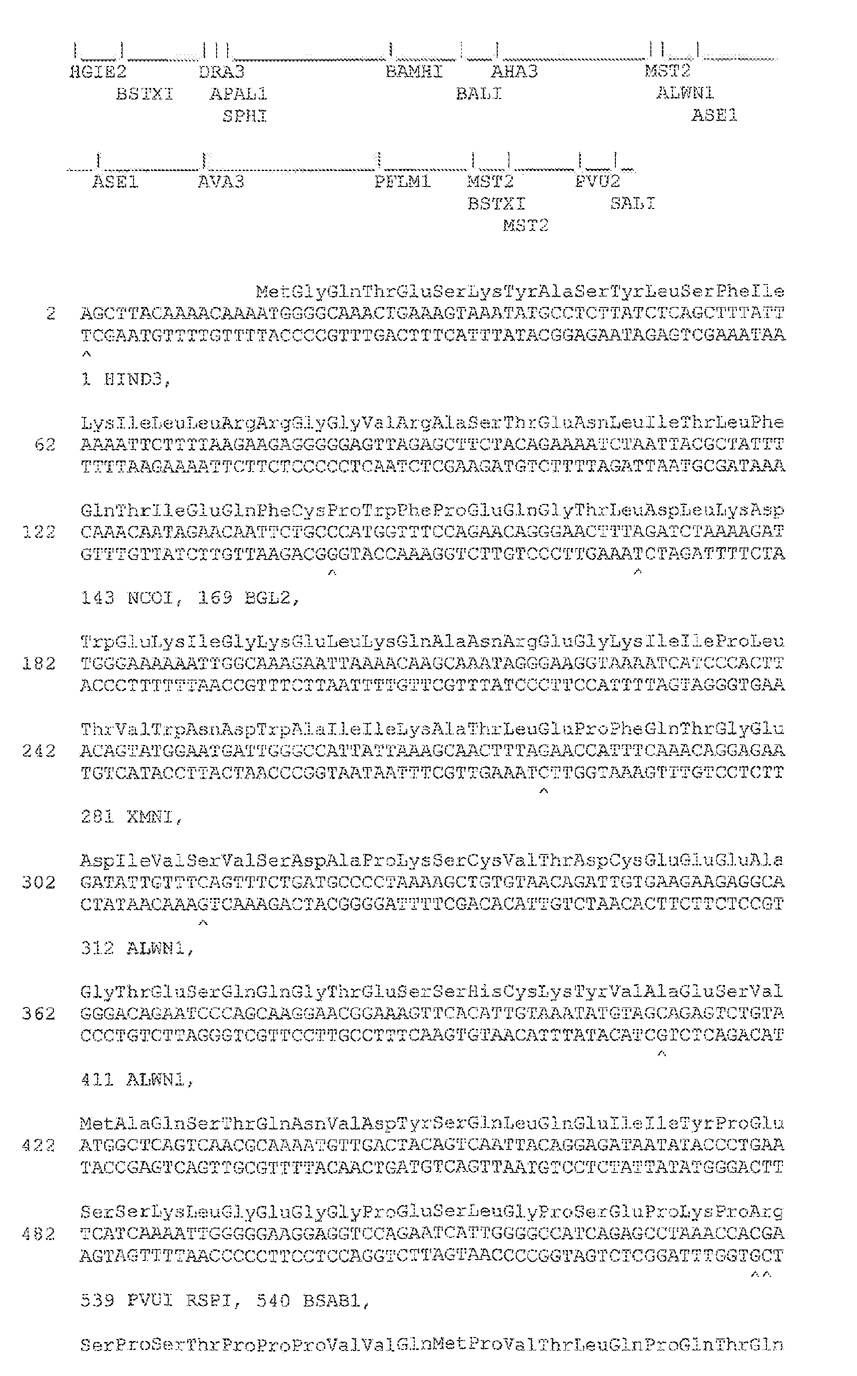

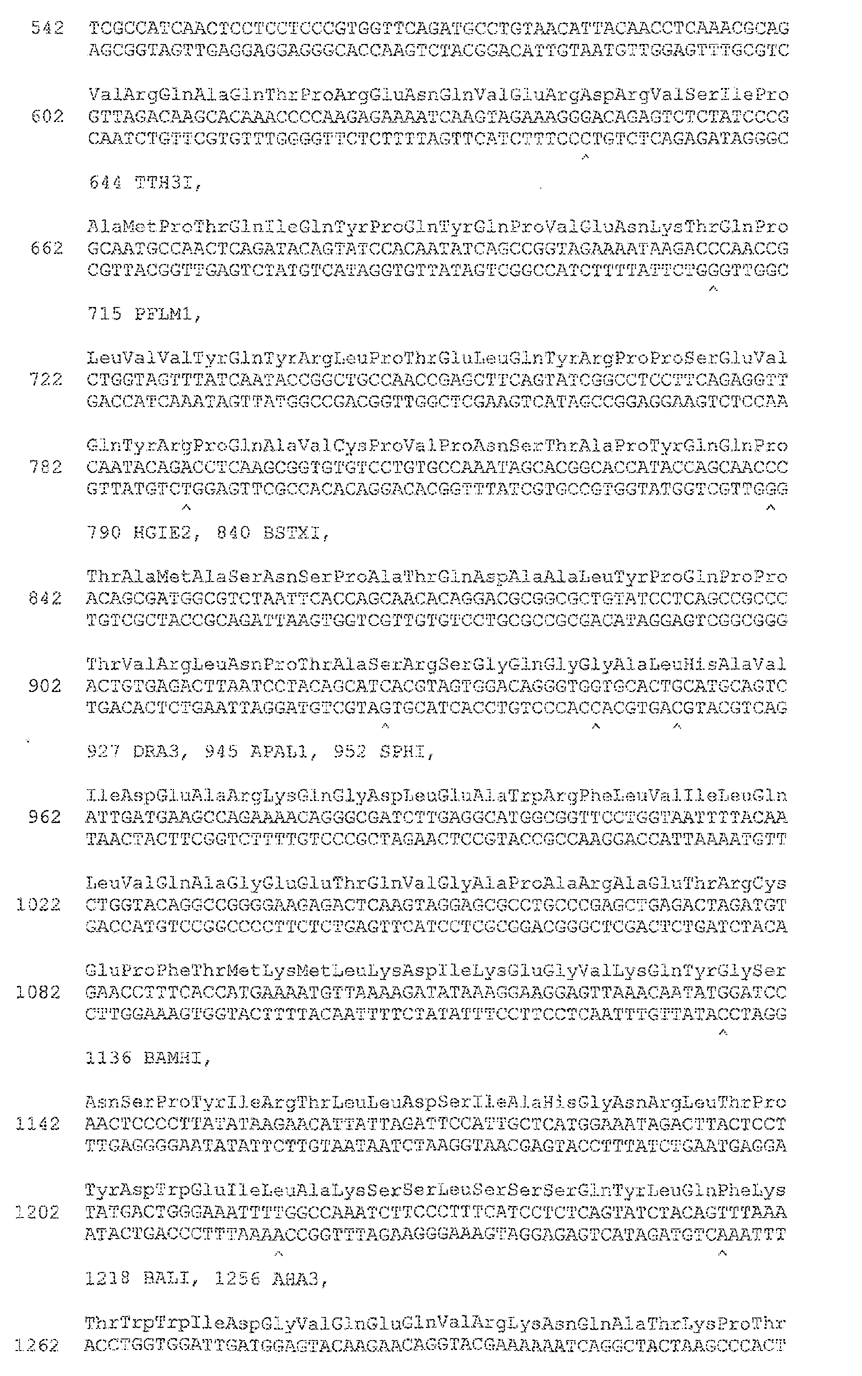

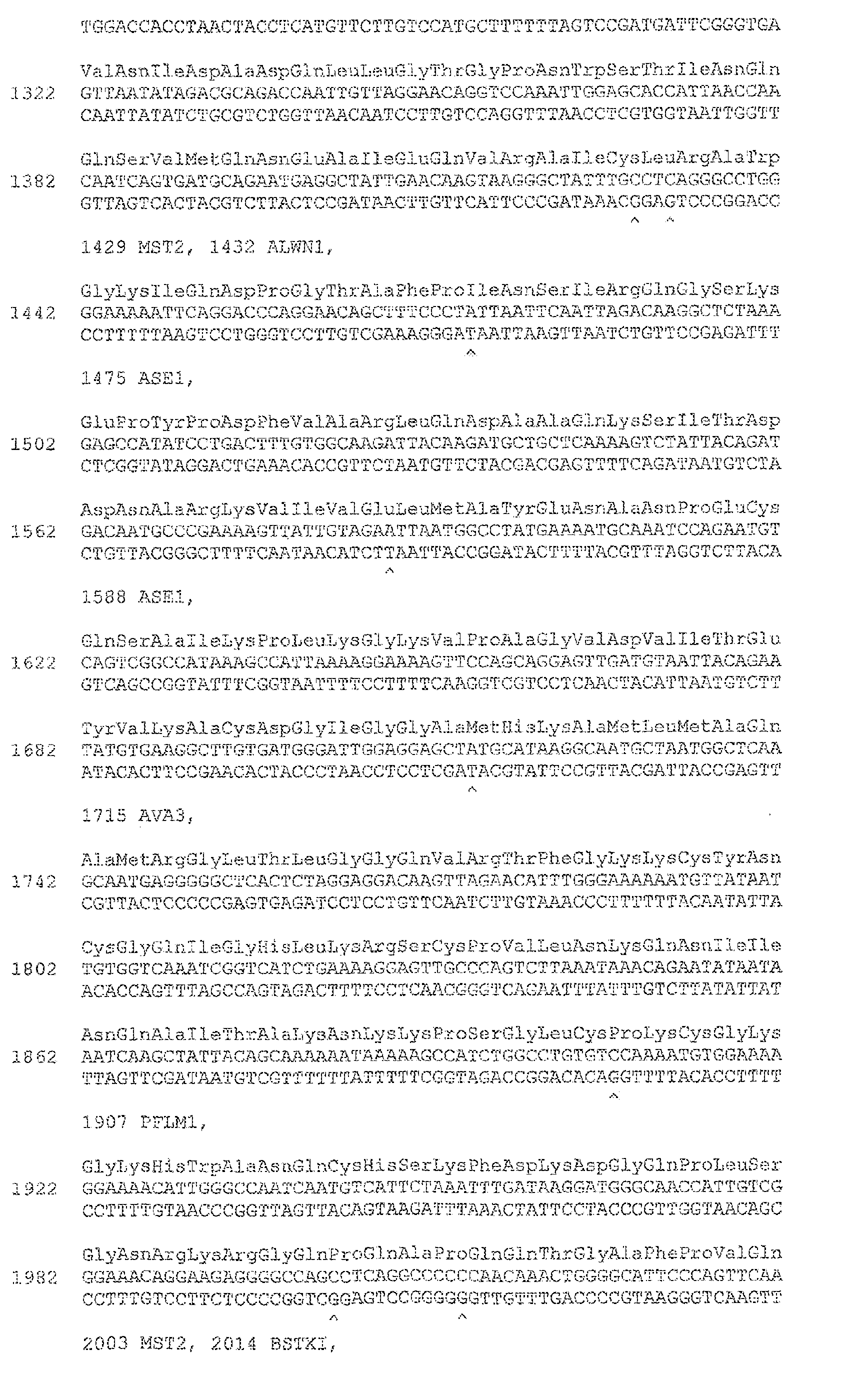

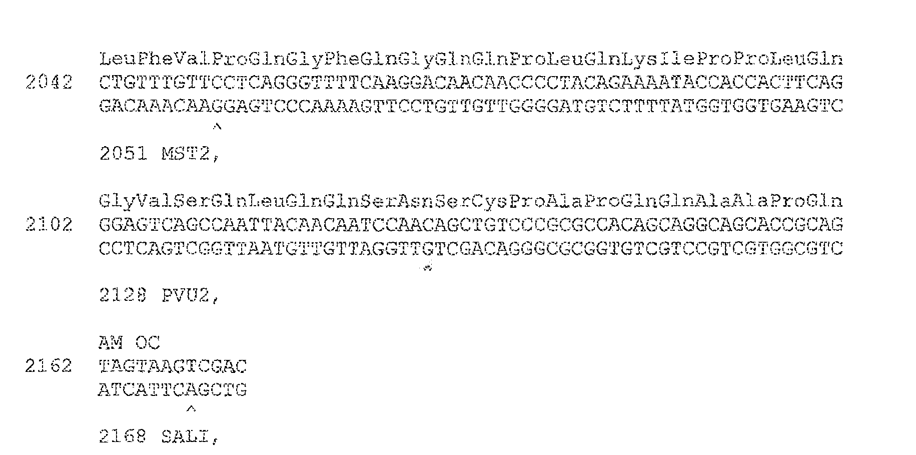

An alignment of the encoded proteins is below:

| | | | | |
|---|---|---|---|---|
| # | 1_{:} | y.MDA.2b1112.44.aa | 715 | 78.60% |
| # | 2: | y.orf99.aa (LNCap) | 663 | 84.77% |

ALIGNMENT MAP
- showing sequences and aligned repeats (in brackets)
- in each given alphabet

In alphabet in which alignment was found:

| | |
|---|---|
| 0 | {MGQTESKYASYLSFIKILL} r {RGGVR} aste {NLI} t {LFQ} t (IEQFC |
| 0 | {MGQTESKYASYLSFIKILL} k {RGGVR} vstk {NLI} k {LFQ} i {IEQFC |
| | |
| 42 | PWFPEQGTLDLKDW} ekigk {ELKQA} nregk {IIPLTVWNDWAIIKA} t |
| 42 | PWFPEQGTLDLKDW} krige {ELKQA} grkgn {IIPLTVWNDWAIIKA} a |
| | |
| 90 | {LEPEQT}gedi {VSVSDAP} k {SCV} tdceeeagtesqqg {TES} shckyvaes |
| 90 | (LEPFQI) keds {VSVSDAP} g {SCV} idcnektgrksqke {TES} lhceyvtep |
| | |
| 134 | {VMAQSTQNVD} s {QLQ} ei {IYPE} ssklgeg (GPE) sl {GPSE} p |
| 134 | {VMAOSTQNVDY} n {QLQ} gv {IYPE} tlklegk {GPE} lv {GPSE} |
| | |
| 174 | {KPR} spstpppvvqm {PVTLQPQTQV} rqaqtprenqverdrvsipamptqiqypqyqp |
| 174 | {KPR} gpsplpagqv. {PVTLQPQTQV} k..... |
| | |
| 228 | v{ENKTQP} lvv {YQY} rlpt {ELQY} rppsevqyrpqavcpvpnstapyqqpt |
| 196 | . {ENKTQP} pva {YQY} wppa {ELQY} lpppesqygypgmppalqgrap..... |
| | |
| 276 | amasnspatqdaal {YPQPPTVRLNPTASRSGQGG} a {LHAVIDEARKQGDLEAWRF |
| 238 | .....{YPQPPTVRLNPTASRSGQGG} t {LHAVIDEARKQGDLEAWRF |
| | |
| 330 | LVILQLVQAGEETQVGAPARAETRCEPFTMKMLKDIKEGVKQYGSNSPYIRTLLDSIAHG |
| 278 | LVILQLVQAGEETQVGAPARAETRCEPFTMKMLKDIKEGVKQYGSNSPYIRTLLDSIAHG |
| | |
| 390 | NRLTPYDWE} i {LAKSSLSSSQYLQFKTWWIDGVQEQVRKNQATKPTVNIDADQLLGT |
| 338 | NRLTPYDWE} s {LAKSSLSSSQYLQFKTWWIDGVQEQVRKNQATKPTVNIDADQLLGT |
| | |
| 446 | CPNWSTINQQSVMQNEAIEQVRAICLRAWGKIQDPGTAFPINSIRQGSKEPYPDFVARLQ |
| 394 | CPNWSTINQQSVMQNEAIEQVRAICLRAWGKIQDPGTAFPINSIRQGSKEPYPDFVARLQ |
| | |
| 506 | DAAQKSITDDNARKVIVELMAYENANPECQSAIKPLKGKVPAGVDVITEYVKACDGIGGA |
| 454 | DAAQKSITDDNARKVIVELMAYENANPECQSAIKPLKGKVPAGVDVITEYVKACDGIGGA |
| 566 | MHKAMLMAQAMRGLTLGGQVRTFGKKCYNCGQIGHLKRSCPVLNKQNIINQAITAKNKKP |
| 514 | MHKAMLMAQAMRGLTLGGQVRTFGKKCYNCGQIGHLKRSCPVLNKQNIINQAITAKNKKP |
| | |
| 626 | SGLCPKCGKGKHWANQCHSKFDKDGQPLSGNRKRGQPQAPQQTGAFPVQLFVPQGFQGQQ |
| 574 | SGLCPKCGKGKHWANQCHSKFDKDGQPLSGNRKRGQPQAPQQTGAFPVQLFVPQGFQGQQ |
| | |
| 686 | PLQKIPPLQGVSQLQQSNSCPAPQQAAPQ} |
| 634 | PLQKIPPLQGVSQLQQSNSCPAPQQAAPQ} |

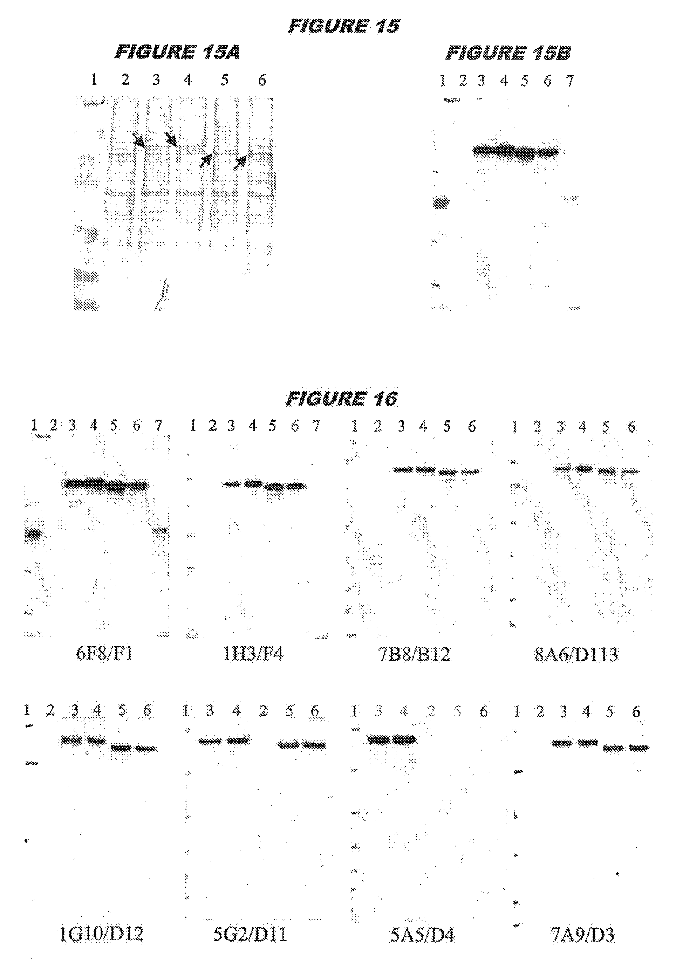

*S.cerevisiae* AD3 strain (mata,leu2,trp1,ura3-52,prb-1122,pep4-3,prc1-407,cir°,trp+ : DM15[GAP/ADR]) was transformed and single transformants were checked for expression after depletion of glucose in the medium. The recombinant proteins were expressed at high level in yeast, as detected in total yeast extracts by Coomassie blue staining (Figure 15A). The expressed proteins were easily observed in a total yeast extract (arrows), with "new" gag in lanes 5 & 6 and the hybrid gag in lanes 3 & 4. Un-transformed control cells are shown in lane 2.

After a large-scale fermentation, proteins were purified and used for monoclonal antibody production. Eight mAbs were obtained in large quantities and they were tested for their ability to recognize both gag proteins in Western blots (Figure 16). Of the 8 mAbs, 7 recognize both of the recombinant proteins and one (5A5/D4) recognizes only the PCAV/HERV-K hybrid gag protein. Antibody 5G2 cross-reacts with both old and new gag antigens:

| **mAb** | **Antigen** | **"New" HERV-K gag** | **PCAV/ HERV-K hybrid gag** |
|---|---|---|---|
| 5G2/D11 | "New" HERV-K gag | POSITIVE | POSITIVE |
| 7B8/B12 | "New" HERV-K gag | POSITVE | POSITIVE |
| 8A6/D113 | "New" HERV-K gag | POSITIVE | POSITIVE |
| 7A9/D3 | "New" HERV-K gag | POSITIVE | POSITIVE |
| 1G10/D12 | "New" HERV-K gag | POSITTVE | POSITIVE |
| 1H3/F4 | "New" HERV-K gag | POSITIVE | POSITIVE |
| 5A5/D4 | PCAV/HERV-K hybrid gag | NEGATIVE | POSITIVE |
| 6F8/F1 | PCAV/HERV-K hybrid gag | POSITIVE | POSITIVE |

mAb 6F8/F1 was used in a Western blot (Figure 15B) of a gel containing the yeast extracts in the same order and in Figure 15A. To reduce signal intensity, the samples containing the gag recombinant proteins were diluted 50-fold relative to the samples shown in Figure 15A using the yeast extract containing no recombinant protein.

5G2 antibody binds to MDA PCA 2b cells (figure 12B). The cells did not fluoresce in the absence of the antibody (figure 12A). Prostate cell line PC3 was also reactive (figure 12C), but less so than MDA PCA 2b. A transformed fibroblast cell line (NIH3T3) was not reactive with anti-HERV-K-gag antibody (figure 12D).

The gag mRNA structure found in MDA PCA 2b cells begins in the first 5' LTR and splices out the second 5' LTR. Such an arrangement is necessary in order for the RNA to be translationally competent because the second 5' LTR contains many stop codons which, in unspliced mRNA, would prevent gag translation.

### PCAV sequence analysis

The genomic sequence of PCAV from chromosome 22 is given as SEQ ID 1. This sequence extends from the start of the first 5' LTR in the genome to the end of the final fragment of the 3' LTR. It is 12366 bp in total.

Within SEQ ID 1, the first 5' LTR (new) is nucleotides 1-968. This is followed by HERV-K sequence up to nucleotide 1126. Nucleotides 1127-1678 are non-viral, including TG repeats at 1464-1487. The second 5' LTR (old) is from nucleotides 1679-2668. The 3' LTR is fragmented as nucleotides 10520-10838 and 11929-12366. The MER11a insertion is at nucleotides 10839-11834, with its polyA signal located between 11654-11659. The polyA addition site is located between 11736 and 11739, but it is not possible to say precisely where, because these four nucleotides are already As.

Basic coding regions within SEQ ID 1 are:

| **Product** | Gag-pol frag | PCAP6 | Gag | Prt | Pol-Env frag | Env frag |
|---|---|---|---|---|---|---|
| **Start (5')** | 2669 | 2680 | 2813 | 4762 | 8513 | 10244 |
| **End (3')** | 8227 | 2777 | 4960 | 5688 | 9946 | 10463 |

Splice donor (5'SS) sites are located at nudeotides 999-1004, 1076-1081, 2778-2783, 8243-8249, 8372-8378, 8429-8436, 8634-8641, 8701-8708 and 8753-8760. Splice acceptor (3'SS) sites are located at nucleotides 2593-2611, 2680-2699, 8112-8131, 8143-8165 and 10408-10423.

After the first transcribed region, there are three main downstream exons located at nucleotides 2700-2777,8166-8244 and 10424-11739.

The gag gene (nucleotides 2813-4960 of SEQ ID 1; SEQ ID 57) encodes a 715aa polypeptide (SEQ ID 54).

The protease gene (nucleotides 4762-5688 of SEQ ID 1; SEQ ID 58) is interrupted by three stop codons:

WATIVWKQEEGPASGPPTNWGIPS*TVCSSGFSRTTTPTENTTTSGSQPITTIQQLS
RATAGSTAVDLCSTQMVFLLPGKPPQKIPRGVYGPLPEGRVGL*GRSSLNLKGVQIH
TGVIYSDYKGGIQLVISSTVPRSANPGDRIAQLLLLPYVKIGENKKERTGGFGSTNP
AGKAAYWANQVSEDRPVCTVTIQGKSLKDVDTQADVSVIGIGTASEVYQSAMILHCP
GSDNQESTVQPVITSFIPINLWGRDLLQQWHAEITIPASLYSPRNKKIMTKMG*LPK
KGLGKKEVPIEAEKNQKRKGIGHPF

The four amino acid sequences between stop codons are SEQ IDs 59 to 62.

The pol gene (SEQ ID 86) is also interrupted. Alignment with known pol sequences reveals various fragments of amino acid sequences (SEQ IDs 92 to 97):

ESSKLSIT*LKEQSWLPSLQC*QDFNQSINIVSDSAYVVQATKDIERALIKYIMDDQ
LNPLFNLLQQNVRKRNFPFYITHIRAHTNLPGPLTKANEQADLLVSSAFMEAQELHA
LTHVNAIGLKNKFDITWKQTKNIVQHCTQCQILHLATQEARVNPRGLCPNVLWQMDV
MHVPSFGKLSFVHVTVDTYSHFIWATCQTGESTSHVKRHLLSCFPVMGVPEKVKTDN
GPGYCSKAVQKFLNQWKITHTIGILYNSQGQAIIERTNRTLKAQLVKQKKGKDRSIT
LPRCNLI


*IITLCGNDPDKIT
VPFNKQQVRQAFINSGAWQIGLADFVGIIDNRYPKTKIFQFLKLTTWILPKVTKHKP
LKNALAVFTDGSSNGKVAYTGPKE*

*TKKRKRQEYNTPQMQLNLALYTLNVLNIYRNQTTTSAEQHLTGKRNSPHEGKLIWW
KDNKNKTWEMGKVITWGRGFACVSPGENQLPVWIPTRHLKFYNELTGDAKKSVEMET
PQSTRQVNKMVISEEQKKLPSIKEAELPI

The env gene (nucleotides 9165-9816 of SEQ ID 1; SEQ ID 63) is interrupted by stop codons. The longest uninterrupted sequence encodes amino acid sequence SEQ ID 64. The reading frame +1 to SEQ ID 63 contains several short amino acid sequences (SEQ IDs 65 to 80) between stop codons:

HPELGSLLWPHTTLEFVLEIKL*EQEIVSHIILST*IPV*QFLCKIV*NSLILLLVVG
KT*LLNLIPKP*SVKIVECLLALI*LLIGSTVFY*EEQERVCGSLCPWTDHGRLRYP
SIF*RKY*KEF*LDPKDSFLL*WQ*LWASLQSQLLLRLLELLYTPLFKLQNT*MIGK
RIPQNCGILLRSK*IKNWQTKLMILDKLSFGWERLMSLEYLFQLRC

Nucleotides 8916-9155 ofSEQ ID 1 (SEQ ID 81) are also interrupted to give several short amino acid sequences (SEQ IDs 82 to 85):

VQNNEF*TMIDWVP*GQLYHNCTGQTHSCSQAPSIWPINPAYDGDVTERLDQVYRRL
ESLCPRKWGEKGISSP*PKLVLLLVL

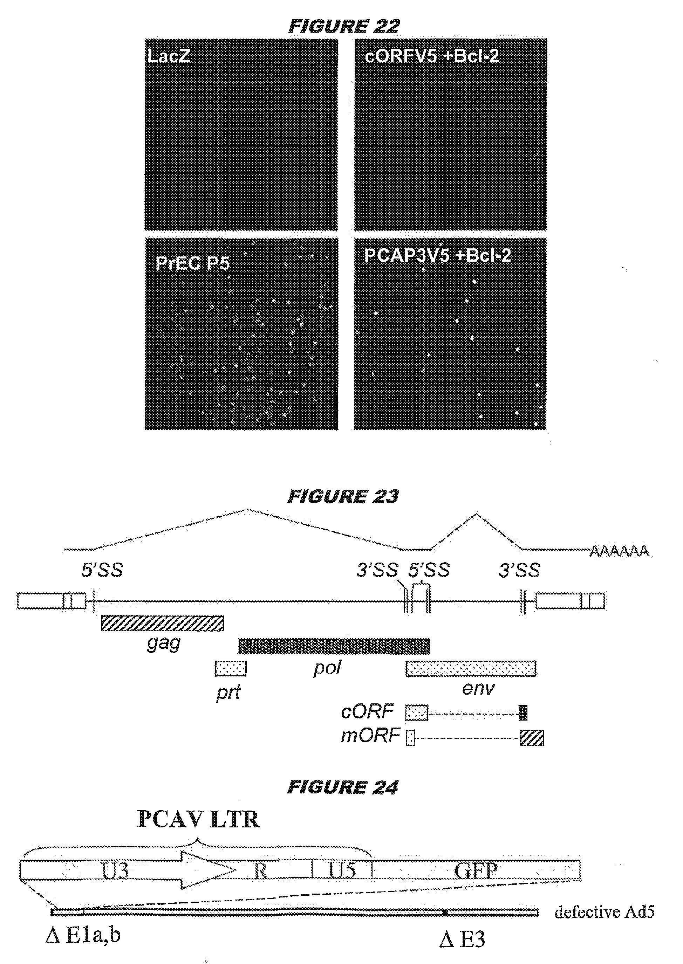

A polypeptide product called 'morf' or 'PCAP3' (SEQ ID 87) is roughly equivalent to the 'cORF' product previously seen for HERV-Ks. Its coding sequence begins at nucleotide 8183 of SEQ ID 1, with splicing occurring after nucleotide 8244 and joining to nucleotide 10424. The splice junction forms a AGT serine codon within SEQ ID 88 (Figure 23):

ATGAACTCACTGGAGATGCAAAGAAAAGTGTGGAGATGGAGACACCCCAATCGACTCGCCAGgtaaacaaa 8253
M N S L E M Q R K V W R W R H P N R L A r *

...cctgttctgtctgttgttagTCTACAGGTGTATCCAGCAGCTCCAAAGAGACAGCAACCAGCAAGAATGGGCCATAG 10480
L Q V Y P A A P K R Q Q P A R M G H S

TGACGATGGTGGTTTTGTCAAAAAGAAAAGGGGGGGATATGTAAGGAAAAGAGAGATCAGACTTTCACTGTGTCTATGTA 10560
D D G G F V K K K R G G Y V R K R E I R L S L C L C R

GAAAAGGAAGACATAAGAAACTCCATTTTGATCTGTACTAA 10601
K G R H K K L H F D L Y *

Further details about PCAP3 are given below.

### Unique DNA sequence within PCAV gag

PCAV gag contains a 48 nucleotide sequence (SEQ ID 53) which is not found in the closely-related HERV-Ks on chromosomes 3, 6 and 16. The 48mer encodes 16mer SEQ ID 110, which is not found in new or in other old HERV-Ks. The top 5 hits in BLAST analysis of a 99mer (3614 to 3712 from SEQ ID 1) comprising SEQ ID 53 shows:

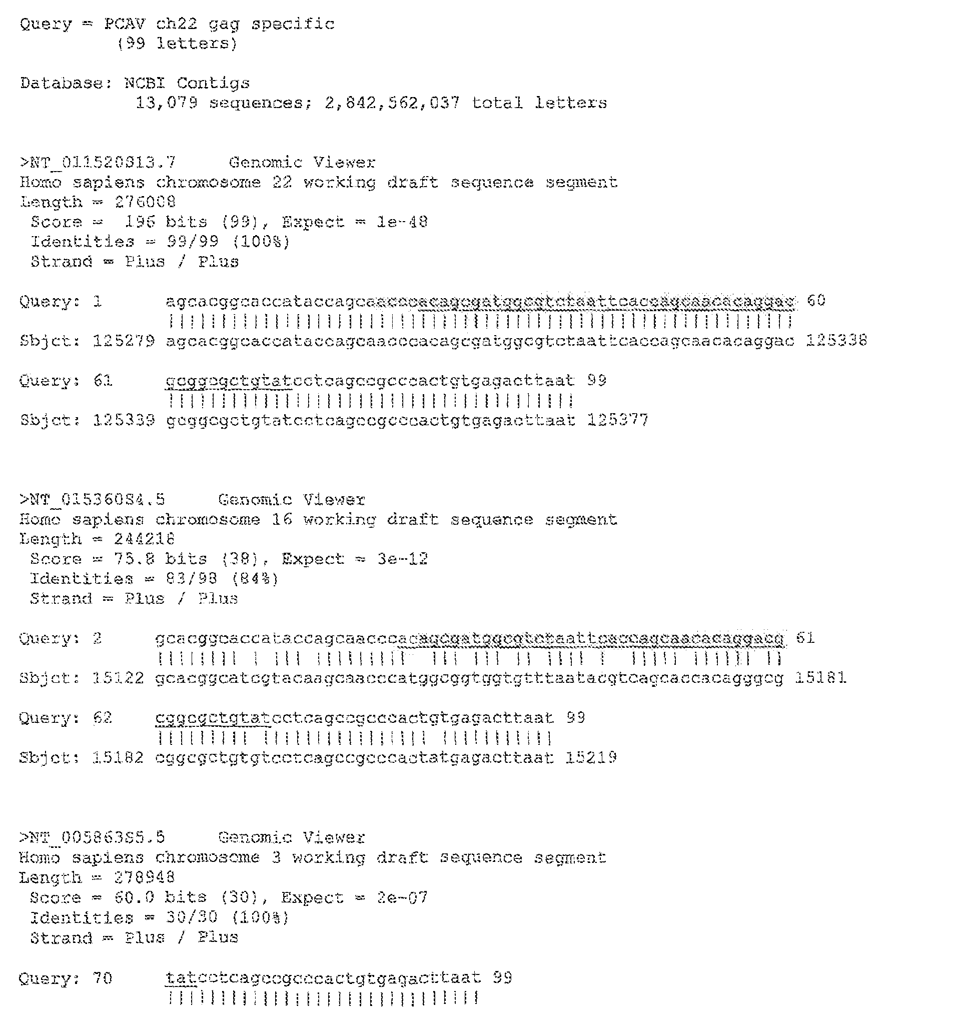

Sbjct: 116212 tatcctcagccgcccactgtgagacttaat 116241

>NT_023409S14.5     Genomic Viewer
Homo sapiens chromosome 6 working draft sequence segment
Length = 238047
 Score = 52.0 bits (26), Expect = 5e-05
 Identities = 26/26 (100%)
 Strand = Plus / Minus

Query: 1     agcacggcaccataccagcaacccac 26
             ||||||||||||||||||||||||||
Sbjct: 63402 agcacggcaccataccagcaacccac 63377

>NT_007592S47.5     Genomic Viewer
Homo sapiens chromosome 6 working draft sequence segment
Length = 250001
 Score = 50.1 bits (25), Expect = 2e-04
 Identities = 28/29 (96%)
 Strand = Plus / Minus

Query: 71    atcctcagccgcccactgtgagacttaat 99
             ||||||||||| |||||||||||||||||
Sbjct: 81143 atcctcagccgtccactgtgagacttaat 81115

### Epitopes within PCAV gag

An alignment of the N-termini of various HERV-Ks is shown below:

1                                                  50
HERV-K gag tandem (1) MGQTKSKIKSKYASYLSFIKILLKRGGVKVSTKNLIKLFQIIEQFCPWFP
PCAV gag CH8 8.032mb (1) MGQTE----SKYASYLSFIKILLRRGGVKASTENLVTLFQTIEQFRPWFP
PCAV gag CH8 7.37mb (1) MGQTE----SKYASYLSFIKILLRRGGVKASTENLVTLFQTIEQFRPWFP
PCAV gag CH6 47.1 mb (1) MGQTE----SKYASYLSFIKILLRRGGVRASTENLITLFQTIEQFCPWFP
PCAV ch22 20.428mb + LTRs (1) MGQTE----SKYASYLSFIKILLRRGGVRASTENLITLFQTIEQFCPWFP
PCAV gag ch6 30.9Mb (1) MGQTE----SKYASYLSSIKILLKRGGVRVSTKNLIMLFQTIEQFCPWFL
PCAV gag CH3 103.75mb (1) MGQTKSKTKSKYASYLSFIKILLKRGGVRVSTKNLIKLFQIIEQFCPWFP
PCAV gag ch5 151.108mb (1) MGQTE----SKYASYLSFIKIILRRGGVKASTENLIMLFQTIEQFCPWFP
PCAV gag ch8 142.771mb (1) MGQTE----SKYASYLSFIKILLRRGGVKASTENLITLFQTTEQFCPWFP
PCAV gag ch11 57.875mb (1) MGQTE----SKYASYLSFIKILLKRGELESLQKNLITLFQTIEQFCPWFL

51                                                 100
HERV-K gag tandem (51) EQGTLDLKDWKRIGKELKQAGRKGNIIPLTVWNDWAIIKAA-LEPFQTEE
PCAV gag CH8 8.032mb (47) EKGTLDLKDWEKIGKELKQAIREGKIIPLTVWSDWATIKAT-LEQLQIEE
PCAV gag CH8 7.37mb (47) EKGTLDLKDWEKIGKELKQAIREGKIIPLTVWSDWATIKAT-LEQFQIEE
PCAV gag CH6 47.1 mb (47) EQRTLDLKDWEKNWQRIKTNKREGKIILLTVWNDWAIIKAT-LEPFQTEE
PCAV ch22 20.428mb + LTRs (47) EQGTLDLKDWEKIGKELKQANREGKIIPLTVWNDWAIIKAT-LEPFQTGE
PCAV gag ch6 30.9Mb (47) EQGTLDLKDWEKIGKELKQTIREGKIIPLTVWNDWVIIKVA-LELFQTGE
PCAV gag CH3 103.75mb (51) EQGTLDLKDWKRIGEELKQAGRKGNIIPLTVWNDWAIIKAA-LEPFQTKE
PCAV gag ch5 151.108mb (47) EQGTLYLKDWEKIGKELKQASREGKIIPLTVWNDWAIIKAT-LEPFQIEE
PCAV gag ch8 142.771mb (47) EQGTLDLKDWEKIGK-LKQASREGKIIPLTVWNDWAIIKAT-LEPFQTGE
PCAV gag ch11 57.875mb (47) EQGTLELKDWEKIGKELKQAGRKGKIIPLTVWNDWAIIKAANLELFQTGE

101                                                150
HERV-K gag tandem (100) ESVSVSDAPGSCIIDCNENTRKKSQKETEGLHCEYVAE----PVMAQSTQ
PCAV gag CH8 8.032mb (96) ERVSVFDAPESCVIDCEEEAGTEFKKGMESSHCKNAVE----PVLTWSMQ
PCAV gag CH8 7.37mb (96) ERVSVFDAPESCVIDCEEEAGTEFKKGMESSHCKNAVE----PVLTWSMQ
PCAV gag CH6 47.1 mb (96) ESIS--AAPESCVIDCEEEAETEFRKGMESSHCKYVAE----SVMAWSTQ
PCAV ch22 20.428mb + LTRs (96) DIVSVSDAPKSCVTDCEEEAGTESQQGTESSHCKYVAE----SVMAQSTQ
PCAV gag ch6 30.9Mb (96) ESVSVSDAPESCVIDCEEEAGIESQKGTESSHCKYVAE----LVMTRSMQ
PCAV gag CH3 103.75mb (100) ESVSVSDAPGSCVIDCNEKTGRKSQKETESLHCEYVTE----PVMAQSTQ
PCAV gag ch5 151.108mb (96) ESVSVSDAPESCVIDCEEEAGTEFKKGMESSHCKNVAE----SVMARSMQ
PCAV gag ch8 142.771mb (95) ESVSVSDAPESCVIDCEEETGTEFKKGMESSHYKNVVE----SVMAQSMQ

PCAV gag ch11 57.875mb (97) DSVSVADAPESCVIDCEE-AGIESQKGMESSKVHIVNMQSLWLGQCKMLT

151 200
HERV-K gag tandem (146) NVDYNQLQEVIYPETLKLEGKGPELVGPSESKPRG-TSPLPAGQVPVTLQ
PCAV gag CH8 8.032mb (142) NVDYNQLQEVIYPESSKLGEGGPELFGPSEFRPRWPPTPSPAVQMPVMSQ
PCAV gag CH8 7.37mb (142) NVDYNQLQEVIYPESSKLGEGGPELFGPSEFRPRWPPTPSPAVQMPVMSQ
PCAV gag CH6 47.1 mb (140) NVDYNQLKEVIYHE-SKLGEGGPELLGPSEPKPRWPSTP-PVVQIPVTLQ
PCAV ch22 20.428mb + LTRs (142) NVDYSQLQEIIYPESSKLGEGGPESLGPSEPKPRSPSTPPPVVQMPVTLQ
PCAV gag ch6 30.9Mb (142) NVDYNQLQEVIYPETLKLEEKGPKLAGPSESKPQW-PTPLPAVQMPVTLQ
PCAV gag CH3 103.75mb (146) NVDYNQLQGVIYPETLKLEGKGPELVGPSESKPRG-PSPLPAGQVPVTLQ
PCAV gag ch5 151.108mb (142) NC-L-SITGGNIS-IIKIGRRRSRIIWVIRV-TTTAINSSSCGSDACDIT
PCAV gag ch8 142.771mb (141) NVDYNQLQEVIYPESSKLGEGGPELFGPSEFRPRWPPTPSPVVQMPVTLQ
PCAV gag ch11 57.875mb (146) TINYRR-YIIKH-NKKKVQNRGHQSINHDGQLLFQRFRCL-HYNIKCRLD

201 250
HERV-K gag tandem (195) PQ----------------------------KQVKENKTQPPVAYQY
PCAV gag CH8 8.032mb (192) SQ----------------------MPIQAQYPQYQPVENKTQPSVVYQH
PCAV gag CH8 7.37mb (192) SQ----------------------MPIQAQYPQYQPVENKTQPSVVYQH
PCAV gag CH6 47.1 mb (188) PQREVRQVQTPREYQVEKDRVSIPAMPIQMQYPQYKLVENKTQPPVIYQY
PCAV ch22 20.428mb + LTRs (192) PQTQVRQAQTPRENQVERDRVSIPAMPTQIQYPQYQPVENKTQPLVVYQY
PCAV gag ch6 30.9Mb (191) PQMQVRQVQTPEEYQIEKDKVSAMAMPIQIQYPQYQQVENKTQLPVAYQY
PCAV gag CH3 103.75mb (195) PQ-------------------------------TQVKENKTQPPVAYQY
PCAV gag ch5 151.108mb (188) TSNAG-----------------------TSTNCKRISKKIKPNHW-FI
PCAV gag ch8 142.771mb (191) PQ----------------------MPIQTQYLQYQLVENKTQPLVVYQH
PCAV gag ch11 57.875mb (193) KYKPQENIK----------RVSVMATPIQIQYPQYQPVENKTQPPVAYQY

251 300
HERV-K gag tandem (213) WPPAELQYRPPPESQYGYPGMPPAPQGRAPY-----------------
PCAV gag CH8 8.032mb (219) QPPAAFQYPPSPEVQYGSQAVRPVPNSKALY-Q----------------
PCAV gag CH8 7.37mb (219) QPPAAFQYPPSPEVQYGSQAVRPVPNSKALYQQ----------------
PCAV gag CH6 47.1 mb (238) WPPADLQYRPPPEVKYRPQVVCPVPNSTAPYQQ----------------
PCAV ch22 20.428mb + LTRs (242) RLPTELQYRPPSEVQYRPQAVCPVPNSTAPYQQPTAMASNSPATQDAALY
PCAV gag ch6 30.9Mb (241) WPPAELQYRPPPENQYGHPGMFPAPQGRALD-----------------
PCAV gag CH3 103.75mb (213) WPPAELQYLPPPESQYGYPGMPPALQGR------------------APY
PCAV gag ch5 151.108mb (212) NTGRQSNFSISCFQRFSIDLRRGVLSQIAGHYISNPQRWRLILQH-----
PCAV gag ch8 142.771mb (218) WPPAEFQYRPSPEVQYRSQMGSCAK--GTIISTTHGDGV-----------
PCAV gag ch11 57.875mb (233) WPPAELQYRPPPENQYGQPGVFPAPSGRAL-------------------

301 350
HERV-K gag tandem (244) PQPPTRRLNPTAPPSRQGSKLHEIIDKSRKEGDTEAWQFPVTLEPVPPGE
PCAV gag CH8 8.032mb (251) --PTAMAFDLTVPPSGQDSALHETIATARKQGDLEAWQYPVVLQPVPAGK
PCAV gag CH8 7.37mb (252) --PTAMAFDLTVPPSGQDSALHETIATARKQGDLEAWQYPVVLQPVPAGK
PCAV gag CH6 47.1 mb (271) --PTAVVFNPTAPPSGQGSTLHEIIDKASKQGDLEAWEFPVILQPIPAGK
PCAV ch22 20.428mb + LTRs (292) PQPPTVRLNPTASRSGQGGALHAVIDEARKQGDLEAWRFLVILQLVQAGE
PCAV gag ch6 30.9Mb (272) PQPSTVRLNPTALPSGQHSALHKIIDKARKQGDTEA-QFPVTLELIPPGE
PCAV gag CH3 103.75mb (244) PQPPTVRLNPTASRSGQGGTLHAVIDEARKQGDLEAWRFLVILQLVQAGE
PCAV gag ch5 151.108mb (257) --------------QLVYKIVHCIKPLARKQGDLEAWQYPVILQPILAGK
PCAV gag ch8 142.771mb (254) ------SYSTSWTR-CTA-DR-YSQKTGRS-GMAVSGNVTTDTGRERARK
PCAV gag ch11 57.875mb (263) PQPPTIRLYPTAPPSGQGSALHKIIDKARKQGDIEAWQFPV---PMPPGE

351 400
HERV-K gag tandem (294) GAQEGEPPTVEARYKSFSIKKLKDVKEGVKQYGPNSPYMRTLLDSIAHGH
PCAV gag CH8 8.032mb (299) GSQ-----------------------AGVKQYGPNSPYTRILLNSIAHGN
PCAV gag CH8 7.37mb (300) GSQ-----------------------AGVKQYGPNSPYIRILLNSIAHGN
PCAV gag CH6 47.1 mb (319) GTLAG---------------------ASVQTEARYESFTMKMLKDVKEGV
PCAV ch22 20.428mb + LTRs (342) ETQVGAPARAETRCEPFTMKMLKDIKEGVKQYGSNSPYIRTLLDSIAHGN
PCAV gag ch6 30.9Mb (321) GAQEGVPPLAEARYESFSIKMLKDVKEGVKQYGPSSPYMKILLDSIAHGQ
PCAV gag CH3 103.75mb (294) ETQVGAPARAETRCEPFTMKMLKDIKEGVKQYGSNSPYIRTLLDSIAHGN
PCAV gag ch5 151.108mb (293) GSQAGASVQTEIRYESFTLKMLKDVKEGVKQYGPNSPYMRTLLDSIAHGN
PCAV gag ch8 142.771mb (294) GSQAGASVRTEAR-EYFTMKMLKDIKEGAKQYGPNSPYMRTLLDSIAHGN
PCAV gag ch11 57.875mb (310) GAQEGAPTLVGARYESFSIKMLKDVKEGVKQYGPNSPYMRTLLGSTAHGH

Two regions are particularly useful for generating PCAV-specific detection reagents. The first is from amino acid 203 to 225 in the alignment (SEQ ID 55; encoded by SEQ m 111). Although this region is present in two other HERV-Ks on chromosome 6, those two viruses are in the old HERV-K group. Background ("ubiquitous") expression of new HERV-Ks is seen in many tissues *(e.g.* Figure 10), but not of old HERV-Ks. Detection of SEQ ID 55 therefore distinguishes over background expression of new HERV-Ks and can be used to detect PCAV expression.

The second region is found from amino acids 284-300 (SEQ ID 56; encoded by SEQ ID 112), as this sequence is unique to PCAV. SEQ ID 110 (SEQ ID 53) is a single amino acid truncation fragment of SEQ ID 56.

TBLASTN analysis of SEQ ID 110 against the human genome sequence reveals 100% matches in clones KB208E9 and KB1572G7 at chromosome 22q11.2 but nowhere else. BLASTP analysis fails to identity any matches.

BLASTN analysis of SEQ ID 53 against the human genome sequence reveals a 100% match at nucleotides 3180761 to 3180808 of the Homo sapiens chromosome 22 working draft sequence, and no further hits.

The top five BLASTP hits using SEQ ID 110 against the non-redundant GenBank CDS database are shown below:

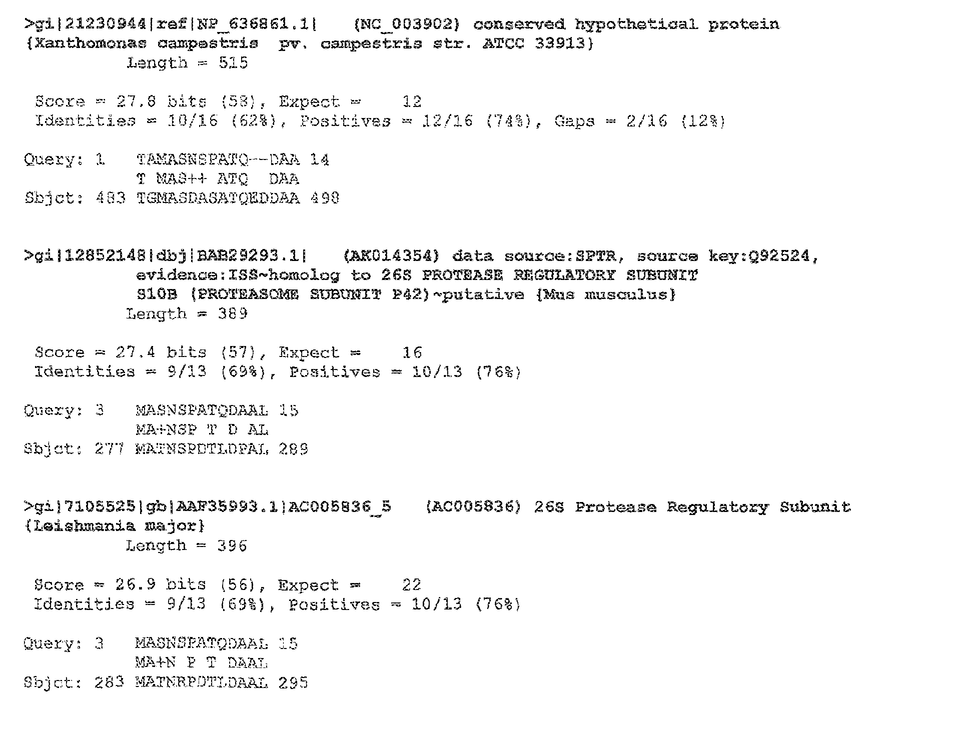

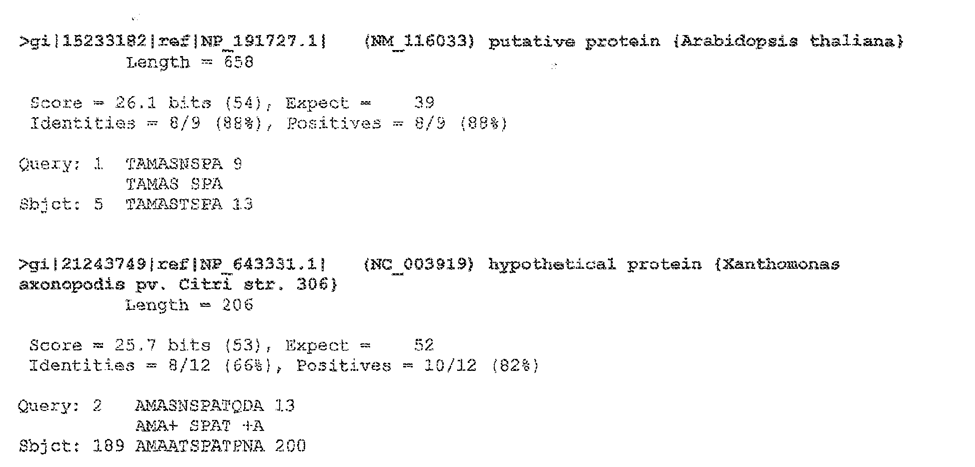

SEQ ID 110 is therefore unique to PCAV.

### Prediction of cDNA sequences

On the basis of splice donor and acceptor sites, SEQ IDs 99 to 109 were constructed. SEQ ID 109 begins in the second 5' LTR.

SEQ IDs 99 to 108 align to SEQ ID 10 as follows:

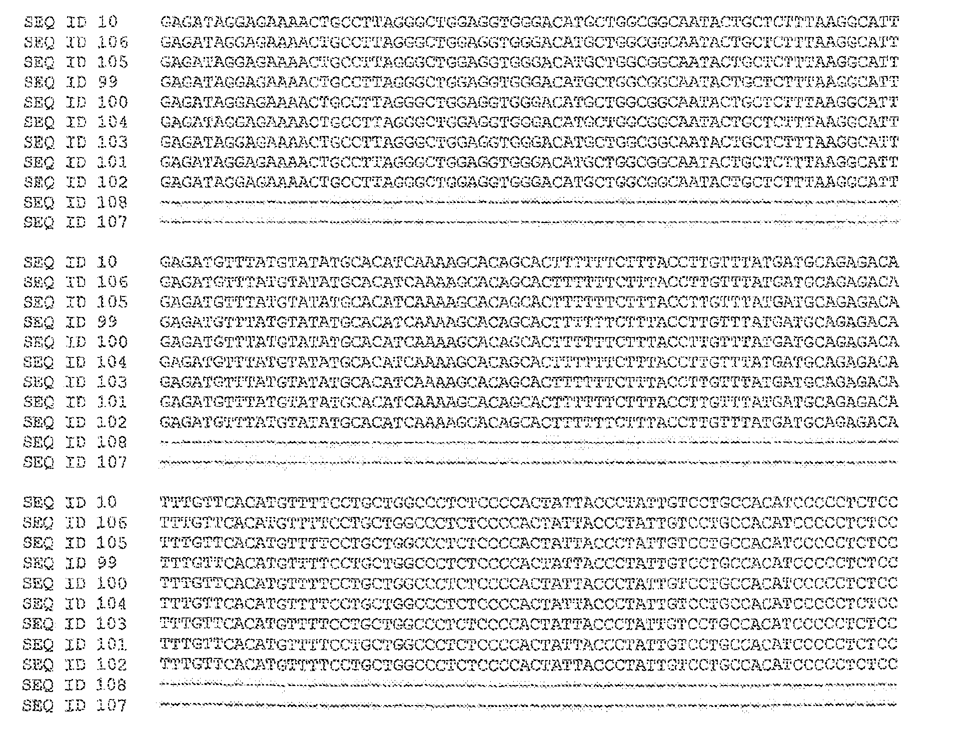

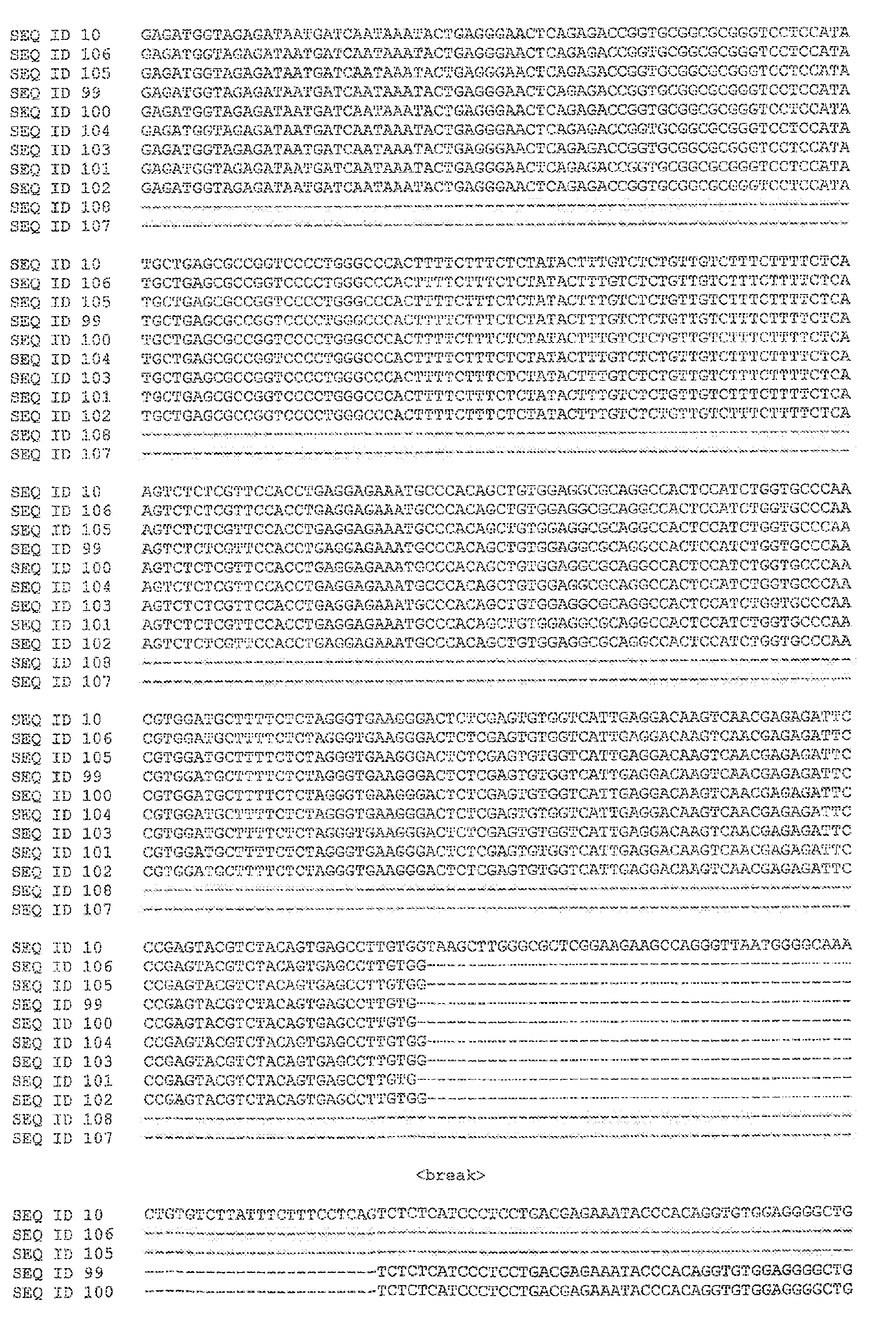

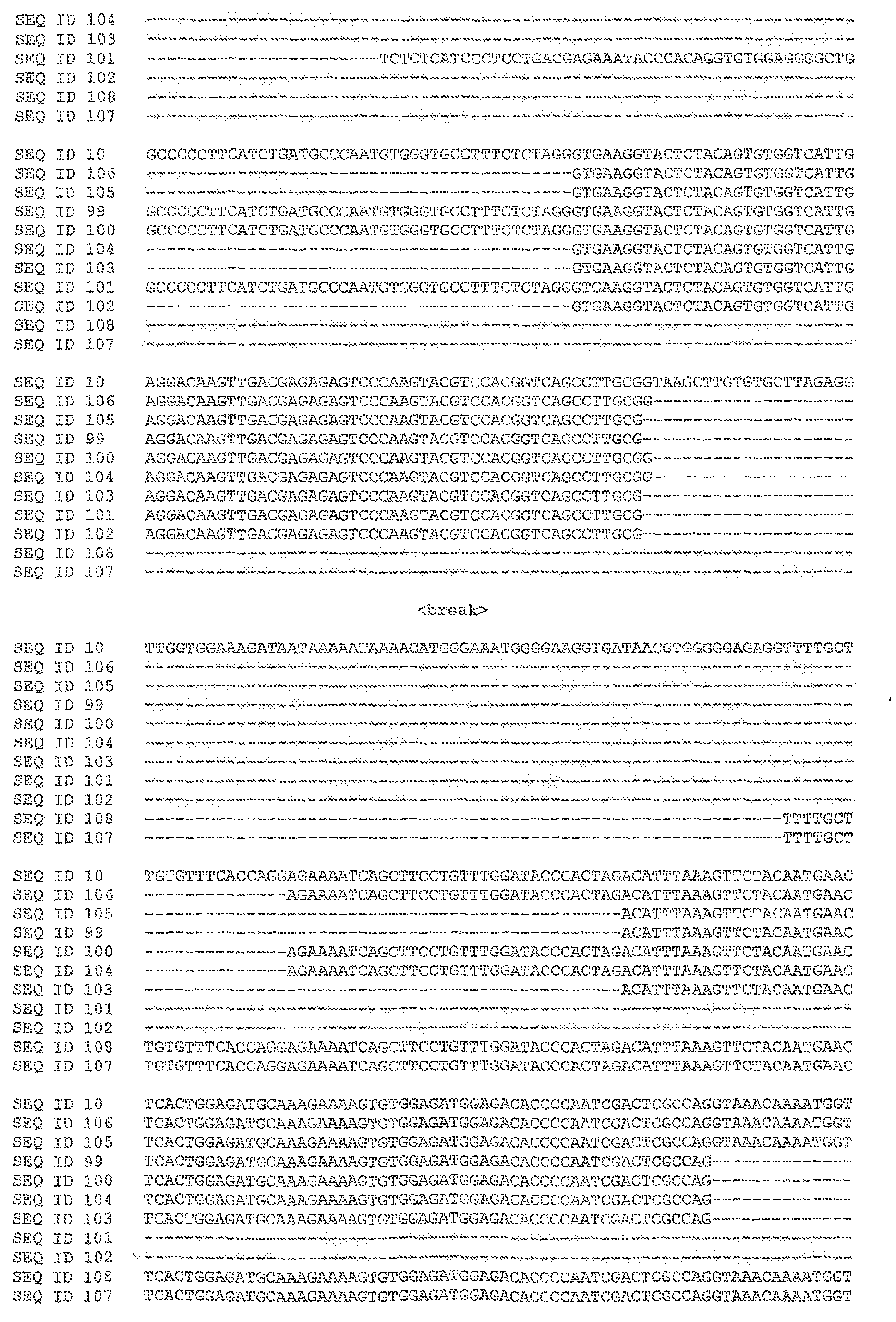

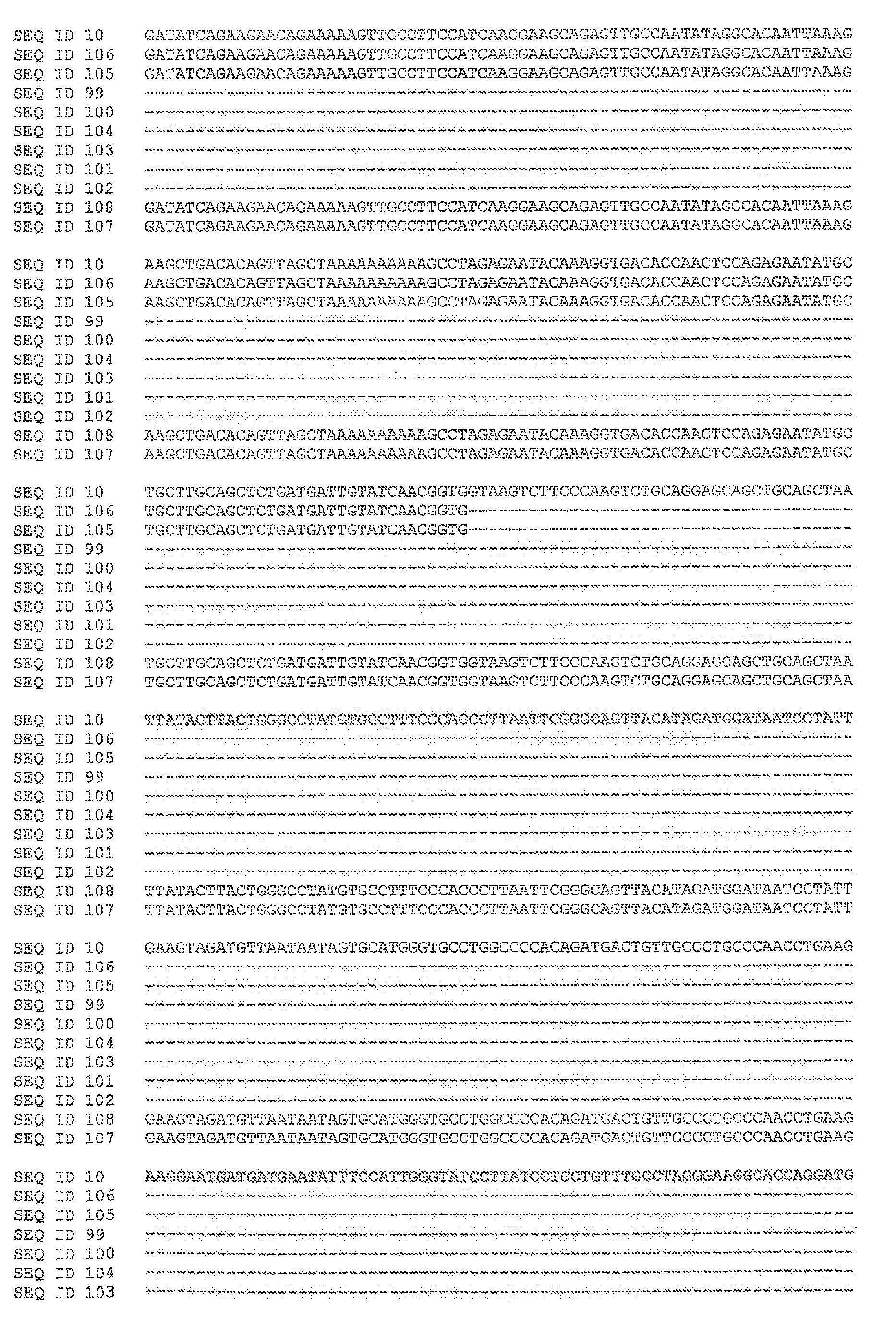

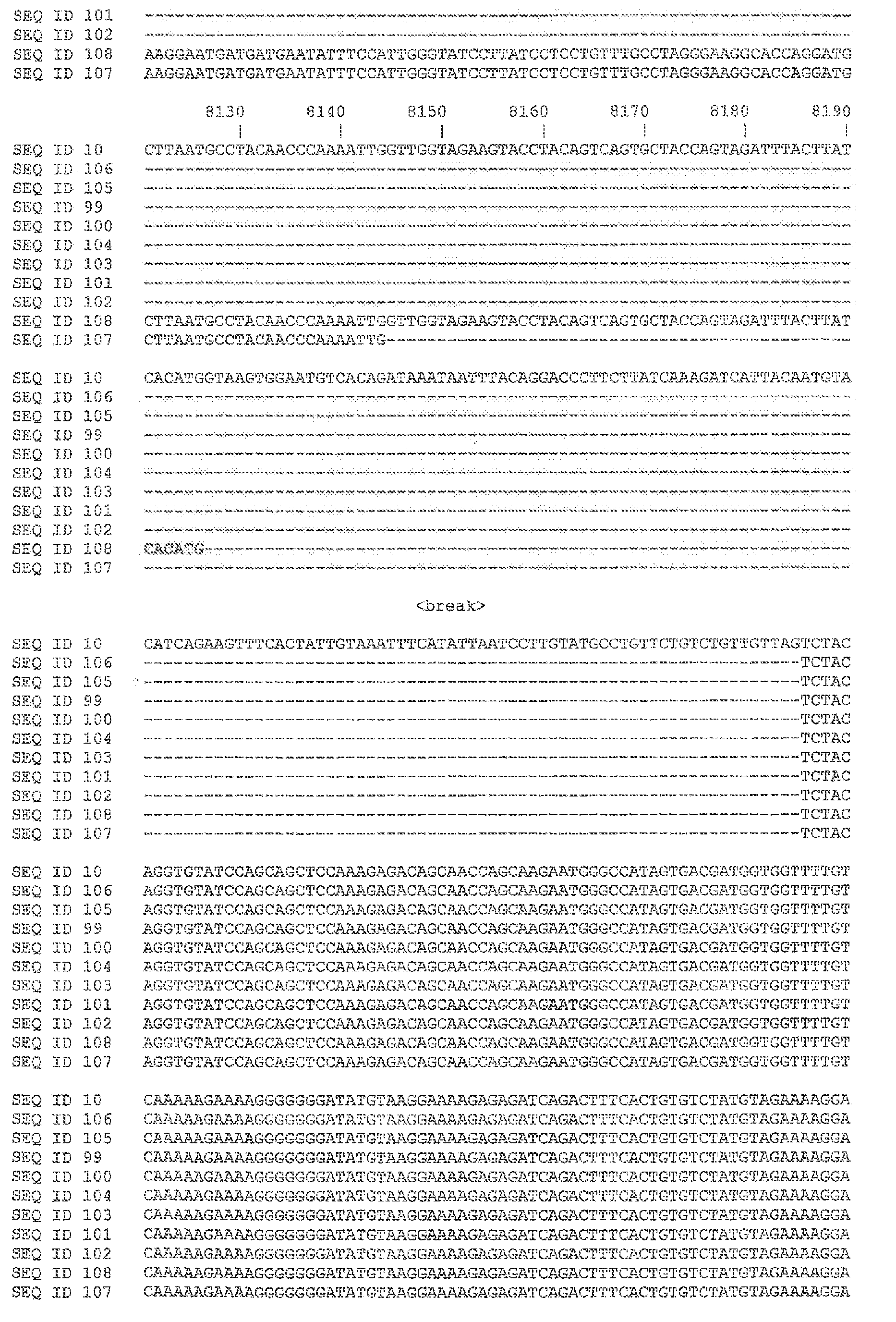

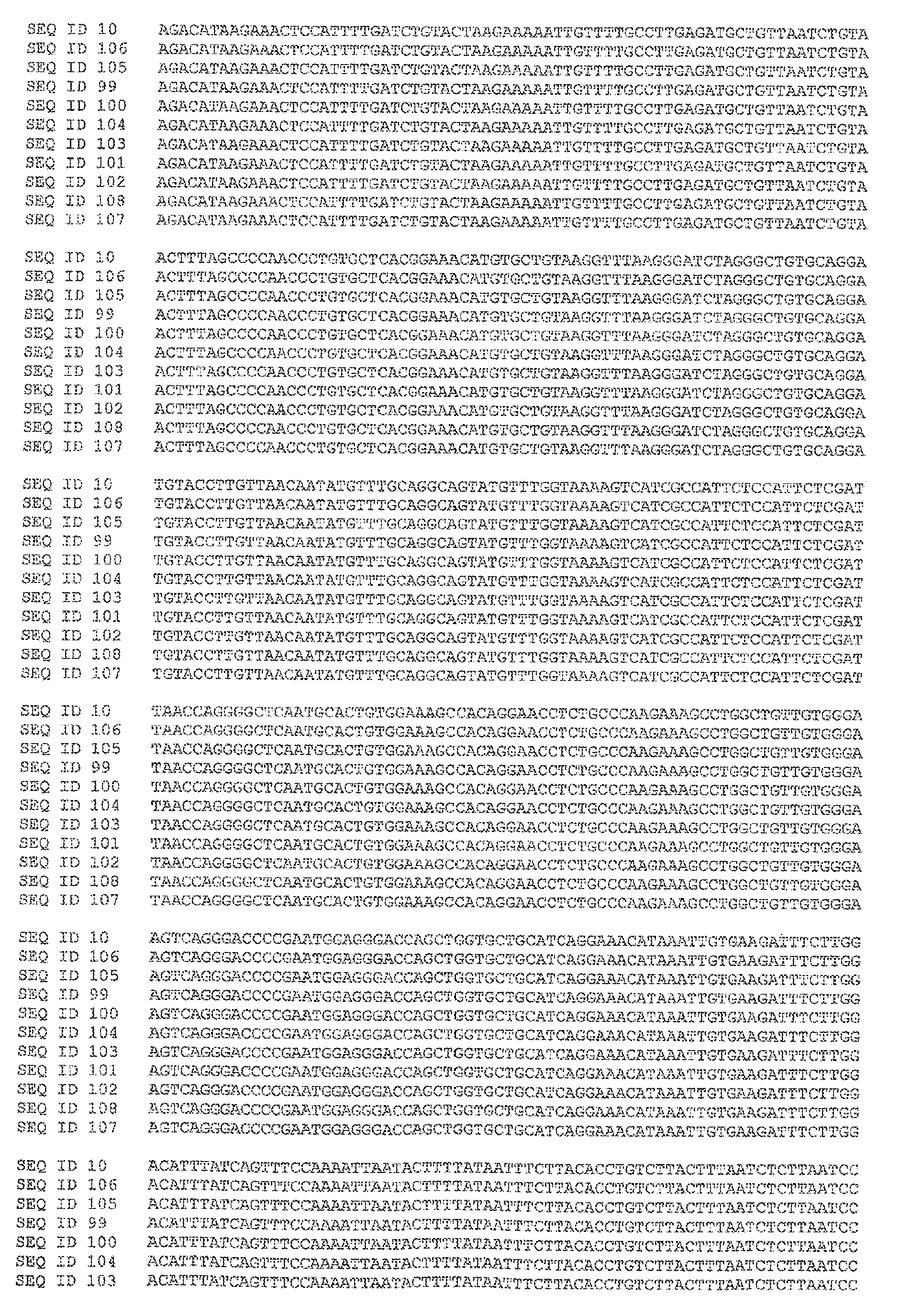

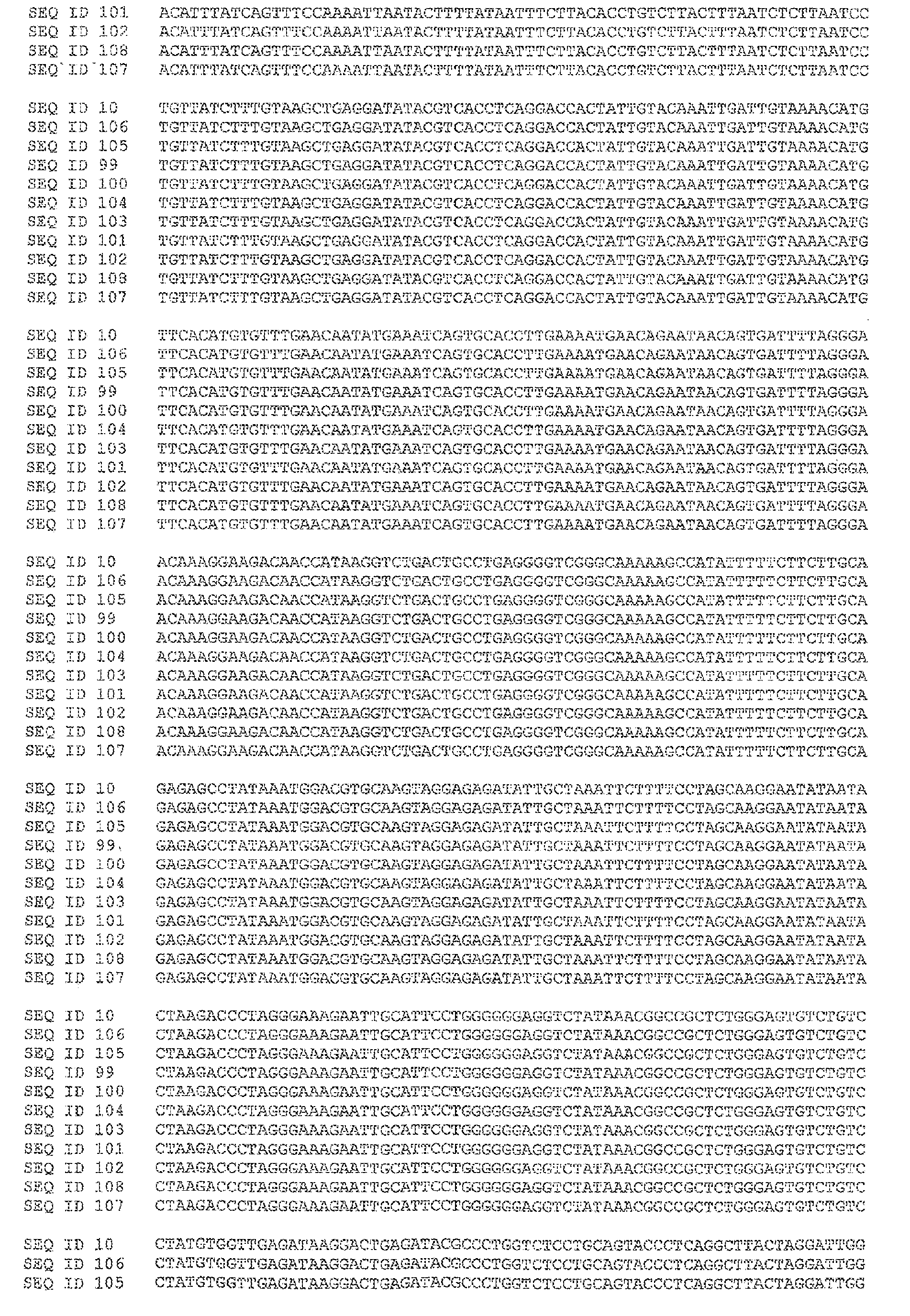

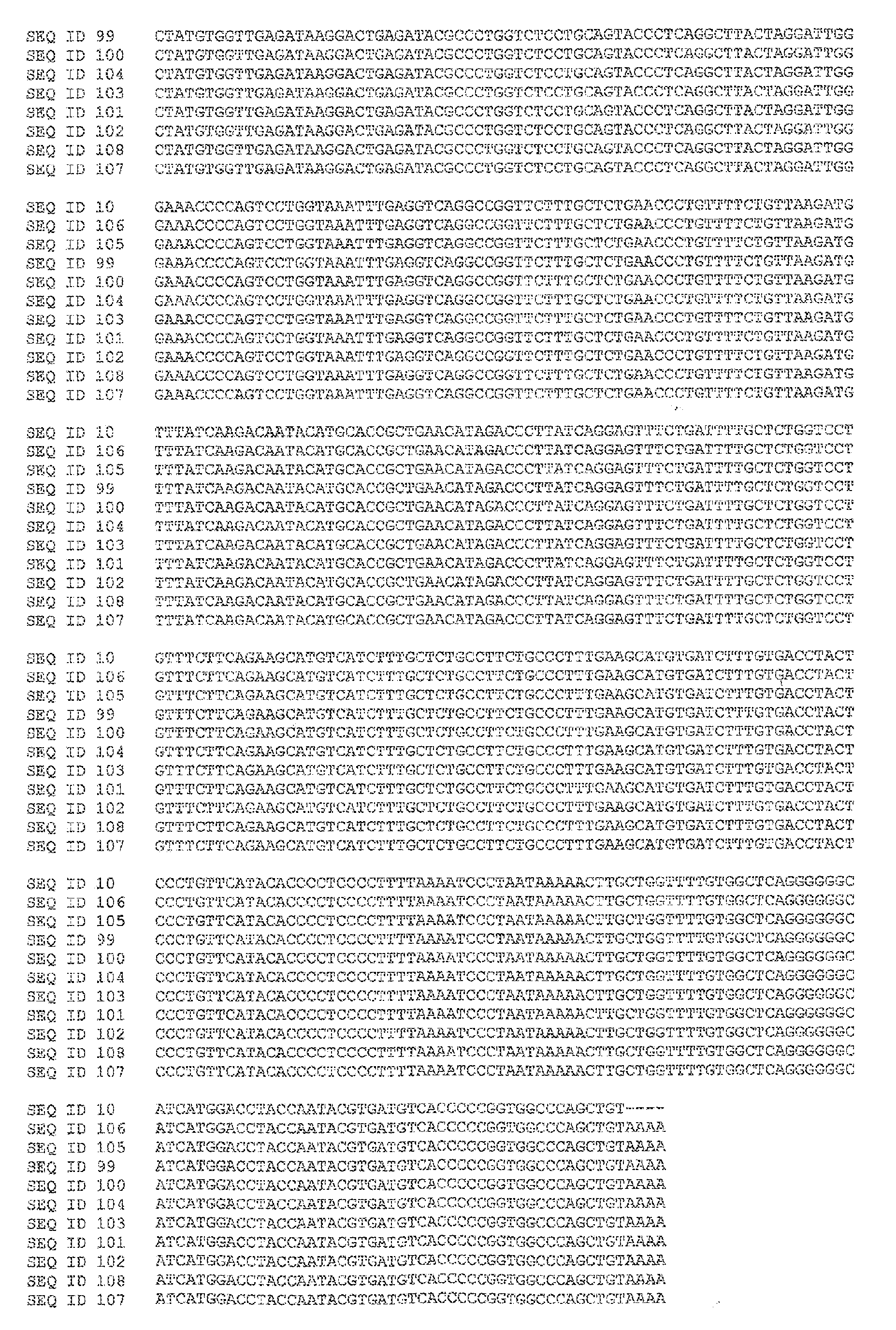

### THE TRANSCRIPTION START SITE OF PCAV

By homology to other retroviruses, the 5' end of PCAV-mRNA (*i.e.* the transcription start site within the PCAV genome) should fall 30 bases downstream of the canonical TATA sequence, at nucleotide 559 in SEQ ID 1.

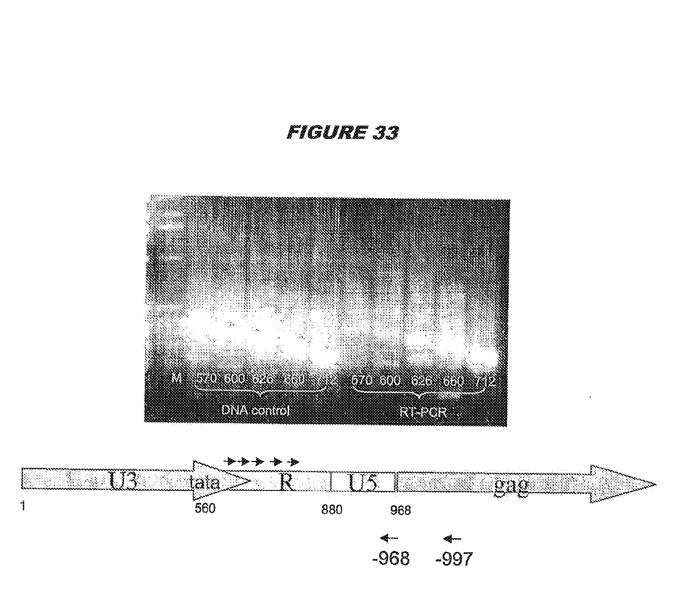

However, empirical work suggests that the 5' end of PCAV-mRNA is further downstream. Figure 33 shows the results of a RT-PCR scanning assay used to map the 5' end. cDNA of the 5' LTR was prepared by priming total Tera1 RNA with an antisense oligonucleotide spanning 997 to 972 in the proviral genome (SEQ ID 1202). This cDNA was then divided and run in PCR analyses with an antisense primer from 968 to 950 (SEQ ID 1203) combined with a sense primer from a set of primers designed to cover the likely 5'ends: 1) 571 <SEQ ID 1204>, 2) 600 <SEQ ID 1205>, 3) 626 <SEQ ID 1206>, 4) 660 <SEQ ID 1207>, 5) 712 <SEQ ID 1208>. Duplicate PCR reactions on 1 µg genomic HeLa DNA were used as a positive control, and these reactions showed all primer pairs were effective. The reactions primed with cDNA showed a marked difference between primers 600 and 626, suggesting that the 5' end lies near position 626 in the proviral genome.

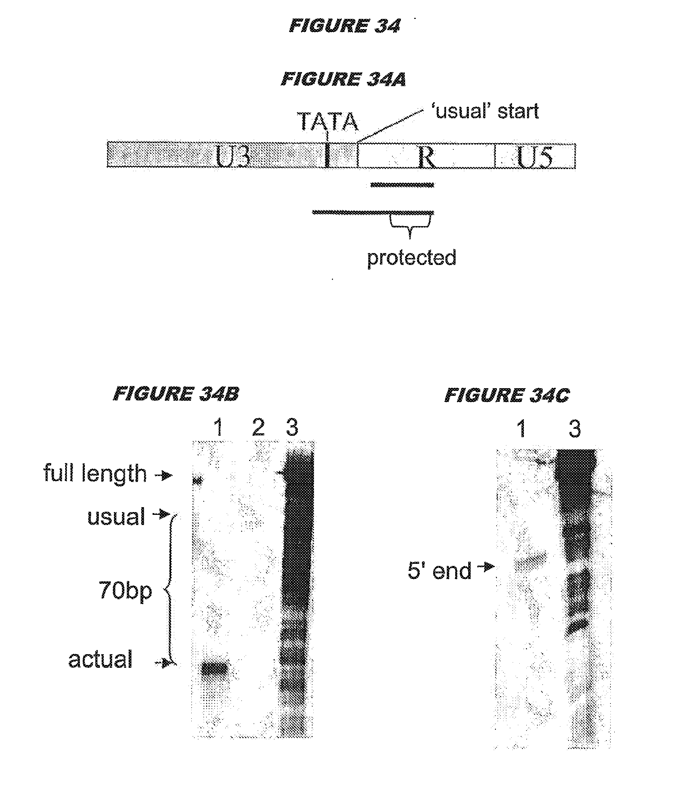

This result was confirmed using RNase protection assays (Figure 34). Labeled antisense RNA probes covering bases (34B) 509-735 and (34C) 600-735 in the proviral genome were hybridized to total RNA from Tera1 cells and digested with RNase under standard conditions. After processing and detection by urea-containing PAGE, both probes gave 100 base products. These two results agree and show that 5' end of HFRV-K RNA is around base 635 in the proviral genome *i.e.* around 100bp downstream of the TATA signal, rather than the 30bp which is usual for TATA-dependeat genes.

### PCAP3

Within the final exon in the env region of PCAV, reading frames 1 and 2 encode env and cORF, respectively (Figure 23). SEQ ID 87 is PCAP3, which shares the same 5' region and start codon as env, but in which a splicing event removes env-coding sequences and shifts to a reading frame +2 relative to that of env (SEQ IDs 88 & 1191):

ATGAACTCACTGGAGATGCAAAGAAAAGTGTGGAGATGGAGACACCCCAATCGACTCGCCAGgtaaacaaa 8253
M N S L E M Q R K V W R W R H P N R L A

...cctgttctgtctgttgttagTCTACAGGTGTATCCAGCAGCTCCAAAGAGACAGCAACCAGCAAGAATGGGCCATAG 10480
L Q V.Y P A A P K R Q Q P A R M G H S

TGACGATGGTGGTTTTGTCAAAAAGAAAAGGGGGGGATATGTAAGGAAAAGAGAGATCAGACTTTCACTGTGTCTATGTA 10560
D D G G F V K K K R G G Y V R K R E I R L S L C L C R

GAAAAGGAAGACATAAGAAACTCCATTTTGATCTGTACTAA 10601
K G R H K K L H F D L Y *

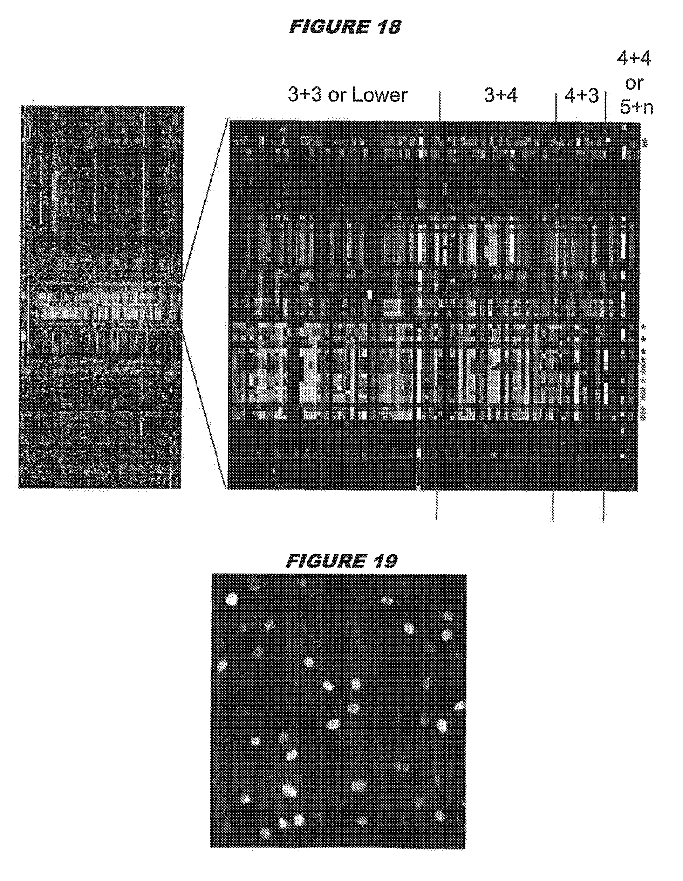

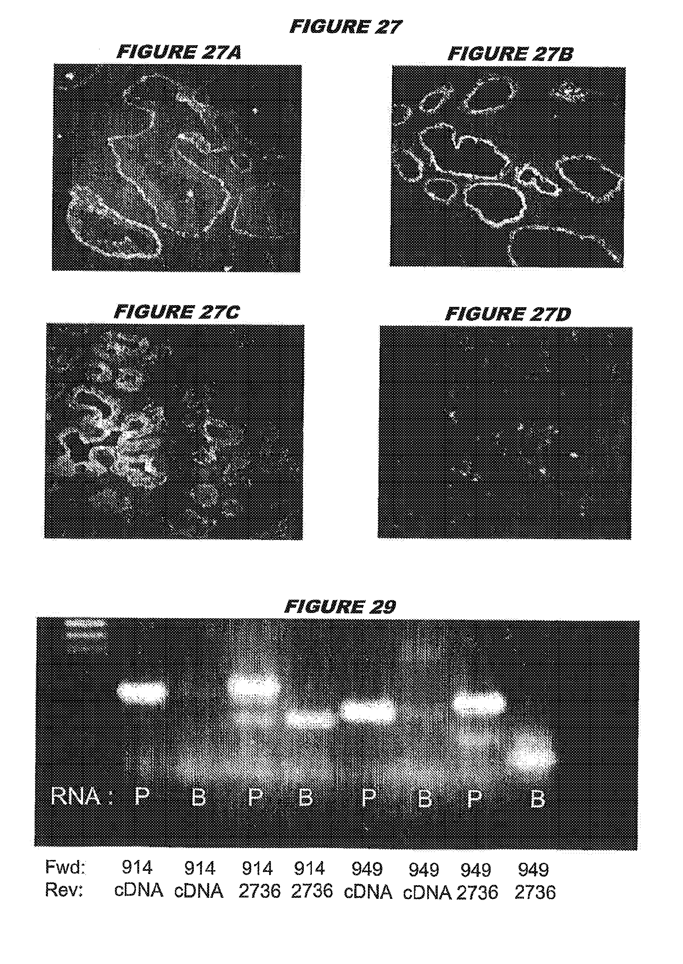

The majority of the coding sequence is thus located after the splice, within the exon which contains the 3' LTR. Although the +2 reading frame has no known function in HERV-K, cDNA prepared from prostate cancer cell line MDA Pca-2b included these transcripts, as did prostate cancer mRNA. For example, spot 34058 (see above) encodes PCAP3 and was up-regulated more than 2-fold in 79% of patient samples and more than 5-fold in 53%. These figures support the view that PCAP3 is involved in many prostate cancers. Furthermore, the figures do not reflect the whole relationship between cancer and PCAP3 expression - if patients are grouped according to Gleason grades, grade 3 tumors show high up-regulation of PCAP3 whereas more developed grade 4 tumors seem to show PCAP3 suppression. Figure 18 shows microarray analysis of prostate cancer employing 6000 random ESTs from a normalized prostate library. RNA levels prepared from laser-captured, micro-dissected tumor is compared to peri-tumor normal tissue RNA. The sequences tagged with asterisks in Figure 18 are up-regulated and are all from a single 12kb site in chromosome 22. These sequences span all portions of PCAV. Relative PCAV expression is very high in grade 3 tumors, with many of the patients having tumor/normal ratios in the 10 to 50 fold range. In Gleason grade 4 and above, however, the ratios return to 1 and in some cases the virus expression is suppressed. A similar pattern is seen with gag expression (Figure 27), suggestion that PCAV expression is involved in the early stages of prostate cancer.

PCAP3 is similar to the cORP protein, and the two ORFs share a start codon, but two small deletions in PCAV introduce both a frameshift and an 'old virus' 5' splice site (splice acceptor), thereby permitting the PCAP3-specific splice event. Inspection of various aligned HERV-K genomes gives further evidence that PCAP3 is a mutated form of an original protein. The protein is thus unlikely to be functioning in its original capacity, and oncogenic activity could arise through retention of a functional domain. The coding exon common to env, cORF and PCAP3 contains a RNA-binding domain that also functions as a nuclear localization signal (NLS).

To study the subcellular localization of PCAP3, in order to better understand its role, an adenovirus expressing PCAP3 with a C-terminal V5 tag (SEQ ID 1189) was used to infect primary prostate epithelial cells. The protein was relatively stable and was labeled in the nucleoplasm by anti-V5 (Figure 19). The concentration of this small protein in this cellular location shows that it is specifically interacting with something within the nucleus.

A functional expression assay was also designed. The first component of the assay is an adenovirus vector with a PCAV LTR (SEQ ID 1190) driving GFP expression (Figure 24). A variety of human cell lines were infected with this virus and fluorescence was measured either by fluorescent microscopy or by FACS. As a positive control, a vector was used in which GFP expression was driven by the EF-a promoter, which should be active in all eukaryotic cells.

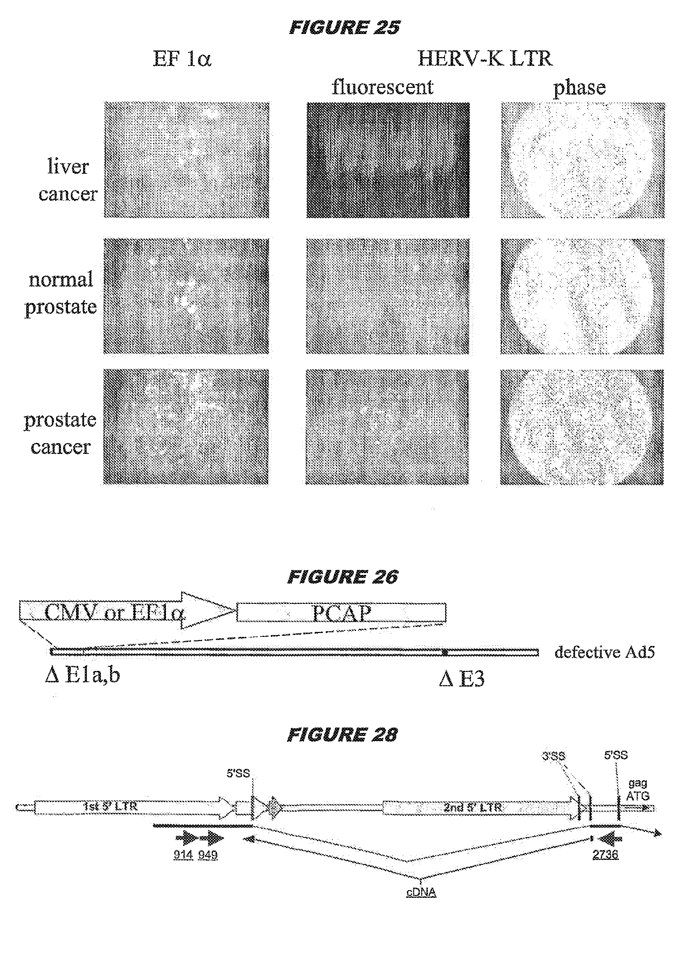

GFP expression was minimal in ovarian, colon and liver cancer cells. It was also minimal in 293 cells, an immortalized kidney cell line, and in primary prostate epithelium cells. GFP was easily detected in various prostate cancer cell lines (PC3, LNCaP, MDA2B PCA, DU145). Representative data are shown in figure 25. The GFP expression pattern exactly matches genomics results from patient samples. These data indicate that expression driven from a PCAV-mRNA LTR is a marker for prostate cancer.

As GFP expression from the LTR appeared to be silent in primary prostate cells, but active in prostate cancer tissue, PCAP3 was tested for its ability to activate expression in primary prostate cells. The coding sequence was inserted into an expression cassette and incorporated into an adenovirus vector (Figure 26). The vector was co-infected with the GFP vector into primary prostate epithelial cells, and PCAP3 weakly activated GFP expression.

In a separate experiment, high passage PrECs (approaching senescence) were co-infected with an adenovirus vector expressing GFP from an old-type HERV-K LTR ('MDALTR': SEQ ID 1196), and a second vector expressing PCAP3 at moi of about 20. After 3 days, the fluorescent intensity was measured by FACs and activation by PCAP3 was seen. In a similar experiment with LTR60, however, there was no activation.

### PCAP3 AND SENESCENCE

Prostate cancer is believed to arise in the luminal epithelial layer, but normal luminal epithelial cells are capable of very few cell divisions. In contrast, NIH3T3 and RWPE1 cells (see Figures 11 & 12) are immortal. Because PCAV seems to be involved in early stages of cancer, the effects of PCAP3 on primary prostate epithelial cells (PrEC), which normally senesce rapidly, were tested.

Primary human epithelial cells have a very limited division potential. Alter a certain number of divisions the cells will enter senescence. Senescence is distinct from quiescence (immortal or pre-senescent cells enter quiescence when a positive growth signal is withdrawn, or when an inhibitory signal such as cell-cell contact is received, but can be induced to divide again by adding growth factors or by re-plating the cells at lower density) and is a permanent arrest in division, although senescent cells can live for many months without dividing if growth medium is regularly renewed.

Certain genes, particularly viral oncogenes (*e.g.* SV40 T-antigen) force cells to ignore senescence signals. T-antigen stimulates cells to continue division up to a further expansion barrier termed 'replicative crisis'. Two processes occur in crisis: cells continue to divide, but cells die in parallel at a very high rate from accumulated genetic damage. When cell death exceeds division then virtually all cells die in a short period. The rare cells which grow out after crisis have become immortal and yield cell lines. Cell lines typically have obvious genetic rearrangements: they are frequently close to tetraploid, there are frequent non-reciprocal chromosomal translocations, and many chromosomes have deletions and amplifications of multiple loci {169,170,171}.

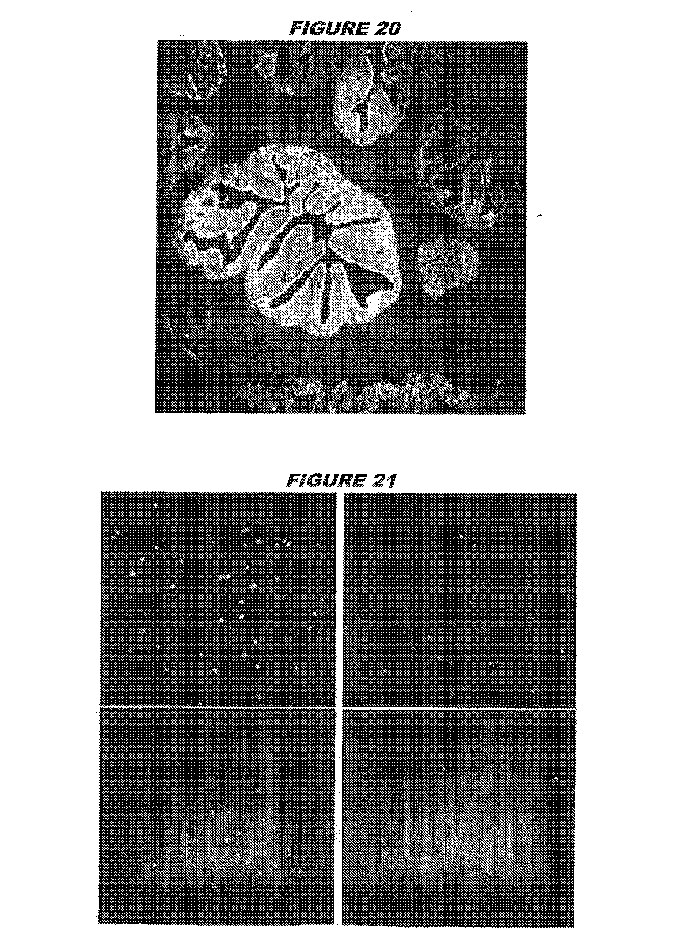

Gene products that lead to crisis are particularly interesting because prostate cancers exhibit high genomic instability, which could be caused by post-senescence replication. Current theory holds that prostate cancer arises from lesions termed prostatic intraepithelial neoplasia (PIN) {172}. Genetic analyses of PIN show that many of the genetic rearrangements characteristic of prostate cancer have already occurred at this stage {173}. PIN cells were thus tested for PCAV expression to determine if the virus could play a role in the earliest stages of prostate cancer. PCAV gag was found to be abundantly expressed (Figure 20), indicating that PCAV expression is high at the time when the genetic changes associated with prostate cancer occur. As PCAP3 was seen to be expressed in prostate cancer, its role was investigated by seeing if it is capable of inducing cell division in PrEC after senescence.

Initial attempts to select drug-resistant PrECs after transfection with PCAP expression plasmids failed. Analysis of PrEC after infection with adenovirus vectors expressing either GFP or PCAP3 revealed abundant cell death on day 4 post-infection in the PCAP3 cells. A dose-dependent increase in terminal deoxytransferase end labeling (TUNEL), to mark nuclei with nicked DNA, confirmed that the cells were undergoing apoptosis (Figure 21). This apoptosis may explain the failure to isolate drug-resistant PrECs, and is consistent with engagement of cell division machinery by PCAP3, as an unbalanced growth signal is an inducer of apoptosis.

These results suggested that apoptosis would have to be blocked before the effect of PCAP3 expression in PrECs could be assessed. Plasmids encoding PCAP3 plus a neomycin marker were thus co-transfected with an expression plasmid encoding bcl-2 (anti-apoptosis) and lacZ (marker). As controls, cells were transfected with plasmids expressing neomycin and either lacZ, bcl-2, bcl-X_{L}, or PCAP3. After two weeks under selection, the lacZ, bcl-2 and bcl-XL dishes all had numerous resistant cells that grew to fill in a faction of the dish. When these cell were split they failed to divide further, but were viable and resembled senescent parental cells. In contrast, the cells which expressed PCAP3 and bcl-2 yielded some colonies made up of small cells which divided to fill the initial plate and continued to divide when split.

In parallel to the above drug selections, the growth potential of cells was assessed. The parental PrECs went through seven population doublings before reaching senescence. In contrast, drug-resistant cells co-transfected with an anti-apoptotic gene plus PCAP3 expanded well beyond the senescence point before ceasing to grow, going through sixteen doublings. After rapid growth for around two weeks, expansion of the cells slowed and finally ceased. Concomitantly, the number of floating and dead cells increased and the appearance of the cells changed - they no longer had the regular "cobblestone" appearance of epithelial cells, but instead had several morphologies, and there were many multinucleate cells. Cells died two weeks later, while the cells transfected with lacZ or lacZ+bcl-2 were still alive one month later.

Neither senescent cells nor cells approaching crisis expand in number. One difference between them, however, is that cells approaching crisis are dividing and dying at an appreciable rate, and so cell division can distinguish between the two states. After labeling with bromo-deoxyuridine, 30% of pre-senescent PrECs were labeled, as were 10% of PrEC transfected with PCAP3 + bcl-2, but none of the senescent lacZ or cORF + bcl-2 controls were labeled (Fig. 22).

These results show that PCAP3 is capable of inducing growth in prostate epithelial cells, and this growth could be an underlying cause of prostate cancer.

### PCAV DETECTION BY PCR

Primer pairs were tested to determine those which produced the expected PCAV product on prostate samples (P) and little or no product on breast sample (B). The primers are shown on the map of the 5' LTRs of PCAV in Figure 28. Forward primers were '914' (SEQ ID 1192) or '949' (SEQ ID 1193); reverse primers were '2736' (SEQ ID 1194) or 'cDNA' (SEQ ID 1195). The cDNA primer spans the splice junction. Each reaction was run for 30 cycles on dT-primed cDNA prepared from total RNA extracted from either MCF7 (B) or MDA PCA 2b (P) cells.

Results are shown in Figure 29. The primers clearly show preferential amplification in the prostate cells, and the primer bridging the splice junction ('cDNA') is highly specific.

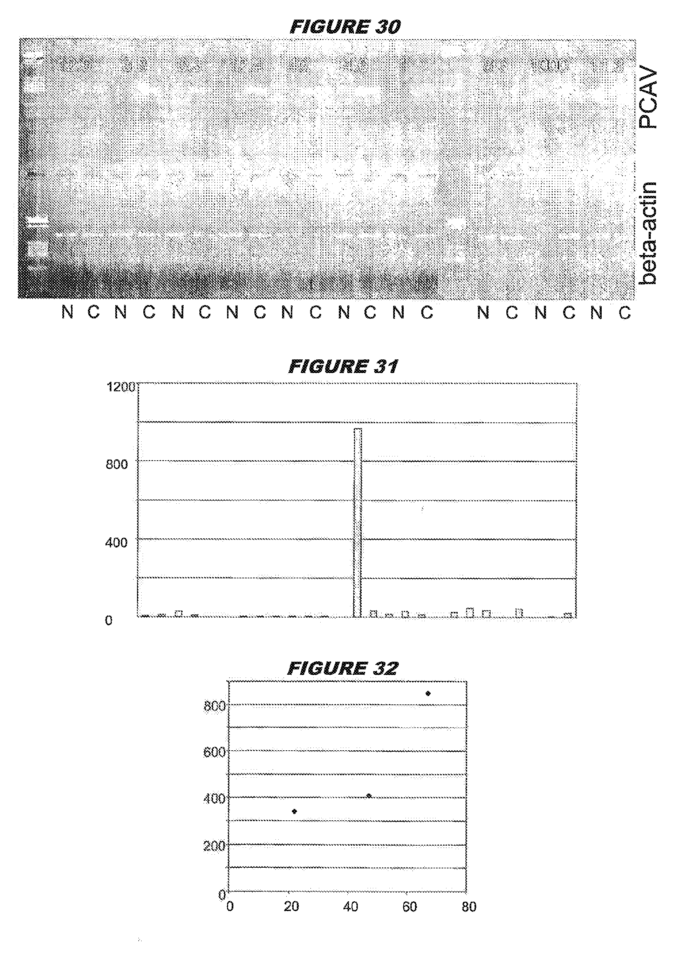

Semi-quantitative RT-PCR experiments were also performed. Amplified RNA from LCM-derived prostate tissue from 10 patients was reverse transcribed using the 2736 primer, followed by PCR amplification either with the '914' and 'cDNA' primer pairs (28 cycles), or with standard primers for human β-actin (25 cycles). Results are shown in Figure 30. Matched samples of normal (N) or cancer (C) were amplified. The signal ratio in cancer tissue compared to normal tissue for each pair is shown above the PCAV PCR products.

Primers '914' and 'cDNA' were also tested in quantitative PCR against dT-primed cDNA from a variety of tissues. As shown, in Figure 31, only prostate tissue from a 47 year old patient gave a significant signal.

RT-PCR was also performed on prostate tissue from patients of various ages. Expression levels were compared to gusB (β-glucuronidase). Results were as follows:

| **Age** | **PCAV RT-PCR** | **GusB RT-PCR** | **Normalized PCAV** | **Normalized GusB** |
|---|---|---|---|---|
| 22 | 546 | 1105 | 1.60 | 340 |
| 47 | 430 | 729 | 1.06 | 406 |
| 67 | 848 | 689 | 1 | 848 |

The normalized PCAV figures are also shown in Figure 32.

The above description of preferred embodiments of the invention has been presented by way of illustration and example for purposes of clarity and understanding. It is not intended to be exhaustive or to limit the invention to the precise forms disclosed. It will be readily apparent to those of ordinary skill in the art in light of the teachings of this invention that many changes and modifications may be made thereto without departing from the spirit of the invention. It is intended that the scope of the invention be defined by the appended claims and their equivalents.

All patents, applications and references cited herein are incorporated by reference in their entirety.

### SEQUENCE LISTING INDEX

| **SEQ ID** | **DESCRIPTION** |
|---|---|
| 1 | PCAV, from the beginning of its first 5' LTR to the end of its fragmented 3' LTR |
| 2 | Fragment of SEQ ID 1, from predicted transcription start site (559) to conserved splice donor site (1075) |
| 3 | Fragment of SEQ ID 1, following a splice acceptor site within second 5' LTR (2611-2620) |
| 4 | Fragment of SEQ ID 1, following a splice acceptor site downstream of second 5' LTR (2700-2709) |
| 5 | SEQ ID 2 + SEQ ID 3 |
| 6 | SEQ ID 2 + SEQ ID 4 |
| 7 | Fragment of SEQ ID 1: 5' end of 3' LTR (10520-10338) |
| 8 | Fragment of SEQ ID 1: MER11a insertion within 3' LTR, up to polyA site (10839-11736) |
| 9 | SEQ ID 7 + SEQ ID 8 |
| 10 | Fragment of SEQ ID 1, from transcription start site to poly-A signal |
| 11 | Four 3' nucleotides of SEQ ID 2 + four 5' nucleotides of SEQ ID 3 |
| 12 | Four 3' nucleotides of SEQ ID 2 + four 5' nucleotides of SEQ ID 4 |
| 13 | Four 3' nucleotides of SEQ ID 7 + four 5' nucleotides of SEQ ID 8 |
| 14 | 27378 |
| 15 | 34058 |
| 16 | 26254 |
| 17 | Contig AP000345 |
| 18 | Contig AP000346 |
| 19 | cDNA sequence SP MDA#6 x SP6 rev |
| 20-22 | RACE primers |
| 23 | mRNA form of SEQ ID 10 |
| 24 | mRNA form of SEQ ID 5 |
| 25 | mRNA form of SEQ ID 6 |
| 26 | mRNA form of SEQ ID 2 |
| 27 | mRNA form of SEQ ID 3 |
| 28 | mRNA form of SEQ ID 4 |
| 29 | mRNA form of SEQ ID 9 |
| 30 | mRNA form of SEQ ID 7 |
| 31 | mRNA form of SEQ ID 8 |
| 32 | The alu interruption of env (9938-10244 of SEQ ID 1) |
| 33 | The 10 nucleotides upstream of SEQ ID 32 in SEQ ID 1 |
| 34 | The 10 nucleotides downstream of SEQ ID 32 in SEQ ID 1 |
| 35 | First 10 nucleotides of SEQ ID 32 |
| 36 | SEQ ID 33 + SEQ ID 35 |
| 37 | The 100 nucleotides upstream of SEQ ID 32 in SEQ ID 1 |
| 38 | SEQ ID 37 + SEQ ID 32 |
| 39 | Four 3' nucleotides of SEQ ID 37 + four 5' nucleotides of SEQ ID 32 |
| 40 | The 100 nucleotides downstream of SEQ ID 32 in SEQ ID 1 |
| 41 | Last 10 nucleotides of SEQ ID 32 |
| 42 | SEQ ID 41 + SEQ ID 40 |
| 43 | SEQ ID 32 + SEQ ID 40 |
| 44 | Four 3' nucleotides of SEQ ID 32 + four 5' nucleotides of SEQ ID 40 |
| 45 | Ten 3' nucleotides of SEQ ID 32 + ten 5' nucleotides of SEQ ID 40 |
| 46 | Fragment of SEQ ID 1, following a splice acceptor site within second 5' LTR (2611-2710) |
| 47 | SEQ ID 2 + SEQ ID 46 |
| 48 | Fragment of SEQ ID 1, following a splice acceptor site downstream of second 5' LTR (2700-2799) |
| 49 | SEQ ID 2 + SEQ ID 48 |
| 50 Ten | 3' nucleotides of SEQ ID 2 + SEQ ID 3 |
| 51 | Ten 3' nucleotides of SEQ ID 2 + SEQ ID 4 |
| 52 | Ten 3' nucleotides of SEQ ID 7 + ten 5' nucleotides of SED ID 8 |
| 53 | Gag nucleotide sequence unique to PCAV |
| 54 | PCAV gag |
| 55 | Cag fragment of SEQ ID 54 |
| 56 | Gag fragment of SEQ ID 54 |
| 57 | Gag (encodes SEQ ID 54) |
| 58 | Prt |
| 59-62 | Prt amino acid fragments |
| 63 | Env |
| 64-80 | Env amino acid fragments |
| 81 | Env |
| 82-85 | Env amino acid fragments |
| 86 | Pol |
| 87 | PCAP3 amino acid sequence |
| 88 | PCAP3 gene (spliced) |
| 89 | MDARU3#1xT7rev |
| 90 | MDARU3#2xSP6REV |
| 91 | MDARU3#4xSP6rev |
| 92-97 | Pol amino acid fragment |
| 98 | Variant of SEQ ID 87 |
| 99-109 | Sequences of spliced cDNAs |
| 110 | Amino acids encoded by SEQ ID 53 |
| 111 | Nucleotides encoding SEQ ID 55 |
| 112 | Nucleotides encoding SEQ ID 56 |
| 113-119 | Hybridizing sequences with homology to chromosome 22 |
| 120-599 | 25mer PCAV fragments |
| 600-1184 | 25mer PCAV fragments with good predicted Tm values |
| 1185 | "New" gag construct |
| 1186 | "New" gag protein |
| 1187 | "Hybrid" gag construct |
| 1188 | "Hybrid" gag protein |
| 1189 | V5 tag |
| 1190 | HML-2 LTR |
| 1191 | cDNA sequence encoding PCAP3 |
| 1192-95 | PCAV-specific primers |
| 1196 | MDALTR |
| 1197 | SEQ ID 23 excluding its 77 5' nucleotides |
| 1198 | SEQ ID 23 excluding its 100 5' nucleotides |
| 1199 | SEQ ID 24 excluding its 77 5' nucleotides |
| 1200 | SEQ ID 25 excluding its 77 5' nucleotides |
| 1201 | SEQ ID 26 excluding its 77 5' nucleotides |
| 1202-08 | Oligonucleotides used during RT-PCR mapping of transcription start site |

### REFERENCES (the contents of which are hereby incorporated in full by reference)

{1} International patent application WO02/46477 (PCT/US01/47824. filed December 7,2001).
{2} US patent application 10/016,604 (filed December 7, 2001).
{3} Reus et al. (2001) J. Virol. 75:8917-8926.
{4} Dunham et al. (1999) Nature 402:489-495.
{5} Prediger (2001) Methods Mol Biol160:49-63.
{6} Bustin (2000) J. Mol. Endocrinol. 25:169-193.
{7} Gene Cloning and Analysis by RT-PCR (eds. Siebert et al.) ISBN: 1881299147.
{8} RT-PCR Protocols (ed. O'Connell) ISBN: 0896038750.
{9} The PCR Technique: RT-PCR (ed. Siebert) ISBN: 1881299139.
{10} Thaker (1999) Methods Mol Biol 115:379-402.
{11} Seiden & Sklar (1996) Important Adv Oncol 191-204.
{12} Hagen-Mann & Mann (1995) Exp Clin Endocrinol Diabetes 103:150-155.
{13} Clementi et al. (1993) PCR Methods Appl 2:191-196.
{14} Robbins et al. (1997) Clin Lab Sci 10(5):265-71.
{15} de la Taille (1999) Prog Urol 9:1084-1089.
{16} Ylikoski et al. (1999) Clin Chem 45(9):1397-1407.
{17} Yao et al. (1996) Cancer Treat Res 88:77-91*.*
{18} Ylikoski et al. (2001) Biotechniques 30:832-840
{19} Shirahata & Pegg (1986) J. Biol. Chem. 261(29):13833-7.
{20} RNA Methodologies (Farrell, 1998) (Academic Press; ISBN 0-12-249695-7).
{21} Sambrook et al. (1989) Molecular Cloning: A Laboratory Manual. NY, Cold Spring Harbor Laboratory
{22} Yang et al. (1999) Proc Natl Acad Sci U S A 96(23):13404-8
{23} Short protocols in molecular biology (4th edition, 1999) Ausubel et al. eds. ISBN 0-471-32938-X.
{24} US patent 5,707,829
{25} Fille et al. (1997) Biotechniques 23:34-36.
{26} EP-B-0509612
{27} EP-B-0505012
{28} Current Protocols in Molecular Biology (F.M. Ausubel et al. eds., 1987) Supplement 30.
{29} International patent application WO00/73801
{30} International patent application WO01/51633
{31} International patent application WO01/73032
{32} US patent application 20020022248.
{33} International patent application WO01/57270.
{34} International patent application WO01/75067.
{35} International patent application WO01/57182.
{36} International patent application WO01/57277.
{37} International patent application WO01/57274.
{38} International patent application WO01/57275.
{39} International patent application WO01/57276.
{40} International patent application WO01/57278.
{41} International patent application WO01/57272.
{42} International patent application WO01/42467.
{43} European patent application EP-A-1074617.
{44} Mayer et al. (1999) Nat. Genet. 21 (3), 257-258
{45} Löwer et al. (1996) Proc. Natl. Acad. Sci USA 93:5177
{46} Berkhout et al. (1999) J. Virol. 73:2365-2375.
{47} Löwer et al. (1995) J. Virol. 69:141-149.
{48} Magin et al. (1999) J. Viral. 73:9496-9507.
{49} Magin et al. (2000) Virology 274:11-16.
{50} Boese et al. (2001) FEBS Lett 493(2-3):117-21.
{51} Mueller- Lantzsch et al. AIDS Research and Human Retroviruses 9:343-350 (1993)
{52} Hasbido et al. (1992) Biochem. Biophys. Res. Comm. 187:1241-1248.
{53} Vogetseder et al. (1995) Exp Clin Immunogenet. 12:96-102.
{54} Sauter et al. (1995) J. Virol. 69:414-421.
{55} Geysen et al. (1984) PNAS USA 81:3998-4002.
{56} Carter (1994) Methods Mol Biol 36:207-23.
{57} Jameson, BA et al. 1988, CABIOS 4(1):181-186.
{58} Raddrizzani & Hammer (2000) Brief Bioinform 1(2):179-89.
{59} De Lalla et al. (1999) J. Immunol. 163:1725-29.
{60} Brusic et al. (1998) Bioinformatics 14(2):121-30
{61} Moister et al. (1995) Vaccine 13(6):581-91.
{62} Roberts et al. (1996) AIDS Res Hum Retroviruses 12(7):593-610.
{63} Maksyatov & Zagrebelnaya (1993) Comput Appl Biosci 9(3):291-7.
{64} Feller & de la Cruz (1991) Nature 349(6311):720-1_{.}
{65} Hopp (1993) Peptide Research 6:183-190.
{66} Welling et al. (1985) FEBS Lett. 188:215-218.
{67} Davenport et al. (1995) Immunogenetics 42:392-297.
{68} Go et al. (1980) Int. J. Peptide Protein Res. 15:211
{69} Querol et al. (1996) Prot. Eng. 9:265
{70} Olsen & Thomsen (1991) J. Gen. Microbiol. 137:579
{71} Clarke et al. (1993) Biochemistry 32:4322
{72} Wakarchuk et al. (1994) Protein Eng. 7:1379
{73} Toma et al. (1991) Biochemistry 30:97
{74} Haezerbrouck et al. (1993) Protein Eng. 6:643
{75} Masul et al. (1994) Appl. Env. Microbiol. (1994) 60:3579
{76} US patent 4,959,314
{77} Smith & Waterman (1981) Adv. Appl. Math. 2: 482-489.
{78} Breedveld (2000) Lancet 355(9205):735-740.
{79} Gorman & Clark (1990) Semin. Immunol. 2:457-466
{80} Jones et al. (1986) Nature 321:522-525.
{81} Morrison et al. (1984) Proc. Natl. Acad. Sci, US.A., 81:6851-6855.
{82} Morrison & Oi (1988) Adv. Immunol., 44:65-92.
{83} Verhoeyer et al. (1988) Science 239:1534-1536.
{84} Padlan (1991) Molec. Immun. 28:489-498.
{85} Padlan (1994) Molec. Immunol. 31(3):169-217.
{86} Kettleborough et al. (1991) Protein Eng. 4(7):773-83
{87} Chothia et al.(1987) J. Mol. Biol. 196:901-917.
{88} Kabat *et al.* U.S. Dept. of Health and Human Services NIH Publication No. 91-3242 (1991)
{89} WO 98/24893
{90} WO 91/10741
{91} WO 96/30498
{92} WO 94/02602
{93} US Patent 5,939,598.
{94} WO 96/33735
{95} Gennaro (2000) Remington: The Science and Practice of Pharmacy. 20th edition, ISBN: 0683306472.
{96} WO 93/14778
{97} Findeis et al. (1993) Trends Biotechnol. 11:202
{98} Chiou et al. (1994) Gene Therapeutics: Methods And Applications Of Direct Gene Transfer. ed. Wolff
{99} Wu et al. (1988), J. Biol. Chem. 263:621
{100} Wu et al. (1994) J. Biol. Chem. 269:542
{101} Zenke et al. (1998) Proc. Natl. Acad. Sci. (USA) 87:3655
{102} Wu et al. (1991) J. Biol. Chem. 266:338.
{103} Jolly (1994) Cancer Gene Therapy 1:51.
{104} Kimura (1994) Human Gene Therapy 5:845
{105} Connelly (1995) Human Gene Therapy 1:185
{106} Kaplitt (1994) Nature Genetics 6:148
{107} WO 90/07936
{108} WO 94/03622
{109} WO 93/25698
{110} WO 93/25234
{111} US patent 5,219,740
{112} WO 93/11230
{113} WO 93/10218
{114} US patent 4,777,127
{115} GB Patent No. 2,200,651
{116} EP-A~ 0 345 242
{117} WO 91/02805
{118} WO 94/12649
{119} WO 93/03769
{120} WO 93/19191
{121} WO 94/28938
{122} WO 95/11984
{123} WO 95/00655
{124} Curiel (1992) Hum. Gene Ther. 3:147
{125} Wu, (1989) J. Biol. Chem. 264:16985
{126} US patent 5,814,482
{127} WO 95/07994
{128} WO 96/17072
{129} WO 95/30763
{130} WO 97/42338
{131} WO 90/11092
{132} US patent 5,580,859
{133} US patent 5,422,120
{134} WO 95/13796
{135} WO 94/23697
{136} WO 91/14445
{137} EP 0524968
{138} Philip (1994) Mol. Cell Biol. 14:2411
{139} Woffendin(1994) Proc. Natl. Acad. Sci. USA 91:11581
{140} US patent 5,206,152
{141} WO 92/11033
{142} US patent 5,149,655
{143} WO90/14837
{144} Vaccine Design - the subunit and adjuvant approach (1995) eds. Powell & Newman. ASIN: 030644867X
{145} WO00/07621
{146} GB-2220221
{147} EP-A-0689454
{148} EP-A-0835318
{149} EP-A-0735898
{150} EP-A-0761231
{151} WO99/52549
{152} WO01/21207
{153} WO01/21152
{154} WO00/62800
{155} WO00/23105
{156} WO99/11241
{157} WO98/57659
{158} WO93/13202.
{159} McSharry (1999) Antiviral Res 43(1):1-21.
{160} Weissman (1987) Mol Biol. Med. 4(3):133-143
{161} Patanjali et al. (1991) Proc. Natl. Acad. Sci. USA 88: 1943-1947
{162} Simone et al. (2000) Am J Pathol. 156(2):445-52.
{163} Claverie (1996) Meth. Enzymol. 266:212-227.
{164} Chapter 36 (page 267ff) of Automated DNA Sequencing and Analysis Techniques (eds. Adams et al.) ISBN: 0127170103.
{165} Claverie et al. (1993) Comput, Chem. 17:191
{166} Altschul et al. (1990), J. Mol. Biol. 215:403-410.
{167} Pearson & Lipman (1988) PNAS USA, 85:2444.
{168} Luo et al. (1999) Nature Med 5:117-122.
{169} Sedivy (1998) Proc Natl Acad Sci U S A 95:9078-9081.
{170} Hahn et al. (2002) Mol Cell Biol. 22(7):2111~2123.
{171} Hahn et al. (1999) Nature 400(6743):464-468.
{172} De Marzo et al. (1998) J Urol. 160:2381-2392.
{173} Sakr & Partin (2001) Urology 57(4 Suppl 1):115∼120.

SEQUENCE LISTING
SEQ ID 1
TGTGGGGAAAAGAAAGAGAGATCAGACTGTTACTGTGTCTATGTAGAAAGAAATAGACATAAGAGACTCCATTTTGTTCTGTACTAAGA
AAAATTCTTCTGCTTTGAGATGCTGTTAATCTGTAACCCTAGCCCCAACCCTGTGCTCACAGAAACAGGTGCTGTGTTGACTCAAGGTT
TAATGGATTCAGGGCTGTGCAGGATGTGCTTTGTTAAACAAATGCTTGAAGGCAGCAAGCTTGTTAAGAGTCATCACCACTCCCTAATC
TCAAGTAAGCAGGGACACAAACACTGCGGAAGGCCGCAGGGACCTCTGCCTAGGAAAGCCAGGTGTTGTCCAAGGTTTCTCCCCATGTG
ACAGTCTGAAATATGGCCTCTTGGGAAGGGAAAGACCTGACTGTCCCCTGGCCCGACACCCGTAAAGGGTCTGTGCTGAGGATTAGTAA
AAGAGGAAGGAAGGCCTCTTTGCAGTTGAGATAAGAGGAAGGCATCTGTCTCCTGCTCATCCCTGGGCAATGGAATGTCTTGGTGTAAA
GCCTGATTGTATATGCCATCTACTGAGATAGGAGAAAACTGCCTTAGGGCTGGAGGTGGGACATGCTGGCGGCAATACTGCTCTTTAAG
GCATTGAGATGTTTATGTATATGCACATCAAAAGCACAGCACTTTTTTCTTTACCTTGTTTATGATGCAGAGACATTTGTTCACATGTT
TTCCTGCTGGCCCTCTCCCCACTATTACCCTATTGTCCTGCCACATCCCCCTCTCCGAGATGGTAGAGATAATGATCAATAAATACTGA
GGGAACTCAGAGACCGGTGCGGCGCGGGTCCTCCATATGCTGAGCGCCGGTCCCCTGGGCCCACTTTTCTTTCTCTATACTTTGTCTCT
GTTGTCTTTCTTTTCTCAAGTCTCTCGTTCCACCTGAGGAGAAATGCCCACAGCTGTGGAGGCGCAGGCCACTCCATCTGGTGCCCAAC
GTGGATGCTTTTCTCTAGGGTGAAGGGACTCTCGAGTGTGGTCATTGAGGACAAGTCAACGAGAGATTCCCGAGTACGTCTACAGTGAG
CCTTGTGGTAAGCTTGGGCGCTCGGAAGAAGCCAGGGTTAATGGGGCAAACTAAAAGTAAAGTCTCTCATTCCACCTGATGAGAAACAC
CCAGAGGTGTGGAGGGGCAGGCCACCCCTTCAGGGTAGGGTCCCCTCCATGCAGACCATAGAGCACAGGTGTGCCCCAAAGAGGAGCAG
AGAGAAGGAGGGAGAGGGCCCACGAGAGACTTGGAAATGAATGGCAGGATTTTAGGCGCTGGACTTGGGTTCGGGGCACCTGGCCTTTC
CTTGTGTATTTCTCCTACTGTCTGCCTAACTATTTAATACAATAAAAGAAAACCAGCCCCTGGTTCTTGTGGTGTTTCCACCCTCCCGG
GTCCCCGCTGGCTGCCTGGCTTCCTCCCGCAGCTCCTGCTGTGTGTGTATGTGTGTGTGTGTGCACATCTGTGGGGCGTATGTGTGTTC
GTCTTTGTAATTGAGGCTGCAGAGTGGAGAGAGCAGGGGTTTCTCTGGGGACCCAGAGAGAAGGAGGCGTTTTCACCACAGCCGAACA
GGGCAGGACCCCAGCACCCGGGACCCAGCGGGACTTTGCCAAGGGGATGGACCTGGCTGGGCCACGCGGCTGTTTGTGTAGGGAAAAGA
AAGAGAGATCACACTGTTACTGTGTCTATGTAGAAAAGGAAGACATAAACTCCATTTTGAGCTGTACTAAGAAAAATTATTTTGCCTTG
ACCTGCTGTTAACCTGTAACTGTAGCCCCAACCCTGTGCTCAAAGAAACATGTGCTGTATGGAATCAAGGTTTAAGGGATCAAGGGCTG
TACAGGATGTGCCTTGTTAACAATGTGTTTACAGGCAGTATGCTTGGTAAAAGTCATCGCCATTCTCCATTCTCCATTAATCAGGGGCA
CGATGCACTGCGGAAAGCCACAGGGACCTCTGCCCGAGAAAGCCTGGGTATTGTCCAAGGCTTCCCCCCACTGAGACAGCCTGAGATAC
GGCCTCGTGGGAAGGGAAAGACCTGACCGTCCCCCAGCCCGACACCCGTAAAGGGTCTGTGCTGAGGAGGATTAGTAAAAGGGGAAGGC
CTCTTGCAGTTGAGATAAGAGGAAGGCCTCCGTCTCCTGCATGTCCTTGGGAATGGAATGTCTTGGTGTAAAACCCGATAGTACATTCC
TTCTATTCTGAGAGAAGAAAACCACCCTGTGGCTGGAGGTGAGATATGCTAGCGGCAATGCTGCTCTGTTACTCTTTGCTACACTGAGA
TGTTTGGGTGGAGAGAAGCATAAATCTGGCCTATGTGCACATCTGGGCACAGAACCTCCCCTTGAACTTGTGACACAGATTCCTTTGTT
CACATGTTTTCCTGCTGACCTTCTCCCCACTATCGCCCTGTTCTCCCACCGCATTCCCCTTGCTGAGATAGTGAAAATAGTAATCTGTA
GATACCAAGGGAACTCAGAGACCATGGCCGGTGCACATCCTCCGTACGCTGAGCGCTGGTCCCCTGGGCCCATTGTTCTTTCTCTATAC
TTTGTCTCTGTGTCTTATTTCTTTCCTCAGTCTCTCATCCCTCCTGACGAGAAATACCCACAGGTGTGGAGGGGCTGGCCCCCTTCATC
TGATGCCCAATGTGGGTGCCTTTCTCTAGGGTGAAGGTACTCTACAGTGTGGTCATTGAGGACAAGTTGACGAGAGAGTCCCAAGTACG
TCCACGGTCAGCCTTGCGGTAAGCTTGTGTGCTTAGAGGAACCCAGGGTAACGATGGGGCAAACTGAAAGTAAATATGCCTCTTATCTC
AGCTTTATTAAAATTCTTTTAAGAAGAGGGGGAGTTAGAGCTTCTACAGAAAATCTAATTACGCTATTTCAAACAATAGAACAATTCTG
CCCATGGTTTCCAGAACAGGGAACTTTAGATCTAAAAGATTGGAAAAAATTGGCAAAGAATTAAAACAAGCAAATAGGGAAGGTAAAA
TCATCCCACTTACAGTATGGAATGATTGGGCCATTATTAAAGCAACTTTAGAACCATTTCAAACAGGAGAAGATATTGTTTCAGTTTCT
GATGCCCCTAAAAGCTGTGTAACAGATTGTGAAGAAGAGGCAGGGACAGAATCCCAGCAAGGAACGGAAAGTTCACATTGTAAATATGT
AGCAGAGTCTGTAATGGCTCAGTCAACGCAAAATGTTGACTACAGTCAATTACAGGAGATAATATACCCTGAATCATCAAAATTGGGGG
AAGGAGGTCCAGAATCATTGGGGCCATCAGAGCCTAAACCACGATCGCCATCAACTCCTCCTCCCGTGGTTCAGATGCCTGTAACATTA
CAACCTCAAACGCAGGTTAGACAAGCACAAACCCCAAGAGAAAATCAAGTAGAAAGGGACAGAGTCTCTATCCCGGCAATGCCAACTCA
GATACAGTATCCACAATATCAGCCGGTAGAAAATAAGACCCAACCGCTGGTAGTTTATCAATACCGGCTGCCAACCGAGCTTCAGTATC
GGCCTCCTTCAGAGGTTCAATACAGACCTCAAGCGGTGTGTCCTGTGCCAAATAGCACGGCACCATACCAGCAACCCACAGCGATGGCG
TCTAATTCACCAGCAACACAGGACGCGGCGCTGTATCCTCAGCCGCCCACTGTGAGACTTAATCCTACAGCATCACGTAGTGGACAGGG
TGGTGCACTGCATGCAGTCATTGATGAAGCCAGAAAACAGGGCGATCTTGAGGCATGGCGGTTCCTGGTAATTTTACAACTGGTACAGG
CCGGGGAAGAGACTCAAGTAGGAGCGCCTGCCCGAGCTGAGACTAGATGTGAACCTTTCACCATGAAAATGTTAAAAGATATAAAGGAA
GGAGTTAAACAATATGGATCCAACTCCCCTTATATAAGAACATTATTAGATTCCATTGCTCATGGAAATAGACTTACTCCTTATGACTG
GGAAATTTTGGCCAAATCTTCCCTTTCATCCTCTCAGTATCTACAGTTTAAAACCTGGTGGATTGATGGAGTACAAGAACAGGTACGAA
AAAATCAGGCTACTAAGCCCACTGTTAATATAGACGCAGACCAATTGTTAGGAACAGGTCCAAATTGGAGCACCATTAACCAACAATCA
GTGATGCAGAATGAGGCTATTGAACAAGTAAGGGCTATTTGCCTCAGGGCCTGGGAAAAAATTCAGGACCCAGGAACAGCTTTCCCTAT
TAATTCAATTAGACAAGGCTCTAAAGAGCCATATCCTGACTTTGTGGCAAGATTACAAGATGCTGCTCAAAAGTCTATTACAGATGACA
ATGCCCGAAAAGTTATTGTAGAATTAATGGCCTATGAAAATGCAAATCCAGAATGTCAGTCGGCCATAAAGCCATTAAAAGGAAAAGTT

CCAGCAGGAGTTGATGTAATTACAGAATATGTGAAGGCTTGTGATGGGATTGGAGGAGCTATGCATAAGGCAATGCTAATGGCTCAAGC
AATGAGGGGGCTCACTCTAGGAGGACAAGTTAGAACATTTGGGAAAAAATGTTATAATTGTGGTCAAATCGGTCATCTGAAAAGGAGTT
GCCCAGGCTTAAATAAACAGAATATAATAAATCAAGCTATTACAGCAAAAAATAAAAAGCCATCTGGCCTGTGTCCAAAATGTGGAAAA
GCAAAACATTGGGCCAATCAATGTCATTCTAAATTTGATAAAGATGGGCAACCATTGTCTGGAAACAGGAAGAGGGGCCAGCCTCAGGC
CCCCCAACAAACTGGGGCATTCCCAGTTAAACTGTTTGTTCCTCAGGGTTTTCAAGGACAACAACCCCTACAGAAAATACCACCACTTC
AGGGAGTCAGCCAATTACAACAATCCAACAGCTGTCCCGCGCCACAGCAGGCAGCACCGCAGTAGATTTATGTTCCACCCAAATGGTCT
TTTTACTCCCTGGAAAGCCCCCACAAAAGATTCCTAGAGGGGTATATGGCCCGCTGCCAGAAGGGAGGGTAGGCCTTTGAGGGAGATCA
AGTCTAAATTTGAAGGGAGTCCAAATTCATACTGGGGTAATTTATTCAGATTATAAAGGGGGAATTCAGTTAGTGATCAGCTCCACTGT
TCCCCGGAGTGCCAATCCAGGTGATAGAATTGCTCAATTACTGCTTTTGCCTTATGTTAAAATTGGGGAAAACAAAAAGGAAAGAACAG
GAGGGTTTGGAAGTACCAACCCTGCAGGAAAAGCTGCTTATTGGGCTAATCAGGTCTCAGAGGATAGACCCGTGTGTACAGTCACTATT
CAGGGAAAGAGTTTGAAGGATTAGTGGATACCCAGGCTGATGTTTCTGTCATCGGCATAGGTACTGCCTCAGAAGTGTATCAAAGTGCC
ATGATTTTACATTGTCCAGGATCTGATAATCAAGAAAGTACGGTTCAGCCTGTGATCACTTCATTCCAATCAATTTATGGGGCCGAGAC
TTGTTACAACAATGGCATGCAGAGATTACTATCCCAGCCTCCCTATACAGCCCCAGGAATAAAAAAATCATGACTAAAATGGGATAGCT
CCCTAAAAAGGGACTAGGAAAGAAGTCCCAATTGAGGCTGAAAAAAATCAAAAAAGAAAAGGAATAGGGCATCCTTTTTAGGAGCGGTC
ACTGTAGAGCCTCCAAAACCCATTCCATTAACTTGGGGGAAAAAAAAACAACTGTATGGTAAATCAGCAGCGCTTCCAAAACAAAAACT
GGAGGCTTTACATTTATTAGCAAAGAAACAATTAGAAAAAGGACATTGAGCCTTCATTTTCGCCTTGGAATTCTGTTTGTAATTCAGAA
AAAATCCGGCAGATGGCGTATAATGCCGTAATTCAACCCATGGGGGCTCTCCCACCCCGGTTGCCCTCTCCAGCCATGGTCCCCTTTAA
TTATAATTGATCTGAAGGATTGCTTTTTTACCATTCCTCTGGCAAAACAGGATTTTGAAAAATTTGCTTTTACCACACCAGCCTAAATA
ATAAAGAACCAGCCACCAGGTTTCAGTGGAAAGTATTGCCTCAGGGAATGCTTAATAGTTCAACTATTTGTCAGCTCAAGCTCTGCAAC
CAGTTAGAGACAAGTTTTCAGACTGTTACATCGTTCACTATGTTGATATTTTGTGTGCTGCAGAAACGAGAGACAAATTAATTGACCGT
TACACATTTCTGCAGACAGAGGTTGCCAACGCGGGACTGACAATAACATCTGATAAGATTCAAACCTCTACTCCTTTCCGTTACTTGGG
AATGCAGGTAGAGGAAAGGAAAATTAAACCACAAAAAATAGAAATAAGAAAAGACACATTAAAAGCATTAAATGAGTTTCAAAAGTTGC
TAGGAGATACTAATTGGATTTGGAGATATTAATTGGATTTGGCCAACTCTAGGCATTCCTACTTATGCCATGTCAAATTTGTTCTCTTT
CTTAAGAGGGGACTCGGAATTAAATAGTGAAAGAACGTTAACTCCAGAGGCAACTAAAGAAATTAAATTAATTGAAGAAAAAATTCGGT
CAGCACAAGTAAATAGAATAGATCACTTGGCCCCACTCCAAATTTTGATTTTTGCTACTGCACATTCCCTAACAGGCATCATTGTTCAA
AATACAGATCTTGTGGAGTGGTCCTTCCTTCCTCACAGTACAATTAAGACTTTTACATTGTACTTGGATCAAATGGCTACATTAATTGG
TCAGGGAAGATTATGAATAATAACATTGTGTGGAAATGACCCAGATAAAATCACTGTTCCTTTCAACAAGCAACAGGTTAGACAAGCCT
TTATCAATTCTGGTGCATGGCAGATTGGTCTTGCCGATTTTGTGGGAATTATTGACAATCGTTACCCCAAAACAAAAATCTTCCAGTTT
TTAAAATTGACTACTTGGATTTTACCTAAAGTTACCAAACATAAGCCTTTAAAAAATGCTCTGGCAGTGTTTACTGATGGTTCCAGCAA
TGGAAAAGTGGCTTACACCGGGCCAAAAGAATGAGTCATCAAAACTCAGTATCACTTGACTCAAAGAGCAGAGTTGGTTGCCGTCATTA
CAGTGTTAACAAGATTTTAATCAGTCTATTAACATTGTATCAGATTCTGCATATGTAGTACAGGCTACAAAGGATATTGAGAGAGCCCT
AATCAAATACATTATGGATGATCAGTTAAACCCGCTGTTTAATTTGTTACAACAAAATGTAAGAAAAAGAAATTTCCCATTTTATATTA
CTCATATTCGAGCACACACTAATTTACCAGGGCCTTTAACTAAAGCAAATGAACAAGCTGACTTGCTAGTATCATCTGCATTCATGGAA
GCACAAGAACTTCATGCCTTGACTCATGTAAATGCAATAGGATTAAAAAATAAATTTGATATCACATGGAAACAGACAAAAAATATTGT
ACAACATTGCACCCAGTGTCAGATTCTACACCTGGCCACTCAGGAGGCAAGAGTTAATCCCAGAGGTCTATGTCCTAATGTGTTATGGC
AAATGGATGTCATGCACGTACCTTCATTTGGAAAATTGTCATTTGTCCATGTGACAGTTGATACTTATTCACATTTCATATGGGCAACC
TGCCAGACAGGAGAAAGTACTTCCCATGTTAAAAGACATTTATTATCTTGTTTTCCTGTCATGGGAGTTCCAGAAAAAGTTAAAACAGA
CAATGGGCCAGGTTACTGTAGTAAAGCAGTTCAAAAATTCTTAAATCAGTGGAAAATTACACATACAATAGGAATTCCTTATAATTCCC
AAGGACAGGCCATAATTGAAAGAACTAATAGAACACTCAAAGCTCAATTGGTTAAACAAAAAAAAGGAAAAGACAGGAGTATAACACTC
CCCAGATGCAACTTAATCTAGCACTCTATACTTTAAATGTTTTAAACATTTATAGAAATCAGACCACTACCTCTGCAGAACAACATCTT
ACTGGTAAAAGGAACAGCCCACATGAAGGAAAACTGATTTGGTGGAAAGATAATAAAAATAAAACATGGGAAATGGGGAAGGTGATAAC
GTGGGGGAGAGGTTTTGCTTGTGTTTCACCAGGAGAAAATCAGCTTCCTGTTTGGATACCCACTAGACATTTAAAGTTCTACAATGAAC
TCACTGGAGATGCAAAGAAAAGTGTGGAGATGGAGACACCCCAATCGACTCGCCAGGTAAACAAAATGGTGATATCAGAAGAACAGAAA
AAGTTGCCTTCCATCAAGGAAGCAGAGTTGCCAATATAGGCACAATTAAAGAAGCTGACACAGTTAGCTAAAAAAAAAAGCCTAGAGAA
TACAAAGGTGACACCAACTCCAGAGAATATGCTGCTTGCAGCTCTGATGATTGTATCAACGGTGGTAAGTCTTCCCAAGTCTGCAGGAG
CAGCTGCAGCTAATTATACTTACTGGGCCTATGTGCCTTTCCCACCCTTAATTCGGGCAGTTACATAGATGGATAATCCTATTGAAGTA
GATGTTAATAATAGTGCATGGGTGCCTGGCCCCACAGATGACTGTTGCCCTGCCCAACCTGAAGAAGGAATGATGATGAATATTTCCAT
TGGGTATCCTTATCCTCCTGTTTGCCTAGGGAAGGCACCAGGATGCTTAATGCCTACAACCCAAAATTGGTTGGTAGAAGTACCTACAG
TCAGTGCTACCAGTAGATTTACTTATCACATGGTAAGTGGAATGTCACAGATAAATAATTTACAGGACCCTTCTTATCAAAGATCATTA
CAATGTAGGCCTAAGGGGAAGGCTTGCCCCAAGGAAATTCCCAAAGAATCAAAAAGCCCAGAAGTCTTAGTCTGCGGAGAATGTGTGGC
TGATACTGCAGTGTAGTACAAAACAATGAATTTTGAACTATGATAGACTGGGTCCCTTGAGGCCAATTATATCATAACTGTACAGGCCA
GACTCATTCATGTTCACAGGCCCCATCCATCTGGCCCATTAATCCAGCCTATGACGGTGATGTAACTGAAAGGCTGGACCAGGTTTATA
GAAGGTTAGAATCACTCTGTCCAAGGAAATGGGGTGAAAAGGGAATTTCATCACCTTGACCAAAGTTAGTCCTGTTACTGGTCCTGAAC

ATCCAGAATTAGGAAGCTTACTGTGGCCTCACACCACATTAGAATTTGTTCTGGAAATCAAGCTATAGGAACAAGAGATCGTAAGTCAT
ATTATACTATCAACCTAAATTCCAGTCTGACAATTCCTTTGCAAAATTGTGTAAAACTCCCTTATATTGCTAGTTGTAGGAAAAACATA
GTTATTAAACCTGATTCCCAAACCATAATCTGTGAAAATTGTGGAATGTTTACTTGCATTGATTTGACTTTTAATTGGCAGCACCGTAT
TCTACTAGGAAGAGCAAGAGAGGGTGTGTGGATCCTTGTGTCCATGGACCGACCATGGGAGGCTTCGCTATCCATCCATATTTTAACGG
AAGTATTAAAAGGAATTCTAACTAGATCCAAAAGATTCATTTTTACTTTGATGGCAGTGATTATGGCCTCATTGCAGTCACAGCTACT
GCTGCGGCTGCTGGAATTGCTTTACACTCCTCTGTTCAAACTGCAGAATACGTAAATGATTGGCAAAAGAATTCCTCAAAATTGTGGAA
TTCTCAGATCCAAATAGATCAAAAATTGGCAAACCAAATTAATGATCTTAGACAAACTGTCATTTGGATGGAGAGGCTCATGAGCTTG
GAATATCTTTTTCAGTTACGATGTGACTGGAATACATCAGATTTTTGTGTTACACCACAAGCCTATAATGAGTCTGAGCATCACTGGGA
CATGGTTAGATGCCATCTGCAAGGAGGAGAAGATAATCTTACTTTAGACATTTCAAAATTAAAAGAATTTTTTTTTCTTTGAGACAGA
GTCTCGCTCTGTCGCCCAGGCTGGAGTGCAGTGGCGTGATCTCAGCTCACTGCAAGTTCCGCCTCCTGGGTTTACACCATTCTCCTGCC
TCAGCCTCCCAAGTAGTTGGGACTACAGGAGCCCACCACCATGCCTGGCTAATTTTTTTTGGGTTTTTAATAGAGATGGAGTTTCACCG
TGTTAGCCAGGATGGTCTCGATCTCCTGACCTTGTGATCTGCCCACCTTGGCCTCCCAAAGTGCTGGGATTACAGTCGTGAGCCACCGT
GCCCAGCCAAGAAAAAATTTTTGAGGCATCAAAAGCCCATTTAAATTTGGTGCCAGGAACGGAGACAATCGTGAAAGCTGCTGATAGCC
TCACAAATCTTAAGCCAGTCACTTGGGTTAAAAGCATCAGAAGTTTCACTATTGTAAATTTCATATTAATCCTTGTATGCCTGTTCTGT
CTGTTGTTAGTCTACAGGTGTATCCAGCAGCTCCAAAGAGACAGCAACCAGCAAGAATGGGCCATAGTGACGATGGTGGTTTTGTCAAA
AAGAAAAGGGGGGGATATGTAAGGAAAAGAGAGATCAGACTTTCACTGTGTCTATGTAGAAAAGGAAGACATAAGAAACTCCATTTTGA
TCTGTACTAAGAAAAATTGTTTTGCCTTGAGATGCTGTTAATCTGTAACTTTAGCCCCAACCCTGTGCTCACGGAAACATGTGCTGTAA
GGTTTAAGGGATCTAGGGCTGTGCAGGATGTACCTTGTTAACAATATGTTTGCAGGCAGTATGTTTGGTAAAAGTCATCGCCATTCTCC
ATTCTCGATTAACCAGGGGCTCAATGCACTGTGGAAAGCCACAGGAACCTCTGCCCAAGAAAGCCTGGCTGTTGTGGGAAGTCAGGGAC
CCCGAATGGAGGGACCAGCTGGTGCTGCATCAGGAAACATAAATTGTGAAGATTTCTTGGACATTTATCAGTTTCCAAAATTAATACTT
TTATAATTTCTTACACCTGTCTTACTTTAATCTCTTAATCCTGTTATCTTTGTAAGCTGAGGATATACGTCACCTCAGGACCACTATTG
TACAAATTGATTGTAAAACATGTTCACATGTGTTTGAACAATATGAAATCAGTGCACCTTGAAAATGAACAGAATAACAGTGATTTTAG
GGAACAAAGGAAGACAACCATAAGGTCTGACTGCCTGAGGGGTCGGGCAAAAAGCCATATTTTTCTTCTTGCAGAGAGCCTATAAATGG
ACGTGCAAGTAGGAGAGATATTGCTAAATTCTTTTCCTAGCAAGGAATATAATACTAAGACCCTAGGGAAAGAATTGCATTCCTGGGGG
GAGGTCTATAAACGGCCGCTCTGGGAGTGTCTGTCCTATGTGGTTGAGATAAGGACTGAGATACGCCCTGGTCTCCTGCAGTACCCTCA
GGCTTACTAGGATTGGGAAACCCCAGTCCTGGTAAATTTGAGGTCAGGCCGGTTCTTTGCTCTGAACCCTGTTTTCTGTTAAGATGTTT
ATCAAGACAATACATGCACCGCTGAACATAGACCCTTATCAGGAGTTTCTGATTTTGCTCTGGTCCTGTTTCTTCAGAAGCATGTCATC
TTTGCTCTGCCTTCTGCCCTTTGAAGCATGTGATCTTTGTGACCTACTCCCTGTTCATACACCCCTCCCCTTTTAAAATCCCTAATAAA
AACTTGCTGGTTTTGTGGCTCAGGGGGGCATCATGGACCTACCAATACGTGATGTCACCCCCGGTGGCCCAGCTGTAAAATTCCTTTCT
TTATACTCTTATTTCTCAGACCAGCTGACACTTAGGGAAAATAGAAAGAACCTATGTTGAAATATTGGAGGCGGGTTCCCCCGATACCT
GGGTATTGTCCAAGGTTTCCTTTGCTGAGGAGGATTAGTAAAAGGAATGCCTCCATCTCCTGCATGTCCCTGGAACAGAATGTTCCCA
CCAACCACCCTGTGGCTGGAGGCGGGATATGCTGGCAGCAATGCTGCTCTATTACTCTTTGCTACACTGAGATGTTTGGGTGGAGAGAA
GCATAAATCTGGCCTATGTGCACATCTGGGCACAGCACCTTCCTTTGAACTTATTTGTGACACAGATTCCTTTGCTCACGTTTTCCTGT
TGACTTTCTCACCACTCACCCTATTCTCCTGTGGCATTCGCCTTGCGGAGATAGTGAAAATAGTAATAAATACTGAGGGAACTCAGACT
GAGGGAACTCAGACTGGGCAGACCGGGGCCAGTGTGGGTCCTCCATATGCTGAGCGCCGGTTCCCTGGGCCCACTGTTCTTTCTCTATA
CTTTGTCTCTGTGCCTTATTTTCTCAGTCTCTCATTCCACCTGATGAGAAATACCCACAGGTGTGGAGGGGCTGGCCCCCTTCA
SEQ ID 2
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTG
SEQ ID 3
TCTCTCATCC
SEQ ID 4
GGTGAAGGTA
SEQ ID 5
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC

TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGTCTCTCATCC
SEQ ID 6
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGGGTGAAGGTA
SEQ ID 7
TGTAAGGAAAAGAGAGATCAGACTTTCACTGTGTCTATGTAGAAAAGGAAGACATAAGAAACTCCATTTTGATCTGTACTAAGAAAAAT
TGTTTTGCCTTGAGATGCTGTTAATCTGTAACTTTAGCCCCAACCCTGTGCTCACGGAAACATGTGCTGTAAGGTTTAAGGGATCTAGG
GCTGTGCAGGATGTACCTTGTTAACAATATGTTTGCAGGCAGTATGTTTGGTAAAAGTCATCGCCATTCTCCATTCTCGATTAACCAGG
GGCTCAATGCACTGTGGAAAGCCACAGGAACCTCTGCCCAAGAAAGCCTGGC
SEQ ID 8
TGTTGTGGGAAGTCAGGGACCCCGAATGGAGGGACCAGCTGGTGCTGCATCAGGAAACATAAATTGTGAAGATTTCTTGGACATTTATC
AGTTTCCAAAATTAATACTTTTATAATTTCTTACACCTGTCTTACTTTAATCTCTTAATCCTGTTATCTTTGTAAGCTGAGGATATACG
TCACCTCAGGACCACTATTGTACAAATTGATTGTAAAACATGTTCACATGTGTTTGAACAATATGAAATCAGTGCACCTTGAAAATGAA
CAGAATAACAGTGATTTTAGGGAACAAAGGAAGACAACCATAAGGTCTGACTGCCTGAGGGGTCGGGCAAAAAGCCATATTTTTCTTCT
TGCAGAGAGCCTATAAATGGACGTGCAAGTAGGAGAGATATTGCTAAATTCTTTTCCTAGCAAGGAATATAATACTAAGACCCTAGGGA
AAGAATTGCATTCCTGGGGGGAGGTCTATAAACGGCCGCTCTGGGAGTGTCTGTCCTATGTGGTTGAGATAAGGACTGAGATACGCCCT
GGTCTCCTGCAGTACCCTCAGGCTTACTAGGATTGGGAAACCCCAGTCCTGGTAAATTTGAGGTCAGGCCGGTTCTTTGCTCTGAACCC
TGTTTTCTGTTAAGATGTTTATCAAGACAATACATGCACCGCTGAACATAGACCCTTATCAGGAGTTTCTGATTTTGCTCTGGTCCTGT
TTCTTCAGAAGCATGTCATCTTTGCTCTGCCTTCTGCCCTTTGAAGCATGTGATCTTTGTGACCTACTCCCTGTTCATACACCCCTCCC
CTTTTAAAATCCCTAATAAAAACTTGCTGGTTTTGTGGCTCAGGGGGGCATCATGGACCTACCAATACGTGATGTCACCCCCGGTGGCC
CAGCTGT
SEQ ID 9
TGTAAGGAAAAGAGAGATCAGACTTTCACTGTGTCTATGTAGAAAAGGAAGACATAAGAAACTCCATTTTGATCTGTACTAAGAAAAAT
TGTTTTGCCTTGAGATGCTGTTAATCTGTAACTTTAGCCCCAACCCTGTGCTCACGGAAACATGTGCTGTAAGGTTTAAGGGATCTAGG
GCTGTGCAGGATGTACCTTGTTAACAATATGTTTGCAGGCAGTATGTTTGGTAAAAGTCATCGCCATTCTCCATTCTCGATTAACCAGG
GGCTCAATGCACTGTGGAAAGCCACAGGAACCTCTGCCCAAGAAAGCCTGGCTGTTGTGGGAAGTCAGGGACCCCGAATGGAGGGACCA
GCTGGTGCTGCATCAGGAAACATAAATTGTGAAGATTTCTTGGACATTTATCAGTTTCCAAAATTAATACTTTTATAATTTCTTACACC
TGTCTTACTTTAATCTCTTAATCCTGTTATCTTTGTAAGCTGAGGATATACGTCACCTCAGGACCACTATTGTACAAATTGATTGTAAA
ACATGTTCACATGTGTTTGAACAATATGAAATCAGTGCACCTTGAAAATGAACAGAATAACAGTGATTTTAGGGAACAAAGGAAGACAA
CCATAAGGTCTGACTGCCTGAGGGGTCGGGCAAAAAGCCATATTTTTCTTCTTGCAGAGAGCCTATAAATGGACGTGCAAGTAGGAGAG
ATATTGCTAAATTCTTTTCCTAGCAAGGAATATAATACTAAGACCCTAGGGAAAGAATTGCATTCCTGGGGGGAGGTCTATAAACGGCC
GCTCTGGGAGTGTCTGTCCTATGTGGTTGAGATAAGGACTGAGATACGCCCTGGTCTCCTGCAGTACCCTCAGGCTTACTAGGATTGGG
AAACCCCAGTCCTGGTAAATTTGAGGTCAGGCCGGTTCTTTGCTCTGAACCCTGTTTTCTGTTAAGATGTTTATCAAGACAATACATGC
ACCGCTGAACATAGACCCTTATCAGGAGTTTCTGATTTTGCTCTGGTCCTGTTTCTTCAGAAGCATGTCATCTTTGCTCTGCCTTCTGC
CCTTTGAAGCATGTGATCTTTGTGACCTACTCCCTGTTCATACACCCCTCCCCTTTTAAAATCCCTAATAAAAACTTGCTGGTTTTGTG
GCTCAGGGGGGCATCATGGACCTACCAATACGTGATGTCACCCCCGGTGGCCCAGCTGT
SEQ ID 10
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGGTAAGCTTGGGCGCTCG
GAAGAAGCCAGGGTTAATGGGGCAAACTAAAAGTAAAGTCTCTCATTCCACCTGATGAGAAACACCCAGAGGTGTGGAGGGGCAGGCCA
CCCCTTCAGGGTAGGGTCCCCTCCATGCAGACCATAGAGCACAGGTGTGCCCCAAAGAGGAGCAGAGAGAAGGAGGGAGAGGGCCCACG
AGAGACTTGGAAATGAATGGCAGGATTTTAGGCGCTGGACTTGGGTTCGGGGCACCTGGCCTTTCCTTGTGTATTTCTCCTACTGTCTG
CCTAACTATTTAATACAATAAAAGAAAACCAGCCCCTGGTTCTTCTGGTGTTTCCACCCTCCCGGGTCCCCGCTGGCTGCCTGGCTTCC

TCCCGCAGCTCCTGCTGTGTGTGTATGTGTGTGTGTGTGCACATCTGTGGGGCGTATGTGTGTTCGTCTTTGTAATTGAGGCTGCAGAG
TGGAGAGAGCAGGGGTTTTCTCTGGGGACCCAGAGAGAAGGAGGCGTTTTCACCACAGCCGAACAGGGCAGGACCCCAGCACCCGGGAC
CCAGCGGGACTTTGCCAAGGGGATGGACCTGGCTGGGCCACGCGGCTGTTTGTGTAGGGAAAAGAAAGAGAGATCACACTGTTACTGTG
TCTATGTAGAAAAGGAAGACATAAACTCCATTTTGAGCTGTACTAAGAAAAATTATTTTGCCTTGACCTGCTGTTAACCTGTAACTGTA
GCCCCAACCCTGTGCTCAAAGAAACATGTGCTGTATGGAATCAAGGTTTAAGGGATCAAGGGCTGTACAGGATGTGCCTTGTTAACAAT
GTGTTTACAGGCAGTATGCTTGGTAAAAGTCATCGCCATTCTCCATTCTCCATTAATCAGGGGCACGATGCACTGCGGAAAGCCACAGG
GACCTCTGCCCGAGAAAGCCTGGGTATTGTCCAAGGCTTCCCCCCACTGAGACAGCCTGAGATACGGCCTCGTGGGAAGGGAAAGACCT
GACCGTCCCCCAGCCCGACACCCGTAAAGGGTCTGTGCTGAGGAGGATTAGTAAAAGGGGAAGGCCTCTTGCAGTTGAGATAAGAGGAA
GGCCTCCGTCTCCTGCATGTCCTTGGGAATGGAATGTCTTGGTGTAAAACCCGATAGTACATTCCTTCTATTCTGAGAGAAGAAAACCA
CCCTGTGGCTGGAGGTGAGATATGCTAGCGGCAATGCTGCTCTGTTACTCTTTGCTACACTGAGATGTTTGGGTGGAGAGAAGCATAAA
TCTGGCCTATGTGCACATCTGGGCACAGAACCTCCCCTTGAACTTGTGACACAGATTCCTTTGTTCACATGTTTTCCTGCTGACCTTCT
CCCCACTATCGCCCTGTTCTCCCACCGCATTCCCCTTGCTGAGATAGTGAAAATAGTAATCTGTAGATACCAAGGGAACTCAGAGACCA
TGGCCGGTGCACATCCTCCGTACGCTGAGCGCTGGTCCCCTGGCCCATTGTTCTTTCTCTATACTTTGTCTCTGTGTCTTATTTCTTT
CCTCAGTCTCTCATCCCTCCTGACGAGAAATACCCACAGGTGTGGAGGGGCTGGCCCCCTTCATCTGATGCCCAATGTGGGTGCCTTTC
TCTAGGGTGAAGGTACTCTACAGTGTGGTCATTGAGGACAAGTTGACGAGAGAGTCCCAAGTACGTCCACGGTCAGCCTTGCGGTAAGC
TTGTGTGCTTAGAGGAACCCAGGGTAACGATGGGGCAAACTGAAAGTAAATATGCCTCTTATCTCAGCTTTATTAAAATTCTTTTAAGA
AGAGGGGGAGTTAGAGCTTCTACAGAAAATCTAATTACGCTATTTCAAACAATAGAACAATTCTGCCCATGGTTTCCAGAACAGGGAAC
TTTAGATCTAAAAGATTGGGAAAAAATTGGCAAAGAATTAAAACAAGCAAATAGGGAAGGTAAAATCATCCCACTTACAGTATGGAATG
ATTGGGCCATTATTAAAGCAACTTTAGAACCATTTCAAACAGGAGAAGATATTGTTTCAGTTTCTGATGCCCCTAAAAGCTGTGTAACA
GATTGTGAAGAAGAGGCAGGGACAGAATCCCAGCAAGGAACGGAAAGTTCACATTGTAAATATGTAGCAGAGTCTGTAATGGCTCAGTC
AACGCAAAATGTTGACTACAGTCAATTACAGGAGATAATATACCCTGAATCATCAAAATTGGGGGAAGGAGGTCCAGAATCATTGGGGC
CATCAGAGCCTAAACCACGATCGCCATCAACTCCTCCTCCCGTGGTTCAGATGCCTGTAACATTACAACCTCAAACGCAGGTTAGACAA
GCACAAACCCCAAGAGAAAATCAAGTAGAAAGGGACAGAGTCTCTATCCCGGCAATGCCAACTCAGATACAGTATCCACAATATCAGCC
GGTAGAAAATAAGACCCAACCGCTGGTAGTTTATCAATACCGGCTGCCAACCGAGCTTCAGTATCGGCCTCCTTCAGAGGTTCAATACA
GACCTCAAGCGGTGTGTCCTGTGCCAAATAGCACGGCACCATACCAGCAACCCACAGCGATGGCGTCTAATTCACCAGCAACACAGGAC
GCGGCGCTGTATCCTCAGCCGCCCACTGTGAGACTTAATCCTACAGCATCACGTAGTGGACAGGGTGGTGCACTGCATGCAGTCATTGA
TGAAGCCAGAAAACAGGGCGATCTTGAGGCATGGCGGTTCCTGGTAATTTTACAACTGGTACAGGCCGGGGAAGAGACTCAAGTAGGAG
CGCCTGCCCGAGCTGAGACTAGATGTGAACCTTTCACCATGAAAATGTTAAAAGATATAAAGGAAGGAGTTAAACAATATGGATCCAAC
TCCCCTTATATAAGAACATTATTAGATTCCATTGCTCATGGAAATAGACTTACTCCTTATGACTGGGAAATTTTGGCCAAATCTTCCCT
TTCATCCTCTCAGTATCTACAGTTTAAAACCTGGTGGATTGATGGAGTACAAGAACAGGTACGAAAAAATCAGGCTACTAAGCCCACTG
TTAATATAGACGCAGACCAATTGTTAGGAACAGGTCCAAATTGGAGCACCATTAACCAACAATCAGTGATGCAGAATGAGGCTATTGAA
CAAGTAAGGGCTATTTGCCTCAGGGCCTGGGGAAAAATTCAGGACCCAGGAACAGCTTTCCCTATTAATTCAATTAGACAAGGCTCTAA
AGAGCCATATCCTGACTTTGTGGCAAGATTACAAGATGCTGCTCAAAAGTCTATTACAGATGACAATGCCCGAAAAGTTATTGTAGAAT
TAATGGCCTATGAAAATGCAAATCCAGAATGTCAGTCGGCCATAAAGCCATTAAAAGGAAAAGTTCCAGCAGGAGTTGATGTAATTACA
GAATATGTGAAGGCTTGTGATGGGATTGGAGGAGCTATGCATAAGGCAATGCTAATGGCTCAAGCAATGAGGGGGCTCACTCTAGGAGG
ACAAGTTAGAACATTTGGAAAAAAATGTTATAATTGTGGTCAAATCGGTCATCTGAAAAGGAGTTGCCCAGGCTTAAATAAACAGAATA
TAATAAATCAAGCTATTACAGCAAAAAATAAAAAGCCATCTGGCCTGTGTCCAAAATGTGGAAAAGCAAAACATTGGGCCAATCAATGT
CATTCTAAATTTGATAAAGATGGGCAACCATTGTCTGGAAACAGGAAGAGGGGCCAGCCTCAGGCCCCCCAACAAACTGGGGCATTCCC
AGTTAAACTGTTTGTTCCTCAGGGTTTTCAAGGACAACAACCCCTACAGAAAATACCACCACTTCAGGGAGTCAGCCAATTACAACAAT
CCAACAGCTGTCCCGCGCCACAGCAGGCAGCACCGCAGTAGATTTATGTTCCACCCAAATGGTCTTTTTACTCCCTGGAAAGCCCCCAC
AAAAGATTCCTAGAGGGGTATATGGCCCGCTGCCAGAAGGGAGGGTAGGCCTTTGAGGGAGATCAAGTCTAAATTTGAAGGGAGTCCAA
ATTCATACTGGGGTAATTTATTCAGATTATAAAGGGGGAATTCAGTTAGTGATCAGCTCCACTGTTCCCCGGAGTGCCAATCCAGGTGA
TAGAATTGCTCAATTACTGCTTTTGCCTTATGTTAAAATTGGGGAAAACAAAAAGGAAAGAACAGGAGGGTTTGGAAGTACCAACCCTG
CAGGAAAAGCTGCTTATTGGGCTAATCAGGTCTCAGAGGATAGACCCGTGTGTACAGTCACTATTCAGGGAAAGAGTTTGAAGGATTAG
TGGATACCCAGGCTGATGTTTCTGTCATCGGCATAGGTACTGCCTCAGAAGTGTATCAAAGTGCCATGATTTTACATTGTCCAGGATCT
GATAATCAAGAAAGTACGGTTCAGCCTGTGATCACTTCATTCCAATCAATTTATGGGGCCGAGACTTGTTACAACAATGGCATGCAGAG
ATTACTATCCCAGCCTCCCTATACAGCCCCAGGAATAAAAAAATCATGACTAAAATGGGATAGCTCCCTAAAAAGGGACTAGGAAAGAA
GTCCCAATTGAGGCTGAAAAAAATCAAAAAAGAAAAGGAATAGGGCATCCTTTTTAGGAGCGGTCACTGTAGAGCCTCCAAAACCCATT
CCATTAACTTGGGGGAAAAAAAAACAACTGTATGGTAAATCAGCAGCGCTTCCAAAACAAAAACTGGAGGCTTTACATTTATTAGCAAA
GAAACAATTAGAAAAAGGACATTGAGCCTTCATTTTCGCCTTGGAATTCTGTTTGTAATTCAGAAAAAATCCGGCAGATGGCGTATAAT
GCCGTAATTCAACCCATGGGGGCTCTCCCACCCCGGTTGCCCTCTCCAGCCATGGTCCCCTTTAATTATAATTGATCTGAAGGATTGCT
TTTTTACCATTCCTCTGGCAAAACAGGATTTTGAAAAATTTGCTTTTACCACACCAGCCTAAATAATAAAGAACCAGCCACCAGGTTTC
AGTGGAAAGTATTGCCTCAGGGAATGCTTAATAGTTCAACTATTTGTCAGCTCAAGCTCTGCAACCAGTTAGAGACAAGTTTTCAGACT

GTTACATCGTTCACTATGTTGATATTTTGTGTGCTGCAGAAACGAGAGACAAATTAATTGACCGTTACACATTTCTGCAGACAGAGGTT
GCCAACGCGGGACTGACAATAACATCTGATAAGATTCAAACCTCTACTCCTTTCCGTTACTTGGGAATGCAGGTAGAGGAAAGGAAAAT
TAAACCACAAAAAATAGAAATAAGAAAAGACACATTAAAAGCATTAAATGAGTTTCAAAAGTTGCTAGGAGATACTAATTGGATTTGGA
GATATTAATTGGATTTGGCCAACTCTAGGCATTCCTACTTATGCCATGTCAAATTTGTTCTCTTTCTTAAGAGGGGACTCGGAATTAAA
TAGTGAAAGAACGTTAACTCCAGAGGCAACTAAAGAAATTAAATTAATTGAAGAAAAAATTCGGTCAGCACAAGTAAATAGAATAGATC
ACTTGGCCCCACTCCAAATTTTGATTTTTGCTACTGCACATTCCCTAACAGGCATCATTGTTCAAAATACAGATCTTGTGGAGTGGTCC
TTCCTTCCTCACAGTACAATTAAGACTTTTACATTGTACTTGGATCAAATGGCTACATTAATTGGTCAGGGAAGATTATGAATAATAAC
ATTGTGTGGAAATGACCCAGATAAAATCACTGTTCCTTTCAACAAGCAACAGGTTAGACAAGCCTTTATCAATTCTGGTGCATGGCAGA
TTGGTCTTGCCGATTTTGTGGGAATTATTGACAATCGTTACCCCAAAACAAAAATCTTCCAGTTTTTAAAATTGACTACTTGGATTTTA
CCTAAAGTTACCAAACATAAGCCTTTAAAAAATGCTCTGGCAGTGTTTACTGATGGTTCCAGCAATGGAAAAGTGGCTTACACCGGGCC
AAAAGAATGAGTCATCAAAACTCAGTATCACTTGACTCAAAGAGCAGAGTTGGTTGCCGTCATTACAGTGTTAACAAGATTTTAATCAG
TCTATTAACATTGTATCAGATTCTGCATATGTAGTACAGGCTACAAAGGATATTGAGAGAGCCCTAATCAAATACATTATGGATGATCA
GTTAAACCCGCTGTTTAATTTGTTACAACAAAATGTAAGAAAAAGAAATTTCCCATTTTATATTACTCATATTCGAGCACACACTAATT
TACCAGGGCCTTTAACTAAAGCAAATGAACAAGCTGACTTGCTAGTATCATCTGCATTCATGGAAGCACAAGAACTTCATGCCTTGACT
CATGTAAATGCAATAGGATTAAAAAATAAATTTGATATCACATGGAAACAGACAAAAAATATTGTACAACATTGCACCCAGTGTCAGAT
TCTACACCTGGCCACTCAGGAGGCAAGAGTTAATCCCAGAGGTCTATGTCCTAATGTGTTATGGCAAATGGATGTCATGCACGTACCTT
CATTTGGAAAATTGTCATTTGTCCATGTGACAGTTGATACTTATTCACATTTCATATGGGCAACCTGCCAGACAGGAGAAAGTACTTCC
CATGTTAAAAGACATTTATTATCTTGTTTTCCTGTCATGGGAGTTCCAGAAAAAGTTAAAACAGACAATGGGCCAGGTTACTGTAGTAA
AGCAGTTCAAAAATTCTTAAATCAGTGGAAAATTACACATACAATAGGAATTCTCTATAATTCCCAAGGACAGGCCATAATTGAAAGAA
CTAATAGAACACTCAAAGCTCAATTGGTTAAACAAAAAAAGGAAAAGACAGGAGTATAACACTCCCCAGATGCAACTTAATCTAGCAC
TCTATACTTTAAATGTTTTAAACATTTATAGAAATCAGACCACTACCTCTGCAGAACAACATCTTACTGGTAAAAGGAACAGCCCACAT
GAAGGAAAACTGATTTGGTGGAAAGATAATAAAAATAAAACATGGGAAATGGGGAAGGTGATAACGTGGGGGAGAGGTTTTGCTTGTGT
TTCACCAGGAGAAAATCAGCTTCCTGTTTGGATACCCACTAGACATTTAAAGTTCTACAATGAACTCACTGGAGATGCAAAGAAAAGTG
TGGAGATGGAGACACCCCAATCGACTCGCCAGGTAAACAAAATGGTGATATCAGAAGAACAGAAAAAGTTGCCTTCCATCAAGGAAGCA
GAGTTGCCAATATAGGCACAATTAAAGAAGCTGACACAGTTAGCTAAAAAAAAAAGCCTAGAGAATACAAAGGTGACACCAACTCCAGA
GAATATGCTGCTTGCAGCTCTGATGATTGTATCAACGGTGGTAAGTCTTCCCAAGTCTGCAGGAGCAGCTGCAGCTAATTATACTTACT
GGGCCTATGTGCCTTTCCCACCCTTAATTCGGGCAGTTACATAGATGGATAATCCTATTGAAGTAGATGTTAATAATAGTGCATGGGTG
CCTGGCCCCACAGATGACTGTTGCCCTGCCCAACCTGAAGAAGGAATGATGATGAATATTTCCATTGGGTATCCTTATCCTCCTGTTTG
CCTAGGGAAGGCACCAGGATGCTTAATGCCTACAACCCAAAATTGGTTGGTAGAAGTACCTACAGTCAGTGCTACCAGTAGATTTACTT
ATCACATGGTAAGTGGAATGTCACAGATAAATAATTTACAGGACCCTTCTTATCAAAGATCATTACAATGTAGGCCTAAGGGGAAGGCT
TGCCCCAAGGAAATTCCCAAAGAATCAAAAAGCCCAGAAGTCTTAGTCTGCGGAGAATGTGTGGCTGATACTGCAGTGTAGTACAAAAC
AATGAATTTTGAACTATGATAGACTGGGTCCCTTGAGGCCAATTATATCATAACTGTACAGGCCAGACTCATTCATGTTCACAGGCCCC
ATCCATCTGGCCCATTAATCCAGCCTATGACGGTGATGTAACTGAAAGGCTGGACCAGGTTTATAGAAGGTTAGAATCACTCTGTCCAA
GGAAATGGGGTGAAAAGGGAATTTCATCACCTTGACCAAAGTTAGTCCTGTTACTGGTCCTGAACATCCAGAATTAGGAAGCTTACTGT
GGCCTCACACCACATTAGAATTTGTTCTGGAAATCAAGCTATAGGAACAAGAGATCGTAAGTCATATTATACTATCAACCTAAATTCCA
GTCTGACAATTCCTTTGCAAAATTGTGTAAAACTCCCTTATATTGCTAGTTGTAGGAAAAACATAGTTATTAAACCTGATTCCCAAACC
ATAATCTGTGAAAATTGTGGAATGTTTACTTGCATTGATTTGACTTTTAATTGGCAGCACCGTATTCTACTAGGAAGAGCAAGAGAGGG
TGTGTGGATCCTTGTGTCCATGGACCGACCATGGGAGGCTTCGCTATCCATCCATATTTTAACGGAAGTATTAAAAGGAATTCTAACTA
GATCCAAAAGATTCATTTTTACTTTGATGGCAGTGATTATGGGCCTCATTGCAGTCACAGCTACTGCTGCGGCTGCTGGAATTGCTTTA
CACTCCTCTGTTCAAACTGCAGAATACGTAAATGATTGGCAAAAGAATTCCTCAAAATTGTGGAATTCTCAGATCCAAATAGATCAAAA
ATTGGCAAACCAAATTAATGATCTTAGACAAACTGTCATTTGGATGGGAGAGGCTCATGAGCTTGGAATATCTTTTTCAGTTACGATGT
GACTGGAATACATCAGATTTTTGTGTTACACCACAAGCCTATAATGAGTCTGAGCATCACTGGGACATGGTTAGATGCCATCTGCAAGG
AGGAGAAGATAATCTTACTTTAGACATTTCAAAATTAAAAGAATTTTTTTTTTCTTTGAGACAGAGTCTCGCTCTGTCGCCCAGGCTGG
AGTGCAGTGGCGTGATCTCAGCTCACTGCAAGTTCCGCCTCCTGGGTTTACACCATTCTCCTGCCTCAGCCTCCCAAGTAGTTGGGACT
ACAGGAGCCCACCACCATGCCTGGCTAATTTTTTTTGGGTTTTTAATAGAGATGGAGTTTCACCGTGTTAGCCAGGATGGTCTCGATCT
CCTGACCTTGTGATCTGCCCACCTTGGCCTCCCAAAGTGCTGGGATTACAGTCGTGAGCCACCGTGCCCAGCCAAGAAAAAATTTTTGA
GGCATCAAAAGCCCATTTAAATTTGGTGCCAGGAACGGAGACAATCGTGAAAGCTGCTGATAGCCTCACAAATCTTAAGCCAGTCACTT
GGGTTAAAAGCATCAGAAGTTTCACTATTGTAAATTTCATATTAATCCTTGTATGCCTGTTCTGTCTGTTGTTAGTCTACAGGTGTATC
CAGCAGCTCCAAAGAGACAGCAACCAGCAAGAATGGGCCATAGTGACGATGGTGGTTTTGTCAAAAAGAAAAGGGGGGGATATGTAAGG
AAAAGAGAGATCAGACTTTCACTGTGTCTATGTAGAAAAGGAAGACATAAGAAACTCCATTTTGATCTGTACTAAGAAAAATTGTTTTG
CCTTGAGATGCTGTTAATCTGTAACTTTAGCCCCAACCCTGTGCTCACGGAAACATGTGCTGTAAGGTTTAAGGGATCTAGGGCTGTGC
AGGATGTACCTTGTTAACAATATGTTTGCAGGCAGTATGTTTGGTAAAAGTCATCGCCATTCTCCATTCTCGATTAACCAGGGGCTCAA
TGCACTGTGGAAAGCCACAGGAACCTCTGCCCAAGAAAGCCTGGCTGTTGTGGGAAGTCAGGGACCCCGAATGGAGGGACCAGCTGGTG

CTGCATCAGGAAACATAAATTGTGAAGATTTCTTGGACATTTATCAGTTTCCAAAATTAATACTTTTATAATTTCTTACACCTGTCTTA
CTTTAATCTCTTAATCCTGTTATCTTTGTAAGCTGAGGATATACGTCACCTCAGGACCACTATTGTACAAATTGATTGTAAAACATGTT
CACATGTGTTTGAACAATATGAAATCAGTGCACCTTGAAAATGAACAGAATAACAGTGATTTTAGGGAACAAAGGAAGACAACCATAAG
GTCTGACTGCCTGAGGGGTCGGGCAAAAAGCCATATTTTTCTTCTTGCAGAGAGCCTATAAATGGACGTGCAAGTAGGAGAGATATTGC
TAAATTCTTTTCCTAGCAAGGAATATAATACTAAGACCCTAGGGAAAGAATTGCATTCCTGGGGGGAGGTCTATAAACGGCCGCTCTGG
GAGTGTCTGTCCTATGTGGTTGAGATAAGGACTGAGATACGCCCTGGTCTCCTGCAGTACCCTCAGGCTTACTAGGATTGGGAAACCCC
AGTCCTGGTAAATTTGAGGTCAGGCCGGTTCTTTGCTCTGAACCCTGTTTTCTGTTAAGATGTTTATCAAGACAATACATGCACCGCTG
AACATAGACCCTTATCAGGAGTTTCTGATTTTGCTCTGGTCCTGTTTCTTCAGAAGCATGTCATCTTTGCTCTGCCTTCTGCCCTTTGA
AGCATGTGATCTTTGTGACCTACTCCCTGTTCATACACCCCTCCCCTTTTAAAATCCCTAATAAAAACTTGCTGGTTTTGTGGCTCAGG
GGGGCATCATGGACCTACCAATACGTGATGTCACCCCCGGTGGCCCAGCTGT
SEQ ID 11
TAGGTCTC
SEQ ID 12
TAGGGGTG
SEQ ID 13
TGGCTGTT
SEQ ID 14
AACGTGGATGCTTTTCTCTAGGGTGAAGGGACTCTCGAGTGTGGTCATTGAGGACAAGTCAACGAGAGATTCCCGAGTACGTCTACAGT
GAGCCTTGTGGGTGAAGGTACTCTACAGTGTGGTCATTGAGGACAAGTTGACGAGAGAGTCCCAAGTACGTCCACGGTCAGCCTTGCGA
CATTTTAAAGTTCTACAATGAACTCA
SEQ ID 15
ACAGAAGGGTACATGAAGGAAAACTGATTTGGTGGAAAGATAATAAAAATAAAACATGGGAAATGGGGAAGGTGATAACGTGGGGGAGA
GGTTTTGCTTGTGTTTCACCAGGAGAAAATCAGCTTCCTGTTTGGATACCCACTAGACATTTAAAGTTCTACAATGAACTCACTGGAGA
TGCAAAGAAAAGTGTGGAGATGGAGACACCCCAATCGACTCGCCAGTCTACAGGTGTATCCAGCAGCTCCAAAGAGACAGCAACCAGCA
AGAATGGGCCATAGTGACGATGGTGGTTTTGTCAAAAAGAAAAGGGGGGGATATGTAAGGAAAAGAGAGATCAGACTTTCACTGTGTCT
ATGTAGAAAAGGAAGACATAAGAAACTCCATTTTGATCTGTACTAAGAAAAATTGTTTTGCCTTGAGATGCTGTTAATCTGTAACTTTA
GCCCCAGCCCTGTGCTCACGGAAACATGTGCTGTAAGGTTTAAGGGATCTAGGGCTGT
SEQ ID 16
AGTCATCAAAACTCAGTATCACTTGACTCAAAGAGCAGAGTTGGTTGCCGTCATTACAGTGTTAACAAGATTTTAATCAGTCTATTAAC
ATTGTATCAGATTCTGCATATGTAGTACAGGCTACAAAGGATATTGAGAGAGCCCTAATCAAATACATTATGGATGATCAGTTAAACCC
GCTGTTTAATTTGTTACAACAAAATGTAAGAAAAAGAAATTTCCCATTTTATATTACTCATATTCGAGCACACACTAATTTACCAGGGC
CTTTAACTAAAGCAAATGAACAAGCTGACTCGCTAGTATCATCTGCATTCATGGAAGCACAAGACCTTCATGCCTTGACTCATGTAAAT
GCAATAGGATTAAAAAATAAATTTAATATCACATGGAAACAGACAAAAAATATTGTACAACATTGCACCCAGTGTCAGATTCTACACCT
GGCCACTCAGGAGGCAAGAGTTAATCCCAGAGGTCTATGTCCTAATGTGTTATGGCAAATGGATGTCATGCACGTACCTTCATTTGGAA
AATTGTCATTTGTCCATGTGACAGTTGATACTTATTCACATTTCATATGGGCAACCTGCCAGACAGGAGAAAGTACTTCCCATGTTAAG
AGACATTTATTATC
SEQ ID 17
AGTCATCAAAACTCAGTATCACTTGACTCAAAGAGCAGAGTTGGTTGCCGTCATTACAGTGTTAACAAGATTTTAATCAGTCTATTAAC
ATTGTATCAGATTCTGCATATGTAGTACAGGCTACAAAGGATATTGAGAGAGCCCTAATCAAATACATTATGGATGATCAGTTAAACCC
GCTGTTTAATTTGTTACAACAAAATGTAAGAAAAAGAAATTTCCCATTTTATATTACTCATATTCGAGCACACACTAATTTACCAGGGC
CTTTAACTAAAGCAAATGAACAAGCTGACTTGCTAGTATCATCTGCATTCATGGAAGCACAAGAACTTCATGCCTTGACTCATGTAAAT
GCAATAGGATTAAAAAATAAATTTGATATCACATGGAAACAGACAAAAAATATTGTACAACATTGCACCCAGTGTCAGATTCTACACCT
GGCCACTCAGGAGGCAAGAGTTAATCCCAGAGGTCTATGTCCTAATGTGTTATGGCAAATGGATGTCATGCACGTACCTTCATTTGGAA
AATTGTCATTTGTCCATGTGACAGTTGATACTTATTCACATTTCATATGGGCAACCTGCCAGACAGGAGAAAGTACTTCCCATGTTAAA
AGACATTTATTATCTTGTTTTCCTGTC
SEQ ID 18
AGTCATCAAAACTCAGTATCACTTGACTCAAAGAGCAGAGTTGGTTGCCGTCATTACAGTGTTAACAAGATTTTAATCAGTCTATTAAC
ATTGTATCAGATTCTGCATATGTAGTACAGGCTACAAAGGATATTGAGAGAGCCCTAATCAAATACATTATGGATGATCAGTTAAACCC
GCTGTTTAATTTGTTACAACAAAATGTAAGAAAAAGAAATTTCCCATTTTATATTACTCATATTCGAGCACACACTAATTTACCAGGGC
CTTTAACTAAAGCAAATGAACAAGCTGACTTGCTAGTATCATCTGCATTCATGGAAGCACAAGAACTTCATGCCTTGACTCATGTAAAT
GCAATAGGATTAAAAAATAAATTTGATATCACATGGAAACAGACAAAAAATATTGTACAACATTGCACCCAGTGTCAGATTCTACACCT

GGCCACTCAGGAGGCAAGAGTTAATCCCAGAGGTCTATGTCCTAATGTGTTATGGCAAATGGATGTCATGCACGTACCTTCATTTGGAA
AATTGTCATTTGTCCATGTGACAGTTGATACTTATTCACATTTCATATGGGCAACCTGCCAGACAGGAGAAAGTACTTCCCATGTTAAA
AGACATTTATTATCTTGTTTTCCTGTC
SEQ ID 19
AGATCTGATCATCTGGTGCCCAACGTGGAGGCTTTTCTCTAGGGTGAAGGGACTCTCGAGTGTGGTCATTGAGGACAAGTCAACGAGAG
ATTCCCGAGTACGTCTACAGTGAGCCTTGTGGGTGAAGGTACTCTACAGTGTGGTCATTGAGGACAAGTTGACGAGAGAGTCCCAAGTA
CGTCCACGGTCAGCCTTGCGAC
SEQ ID 20
AACTGGAAGAATTCGCGGCCGCATTTTTTTTTTTTTTTTTTTTTTTTT
SEQ ID 21
CAGGTGTAYCCARCAGCTCC
SEQ ID 22
AACTGGAAGAATTCGCGGCCGCA
SEQ ID 23
GAGAUAGGAGAAAACUGCCCUUAGGGCUGGAGGUGGGACAUGCUGGCGGCAAUACUGCUCUUUAAGGCAUUGAGAUGUUUAUGUAUAUGC
ACAUCAAAAGCACAGCACUUUUUUCUUUACCUUGUUUAUGAUGCAGAGACAUUUGUUCACAUGUUUUCCUGCUGGCCCUCUCCCCACUA
UUACCCUAUUGUCCUGCCACAUCCCCCUCUCCGAGAUGGUAGAGAUAAUGAUCAAUAAAUACUGAGGGAACUCAGAGACCGGUGCGGCG
CGGGUCCUCCAUAUGCUGAGCGCCGGUCCCCUGGGCCCACUUUUCUUUCUCUAUACUUUGUCUCUGUUGUCUUUCUUUUCUCAAGUCUC
UCGUUCCACCUGAGGAGAAAUGCCCACAGCUGUGGAGGCGCAGGCCACUCCAUCUGGUGCCCAACGUGGAUGCUUUUCUCUAGGGUGAA
GGGACUCUCGAGUGUGGUCAUUGAGGACAAGUCAACGAGAGAUUCCCGAGUACGUCUACAGUGAGCCUUGUGGUAAGCUUGGGCGCUCG
GAAGAAGCCAGGGUUAAUGGGGCAAACUAAAAGUAAAGUCUCUCAUUCCACCUGAUGAGAAACACCCAGAGGUGUGGAGGGGCAGGCCA
CCCCUUCAGGGUAGGGUCCCCUCCAUGCAGACCAUAGAGCACAGGUGUGCCCCAAAGAGGAGCAGAGAGAAGGAGGGAGAGGGCCCACG
AGAGACUUGGAAAUGAAUGGCAGGAUUUUAGGCGCUGGACUUGGGUUCGGGGCACCUGGCCUUUCCUUGUGUAUUUCUCCUACUGUCUG
CCUAACUAUUUAAUACAAUAAAAGAAAACCAGCCCCUGGUUCUUGUGGUGUUUCCACCCUCCCGGGUCCCCGCUGGCUGCCUGGCUUCC
UCCCGCAGCUCCUGCUGUGUGUGUAUGUGUGUGUGUGCACAUCUGUGGGGCGUAUGUGUGUUCGUCUUUGUAAUUGAGGCUGCAGAG
UGGAGAGAGCAGGGGUUUUCUCUGGGGACCCAGAGAGAAGGAGGCGUUUUCACCACAGCCGAACAGGGCAGGACCCCAGCACCCGGGAC
CCAGCGGGACUUUGCCAAGGGGAUGGACCUGGCUGGGCCACGCGGCUGUUUGUGUAGGGAAAAGAAAGAGAGAUCACACUGUUACUGUG
UCUAUGUAGAAAAGGAAGACAUAAACUCCAUUUUGAGCUGUACUAAGAAAAAUUAUUUUGCCUUGACCUGCUGUUAACCUGUAACUGUA
GCCCCAACCCUGUGCUCAAAGAAACAUGUGCUGUAUGGAAUCAAGGUUUAAGGGAUCAAGGGCUGUACAGGAUGUGCCUUGUUAACAAU
GUGUUUACAGGCAGUAUGCUUGGUAAAAGUCAUCGCCAUUCUCCAUUCUCCAUUAAUCAGGGGCACGAUGCACUGCGGAAAGCCACAGG
GACCUCUGCCCGAGAAAGCCUGGGUAUUGUCCAAGGCUUCCCCCCACUGAGACAGCCUGAGAUACGGCCUCGUGGGAAGGGAAAGACCU
GACCGUCCCCCAGCCCGACACCCGUAAAGGGUCUGUGCUGAGGAGGAUUAGUAAAAGGGGAAGGCCUCUUGCAGUUGAGAUAAGAGGAA
GGCCUCCGUCUCCUGCAUGUCCUUGGGAAUGGAAUGUCUUGGUGUAAAACCCGAUAGUACAUUCCUUCUAUUCUGAGAGAAGAAAACCA
CCCUGUGGCUGGAGGUGAGAUAUGCUAGCGGCAAUGCUGCUCUGUUACUCUUUGCUACACUGAGAUGUUUGGGUGGAGAGAAGCAUAAA
UCUGGCCUAUGUGCACAUCUGGGCACAGAACCUCCCCUUGAACUUGUGACACAGAUUCCUUUGUUCACAUGUUUCCUGCUGACCUUCU
CCCCACUAUCGCCCUGUUCUCCCACCGCAUUCCCCUUGCUGAGAUAGUGAAAAUAGUAAUCUGUAGAUACCAAGGGAACUCAGAGACCA
UGGCCGGUGCACAUCCUCCGUACGCUGAGCGCUGGUCCCCUGGGCCCAUUGUUCUUUCUCUAUACUUUGUCUCUGUGUCUUAUUUCUUU
CCUCAGUCUCUCAUCCCUCCUGACGAGAAAUACCCACAGGUGUGGAGGGGCUGGCCCCCUUCAUCUGAUGCCCAAUGUGGGUGCCUUUC
UCUAGGGUGAAGGUACUCUACAGUGUGGUCAUUGAGGACAAGUUGACGAGAGAGUCCCAAGUACGUCCACGGUCAGCCUUGCGGUAAGC
UUGUGUGCUUAGAGGAACCCAGGGUAACGAUGGGGCAAACUGAAAGUAAAUAUGCCUCUUAUCUCAGCUUUAUUAAAAUUCUUUUAAGA
AGAGGGGGAGUUAGAGCUUCUACAGAAAAUCUAAUUACGCUAUUUCAAACAAUAGAACAAUUCUGCCCAUGGUUUCCAGAACAGGGAAC
UUUAGAUCUAAAAGAUUGGGAAAAAAUUGGCAAAGAAUUAAAACAAGCAAAUAGGGAAGGUAAAAUCAUCCCACUUACAGUAUGGAAUG
AUUGGGCCAUUAUUAAAGCAACUUUAGAACCAUUUCAAACAGGAGAAGAUAUUGUUUCAGUUUCUGAUGCCCCUAAAAGCUGUGUAACA
GAUUGUGAAGAAGAGGCAGGGACAGAAUCCCAGCAAGGAACGGAAAGUUCACAUUGUAAAUAUGUAGCAGAGUCUGUAAUGGCUCAGUC
AACGCAAAAUGUUGACUACAGUCAAUUACAGGAGAUAAUAUACCCUGAAUCAUCAAAAUUGGGGGAAGGAGGUCCAGAAUCAUUGGGGC
CAUCAGAGCCUAAACCACGAUCGCCAUCAACUCCUCCUCCCGUGGUUCAGAUGCCUGUAACAUUACAACCUCAAACGCAGGUUAGACAA
GCACAAACCCCAAGAGAAAAUCAAGUAGAAAGGGACAGAGUCUCUAUCCCGGCAAUGCCAACUCAGAUACAGUAUCCACAAUAUCAGCC
GGUAGAAAAUAAGACCCAACCGCUGGUAGUUUAUCAAUACCGGCUGCCAACCGAGCUUCAGUAUCGGCCUCCUUCAGAGGUUCAAUACA
GACCUCAAGCGGUGUGUCCUGUGCCAAAUAGCACGGCACCAUACCAGCAACCCACAGCGAUGGCGUCUAAUUCACCAGCAACACAGGAC
GCGGCGCUGUAUCCUCAGCCGCCCACUGUGAGACUUAAUCCUACAGCAUCACGUAGUGGACAGGGUGGUGCACUGCAUGCAGUCAUUGA
UGAAGCCAGAAAACAGGGCGAUCUUGAGGCAUGGCGGUUCCUGGUAAUUUUACAACUGGUACAGGCCGGGGAAGAGACUCAAGUAGGAG
CGCCUGCCCGAGCUGAGACUAGAUGUGAACCUUUCACCAUGAAAAUGUUAAAAGAUAUAAAGGAAGGAGUUAAACAAUAUGGAUCCAAC

UCCCCUUAUAUAAGAACAUUAUUAGAUUCCAUUGCUCAUGGAAAUAGACUUACUCCUUAUGACUGGGAAAUUUUGGCCAAAUCUUCCCU
UUCAUCCUCUCAGUAUCUACAGUUUAAAACCUGGUGGAUUGAUGGAGUACAAGAACAGGUACGAAAAAAUCAGGCUACUAAGCCCACUG
UUAAUAUAGACGCAGACCAAUUGUUAGGAACAGGUCCAAAUUGGAGCACCAUUAACCAACAAUCAGUGAUGCAGAAUGAGGCUAUUGAA
CAAGUAAGGGCUAUUUGCCUCAGGGCCUGGGGAAAAAUUCAGGACCCAGGAACAGCUUUCCCUAUUAAUUCAAUUAGACAAGGCUCUAA
AGAGCCAUAUCCUGACUUUGUGGCAAGAUUACAAGAUGCUGCUCAAAAGUCUAUUACAGAUGACAAUGCCCGAAAAGUUAUUGUAGAAU
UAAUGGCCUAUGAAAAUGCAAAUCCAGAAUGUCAGUCGGCCAUAAAGCCAUUAAAAGGAAAAGUUCCAGCAGGAGUUGAUGUAAUUACA
GAAUAUGUGAAGGCUUGUGAUGGGAUUGGAGGAGCUAUGCAUAAGGCAAUGCUAAUGGCUCAAGCAAUGAGGGGGCUCACUCUAGGAGG
ACAAGUUAGAACAUUUGGGAAAAAAUGUUAUAAUUGUGGUCAAAUCGGUCAUCUGAAAAGGAGUUGCCCAGGCUUAAAUAAACAGAAUA
UAAUAAAUCAAGCUAUUACAGCAAAAAAUAAAAAGCCAUCUGGCCUGUGUCCAAAAUGUGGAAAAGCAAAACAUUGGGCCAAUCAAUGU
CAUUCUAAAUUUGAUAAAGAUGGGCAACCAUUGUCUGGAAACAGGAAGAGGGGCCAGCCUCAGGCCCCCCAACAAACUGGGGCAUUCCC
AGUUAAACUGUUUGUUCCUCAGGGUUUUCAAGGACAACAACCCCUACAGAAAAUACCACCACUUCAGGGAGUCAGCCAAUUACAACAAU
CCAACAGCUGUCCCGCGCCACAGCAGGCAGCACCGCAGUAGAUUUAUGUUCCACCCAAAUGGUCUUUUUACUCCCUGGAAAGCCCCCAC
AAAAGAUUCCUAGAGGGGUAUAUGGCCCGCUGCCAGAAGGGAGGGUAGGCCUUUGAGGGAGAUCAAGUCUAAAUUUGAAGGGAGUCCAA
AUUCAUACUGGGGUAAUUUAUUCAGAUUAUAAAGGGGGAAUUCAGUUAGUGAUCAGCUCCACUGUUCCCCGGAGUGCCAAUCCAGGUGA
UAGAAUUGCUCAAUUACUGCUUUUGCCUUAUGUUAAAAUUGGGGAAAACAAAAAGGAAAGAACAGGAGGGUUUGGAAGUACCAACCCUG
CAGGAAAAGCUGCUUAUUGGGCUAAUCAGGUCUCAGAGGAUAGACCCGUGUGUACAGUCACUAUUCAGGGAAAGAGUUUGAAGGAUUAG
UGGAUACCCAGGCUGAUGUUUCUGUCAUCGGCAUAGGUACUGCCUCAGAAGUGUAUCAAAGUGCCAUGAUUUUACAUUGUCCAGGAUCU
GAUAAUCAAGAAAGUACGGUUCAGCCUGUGAUCACUUCAUUCCAAUCAAUUUAUGGGGCCGAGACUUGUUACAACAAUGGCAUGCAGAG
AUUACUAUCCCAGCCUCCCUAUACAGCCCCAGGAAUAAAAAAAUCAUGACUAAAAUGGGAUAGCUCCCUAAAAAGGGACUAGGAAAGAA
GUCCCAAUUGAGGCUGAAAAAAAUCAAAAAAGAAAAGGAAUAGGGCAUCCUUUUUAGGAGCGGUCACUGUAGAGCCUCCAAAACCCAUU
CCAUUAACUUGGGGGAAAAAAAAACAACUGUAUGGUAAAUCAGCAGCGCUUCCAAAACAAAAACUGGAGGCUUUACAUUUAUUAGCAAA
GAAACAAUUAGAAAAAGGACAUUGAGCCUUCAUUUUCGCCUUGGAAUUCUGUUUGUAAUUCAGAAAAAAUCCGGCAGAUGGCGUAUAAU
GCCGUAAUUCAACCCAUGGGGGCUCUCCCACCCCGGUUGCCCUCUCCAGCCAUGGUCCCCUUUAAUUAUAAUUGAUCUGAAGGAUUGCU
UUUUUACCAUUCCUCUGGCAAAACAGGAUUUUGAAAAAUUUGCUUUUACCACACCAGCCUAAAUAAUAAAGAACCAGCCACCAGGUUUC
AGUGGAAAGUAUUGCCUCAGGGAAUGCUUAAUAGUCAACUAUUUGUCAGCUCAAGCUCUGCAACCAGUUAGAGACAAGUUUUCAGACU
GUUACAUCGUUCACUAUGUUGAUAUUUUGUGUGCUGCAGAAACGAGAGACAAAUUAAUUGACCGUUACACAUUUCUGCAGACAGAGGUU
GCCAACGCGGGACUGACAAUAACAUCUGAUAAGAUUCAAACCUCUACUCCUUUCCGUUACUUGGGAAUGCAGGUAGAGGAAAGGAAAAU
UAAACCACAAAAAAUAGAAAUAAGAAAAGACACAUUAAAAGCAUUAAAUGAGUUUCAAAAGUUGCUAGGAGAUACUAAUUGGAUUUGGA
GAUAUUAAUUGGAUUUGGCCAACUCUAGGCAUUCCUACUUAUGCCAUGUCAAAUUUGUUCUCUUUCUUAAGAGGGGACUCGGAAUUAAA
UAGUGAAAGAACGUUAACUCCAGAGGCAACUAAAGAAAUUAAAUUAAUUGAAGAAAAAAUUCGGUCAGCACAAGUAAAUAGAAUAGAUC
ACUUGGCCCCACUCCAAAUUUUGAUUUUUGCUACUGCACAUUCCCUAACAGGCAUCAUUGUUCAAAAUACAGAUCUUGUGGAGUGGUCC
UUCCUUCCUCACAGUACAAUUAAGACUUUUACAUUGUACUUGGAUCAAAUGGCUACAUUAAUUGGUCAGGGAAGAUUAUGAAUAAUAAC
AUUGUGUGGAAAUGACCCAGAUAAAAUCACUGUUCCUUUCAACAAGCAACAGGUUAGACAAGCCUUUAUCAAUUCUGGUGCAUGGCAGA
UUGGUCUUGCCGAUUUUGUGGGAAUUAUUGACAAUCGUUACCCCAAAACAAAAAUCUUCCAGUUUUUAAAAUUGACUACUUGGAUUUUA
CCUAAAGUUACCAAACAUAAGCCUUUAAAAAAUGCUCUGGCAGUGUUUACUGAUGGUUCCAGCAAUGGAAAAGUGGCUUACACCGGGCC
AAAAGAAUGAGUCAUCAAAACUCAGUAUCACUUGACUCAAAGAGCAGAGUUGGUUGCCGUCAUUACAGUGUUAACAAGAUUUUAAUCAG
UCUAUUAACAUUGUAUCAGAUUCUGCAUAUGUAGUACAGGCUACAAAGGAUAUUGAGAGAGCCCUAAUCAAAUACAUUAUGGAUGAUCA
GUUAAACCCGCUGUUUAAUUUGUUACAACAAAAUGUAAGAAAAAGAAAUUUCCCAUUUUAUAUUACUCAUAUUCGAGCACACACUAAUU
UACCAGGGCCUUUAACUAAAGCAAAUGAACAAGCUGACUUGCUAGUAUCAUCUGCAUUCAUGGAAGCACAAGAACUUCAUGCCUUGACU
CAUGUAAAUGCAAUAGGAUUAAAAAAUAAAUUUGAUAUCACAUGGAAACAGACAAAAAAUAUUGUACAACAUUGCACCCAGUGUCAGAU
UCUACACCUGGCCACUCAGGAGGCAAGAGUUAAUCCCAGAGGUCUAUGUCCUAAUGUGUUAUGGCAAAUGGAUGUCAUGCACGUACCUU
CAUUUGGAAAAUUGUCAUUUGUCCAUGUGACAGUUGAUACUUAUUCACAUUUCAUAUGGGCAACCUGCCAGACAGGAGAAAGUACUUCC
CAUGUUAAAAGACAUUUAUUAUCUUGUUUUCCUGUCAUGGGAGUUCCAGAAAAAGUUAAAACAGACAAUGGGCCAGGUUACUGUAGUAA
AGCAGUUCAAAAAUUCUUAAAUCAGUGGAAAAUUACACAUACAAUAGGAAUUCUCUAUAAUUCCCAAGGACAGGCCAUAAUUGAAAGAA
CUAAUAGAACACUCAAAGCUCAAUUGGUUAAACAAAAAAAAGGAAAAGACAGGAGUAUAACACUCCCCAGAUGCAACUUAAUCUAGCAC
UCUAUACUUUAAAUGUUUUAAACAUUUAUAGAAAUCAGACCACUACCUCUGCAGAACAACAUCUUACUGGUAAAAGGAACAGCCCACAU
GAAGGAAAACUGAUUUGGUGGAAAGAUAAUAAAAAUAAAACAUGGGAAAUGGGGAAGGUGAUAACGUGGGGGAGAGGUUUUGCUUGUGU
UUCACCAGGAGAAAAUCAGCUUCCUGUUUGGAUACCCACUAGACAUUUAAAGUUCUACAAUGAACUCACUGGAGAUGCAAAGAAAAGUG
UGGAGAUGGAGACACCCCAAUCGACUCGCCAGGUAAACAAAAUGGUGAUAUCAGAAGAACAGAAAAAGUUGCCUUCCAUCAAGGAAGCA
GAGUUGCCAAUAUAGGCACAAUUAAAGAAGCUGACACAGUUAGCUAAAAAAAAAAGCCUAGAGAAUACAAAGGUGACACCAACUCCAGA
GAAUAUGCUGCUUGCAGCUCUGAUGAUUGUAUCAACGGUGGUAAGUCUUCCCAAGUCUGCAGGAGCAGCUGCAGCUAAUUAUACUUACU
GGGCCUAUGUGCCUUUCCCACCCUUAAUUCGGGCAGUUACAUAGAUGGAUAAUCCUAUUGAAGUAGAUGUUAAUAAUAGUGCAUGGGUG
CCUGGCCCCACAGAUGACUGUUGCCCUGCCCAACCUGAAGAAGGAAUGAUGAUGAAUAUUUCCAUUGGGUAUCCUUAUCCUCCUGUUUG

CCUAGGGAAGGCACCAGGAUGCUUAAUGCCUACAACCCAAAAUUGGUUGGUAGAAGUACCUACAGUCAGUGCUACCAGUAGAUUUACUU
AUCACAUGGUAAGUGGAAUGUCACAGAUAAAUAAUUUACAGGACCCUUCUUAUCAAAGAUCAUUACAAUGUAGGCCUAAGGGGAAGGCU
UGCCCCAAGGAAAUUCCCAAAGAAUCAAAAAGCCCAGAAGUCUUAGUCUGCGGAGAAUGUGUGGCUGAUACUGCAGUGUAGUACAAAAC
AAUGAAUUUUGAACUAUGAUAGACUGGGUCCCUUGAGGCCAAUUAUAUCAUAACUGUACAGGCCAGACUCAUUCAUGUUCACAGGCCCC
AUCCAUCUGGCCCAUUAAUCCAGCCUAUGACGGUGAUGUAACUGAAAGGCUGGACCAGGUUUAUAGAAGGUUAGAAUCACUCUGUCCAA
GGAAAUGGGGUGAAAAGGGAAUUUCAUCACCUUGACCAAAGUUAGUCCUGUUACUGGUCCUGAACAUCCAGAAUUAGGAAGCUUACUGU
GGCCUCACACCACAUUAGAAUUUGUUCUGGAAAUCAAGCUAUAGGAACAAGAGAUCGUAAGUCAUAUUAUACUAUCAACCUAAAUUCCA
GUCUGACAAUUCCUUUGCAAAAUUGUGUAAAACUCCCUUAUAUUGCUAGUUGUAGGAAAAACAUAGUUAUUAAACCUGAUUCCCAAACC
AUAAUCUGUGAAAAUUGUGGAAUGUUUACUUGCAUUGAUUUGACUUUUAAUUGGCAGCACCGUAUUCUACUAGGAAGAGCAAGAGAGGG
UGUGUGGAUCCUUGUGUCCAUGGACCGACCAUGGGAGGCUUCGCUAUCCAUCCAUAUUUAACGGAAGUAUUAAAAGGAAUUCUAACUA
GAUCCAAAAGAUUCAUUUUUACUUUGAUGGCAGUGAUUAUGGGCCUCAUUGCAGUCACAGCUACUGCUGCGGCUGCUGGAAUUGCUUUA
CACUCCUCUGUUCAAACUGCAGAAUACGUAAAUGAUUGGCAAAAGAAUUCCUCAAAAUUGUGGAAUUCUCAGAUCCAAAUAGAUCAAAA
AUUGGCAAACCAAAUUAAUGAUCUUAGACAAACUGUCAUUUGGAUGGGAGAGGCUCAUGAGCUUGGAAUAUCUUUUUCAGUUACGAUGU
GACUGGAAUACAUCAGAUUUUUGUGUUACACCACAAGCCUAUAAUGAGUCUGAGCAUCACUGGGACAUGGUUAGAUGCCAUCUGCAAGG
AGGAGAAGAUAAUCUUACUUUAGACAUUUCAAAAUUAAAAGAAUUUUUUUUUCUUUGAGACAGAGUCUCGCUCUGUCGCCCAGGCUGG
AGUGCAGUGGCGUGAUCUCAGCUCACUGCAAGUUCCGCCUCCUGGGUUUACACCAUUCUCCUGCCUCAGCCUCCCAAGUAGUUGGGACU
ACAGGAGCCCACCACCAUGCCUGGCUAAUUUUUUUUGGGUUUUUAAUAGAGAUGGAGUUUCACCGUGUUAGCCAGGAUGGUCUCGAUCU
CCUGACCUUGUGAUCUGCCCACCUUGGCCUCCCAAAGUGCUGGGAUUACAGUCGUGAGCCACCGUGCCCAGCCAAGAAAAAAUUUUUGA
GGCAUCAAAAGCCCAUUUAAAUUUGGUGCCAGGAACGGAGACAAUCGUGAAAGCUGCUGAUAGCCUCACAAAUCUUAAGCCAGUCACUU
GGGUUAAAAGCAUCAGAAGUUUCACUAUUGUAAAUUUCAUAUUAAUCCUUGUAUGCCUGUUCUGUCUGUUGUUAGUCUACAGGUGUAUC
CAGCAGCUCCAAAGAGACAGCAACCAGCAAGAAUGGCCAUAGUGACGAUGGUGGUUUUGUCAAAAAGAAAAGGGGGGAUAUGUAAGG
AAAAGAGAGAUCAGACUUUCACUGUGUCUAUGUAGAAAAGGAAGACAUAAGAAACUCCAUUUUGAUCUGUACUAAGAAAAAUUGUUUUG
CCUUGAGAUGCUGUUAAUCUGUAACUUUAGCCCCAACCCUGUGCUCACGGAAACAUGUGCUGUAAGGUUUAAGGGAUCUAGGGCUGUGC
AGGAUGUACCUUGUUAACAAUAUGUUUGCAGGCAGUAUGUUUGGUAAAAGUCAUCGCCAUUCUCCAUUCUCGAUUAACCAGGGGCUCAA
UGCACUGUGGAAAGCCACAGGAACCUCUGCCCAAGAAAGCCUGGCUGUUGUGGGAAGUCAGGGACCCCGAAUGGAGGGACCAGCUGGUG
CUGCAUCAGGAAACAUAAAUUGUGAAGAUUUCUUGGACAUUUAUCAGUUUCCAAAAUUAAUACUUUUAUAAUUUCUUACACCUGUCUUA
CUUUAAUCUCUUAAUCCUGUUAUCUUUGUAAGCUGAGGAUAUACGUCACCUCAGGACCACUAUUGUACAAAUUGAUUGUAAAACAUGUU
CACAUGUGUUUGAACAAUAUGAAAUCAGUGCACCUUGAAAAUGAACAGAAUAACAGUGAUUUUAGGGAACAAAGGAAGACAACCAUAAG
GUCUGACUGCCUGAGGGGUCGGGCAAAAAGCCAUAUUUUUCUUCUUGCAGAGAGCCUAUAAAUGGACGUGCAAGUAGGAGAGAUAUUGC
UAAAUUCUUUUCCUAGCAAGGAAUAUAAUACUAAGACCCUAGGGAAAGAAUUGCAUUCCUGGGGGGAGGUCUAUAAACGGCCGCUCUGG
GAGUGUCUGUCCUAUGUGGUUGAGAUAAGGACUGAGAUACGCCCUGGUCUCCUGCAGUACCCUCAGGCUUACUAGGAUUGGGAAACCCC
AGUCCUGGUAAAUUUGAGGUCAGGCCGGUUCUUUGCUCUGAACCCUGUUUUCUGUUAAGAUGUUUAUCAAGACAAUACAUGCACCGCUG
AACAUAGACCCUUAUCAGGAGUUUCUGAUUUUGCUCUGGUCCUGUUUCUUCAGAAGCAUGUCAUCUUUGCUCUGCCUUCUGCCCUUUGA
AGCAUGUGAUCUUUGUGACCUACUCCCUGUUCAUACACCCCUCCCCUUUUAAAAUCCCUAAUAAAAACUUGCUGGUUUUGUGGCUCAGG
GGGGCAUCAUGGACCUACCAAUACGUGAUGUCACCCCCGGUGGCCCAGCUGU

SEQ ID 24
GAGAUAGGAGAAAACUGCCUUAGGGCUGGAGGUGGGACAUGCUGGCGGCAAUACUGCUCUUUAAGGCAUUGAGAUGUUUAUGUAUAUGC
ACAUCAAAAGCACAGCACUUUUUUCUUUACCUUGUUUAUGAUGCAGAGACAUUUGUUCACAUGUUUCCUGCUGGCCCUCUCCCCACUA
UUACCCUAUUGUCCUGCCACAUCCCCCUCUCCGAGAUGGUAGAGAUAAUGAUCAAUAAAUACUGAGGGAACUCAGAGACCGGUGCGGCG
CGGGUCCUCCAUAUGCUGAGCGCCGGUCCCCUGGGCCCACUUUUCUUUCUCUAUACUUUGUCUCUGUUGUCUUUCUUUUCUCAAGUCUC
UCGUUCCACCUGAGGAGAAAUGCCCACAGCUGUGGAGGCGCAGGCCACUCCAUCUGGUGCCCAACGUGGAUGCUUUUCUCUAGGGUGAA
GGGACUCUCGAGUGUGGUCAUUGAGGACAAGUCAACGAGAGAUUCCCGAGUACGUCUACAGUGAGCCUUGUGUCUCUCAUCC

SEQ ID 25
GAGAUAGGAGAAAACUGCCUUAGGGCUGGAGGUGGGACAUGCUGGCGGCAAUACUGCUCUUUAAGGCAUUGAGAUGUUUAUGUAUAUGC
ACAUCAAAAGCACAGCACUUUUUUCUUUACCUUGUUUAUGAUGCAGAGACAUUUGUUCACAUGUUUCCUGCUGGCCCUCUCCCCACUA
UUACCCUAUUGUCCUGCCACAUCCCCCUCUCCGAGAUGGUAGAGAUAAUGAUCAAUAAAUACUGAGGGAACUCAGAGACCGGUGCGGCG
CGGGUCCUCCAUAUGCUGAGCGCCGGUCCCCUGGGCCCACUUUUCUUUCUCUAUACUUUGUCUCUGUUGUCUUUCUUUUCUCAAGUCUC
UCGUUCCACCUGAGGAGAAAUGCCCACAGCUGUGGAGGCGCAGGCCACUCCAUCUGGUGCCCAACGUGGAUGCUUUUCUCUAGGGUGAA
GGGACUCUCGAGUGUGGUCAUUGAGGACAAGUCAACGAGAGAUUCCCGAGUACGUCUACAGUGAGCCUUGUGGGUGAAGGUA

SEQ ID 26
GAGAUAGGAGAAAACUGCCUUAGGGCUGGAGGUGGGACAUGCUGGCGGCAAUACUGCUCUUUAAGGCAUUGAGAUGUUUAUGUAUAUGC
ACAUCAAAAGCACAGCACUUUUUUCUUUACCUUGUUUAUGAUGCAGAGACAUUUGUUCACAUGUUUCCUGCUGGCCCUCUCCCCACUA

UUACCCUAUUGUCCUGCCACAUCCCCCUCUCCGAGAUGGUAGAGAUAAUGAUCAAUAAAUACUGAGGGAACUCAGAGACCGGUGCGGCG
CGGGUCCUCCAUAUGCUGAGCGCCGGUCCCCUGGGCCCACUUUUCUUUCUCUAUACUUUGUCUCUGUGUCUUUCUUUUCUCAAGUCUC
UCGUUCCACCUGAGGAGAAAUGCCCACAGCUGUGGAGGCGCAGGCCACUCCAUCUGGUGCCCAACGUGGAUGCUUUUCUCUAGGGUGAA
GGGACUCUCGAGUGUGGUCAUUGAGGACAAGUCAACGAGAGAUUCCCGAGUACGUCUACAGUGAGCCUUGUG
SEQ ID 27
UCUCUCAUCC
SEQ ID 28
GGUGAAGGUA
SEQ ID 29
UGUAAGGAAAAGAGAGAUCAGACUUUCACUGUGUCUAUGUAGAAAAGGAAGACAUAAGAAACUCCAUUUUGAUCUGUACUAAGAAAAAU
UGUUUUGCCUUGAGAUGCUGUUAAUCUGUAACUUUAGCCCCAACCCUGUGCUCACGGAAACAUGUGCUGUAAGGUUUAAGGGAUCUAGG
GCUGUGCAGGAUGUACCUUGUUAACAAUAUGUUUGCAGGCAGUAUGUUUGGUAAAAGUCAUCGCCAUUCUCCAUUCUCGAUUAACCAGG
GGCUCAAUGCACUGUGGAAAGCCACAGGAACCUCUGCCCAAGAAAGCCUGGCUGUUGUGGGAAGUCAGGGACCCCGAAUGGAGGGACCA
GCUGGUGCUGCAUCAGGAAACAUAAAUUGUGAAGAUUUCUUGGACAUUUAUCAGUUUCCAAAAUUAAUACUUUUAUAAUUUCUUACACC
UGUCUUACUUUAAUCUCUUAAUCCUGUUAUCUUUGUAAGCUGAGGAUAUACGUCACCUCAGGACCACUAUUGUACAAAUUGAUUGUAAA
ACAUGUUCACAUGUGUUUGAACAAUAUGAAAUCAGUGCACCUUGAAAAUGAACAGAAUAACAGUGAUUUUAGGGAACAAAGGAAGACAA
CCAUAAGGUCUGACUGCCUGAGGGGUCGGGCAAAAAGCCAUAUUUUUCUUCUUGCAGAGAGCCUAUAAAUGGACGUGCAAGUAGGAGAG
AUAUUGCUAAAUUCUUUUCCUAGCAAGGAAUAUAAUACUAAGACCCUAGGGAAAGAAUUGCAUUCCUGGGGGGAGGUCUAUAAACGGCC
GCUCUGGGAGUGUCUGUCCUAUGUGGUUGAGAUAAGGACUGAGAUACGCCCUGGUCUCCUGCAGUACCCUCAGGCUUACUAGGAUUGGG
AAACCCCAGUCCUGGUAAAUUUGAGGUCAGGCCGGUUCUUUGCUCUGAACCCUGUUUUCUGUUAAGAUGUUUAUCAAGACAAUACAUGC
ACCGCUGAACAUAGACCCUUAUCAGGAGUUUCUGAUUUUGCUCUGGUCCUGUUUCUUCAGAAGCAUGUCAUCUUUGCUCUGCCUUCUGC
CCUUUGAAGCAUGUGAUCUUUGUGACCUACUCCCUGUUCAUACACCCCUCCCCUUUUAAAAUCCCUAAUAAAAACUUGCUGGUUUUGUG
GCUCAGGGGGGCAUCAUGGACCUACCAAUACGUGAUGUCACCCCCGGUGGCCCAGCUGU
SEQ ID 30
UGUAAGGAAAAGAGAGAUCAGACUUUCACUGUGUCUAUGUAGAAAAGGAAGACAUAAGAAACUCCAUUUUGAUCUGUACUAAGAAAAAU
UGUUUUGCCUUGAGAUGCUGUUAAUCUGUAACUUUAGCCCCAACCCUGUGCUCACGGAAACAUGUGCUGUAAGGUUUAAGGGAUCUAGG
GCUGUGCAGGAUGUACCUUGUUAACAAUAUGUUUGCAGGCAGUAUGUUUGGUAAAAGUCAUCGCCAUUCUCCAUUCUCGAUUAACCAGG
GGCUCAAUGCACUGUGGAAAGCCACAGGAACCUCUGCCCAAGAAAGCCUGGC
SEQ ID 31
UGUUGUGGGAAGUCAGGGACCCCGAAUGGAGGGACCAGCUGGUGCUGCAUCAGGAAACAUAAAUUGUGAAGAUUUCUUGGACAUUUAUC
AGUUUCCAAAAUUAAUACUUUUAUAAUUUCUUACACCUGUCUUACUUUAAUCUCUUAAUCCUGUUAUCUUUGUAAGCUGAGGAUAUACG
UCACCUCAGGACCACUAUUGUACAAAUUGAUUGUAAAACAUGUUCACAUGUGUUUGAACAAUAUGAAAUCAGUGCACCUUGAAAAUGAA
CAGAAUAACAGUGAUUUUAGGGAACAAAGGAAGACAACCAUAAGGUCUGACUGCCUGAGGGGUCGGGCAAAAAGCCAUAUUUUUCUUCU
UGCAGAGAGCCUAUAAAUGGACGUGCAAGUAGGAGAGAUAUUGCUAAAUUCUUUUCCUAGCAAGGAAUAUAAUACUAAGACCCUAGGGA
AAGAAUUGCAUUCCUGGGGGGAGGUCUAUAAACGGCCGCUCUGGGAGUGUCUGUCCUAUGUGGUUGAGAUAAGGACUGAGAUACGCCCU
GGUCUCCUGCAGUACCCUCAGGCUUACUAGGAUUGGGAAACCCCAGUCCUGGUAAAUUUGAGGUCAGGCCGGUUCUUUGCUCUGAACCC
UGUUUUCUGUUAAGAUGUUUAUCAAGACAAUACAUGCACCGCUGAACAUAGACCCUUAUCAGGAGUUUCUGAUUUUGCUCUGGUCCUGU
UUCUUCAGAAGCAUGUCAUCUUUGCUCUGCCUUCUGCCCUUUGAAGCAUGUGAUCUUUGUGACCUACUCCCUGUUCAUACACCCCUCCC
CUUUUAAAAUCCCUAAUAAAAACUUGCUGGUUUUGUGGCUCAGGGGGGCAUCAUGGACCUACCAAUACGUGAUGUCACCCCCGGUGGCC
CAGCUGU
SEQ ID 32
TTAAAAGAATTTTTTTTTCTTTGAGACAGAGTCTCGCTCTGTCGCCCAGGCTGGAGTGCAGTGGCGTGATCTCAGCTCACTGCAAGTT
CCGCCTCCTGGGTTTACACCATTCTCCTGCCTCAGCCTCCCAAGTAGTTGGGACTACAGGAGCCCACCACCATGCCTGGCTAATTTTTT
TTGGGTTTTTAATAGAGATGGAGTTTCACCGTGTTAGCCAGGATGGTCTCGATCTCCTGACCTTGTGATCTGCCCACCTTGGCCTCCCA
AAGTGCTGGGATTACAGTCGTGAGCCACCGTGCCCAGCCA
SEQ ID 33
CATTTCAAAA
SEQ ID 34
AGAAAAAATT
SEQ ID 35
TTAAAAGAAT

SEQ ID 36
CATTTCAAAATTAAAAGAAT
SEQ ID 37
TGTTACACCACAAGCCTATAATGAGTCTGAGCATCACTGGGACATGGTTAGATGCCATCTGCAAGGAGGAGAAGATAATCTTACTTTAG
ACATTTCAAAA
SEQ ID 38
TGTTACACCACAAGCCTATAATGAGTCTGAGCATCACTGGGACATGGTTAGATGCCATCTGCAAGGAGGAGAAGATAATCTTACTTTAG
ACATTTCAAAATTAAAAGAATTTTTTTTTCTTTGAGACAGAGTCTCGCTCTGTCGCCCAGGCTGGAGTGCAGTGGCGTGATCTCAGCT
CACTGCAAGTTCCGCCTCCTGGGTTTACACCATTCTCCTGCCTCAGCCTCCCAAGTAGTTGGGACTACAGGAGCCCACCACCATGCCTG
GCTAATTTTTTTTGGGTTTTTAATAGAGATGGAGTTTCACCGTGTTAGCCAGGATGGTCTCGATCTCCTGACCTTGTGATCTGCCCACC
TTGGCCTCCCAAAGTGCTGGGATTACAGTCGTGAGCCACCGTGCCCAGCCA
SEQ ID 39
AAAATTAA
SEQ ID 40
AGAAAAAAATTTTTGAGGCATCAAAAGCCCATTTAAATTTGGTGCCAGGAACGGAGACAATCGTGAAAGCTGCTGATAGCCTCACAAATC
TTAAGCCAGTC
SEQ ID 41
TGCCCAGCCA
SEQ ID 42
TGCCCAGCCAAGAAAAAAATTTTTGAGGCATCAAAAGCCCATTTAAATTTGGTGCCAGGAACGGAGACAATCGTGAAAGCTGCTGATAGC
CTCACAAATCTTAAGCCAGTC
SEQ ID 43
TTAAAAGAATTTTTTTTTCTTTGAGACAGAGTCTCGCTCTGTCGCCCAGGCTGGAGTGCAGTGGCGTGATCTCAGCTCACTGCAAGTT
CCGCCTCCTGGGTTTACACCATTCTCCTGCCTCAGCCTCCCAAGTAGTTGGGACTACAGGAGCCCACCACCATGCCTGGCTAATTTTTT
TTGGGTTTTTAATAGAGATGGAGTTTCACCGTGTTAGCCAGGATGGTCTCGATCTCCTGACCTTGTGATCTGCCCACCTTGGCCTCCCA
AAGTGCTGGGATTACAGTCGTGAGCCACCGTGCCCAGCCAAGAAAAAAATTTTTGAGGCATCAAAAGCCCATTTAAATTTGGTGCCAGGA
ACGGAGACAATCGTGAAAGCTGCTGATAGCCTCACAAATCTTAAGCCAGTC
SEQ ID 44
GCCAAGAA
SEQ ID 45
TGCCCAGCCAAGAAAAAAATT
SEQ ID 46
TCTCTCATCCCTCCTGACGAGAAATACCCACAGGTGTGGAGGGGCTGGCCCCCTTCATCTGATGCCCAATGTGGGTGCCTTTCTCTAGG
GTGAAGGTACT
SEQ ID 47
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGTCTCTCATCCCTCCTGA
CGAGAAATACCCACAGGTGTGGAGGGGCTGGCCCCCTTCATCTGATGCCCAATGTGGGTGCCTTTCTCTAGGGTGAAGGTACT
SEQ ID 48
GGTGAAGGTACTCTACAGTGTGGTCATTGAGGACAAGTTGACGAGAGAGTCCCAAGTACGTCCACGGTCAGCCTTGCGGTAAGCTTGTG
TGCTTAGAGGA
SEQ ID 49
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC

TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGGGTGAAGGTACTCTACA
GTGTGGTCATTGAGGACAAGTTGACGAGAGAGTCCCAAGTACGTCCACGGTCAGCCTTGCGGTAAGCTTGTGTGCTTAGAGGA
SEQ ID 50
GAGCCTTGTGTCTCTCATCC
SEQ ID 51
GAGCCTTGTGGGTGAAGGTA
SEQ ID 52
AAAGCCTGGCTGTTGTGGGA
SEQ ID 53
ACAGCGATGGCGTCTAATTCACCAGCAACACAGGACGCGGCGCTGTAT
SEQ ID 54
MGQTESKYASYLSFIKILLRRGGVRASTENLITLFQTIEQFCPWFPEQGTLDLKDWEKIGKELKQANREGKIIPLTVWNDWAIIKATLE
PFQTGEDIVSVSDAPKSCVTDCEEEAGTESQQGTESSHCKYVAESVMAQSTQNVDYSQLQEIIYPESSKLGEGGPESLGPSEPKPRSPS
TPPPVVQMPVTLQPQTQVRQAQTPRENQVERDRVSIPAMPTQIQYPQYQPVENKTQPLVVYQYRLPTELQYRPPSEVQYRPQAVCPVPN
STAPYQQPTAMASNSPATQDAALYPQPPTVRLNPTASRSGQGGALHAVIDEARKQGDLEAWRFLVILQLVQAGEETQVGAPARAETRCE
PFTMKMLKDIKEGVKQYGSNSPYIRTLLDSIAHGNRLTPYDWEILAKSSLSSSQYLQFKTWWIDGVQEQVRKNQATKPTVNIDADQLLG
TGPNWSTINQQSVMQNEAIEQVRAICLRAWGKIQDPGTAFPINSIRQGSKEPYPDFVARLQDAAQKSITDDNARKVIVELMAYENANPE
CQSAIKPLKGKVPAGVDVITEYVKACDGIGGAMHKAMLMAQAMRGLTLGGQVRTFGKKCYNCGQIGHLKRSCPGLNKQNIINQAITAKN
KKPSGLCPKCGKAKHWANQCHSKFDKDGQPLSGNRKRGQPQAPQQTGAFPVKLFVPQGFQGQQPLQKIPPLQGVSQLQQSNSCPAPQQA
APQ
SEQ ID 55
TQVRQAQTPRENQVERDRVSIPA
SEQ ID 56
PTAMASNSPATQDAALY
SEQ ID 57
ATGGGGCAAACTGAAAGTAAATATGCCTCTTATCTCAGCTTTATTAAAATTCTTTTAAGAAGAGGGGGAGTTAGAGCTTCTACAGAAAA
TCTAATTACGCTATTTCAAACAATAGAACAATTCTGCCCATGGTTTCCAGAACAGGGAACTTTAGATCTAAAAGATTGGGAAAAAATTG
GCAAAGAATTAAAACAAGCAAATAGGGAAGGTAAAATCATCCCACTTACAGTATGGAATGATTGGGCCATTATTAAAGCAACTTTAGAA
CCATTTCAAACAGGAGAAGATATTGTTTCAGTTTCTGATGCCCCTAAAAGCTGTGTAACAGATTGTGAAGAAGAGGCAGGGACAGAATC
CCAGCAAGGAACGGAAAGTTCACATTGTAAATATGTAGCAGAGTCTGTAATGGCTCAGTCAACGCAAAATGTTGACTACAGTCAATTAC
AGGAGATAATATACCCTGAATCATCAAAATTGGGGGAAGGAGGTCCAGAATCATTGGGGCCATCAGAGCCTAAACCACGATCGCCATCA
ACTCCTCCTCCCGTGGTTCAGATGCCTGTAACATTACAACCTCAAACGCAGGTTAGACAAGCACAAACCCCAAGAGAAAATCAAGTAGA
AAGGGACAGAGTCTCTATCCCGGCAATGCCAACTCAGATACAGTATCCACAATATCAGCCGGTAGAAAATAAGACCCAACCGCTGGTAG
TTTATCAATACCGGCTGCCAACCGAGCTTCAGTATCGGCCTCCTTCAGAGGTTCAATACAGACCTCAAGCGGTGTGTCCTGTGCCAAAT
AGCACGGCACCATACCAGCAACCCACAGCGATGGCGTCTAATTCACCAGCAACACAGGACGCGGCGCTGTATCCTCAGCCGCCCACTGT
GAGACTTAATCCTACAGCATCACGTAGTGGACAGGGTGGTGCACTGCATGCAGTCATTGATGAAGCCAGAAAACAGGGCGATCTTGAGG
CATGGCGGTTCCTGGTAATTTTACAACTGGTACAGGCCGGGGAAGAGACTCAAGTAGGAGCGCCTGCCCGAGCTGAGACTAGATGTGAA
CCTTTCACCATGAAAATGTTAAAAGATATAAAGGAAGGAGTTAAACAATATGGATCCAACTCCCCTTATATAAGAACATTATTAGATTC
CATTGCTCATGGAAATAGACTTACTCCTTATGACTGGGAAATTTTGGCCAAATCTTCCCTTTCATCCTCTCAGTATCTACAGTTTAAAA
CCTGGTGGATTGATGGAGTACAAGAACAGGTACGAAAAAATCAGGCTACTAAGCCCACTGTTAATATAGACGCAGACCAATTGTTAGGA
ACAGGTCCAAATTGGAGCACCATTAACCAACAATCAGTGATGCAGAATGAGGCTATTGAACAAGTAAGGGCTATTTGCCTCAGGGCCTG
GGGAAAAATTCAGGACCCAGGAACAGCTTTCCCTATTAATTCAATTAGACAAGGCTCTAAAGAGCCATATCCTGACTTTGTGGCAAGAT
TACAAGATGCTGCTCAAAAGTCTATTACAGATGACAATGCCCGAAAAGTTATTGTAGAATTAATGGCCTATGAAAATGCAAATCCAGAA
TGTCAGTCGGCCATAAAGCCATTAAAAGGAAAAGTTCCAGCAGGAGTTGATGTAATTACAGAATATGTGAAGGCTTGTGATGGGATTGG
AGGAGCTATGCATAAGGCAATGCTAATGGCTCAAGCAATGAGGGGGCTCACTCTAGGAGGACAAGTTAGAACATTTGGGAAAAAATGTT
ATAATTGTGGTCAAATCGGTCATCTGAAAAGGAGTTGCCCAGGCTTAAATAAACAGAATATAATAAATCAAGCTATTACAGCAAAAAAT
AAAAAGCCATCTGGCCTGTGTCCAAAATGTGGAAAAGCAAAACATTGGGCCAATCAATGTCATTCTAAATTTGATAAAGATGGGCAACC
ATTGTCTGGAAACAGGAAGAGGGGCCAGCCTCAGGCCCCCCAACAAACTGGGGCATTCCCAGTTAAACTGTTTGTTCCTCAGGGTTTTC
AAGGACAACAACCCCTACAGAAAATACCACCACTTCAGGGAGTCAGCCAATTACAACAATCCAACAGCTGTCCCGCGCCACAGCAGGCA
GCACCGCAG

SEQ ID 58
TGGGCAACCATTGTCTGGAAACAGGAAGAGGGGCCAGCCTCAGGCCCCCCAACAAACTGGGGCATTCCCAGTTAAACTGTTTGTTCCTC
AGGGTTTTCAAGGACAACAACCCCTACAGAAAATACCACCACTTCAGGGAGTCAGCCAATTACAACAATCCAACAGCTGTCCCGCGCCA
CAGCAGGCAGCACCGCAGTAGATTTATGTTCCACCCAAATGGTCTTTTTACTCCCTGGAAAGCCCCCACAAAAGATTCCTAGAGGGGTA
TATGGCCCGCTGCCAGAAGGGAGGGTAGGCCTTTGAGGGAGATCAAGTCTAAATTTGAAGGGAGTCCAAATTCATACTGGGGTAATTTA
TTCAGATTATAAAGGGGGAATTCAGTTAGTGATCAGCTCCACTGTTCCCCGGAGTGCCAATCCAGGTGATAGAATTGCTCAATTACTGC
TTTTGCCTTATGTTAAAATTGGGGAAAACAAAAAGGAAAGAACAGGAGGGTTTGGAAGTACCAACCCTGCAGGAAAAGCTGCTTATTGG
GCTAATCAGGTCTCAGAGGATAGACCCGTGTGTACAGTCACTATTCAGGGAAAGAGTTTGAAGGATTAGTGGATACCCAGGCTGATGTT
TCTGTCATCGGCATAGGTACTGCCTCAGAAGTGTATCAAAGTGCCATGATTTTACATTGTCCAGGATCTGATAATCAAGAAAGTACGGT
TCAGCCTGTGATCACTTCATTCCAATCAATTTATGGGGCCGAGACTTGTTACAACAATGGCATGCAGAGATTACTATCCCAGCCTCCCT
ATACAGCCCCAGGAATAAAAAAATCATGACTAAAATGGGATAGCTCCCTAAAAAGGGACTAGGAAAGAAGTCCCAATTGAGGCTGAAAA
AAATCAAAAAAGAAAAGGAATAGGGCATCCTTTTTAG
SEQ ID 59
WATIVWKQEEGPASGPPTNWGIPS
SEQ ID 60
TVCSSGFSRTTTPTENTTTSGSQPITTIQQLSRATAGSTAVDLCSTQMVFLLPGKPPQKIPRGVYGPLPEGRVGL
SEQ ID 61
GRSSLNLKGVQIHTGVIYSDYKGGIQLVISSTVPRSANPGDRIAQLLLLPYVKIGENKKERTGGFGSTNPAGKAAYWANQVSEDRPVCT
VTIQGKSLKDVDTQADVSVIGIGTASEVYQSAMILHCPGSDNQESTVQPVITSFIPINLWGRDLLQQWHAEITIPASLYSPRNKKIMTK
MG
SEQ ID 62
LPKKGLGKKEVPIEAEKNQKRKGIGHPF
SEQ ID 63
ACATCCAGAATTAGGAAGCTTACTGTGGCCTCACACCACATTAGAATTTGTTCTGGAAATCAAGCTATAGGAACAAGAGATCGTAAGTC
ATATTATACTATCAACCTAAATTCCAGTCTGACAATTCCTTTGCAAAATTGTGTAAAACTCCCTTATATTGCTAGTTGTAGGAAAAACA
TAGTTATTAAACCTGATTCCCAAACCATAATCTGTGAAAATTGTGGAATGTTTACTTGCATTGATTTGACTTTTAATTGGCAGCACCGT
ATTCTACTAGGAAGAGCAAGAGAGGGTGTGTGGATCCTTGTGTCCATGGACCGACCATGGGAGGCTTCGCTATCCATCCATATTTTAAC
GGAAGTATTAAAAGGAATTCTAACTAGATCCAAAAGATTCATTTTTACTTTGATGGCAGTGATTATGGGCCTCATTGCAGTCACAGCTA
CTGCTGCGGCTGCTGGAATTGCTTTACACTCCTCTGTTCAAACTGCAGAATACGTAAATGATTGGCAAAAGAATTCCTCAAAATTGTGG
AATTCTCAGATCCAAATAGATCAAAAATTGGCAAACCAAATTAATGATCTTAGACAAACTGTCATTTGGATGGGAGAGGCTCATGAGCT
TGGAATATCTTTTTCAGTTACGATGTGA
SEQ ID 64
TSRIRKLTVASHHIRICSGNQAIGTRDRKSYYTINLNSSLTIPLQNCVKLPYIASCRKNIVIKPDSQTIICENCGMFTCIDLTFNWQHR
ILLGRAREGVWILVSMDRPWEASLSIHILTEVLKGILTRSKRFIFTLMAVIMGLIAVTATAAAAGIALHSSVQTAEYVNDWQKNSSKLW
NSQIQIDQKLANQINDLRQTVIWMGEAHELGISFSVTM
SEQ ID 65
HPELGSLLWPHTTLEFVLEIKL
SEQ ID 66
EQEIVSHIILST
SEQ ID 67
IPV
SEQ ID 68
QFLCKIV
SEQ ID 69
NSLILLVVGKT
SEQ ID 70
LLNLIPKP
SEQ ID 71
SVKIVECLLALI

SEQ ID 72
LLIGSTVFY
SEQ ID 73
EEQERVCGSLCPWTDHGRLRYPSIF
SEQ ID 74
RKY
SEQ ID 75
KEF
SEQ ID 76
LDPKDSFLL
SEQ ID 77
WQ
SEQ ID 78
LWASLQSQLLLRLLELLYTPLFKLQNT
SEQ ID 79
MIGKRIPQNCGILRSK
SEQ ID 80
IKNWQTKLMILDKLSFGWERLMSLEYLFQLRC
SEQ ID 81
GTACAAAACAATGAATTTTGAACTATGATAGACTGGGTCCCTTGAGGCCAATTATATCATAACTGTACAGGCCAGACTCATTCATGTTC
ACAGGCCCCATCCATCTGGCCCATTAATCCAGCCTATGACGGTGATGTAACTGAAAGGCTGGACCAGGTTTATAGAAGGTTAGAATCAC
TCTGTCCAAGGAAATGGGGTGAAAAGGGAATTTCATCACCTTGACCAAAGTTAGTCCTGTTACTGGTCCTG
SEQ ID 82
VQNNEF
SEQ ID 83
TMIDWVP
SEQ ID 84
GQLYHNCTGQTHSCSQAPSIWPINPAYDGDVTERLDQVYRRLESLCPRKWGEKGISSP
SEQ ID 85
PKLVLLLVL
SEQ ID 86
ATGTCAAATTTGTTCTCTTTCTTAAGAGGGGACTCGGAATTAAATAGTGAAAGAACGTTAACTCCAGAGGCAACTAAAGAAATTAAATT
AATTGAAGAAAAAATTCGGTCAGCACAAGTAAATAGAATAGATCACTTGGCCCCACTCCAAATTTTGATTTTTGCTACTGCACATTCCC
TAACAGGCATCATTGTTCAAAATACAGATCTTGTGGAGTGGTCCTTCCTTCCTCACAGTACAATTAAGACTTTTACATTGTACTTGGAT
CAAATGGCTACATTAATTGGTCAGGGAAGATTATGAATAATAACATTGTGTGGAAATGACCCAGATAAAATCACTGTTCCTTTCAACAA
GCAACAGGTTAGACAAGCCTTTATCAATTCTGGTGCATGGCAGATTGGTCTTGCCGATTTTGTGGGAATTATTGACAATCGTTACCCCA
AAACAAAAATCTTCCAGTTTTTAAAATTGACTACTTGGATTTTACCTAAAGTTACCAAACATAAGCCTTTAAAAAATGCTCTGGCAGTG
TTTACTGATGGTTCCAGCAATGGAAAAGTGGCTTACACCGGGCCAAAAGAATGAGTCATCAAAACTCAGTATCACTTGACTCAAAGAGC
AGAGTTGGTTGCCGTCATTACAGTGTTAACAAGATTTTAATCAGTCTATTAACATTGTATCAGATTCTGCATATGTAGTACAGGCTACA
AAGGATATTGAGAGAGCCCTAATCAAATACATTATGGATGATCAGTTAAACCCGCTGTTTAATTTGTTACAACAAAATGTAAGAAAAAG
AAATTTCCCATTTTATATTACTCATATTCGAGCACACACTAATTTACCAGGGCCTTTAACTAAAGCAAATGAACAAGCTGACTTGCTAG
TATCATCTGCATTCATGGAAGCACAAGAACTTCATGCCTTGACTCATGTAAATGCAATAGGATTAAAAAATAAATTTGATATCACATGG
AAACAGACAAAAAATATTGTACAACATTGCACCCAGTGTCAGATTCTACACCTGGCCACTCAGGAGGCAAGAGTTAATCCCAGAGGTCT
ATGTCCTAATGTGTTATGGCAAATGGATGTCATGCACGTACCTTCATTTGGAAAATTGTCATTTGTCCATGTGACAGTTGATACTTATT
CACATTTCATATGGGCAACCTGCCAGACAGGAGAAAGTACTTCCCATGTTAAAAGACATTTATTATCTTGTTTTCCTGTCATGGGAGTT
CCAGAAAAAGTTAAAACAGACAATGGGCCAGGTTACTGTAGTAAAGCAGTTCAAAAATTCTTAAATCAGTGGAAAATTACACATACAAT
AGGAATTCCTTATAATTCCCAAGGACAGGCCATAATTGAAAGAACTAATAGAACACTCAAAGCTCAATTGGTTAAACAAAAAAAAGGAA
AAGACAGGAGTATAACACTCCCCAGATGCAACTTAATCTAGCACTCTATACTTTAAATGTTTTAAACATTTATAGAAATCAGACCACTA
CCTCTGCAGAACAACATCTTACTGGTAAAAGGAACAGCCCACATGAAGGAAAACTGATTTGGTGGAAAGATAATAAAAATAAAACATGG
GAAATGGGGAAGGTGATAACGTGGGGGAGAGGTTTTGCTTGTGTTTCACCAGGAGAAAATCAGCTTCCTGTTTGGATACCCACTAGACA

TTTAAAGTTCTACAATGAACTCACTGGAGATGCAAAGAAAAGTGTGGAGATGGAGACACCCCAATCGACTCGCCAGGTAAACAAAATGG
TGATATCAGAAGAACAGAAAAAGTTGCCTTCCATCAAGGAAGCAGAGTTGCCAATATAG
SEQ ID 87
MNSLEMQRKVWRWRHPNRLASLQVYPAAPKRQQPARMGHSDDGGFVKKKRGGYVRKREIRLSLCLCRKGRHKKLHFDLY
SEQ ID 88
ATGAACTCACTGGAGATGCAAAGAAAAGTGTGGAGATGGAGACACCCCAATCGACTCGCCAGTCTACAGGTGTATCCAGCAGCTCCAAA
GAGACAGCAACCAGCAAGAATGGGCCATAGTGACGATGGTGGTTTTGTCAAAAAGAAAAGGGGGGGATATGTAAGGAAAAGAGAGATCA
GACTTTCACTGTGTCTATGTAGAAAAGGAAGACATAAGAAACTCCATTTTGATCTGTAC
SEQ ID 89
GGCCCACTATTGTACAAATTGATTGTAAAACATGTTCACATGTGTTGAACAATATNAAATCAGGGCCCNTTGAAAATGAACAGAATAAC
AGTGATTTTAGGGAACAAAGGAAGNCANCCATAAGGTCTGCCTGCCTGAGGGGTCGGGCAAAAACCCATATTTTTNTTNTTGCAGAGAG
CCTATAAATGGACGTGCAAGTAGGANAGANATTGCTAAATTCTTTTCCTAGCAAGGAATATAATACTAAGNCCCTAGGGAAAGAATTGC
ATTCCTGGGGGGAGGTNTATAAACGGCCGCTNTGGGAGTGTCTGTCCTATGTGGTTGANANAAGGACTGANANACGCCCTGGTCGCCTG
CAGTACCCTCAGGCTTACTAGGATTGGGAAACCCCAGTCCTGGTAAATTTGAGGTCAGGCCGGTTNTTTGCTNTGAACCCTGTTTTNTG
TTAANATGTTTATCAAGACAATACGTGCNCCGCTGAACATAGACCCTTATCAGGAGTTTNTGATTTTGCTCTGGTCCTGTTTNTTCANA
AGCATGTCATCTTTGCTCTGCCTTNTGCCCTTTGAAGCATGTGATCTTTGTGACCTACTCCCTGTTCATACACCCCTCCCCTTTTAAAA
TCCCTAATAAAAACTTGCTGGTTTTGTGGCTCAGGGGGGCATCATGGACCTACCAATACGTGATGTCACCCCCGGTGGCCCAGCTGTAA
AAAAAAAAAAA
SEQ ID 90
CNNGNNCCAAAATTTTGATTTTGNAAAAAAAAATTTTTCCCCCATTGTGGTTTTGGNCCCAAATAATNAAAAACCCGGNGGCCCCNTTT
GAAAAAATGGNCNCANAAATTNCCCGGGTNTTTTTTAGGGAACAAANGGGAAGCCCCCCCAATAAGGGTNTNNCCTCCCNTGAGGGGG
TGGGGGAAAAAAACCCAATTTTTTTTTTTTTTGCNNNAGAGCCTTAAAAATGGCGGTGCAAGTAGNAAAAANATTGNTAAATTNTTTTC
CCAGCAAGGAANATAATTNTAAGCCCCTAGGGAAAAAATTGCNTTNTTGGGGGGAGGTTTATAAACGNCCNCTCTGGGAGTGTNTGTCN
TATGTGGTTGANANAAGGATTNAAANACNCCNTGGTCGCCTGNAGNACCCTCNGGCTTATTAGGATTGGGAAACCCCAGTCNTGGTAAA
TTTNAGGTCNGGCNGGTTTTTTNNTTTNANCCCTGTTTTTTGTTAANATNTTTATCAANACAANANGNGCCCCGNTGAANANANACCCT
TATCAGGAGTTTNTGATTTTGCTCGGGTCCTGTTTNTTCAAAAGCATGTCATTTTTGNTTTGCCTTNTGCCCTTTNAAGNATGTGATNT
TTGTGACCTACTCCCTGTTCANACNCCCCNCCCCTTTTAAAATCCCTAATAAAAACTTGCTGGTTTTGTGGCTCAGGGGGGCATCATGG
ACCTACCAANACGTGATGTCNCCCCCGGTGGCCCAGCTGTAAAAAAAAAAAAA
SEQ ID 91
ANCCCANTTTTTGGTNNCAAAATTTGAATTGTAAAAAACAATGGTTNCCACCATTGTTGTTTTGGANCCAAATATTGNAAANTCCAGNG
GCNCCCTTNGAAAAATGGAANCANGAAATAANCCAGGTGNTTTTTTAGGGGGAACCAAAAGGAAAGNCCACCCCATAAAGGGTNTTGAC
TTGCCTTGAGGGGGTCGGGGGCAAAAAAAGCCAATATTTTTNTTTNTTTGCAGAGAGCCTATAAATGGACGTGCAAAGTAGGAAAAATA
TTGCTAAATTCTTTTCCTAGCAAGGAATATAATATTAAGNCCCTAGGAAAGAATTGCATTCCTGGGGGGAGGTCTATAAACGGCCGCTC
TGGGAGTGTCTGTCCTATGTGGTTAAGANAAGGATTGAGANACGCCCCTGGTCGCCTGCAGTACCCTCAGGCTTANTAGGATTGGGAAA
CCCCAGTCCTGGTAAATTTGAGGTCAGGCCGGTTNTTTGCTTTGAACCCTGTTTTNTGTTAANATGTTTATCAAGACAANACGTGCNCC
GCTGAACANANACCCTTATCAGGAGTTTCTGATTTTGCTCTGGTCCTGTTTNTTCANAAGCATGTCATCTTTGCTNTGCCTTCTGCCCT
TTGAAGCATGTGATNTTTGTGACCTACTCCCTGTTCATACACCCCTCCCCTTTTAAAATCCCTAATAAAAACTTGCTGGTTTTGTGGCT
CAGGGGGGCATCATGGACCTACCAATACGTGATGTCACCCCCGGTGGCCCAGCTGTAAAAAAAAAAAA
SEQ ID 92
ESSKLSIT
SEQ ID 93
LKEQSWLPSLQC
SEQ ID 94
QDFNQSINIVSDSAYVVQATKDIERALIKYIMDDQLNPLFNLLQQNVRKRNFPFYITHIRAHTNLPGPLTKANEQADLLVSSAFMEAQE
LHALTHVNAIGLKNKFDITWKQTKNIVQHCTQCQILHLATQEARVNPRGLCPNVLWQMDVMHVPSFGKLSFVHVTVDTYSHFIWATCQT
GESTSHVKRHLLSCFPVMGVPEKVKTDNGPGYCSKAVQKFLNQWKITHTIGILYNSQGQAIIERTNRTLKAQLVKQKKGKDRSITLPRC
NLI
SEQ ID 95



SEQ ID 96
IITLCGNDPDKITVPFNKQQVRQAFINSGAWQIGLADFVGIIDNRYPKTKIFQFLKLTTWILPKVTKHKPLKNALAVFTDGSSNGKVAY
TGPKE
SEQ ID 97
TKKRKRQEYNTPQMQLNLALYTLNVLNIYRNQTTTSAEQHLTGKRNSPHEGKLIWWKDNKNKTWEMGKVITWGRGFACVSPGENQLPVW
IPTRHLKFYNELTGDAKKSVEMETPQSTRQVNKMVISEEQKKLPSIKEAELPI
SEQ ID 98

SEQ ID 99
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGTCTCTCATCCCTCCTGA
CGAGAAATACCCACAGGTGTGGAGGGGCTGGCCCCCTTCATCTGATGCCCAATGTGGGTGCCTTTCTCTAGGGTGAAGGTACTCTACAG
TGTGGTCATTGAGGACAAGTTGACGAGAGAGTCCCAAGTACGTCCACGGTCAGCCTTGCGACATTTAAAGTTCTACAATGAACTCACTG
GAGATGCAAAGAAAAGTGTGGAGATGGAGACACCCCAATCGACTCGCCAGTCTACAGGTGTATCCAGCAGCTCCAAAGAGACAGCAACC
AGCAAGAATGGGCCATAGTGACGATGGTGGTTTTGTCAAAAAGAAAAGGGGGGGATATGTAAGGAAAAGAGAGATCAGACTTTCACTGT
GTCTATGTAGAAAAGGAAGACATAAGAAACTCCATTTTGATCTGTACTAAGAAAAATTGTTTTGCCTTGAGATGCTGTTAATCTGTAAC
TTTAGCCCCAACCCTGTGCTCACGGAAACATGTGCTGTAAGGTTTAAGGGATCTAGGGCTGTGCAGGATGTACCTTGTTAACAATATGT
TTGCAGGCAGTATGTTTGGTAAAAGTCATCGCCATTCTCCATTCTCGATTAACCAGGGGCTCAATGCACTGTGGAAAGCCACAGGAACC
TCTGCCCAAGAAAGCCTGGCTGTTGTGGGAAGTCAGGGACCCCGAATGGAGGGACCAGCTGGTGCTGCATCAGGAAACATAAATTGTGA
AGATTTCTTGGACATTTATCAGTTTCCAAAATTAATACTTTTATAATTTCTTACACCTGTCTTACTTTAATCTCTTAATCCTGTTATCT
TTGTAAGCTGAGGATATACGTCACCTCAGGACCACTATTGTACAAATTGATTGTAAAACATGTTCACATGTGTTTGAACAATATGAAAT
CAGTGCACCTTGAAAATGAACAGAATAACAGTGATTTTAGGGAACAAAGGAAGACAACCATAAGGTCTGACTGCCTGAGGGGTCGGGCA
AAAAGCCATATTTTTCTTCTTGCAGAGAGCCTATAAATGGACGTGCAAGTAGGAGAGATATTGCTAAATTCTTTTCCTAGCAAGGAATA
TAATACTAAGACCCTAGGGAAAGAATTGCATTCCTGGGGGGAGGTCTATAAACGGCCGCTCTGGGAGTGTCTGTCCTATGTGGTTGAGA
TAAGGACTGAGATACGCCCTGGTCTCCTGCAGTACCCTCAGGCTTACTAGGATTGGGAAACCCCAGTCCTGGTAAATTTGAGGTCAGGC
CGGTTCTTTGCTCTGAACCCTGTTTTCTGTTAAGATGTTTATCAAGACAATACATGCACCGCTGAACATAGACCCTTATCAGGAGTTTC
TGATTTTGCTCTGGTCCTGTTTCTTCAGAAGCATGTCATCTTTGCTCTGCCTTCTGCCCTTTGAAGCATGTGATCTTTGTGACCTACTC
CCTGTTCATACACCCCTCCCCTTTTAAAATCCCTAATAAAAACTTGCTGGTTTTGTGGCTCAGGGGGGCATCATGGACCTACCAATACG
TGATGTCACCCCCGGTGGCCCAGCTGTAAAA
SEQ ID 100
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGTCTCTCATCCCTCCTGA
CGAGAAATACCCACAGGTGTGGAGGGGCTGGCCCCCTTCATCTGATGCCCAATGTGGGTGCCTTTCTCTAGGGTGAAGGTACTCTACAG
TGTGGTCATTGAGGACAAGTTGACGAGAGAGTCCCAAGTACGTCCACGGTCAGCCTTGCGGAGAAAATCAGCTTCCTGTTTGGATACCC
ACTAGACATTTAAAGTTCTACAATGAACTCACTGGAGATGCAAAGAAAAGTGTGGAGATGGAGACACCCCAATCGACTCGCCAGTCTAC
AGGTGTATCCAGCAGCTCCAAAGAGACAGCAACCAGCAAGAATGGGCCATAGTGACGATGGTGGTTTTGTCAAAAAGAAAAGGGGGGGA
TATGTAAGGAAAAGAGAGATCAGACTTTCACTGTGTCTATGTAGAAAAGGAAGACATAAGAAACTCCATTTTGATCTGTACTAAGAAAA
ATTGTTTTGCCTTGAGATGCTGTTAATCTGTAACTTTAGCCCCAACCCTGTGCTCACGGAAACATGTGCTGTAAGGTTTAAGGGATCTA
GGGCTGTGCAGGATGTACCTTGTTAACAATATGTTTGCAGGCAGTATGTTTGGTAAAAGTCATCGCCATTCTCCATTCTCGATTAACCA
GGGGCTCAATGCACTGTGGAAAGCCACAGGAACCTCTGCCCAAGAAAGCCTGGCTGTTGTGGGAAGTCAGGGACCCCGAATGGAGGGAC
CAGCTGGTGCTGCATCAGGAAACATAAATTGTGAAGATTTCTTGGACATTTATCAGTTTCCAAAATTAATACTTTTATAATTTCTTACA
CCTGTCTTACTTTAATCTCTTAATCCTGTTATCTTTGTAAGCTGAGGATATACGTCACCTCAGGACCACTATTGTACAAATTGATTGTA
AAACATGTTCACATGTGTTTGAACAATATGAAATCAGTGCACCTTGAAAATGAACAGAATAACAGTGATTTTAGGGAACAAAGGAAGAC
AACCATAAGGTCTGACTGCCTGAGGGGTCGGGCAAAAAGCCATATTTTTCTTCTTGCAGAGAGCCTATAAATGGACGTGCAAGTAGGAG

AGATATTGCTAAATTCTTTTCCTAGCAAGGAATATAATACTAAGACCCTAGGGAAAGAATTGCATTCCTGGGGGGAGGTCTATAAACGG
CCGCTCTGGGAGTGTCTGTCCTATGTGGTTGAGATAAGGACTGAGATACGCCCTGGTCTCCTGCAGTACCCTCAGGCTTACTAGGATTG
GGAAACCCCAGTCCTGGTAAATTTGAGGTCAGGCCGGTTCTTTGCTCTGAACCCTGTTTTCTGTTAAGATGTTTATCAAGACAATACAT
GCACCGCTGAACATAGACCCTTATCAGGAGTTTCTGATTTTGCTCTGGTCCTGTTTCTTCAGAAGCATGTCATCTTTGCTCTGCCTTCT
GCCCTTTGAAGCATGTGATCTTTGTGACCTACTCCCTGTTCATACACCCCTCCCCTTTTAAAATCCCTAATAAAAACTTGCTGGTTTTG
TGGCTCAGGGGGGCATCATGGACCTACCAATACGTGATGTCACCCCCGGTGGCCCAGCTGTAAAA
SEQ ID 101
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGTCTCTCATCCCTCCTGA
CGAGAAATACCCACAGGTGTGGAGGGGCTGGCCCCCTTCATCTGATGCCCAATGTGGGTGCCTTTCTCTAGGGTGAAGGTACTCTACAG
TGTGGTCATTGAGGACAAGTTGACGAGAGAGTCCCAAGTACGTCCACGGTCAGCCTTGCGTCTACAGGTGTATCCAGCAGCTCCAAAGA
GACAGCAACCAGCAAGAATGGGCCATAGTGACGATGGTGGTTTTGTCAAAAAGAAAAGGGGGGGATATGTAAGGAAAAGAGAGATCAGA
CTTTCACTGTGTCTATGTAGAAAAGGAAGACATAAGAAACTCCATTTTGATCTGTACTAAGAAAAATTGTTTTGCCTTGAGATGCTGTT
AATCTGTAACTTTAGCCCCAACCCTGTGCTCACGGAAACATGTGCTGTAAGGTTTAAGGGATCTAGGGCTGTGCAGGATGTACCTTGTT
AACAATATGTTTGCAGGCAGTATGTTTGGTAAAAGTCATCGCCATTCTCCATTCTCGATTAACCAGGGGCTCAATGCACTGTGGAAAGC
CACAGGAACCTCTGCCCAAGAAAGCCTGGCTGTTGTGGGAAGTCAGGGACCCCGAATGGAGGGACCAGCTGGTGCTGCATCAGGAAACA
TAAATTGTGAAGATTTCTTGGACATTTATCAGTTTCCAAAATTAATACTTTTATAATTTCTTACACCTGTCTTACTTTAATCTCTTAAT
CCTGTTATCTTTGTAAGCTGAGGATATACGTCACCTCAGGACCACTATTGTACAAATTGATTGTAAAACATGTTCACATGTGTTTGAAC
AATATGAAATCAGTGCACCTTGAAAATGAACAGAATAACAGTGATTTTAGGGAACAAAGGAAGACAACCATAAGGTCTGACTGCCTGAG
GGGTCGGGCAAAAAGCCATATTTTTCTTCTTGCAGAGAGCCTATAAATGGACGTGCAAGTAGGAGAGATATTGCTAAATTCTTTTCCTA
GCAAGGAATATAATACTAAGACCCTAGGGAAAGAATTGCATTCCTGGGGGGAGGTCTATAAACGGCCGCTCTGGGAGTGTCTGTCCTAT
GTGGTTGAGATAAGGACTGAGATACGCCCTGGTCTCCTGCAGTACCCTCAGGCTTACTAGGATTGGGAAACCCCAGTCCTGGTAAATTT
GAGGTCAGGCCGGTTCTTTGCTCTGAACCCTGTTTTCTGTTAAGATGTTTATCAAGACAATACATGCACCGCTGAACATAGACCCTTAT
CAGGAGTTTCTGATTTTGCTCTGGTCCTGTTTCTTCAGAAGCATGTCATCTTTGCTCTGCCTTCTGCCCTTTGAAGCATGTGATCTTTG
TGACCTACTCCCTGTTCATACACCCCTCCCCTTTTAAAATCCCTAATAAAAACTTGCTGGTTTTGTGGCTCAGGGGGGCATCATGGACC
TACCAATACGTGATGTCACCCCCGGTGGCCCAGCTGTAAAA
SEQ ID 102
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGGGTGAAGGTACTCTACA
GTGTGGTCATTGAGGACAAGTTGACGAGAGAGTCCCAAGTACGTCCACGGTCAGCCTTGCGTCTACAGGTGTATCCAGCAGCTCCAAAG
AGACAGCAACCAGCAAGAATGGGCCATAGTGACGATGGTGGTTTTGTCAAAAAGAAAAGGGGGGGATATGTAAGGAAAAGAGAGATCAG
ACTTTCACTGTGTCTATGTAGAAAAGGAAGACATAAGAAACTCCATTTTGATCTGTACTAAGAAAAATTGTTTTGCCTTGAGATGCTGT
TAATCTGTAACTTTAGCCCCAACCCTGTGCTCACGGAAACATGTGCTGTAAGGTTTAAGGGATCTAGGGCTGTGCAGGATGTACCTTGT
TAACAATATGTTTGCAGGCAGTATGTTTGGTAAAAGTCATCGCCATTCTCCATTCTCGATTAACCAGGGGCTCAATGCACTGTGGAAAG
CCACAGGAACCTCTGCCCAAGAAAGCCTGGCTGTTGTGGGAAGTCAGGGACCCCGAATGGAGGGACCAGCTGGTGCTGCATCAGGAAAC
ATAAATTGTGAAGATTTCTTGGACATTTATCAGTTTCCAAAATTAATACTTTTATAATTTCTTACACCTGTCTTACTTTAATCTCTTAA
TCCTGTTATCTTTGTAAGCTGAGGATATACGTCACCTCAGGACCACTATTGTACAAATTGATTGTAAAACATGTTCACATGTGTTTGAA
CAATATGAAATCAGTGCACCTTGAAAATGAACAGAATAACAGTGATTTTAGGGAACAAAGGAAGACAACCATAAGGTCTGACTGCCTGA
GGGGTCGGGCAAAAAGCCATATTTTTCTTCTTGCAGAGAGCCTATAAATGGACGTGCAAGTAGGAGAGATATTGCTAAATTCTTTTCCT
AGCAAGGAATATAATACTAAGACCCTAGGGAAAGAATTGCATTCCTGGGGGGAGGTCTATAAACGGCCGCTCTGGGAGTGTCTGTCCTA
TGTGGTTGAGATAAGGACTGAGATACGCCCTGGTCTCCTGCAGTACCCTCAGGCTTACTAGGATTGGGAAACCCCAGTCCTGGTAAATT
TGAGGTCAGGCCGGTTCTTTGCTCTGAACCCTGTTTTCTGTTAAGATGTTTATCAAGACAATACATGCACCGCTGAACATAGACCCTTA
TCAGGAGTTTCTGATTTTGCTCTGGTCCTGTTTCTTCAGAAGCATGTCATCTTTGCTCTGCCTTCTGCCCTTTGAAGCATGTGATCTTT
GTGACCTACTCCCTGTTCATACACCCCTCCCCTTTTAAAATCCCTAATAAAAACTTGCTGGTTTTGTGGCTCAGGGGGGCATCATGGAC
CTACCAATACGTGATGTCACCCCCGGTGGCCCAGCTGTAAA[?]

SEQ ID 103
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGGGTGAAGGTACTCTACA
GTGTGGTCATTGAGGACAAGTTGACGAGAGAGTCCCAAGTACGTCCACGGTCAGCCTTGCGACATTTAAAGTTCTACAATGAACTCACT
GGAGATGCAAAGAAAAGTGTGGAGATGGAGACACCCCAATCGACTCGCCAGTCTACAGGTGTATCCAGCAGCTCCAAAGAGACAGCAAC
CAGCAAGAATGGGCCATAGTGACGATGGTGGTTTTGTCAAAAAGAAAAGGGGGGGATATGTAAGGAAAAGAGAGATCAGACTTTCACTG
TGTCTATGTAGAAAAGGAAGACATAAGAAACTCCATTTTGATCTGTACTAAGAAAAATTGTTTTGCCTTGAGATGCTGTTAATCTGTAA
CTTTAGCCCCAACCCTGTGCTCACGGAAACATGTGCTGTAAGGTTTAAGGGATCTAGGGCTGTGCAGGATGTACCTTGTTAACAATATG
TTTGCAGGCAGTATGTTTGGTAAAAGTCATCGCCATTCTCCATTCTCGATTAACCAGGGGCTCAATGCACTGTGGAAAGCCACAGGAAC
CTCTGCCCAAGAAAGCCTGGCTGTTGTGGGAAGTCAGGGACCCCGAATGGAGGGACCAGCTGGTGCTGCATCAGGAAACATAAATTGTG
AAGATTTCTTGGACATTTATCAGTTTCCAAAATTAATACTTTTATAATTTCTTACACCTGTCTTACTTTAATCTCTTAATCCTGTTATC
TTTGTAAGCTGAGGATATACGTCACCTCAGGACCACTATTGTACAAATTGATTGTAAAACATGTTCACATGTGTTTGAACAATATGAAA
TCAGTGCACCTTGAAAATGAACAGAATAACAGTGATTTTAGGGAACAAAGGAAGACAACCATAAGGTCTGACTGCCTGAGGGGTCGGGC
AAAAAGCCATATTTTTCTTCTTGCAGAGAGCCTATAAATGGACGTGCAAGTAGGAGAGATATTGCTAAATTCTTTTCCTAGCAAGGAAT
ATAATACTAAGACCCTAGGGAAAGAATTGCATTCCTGGGGGGAGGTCTATAAACGGCCGCTCTGGGAGTGTCTGTCCTATGTGGTTGAG
ATAAGGACTGAGATACGCCCTGGTCTCCTGCAGTACCCTCAGGCTTACTAGGATTGGGAAACCCCAGTCCTGGTAAATTTGAGGTCAGG
CCGGTTCTTTGCTCTGAACCCTGTTTTCTGTTAAGATGTTTATCAAGACAATACATGCACCGCTGAACATAGACCCTTATCAGGAGTTT
CTGATTTTGCTCTGGTCCTGTTTCTTCAGAAGCATGTCATCTTTGCTCTGCCTTCTGCCCTTTGAAGCATGTGATCTTTGTGACCTACT
CCCTGTTCATACACCCCTCCCCTTTTAAAATCCCTAATAAAAACTTGCTGGTTTTGTGGCTCAGGGGGGCATCATGGACCTACCAATAC
GTGATGTCACCCCCGGTGGCCCAGCTGTAAAA
SEQ ID 104
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGGGTGAAGGTACTCTACA
GTGTGGTCATTGAGGACAAGTTGACGAGAGAGTCCCAAGTACGTCCACGGTCAGCCTTGCGGAGAAAATCAGCTTCCTGTTTGGATACC
CACTAGACATTTAAAGTTCTACAATGAACTCACTGGAGATGCAAAGAAAAGTGTGGAGATGGAGACACCCCAATCGACTCGCCAGTCTA
CAGGTGTATCCAGCAGCTCCAAAGAGACAGCAACCAGCAAGAATGGGCCATAGTGACGATGGTGGTTTTGTCAAAAAGAAAAGGGGGGG
ATATGTAAGGAAAAGAGAGATCAGACTTTCACTGTGTCTATGTAGAAAAGGAAGACATAAGAAACTCCATTTTGATCTGTACTAAGAAA
AATTGTTTTGCCTTGAGATGCTGTTAATCTGTAACTTTAGCCCCAACCCTGTGCTCACGGAAACATGTGCTGTAAGGTTTAAGGGATCT
AGGGCTGTGCAGGATGTACCTTGTTAACAATATGTTTGCAGGCAGTATGTTTGGTAAAAGTCATCGCCATTCTCCATTCTCGATTAACC
AGGGGCTCAATGCACTGTGGAAAGCCACAGGAACCTCTGCCCAAGAAAGCCTGGCTGTTGTGGGAAGTCAGGGACCCCGAATGGAGGGA
CCAGCTGGTGCTGCATCAGGAAACATAAATTGTGAAGATTTCTTGGACATTTATCAGTTTCCAAAATTAATACTTTTATAATTTCTTAC
ACCTGTCTTACTTTAATCTCTTAATCCTGTTATCTTTGTAAGCTGAGGATATACGTCACCTCAGGACCACTATTGTACAAATTGATTGT
AAAACATGTTCACATGTGTTTGAACAATATGAAATCAGTGCACCTTGAAAATGAACAGAATAACAGTGATTTTAGGGAACAAAGGAAGA
CAACCATAAGGTCTGACTGCCTGAGGGGTCGGGCAAAAAGCCATATTTTTCTTCTTGCAGAGAGCCTATAAATGGACGTGCAAGTAGGA
GAGATATTGCTAAATTCTTTTCCTAGCAAGGAATATAATACTAAGACCCTAGGGAAAGAATTGCATTCCTGGGGGGAGGTCTATAAACG
GCCGCTCTGGGAGTGTCTGTCCTATGTGGTTGAGATAAGGACTGAGATACGCCCTGGTCTCCTGCAGTACCCTCAGGCTTACTAGGATT
GGGAAACCCCAGTCCTGGTAAATTTGAGGTCAGGCCGGTTCTTTGCTCTGAACCCTGTTTTCTGTTAAGATGTTTATCAAGACAATACA
TGCACCGCTGAACATAGACCCTTATCAGGAGTTTCTGATTTTGCTCTGGTCCTGTTTCTTCAGAAGCATGTCATCTTTGCTCTGCCTTC
TGCCCTTTGAAGCATGTGATCTTTGTGACCTACTCCCTGTTCATACACCCCTCCCCTTTTAAAATCCCTAATAAAAACTTGCTGGTTTT
GTGGCTCAGGGGGGCATCATGGACCTACCAATACGTGATGTCACCCCCGGTGGCCCAGCTGTAAAA
SEQ ID 105
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCAC[?][?][?][?][?]TTTCTCTATACTTTGTCTCTGTTGTCTTTCTTTTCTCAAGTCTC

TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGGGTGAAGGTACTCTACA
GTGTGGTCATTGAGGACAAGTTGACGAGAGAGTCCCAAGTACGTCCACGGTCAGCCTTGCGACATTTAAAGTTCTACAATGAACTCACT
GGAGATGCAAAGAAAAGTGTGGAGATGGAGACACCCCAATCGACTCGCCAGGTAAACAAAATGGTGATATCAGAAGAACAGAAAAAGTT
GCCTTCCATCAAGGAAGCAGAGTTGCCAATATAGGCACAATTAAAGAAGCTGACACAGTTAGCTAAAAAAAAAAGCCTAGAGAATACAA
AGGTGACACCAACTCCAGAGAATATGCTGCTTGCAGCTCTGATGATTGTATCAACGGTGTCTACAGGTGTATCCAGCAGCTCCAAAGAG
ACAGCAACCAGCAAGAATGGGCCATAGTGACGATGGTGGTTTTGTCAAAAAGAAAAGGGGGGGATATGTAAGGAAAAGAGAGATCAGAC
TTTCACTGTGTCTATGTAGAAAAGGAAGACATAAGAAACTCCATTTTGATCTGTACTAAGAAAAATTGTTTTGCCTTGAGATGCTGTTA
ATCTGTAACTTTAGCCCCAACCCTGTGCTCACGGAAACATGTGCTGTAAGGTTTAAGGGATCTAGGGCTGTGCAGGATGTACCTTGTTA
ACAATATGTTTGCAGGCAGTATGTTTGGTAAAAGTCATCGCCATTCTCCATTCTCGATTAACCAGGGGCTCAATGCACTGTGGAAAGCC
ACAGGAACCTCTGCCCAAGAAAGCCTGGCTGTTGTGGGAAGTCAGGGACCCCGAATGGAGGGACCAGCTGGTGCTGCATCAGGAAACAT
AAATTGTGAAGATTTCTTGGACATTTATCAGTTTCCAAAATTAATACTTTTATAATTTCTTACACCTGTCTTACTTTAATCTCTTAATC
CTGTTATCTTTGTAAGCTGAGGATATACGTCACCTCAGGACCACTATTGTACAAATTGATTGTAAAACATGTTCACATGTGTTTGAACA
ATATGAAATCAGTGCACCTTGAAAATGAACAGAATAACAGTGATTTAGGGAACAAAGGAAGACAACCATAAGGTCTGACTGCCTGAGG
GGTCGGGCAAAAAGCCATATTTTTCTTCTTGCAGAGAGCCTATAAATGGACGTGCAAGTAGGAGAGATATTGCTAAATTCTTTTCCTAG
CAAGGAATATAATACTAAGACCCTAGGGAAAGAATTGCATTCCTGGGGGGAGGTCTATAAACGGCCGCTCTGGGAGTGTCTGTCCTATG
TGGTTGAGATAAGGACTGAGATACGCCCTGGTCTCCTGCAGTACCCTCAGGCTTACTAGGATTGGGAAACCCCAGTCCTGGTAAATTTG
AGGTCAGGCCGGTTCTTTGCTCTGAACCCTGTTTTCTGTTAAGATGTTTATCAAGACAATACATGCACCGCTGAACATAGACCCTTATC
AGGAGTTTCTGATTTTGCTCTGGTCCTGTTTCTTCAGAAGCATGTCATCTTTGCTCTGCCTTCTGCCCTTTGAAGCATGTGATCTTTGT
GACCTACTCCCTGTTCATACACCCCTCCCCTTTTAAAATCCCTAATAAAAACTTGCTGGTTTTGTGGCTCAGGGGGGCATCATGGACCT
ACCAATACGTGATGTCACCCCCGGTGGCCCAGCTGTAAAA
SEQ ID 106
GAGATAGGAGAAAACTGCCTTAGGGCTGGAGGTGGGACATGCTGGCGGCAATACTGCTCTTTAAGGCATTGAGATGTTTATGTATATGC
ACATCAAAAGCACAGCACTTTTTTCTTTACCTTGTTTATGATGCAGAGACATTTGTTCACATGTTTTCCTGCTGGCCCTCTCCCCACTA
TTACCCTATTGTCCTGCCACATCCCCCTCTCCGAGATGGTAGAGATAATGATCAATAAATACTGAGGGAACTCAGAGACCGGTGCGGCG
CGGGTCCTCCATATGCTGAGCGCCGGTCCCCTGGGCCCACTTTTCTTTCTCTATACTTTGTCTCTGTTGTCTTTCTTTTTCTCAAGTCTC
TCGTTCCACCTGAGGAGAAATGCCCACAGCTGTGGAGGCGCAGGCCACTCCATCTGGTGCCCAACGTGGATGCTTTTCTCTAGGGTGAA
GGGACTCTCGAGTGTGGTCATTGAGGACAAGTCAACGAGAGATTCCCGAGTACGTCTACAGTGAGCCTTGTGGGTGAAGGTACTCTACA
GTGTGGTCATTGAGGACAAGTTGACGAGAGAGTCCCAAGTACGTCCACGGTCAGCCTTGCGGAGAAAATCAGCTTCCTGTTTGGATACC
CACTAGACATTTAAAGTTCTACAATGAACTCACTGGAGATGCAAAGAAAAGTGTGGAGATGGAGACACCCCAATCGACTCGCCAGGTAA
ACAAAATGGTGATATCAGAAGAACAGAAAAAGTTGCCTTCCATCAAGGAAGCAGAGTTGCCAATATAGGCACAATTAAAGAAGCTGACA
CAGTTAGCTAAAAAAAAAAGCCTAGAGAATACAAAGGTGACACCAACTCCAGAGAATATGCTGCTTGCAGCTCTGATGATTGTATCAAC
GGTGTCTACAGGTGTATCCAGCAGCTCCAAAGAGACAGCAACCAGCAAGAATGGGCCATAGTGACGATGGTGGTTTTGTCAAAAAGAAA
AGGGGGGGATATGTAAGGAAAAGAGAGATCAGACTTTCACTGTGTCTATGTAGAAAAGGAAGACATAAGAAACTCCATTTTGATCTGTA
CTAAGAAAAATTGTTTTGCCTTGAGATGCTGTTAATCTGTAACTTTAGCCCCAACCCTGTGCTCACGGAAACATGTGCTGTAAGGTTTA
AGGGATCTAGGGCTGTGCAGGATGTACCTTGTTAACAATATGTTTGCAGGCAGTATGTTTGGTAAAAGTCATCGCCATTCTCCATTCTC
GATTAACCAGGGGCTCAATGCACTGTGGAAAGCCACAGGAACCTCTGCCCAAGAAAGCCTGGCTGTTGTGGGAAGTCAGGGACCCCGAA
TGGAGGGACCAGCTGGTGCTGCATCAGGAAACATAAATTGTGAAGATTTCTTGGACATTTATCAGTTTCCAAAATTAATACTTTTATAA
TTTCTTACACCTGTCTTACTTTAATCTCTTAATCCTGTTATCTTTGTAAGCTGAGGATATACGTCACCTCAGGACCACTATTGTACAAA
TTGATTGTAAAACATGTTCACATGTGTTTGAACAATATGAAATCAGTGCACCTTGAAAATGAACAGAATAACAGTGATTTAGGGAACA
AAGGAAGACAACCATAAGGTCTGACTGCCTGAGGGGTCGGGCAAAAAGCCATATTTTTCTTCTTGCAGAGAGCCTATAAATGGACGTGC
AAGTAGGAGAGATATTGCTAAATTCTTTTCCTAGCAAGGAATATAATACTAAGACCCTAGGGAAAGAATTGCATTCCTGGGGGGAGGTC
TATAAACGGCCGCTCTGGGAGTGTCTGTCCTATGTGGTTGAGATAAGGACTGAGATACGCCCTGGTCTCCTGCAGTACCCTCAGGCTTA
CTAGGATTGGGAAACCCCAGTCCTGGTAAATTTGAGGTCAGGCCGGTTCTTTGCTCTGAACCCTGTTTTCTGTTAAGATGTTTATCAAG
ACAATACATGCACCGCTGAACATAGACCCTTATCAGGAGTTTCTGATTTTGCTCTGGTCCTGTTTCTTCAGAAGCATGTCATCTTTGCT
CTGCCTTCTGCCCTTTGAAGCATGTGATCTTTGTGACCTACTCCCTGTTCATACACCCCTCCCCTTTTAAAATCCCTAATAAAAACTTG
CTGGTTTTGTGGCTCAGGGGGGCATCATGGACCTACCAATACGTGATGTCACCCCCGGTGGCCCAGCTGTAAAA
SEQ ID 107
TTTTGCTTGTGTTTCACCAGGAGAAAATCAGCTTCCTGTTTGGATACCCACTAGACATTTAAAGTTCTACAATGAACTCACTGGAGATG
CAAAGAAAAGTGTGGAGATGGAGACACCCCAATCGACTCGCCAGGTAAACAAAATGGTGATATCAGAAGAACAGAAAAAGTTGCCTTCC
ATCAAGGAAGCAGAGTTGCCAATATAGGCACAATTAAAGAAGCTGACACAGTTAGCTAAAAAAAAAAGCCTAGAGAATACAAAGGTGAC
ACCAACTCCAGAGAATATGCTGCTTGCAGCTCTGATGATTGTATCAACGGTGGTAAGTCTTCCCAAGTCTGCAGGAGCAGCTGCAGCTA
ATTATACTTACTGGGCCTATGTGCCTTTCCCACCCTTAATT[?][?][?][?]CAGTTACATAGATGGATAATCCTATTGAAGTAGATGTTAATAAT

AGTGCATGGGTGCCTGGCCCCACAGATGACTGTTGCCCTGCCCAACCTGAAGAAGGAATGATGATGAATATTTCCATTGGGTATCCTTA
TCCTCCTGTTTGCCTAGGGAAGGCACCAGGATGCTTAATGCCTACAACCCAAAATTGTCTACAGGTGTATCCAGCAGCTCCAAAGAGAC
AGCAACCAGCAAGAATGGGCCATAGTGACGATGGTGGTTTTGTCAAAAAGAAAAGGGGGGATATGTAAGGAAAAGAGAGATCAGACTT
TCACTGTGTCTATGTAGAAAAGGAAGACATAAGAAACTCCATTTTGATCTGTACTAAGAAAAATTGTTTTGCCTTGAGATGCTGTTAAT
CTGTAACTTTAGCCCCAACCCTGTGCTCACGGAAACATGTGCTGTAAGGTTTAAGGGATCTAGGGCTGTGCAGGATGTACCTTGTTAAC
AATATGTTTGCAGGCAGTATGTTTGGTAAAAGTCATCGCCATTCTCCATTCTCGATTAACCAGGGGCTCAATGCACTGTGGAAAGCCAC
AGGAACCTCTGCCCAAGAAAGCCTGGCTGTTGTGGGAAGTCAGGGACCCCGAATGGAGGGACCAGCTGGTGCTGCATCAGGAAACATAA
ATTGTGAAGATTTCTTGGACATTTATCAGTTTCCAAAATTAATACTTTTATAATTTCTTACACCTGTCTTACTTTAATCTCTTAATCCT
GTTATCTTTGTAAGCTGAGGATATACGTCACCTCAGGACCACTATTGTACAAATTGATTGTAAAACATGTTCACATGTGTTTGAACAAT
ATGAAATCAGTGCACCTTGAAAATGAACAGAATAACAGTGATTTTAGGGAACAAAGGAAGACAACCATAAGGTCTGACTGCCTGAGGGG
TCGGGCAAAAAGCCATATTTTTCTTCTTGCAGAGAGCCTATAAATGGACGTGCAAGTAGGAGAGATATTGCTAAATTCTTTTCCTAGCA
AGGAATATAATACTAAGACCCTAGGGAAAGAATTGCATTCCTGGGGGGAGGTCTATAAACGGCCGCTCTGGGAGTGTCTGTCCTATGTG
GTTGAGATAAGGACTGAGATACGCCCTGGTCTCCTGCAGTACCCTCAGGCTTACTAGGATTGGGAAACCCCAGTCCTGGTAAATTTGAG
GTCAGGCCGGTTCTTTGCTCTGAACCCTGTTTTCTGTTAAGATGTTTATCAAGACAATACATGCACCGCTGAACATAGACCCTTATCAG
GAGTTTCTGATTTTGCTCTGGTCCTGTTTCTTCAGAAGCATGTCATCTTTGCTCTGCCTTCTGCCCTTTGAAGCATGTGATCTTTGTGA
CCTACTCCCTGTTCATACACCCCTCCCCTTTTAAAATCCCTAATAAAAACTTGCTGGTTTTGTGGCTCAGGGGGGCATCATGGACCTAC
CAATACGTGATGTCACCCCCGGTGGCCCAGCTGTAAAA
SEQ ID 108
TTTTGCTTGTGTTTCACCAGGAGAAAATCAGCTTCCTGTTTGGATACCCACTAGACATTTAAAGTTCTACAATGAACTCACTGGAGATG
CAAAGAAAAGTGTGGAGATGGAGACACCCCAATCGACTCGCCAGGTAAACAAAATGGTGATATCAGAAGAACAGAAAAAGTTGCCTTCC
ATCAAGGAAGCAGAGTTGCCAATATAGGCACAATTAAAGAAGCTGACACAGTTAGCTAAAAAAAAAAGCCTAGAGAATACAAAGGTGAC
ACCAACTCCAGAGAATATGCTGCTTGCAGCTCTGATGATTGTATCAACGGTGGTAAGTCTTCCCAAGTCTGCAGGAGCAGCTGCAGCTA
ATTATACTTACTGGGCCTATGTGCCTTTCCCACCCTTAATTCGGGCAGTTACATAGATGGATAATCCTATTGAAGTAGATGTTAATAAT
AGTGCATGGGTGCCTGGCCCCACAGATGACTGTTGCCCTGCCCAACCTGAAGAAGGAATGATGATGAATATTTCCATTGGGTATCCTTA
TCCTCCTGTTTGCCTAGGGAAGGCACCAGGATGCTTAATGCCTACAACCCAAAATTGGTTGGTAGAAGTACCTACAGTCAGTGCTACCA
GTAGATTTACTTATCACATGTCTACAGGTGTATCCAGCAGCTCCAAAGAGACAGCAACCAGCAAGAATGGGCCATAGTGACGATGGTGG
TTTTGTCAAAAAGAAAAGGGGGGATATGTAAGGAAAAGAGAGATCAGACTTTCACTGTGTCTATGTAGAAAAGGAAGACATAAGAAAC
TCCATTTTGATCTGTACTAAGAAAAATTGTTTTGCCTTGAGATGCTGTTAATCTGTAACTTTAGCCCCAACCCTGTGCTCACGGAAACA
TGTGCTGTAAGGTTTAAGGGATCTAGGGCTGTGCAGGATGTACCTTGTTAACAATATGTTTGCAGGCAGTATGTTTGGTAAAAGTCATC
GCCATTCTCCATTCTCGATTAACCAGGGGCTCAATGCACTGTGGAAAGCCACAGGAACCTCTGCCCAAGAAAGCCTGGCTGTTGTGGGA
AGTCAGGGACCCCGAATGGAGGGACCAGCTGGTGCTGCATCAGGAAACATAAATTGTGAAGATTTCTTGGACATTTATCAGTTTCCAAA
ATTAATACTTTTATAATTTCTTACACCTGTCTTACTTTAATCTCTTAATCCTGTTATCTTTGTAAGCTGAGGATATACGTCACCTCAGG
ACCACTATTGTACAAATTGATTGTAAAACATGTTCACATGTGTTTGAACAATATGAAATCAGTGCACCTTGAAAATGAACAGAATAACA
GTGATTTTAGGGAACAAAGGAAGACAACCATAAGGTCTGACTGCCTGAGGGGTCGGGCAAAAAGCCATATTTTTCTTCTTGCAGAGAGC
CTATAAATGGACGTGCAAGTAGGAGAGATATTGCTAAATTCTTTTCCTAGCAAGGAATATAATACTAAGACCCTAGGGAAAGAATTGCA
TTCCTGGGGGGAGGTCTATAAACGGCCGCTCTGGGAGTGTCTGTCCTATGTGGTTGAGATAAGGACTGAGATACGCCCTGGTCTCCTGC
AGTACCCTCAGGCTTACTAGGATTGGGAAACCCCAGTCCTGGTAAATTTGAGGTCAGGCCGGTTCTTTGCTCTGAACCCTGTTTTCTGT
TAAGATGTTTATCAAGACAATACATGCACCGCTGAACATAGACCCTTATCAGGAGTTTCTGATTTTGCTCTGGTCCTGTTTCTTCAGAA
GCATGTCATCTTTGCTCTGCCTTCTGCCCTTTGAAGCATGTGATCTTTGTGACCTACTCCCTGTTCATACACCCCTCCCCTTTTAAAAT
CCCTAATAAAAACTTGCTGGTTTTGTGGCTCAGGGGGGCATCATGGACCTACCAATACGTGATGTCACCCCCGGTGGCCCAGCTGTAAA
A
SEQ ID 109
GAGAAGAAAACCACCCTGTGGCTGGAGGTGAGATATGCTAGCGGCAATGCTGCTCTGTTACTCTTTGCTACACTGAGATGTTTGGGTGG
AGAGAAGCATAAATCTGGCCTATGTGCACATCTGGGCACAGAACCTCCCCTTGAACTTGTGACACAGATTCCTTTGTTCACATGTTTTC
CTGCTGACCTTCTCCCCACTATCGCCCTGTTCTCCCACCGCATTCCCCTTGCTGAGATAGTGAAAATAGTAATCTGTAGATACCAAGGG
AACTCAGAGACCATGGCCGGTGCACATCCTCCGTACGCTGAGCGCTGGTCCCCTGGGCCCATTGTTCTTTCTCTATACTTTGTCTCTGT
GTCTTATTTCTTTCCTCAGTCTCTCATCCCTCCTGACGAGAAATACCCACAGGTGTGGAGGGGCTGGCCCCCTTCATCTGATGCCCAAT
GTGGGTGCCTTTCTCTAGGGTGAAGGTACTCTACAGTGTGGTCATTGAGGACAAGTTGACGAGAGAGTCCCAAGTACGTCCACGGTCAG
CCTTGCGACATTTAAAGTTCTACAATGAACTCACTGGAGATGCAAAGAAAAGTGTGGAGATGGAGACACCCCAATCGACTCGCCAGTCT
ACAGGTGTATCCAGCAGCTCCAAAGAGACAGCAACCAGCAAGAATGGGCCATAGTGACGATGGTGGTTTTGTCAAAAAGAAAAGGGGGG
GATATGTAAGGAAAAGAGAGATCAGACTTTCACTGTGTCTATGTAGAAAAGGAAGACATAAGAAACTCCATTTTGATCTGTACTAAGAA
AAATTGTTTTGCCTTGAGATGCTGTTAATCTGTAACTTTAGCCCCAACCCTGTGCTCACGGAAACATGTGCTGTAAGGTTTAAGGGATC
TAGGGCTGTGCAGGATGTACCTTGTTAACAATATGTTTGC[?][?][?][?]AGTATGTTTGGTAAAAGTCATCGCCATTCTCCATTCTCGATTAAC

CAGGGGCTCAATGCACTGTGGAAAGCCACAGGAACCTCTGCCCAAGAAAGCCTGGCTGTTGTGGGAAGTCAGGGACCCCGAATGGAGGG
ACCAGCTGGTGCTGCATCAGGAAACATAAATTGTGAAGATTTCTTGGACATTTATCAGTTTCCAAAATTAATACTTTTATAATTTCTTA
CACCTGTCTTACTTTAATCTCTTAATCCTGTTATCTTTGTAAGCTGAGGATATACGTCACCTCAGGACCACTATTGTACAAATTGATTG
TAAAACATGTTCACATGTGTTTGAACAATATGAAATCAGTGCACCTTGAAAATGAACAGAATAACAGTGATTTTAGGGAACAAAGGAAG
ACAACCATAAGGTCTGACTGCCTGAGGGGTCGGCAAAAAGCCATATTTTTCTTCTTGCAGAGAGCCTATAAATGGACGTGCAAGTAGG
AGAGATATTGCTAAATTCTTTTCCTAGCAAGGAATATAATACTAAGACCCTAGGGAAAGAATTGCATTCCTGGGGGGAGGTCTATAAAC
GGCCGCTCTGGGAGTGTCTGTCCTATGTGGTTGAGATAAGGACTGAGATACGCCCTGGTCTCCTGCAGTACCCTCAGGCTTACTAGGAT
TGGGAAACCCCAGTCCTGGTAAATTTGAGGTCAGGCCGGTTCTTTGCTCTGAACCCTGTTTTCTGTTAAGATGTTTATCAAGACAATAC
ATGCACCGCTGAACATAGACCCTTATCAGGAGTTTCTGATTTTGCTCTGGTCCTGTTTCTTCAGAAGCATGTCATCTTTGCTCTGCCTT
CTGCCCTTTGAAGCATGTGATCTTTGTGACCTACTCCCTGTTCATACACCCCTCCCCTTTTAAAATCCCTAATAAAAACTTGCTGGTTT
TGTGGCTCAGGGGGGCATCATGGACCTACCAATACGTGATGTCACCCCCGGTGGCCCAGCTGTAAAA
SEQ ID 110
TAMASNSPATQDAALY
SEQ ID 111
ACGCAGGTTAGACAAGCACAAACCCCAAGAGAAAATCAAGTAGAAAGGGACAGAGTCTCTATCCCGGCA
SEQ ID 112
CCCACAGCGATGGCGTCTAATTCACCAGCAACACAGGACGCGGCGCTGTAT
SEQ ID 113
NCGGCCTNCGGCTGCGNNTANTCGACAGNANGGNGGGTAGGCCTTATTTTAGGGAGATCAAGTCTAAATTTGAAGGGAGTCCAAATTCA
TACTGGGGTAATTTATTCAGATTATAAAGGGGGAATTCAGTTAGTGTCAGCTCCACTGTTCCCCGGAGTGCCAATCCAGGTGATAGAAT
TGCTCAATTACTGCTTTTGCCTTATGTTAAAATTGGGGAAAACAAAACGGAAAGAACAGGAGGGTTTGGAAGTACCAACCCTGCAGGAA
AAGCTGCTTATTGGGCTAATCAGGTCTCAGAAGATAGACCCGTGTGTACAGTCACTATTCAGGGAAAGAGTTTGAAGGATTAGTGGATA
CCCAGGCTGATTCTATCATCGGCATAGGTACCGCCTCAGAAGTGTATCAAAGTGCCATGATTTTACATTGTCTAGGATCTGATAATCAA
GAAAGTACGGTTCAGCCTGTGATCACTTCATTCCAATCAATTTATGGGGCCGAGACTTGTTACAACAATGGCATGCAGAGATTACTATC
CCAGCCTCCCTATACAGCCCCAGGAATCAAAAAATCATGACTAAAATGGGATAGCTCCCTAAAAAGGGACTAGGAAAGAAAGAAGTCCC
AATTGAGGCTGAAAAAATCAAAAAAGAAANGAATAGGGCATCCTTTTTAGGAGCGTCACTGTANAGCCTCCAAACCCATTCATTAACTT
GGGAAAAAAAAACTGNNTGGTAAATCANCANCCGCTTCCAAAAAAAAAAAAAAAAAAAACCCNNNNCCN
SEQ ID 114
ATCTTTACCCTGTATAAACATCTTTCTCTTCCCAGTATTTCTAAGCATGTGACAATGAATATGCAAAGGAAGCGCAGCAGTCCACCAGG
TGTGGGATATGTGTGGCACAATTCAAGACAATGATTAAACCTCCACTTGATGTTGCAAAAGAGATTTTGAAAAATTTGCTTTCACCACA
CCAGCCTAAATAATAAAGAACCAGCCACCAGGTTTCAGTGGAAAGTATTGCCTCAGGGAATGCTTAATAGTTCAACTATTTGTCAGCTC
AAGCTCTGCAACCAGTTAGAGACAAGTTTTCAGACTGTTACATCGTTCACTATGTTGATATTTTGTGTGCTGCAGAAACGAGAGACAAA
TTAATTGACCGTTACACATTTCTGCAGACAGAGGTTGCCAACGCGGGACTGACAATAACATCTGATAAGATTCAAGCCTCTACTCCTTT
CCGTTACTTGGGAATGCAGGTAGAGGAAAGGAAAATTAAACCACAAAAAAATAGAAATAAGAAAAGACACATTAAAAGCATTAAATGAG
TTTCAAAAGTTGCTAGGAGATACTAATTGGATTTGGAGATATTAATTGGATTTGGCCAACTCTAGGCATTCCTACTTATGCCATGTCAA
ATTTGTTCTCTTTCTTAAGAGGGGACTCGGAATTAAATAGTGAAAGAACGTTAACTCCAGAGGCAACTAAAGAAATTAAATTAATTGAA
GAAAAAATTCGGTCAGCACAAGTAAATAGAATAGATCACTTGGCCCCACTCCAAATTTTGATTTTTACTACTGCACATTCCCTAACAGG
CATCATTGTTCAAAACACAGATCTTGTGGAGTGGTCCTTCCTTCCTCACAGTACAATTAAGACTTTTACATTGTACTTGGATCAAATGG
CTACATTAATTGGTCAGGGAAGATTATGAATAATAACATTGTGTGGAAATGACCCAGATAAAATCACTGTTCCTTTCAACAAGCAACAG
GTTAGACAAGCCTTTATCAATTCTGGTGCATGGCAGATTGGTCTTGCCGATTTTGTGGGAATTATTGACAATCGTTACC
SEQ ID 115
CCAAAAGAATGAGTCATCAAAACTCAGTATCACTTGACTCAAAGAGCAGAGTTGGTTGCCGTCATTACAGTGTTAACAAGATTTTAATC
AGTCTATTAACATTGTATCAGATTCTGCATATGTAGTACAGGCTACAAAGGATATTGAGAGAGCCCTAATCAAATACATTATGGATGAT
CAGTTAAACCCGCTGTTTAATTTGTTACAACAAAATGTAAGAAAAAGAAATTTCCCATTTTATATTACTCATATTCGAGCACACACTAA
TTTACCAGGGCCTTTAACTAAAGCAAATGAACAAGCTGACTCGCTAGTATCATCTGCATTCATGGAAGCACAAGACCTTCATGCCTTGA
CTCATGTAAATGCAATAGGATTAAAAAATAAATTTAATATCACATGGAAACAGACAAAAAAATATTGTACAACATTGCACCCAGTGTCAG
ATTCTACACCTGGCCACTCAGGAGGCAAGAGTTAATCCCAGAGGTCTATGTCCTAATGTGTTATGGCAAATGGATGTCATGCACGTACC
TTCATTTGGAAAATTGTCATTTGTCCATGTGACAGTTGATACTTATTCACATTTCATATGGGCAACCTGCCAGACAGGAGAAAGTACTT
CCCATGTTAAGAGACATTTATTATCTTGTTTTCCTGTCATGGGAGTTCCAGAAAAAGTTAAAACAGACAATGGGCCAGGTTACTGTAGT
AAAGCAGTTCAAAAAATTCTTAAATCAGTGGAAAATTACACATACAATAGGAATTCTCTATAATTCCCAAGGACAGGCCATAATTGAAAG
AACTAATAGAACACTCAAAGCTCAATTGGTTAAACAAAAAA

SEQ ID 116
CCTTACGGCCGGGAAGGGCCGGAAAGGGAGTCAAGCAGGAGCGTCTGTCCGAACGGAGGCTAGGTAAGAATATTTCACCATGAAAATGT
TAAAAGACATAAAGGAAGGAGCTAAACAATATGGACCCAACTCTCCTTATATGAGAACGTTATTAGATTCCATTGCTCATGGAAATAGA
CTTATTCCTTATGATTGGGAAATTTTACCTAAATCTTCCCTTTCACCCTCTCAGTATCTACAGTTTAAAACCTGGTGGATTGATGGAGT
ACAAGAACAGGTACGGAAAAATCAGGCTACTTATCCTGTTGTTAATATAGATGCAGACCAATTGCTAGGAACACGTCCAAATTGGAGCA
CTATTAACCAACAATCAGTAATGCAAAATGAGGCTATTGAACAACTAGGGGCTATTTGCCTCAGGGCCTGGGAAAAGATTCAGGACCCA
GGAACCAGTTAGAGACAGTTTTCAGACTGTTATATCATTCATTATGTTGATGATATTTTGTGTGCTGCAGAAACAAGAGACAAATTAAT
TGACTTTTACATGTTTCTGCAGACAGAGGTTGCAAACACAGGCCTGACAATAGCATCTGATAAGATTCAGACCTCCACTCCTTTTAATT
ATTTGGGAATGCAGGTAGAGGAAAGAAAAATTAAACCA
SEQ ID 117
CTGAAAAAAATCAAAAAAGAAAAGGAATAGGGCATCCTTTTTAGGAGCGGTCACTGTAGAGCCTCCAAAACCCATTCCATTAACTTGGG
GGAAAAAAAAACAACTGTATGGTAAATCAGCAGCGCTTCCAAAACAAAAACTGGAGGCTTTACATTTATTAGCAAAGAAACAATTAGAA
AAAGGACATTGAGCCTTCATTTTCGCCTTGGAATTCTGTTTGTAATTCAGAAAAAATCCGGCAGATGGCGTATAATGCCGTAATTCAAC
CCATGGGGGCTCTCCCACCCCGGTTGCCCTCTCCAGCCATGGTCCCCTTTAATTATAATTGATCTGAAGGATTGCTTTTTTACCATTCC
TCTGGCAAAACAGGATTTTGAGAAATTTGCTTTTACCACACCAGCCTAAATAATAAAGAACCAGCCACCAGGTTTCAGTGGAAAGTATT
GCCTCAGGGAATGCTTAATAGTTCAACTATTTGTCAGCTCAAGCTCTGCAACCAGTTAGAGACAAGTTTTCAGACTGTTACATCGTTCA
CTATGTTGATATTTTGTGTGCTGCAGAAACGAGAGACAAATTAATTGACCGTTACACATTTCTGCAGACAGAGGTTGCCAACGCGGGAC
TGACAATAACATCTGATAAGATTCAAACCTCTACTCCTTTCCGTTACTTGGGAATGCAGGTAGAGGAAAGGAAAATTAAACCACAAAA
SEQ ID 118
ACAACAATGGCATGCAGAGATTACTATCCCAGCCTCCCTATACAGCCCCAGGAATCAAAAAATCATGACTAAAATGGGATAGCTCCCTA
AAAAGGGACTAGGAAAGAAAGAAGTCCCAATTGAGGCTGAAAAAAATTAAAAAAGAAAAGGAATAGGGCATCCTTTTTAGGAGCGGTCA
CTGTAGAGCCTCCAAAACCCATTCCATTAACTTGGGAAAAAAAAAACTGTATGGTAAATCAGCAGCCGCTTCCAAAACAAAAGCTGGAG
GCCTTACACTTATTAGCAAAGAAACCATTAGAAAAAGGACATTGAGCCTTCATTTTCGCCTTGGAATTCTGTTTGTGATTCAGAAAAAA
TCCGGCAGATGGCGTATGCTAACTGAGCCATTAATGCCGTAATTCAACCCATGGGGGCTCTCCCACCCCGGTTGCCCTCTCCAGCCATG
GTCCCCTTTAATTATAATTGATCTGAAGGATTGCTTTTTTACCATTCCTCTGGCAAAACAGGATTTTGAAAAATTTGCTTTTACCACAC
CAGCCTAAATAATAAAGAACCAGCCACCAGGTTTCAGTGGAAAGTATTGCCTCAGGGAATGCTTAATAGTTCAACTATTTGTCAGCTCA
AGCTCTGCAACCAGTTAGAGACAAGTTTTCAGACTGTTACATCGTTCACTATGTTGATATTTTGTGTGCTGCAGAAACGAGAGACAAAT
TAATTGACCGTTACACATTTCTGCAGACAGAGGTTGCCAACGCGGGGCTGACAATAACATCTGATAAGATTCAAACCTCTACTCCTTTC
CGTTACTTGGGAATGCAGGTAGAGGAAAGGAAAATTA
SEQ ID 119
CATTAGAAAAAGGACATTGAGCCTTCATTTTCGCCTTGGAATTCTGTTTGTAATTCAGAAAAAATCCGGCAGATGGCGTATGCTAACTG
AGCCATTAATGCCGTAATTCAACCCATGGGGGCTCTCCCACCCCGGTTGCCCTCTCCAGCCATGGTCCCCTTTAATTATAATTGATCTG
AAGGATTGCTTTTTTACCATTCCTCTGGCAAAACAGGATTTTGAAAAATTTGCTTTTACCACACCAGCCTAAATAATAAAGAACCAGCC
ACCAGGTTTCAGTGGAAAGTATTGCCTCAGGGAATGCTTAATAGTTCAACTATTTGTCAGCTCAAGCTCTGCAACCAGTTAGAGACAAG
TTTTCAGACTGTTACATCGTTCACTATGTTGATATTTTGTGTGCTGCAGAAACGAGAGACAAATTAATTGACCGTTACACATTTCTGCA
GACAGAGGTTGCCAACGCGGGACTGACAATAACATCTGATAAGATTCAAACCTCTACTCCTTTCCGTTACTTGGGAATGCAGGTAGAGG
AAAGGAAAATTAAACCACAAAAAATAGAAATAAGAAAAGACACATTAAAAGCATTAAATGAGTTTCAAAAGTTGCTAGGAGATACTAAT
TGGATTTGGAGATATTAATTGGATTTGGCCAACTCTAGGCATTCCTACTTATGCCATGTCAAATTTGTACTCTTTCTTAAGAGGGGACT
CGGAATTAAATAGTGAAAGAACGTTAACTCCAGAGGCAACTAAAGAAAAA
SEQ ID 120
ACTGAGATAGGAGAAAACTGCCTTA
SEQ ID 121
GATAGGAGAAAACTGCCTTAGGGCT
SEQ ID 122
GAGAAAACTGCCTTAGGGCTGGAGG
SEQ ID 123
AACTGCCTTAGGGCTGGAGGTGGGA
SEQ ID 124
CCTTAGGGCTGGAGGTGGGACATGC
SEQ ID 125
GGGCTGGAGGTGGGACATGCTGGCG

SEQ ID 126
GGAGGTGGGACATGCTGGCGGCAAT
SEQ ID 127
TGGGACATGCTGGCGGCAATACTGC
SEQ ID 128
CATGCTGGCGGCAATACTGCTCTTT
SEQ ID 129
TGGCGGCAATACTGCTCTTTAAGGC
SEQ ID 130
GCAATACTGCTCTTTAAGGCATTGA
SEQ ID 131
ACTGCTCTTTAAGGCATTGAGATGT
SEQ ID 132
TCTTTAAGGCATTGAGATGTTTATG
SEQ ID 133
AAGGCATTGAGATGTTTATGTATAT
SEQ ID 134
ATTGAGATGTTTATGTATATGCACA
SEQ ID 135
GATGTTTATGTATATGCACATCAAA
SEQ ID 136
TTATGTATATGCACATCAAAAGCAC
SEQ ID 137
TATATGCACATCAAAAGCACAGCAC
SEQ ID 138
GCACATCAAAAGCACAGCACTTTTT
SEQ ID 139
TCAAAAGCACAGCACTTTTTTCTTT
SEQ ID 140
AGCACAGCACTTTTTTCTTTACCTT
SEQ ID 141
AGCACTTTTTTCTTTACCTTGTTTA
SEQ ID 142
TTTTTTCTTTACCTTGTTTATGATG
SEQ ID 143
TCTTTACCTTGTTTATGATGCAGAG
SEQ ID 144
ACCTTGTTTATGATGCAGAGACATT
SEQ ID 145
GTTTATGATGCAGAGACATTTGTTC
SEQ ID 146
TGATGCAGAGACATTTGTTCACATG
SEQ ID 147
CAGAGACATTTGTTCACATGTTTTC
SEQ ID 148
ACATTTGTTCACATGTTTTCCTGCT
SEQ ID 149
TGTTCACATGTTTTCCTGCTGGCCC
SEQ ID 150
ACATGTTTTCCTGCTGGCCCTCTCC
SEQ ID 151
TTTTCCTGCTGGCCCTCTCCCCACT

SEQ ID 152
CTGCTGGCCCTCTCCCCACTATTAC
SEQ ID 153
GGCCCTCTCCCCACTATTACCCTAT
SEQ ID 154
TCTCCCCACTATTACCCTATTGTCC
SEQ ID 155
CCACTATTACCCTATTGTCCTGCCA
SEQ ID 156
ATTACCCTATTGTCCTGCCACATCC
SEQ ID 157
CCTATTGTCCTGCCACATCCCCCTC
SEQ ID 158
TGTCCTGCCACATCCCCCTCTCCGA
SEQ ID 159
TGCCACATCCCCCTCTCCGAGATGG
SEQ ID 160
CATCCCCCTCTCCGAGATGGTAGAG
SEQ ID 161
CCCTCTCCGAGATGGTAGAGATAAT
SEQ ID 162
TCCGAGATGGTAGAGATAATGATCA
SEQ ID 163
GATGGTAGAGATAATGATCAATAAA
SEQ ID 164
TAGAGATAATGATCAATAAATACTG
SEQ ID 165
ATAATGATCAATAAATACTGAGGGA
SEQ ID 166
GATCAATAAATACTGAGGGAACTCA
SEQ ID 167
ATAAATACTGAGGGAACTCAGAGAC
SEQ ID 168
TACTGAGGGAACTCAGAGACCGGTG
SEQ ID 169
AGGGAACTCAGAGACCGGTGCGGCG
SEQ ID 170
ACTCAGAGACCGGTGCGGCGCGGGT
SEQ ID 171
GAGACCGGTGCGGCGCGGGTCCTCC
SEQ ID 172
CGGTGCGGCGCGGGTCCTCCATATG
SEQ ID 173
CGGCGCGGGTCCTCCATATGCTGAG
SEQ ID 174
CGGGTCCTCCATATGCTGAGCGCCG
SEQ ID 175
CCTCCATATGCTGAGCGCCGGTCCC
SEQ ID 176
ATATGCTGAGCGCCGGTCCCCTGGG
SEQ ID 177
CTGAGCGCCGGTCCCCTGGGCCCAC

SEQ ID 178
CGCCGGTCCCCTGGGCCCACTTTTC
SEQ ID 179
GTCCCCTGGGCCCACTTTTCTTTCT
SEQ ID 180
CTGGGCCCACTTTTCTTTCTCTATA
SEQ ID 181
CCCACTTTTCTTTCTCTATACTTTG
SEQ ID 182
TTTTCTTTCTCTATACTTTGTCTCT
SEQ ID 183
TTTCTCTATACTTTGTCTCTGTTGT
SEQ ID 184
CTATACTTTGTCTCTGTTGTCTTTC
SEQ ID 185
CTTTGTCTCTGTTGTCTTTCTTTTC
SEQ ID 186
TCTCTGTTGTCTTTCTTTTCTCAAG
SEQ ID 187
GTTGTCTTTCTTTTCTCAAGTCTCT
SEQ ID 188
CTTTCTTTTCTCAAGTCTCTCGTTC
SEQ ID 189
TTTTCTCAAGTCTCTCGTTCCACCT
SEQ ID 190
TCAAGTCTCTCGTTCCACCTGAGGA
SEQ ID 191
TCTCTCGTTCCACCTGAGGAGAAAT
SEQ ID 192
CGTTCCACCTGAGGAGAAATGCCCA
SEQ ID 193
CACCTGAGGAGAAATGCCCACAGCT
SEQ ID 194
GAGGAGAAATGCCCACAGCTGTGGA
SEQ ID 195
GAAATGCCCACAGCTGTGGAGGCGC
SEQ ID 196
GCCCACAGCTGTGGAGGCGCAGGCC
SEQ ID 197
CAGCTGTGGAGGCGCAGGCCACTCC
SEQ ID 198
GTGGAGGCGCAGGCCACTCCATCTG
SEQ ID 199
GGCGCAGGCCACTCCATCTGGTGCC
SEQ ID 200
AGGCCACTCCATCTGGTGCCCAACG
SEQ ID 201
ACTCCATCTGGTGCCCAACGTGGAT
SEQ ID 202
ATCTGGTGCCCAACGTGGATGCTTT
SEQ ID 203
GTGCCCAACGTGGATGCTTTTCTCT

SEQ ID 204
CAACGTGGATGCTTTTCTCTAGGGT
SEQ ID 205
TGGATGCTTTTCTCTAGGGTGAAGG
SEQ ID 206
GCTTTTCTCTAGGGTGAAGGGACTC
SEQ ID 207
TCTCTAGGGTGAAGGGACTCTCGAG
SEQ ID 208
AGGGTGAAGGGACTCTCGAGTGTGG
SEQ ID 209
GAAGGGACTCTCGAGTGTGGTCATT
SEQ ID 210
GACTCTCGAGTGTGGTCATTGAGGA
SEQ ID 211
TCGAGTGTGGTCATTGAGGACAAGT
SEQ ID 212
TGTGGTCATTGAGGACAAGTCAACG
SEQ ID 213
TCATTGAGGACAAGTCAACGAGAGA
SEQ ID 214
GAGGACAAGTCAACGAGAGATTCCC
SEQ ID 215
CAAGTCAACGAGAGATTCCCGAGTA
SEQ ID 216
CAACGAGAGATTCCCGAGTACGTCT
SEQ ID 217
AGAGATTCCCGAGTACGTCTACAGT
SEQ ID 218
TTCCCGAGTACGTCTACAGTGAGCC
SEQ ID 219
GAGTACGTCTACAGTGAGCCTTGTG
SEQ ID 220
gagaaaatcagcttcctgtttggat
SEQ ID 221
aatcagcttcctgtttggataccca
SEQ ID 222
gcttcctgtttggatacccactaga
SEQ ID 223
ctgtttggatacccactagacattt
SEQ ID 224
acccactagacatttaaagttctac
SEQ ID 225
ctagacatttaaagttctacaatga
SEQ ID 226
GGTGAAGGTACTCTACAGTGTGGTC
SEQ ID 227
AGGTACTCTACAGTGTGGTCATTGA
SEQ ID 228
CTCTACAGTGTGGTCATTGAGGACA
SEQ ID 229
CAGTGTGGTCATTGAGGACAAGTTG

SEQ ID 230
TGGTCATTGAGGACAAGTTGACGAG
SEQ ID 231
ATTGAGGACAAGTTGACGAGAGAGT
SEQ ID 232
GGACAAGTTGACGAGAGAGTCCCAA
SEQ ID 233
AGTTGACGAGAGAGTCCCAAGTACG
SEQ ID 234
ACGAGAGAGTCCCAAGTACGTCCAC
SEQ ID 235
AGAGTCCCAAGTACGTCCACGGTCA
SEQ ID 236
CCCAAGTACGTCCACGGTCAGCCTT
SEQ ID 237
GTACGTCCACGGTCAGCCTTGCGAC
SEQ ID 238
TCCACGGTCAGCCTTGCGACATTTA
SEQ ID 239
GGTCAGCCTTGCGACATTTAAAGTT
SEQ ID 240
GCCTTGCGACATTTAAAGTTCTACA
SEQ ID 241
GCGACATTTAAAGTTCTACAATGAA
SEQ ID 242
ATTTAAAGTTCTACAATGAACTCAC
SEQ ID 243
AAGTTCTACAATGAACTCACTGGAG
SEQ ID 244
CTACAATGAACTCACTGGAGATGCA
SEQ ID 245
ATGAACTCACTGGAGATGCAAAGAA
SEQ ID 246
CTCACTGGAGATGCAAAGAAAAGTG
SEQ ID 247
TGGAGATGCAAAGAAAAGTGTGGAG
SEQ ID 248
ATGCAAAGAAAAGTGTGGAGATGGA
SEQ ID 249
AAGAAAAGTGTGGAGATGGAGACAC
SEQ ID 250
AAGTGTGGAGATGGAGACACCCCAA
SEQ ID 251
TGGAGATGGAGACACCCCAATCGAC
SEQ ID 252
ATGGAGACACCCCAATCGACTCGCC
SEQ ID 253
GACACCCCAATCGACTCGCCAGTCT
SEQ ID 254
CCCAATCGACTCGCCAGTCTACAGG
SEQ ID 255
TCGACTCGCCAGTCTACAGGTGTAT

SEQ ID 256
TCGCCAGTCTACAGGTGTATCCAGC
SEQ ID 257
AGTCTACAGGTGTATCCAGCAGCTC
SEQ ID 258
ACAGGTGTATCCAGCAGCTCCAAAG
SEQ ID 259
TGTATCCAGCAGCTCCAAAGAGACA
SEQ ID 260
CCAGCAGCTCCAAAGAGACAGCAAC
SEQ ID 261
AGCTCCAAAGAGACAGCAACCAGCA
SEQ ID 262
CAAAGAGACAGCAACCAGCAAGAAT
SEQ ID 263
AGACAGCAACCAGCAAGAATGGGCC
SEQ ID 264
GCAACCAGCAAGAATGGGCCATAGT
SEQ ID 265
CAGCAAGAATGGGCCATAGTGACGA
SEQ ID 266
AGAATGGGCCATAGTGACGATGGTG
SEQ ID 267
GGGCCATAGTGACGATGGTGGTTTT
SEQ ID 268
ATAGTGACGATGGTGGTTTTGTCAA
SEQ ID 269
GACGATGGTGGTTTTGTCAAAAAGA
SEQ ID 270
TGGTGGTTTTGTCAAAAAGAAAAGG
SEQ ID 271
GTTTTGTCAAAAAGAAAAGGGGGGG
SEQ ID 272
GTCAAAAAGAAAAGGGGGGGATATG
SEQ ID 273
AAAGAAAAGGGGGGGATATGTAAGG
SEQ ID 274
AAAGGGGGGGATATGTAAGGAAAAG
SEQ ID 275
GGGGGATATGTAAGGAAAAGAGAGA
SEQ ID 276
ATATGTAAGGAAAAGAGAGATCAGA
SEQ ID 277
TAAGGAAAAGAGAGATCAGACTTTC
SEQ ID 278
AAAAGAGAGATCAGACTTTCACTGT
SEQ ID 279
AGAGATCAGACTTTCACTGTGTCTA
SEQ ID 280
TCAGACTTTCACTGTGTCTATGTAG
SEQ ID 281
CTTTCACTGTGTCTATGTAGAAAAG

SEQ ID 282
ACTGTGTCTATGTAGAAAAGGAAGA
SEQ ID 283
GTCTATGTAGAAAAGGAAGACATAA
SEQ ID 284
TGTAGAAAAGGAAGACATAAGAAAC
SEQ ID 285
AAAAGGAAGACATAAGAAACTCCAT
SEQ ID 286
GAAGACATAAGAAACTCCATTTTGA
SEQ ID 287
CATAAGAAACTCCATTTTGATCTGT
SEQ ID 288
GAAACTCCATTTTGATCTGTACTAA
SEQ ID 289
TCCATTTTGATCTGTACTAAGAAAA
SEQ ID 290
TTTGATCTGTACTAAGAAAAATTGT
SEQ ID 291
TCTGTACTAAGAAAAATTGTTTTGC
SEQ ID 292
ACTAAGAAAAATTGTTTTGCCTTGA
SEQ ID 293
GAAAAATTGTTTTGCCTTGAGATGC
SEQ ID 294
ATTGTTTTGCCTTGAGATGCTGTTA
SEQ ID 295
TTTGCCTTGAGATGCTGTTAATCTG
SEQ ID 296
CTTGAGATGCTGTTAATCTGTAACT
SEQ ID 297
GATGCTGTTAATCTGTAACTTTAGC
SEQ ID 298
TGTTAATCTGTAACTTTAGCCCCAA
SEQ ID 299
ATCTGTAACTTTAGCCCCAACCCTG
SEQ ID 300
TAACTTTAGCCCCAACCCTGTGCTC
SEQ ID 301
TTAGCCCCAACCCTGTGCTCACGGA
SEQ ID 302
CCCAACCCTGTGCTCACGGAAACAT
SEQ ID 303
CCCTGTGCTCACGGAAACATGTGCT
SEQ ID 304
TGCTCACGGAAACATGTGCTGTAAG
SEQ ID 305
ACGGAAACATGTGCTGTAAGGTTTA
SEQ ID 306
AACATGTGCTGTAAGGTTTAAGGGA
SEQ ID 307
GTGCTGTAAGGTTTAAGGGATCTAG

SEQ ID 308
GTAAGGTTTAAGGGATCTAGGGCTG
SEQ ID 309
GTTTAAGGGATCTAGGGCTGTGCAG
SEQ ID 310
AGGGATCTAGGGCTGTGCAGGATGT
SEQ ID 311
TCTAGGGCTGTGCAGGATGTACCTT
SEQ ID 312
GGCTGTGCAGGATGTACCTTGTTAA
SEQ ID 313
TGCAGGATGTACCTTGTTAACAATA
SEQ ID 314
GATGTACCTTGTTAACAATATGTTT
SEQ ID 315
ACCTTGTTAACAATATGTTTGCAGG
SEQ ID 316
GTTAACAATATGTTTGCAGGCAGTA
SEQ ID 317
CAATATGTTTGCAGGCAGTATGTTT
SEQ ID 318
TGTTTGCAGGCAGTATGTTTGGTAA
SEQ ID 319
GCAGGCAGTATGTTTGGTAAAAGTC
SEQ ID 320
CAGTATGTTTGGTAAAAGTCATCGC
SEQ ID 321
TGTTTGGTAAAAGTCATCGCCATTC
SEQ ID 322
GGTAAAAGTCATCGCCATTCTCCAT
SEQ ID 323
AAGTCATCGCCATTCTCCATTCTCG
SEQ ID 324
ATCGCCATTCTCCATTCTCGATTAA
SEQ ID 325
CATTCTCCATTCTCGATTAACCAGG
SEQ ID 326
TCCATTCTCGATTAACCAGGGGCTC
SEQ ID 327
TCTCGATTAACCAGGGGCTCAATGC
SEQ ID 328
ATTAACCAGGGGCTCAATGCACTGT
SEQ ID 329
CCAGGGGCTCAATGCACTGTGGAAA
SEQ ID 330
GGCTCAATGCACTGTGGAAAGCCAC
SEQ ID 331
AATGCACTGTGGAAAGCCACAGGAA
SEQ ID 332
ACTGTGGAAAGCCACAGGAACCTCT
SEQ ID 333
GGAAAGCCACAGGAACCTCTGCCCA

SEQ ID 334
GCCACAGGAACCTCTGCCCAAGAAA
SEQ ID 335
AGGAACCTCTGCCCAAGAAAGCCTG
SEQ ID 336
CCTCTGCCCAAGAAAGCCTGGCTGT
SEQ ID 337
TGTGGGGAAAAGAAAGAGAGATCAG
SEQ ID 338
GGAAAAGAAAGAGAGATCAGACTGT
SEQ ID 339
AGAAAGAGAGATCAGACTGTTACTG
SEQ ID 340
GAGAGATCAGACTGTTACTGTGTCT
SEQ ID 341
ATCAGACTGTTACTGTGTCTATGTA
SEQ ID 342
ACTGTTACTGTGTCTATGTAGAAAG
SEQ ID 343
TACTGTGTCTATGTAGAAAGAAATA
SEQ ID 344
TGTCTATGTAGAAAGAAATAGACAT
SEQ ID 345
ATGTAGAAAGAAATAGACATAAGAG
SEQ ID 346
GAAAGAAATAGACATAAGAGACTCC
SEQ ID 347
AAATAGACATAAGAGACTCCATTTT
SEQ ID 348
GACATAAGAGACTCCATTTTGTTCT
SEQ ID 349
AAGAGACTCCATTTTGTTCTGTACT
SEQ ID 350
ACTCCATTTTGTTCTGTACTAAGAA
SEQ ID 351
ATTTTGTTCTGTACTAAGAAAAATT
SEQ ID 352
GTTCTGTACTAAGAAAAATTCTTCT
SEQ ID 353
GTACTAAGAAAAATTCTTCTGCTTT
SEQ ID 354
AAGAAAAATTCTTCTGCTTTGAGAT
SEQ ID 355
AAATTCTTCTGCTTTGAGATGCTGT
SEQ ID 356
CTTCTGCTTTGAGATGCTGTTAATC
SEQ ID 357
GCTTTGAGATGCTGTTAATCTGTAA
SEQ ID 358
GAGATGCTGTTAATCTGTAACCCTA
SEQ ID 359
GCTGTTAATCTGTAACCCTAGCCCC

SEQ ID 360
TAATCTGTAACCCTAGCCCCAACCC
SEQ ID 361
TGTAACCCTAGCCCCAACCCTGTGC
SEQ ID 362
CCCTAGCCCCAACCCTGTGCTCACA
SEQ ID 363
GCCCCAACCCTGTGCTCACAGAAAC
SEQ ID 364
AACCCTGTGCTCACAGAAACAGGTG
SEQ ID 365
GAAACAGGTGCTGTGTTGACTCAAG
SEQ ID 366
AGGTGCTGTGTTGACTCAAGGTTTA
SEQ ID 367
CTGTGTTGACTCAAGGTTTAATGGA
SEQ ID 368
TTGACTCAAGGTTTAATGGATTCAG
SEQ ID 369
ATGGATTCAGGGCTGTGCAGGATGT
SEQ ID 370
TTCAGGGCTGTGCAGGATGTGCTTT
SEQ ID 371
GGCTGTGCAGGATGTGCTTTGTTAA
SEQ ID 372
TGCAGGATGTGCTTTGTTAAACAAA
SEQ ID 373
GATGTGCTTTGTTAAACAAATGCTT
SEQ ID 374
GCTTTGTTAAACAAATGCTTGAAGG
SEQ ID 375
GTTAAACAAATGCTTGAAGGCAGCA
SEQ ID 376
GTTAAGAGTCATCACCACTCCCTAA
SEQ ID 377
GAGTCATCACCACTCCCTAATCTCA
SEQ ID 378
ATCACCACTCCCTAATCTCAAGTAA
SEQ ID 379
GCAGGGACACAAACACTGCGGAAGG
SEQ ID 380
GACACAAACACTGCGGAAGGCCGCA
SEQ ID 381
AAACACTGCGGAAGGCCGCAGGGAC
SEQ ID 382
CTGCGGAAGGCCGCAGGGACCTCTG
SEQ ID 383
GAAGGCCGCAGGGACCTCTGCCTAG
SEQ ID 384
CCGCAGGGACCTCTGCCTAGGAAAG
SEQ ID 385
GGGACCTCTGCCTAGGAAAGCCAGG

SEQ ID 386
CTCTGCCTAGGAAAGCCAGGTGTTG
SEQ ID 387
CCTAGGAAAGCCAGGTGTTGTCCAA
SEQ ID 388
GAAAGCCAGGTGTTGTCCAAGGTTT
SEQ ID 389
CCAGGTGTTGTCCAAGGTTTCTCCC
SEQ ID 390
TGTTGTCCAAGGTTTCTCCCCATGT
SEQ ID 391
TCCAAGGTTTCTCCCCATGTGACAG
SEQ ID 392
GGTTTCTCCCCATGTGACAGTCTGA
SEQ ID 393
CTCCCCATGTGACAGTCTGAAATAT
SEQ ID 394
CATGTGACAGTCTGAAATATGGCCT
SEQ ID 395
TCTGAAATATGGCCTCTTGGGAAGG
SEQ ID 396
AATATGGCCTCTTGGGAAGGGAAAG
SEQ ID 397
GGCCTCTTGGGAAGGGAAAGACCTG
SEQ ID 398
CTTGGGAAGGGAAAGACCTGACTGT
SEQ ID 399
GAAGGGAAAGACCTGACTGTCCCCT
SEQ ID 400
GGCCCGACACCCGTAAAGGGTCTGT
SEQ ID 401
GACACCCGTAAAGGGTCTGTGCTGA
SEQ ID 402
CCGTAAAGGGTCTGTGCTGAGGATT
SEQ ID 403
GCTGAGGATTAGTAAAAGAGGAAGG
SEQ ID 404
GGATTAGTAAAAGAGGAAGGAAGGC
SEQ ID 405
AGTAAAAGAGGAAGGAAGGCCTCTT
SEQ ID 406
AAGGCCTCTTTGCAGTTGAGATAAG
SEQ ID 407
CTCTTTGCAGTTGAGATAAGAGGAA
SEQ ID 408
TGCAGTTGAGATAAGAGGAAGGCAT
SEQ ID 409
TTGAGATAAGAGGAAGGCATCTGTC
SEQ ID 410
ATAAGAGGAAGGCATCTGTCTCCTG
SEQ ID 411
AGGAAGGCATCTGTCTCCTGCTCAT

SEQ ID 412
GGCATCTGTCTCCTGCTCATCCCTG
SEQ ID 413
CTGTCTCCTGCTCATCCCTGGGCAA
SEQ ID 414
TCCTGCTCATCCCTGGGCAATGGAA
SEQ ID 415
CTCATCCCTGGGCAATGGAATGTCT
SEQ ID 416
TTGTATATGCCATCTACTGAGATAG
SEQ ID 417
CGTCTACAGTGAGCCTTGTGGGTGA
SEQ ID 418
ACAGTGAGCCTTGTGGGTGAAGGTA
SEQ ID 419
GAGCCTTGTGGGTGAAGGTACTCTA
SEQ ID 420
TTGTGGGTGAAGGTACTCTACAGTG
SEQ ID 421
GCCCAAGAAAGCCTGGCTGTTGTGG
SEQ ID 422
AGAAAGCCTGGCTGTTGTGGGAAGT
SEQ ID 423
GCCTGGCTGTTGTGGGAAGTCAGGG
SEQ ID 424
GCTGTTGTGGGAAGTCAGGGACCCC
SEQ ID 425
TGTGGGAAGTCAGGGACCCCGAATG
SEQ ID 426
GAAGTCAGGGACCCCGAATGGAGGG
SEQ ID 427
CAGGGACCCCGAATGGAGGGACCAG
SEQ ID 428
ACCCCGAATGGAGGGACCAGCTGGT
SEQ ID 429
GAATGGAGGGACCAGCTGGTGCTGC
SEQ ID 430
GAGGGACCAGCTGGTGCTGCATCAG
SEQ ID 431
ACCAGCTGGTGCTGCATCAGGAAAC
SEQ ID 432
CTGGTGCTGCATCAGGAAACATAAA
SEQ ID 433
GCTGCATCAGGAAACATAAATTGTG
SEQ ID 434
ATCAGGAAACATAAATTGTGAAGAT
SEQ ID 435
GAAACATAAATTGTGAAGATTTCTT
SEQ ID 436
ATAAATTGTGAAGATTTCTTGGACA
SEQ ID 437
TTGTGAAGATTTCTTGGACATTTAT

SEQ ID 438
AAGATTTCTTGGACATTTATCAGTT
SEQ ID 439
TTCTTGGACATTTATCAGTTTCCAA
SEQ ID 440
GGACATTTATCAGTTTCCAAAATTA
SEQ ID 441
TTTATCAGTTTCCAAAATTAATACT
SEQ ID 442
CAGTTTCCAAAATTAATACTTTTAT
SEQ ID 443
TCCAAAATTAATACTTTTATAATTT
SEQ ID 444
AATTAATACTTTTATAATTTCTTAC
SEQ ID 445
ATACTTTTATAATTTCTTACACCTG
SEQ ID 446
TTTATAATTTCTTACACCTGTCTTA
SEQ ID 447
AATTTCTTACACCTGTCTTACTTTA
SEQ ID 448
CTTACACCTGTCTTACTTTAATCTC
SEQ ID 449
ACCTGTCTTACTTTAATCTCTTAAT
SEQ ID 450
TCTTACTTTAATCTCTTAATCCTGT
SEQ ID 451
CTTTAATCTCTTAATCCTGTTATCT
SEQ ID 452
ATCTCTTAATCCTGTTATCTTTGTA
SEQ ID 453
TTAATCCTGTTATCTTTGTAAGCTG
SEQ ID 454
CCTGTTATCTTTGTAAGCTGAGGAT
SEQ ID 455
TATCTTTGTAAGCTGAGGATATACG
SEQ ID 456
TTGTAAGCTGAGGATATACGTCACC
SEQ ID 457
AGCTGAGGATATACGTCACCTCAGG
SEQ ID 458
AGGATATACGTCACCTCAGGACCAC
SEQ ID 459
ATACGTCACCTCAGGACCACTATTG
SEQ ID 460
TCACCTCAGGACCACTATTGTACAA
SEQ ID 461
TCAGGACCACTATTGTACAAATTGA
SEQ ID 462
ACCACTATTGTACAAATTGATTGTA
SEQ ID 463
TATTGTACAAATTGATTGTAAAACA

SEQ ID 464
TACAAATTGATTGTAAAACATGTTC
SEQ ID 465
ATTGATTGTAAAACATGTTCACATG
SEQ ID 466
TTGTAAAACATGTTCACATGTGTTT
SEQ ID 467
AAACATGTTCACATGTGTTTGAACA
SEQ ID 468
TGTTCACATGTGTTTGAACAATATG
SEQ ID 469
ACATGTGTTTGAACAATATGAAATC
SEQ ID 470
TGTTTGAACAATATGAAATCAGTGC
SEQ ID 471
GAACAATATGAAATCAGTGCACCTT
SEQ ID 472
ATATGAAATCAGTGCACCTTGAAAA
SEQ ID 473
AAATCAGTGCACCTTGAAAATGAAC
SEQ ID 474
AGTGCACCTTGAAAATGAACAGAAT
SEQ ID 475
ACCTTGAAAATGAACAGAATAACAG
SEQ ID 476
GAAAATGAACAGAATAACAGTGATT
SEQ ID 477
TGAACAGAATAACAGTGATTTTAGG
SEQ ID 478
AGAATAACAGTGATTTTAGGGAACA
SEQ ID 479
AACAGTGATTTTAGGGAACAAAGGA
SEQ ID 480
TGATTTTAGGGAACAAAGGAAGACA
SEQ ID 481
TTAGGGAACAAAGGAAGACAACCAT
SEQ ID 482
GAACAAAGGAAGACAACCATAAGGT
SEQ ID 483
AAGGAAGACAACCATAAGGTCTGAC
SEQ ID 484
AGACAACCATAAGGTCTGACTGCCT
SEQ ID 485
ACCATAAGGTCTGACTGCCTGAGGG
SEQ ID 486
AAGGTCTGACTGCCTGAGGGGTCGG
SEQ ID 487
CTGACTGCCTGAGGGGTCGGGCAAA
SEQ ID 488
TGCCTGAGGGGTCGGGCAAAAAGCC
SEQ ID 489
GAGGGGTCGGGCAAAAAGCCATATT

SEQ ID 490
GTCGGGCAAAAAGCCATATTTTTCT
SEQ ID 491
GCAAAAAGCCATATTTTTCTTCTTG
SEQ ID 492
AAGCCATATTTTTCTTCTTGCAGAG
SEQ ID 493
ATATTTTTCTTCTTGCAGAGAGCCT
SEQ ID 494
TTTCTTCTTGCAGAGAGCCTATAAA
SEQ ID 495
TCTTGCAGAGAGCCTATAAATGGAC
SEQ ID 496
CAGAGAGCCTATAAATGGACGTGCA
SEQ ID 497
AGCCTATAAATGGACGTGCAAGTAG
SEQ ID 498
ATAAATGGACGTGCAAGTAGGAGAG
SEQ ID 499
TGGACGTGCAAGTAGGAGAGATATT
SEQ ID 500
GTGCAAGTAGGAGAGATATTGCTAA
SEQ ID 501
AGTAGGAGAGATATTGCTAAATTCT
SEQ ID 502
GAGAGATATTGCTAAATTCTTTTCC
SEQ ID 503
ATATTGCTAAATTCTTTTCCTAGCA
SEQ ID 504
GCTAAATTCTTTTCCTAGCAAGGAA
SEQ ID 505
ATTCTTTTCCTAGCAAGGAATATAA
SEQ ID 506
TTTCCTAGCAAGGAATATAATACTA
SEQ ID 507
TAGCAAGGAATATAATACTAAGACC
SEQ ID 508
AGGAATATAATACTAAGACCCTAGG
SEQ ID 509
TATAATACTAAGACCCTAGGGAAAG
SEQ ID 510
TACTAAGACCCTAGGGAAAGAATTG
SEQ ID 511
AGACCCTAGGGAAAGAATTGCATTC
SEQ ID 512
CTAGGGAAAGAATTGCATTCCTGGG
SEQ ID 513
GAAAGAATTGCATTCCTGGGGGGAG
SEQ ID 514
AATTGCATTCCTGGGGGGAGGTCTA
SEQ ID 515
CATTCCTGGGGGGAGGTCTATAAAC

SEQ ID 516
CTGGGGGGAGGTCTATAAACGGCCG
SEQ ID 517
GGGAGGTCTATAAACGGCCGCTCTG
SEQ ID 518
GTCTATAAACGGCCGCTCTGGGAGT
SEQ ID 519
TAAACGGCCGCTCTGGGAGTGTCTG
SEQ ID 520
GGCCGCTCTGGGAGTGTCTGTCCTA
SEQ ID 521
CTCTGGGAGTGTCTGTCCTATGTGG
SEQ ID 522
GGAGTGTCTGTCCTATGTGGTTGAG
SEQ ID 523
GTCTGTCCTATGTGGTTGAGATAAG
SEQ ID 524
TCCTATGTGGTTGAGATAAGGACTG
SEQ ID 525
TGTGGTTGAGATAAGGACTGAGATA
SEQ ID 526
TTGAGATAAGGACTGAGATACGCCC
SEQ ID 527
ATAAGGACTGAGATACGCCCTGGTC
SEQ ID 528
GACTGAGATACGCCCTGGTCTCCTG
SEQ ID 529
AGATACGCCCTGGTCTCCTGCAGTA
SEQ ID 530
CGCCCTGGTCTCCTGCAGTACCCTC
SEQ ID 531
TGGTCTCCTGCAGTACCCTCAGGCT
SEQ ID 532
TCCTGCAGTACCCTCAGGCTTACTA
SEQ ID 533
CAGTACCCTCAGGCTTACTAGGATT
SEQ ID 534
CCCTCAGGCTTACTAGGATTGGGAA
SEQ ID 535
AGGCTTACTAGGATTGGGAAACCCC
SEQ ID 536
TACTAGGATTGGGAAACCCCAGTCC
SEQ ID 537
GGATTGGGAAACCCCAGTCCTGGTA
SEQ ID 538
GGGAAACCCCAGTCCTGGTAAATTT
SEQ ID 539
ACCCCAGTCCTGGTAAATTTGAGGT
SEQ ID 540
AGTCCTGGTAAATTTGAGGTCAGGC
SEQ ID 541
TGGTAAATTTGAGGTCAGGCCGGTT

SEQ ID 542
AATTTGAGGTCAGGCCGGTTCTTTG
SEQ ID 543
GAGGTCAGGCCGGTTCTTTGCTCTG
SEQ ID 544
CAGGCCGGTTCTTTGCTCTGAACCC
SEQ ID 545
CGGTTCTTTGCTCTGAACCCTGTTT
SEQ ID 546
CTTTGCTCTGAACCCTGTTTTCTGT
SEQ ID 547
CTCTGAACCCTGTTTTCTGTTAAGA
SEQ ID 548
AACCCTGTTTTCTGTTAAGATGTTT
SEQ ID 549
TGTTTTCTGTTAAGATGTTTATCAA
SEQ ID 550
TCTGTTAAGATGTTTATCAAGACAA
SEQ ID 551
TAAGATGTTTATCAAGACAATACAT
SEQ ID 552
TGTTTATCAAGACAATACATGCACC
SEQ ID 553
ATCAAGACAATACATGCACCGCTGA
SEQ ID 554
GACAATACATGCACCGCTGAACATA
SEQ ID 555
TACATGCACCGCTGAACATAGACCC
SEQ ID 556
GCACCGCTGAACATAGACCCTTATC
SEQ ID 557
GCTGAACATAGACCCTTATCAGGAG
SEQ ID 558
ACATAGACCCTTATCAGGAGTTTCT
SEQ ID 559
GACCCTTATCAGGAGTTTCTGATTT
SEQ ID 560
TTATCAGGAGTTTCTGATTTTGCTC
SEQ ID 561
AGGAGTTTCTGATTTTGCTCTGGTC
SEQ ID 562
TTTCTGATTTTGCTCTGGTCCTGTT
SEQ ID 563
GATTTTGCTCTGGTCCTGTTTCTTC
SEQ ID 564
TGCTCTGGTCCTGTTTCTTCAGAAG
SEQ ID 565
TGGTCCTGTTTCTTCAGAAGCATGT
SEQ ID 566
CTGTTTCTTCAGAAGCATGTCATCT
SEQ ID 567
TCTTCAGAAGCATGTCATCTTTGCT

SEQ ID 568
AGAAGCATGTCATCTTTGCTCTGCC
SEQ ID 569
CATGTCATCTTTGCTCTGCCTTCTG
SEQ ID 570
CATCTTTGCTCTGCCTTCTGCCCTT
SEQ ID 571
TTGCTCTGCCTTCTGCCCTTTGAAG
SEQ ID 572
CTGCCTTCTGCCCTTTGAAGCATGT
SEQ ID 573
TTCTGCCCTTTGAAGCATGTGATCT
SEQ ID 574
CCCTTTGAAGCATGTGATCTTTGTG
SEQ ID 575
TGAAGCATGTGATCTTTGTGACCTA
SEQ ID 576
CATGTGATCTTTGTGACCTACTCCC
SEQ ID 577
GATCTTTGTGACCTACTCCCTGTTC
SEQ ID 578
TTGTGACCTACTCCCTGTTCATACA
SEQ ID 579
ACCTACTCCCTGTTCATACACCCCT
SEQ ID 580
CTCCCTGTTCATACACCCCTCCCCT
SEQ ID 581
TGTTCATACACCCCTCCCCTTTTAA
SEQ ID 582
ATACACCCCTCCCCTTTTAAAATCC
SEQ ID 583
CCCCTCCCCTTTTAAAATCCCTAAT
SEQ ID 584
CCCCTTTTAAAATCCCTAATAAAAA
SEQ ID 585
TTTAAAATCCCTAATAAAAACTTGC
SEQ ID 586
AATCCCTAATAAAAACTTGCTGGTT
SEQ ID 587
CTAATAAAAACTTGCTGGTTTTGTG
SEQ ID 588
AAAAACTTGCTGGTTTTGTGGCTCA
SEQ ID 589
CTTGCTGGTTTTGTGGCTCAGGGGG
SEQ ID 590
TGGTTTTGTGGCTCAGGGGGGCATC
SEQ ID 591
TTGTGGCTCAGGGGGGCATCATGGA
SEQ ID 592
GCTCAGGGGGGCATCATGGACCTAC
SEQ ID 593
GGGGGGCATCATGGACCTACCAATA

SEQ ID 594
GCATCATGGACCTACCAATACGTGA
SEQ ID 595
ATGGACCTACCAATACGTGATGTCA
SEQ ID 596
CCTACCAATACGTGATGTCACCCCC
SEQ ID 597
CAATACGTGATGTCACCCCCGGTGG
SEQ ID 598
CGTGATGTCACCCCCGGTGGCCCAG
SEQ ID 599
TGTCACCCCCGGTGGCCCAGCTGTA
SEQ ID 600
TGTGCTCACAGAAACAGGTGCTGTG
SEQ ID 601
TCACAGAAACAGGTGCTGTGTTGAC
SEQ ID 602
TCAAGGTTTAATGGATTCAGGGCTG
SEQ ID 603
GTTTAATGGATTCAGGGCTGTGCAG
SEQ ID 604
ACAAATGCTTGAAGGCAGCAAGCTT
SEQ ID 605
TGCTTGAAGGCAGCAAGCTTGTTAA
SEQ ID 606
GAAGGCAGCAAGCTTGTTAAGAGTC
SEQ ID 607
CAGCAAGCTTGTTAAGAGTCATCAC
SEQ ID 608
AGCTTGTTAAGAGTCATCACCACTC
SEQ ID 609
CACTCCCTAATCTCAAGTAAGCAGG
SEQ ID 610
CCTAATCTCAAGTAAGCAGGGACAC
SEQ ID 611
TCTCAAGTAAGCAGGGACACAAACA
SEQ ID 612
AGTAAGCAGGGACACAAACACTGCG
SEQ ID 613
GACAGTCTGAAATATGGCCTCTTGG
SEQ ID 614
GAAAGACCTGACTGTCCCCTGGCCC
SEQ ID 615
ACCTGACTGTCCCCTGGCCCGACAC
SEQ ID 616
ACTGTCCCCTGGCCCGACACCCGTA
SEQ ID 617
CCCCTGGCCCGACACCCGTAAAGGG
SEQ ID 618
AAGGGTCTGTGCTGAGGATTAGTAA
SEQ ID 619
TCTGTGCTGAGGATTAGTAAAAGAG

SEQ ID 620
AAGAGGAAGGAAGGCCTCTTTGCAG
SEQ ID 621
GAAGGAAGGCCTCTTTGCAGTTGAG
SEQ ID 622
CCCTGGGCAATGGAATGTCTTGGTG
SEQ ID 623
GGCAATGGAATGTCTTGGTGTAAAG
SEQ ID 624
TGGAATGTCTTGGTGTAAAGCCTGA
SEQ ID 625
TGTCTTGGTGTAAAGCCTGATTGTA
SEQ ID 626
TGGTGTAAAGCCTGATTGTATATGC
SEQ ID 627
TAAAGCCTGATTGTATATGCCATCT
SEQ ID 628
CCTGATTGTATATGCCATCTACTGA
SEQ ID 629
TATGCCATCTACTGAGATAGGAGAA
SEQ ID 630
CATCTACTGAGATAGGAGAAAACTG
SEQ ID 631
GGGCTGGAGGTGGGACATGCTGGCG
SEQ ID 632
GGAGGTGGGACATGCTGGCGGCAAT
SEQ ID 633
TGGGACATGCTGGCGGCAATACTGC
SEQ ID 634
CATGCTGGCGGCAATACTGCTCTTT
SEQ ID 635
TGGCGGCAATACTGCTCTTTAAGGC
SEQ ID 636
GCAATACTGCTCTTTAAGGCATTGA
SEQ ID 637
TTATGTATATGCACATCAAAAGCAC
SEQ ID 638
TATATGCACATCAAAAGCACAGCAC
SEQ ID 639
GCACATCAAAAGCACAGCACTTTTT
SEQ ID 640
TCAAAAGCACAGCACTTTTTTCTTT
SEQ ID 641
AGCACAGCACTTTTTTCTTTACCTT
SEQ ID 642
AGCACTTTTTTCTTTACCTTGTTTA
SEQ ID 643
ACCTTGTTTATGATGCAGAGACATT
SEQ ID 644
GTTTATGATGCAGAGACATTTGTTC
SEQ ID 645
TGATGCAGAGACATTTGTTCACATG

SEQ ID 646
ACTCAGAGACCGGTGCGGCGCGGGT
SEQ ID 647
GAGACCGGTGCGGCGCGGGTCCTCC
SEQ ID 648
CGGTGCGGCGCGGGTCCTCCATATG
SEQ ID 649
CTTTGTCTCTGTTGTCTTTCTTTTC
SEQ ID 650
TCTCTGTTGTCTTTCTTTTCTCAAG
SEQ ID 651
TCAAGTCTCTCGTTCCACCTGAGGA
SEQ ID 652
TCTCTCGTTCCACCTGAGGAGAAAT
SEQ ID 653
CGTTCCACCTGAGGAGAAATGCCCA
SEQ ID 654
CACCTGAGGAGAAATGCCCACAGCT
SEQ ID 655
GAGGAGAAATGCCCACAGCTGTGGA
SEQ ID 656
GAAATGCCCACAGCTGTGGAGGCGC
SEQ ID 657
GCCCACAGCTGTGGAGGCGCAGGCC
SEQ ID 658
CAGCTGTGGAGGCGCAGGCCACTCC
SEQ ID 659
GTGGAGGCGCAGGCCACTCCATCTG
SEQ ID 660
GGCGCAGGCCACTCCATCTGGTGCC
SEQ ID 661
AGGCCACTCCATCTGGTGCCCAACG
SEQ ID 662
ACTCCATCTGGTGCCCAACGTGGAT
SEQ ID 663
GCTTTTCTCTAGGGTGAAGGGACTC
SEQ ID 664
TCTCTAGGGTGAAGGGACTCTCGAG
SEQ ID 665
AGGGTGAAGGGACTCTCGAGTGTGG
SEQ ID 666
GAAGGGACTCTCGAGTGTGGTCATT
SEQ ID 667
GACTCTCGAGTGTGGTCATTGAGGA
SEQ ID 668
TGTGGTCATTGAGGACAAGTCAACG
SEQ ID 669
GAGTACGTCTACAGTGAGCCTTGTG
SEQ ID 670
CGTCTACAGTGAGCCTTGTGGTAAG
SEQ ID 671
ACAGTGAGCCTTGTGGTAAGCTTGG

SEQ ID 672
GAGCCTTGTGGTAAGCTTGGGCGCT
SEQ ID 673
TTGTGGTAAGCTTGGGCGCTCGGAA
SEQ ID 674
CTTGGGCGCTCGGAAGAAGCCAGGG
SEQ ID 675
GCGCTCGGAAGAAGCCAGGGTTAAT
SEQ ID 676
CGGAAGAAGCCAGGGTTAATGGGGC
SEQ ID 677
GAAGCCAGGGTTAATGGGGCAAACT
SEQ ID 678
CAGGGTTAATGGGGCAAACTAAAAG
SEQ ID 679
GGGGCAAACTAAAAGTAAAGTCTCT
SEQ ID 680
AAAAGTAAAGTCTCTCATTCCACCT
SEQ ID 681
TAAAGTCTCTCATTCCACCTGATGA
SEQ ID 682
GGGGCAGGCCACCCCTTCAGGGTAG
SEQ ID 683
AGGCCACCCCTTCAGGGTAGGGTCC
SEQ ID 684
ACCCCTTCAGGGTAGGGTCCCCTCC
SEQ ID 685
TTCAGGGTAGGGTCCCCTCCATGCA
SEQ ID 686
GGTAGGGTCCCCTCCATGCAGACCA
SEQ ID 687
GGTCCCCTCCATGCAGACCATAGAG
SEQ ID 688
CCTCCATGCAGACCATAGAGCACAG
SEQ ID 689
ATGCAGACCATAGAGCACAGGTGTG
SEQ ID 690
GACCATAGAGCACAGGTGTGCCCCA
SEQ ID 691
TAGAGCACAGGTGTGCCCCAAAGAG
SEQ ID 692
CACAGGTGTGCCCCAAAGAGGAGCA
SEQ ID 693
GTGTGCCCCAAAGAGGAGCAGAGAG
SEQ ID 694
CCCCAAAGAGGAGCAGAGAGAAGGA
SEQ ID 695
AAGAGGAGCAGAGAGAAGGAGGGAG
SEQ ID 696
GAGCAGAGAGAAGGAGGGAGAGGGC
SEQ ID 697
GAGAGAAGGAGGGAGAGGGCCCACG

SEQ ID 698
AAGGAGGGAGAGGGCCCACGAGAGA
SEQ ID 699
GGGAGAGGGCCCACGAGAGACTTGG
SEQ ID 700
AGGGCCCACGAGAGACTTGGAAATG
SEQ ID 701
CCACGAGAGACTTGGAAATGAATGG
SEQ ID 702
AGAGACTTGGAAATGAATGGCAGGA
SEQ ID 703
CTTGGAAATGAATGGCAGGATTTTA
SEQ ID 704
AAATGAATGGCAGGATTTTAGGCGC
SEQ ID 705
AATGGCAGGATTTTAGGCGCTGGAC
SEQ ID 706
CAGGATTTTAGGCGCTGGACTTGGG
SEQ ID 707
TTTTAGGCGCTGGACTTGGGTTCGG
SEQ ID 708
GGCGCTGGACTTGGGTTCGGGGCAC
SEQ ID 709
TGGACTTGGGTTCGGGGCACCTGGC
SEQ ID 710
TTGGGTTCGGGGCACCTGGCCTTTC
SEQ ID 711
TTCGGGGCACCTGGCCTTTCCTTGT
SEQ ID 712
GGCACCTGGCCTTTCCTTGTGTATT
SEQ ID 713
CTGGCCTTTCCTTGTGTATTTCTCC
SEQ ID 714
CTTTCCTTGTGTATTTCTCCTACTG
SEQ ID 715
CTTGTGTATTTCTCCTACTGTCTGC
SEQ ID 716
GTATTTCTCCTACTGTCTGCCTAAC
SEQ ID 717
TCTCCTACTGTCTGCCTAACTATTT
SEQ ID 718
AATACAATAAAAGAAAACCAGCCCC
SEQ ID 719
AATAAAAGAAAACCAGCCCCTGGTT
SEQ ID 720
AAGAAAACCAGCCCCTGGTTCTTGT
SEQ ID 721
AACCAGCCCCTGGTTCTTGTGGTGT
SEQ ID 722
GCCCCTGGTTCTTGTGGTGTTTCCA
SEQ ID 723
TGGTTCTTGTGGTGTTTCCACCCTC

SEQ ID 724
CTTGTGGTGTTTCCACCCTCCCGGG
SEQ ID 725
GGTGTTTCCACCCTCCCGGGTCCCC
SEQ ID 726
TTCCACCCTCCCGGGTCCCCGCTGG
SEQ ID 727
CCCTCCCGGGTCCCCGCTGGCTGCC
SEQ ID 728
CCGGGTCCCCGCTGGCTGCCTGGCT
SEQ ID 729
TCCCCGCTGGCTGCCTGGCTTCCTC
SEQ ID 730
GCTGGCTGCCTGGCTTCCTCCCGCA
SEQ ID 731
CTGCCTGGCTTCCTCCCGCAGCTCC
SEQ ID 732
TGGCTTCCTCCCGCAGCTCCTGCTG
SEQ ID 733
TCCTCCCGCAGCTCCTGCTGTGTGT
SEQ ID 734
CCGCAGCTCCTGCTGTGTGTGTATG
SEQ ID 735
GCTCCTGCTGTGTGTGTATGTGTGT
SEQ ID 736
TGCTGTGTGTGTATGTGTGTGTGTG
SEQ ID 737
TGTGTGTATGTGTGTGTGTGTGCAC
SEQ ID 738
GTATGTGTGTGTGTGTGCACATCTG
SEQ ID 739
TGTGTGTGTGTGCACATCTGTGGGG
SEQ ID 740
GTGTGTGCACATCTGTGGGGCGTAT
SEQ ID 741
TGCACATCTGTGGGGCGTATGTGTG
SEQ ID 742
ATCTGTGGGGCGTATGTGTGTTCGT
SEQ ID 743
TGGGGCGTATGTGTGTTCGTCTTTG
SEQ ID 744
CGTATGTGTGTTCGTCTTTGTAATT
SEQ ID 745
GTGTGTTCGTCTTTGTAATTGAGGC
SEQ ID 746
TTCGTCTTTGTAATTGAGGCTGCAG
SEQ ID 747
CTTTGTAATTGAGGCTGCAGAGTGG
SEQ ID 748
TAATTGAGGCTGCAGAGTGGAGAGA
SEQ ID 749
GAGGCTGCAGAGTGGAGAGAGCAGG

SEQ ID 750
TGCAGAGTGGAGAGAGCAGGGGTTT
SEQ ID 751
AGTGGAGAGAGCAGGGGTTTTCTCT
SEQ ID 752
AGAGAGCAGGGGTTTTCTCTGGGGA
SEQ ID 753
GCAGGGGTTTTCTCTGGGGACCCAG
SEQ ID 754
GGTTTTCTCTGGGGACCCAGAGAGA
SEQ ID 755
TCTCTGGGGACCCAGAGAGAAGGAG
SEQ ID 756
GGGGACCCAGAGAGAAGGAGGCGTT
SEQ ID 757
CCCAGAGAGAAGGAGGCGTTTTCAC
SEQ ID 758
AGAGAAGGAGGCGTTTTCACCACAG
SEQ ID 759
AGGAGGCGTTTTCACCACAGCCGAA
SEQ ID 760
GCGTTTTCACCACAGCCGAACAGGG
SEQ ID 761
TTCACCACAGCCGAACAGGGCAGGA
SEQ ID 762
CACAGCCGAACAGGGCAGGACCCCA
SEQ ID 763
CCGAACAGGGCAGGACCCCAGCACC
SEQ ID 764
CAGGGCAGGACCCCAGCACCCGGGA
SEQ ID 765
CAGGACCCCAGCACCCGGGACCCAG
SEQ ID 766
CCCCAGCACCCGGGACCCAGCGGGA
SEQ ID 767
GCACCCGGGACCCAGCGGGACTTTG
SEQ ID 768
CGGGACCCAGCGGGACTTTGCCAAG
SEQ ID 769
CCCAGCGGGACTTTGCCAAGGGGAT
SEQ ID 770
CGGGACTTTGCCAAGGGGATGGACC
SEQ ID 771
CTTTGCCAAGGGGATGGACCTGGCT
SEQ ID 772
CCAAGGGGATGGACCTGGCTGGGCC
SEQ ID 773
GGGATGGACCTGGCTGGGCCACGCG
SEQ ID 774
GGACCTGGCTGGGCCACGCGGCTGT
SEQ ID 775
TGGCTGGGCCACGCGGCTGTTTGTG

SEQ ID 776
GGGCCACGCGGCTGTTTGTGTAGGG
SEQ ID 777
ACGCGGCTGTTTGTGTAGGGAAAAG
SEQ ID 778
GTAGAAAAGGAAGACATAAACTCCA
SEQ ID 779
AAAGGAAGACATAAACTCCATTTTG
SEQ ID 780
AAGACATAAACTCCATTTTGAGCTG
SEQ ID 781
ATAAACTCCATTTTGAGCTGTACTA
SEQ ID 782
AGAAAAATTATTTTGCCTTGACCTG
SEQ ID 783
AATTATTTTGCCTTGACCTGCTGTT
SEQ ID 784
TTTTGCCTTGACCTGCTGTTAACCT
SEQ ID 785
CCTTGACCTGCTGTTAACCTGTAAC
SEQ ID 786
ACCTGCTGTTAACCTGTAACTGTAG
SEQ ID 787
CTGTTAACCTGTAACTGTAGCCCCA
SEQ ID 788
AACCTGTAACTGTAGCCCCAACCCT
SEQ ID 789
TGTAGCCCCAACCCTGTGCTCAAAG
SEQ ID 790
TTTAAGGGATCAAGGGCTGTACAGG
SEQ ID 791
GGGATCAAGGGCTGTACAGGATGTG
SEQ ID 792
CAAGGGCTGTACAGGATGTGCCTTG
SEQ ID 793
ATGTGCCTTGTTAACAATGTGTTTA
SEQ ID 794
CCATTCTCCATTAATCAGGGGCACG
SEQ ID 795
CTCCATTAATCAGGGGCACGATGCA
SEQ ID 796
TTAATCAGGGGCACGATGCACTGCG
SEQ ID 797
GCACGATGCACTGCGGAAAGCCACA
SEQ ID 798
CCACAGGGACCTCTGCCCGAGAAAG
SEQ ID 799
GGGACCTCTGCCCGAGAAAGCCTGG
SEQ ID 800
CTCTGCCCGAGAAAGCCTGGGTATT
SEQ ID 801
GAAAGCCTGGGTATTGTCCAAGGCT

SEQ ID 802
CCTGGGTATTGTCCAAGGCTTCCCC
SEQ ID 803
GTATTGTCCAAGGCTTCCCCCCACT
SEQ ID 804
GTCCAAGGCTTCCCCCCACTGAGAC
SEQ ID 805
AGGCTTCCCCCCACTGAGACAGCCT
SEQ ID 806
CCACTGAGACAGCCTGAGATACGGC
SEQ ID 807
AGGAAGGCCTCCGTCTCCTGCATGT
SEQ ID 808
GGCCTCCGTCTCCTGCATGTCCTTG
SEQ ID 809
CCGTCTCCTGCATGTCCTTGGGAAT
SEQ ID 810
TCCTGCATGTCCTTGGGAATGGAAT
SEQ ID 811
CATGTCCTTGGGAATGGAATGTCTT
SEQ ID 812
CCTTGGGAATGGAATGTCTTGGTGT
SEQ ID 813
GGAATGTCTTGGTGTAAAACCCGAT
SEQ ID 814
GTCTTGGTGTAAAACCCGATAGTAC
SEQ ID 815
GGTGTAAAACCCGATAGTACATTCC
SEQ ID 816
AAAACCCGATAGTACATTCCTTCTA
SEQ ID 817
CCGATAGTACATTCCTTCTATTCTG
SEQ ID 818
AGTACATTCCTTCTATTCTGAGAGA
SEQ ID 819
TTCTATTCTGAGAGAAGAAAACCAC
SEQ ID 820
TTCTGAGAGAAGAAAACCACCCTGT
SEQ ID 821
AGAGAAGAAAACCACCCTGTGGCTG
SEQ ID 822
GAGGTGAGATATGCTAGCGGCAATG
SEQ ID 823
GAGATATGCTAGCGGCAATGCTGCT
SEQ ID 824
ATGCTAGCGGCAATGCTGCTCTGTT
SEQ ID 825
TGGCCTATGTGCACATCTGGGCACA
SEQ ID 826
TATGTGCACATCTGGGCACAGAACC
SEQ ID 827
GCACATCTGGGCACAGAACCTCCCC

SEQ ID 828
TCTGGGCACAGAACCTCCCCTTGAA
SEQ ID 829
GCACAGAACCTCCCCTTGAACTTGT
SEQ ID 830
GAACCTCCCCTTGAACTTGTGACAC
SEQ ID 831
TCCCCTTGAACTTGTGACACAGATT
SEQ ID 832
TTGAACTTGTGACACAGATTCCTTT
SEQ ID 833
CTTGTGACACAGATTCCTTTGTTCA
SEQ ID 834
AGATTCCTTTGTTCACATGTTTTCC
SEQ ID 835
CTCCCCACTATCGCCCTGTTCTCCC
SEQ ID 836
CACTATCGCCCTGTTCTCCCACCGC
SEQ ID 837
TCGCCCTGTTCTCCCACCGCATTCC
SEQ ID 838
CTGTTCTCCCACCGCATTCCCCTTG
SEQ ID 839
CTCCCACCGCATTCCCCTTGCTGAG
SEQ ID 840
TAGTAATCTGTAGATACCAAGGGAA
SEQ ID 841
ATCTGTAGATACCAAGGGAACTCAG
SEQ ID 842
TAGATACCAAGGGAACTCAGAGACC
SEQ ID 843
ACCAAGGGAACTCAGAGACCATGGC
SEQ ID 844
GGGAACTCAGAGACCATGGCCGGTG
SEQ ID 845
CTCAGAGACCATGGCCGGTGCACAT
SEQ ID 846
AGACCATGGCCGGTGCACATCCTCC
SEQ ID 847
ATGGCCGGTGCACATCCTCCGTACG
SEQ ID 848
CGGTGCACATCCTCCGTACGCTGAG
SEQ ID 849
CTGAGCGCTGGTCCCCTGGGCCCAT
SEQ ID 850
CGCTGGTCCCCTGGGCCCATTGTTC
SEQ ID 851
CCTCAGTCTCTCATCCCTCCTGACG
SEQ ID 852
GTCTCTCATCCCTCCTGACGAGAAA
SEQ ID 853
TCATCCCTCCTGACGAGAAATACCC

SEQ ID 854
AGGGGCTGGCCCCCTTCATCTGATG
SEQ ID 855
CTGGCCCCCTTCATCTGATGCCCAA
SEQ ID 856
TCATCTGATGCCCAATGTGGGTGCC
SEQ ID 857
TGATGCCCAATGTGGGTGCCTTTCT
SEQ ID 858
TTTCTCTAGGGTGAAGGTACTCTAC
SEQ ID 859
CTAGGGTGAAGGTACTCTACAGTGT
SEQ ID 860
GTGAAGGTACTCTACAGTGTGGTCA
SEQ ID 861
GGTACTCTACAGTGTGGTCATTGAG
SEQ ID 862
TCTACAGTGTGGTCATTGAGGACAA
SEQ ID 863
GACAAGTTGACGAGAGAGTCCCAAG
SEQ ID 864
GTTGACGAGAGAGTCCCAAGTACGT
SEQ ID 865
CGAGAGAGTCCCAAGTACGTCCACG
SEQ ID 866
CTTAGAGGAACCCAGGGTAACGATG
SEQ ID 867
CAAACAGGAGAAGATATTGTTTCAG
SEQ ID 868
AGGAGAAGATATTGTTTCAGTTTCT
SEQ ID 869
GATGCCCCTAAAAGCTGTGTAACAG
SEQ ID 870
CCCTAAAAGCTGTGTAACAGATTGT
SEQ ID 871
GAAGAAGAGGCAGGGACAGAATCCC
SEQ ID 872
AGAGGCAGGGACAGAATCCCAGCAA
SEQ ID 873
CAGGGACAGAATCCCAGCAAGGAAC
SEQ ID 874
ACAGAATCCCAGCAAGGAACGGAAA
SEQ ID 875
ATCCCAGCAAGGAACGGAAAGTTCA
SEQ ID 876
TGACTACAGTCAATTACAGGAGATA
SEQ ID 877
ACAGGAGATAATATACCCTGAATCA
SEQ ID 878
ACCACGATCGCCATCAACTCCTCCT
SEQ ID 879
GATCGCCATCAACTCCTCCTCCCGT

SEQ ID 880
CCATCAACTCCTCCTCCCGTGGTTC
SEQ ID 881
AACTCCTCCTCCCGTGGTTCAGATG
SEQ ID 882
CCTCAAACGCAGGTTAGACAAGCAC
SEQ ID 883
AACGCAGGTTAGACAAGCACAAACC
SEQ ID 884
AGACAAGCACAAACCCCAAGAGAAA
SEQ ID 885
AGCACAAACCCCAAGAGAAAATCAA
SEQ ID 886
CCAAGAGAAAATCAAGTAGAAAGGG
SEQ ID 887
AGAAAATCAAGTAGAAAGGGACAGA
SEQ ID 888
ATCAAGTAGAAAGGGACAGAGTCTC
SEQ ID 889
GTAGAAAGGGACAGAGTCTCTATCC
SEQ ID 890
AAGGGACAGAGTCTCTATCCCGGCA
SEQ ID 891
TATCCCGGCAATGCCAACTCAGATA
SEQ ID 892
CGGCAATGCCAACTCAGATACAGTA
SEQ ID 893
AAAATAAGACCCAACCGCTGGTAGT
SEQ ID 894
AAGACCCAACCGCTGGTAGTTTATC
SEQ ID 895
CGCTGGTAGTTTATCAATACCGGCT
SEQ ID 896
GTAGTTTATCAATACCGGCTGCCAA
SEQ ID 897
TTATCAATACCGGCTGCCAACCGAG
SEQ ID 898
AATACCGGCTGCCAACCGAGCTTCA
SEQ ID 899
CGGCTGCCAACCGAGCTTCAGTATC
SEQ ID 900
GCCAACCGAGCTTCAGTATCGGCCT
SEQ ID 901
CCGAGCTTCAGTATCGGCCTCCTTC
SEQ ID 902
CTTCAGTATCGGCCTCCTTCAGAGG
SEQ ID 903
GTATCGGCCTCCTTCAGAGGTTCAA
SEQ ID 904
GGCCTCCTTCAGAGGTTCAATACAG
SEQ ID 905
CCTTCAGAGGTTCAATACAGACCTC

SEQ ID 906
CACCATACCAGCAACCCACAGCGAT
SEQ ID 907
GCAACCCACAGCGATGGCGTCTAAT
SEQ ID 908
CCACAGCGATGGCGTCTAATTCACC
SEQ ID 909
GCGATGGCGTCTAATTCACCAGCAA
SEQ ID 910
GGCGTCTAATTCACCAGCAACACAG
SEQ ID 911
CTAATTCACCAGCAACACAGGACGC
SEQ ID 912
TCACCAGCAACACAGGACGCGGCGC
SEQ ID 913
AGCAACACAGGACGCGGCGCTGTAT
SEQ ID 914
CACAGGACGCGGCGCTGTATCCTCA
SEQ ID 915
GACGCGGCGCTGTATCCTCAGCCGC
SEQ ID 916
GGCGCTGTATCCTCAGCCGCCCACT
SEQ ID 917
ATCACGTAGTGGACAGGGTGGTGCA
SEQ ID 918
GTAGTGGACAGGGTGGTGCACTGCA
SEQ ID 919
GGACAGGGTGGTGCACTGCATGCAG
SEQ ID 920
GGGTGGTGCACTGCATGCAGTCATT
SEQ ID 921
GTGCACTGCATGCAGTCATTGATGA
SEQ ID 922
AAATGGTCTTTTTACTCCCTGGAAA
SEQ ID 923
GTCTTTTTACTCCCTGGAAAGCCCC
SEQ ID 924
AGGGAGGGTAGGCCTTTGAGGGAGA
SEQ ID 925
GGGTAGGCCTTTGAGGGAGATCAAG
SEQ ID 926
GGCCTTTGAGGGAGATCAAGTCTAA
SEQ ID 927
CAGGAAAAGCTGCTTATTGGGCTAA
SEQ ID 928
AAAGCTGCTTATTGGGCTAATCAGG
SEQ ID 929
TGTTTCTGTCATCGGCATAGGTACT
SEQ ID 930
CTGTCATCGGCATAGGTACTGCCTC
SEQ ID 931
ATCGGCATAGGTACTGCCTCAGAAG

SEQ ID 932
GGTTCAGCCTGTGATCACTTCATTC
SEQ ID 933
AGCCTGTGATCACTTCATTCCAATC
SEQ ID 934
GTGATCACTTCATTCCAATCAATTT
SEQ ID 935
CACTTCATTCCAATCAATTTATGGG
SEQ ID 936
AGGGACTAGGAAAGAAGTCCCAATT
SEQ ID 937
CTAGGAAAGAAGTCCCAATTGAGGC
SEQ ID 938
CCATTCCATTAACTTGGGGGAAAAA
SEQ ID 939
CCATTAACTTGGGGGAAAAAAAAAC
SEQ ID 940
AACTTGGGGGAAAAAAAAACAACTG
SEQ ID 941
GGGGGAAAAAAAAACAACTGTATGG
SEQ ID 942
AAAACAACTGTATGGTAAATCAGCA
SEQ ID 943
AACTGTATGGTAAATCAGCAGCGCT
SEQ ID 944
TATGGTAAATCAGCAGCGCTTCCAA
SEQ ID 945
TAAATCAGCAGCGCTTCCAAAACAA
SEQ ID 946
CAGCAGCGCTTCCAAAACAAAAACT
SEQ ID 947
ATTAGAAAAAGGACATTGAGCCTTC
SEQ ID 948
AAAAAGGACATTGAGCCTTCATTTT
SEQ ID 949
ATTTTCGCCTTGGAATTCTGTTTGT
SEQ ID 950
CGCCTTGGAATTCTGTTTGTAATTC
SEQ ID 951
AAATCCGGCAGATGGCGTATAATGC
SEQ ID 952
CGGCAGATGGCGTATAATGCCGTAA
SEQ ID 953
GATGGCGTATAATGCCGTAATTCAA
SEQ ID 954
TTTGCTTTTACCACACCAGCCTAAA
SEQ ID 955
TTTTACCACACCAGCCTAAATAATA
SEQ ID 956
AATAGTTCAACTATTTGTCAGCTCA
SEQ ID 957
TTCAACTATTTGTCAGCTCAAGCTC

SEQ ID 958
CTATTTGTCAGCTCAAGCTCTGCAA
SEQ ID 959
TTTTCAGACTGTTACATCGTTCACT
SEQ ID 960
AGACTGTTACATCGTTCACTATGTT
SEQ ID 961
CTCTACTCCTTTCCGTTACTTGGGA
SEQ ID 962
TCGGAATTAAATAGTGAAAGAACGT
SEQ ID 963
ATAGTGAAAGAACGTTAACTCCAGA
SEQ ID 964
GAAAGAACGTTAACTCCAGAGGCAA
SEQ ID 965
TTTTGCTACTGCACATTCCCTAACA
SEQ ID 966
CTACTGCACATTCCCTAACAGGCAT
SEQ ID 967
GCACATTCCCTAACAGGCATCATTG
SEQ ID 968
TTCCCTAACAGGCATCATTGTTCAA
SEQ ID 969
ATTATTGACAATCGTTACCCCAAAA
SEQ ID 970
TGACAATCGTTACCCCAAAACAAAA
SEQ ID 971
ATCGTTACCCCAAAACAAAAATCTT
SEQ ID 972
ACCTAAAGTTACCAAACATAAGCCT
SEQ ID 973
ACATAAGCCTTTAAAAAATGCTCTG
SEQ ID 974
AGCCTTTAAAAAATGCTCTGGCAGT
SEQ ID 975
GGGCCAAAAGAATGAGTCATCAAAA
SEQ ID 976
AAAAGAATGAGTCATCAAAACTCAG
SEQ ID 977
AATGAGTCATCAAAACTCAGTATCA
SEQ ID 978
GTCATCAAAACTCAGTATCACTTGA
SEQ ID 979
CAAAACTCAGTATCACTTGACTCAA
SEQ ID 980
CTCAGTATCACTTGACTCAAAGAGC
SEQ ID 981
TATCACTTGACTCAAAGAGCAGAGT
SEQ ID 982
CTTGACTCAAAGAGCAGAGTTGGTT
SEQ ID 983
TGGTTGCCGTCATTACAGTGTTAAC

SEQ ID 984
GCCGTCATTACAGTGTTAACAAGAT
SEQ ID 985
CAGGCTACAAAGGATATTGAGAGAG
SEQ ID 986
TACAAAGGATATTGAGAGAGCCCTA
SEQ ID 987
AGGATATTGAGAGAGCCCTAATCAA
SEQ ID 988
ATTGAGAGAGCCCTAATCAAATACA
SEQ ID 989
TTATGGATGATCAGTTAAACCCGCT
SEQ ID 990
TCAGTTAAACCCGCTGTTTAATTTG
SEQ ID 991
TAAACCCGCTGTTTAATTTGTTACA
SEQ ID 992
TTGACTCATGTAAATGCAATAGGAT
SEQ ID 993
CATTGCACCCAGTGTCAGATTCTAC
SEQ ID 994
AGTGTCAGATTCTACACCTGGCCAC
SEQ ID 995
CAGATTCTACACCTGGCCACTCAGG
SEQ ID 996
TCTACACCTGGCCACTCAGGAGGCA
SEQ ID 997
ACCTGGCCACTCAGGAGGCAAGAGT
SEQ ID 998
GCCACTCAGGAGGCAAGAGTTAATC
SEQ ID 999
ATGGCAAATGGATGTCATGCACGTA
SEQ ID 1000
AAATGGATGTCATGCACGTACCTTC
SEQ ID 1001
GATGTCATGCACGTACCTTCATTTG
SEQ ID 1002
CATGCACGTACCTTCATTTGGAAAA
SEQ ID 1003
GTTCCAGAAAAAGTTAAAACAGACA
SEQ ID 1004
AGAAAAAGTTAAAACAGACAATGGG
SEQ ID 1005
AAGTTAAAACAGACAATGGGCCAGG
SEQ ID 1006
AAAACAGACAATGGGCCAGGTTACT
SEQ ID 1007
AGACAATGGGCCAGGTTACTGTAGT
SEQ ID 1008
CCAGGTTACTGTAGTAAAGCAGTTC
SEQ ID 1009
AAACAAAAAAAGGAAAAGACAGGA

SEQ ID 1010
AAGGAAAAGACAGGAGTATAACACT
SEQ ID 1011
AAAGACAGGAGTATAACACTCCCCA
SEQ ID 1012
CACTACCTCTGCAGAACAACATCTT
SEQ ID 1013
AAAATAAAACATGGGAAATGGGGAA
SEQ ID 1014
AAAACATGGGAAATGGGGAAGGTGA
SEQ ID 1015
TAAAGTTCTACAATGAACTCACTGG
SEQ ID 1016
TTCTACAATGAACTCACTGGAGATG
SEQ ID 1017
CAATGAACTCACTGGAGATGCAAAG
SEQ ID 1018
AACTCACTGGAGATGCAAAGAAAAG
SEQ ID 1019
ACTGGAGATGCAAAGAAAAGTGTGG
SEQ ID 1020
AGATGCAAAGAAAAGTGTGGAGATG
SEQ ID 1021
CAAAGAAAAGTGTGGAGATGGAGAC
SEQ ID 1022
AAAAGTGTGGAGATGGAGACACCCC
SEQ ID 1023
TGTGGAGATGGAGACACCCCAATCG
SEQ ID 1024
AGATGGAGACACCCCAATCGACTCG
SEQ ID 1025
GAGACACCCCAATCGACTCGCCAGG
SEQ ID 1026
ACCCCAATCGACTCGCCAGGTAAAC
SEQ ID 1027
AATCGACTCGCCAGGTAAACAAAAT
SEQ ID 1028
GGTGATATCAGAAGAACAGAAAAAG
SEQ ID 1029
TATCAGAAGAACAGAAAAAGTTGCC
SEQ ID 1030
GAAGAACAGAAAAAGTTGCCTTCCA
SEQ ID 1031
ACAGAAAAAGTTGCCTTCCATCAAG
SEQ ID 1032
AAAAGTTGCCTTCCATCAAGGAAGC
SEQ ID 1033
TTGCCTTCCATCAAGGAAGCAGAGT
SEQ ID 1034
TTCCATCAAGGAAGCAGAGTTGCCA
SEQ ID 1035
TCAAGGAAGCAGAGTTGCCAATATA

SEQ ID 1036
GAAGCAGAGTTGCCAATATAGGCAC
SEQ ID 1037
AGAGTTGCCAATATAGGCACAATTA
SEQ ID 1038
TGCCAATATAGGCACAATTAAAGAA
SEQ ID 1039
ATATAGGCACAATTAAAGAAGCTGA
SEQ ID 1040
CACAGTTAGCTAAAAAAAAAGCCT
SEQ ID 1041
AAAAAAGCCTAGAGAATACAAAGGT
SEQ ID 1042
AGCCTAGAGAATACAAAGGTGACAC
SEQ ID 1043
AGAGAATACAAAGGTGACACCAACT
SEQ ID 1044
ATACAAAGGTGACACCAACTCCAGA
SEQ ID 1045
AAGGTGACACCAACTCCAGAGAATA
SEQ ID 1046
GACACCAACTCCAGAGAATATGCTG
SEQ ID 1047
GAATATGCTGCTTGCAGCTCTGATG
SEQ ID 1048
ATCAACGGTGGTAAGTCTTCCCAAG
SEQ ID 1049
CGGTGGTAAGTCTTCCCAAGTCTGC
SEQ ID 1050
GTAAGTCTTCCCAAGTCTGCAGGAG
SEQ ID 1051
TCTTCCCAAGTCTGCAGGAGCAGCT
SEQ ID 1052
CCTTAATTCGGGCAGTTACATAGAT
SEQ ID 1053
ATTCGGGCAGTTACATAGATGGATA
SEQ ID 1054
GGCAGTTACATAGATGGATAATCCT
SEQ ID 1055
TAGTGCATGGGTGCCTGGCCCCACA
SEQ ID 1056
CATGGGTGCCTGGCCCCACAGATGA
SEQ ID 1057
GTGCCTGGCCCCACAGATGACTGTT
SEQ ID 1058
TGGCCCCACAGATGACTGTTGCCCT
SEQ ID 1059
GCCCAACCTGAAGAAGGAATGATGA
SEQ ID 1060
CATTGGGTATCCTTATCCTCCTGTT
SEQ ID 1061
GGTATCCTTATCCTCCTGTTTGCCT

SEQ ID 1062
CCTTATCCTCCTGTTTGCCTAGGGA
SEQ ID 1063
CCTACAGTCAGTGCTACCAGTAGAT
SEQ ID 1064
AGTCAGTGCTACCAGTAGATTTACT
SEQ ID 1065
CATGGTAAGTGGAATGTCACAGATA
SEQ ID 1066
TCATTACAATGTAGGCCTAAGGGGA
SEQ ID 1067
ACAATGTAGGCCTAAGGGGAAGGCT
SEQ ID 1068
GTAGGCCTAAGGGGAAGGCTTGCCC
SEQ ID 1069
CAAAAAGCCCAGAAGTCTTAGTCTG
SEQ ID 1070
AGCCCAGAAGTCTTAGTCTGCGGAG
SEQ ID 1071
AGAAGTCTTAGTCTGCGGAGAATGT
SEQ ID 1072
TCTTAGTCTGCGGAGAATGTGTGGC
SEQ ID 1073
GTCTGCGGAGAATGTGTGGCTGATA
SEQ ID 1074
GTGGCTGATACTGCAGTGTAGTACA
SEQ ID 1075
TGATACTGCAGTGTAGTACAAAACA
SEQ ID 1076
CTGCAGTGTAGTACAAAACAATGAA
SEQ ID 1077
TTTTGAACTATGATAGACTGGGTCC
SEQ ID 1078
AACTATGATAGACTGGGTCCCTTGA
SEQ ID 1079
TGATAGACTGGGTCCCTTGAGGCCA
SEQ ID 1080
GGTCCCTTGAGGCCAATTATATCAT
SEQ ID 1081
CTTGAGGCCAATTATATCATAACTG
SEQ ID 1082
ATTATATCATAACTGTACAGGCCAG
SEQ ID 1083
ATCATAACTGTACAGGCCAGACTCA
SEQ ID 1084
AACTGTACAGGCCAGACTCATTCAT
SEQ ID 1085
TACAGGCCAGACTCATTCATGTTCA
SEQ ID 1086
TGGCCCATTAATCCAGCCTATGACG
SEQ ID 1087
CATTAATCCAGCCTATGACGGTGAT

SEQ ID 1088
ATCCAGCCTATGACGGTGATGTAAC
SEQ ID 1089
GCCTATGACGGTGATGTAACTGAAA
SEQ ID 1090
TGACGGTGATGTAACTGAAAGGCTG
SEQ ID 1091
ATAGAAGGTTAGAATCACTCTGTCC
SEQ ID 1092
AGGTTAGAATCACTCTGTCCAAGGA
SEQ ID 1093
AGAATCACTCTGTCCAAGGAAATGG
SEQ ID 1094
CACTCTGTCCAAGGAAATGGGGTGA
SEQ ID 1095
TGTCCAAGGAAATGGGGTGAAAAGG
SEQ ID 1096
TCATCACCTTGACCAAAGTTAGTCC
SEQ ID 1097
ACCTTGACCAAAGTTAGTCCTGTTA
SEQ ID 1098
GACCAAAGTTAGTCCTGTTACTGGT
SEQ ID 1099
CCTGAACATCCAGAATTAGGAAGCT
SEQ ID 1100
ACATCCAGAATTAGGAAGCTTACTG
SEQ ID 1101
CAGAATTAGGAAGCTTACTGTGGCC
SEQ ID 1102
TTAGGAAGCTTACTGTGGCCTCACA
SEQ ID 1103
TTCTGGAAATCAAGCTATAGGAACA
SEQ ID 1104
TCCTTTGCAAAATTGTGTAAAACTC
SEQ ID 1105
TGCAAAATTGTGTAAAACTCCCTTA
SEQ ID 1106
AACTCCCTTATATTGCTAGTTGTAG
SEQ ID 1107
GTTATTAAACCTGATTCCCAAACCA
SEQ ID 1108
TAAACCTGATTCCCAAACCATAATC
SEQ ID 1109
CTGATTCCCAAACCATAATCTGTGA
SEQ ID 1110
TCCCAAACCATAATCTGTGAAAATT
SEQ ID 1111
AACCATAATCTGTGAAAATTGTGGA
SEQ ID 1112
TAATCTGTGAAAATTGTGGAATGTT
SEQ ID 1113
TGTGAAAATTGTGGAATGTTTACTT

SEQ ID 1114
AAATTGTGGAATGTTTACTTGCATT
SEQ ID 1115
GTGGAATGTTTACTTGCATTGATTT
SEQ ID 1116
GCACCGTATTCTACTAGGAAGAGCA
SEQ ID 1117
GTATTCTACTAGGAAGAGCAAGAGA
SEQ ID 1118
CTACTAGGAAGAGCAAGAGAGGGTG
SEQ ID 1119
AGGAAGAGCAAGAGAGGGTGTGTGG
SEQ ID 1120
ATCCTTGTGTCCATGGACCGACCAT
SEQ ID 1121
GACCGACCATGGGAGGCTTCGCTAT
SEQ ID 1122
ACCATGGGAGGCTTCGCTATCCATC
SEQ ID 1123
GGGAGGCTTCGCTATCCATCCATAT
SEQ ID 1124
GCTTCGCTATCCATCCATATTTTAA
SEQ ID 1125
GCTATCCATCCATATTTTAACGGAA
SEQ ID 1126
TTGATGGCAGTGATTATGGGCCTCA
SEQ ID 1127
GGCAGTGATTATGGGCCTCATTGCA
SEQ ID 1128
CAGAATACGTAAATGATTGGCAAAA
SEQ ID 1129
TACGTAAATGATTGGCAAAAGAATT
SEQ ID 1130
TCATTTGGATGGGAGAGGCTCATGA
SEQ ID 1131
TGGATGGGAGAGGCTCATGAGCTTG
SEQ ID 1132
GGGAGAGGCTCATGAGCTTGGAATA
SEQ ID 1133
TCTTTTTCAGTTACGATGTGACTGG
SEQ ID 1134
TTCAGTTACGATGTGACTGGAATAC
SEQ ID 1135
TTACGATGTGACTGGAATACATCAG
SEQ ID 1136
ATCAGATTTTTGTGTTACACCACAA
SEQ ID 1137
ATTTTTGTGTTACACCACAAGCCTA
SEQ ID 1138
TGTGTTACACCACAAGCCTATAATG
SEQ ID 1139
ATGGTTAGATGCCATCTGCAAGGAG

SEQ ID 1140
TAGATGCCATCTGCAAGGAGGAGAA
SEQ ID 1141
GCCATCTGCAAGGAGGAGAAGATAA
SEQ ID 1142
CTGCAAGGAGGAGAAGATAATCTTA
SEQ ID 1143
TAAATTTGGTGCCAGGAACGGAGAC
SEQ ID 1144
TTGGTGCCAGGAACGGAGACAATCG
SEQ ID 1145
GCCAGGAACGGAGACAATCGTGAAA
SEQ ID 1146
GAACGGAGACAATCGTGAAAGCTGC
SEQ ID 1147
GAGACAATCGTGAAAGCTGCTGATA
SEQ ID 1148
GCCTCACAAATCTTAAGCCAGTCAC
SEQ ID 1149
ACAAATCTTAAGCCAGTCACTTGGG
SEQ ID 1150
TCTTAAGCCAGTCACTTGGGTTAAA
SEQ ID 1151
AGCCAGTCACTTGGGTTAAAAGCAT
SEQ ID 1152
TTGGGTTAAAAGCATCAGAAGTTTC
SEQ ID 1153
AGCATCAGAAGTTTCACTATTGTAA
SEQ ID 1154
AGCTCCAAAGAGACAGCAACCAGCA
SEQ ID 1155
CAAAGAGACAGCAACCAGCAAGAAT
SEQ ID 1156
AGACAGCAACCAGCAAGAATGGGCC
SEQ ID 1157
GCAACCAGCAAGAATGGGCCATAGT
SEQ ID 1158
CAGCAAGAATGGGCCATAGTGACGA
SEQ ID 1159
AGAATGGGCCATAGTGACGATGGTG
SEQ ID 1160
GGGCCATAGTGACGATGGTGGTTTT
SEQ ID 1161
ATAGTGACGATGGTGGTTTTGTCAA
SEQ ID 1162
GACGATGGTGGTTTTGTCAAAAAGA
SEQ ID 1163
GTCAAAAAGAAAAGGGGGGGATATG
SEQ ID 1164
AAAGAAAAGGGGGGGATATGTAAGG
SEQ ID 1165
AAAGGGGGGGATATGTAAGGAAAAG

SEQ ID 1166
TAAGGAAAAGAGAGATCAGACTTTC
SEQ ID 1167
AAAAGAGAGATCAGACTTTCACTGT
SEQ ID 1168
CTTTCACTGTGTCTATGTAGAAAAG
SEQ ID 1169
ACTAAGAAAAATTGTTTTGCCTTGA
SEQ ID 1170
TGCTCACGGAAACATGTGCTGTAAG
SEQ ID 1171
ACGGAAACATGTGCTGTAAGGTTTA
SEQ ID 1172
AACATGTGCTGTAAGGTTTAAGGGA
SEQ ID 1173
GTGCTGTAAGGTTTAAGGGATCTAG
SEQ ID 1174
TGCAGGATGTACCTTGTTAACAATA
SEQ ID 1175
ACCTTGTTAACAATATGTTTGCAGG
SEQ ID 1176
CAATATGTTTGCAGGCAGTATGTTT
SEQ ID 1177
TGTTTGCAGGCAGTATGTTTGGTAA
SEQ ID 1178
GCAGGCAGTATGTTTGGTAAAAGTC
SEQ ID 1179
ATTAACCAGGGGCTCAATGCACTGT
SEQ ID 1180
GGCTCAATGCACTGTGGAAAGCCAC
SEQ ID 1181
AATGCACTGTGGAAAGCCACAGGAA
SEQ ID 1182
GGAAAGCCACAGGAACCTCTGCCCA
SEQ ID 1183
GCCACAGGAACCTCTGCCCAAGAAA
SEQ ID 1184
AGGAACCTCTGCCCAAGAAAGCCTG
SEQ ID 1185
AGCTTACAAAACAAAATGGGGCAAACTGAAAGTAAATATGCCTCTTATCTCAGCTTTATTAAAATTCTTTTAAAAAGAGGGGGAGTTAG
AGTATCTACAAAAAATCTAATCAAGCTATTTCAAATAATAGAACAATTTTGCCCATGGTTTCCAGAACAAGGAACTTTAGATCTAAAAG
ATTGGAAAAGAATTGGCGAGGAACTAAAACAAGCAGGTAGAAAGGGTAATATCATTCCACTTACAGTATGGAATGATTGGGCCATTATT
AAAGCAGCTTTAGAACCATTTCAAACAAAAGAAGATAGCGTTTCAGTTTCTGATGCCCCTGGAAGCTGTGTAATAGATTGTAATGAAAA
GACAGGGAGAAAATCCCAGAAAGAAACAGAAAGTTTACATTGCGAATATGTAACAGAGCCAGTAATGGCTCAGTCAACGCAAAATGTTG
ACTATAATCAATTACAGGGGGTGATATATCCTGAAACGTTAAAATTAGAAGGAAAAGGTCCAGAATTAGTGGGGCCATCAGAGTCTAAA
CCACGAGGGCCAAGTCCTCTTCCAGCAGGTCAGGTGCCCGTAACATTACAACCTCAAACGCAGGTTAAAGAAAATAAGACCCAACCGCC
AGTAGCTTATCAATACTGGCCGCCGGCTGAACTTCAGTATCTGCCACCCCCAGAAAGTCAGTATGGATATCCAGGAATGCCCCCAGCAC
TACAGGGCAGGGCGCCATATCCTCAGCCGCCCACTGTGAGACTTAATCCTACAGCATCACGTAGTGGACAAGGTGGTACACTGCACGCA
GTCATTGATGAAGCCAGAAAACAGGGAGATCTTGAGGCATGGCGGTTCCTGGTAATTTTACAACTGGTACAGGCCGGGGAAGAGACTCA
AGTAGGAGCGCCTGCCCGAGCTGAGACTAGATGTGAACCTTTCACCATGAAAATGTTAAAAGATATAAAGGAAGGAGTTAAACAATATG
GATCCAACTCCCCTTATATAAGAACATTATTAGATTCCATTGCTCATGGAAATAGACTTACTCCTTATGACTGGGAAAGTTTGGCCAAA
TCTTCCCTTTCATCCTCTCAGTATCTACAGTTTAAAACCT[?][?][?][?]GATTGATGGAGTACAAGAACAGGTACGAAAAAATCAGGCTACTAA

GCCCACTGTTAATATAGACGCAGACCAATTGTTAGGAACAGGTCCAAATTGGAGCACCATTAACCAACAATCAGTGATGCAGAATGAGG
CTATTGAACAAGTAAGGGCTATTTGCCTCAGGGCCTGGGGAAAAATTCAGGACCCAGGAACAGCTTTCCCTATTAATTCAATTAGACAA
GGCTCTAAAGAGCCATATCCTGACTTTGTGGCAAGATTACAAGATGCTGCTCAAAAGTCTATTACAGATGACAATGCCCGAAAAGTTAT
TGTAGAATTAATGGCCTATGAAAATGCAAATCCAGAATGTCAGTCGGCCATAAAGCCATTAAAAGGAAAAGTTCCAGCAGGAGTTGATG
TAATTACAGAATATGTGAAGGCTTGTGATGGGATTGGAGGAGCTATGCATAAGGCAATGCTAATGGCTCAAGCAATGAGGGGGCTCACT
CTAGGAGGACAAGTTAGAACATTTGGGAAAAAATGTTATAATTGTGGTCAAATCGGTCATCTGAAAAGGAGTTGCCCAGTCTTAAATAA
ACAGAATATAATAAATCAAGCTATTACAGCAAAAAATAAAAAGCCATCTGGCCTGTGTCCAAAATGTGGAAAAGGAAAACATTGGGCCA
ATCAATGTCATTCTAAATTTGATAAGGATGGGCAACCATTGTCGGGAAACAGGAAGAGGGGCCAGCCTCAGGCCCCCCAACAAACTGGG
GCATTCCCAGTTCAACTGTTTGTTCCTCAGGGTTTTCAAGGACAACAACCCCTACAGAAAATACCACCACTTCAGGGAGTCAGCCAATT
ACAACAATCCAACAGCTGTCCCGCGCCACAGCAGGCAGCACCGCAGTAGTAAGTCGAC
SEQ ID 1186
MGQTESKYASYLSFIKILLKRGGVRVSTKNLIKLFQIIEQFCPWFPEQGTLDLKDWKRIGEELKQAGRKGNIIPLTVWNDWAIIKAALE
PFQTKEDSVSVSDAPGSCVIDCNEKTGRKSQKETESLHCEYVTEPVMAQSTQNVDYNQLQGVIYPETLKLEGKGPELVGPSESKPRGPS
PLPAGQVPVTLQPQTQVKENKTQPPVAYQYWPPAELQYLPPPESQYGYPGMPPALQGRAPYPQPPTVRLNPTASRSGQGGTLHAVIDEA
RKQGDLEAWRFLVILQLVQAGEETQVGAPARAETRCEPFTMKMLKDIKEGVKQYGSNSPYIRTLLDSIAHGNRLTPYDWESLAKSSLSS
SQYLQFKTWWIDGVQEQVRKNQATKPTVNIDADQLLGTGPNWSTINQQSVMQNEAIEQVRAICLRAWGKIQDPGTAFPINSIRQGSKEP
YPDFVARLQDAAQKSITDDNARKVIVELMAYENANPECQSAIKPLKGKVPAGVDVITEYVKACDGIGGAMHKAMLMAQAMRGLTLGGQV
RTFGKKCYNCGQIGHLKRSCPVLNKQNIINQAITAKNKKPSGLCPKCGKGKHWANQCHSKFDKDGQPLSGNRKRGQPQAPQQTGAFPVQ
LFVPQGFQGQQPLQKIPPLQGVSQLQQSNSCPAPQQAAPQ
SEQ ID 1187
AGCTTACAAAACAAAATGGGGCAAACTGAAAGTAAATATGCCTCTTATCTCAGCTTTATTAAAATTCTTTTAAGAAGAGGGGGAGTTAG
AGCTTCTACAGAAAATCTAATTACGCTATTTCAAACAATAGAACAATTCTGCCCATGGTTTCCAGAACAGGGAACTTTAGATCTAAAAG
ATTGGGAAAAAATTGGCAAAGAATTAAAACAAGCAAATAGGGAAGGTAAAATCATCCCACTTACAGTATGGAATGATTGGGCCATTATT
AAAGCAACTTTAGAACCATTTCAAACAGGAGAAGATATTGTTTCAGTTTCTGATGCCCCTAAAAGCTGTGTAACAGATTGTGAAGAAGA
GGCAGGGACAGAATCCCAGCAAGGAACGGAAAGTTCACATTGTAAATATGTAGCAGAGTCTGTAATGGCTCAGTCAACGCAAAATGTTG
ACTACAGTCAATTACAGGAGATAATATACCCTGAATCATCAAAATTGGGGGAAGGAGGTCCAGAATCATTGGGGCCATCAGAGCCTAAA
CCACGATCGCCATCAACTCCTCCTCCCGTGGTTCAGATGCCTGTAACATTACAACCTCAAACGCAGGTTAGACAAGCACAAACCCCAAG
AGAAAATCAAGTAGAAAGGGACAGAGTCTCTATCCCGGCAATGCCAACTCAGATACAGTATCCACAATATCAGCCGGTAGAAAATAAGA
CCCAACCGCTGGTAGTTTATCAATACCGGCTGCCAACCGAGCTTCAGTATCGGCCTCCTTCAGAGGTTCAATACAGACCTCAAGCGGTG
TGTCCTGTGCCAAATAGCACGGCACCATACCAGCAACCCACAGCGATGGCGTCTAATTCACCAGCAACACAGGACGCGGCGCTGTATCC
TCAGCCGCCCACTGTGAGACTTAATCCTACAGCATCACGTAGTGGACAGGGTGGTGCACTGCATGCAGTCATTGATGAAGCCAGAAAAC
AGGGCGATCTTGAGGCATGGCGGTTCCTGGTAATTTTACAACTGGTACAGGCCGGGGAAGAGACTCAAGTAGGAGCGCCTGCCCGAGCT
GAGACTAGATGTGAACCTTTCACCATGAAAATGTTAAAAGATATAAAGGAAGGAGTTAAACAATATGGATCCAACTCCCCTTATATAAG
AACATTATTAGATTCCATTGCTCATGGAAATAGACTTACTCCTTATGACTGGGAAATTTTGGCCAAATCTTCCCTTTCATCCTCTCAGT
ATCTACAGTTTAAAACCTGGTGGATTGATGGAGTACAAGAACAGGTACGAAAAAATCAGGCTACTAAGCCCACTGTTAATATAGACGCA
GACCAATTGTTAGGAACAGGTCCAAATTGGAGCACCATTAACCAACAATCAGTGATGCAGAATGAGGCTATTGAACAAGTAAGGGCTAT
TTGCCTCAGGGCCTGGGGAAAAATTCAGGACCCAGGAACAGCTTTCCCTATTAATTCAATTAGACAAGGCTCTAAAGAGCCATATCCTG
ACTTTGTGGCAAGATTACAAGATGCTGCTCAAAAGTCTATTACAGATGACAATGCCCGAAAAGTTATTGTAGAATTAATGGCCTATGAA
AATGCAAATCCAGAATGTCAGTCGGCCATAAAGCCATTAAAAGGAAAAGTTCCAGCAGGAGTTGATGTAATTACAGAATATGTGAAGGC
TTGTGATGGGATTGGAGGAGCTATGCATAAGGCAATGCTAATGGCTCAAGCAATGAGGGGGCTCACTCTAGGAGGACAAGTTAGAACAT
TTGGGAAAAAATGTTATAATTGTGGTCAAATCGGTCATCTGAAAAGGAGTTGCCCAGTCTTAAATAAACAGAATATAATAAATCAAGCT
ATTACAGCAAAAAATAAAAAGCCATCTGGCCTGTGTCCAAAATGTGGAAAAGGAAAACATTGGGCCAATCAATGTCATTCTAAATTTGA
TAAGGATGGGCAACCATTGTCGGGAAACAGGAAGAGGGGCCAGCCTCAGGCCCCCCAACAAACTGGGGCATTCCCAGTTCAACTGTTTG
TTCCTCAGGGTTTTCAAGGACAACAACCCCTACAGAAAATACCACCACTTCAGGGAGTCAGCCAATTACAACAATCCAACAGCTGTCCC
GCGCCACAGCAGGCAGCACCGCAGTAGTAAGTCGAC
SEQ ID 1188
MGQTESKYASYLSFIKILLRRGGVRASTENLITLFQTIEQFCPWFPEQGTLDLKDWEKIGKELKQANREGKIIPLTVWNDWAIIKATLE
PFQTGEDIVSVSDAPKSCVTDCEEEAGTESQQGTESSHCKYVAESVMAQSTQNVDYSQLQEIIYPESSKLGEGGPESLGPSEPKPRSPS
TPPPVVQMPVTLQPQTQVRQAQTPRENQVERDRVSIPAMPTQIQYPQYQPVENKTQPLVVYQYRLPTELQYRPPSEVQYRPQAVCPVPN
STAPYQQPTAMASNSPATQDAALYPQPPTVRLNPTASRSGQGGALHAVIDEARKQGDLEAWRFLVILQLVQAGEETQVGAPARAETRCE
PFTMKMLKDIKEGVKQYGSNSPYIRTLLDSIAHGNRLTPYDWEILAKSSLSSSQYLQFKTWWIDGVQEQVRKNQATKPTVNIDADQLLG
TGPNWSTINQQSVMQNEAIEQVRAICLRAWGKIQDPGTAFPINSIRQGSKEPYPDFVARLQDAAQKSITDDNARKVIVELMAYENANPE

CQSAIKPLKGKVPAGVDVITEYVKACDGIGGAMHKAMLMAQAMRGLTLGGQVRTFGKKCYNCGQIGHLKRSCPVLNKQNIINQAITAKN
KKPSGLCPKCGKGKHWANQCHSKFDKDGQPLSGNRKRGQPQAPQQTGAFPVQLFVPQGFQGQQPLQKIPPLQGVSQLQQSNSCPAPQQA
APQ
SEQ ID 1189
GKPIPNPLLGLDST
SEQ ID 1190
TGTGGGGAAAAGCAAGAGAGATCAGATTGTCACTGTATCTGTGTAGAAAGAAGTAGACATGGGAGACTCCATTTTGTTATGTACTAAGA
AAAATTCTTCTGCCTTGAGATTCTGTGACCTTACCCCCAACCCCGTGCTCTCTGAAACATGTGCTGTGTCAAACTCAGGGTTAAATGGA
TTAAGGGCGGTGCAGGATGTGCTTTGTTAAACAGATGCTTGAAGGCAGCATGCTCCTTAAGAGTCATCACCACTCCCTAATCTCAAGTA
CCCAGGGACACAAACACTGCGGAAGGCCGCAGGGACCTCTGCCTAGGAAAGCCAGGTATTGTCCAAGGTTTCTCCCCATGTGATAGTCT
GAAATATGGCCTCGTGGGAAGGGAAAGACCTGACCGTCCCCCAGCCCGACACCCGTAAAGGGTCTGTGCTGAGGAGGATTAGTAAAAGA
GGAAGGCATGCCTCTTGCAGTTGAGACAAGAGGAAGGCATCTGTCTCCTGCCCGTCCCTGGCAATGGAATGTCTCGGTATAAAACCGG
ATTGTACGTTCCATCTACTGAGATAGGGAAAAAACCGCCTTAGGGCTGGAGGTGGGACCTGCGGGCAGCAATACTGCTTTTTAAAGCATT
GAGATGTTTATGTGTATGCATATCTAAAAGCACAGCACTTAATCCTTTACCTTGTCTATGATGCAAAGATCTTTGTTCACGTGTTTGTC
TGCTGACCCTCTCCCCACTATTGTCTTGTGACCCTGACACATCCCCCTCTCGGAGAAACACCCACGAATGACCAATAAATACTAAAGGG
AACTCAGAGGCTGGCGGGATCCTCCATATGCTGAACGCTGGTTCCCCGGGCCCCCTTATTTCTTTCTCTACACTTTGTCTCTGTGTCTT
TTTCTTTCCTAAGTCTCTCGTTCCACCTTACGAGAAACACCCACAGGTGTGGAGGGGCAACCCACCCCTACA
SEQ ID 1191
GGGTGAAGGTACTCTACAGTGTGGTCATTGAGGACAAGTTGACGAGAGAGTCCCAAGTACGTCCACGGTCAGCCTTGCGACATTTAAAG
TTCTACAATGAACTCACTGGAGATGCAAAGAAAAGTGTGGAGATGGAGACACCCCAATCGACTCGCCAGTCTACAGGTGTATCCAGCAG
CTCCAAAGAGACAGCAACCAGCAAGAATGGGCCATAGTGACGATGGTGGTTTTGTCAAAAAGAAAAGGGGGGGATATGTAAGGAAAAGA
GAGATCAGACTTTCACTGTGTCTATGTAGAAAAGGAAGACATAAGAAACTCCATTTTGTTCTGTACTAAGAAAAATTGTTTTGCCTTGA
GATGCTGT
SEQ ID 1192
CTCGTTCCACCTGAGGAGAAATGCC
SEQ ID 1193
GAGGCGCAGGCCACTCCATC
SEQ ID 1194
CTTGTCCTCAATGACCACACTGTAGAG
SEQ ID 1195
AGAGTACCTTCACCCACAAGGCTC
SEQ ID 1196
TGTAGGGAAAAGAAAGAGAGATCACACTGTTACTGTGTCTATGTAGAAAAAGGAAGACATAAGAAACTCCATTTTGATCTGTACTAAGA
AAAATTCTTCTGCTTTGAAATGCTATTAATCTGTAACCCTAGCCCCAACCCTGTGCTCACAGAAACATGCGCTGTATTGACTCAAGGTT
AATGGATTTAGGGCTGTGCAGGATGTGCTTTGTTAACAATGTGTTTGAAGGCAGTATGCTTGGTAAAGGTCATCGCCATTCTCCAGTCT
TGAGTACCCAGGGACACAATGCACTGTGGAAAGCCATGGGGACCTCTGCCCAAGAAAGCCTGGGTGTTGTCCAGGCTTCCCCACACTGA
GACAGCCTGAGATGTGGCCTCGTTGGAAGGGAAAGACCTTACATTATAGTCCCCCAGCCGGACACCCATAAAAGGTCTGTGCTGAGGAG
GATTACTGAAAGAGGAAGGCCTCTTTGCAGTTAAGAGGAAAGCATCTGTCTCATGATCCCCTGGGAATGGAATGTCTTGGTGTAAAACC
TGATCGTACATTCTATTTACTGAGATAGGAGAAAACCGCCCTATGGCTGGAGGTGAGACATGCTGGTGGCAATACCGATCTTTACTGCA
CGGCAATACTGATCTTTACTGCACTGAGATGTTTATGTAAAGTTAAACATAAATCTAGCCTACGTGCACATTCAGGCATAGCACCTTTC
CTTAAACTTATTTATGACACAGAGTCTTTTGTTCACGTGTTTTCCTGTTGACCCTCTCTCCACCATTACCCTATAGTCCTGCCACATCC
CCCTCACTGAGATAGTAGAGATAATGATCAATAAATACTGAGGGAATTCAGAAACCAGTGCCGGTGCAGGTCCTCACTTGCTGAGTGCC
GGTCCCCTGGGCCCACTTTTCTTCCTCTATGCTTTACCTCTGTGTCTTATTTCTTTTCTCAGTCTCTCGTCTCCACCTTGCGAGAAATA
CCCACAGGTGTGGAGGGGCTGGCCCCCTTCA
SEQ ID 1197
UUAUGUAUAUGCACAUCAAAAGCACAGCACUUUUUUCUUUACCUUGUUUAUGAUGCAGAGACAUUUGUUCACAUGUUUUCCUGCUGGCC
CUCUCCCCACUAUUACCCUAUUGUCCUGCCACAUCCCCCUCUCCGAGAUGGUAGAGAUAAUGAUCAAUAAAUACUGAGGGAACUCAGAG
ACCGGUGCGGCGCGGGUCCUCCAUAUGCUGAGCGCCGGUCCCCUGGGCCCACUUUUCUUUCUCUAUACUUUGUCUCUGUUGUCUUUCUU
UUCUCAAGUCUCUCGUUCCACCUGAGGAGAAAUGCCCACAGCUGUGGAGGCGCAGGCCACUCCAUCUGGUGCCCAACGUGGAUGCUUUU
CUCUAGGGUGAAGGGACUCUCGAGUGUGGUCAUUGAGGACAAGUCAACGAGAGAUUCCCGAGUACGUCUACAGUGAGCCUUGUGGUAAG
CUUGGGCGCUCGGAAGAAGCCAGGGUUAAUGGGGCAAACUAAAAGUAAAGUCUCUCAUUCCACCUGAUGAGAAACACCCAGAGGUGUGG

AGGGGCAGGCCACCCCUUCAGGGUAGGGUCCCCUCCAUGCAGACCAUAGAGCACAGGUGUGCCCCAAAGAGGAGCAGAGAGAAGGAGGG
AGAGGGCCCACGAGAGACUUGGAAAUGAAUGGCAGGAUUUUAGGCGCUGGACUUGGGUUCGGGGCACCUGGCCUUUCCUUGUGUAUUUC
UCCUACUGUCUGCCUAACUAUUUAAUACAAUAAAAGAAAACCAGCCCCUGGUUCUUGUGGUGUUUCCACCCUCCCGGGUCCCCGCUGGC
UGCCUGGCUUCCUCCCGCAGCUCCUGCUGUGUGUGUAUGUGUGUGUGUGCACAUCUGUGGGGCGUAUGUGUGUUCGUCUUUGUAAUU
GAGGCUGCAGAGUGGAGAGAGCAGGGUUUUCUCUGGGGACCCAGAGAGAAGGAGGCGUUUUCACCACAGCCGAACAGGGCAGGACCCC
AGCACCCGGGACCCAGCGGGACUUUGCCAAGGGAUGGACCUGGCUGGGCCACGCGGCUGUUUGUGUAGGGAAAAGAAAGAGAGAUCAC
ACUGUUACUGUGUCUAUGUAGAAAAGGAAGACAUAAACUCCAUUUUGAGCUGUACUAAGAAAAAUUAUUUUGCCUUGACCUGCUGUUAA
CCUGUAACUGUAGCCCCAACCCUGUGCUCAAAGAAACAUGUGCUGUAUGGAAUCAAGGUUUAAGGGAUCAAGGGCUGUACAGGAUGUGC
CUUGUUAACAAUGUGUUUACAGGCAGUAUGCUUGGUAAAAGUCAUCGCCAUUCUCCAUUCUCCAUUAAUCAGGGGCACGAUGCACUGCG
GAAAGCCACAGGGACCUCUGCCCGAGAAAGCCUGGGUAUUGUCCAAGGCUUCCCCCCACUGAGACAGCCUGAGAUACGGCCUCGUGGGA
AGGGAAAGACCUGACCGUCCCCCAGCCCGACACCCGUAAAGGGUCUGUGCUGAGGAGGAUUAGUAAAAGGGGAAGGCCUCUUGCAGUUG
AGAUAAGAGGAAGGCCUCCGUCUCCUGCAUGUCCUUGGGAAUGGAAUGUCUUGGUGUAAAACCCGAUAGUACAUUCCUUCUAUUCUGAG
AGAAGAAAACCACCCUGUGGCUGGAGGUGAGAUAUGCUAGCGGCAAUGCUGCUCUGUUACUCUUUGCUACACUGAGAUGUUUGGGUGGA
GAGAAGCAUAAAUCUGGCCUAUGUGCACAUCUGGGCACAGAACCUCCCCUUGAACUUGUGACACAGAUUCCUUUGUUCACAUGUUUUCC
UGCUGACCUUCUCCCCACUAUCGCCCUGUUCUCCCACCGCAUUCCCCUUGCUGAGAUAGUGAAAAUAGUAAUCUGUAGAUACCAAGGGA
ACUCAGAGACCAUGGCCGGUGCACAUCCUCCGUACGCUGAGCGCUGGUCCCCUGGGCCCAUUGUUCUUUCUCUAUACUUUGUCUCUGUG
UCUUAUUUCUUUCCUCAGUCUCUCAUCCCUCCUGACGAGAAAUACCCACAGGUGUGGAGGGGCUGGCCCCCUUCAUCUGAUGCCCAAUG
UGGGUGCCUUUCUCUAGGGUGAAGGUACUCUACAGUGUGGUCAUUGAGGACAAGUUGACGAGAGAGUCCCAAGUACGUCCACGGUCAGC
CUUGCGGUAAGCUUGUGUGCUUAGAGGAACCCAGGGUAACGAUGGGGCAAACUGAAAGUAAAUAUGCCUCUUAUCUCAGCUUUAUUAAA
AUUCUUUUAAGAAGAGGGGGAGUUAGAGCUUCUACAGAAAAUCUAAUUACGCUAUUUCAAACAAUAGAACAAUUCUGCCCAUGGUUUCC
AGAACAGGGAACUUUAGAUCUAAAAGAUUGGGAAAAAAUUGGCAAAGAAUUAAAACAAGCAAAUAGGGAAGGUAAAAUCAUCCCACUUA
CAGUAUGGAAUGAUUGGGCCAUUAUUAAAGCAACUUUAGAACCAUUUCAAACAGGAGAAGAUAUUGUUUCAGUUUCUGAUGCCCCUAAA
AGCUGUGUAACAGAUUGUGAAGAAGAGGCAGGGACAGAAUCCCAGCAAGGAACGGAAAGUUCACAUUGUAAAUAUGUAGCAGAGUCUGU
AAUGGCUCAGUCAACGCAAAAUGUUGACUACAGUCAAUUACAGGAGAUAAUAUACCCUGAAUCAUCAAAAUUGGGGGAAGGAGGUCCAG
AAUCAUUGGGGCCAUCAGAGCCUAAACCACGAUCGCCAUCAACUCCUCCUCCCGUGGUUCAGAUGCCUGUAACAUUACAACCUCAAACG
CAGGUUAGACAAGCACAAACCCCAAGAGAAAAUCAAGUAGAAAGGGACAGAGUCUCUAUCCCGGCAAUGCCAACUCAGAUACAGUAUCC
ACAAUAUCAGCCGGUAGAAAAUAAGACCCAACCGCUGGUAGUUUAUCAAUACCGGCUGCCAACCGAGCUUCAGUAUCGGCCUCCUUCAG
AGGUUCAAUACAGACCUCAAGCGGUGUGUCCUGUGCCAAAUAGCACGGCACCAUACCAGCAACCCACAGCGAUGGCGUCUAAUUCACCA
GCAACACAGGACGCGGCGCUGUAUCCUCAGCCGCCCACUGUGAGACUUAAUCCUACAGCAUCACGUAGUGGACAGGGUGGUGCACUGCA
UGCAGUCAUUGAUGAAGCCAGAAAACAGGGCGAUCUUGAGGCAUGGCGGUUCCUGGUAAUUUUACAACUGGUACAGGCCGGGGAAGAGA
CUCAAGUAGGAGCGCCUGCCCGAGCUGAGACUAGAUGUGAACCUUUCACCAUGAAAAUGUUAAAAGAUAUAAAGGAAGGAGUUAAACAA
UAUGGAUCCAACUCCCCUUAUAUAAGAACAUUAUUAGAUUCCAUUGCUCAUGGAAAUAGACUUACUCCUUAUGACUGGGAAAUUUUGGC
CAAAUCUUCCCUUUCAUCCUCUCAGUAUCUACAGUUUAAAACCUGGUGGAUUGAUGGAGUACAAGAACAGGUACGAAAAAAUCAGGCUA
CUAAGCCCACUGUUAAUAUAGACGCAGACCAAUUGUUAGGAACAGGUCCAAAUUGGAGCACCAUUAACCAACAAUCAGUGAUGCAGAAU
GAGGCUAUUGAACAAGUAAGGGCUAUUUGCCUCAGGGCCUGGGGAAAAAUUCAGGACCCAGGAACAGCUUUCCCUAUUAAUUCAAUUAG
ACAAGGCUCUAAAGAGCCAUAUCCUGACUUUGUGGCAAGAUUACAAGAUGCUGCUCAAAAGUCUAUUACAGAUGACAAUGCCCGAAAAG
UUAUUGUAGAAUUAAUGGCCUAUGAAAAUGCAAAUCCAGAAUGUCAGUCGGCCAUAAAGCCAUUAAAAGGAAAAGUUCCAGCAGGAGUU
GAUGUAAUUACAGAAUAUGUGAAGGCUUGUGAUGGGAUUGGAGGAGCUAUGCAUAAGGCAAUGCUAAUGGCUCAAGCAAUGAGGGGGCU
CACUCUAGGAGGACAAGUUAGAACAUUUGGGAAAAAAUGUUAUAAUUGUGGUCAAAUCGGUCAUCUGAAAAGGAGUUGCCCAGGCUUAA
AUAAACAGAAUAUAAUAAAUCAAGCUAUUACAGCAAAAAAUAAAAAGCCAUCUGGCCUGUGUCCAAAAUGUGGAAAAGCAAAACAUUGG
GCCAAUCAAUGUCAUUCUAAAUUUGAUAAAGAUGGGCAACCAUUGUCUGGAAACAGGAAGAGGGGCCAGCCUCAGGCCCCCCAACAAAC
UGGGGCAUUCCCAGUUAAACUGUUUGUUCCUCAGGGUUUUCAAGGACAACAACCCCUACAGAAAAUACCACCACUUCAGGGAGUCAGCC
AAUUACAACAAUCCAACAGCUGUCCCGCGCCACAGCAGGCAGCACCGCAGUAGAUUUAUGUUCCACCCAAAUGGUCUUUUUACUCCCUG
GAAAGCCCCCACAAAAGAUUCCUAGAGGGGUAUAUGGCCCGCUGCCAGAAGGGAGGGUAGGCCUUUGAGGGAGAUCAAGUCUAAAUUUG
AAGGGAGUCCAAAUUCAUACUGGGGUAAUUUAUUCAGAUUAUAAAGGGGAAUUCAGUUAGUGAUCAGCUCCACUGUUCCCCGGAGUGC
CAAUCCAGGUGAUAGAAUUGCUCAAUUACUGCUUUUGCCUUAUGUUAAAAUUGGGGAAAACAAAAAGGAAAGAACAGGAGGGUUUGGAA
GUACCAACCCUGCAGGAAAAGCUGCUUAUUGGGCUAAUCAGGUCUCAGAGGAUAGACCCGUGUGUACAGUCACUAUUCAGGGAAAGAGU
UUGAAGGAUUAGUGGAUACCCAGGCUGAUGUUUCUGUCAUCGGCAUAGGUACUGCCUCAGAAGUGUAUCAAAGUGCCAUGAUUUUACAU
UGUCCAGGAUCUGAUAAUCAAGAAAGUACGGUUCAGCCUGUGAUCACUUCAUUCCAAUCAAUUUAUGGGGCCGAGACUUGUUACAACAA
UGGCAUGCAGAGAUUACUAUCCCAGCCUCCCUAUACAGCCCCAGGAAUAAAAAAAUCAUGACUAAAAUGGGAUAGCUCCCUAAAAAGGG
ACUAGGAAAGAAGUCCCAAUUGAGGCUGAAAAAAAUCAAAAAAGAAAAGGAAUAGGGCAUCCUUUUUAGGAGCGGUCACUGUAGAGCCU
CCAAAACCCAUUCCAUUAACUUGGGGGAAAAAAAAACAACUGUAUGGUAAAUCAGCAGCGCUUCCAAAACAAAAACUGGAGGCUUUACA
UUUAUUAGCAAAGAAACAAUUAGAAAAAGGACAUUGAGCCUUCAUUUUCGCCUUGGAAUUCUGUUUGUAAUUCAGAAAAAAUCCGGCAG

AUGGCGUAUAAUGCCGUAAUUCAACCCAUGGGGGCUCUCCCACCCCGGUUGCCCUCUCCAGCCAUGGUCCCCUUUAAUUAUAAUUGAUC
UGAAGGAUUGCUUUUUUACCAUUCCUCUGGCAAAACAGGAUUUUGAAAAUUUGCUUUUACCACACCAGCCUAAAUAAUAAAGAACCAG
CCACCAGGUUUCAGUGGAAAGUAUUGCCUCAGGGAAUGCUUAAUAGUUCAACUAUUUGUCAGCUCAAGCUCUGCAACCAGUUAGAGACA
AGUUUUCAGACUGUUACAUCGUUCACUAUGUUGAUAUUUUGUGUGCUGCAGAAACGAGAGACAAAUUAAUUGACCGUUACACAUUUCUG
CAGACAGAGGUUGCCAACGCGGGACUGACAAUAACAUCUGAUAAGAUUCAAACCUCUACUCCUUUCCGUUACUUGGGAAUGCAGGUAGA
GGAAAGGAAAAUUAAACCACAAAAAAUAGAAAUAAGAAAAGACACAUUAAAAGCAUUAAAUGAGUUUCAAAAGUUGCUAGGAGAUACUA
AUUGGAUUUGGAGAUAUUAAUUGGAUUUGGCCAACUCUAGGCAUUCCUACUUAUGCCAUGUCAAAUUUGUUCUCUUUCUUAAGAGGGGA
CUCGGAAUUAAAUAGUGAAAGAACGUUAACUCCAGAGGCAACUAAAGAAAUUAAAUUAAUUGAAGAAAAAAUUCGGUCAGCACAAGUAA
AUAGAAUAGAUCACUUGGCCCCACUCCAAAUUUUGAUUUUUGCUACUGCACAUUCCCUAACAGGCAUCAUUGUUCAAAAUACAGAUCUU
GUGGAGUGGUCCUUCCUUCCUCACAGUACAAUUAAGACUUUUACAUUGUACUUGGAUCAAAUGGCUACAUUAAUUGGUCAGGGAAGAUU
AUGAAUAAUAACAUUGUGUGGAAAUGACCCAGAUAAAAUCACUGUUCCUUUCAACAAGCAACAGGUUAGACAAGCCUUUAUCAAUUCUG
GUGCAUGGCAGAUUGGUCUUGCCGAUUUUGUGGGAAUUAUUGACAAUCGUUACCCCAAAACAAAAAUCUUCCAGUUUUUAAAAUUGACU
ACUUGGAUUUUACCUAAAGUUACCAAACAUAAGCCUUUAAAAAAUGCUCUGGCAGUGUUUACUGAUGGUUCCAGCAAUGGAAAAGUGGC
UUACACCGGGCCAAAAGAAUGAGUCAUCAAAACUCAGUAUCACUUGACUCAAAGAGCAGAGUUGGUUGCCGUCAUUACAGUGUUAACAA
GAUUUUAAUCAGUCUAUUAACAUUGUAUCAGAUUCUGCAUAUGUAGUACAGGCUACAAAGGAUAUUGAGAGAGCCCUAAUCAAAUACAU
UAUGGAUGAUCAGUUAAACCCGCUGUUUAAUUUGUUACAACAAAAUGUAAGAAAAAGAAAUUUCCCAUUUUAUAUUACUCAUAUUCGAG
CACACACUAAUUUACCAGGGCCUUUAACUAAAGCAAAUGAACAAGCUGACUUGCUAGUAUCAUCUGCAUUCAUGGAAGCACAAGAACUU
CAUGCCUUGACUCAUGUAAAUGCAAUAGGAUUAAAAAAUAAAUUUGAUAUCACAUGGAAACAGACAAAAAAUAUUGUACAACAUUGCAC
CCAGUGUCAGAUUCUACACCUGGCCACUCAGGAGGCAAGAGUUAAUCCCAGAGGUCUAUGUCCUAAUGUGUUAUGGCAAAUGGAUGUCA
UGCACGUACCUUCAUUUGGAAAAUUGUCAUUUGUCCAUGUGACAGUUGAUACUUAUUCACAUUUCAUAUGGGCAACCUGCCAGACAGGA
GAAAGUACUUCCCAUGUUAAAAGACAUUUAUUAUCUUGUUUUCCUGUCAUGGGAGUUCCAGAAAAAGUUAAAACAGACAAUGGGCCAGG
UUACUGUAGUAAAGCAGUUCAAAAAUUCUUAAAUCAGUGGAAAAUUACACAUACAAUAGGAAUUCUCUAUAAUUCCCAAGGACAGGCCA
UAAUUGAAAGAACUAAUAGAACACUCAAAGCUCAAUUGGUUAAACAAAAAAAAGGAAAAGACAGGAGUAUAACACUCCCCAGAUGCAAC
UUAAUCUAGCACUCUAUACUUUAAAUGUUUUAAACAUUUAUAGAAAUCAGACCACUACCUCUGCAGAACAACAUCUUACUGGUAAAAGG
AACAGCCCACAUGAAGGAAAACUGAUUUGGUGGAAAGAUAAUAAAAAUAAAACAUGGGAAAUGGGGAAGGUGAUAACGUGGGGGAGAGG
UUUGCUUGUGUUUCACCAGGAGAAAAUCAGCUUCCUGUUUGGAUACCCACUAGACAUUUAAAGUUCUACAAUGAACUCACUGGAGAUG
CAAAGAAAAGUGUGGAGAUGGAGACACCCCAAUCGACUCGCCAGGUAAACAAAAUGGUGAUAUCAGAAGAACAGAAAAAGUUGCCUUCC
AUCAAGGAAGCAGAGUUGCCAAUAUAGGCACAAUUAAAGAAGCUGACACAGUUAGCUAAAAAAAAAAGCCUAGAGAAUACAAAGGUGAC
ACCAACUCCAGAGAAUAUGCUGCUUGCAGCUCUGAUGAUUGUAUCAACGGUGGUAAGUCUUCCCAAGUCUGCAGGAGCAGCUGCAGCUA
AUUAUACUUACUGGGCCUAUGUGCCUUUCCCACCCUUAAUUCGGGCAGUUACAUAGAUGGAUAAUCCUAUUGAAGUAGAUGUUAAUAAU
AGUGCAUGGGUGCCUGGCCCCACAGAUGACUGUUGCCCUGCCCAACCUGAAGAAGGAAUGAUGAUGAAUAUUUCCAUUGGGUAUCCUUA
UCCUCCUGUUUGCCUAGGGAAGGCACCAGGAUGCUUAAUGCCUACAACCCAAAAUUGGUUGGUAGAAGUACCUACAGUCAGUGCUACCA
GUAGAUUUACUUAUCACAUGGUAAGUGGAAUGUCACAGAUAAAUAAUUUACAGGACCCUUCUUAUCAAAGAUCAUUACAAUGUAGGCCU
AAGGGGAAGGCUUGCCCCAAGGAAAUUCCCAAAGAAUCAAAAAGCCCAGAAGUCUUAGUCUGCGGAGAAUGUGUGGCUGAUACUGCAGU
GUAGUACAAAACAAUGAAUUUUGAACUAUGAUAGACUGGGUCCCUUGAGGCCAAUUAUAUCAUAACUGUACAGGCCAGACUCAUUCAUG
UUCACAGGCCCCAUCCAUCUGGCCCAUUAAUCCAGCCUAUGACGGUGAUGUAACUGAAAGGCUGGACCAGGUUUAUAGAAGGUUAGAAU
CACUCUGUCCAAGGAAAUGGGGUGAAAAGGGAAUUUCAUCACCUUGACCAAAGUUAGUCCUGUUACUGGUCCUGAACAUCCAGAAUUAG
GAAGCUUACUGUGGCCUCACACCACAUUAGAAUUUGUUCUGGAAAUCAAGCUAUAGGAACAAGAGAUCGUAAGUCAUAUUAUACUAUCA
ACCUAAAUUCCAGUCUGACAAUUCCUUUGCAAAAUUGUGUAAAACUCCCUUAUAUUGCUAGUUGUAGGAAAAACAUAGUUAUUAAACCU
GAUUCCCAAACCAUAAUCUGUGAAAAUUGUGGAAUGUUUACUUGCAUUGAUUUGACUUUUAAUUGGCAGCACCGUAUUCUACUAGGAAG
AGCAAGAGAGGGUGUGUGGAUCCUUGUGUCCAUGGACCGACCAUGGGAGGCUUCGCUAUCCAUCCAUAUUUAACGGAAGUAUUAAAAG
GAAUUCUAACUAGAUCCAAAAGAUUCAUUUUUACUUUGAUGGCAGUGAUUAUGGGCCUCAUUGCAGUCACAGCUACUGCUGCGGCUGCU
GGAAUUGCUUUACACUCCUCUGUUCAAACUGCAGAAUACGUAAAUGAUUGGCAAAAGAAUUCCUCAAAAUUGUGGAAUUCUCAGAUCCA
AAUAGAUCAAAAAUUGGCAAACCAAAUUAAUGAUCUUAGACAAACUGUCAUUUGGAUGGGAGAGGCUCAUGAGCUUGGAAUAUCUUUUU
CAGUUACGAUGUGACUGGAAUACAUCAGAUUUUUGUGUUACACCACAAGCCUAUAAUGAGUCUGAGCAUCACUGGGACAUGGUUAGAUG
CCAUCUGCAAGGAGGAGAAGAUAAUCUUACUUUAGACAUUUCAAAAUUAAAAGAAUUUUUUUUUCUUUGAGACAGAGUCUCGCUCUGU
CGCCCAGGCUGGAGUGCAGUGGCGUGAUCUCAGCUCACUGCAAGUUCCGCCUCCUGGGUUUACACCAUUCUCCUGCCUCAGCCUCCCAA
GUAGUUGGGACUACAGGAGCCCACCACCAUGCCUGGCUAAUUUUUUUUGGGUUUUUAAUAGAGAUGGAGUUUCACCGUGUUAGCCAGGA
UGGUCUCGAUCUCCUGACCUUGUGAUCUGCCCACCUUGGCCUCCCAAAGUGCUGGGAUUACAGUCGUGAGCCACCGUGCCCAGCCAAGA
AAAAAUUUUUGAGGCAUCAAAAGCCCAUUUAAAUUUGGUGCCAGGAACGGAGACAAUCGUGAAAGCUGCUGAUAGCCUCACAAAUCUUA
AGCCAGUCACUUGGGUUAAAAGCAUCAGAAGUUUCACUAUUGUAAAUUUCAUAUUAAUCCUUGUAUGCCUGUUCUGUCUGUUGUUAGUC
UACAGGUGUAUCCAGCAGCUCCAAAGAGACAGCAACCAGCAAGAAUGGGCCAUAGUGACGAUGGUGGUUUUGUCAAAAAGAAAAGGGGG
GGAUAUGUAAGGAAAAGAGAGAUCAGACUUUCACUGUGUCUAUGUAGAAAAGGAAGACAUAAGAAACUCCAUUUUGAUCUGUACUAAGA

AAAAUUGUUUUGCCUUGAGAUGCUGUUAAUCUGUAACUUUAGCCCCAACCCUGUGCUCACGGAAACAUGUGCUGUAAGGUUUAAGGAU
CUAGGGCUGUGCAGGAUGUACCUUGUUAACAAUAUGUUUGCAGGCAGUAUGUUUGGUAAAAGUCAUCGCCAUUUCCCAUUCUCGAUUAA
CCAGGGGCUCAAUGCACUGUGGAAAGCCACAGGAACCUCUGCCCAAGAAAGCCUGGCUGUUGUGGGAAGUCAGGGACCCCGAAUGGAGG
GACCAGCUGGUGCUGCAUCAGGAAACAUAAAUUGUGAAGAUUUCUUGGACAUUUAUCAGUUUCCAAAAUUAAUACUUUUAUAAUUUCUU
ACACCUGUCUUACUUUAAUCUCUUAAUCCUGUUAUCUUUGUAAGCUGAGGAUAUACGUCACCUCAGGACCACUAUUGUACAAAUUGAUU
GUAAAACAUGUUCACAUGUGUUUGAACAAUAUGAAAUCAGUGCACCUUGAAAAUGAACAGAAUAACAGUGAUUUUAGGGAACAAAGGAA
GACAACCAUAAGGUCUGACUGCCUGAGGGGUCGGGCAAAAAGCCAUAUUUUUCUUCUUGCAGAGAGCCUAUAAAUGGACGUGCAAGUAG
GAGAGAUAUUGCUAAAUUCUUUUCCUAGCAAGGAAUAUAAUACUAAGACCCUAGGGAAAGAAUUGCAUUCCUGGGGGAGGUCUAUAAA
CGGCCGCUCUGGGAGUGUCUGUCCUAUGUGGUUGAGAUAAGGACUGAGAUACGCCCUGGUCUCCUGCAGUACCCUCAGGCUUACUAGGA
UUGGGAAACCCCAGUCCUGGUAAAUUUGAGGUCAGGCCGGUUCUUUGCUCUGAACCCUGUUUUCUGUUAAGAUGUUUAUCAAGACAAUA
CAUGCACCGCUGAACAUAGACCCUUAUCAGGAGUUUCUGAUUUUGCUCUGGUCCUGUUUCUUCAGAAGCAUGUCAUCUUUGCUCUGCCU
UCUGCCCUUUGAAGCAUGUGAUCUUUGUGACCUACUCCCUGUUCAUACACCCCUCCCCUUUUAAAAUCCCUAAUAAAAACUUGCUGGUU
UUGUGGCUCAGGGGGGCAUCAUGGACCUACCAAUACGUGAUGUCACCCCCGGUGGCCCAGCUGU
SEQ ID 1198
ACAGCACUUUUUUCUUUACCUUGUUUAUGAUGCAGAGACAUUUGUUCACAUGUUUUCCUGCUGGCCCUCUCCCCACUAUUACCCUAUUG
UCCUGCCACAUCCCCCUCUCCGAGAUGGUAGAGAUAAUGAUCAAUAAAUACUGAGGGAACUCAGAGACCGGUGCGGCGCGGUCCUCCA
UAUGCUGAGCGCCGGUCCCCUGGGCCCACUUUUCUUUCUCUAUACUUUGUCUCUGUUGUCUUUCUUUUCUCAAGUCUCUCGUUCCACCU
GAGGAGAAAUGCCCACAGCUGUGGAGGCGCAGGCCACUCCAUCUGGUGCCCAACGUGGAUGCUUUUCUCUAGGGUGAAGGGACUCUCGA
GUGUGGUCAUUGAGGACAAGUCAACGAGAGAUUCCCGAGUACGUCUACAGUGAGCCUUGUGGUAAGCUUGGGCGCUCGGAAGAAGCCAG
GGUUAAUGGGGCAAACUAAAAGUAAAGUCUCUCAUUCCACCUGAUGAGAAACACCCAGAGGUGUGGAGGGGCAGGCCACCCCUUCAGGG
UAGGGUCCCCUCCAUGCAGACCAUAGAGCACAGGUGUGCCCCAAAGAGGAGCAGAGAGAAGGAGGGAGAGGGCCCACGAGAGACUUGGA
AAUGAAUGGCAGGAUUUUAGGCGCUGGACUUGGGUUCGGGGCACCUGGCCUUUCCUUGUGUAUUUCUCCCUACUGUCUGCCUAACUAUUU
AAUACAAUAAAAGAAAACCAGCCCCUGGUUCUUGUGGUGUUUCCACCCUCCCGGGUCCCCGCUGGCUGCCUGGCUUCCUCCCGCAGCUC
CUGCUGUGUGUGUAUGUGUGUGUGUGUGCACAUCUGUGGGGCGUAUGUGUGUUCGUCUUUGUAAUUGAGGCUGCAGAGUGGAGAGAGCA
GGGGUUUUCUCUGGGGACCCAGAGAGAAGGAGGCGUUUUCACCACAGCCGAACAGGGCAGGACCCCAGCACCCGGGACCCAGCGGGACU
UUGCCAAGGGGAUGGACCUGGCUGGGCCACGCGGCUGUUUGUGUAGGGAAAAGAAAGAGAGAUCACACUGUUACUGUGUCUAUGUAGAA
AAGGAAGACAUAAACUCCAUUUUGAGCUGUACUAAGAAAAAUUAUUUUGCCUUGACCUGCUGUUAACCUGUAACUGUAGCCCCAACCCU
GUGCUCAAAGAAACAUGUGCUGUAUGGAAUCAAGGUUUAAGGGAUCAAGGGCUGUACAGGAUGUGCCUUGUUAACAAUGUGUUUACAGG
CAGUAUGCUUGGUAAAAGUCAUCGCCAUUCUCCAUUCUCCAUUAAUCAGGGGCACGAUGCACUGCGGAAAGCCACAGGGACCUCUGCCC
GAGAAAGCCUGGGUAUUGUCCAAGGCUUCCCCCCACUGAGACAGCCUGAGAUACGGCCUCGUGGGAAGGGAAAGACCUGACCGUCCCCC
AGCCCGACACCCGUAAAGGGUCUGUGCUGAGGAGGAUUAGUAAAAGGGGAAGGCCUCUUGCAGUUGAGAUAAGAGGAAGGCCUCCGUCU
CCUGCAUGUCCUUGGGAAUGGAAUGUCUUGGUGUAAAACCCGAUAGUACAUUCCUUCUAUUCUGAGAGAAGAAAACCACCCUGUGGCUG
GAGGUGAGAUAUGCUAGCGGCAAUGCUGCUCUGUUACUCUUUGCUACACUGAGAUGUUUGGGUGGAGAGAAGCAUAAAUCUGGCCUAUG
UGCACAUCUGGGCACAGAACCUCCCCUUGAACUUGUGACACAGAUUCCUUUGUUCACAUGUUUUCCUGCUGACCUUCUCCCCACUAUCG
CCCUGUUCUCCCACCGCAUUCCCCUUGCUGAGAUAGUGAAAAUAGUAAUCUGUAGAUACCAAGGGAACUCAGAGACCAUGGCCGGUGCA
CAUCCUCCGUACGCUGAGCGCUGGUCCCCUGGGCCCAUUGUUCUUUCUCUAUACUUUGUCUCUGUGUCUUAUUUCUUUCCUCAGUCUCU
CAUCCCUCCUGACGAGAAAUACCCACAGGUGUGGAGGGGCUGGCCCCCUUCAUCUGAUGCCCAAUGUGGGUGCCUUUCUCUAGGGUGAA
GGUACUCUACAGUGUGGUCAUUGAGGACAAGUUGACGAGAGAGUCCCAAGUACGUCCACGGUCAGCCUUGCGGUAAGCUUGUGUGCUUA
GAGGAACCCAGGGUAACGAUGGGGCAAACUGAAAGUAAAUAUGCCUCUUAUCUCAGCUUUAUUAAAAUUCUUUUAAGAAGAGGGGGAGU
UAGAGCUUCUACAGAAAAUCUAAUUACGCUAUUUCAAACAAUAGAACAAUUCUGCCCAUGGUUUCCAGAACAGGGAACUUUAGAUCUAA
AAGAUUGGGAAAAAAUUGGCAAAGAAUUAAAACAAGCAAAUAGGGAAGGUAAAAUCAUCCCACUUACAGUAUGGAAUGAUUGGGCCAUU
AUUAAAGCAACUUUAGAACCAUUUCAAACAGGAGAAGAUAUUGUUUCAGUUUCUGAUGCCCCUAAAAGCUGUGUAACAGAUUGUGAAGA
AGAGGCAGGGACAGAAUCCCAGCAAGGAACGGAAAGUUCACAUUGUAAAUAUGUAGCAGAGUCUGUAAUGGCUCAGUCAACGCAAAAUG
UUGACUACAGUCAAUUACAGGAGAUAAUAUACCCUGAAUCAUCAAAAUUGGGGAAGGAGGUCCAGAAUCAUUGGGGCCAUCAGAGCCU
AAACCACGAUCGCCAUCAACUCCUCCUCCCGUGGUUCAGAUGCCUGUAACAUUACAACCUCAAACGCAGGUUAGACAAGCACAAACCCC
AAGAGAAAAUCAAGUAGAAAGGGACAGAGUCUCUAUCCCGGCAAUGCCAACUCAGAUACAGUAUCCACAAUAUCAGCCGGUAGAAAAUA
AGACCCAACCGCUGGUAGUUUAUCAAUACCGGCUGCCAACCGAGCUUCAGUAUCGGCCUCCUUCAGAGGUUCAAUACAGACCUCAAGCG
GUGUGUCCUGUGCCAAAUAGCACGGCACCAUACCAGCAACCCACAGCGAUGGCGUCUAAUUCACCAGCAACACAGGACGCGGCGCUGUA
UCCUCAGCCGCCCACUGUGAGACUUAAUCCUACAGCAUCACGUAGUGGACAGGGUGGUGCACUGCAUGCAGUCAUUGAUGAAGCCAGAA
AACAGGGCGAUCUUGAGGCAUGGCGGUUCCUGGUAAUUUUACAACUGGUACAGGCCGGGGAAGAGACUCAAGUAGGAGCGCCUGCCCGA
GCUGAGACUAGAUGUGAACCUUUCACCAUGAAAAUGUUAAAAGAUAUAAAGGAAGGAGUUAAACAAUAUGGAUCCAACUCCCCUUAUAU
AAGAACAUUAUUAGAUUCCAUUGCUCAUGGAAAUAGACUUACUCCUUAUGACUGGGAAAUUUUGGCCAAAUCUUCCCUUUCAUCCUCUC
AGUAUCUACAGUUUAAAACCUGGUGGAUUGAUGGAGUACAAGAACAGGUACGAAAAAAUCAGGCUACUAAGCCCACUGUUAAUAUAGAC

GCAGACCAAUUGUUAGGAACAGGUCCAAAUUGGAGCACCAUUAACCAACAAUCAGUGAUGCAGAAUGAGGCUAUUGAACAAGUAAGGGC
UAUUUGCCUCAGGGCCUGGGGAAAAAUUCAGGACCCAGGAACAGCUUUCCCUAUUAAUUCAAUUAGACAAGGCUCUAAAGAGCCAUAUC
CUGACUUUGUGGCAAGAUUACAAGAUGCUGCUCAAAAGUCUAUUACAGAUGACAAUGCCCGAAAAGUUAUUGUAGAAUUAAUGGCCUAU
GAAAAUGCAAAUCCAGAAUGUCAGUCGGCCAUAAAGCCAUUAAAAGGAAAAGUUCCAGCAGGAGUUGAUGUAAUUACAGAAUAUGUGAA
GGCUUGUGAUGGGAUUGGAGGAGCUAUGCAUAAGGCAAUGCUAAUGGCUCAAGCAAUGAGGGGGCUCACUCUAGGAGGACAAGUUAGAA
CAUUUGGGAAAAAAUGUUAUAAUUGUGGUCAAAUCGGUCAUCUGAAAAGGAGUUGCCCAGGCUUAAAUAAACAGAAUAUAAUAAAUCAA
GCUAUUACAGCAAAAAAUAAAAAGCCAUCUGGCCUGUGUCCAAAAUGUGGAAAAGCAAAACAUUGGGCCAAUCAAUGUCAUUCUAAAUU
UGAUAAAGAUGGGCAACCAUUGUCUGGAAACAGGAAGAGGGGCCAGCCUCAGGCCCCCCAACAAACUGGGGCAUUCCCAGUUAAACUGU
UUGUUCCUCAGGGUUUUCAAGGACAACAACCCCUACAGAAAAUACCACCACUUCAGGGAGUCAGCCAAUUACAACAAUCCAACAGCUGU
CCCGCGCCACAGCAGGCAGCACCGCAGUAGAUUUAUGUUCCACCCAAAUGGUCUUUUUACUCCCUGGAAAGCCCCCACAAAAGAUUCCU
AGAGGGGUAUAUGGCCCGCUGCCAGAAGGGAGGGUAGGCCUUUGAGGGAGAUCAAGUCUAAAUUUGAAGGGAGUCCAAAUUCAUACUGG
GGUAAUUUAUUCAGAUUAUAAAGGGGGAAUUCAGUUAGUGAUCAGCUCCACUGUUCCCCGGAGUGCCAAUCCAGGUGAUAGAAUUGCUC
AAUUACUGCUUUUGCCCUUAUGUUAAAAUUGGGGAAAACAAAAAGGAAAGAACAGGAGGGUUUGGAAGUACCAACCCUGCAGGAAAAGCU
GCUUAUUGGGCUAAUCAGGUCUCAGAGGAUAGACCCGUGUGUACAGUCACUAUUCAGGGAAAGAGUUUGAAGGAUUAGUGGAUACCCAG
GCUGAUGUUUCUGUCAUCGGCAUAGGUACUGCCUCAGAAGUGUAUCAAAGUGCCAUGAUUUUACAUUGUCCAGGAUCUGAUAAUCAAGA
AAGUACGGUUCAGCCUGUGAUCACUUCAUUCCAAUCAAUUUAUGGGGCCGAGACUUGUUACAACAAUGGCAUGCAGAGAUUACUAUCCC
AGCCUCCCUAUACAGCCCCAGGAAUAAAAAAAUCAUGACUAAAAUGGGAUAGCUCCCUAAAAAGGGACUAGGAAAGAAGUCCCAAUUGA
GGCUGAAAAAAAUCAAAAAAGAAAAGGAAUAGGGCAUCCUUUUUAGGAGCGGUCACUGUAGAGCCUCCAAAACCCAUUCCAUUAACUUG
GGGGAAAAAAAAAACAACUGUAUGGUAAAUCAGCAGCGCUUCCAAAACAAAAACUGGAGGCUUUACAUUUAUUAGCAAAGAAACAAUUAG
AAAAAGGACAUUGAGCCUUCAUUUUCGCCUUGGAAUUCUGUUUGUAAUUCAGAAAAAAUCCGGCAGAUGGCGUAUAAUGCCGUAAUUCA
ACCCAUGGGGGCUCUCCCACCCCGGUUGCCCUCUCCAGCCAUGGUCCCCUUUAAUUAUAAUUGAUCUGAAGGAUUGCUUUUUUACCAUU
CCUCUGGCAAAACAGGAUUUGAAAAAUUUGCUUUUACCACACCAGCCUAAAUAAUAAAGAACCAGCCACCAGGUUUCAGUGGAAAGUA
UUGCCUCAGGGAAUGCUUAAUAGUUCAACUAUUUGUCAGCUCAAGCUCUGCAACCAGUUAGAGACAAGUUUUCAGACUGUUACAUCGUU
CACUAUGUUGAUAUUUUGUGUGCUGCAGAAACGAGAGACAAAUUAAUUGACCGUUACACAUUUCUGCAGACAGAGGUUGCCAACGCGGG
ACUGACAAUAACAUCUGAUAAGAUUCAAACCCUCUACUCCUUUCCGUUACUUGGGAAUGCAGGUAGAGGAAAGGAAAAUUAAACCACAAA
AAAUAGAAAUAAGAAAAGACACAUUAAAAGCAUUAAAUGAGUUUCAAAAGUUGCUAGGAGAUACUAAUUGGAUUUGGAGAUAUUAAUUG
GAUUUGGCCAACUCUAGGCAUUCCUACUUAUGCCAUGUCAAAUUUGUUCUCUUUCUUAAGAGGGGACUCGGAAUUAAAUAGUGAAAGAA
CGUUAACUCCAGAGGCAACUAAAGAAAUUAAAUUAAUUGAAGAAAAAAUUCGGUCAGCACAAGUAAAUAGAAUAGAUCACUUGGCCCCA
CUCCAAAUUUUGAUUUUUGCUACUGCACAUUCCCUAACAGGCAUCAUUGUUCAAAAUACAGAUCUUGUGGAGUGGUCCUUCCUUCCUCA
CAGUACAAUUAAGACUUUUACAUUGUACUUGGAUCAAAUGGCUACAUUAAUUGGUCAGGGAAGAUUAUGAAUAAUAACAUUGUGUGGAA
AUGACCCAGAUAAAAUCACUGUUCCUUUCAACAAGCAACAGGUUAGACAAGCCUUUAUCAAUUCUGGUGCAUGGCAGAUUGGUCUUGCC
GAUUUUGUGGGAAUUAUUGACAAUCGUUACCCCAAAACAAAAAUCUUCCAGUUUUUAAAAUUGACUACUUGGAUUUUACCUAAAGUUAC
CAAACAUAAGCCUUUAAAAAAUGCUCUGGCAGUGUUUACUGAUGGUUCCAGCAAUGGAAAAGUGGCUUACACCGGGCCAAAAGAAUGAG
UCAUCAAAACUCAGUAUCACUUGACUCAAAGAGCAGAGUUGGUUGCCGUCAUUACAGUGUUAACAAGAUUUUAAUCAGUCUAUUAACAU
UGUAUCAGAUUCUGCAUAUGUAGUACAGGCUACAAAGGAUAUUGAGAGAGCCCUAAUCAAAUACAUUAUGGAUGAUCAGUUAAACCCGC
UGUUUAAUUUGUUACAACAAAAUGUAAGAAAAAGAAAUUUCCCAUUUUAUAUUACUCAUAUUCGAGCACACACUAAUUUACCAGGGCCU
UUAACUAAAGCAAAUGAACAAGCUGACUUGCUAGUAUCAUCUGCAUUCAUGGAAGCACAAGAACUUCAUGCCUUGACUCAUGUAAAUGC
AAUAGGAUUAAAAAAUAAAUUUGAUAUCACAUGGAAACAGACAAAAAAUAUUGUACAACAUUGCACCCAGUGUCAGAUUCUACACCUGG
CCACUCAGGAGGCAAGAGUUAAUCCCAGAGGUCUAUGUCCUAAUGUGUUAUGGCAAAUGGAUGUCAUGCACGUACCUUCAUUUGGAAAA
UUGUCAUUUGUCCAUGUGACAGUUGAUACUUAUUCACAUUUCAUAUGGGCAACCUGCCAGACAGGAGAAAGUACUUCCCAUGUUAAAAG
ACAUUUAUUAUCUUGUUUUCCUGUCAUGGGAGUUCCAGAAAAAGUUAAAACAGACAAUGGGCCAGGUUACUGUAGUAAAGCAGUUCAAA
AAUUCUUAAAUCAGUGGAAAAUUACACAUACAAUAGGAAUUCUCUAUAAUUCCCAAGGACAGGCCAUAAUUGAAAGAACUAAUAGAACA
CUCAAAGCUCAAUUGGUUAAACAAAAAAAAGGAAAAGACAGGAGUAUAACACUCCCCAGAUGCAACUUAAUCUAGCACUCUAUACUUUA
AAUGUUUUAAACAUUUAUAGAAAUCAGACCACUACCUCUGCAGAACAACAUCUUACUGGUAAAAGGAACAGCCCACAUGAAGGAAAACU
GAUUUGGUGGAAAGAUAAUAAAAAUAAAACAUGGGAAAUGGGGAAGGUGAUAACGUGGGGGAGAGGUUUUGCUUGUGUUUCACCAGGAG
AAAAUCAGCUUCCUGUUUGGAUACCCACUAGACAUUUAAAGUUCUACAAUGAACUCACUGGAGAUGCAAAGAAAAGUGUGGAGAUGGAG
ACACCCCAAUCGACUCGCCAGGUAAACAAAAUGGUGAUAUCAGAAGAACAGAAAAAGUUGCCUUCCAUCAAGGAAGCAGAGUUGCCAAU
AUAGGCACAAUUAAAGAAGCUGACACAGUUAGCUAAAAAAAAAAGCCUAGAGAAUACAAAGGUGACACCAACUCCAGAGAAUAUGCUGC
UUGCAGCUCUGAUGAUUGUAUCAACGGUGGUAAGUCUUCCCAAGUCUGCAGGAGCAGCUGCAGCUAAUUAUACUUACUGGGCCUAUGUG
CCUUUCCCACCCUUAAUUCGGGCAGUUACAUAGAUGGAUAAUCCUAUUGAAGUAGAUGUUAAUAAUAGUGCAUGGGUGCCUGGCCCCAC
AGAUGACUGUUGCCCUGCCCAACCUGAAGAAGGAAUGAUGAUGAAUAUUUCCAUUGGGUAUCCUUAUCCUCCUGUUUGCCUAGGGAAGG
CACCAGGAUGCUUAAUGCCUACAACCCAAAAUUGGUUGGUAGAAGUACCUACAGUCAGUGCUACCAGUAGAUUUACUUAUCACAUGGUA
AGUGGAAUGUCACAGAUAAAUAAUUUACAGGACCCUUCUUAUCAAAGAUCAUUACAAUGUAGGCCUAAGGGGAAGGCUUGCCCCAAGGA

AAUUCCCAAAGAAUCAAAAAGCCCAGAAGUCUUAGUCUGCGGAGAAUGUGUGGCUGAUACUGCAGUGUAGUACAAAACAAUGAAUUUG
AACUAUGAUAGACUGGGUCCCUUGAGGCCAAUUAUAUCAUAACUGUACAGGCCAGACUCAUUCAUGUUCACAGGCCCCAUCCAUCUGGC
CCAUUAAUCCAGCCUAUGACGGUGAUGUAACUGAAAGGCUGGACCAGGUUUAUAGAAGGUUAGAAUCACUCUGUCCAAGGAAAUGGGGU
GAAAAGGGAAUUUCAUCACCUUGACCAAAGUUAGUCCUGUUACUGGUCCUGAACAUCCAGAAUUAGGAAGCUUACUGUGGCCUCACACC
ACAUUAGAAUUUGUUCUGGAAAUCAAGCUAUAGGAACAAGAGAUCGUAAGUCAUAUUAUACUAUCAACCUAAAUUCCAGUCUGACAAUU
CCUUUGCAAAAUUGUGUAAAACUCCCUUAUAUUGCUAGUUGUAGGAAAAACAUAGUUAUUAAACCUGAUUCCCAAACCAUAAUCUGUGA
AAAUUGUGGAAUGUUUACUUGCAUUGAUUUGACUUUUAAUUGGCAGCACCGUAUUCUACUAGGAAGAGCAAGAGAGGGUGUGUGGAUCC
UUGUGUCCAUGGACCGACCAUGGGAGGCUUCGCUAUCCAUCCAUAUUUAACGGAAGUAUUAAAAGGAAUUCUAACUAGAUCCAAAAGA
UUCAUUUUUACUUUGAUGGCAGUGAUUAUGGGCCUCAUUGCAGUCACAGCUACUGCUGCGGCUGCUGGAAUUGCUUUACACUCCCUCUGU
UCAAACUGCAGAAUACGUAAAUGAUUGGCAAAAGAAUUCCUCAAAAUUGUGGAAUUCUCAGAUCCAAAUAGAUCAAAAAUUGGCAAACC
AAAUUAAUGAUCUUAGACAAACUGUCAUUUGGAUGGGAGAGGCUCAUGAGCUUGGAAUAUCUUUUUCAGUUACGAUGUGACUGGAAUAC
AUCAGAUUUUUGUGUUACACCACAAGCCUAUAAUGAGUCUGAGCAUCACUGGGACAUGGUUAGAUGCCAUCUGCAAGGAGGAGAAGAUA
AUCUUACUUUAGACAUUUCAAAAUUAAAAGAAUUUUUUUUUCUUUGAGACAGAGUCUCGCUCUGUCGCCCAGGCUGGAGUGCAGUGGC
GUGAUCUCAGCUCACUGCAAGUUCCGCCUCCUGGGUUUACACCAUUCUCCUGCCUCAGCCUCCCAAGUAGUUGGGACUACAGGAGCCCA
CCACCAUGCCUGGCUAAUUUUUUUUGGGUUUUUAAUAGAGAUGGAGUUUCACCGUGUUAGCCAGGAUGGUCUCGAUCUCCUGACCUUGU
GAUCUGCCCACCUUGGCCUCCCAAAGUGCUGGGAUUACAGUCGUGAGCCACCGUGCCCAGCCAAGAAAAAAUUUUUGAGGCAUCAAAAG
CCCAUUUAAAUUUGGUGCCAGGAACGGAGACAAUCGUGAAAGCUGCUGAUAGCCUCACAAAUCUUAAGCCAGUCACUUGGGUUAAAAGC
AUCAGAAGUUUCACUAUUGUAAAUUUCAUAUUAAUCCUUGUAUGCCUGUUCUGUCUGUUGUUAGUCUACAGGUGUAUCCAGCAGCUCCA
AAGAGACAGCAACCAGCAAGAAUGGGCCAUAGUGACGAUGGUGGUUUUGUCAAAAAGAAAAGGGGGGAUAUGUAAGGAAAAGAGAGAU
CAGACUUUCACUGUGUCUAUGUAGAAAAGGAAGACAUAAGAAACUCCAUUUUGAUCUGUACUAAGAAAAAUUGUUUUGCCUUGAGAUGC
UGUUAAUCUGUAACUUUAGCCCCAACCCUGUGCUCACGGAAACAUGUGCUGUAAGGUUUAAGGGAUCUAGGGCUGUGCAGGAUGUACCU
UGUUAACAAUAUGUUUGCAGGCAGUAUGUUUGGUAAAAGUCAUCGCCAUUCUCCAUUCUCGAUUAACCAGGGGCUCAAUGCACUGUGGA
AAGCCACAGGAACCUCUGCCCAAGAAAGCCUGGCUGUUGUGGGAAGUCAGGGACCCCGAAUGGAGGGACCAGCUGGUGCUGCAUCAGGA
AACAUAAAUUGUGAAGAUUUCUUGGACAUUUAUCAGUUUCCAAAAUUAAUACUUUUAUAAUUUCUUACACCUGUCUUACUUUAAUCUCU
UAAUCCUGUUAUCUUUGUAAGCUGAGGAUAUACGUCACCUCAGGACCACUAUUGUACAAAUUGAUUGUAAAACAUGUUCACAUGUGUUU
GAACAAUAUGAAAUCAGUGCACCUUGAAAAUGAACAGAAUAACAGUGAUUUUAGGGAACAAAGGAAGACAACCAUAAGGUCUGACUGCC
UGAGGGGUCGGGCAAAAAGCCAUAUUUUUCUUCUUGCAGAGAGCCUAUAAAUGGACGUGCAAGUAGGAGAGAUAUUGCUAAAUUCUUUU
CCUAGCAAGGAAUAUAAUACUAAGACCCUAGGGAAAGAAUUGCAUUCCUGGGGGGAGGUCUAUAAACGGCCGCUCUGGGAGUGUCUGUC
CUAUGUGGUUGAGAUAAGGACUGAGAUACGCCCUGGUCUCCUGCAGUACCCUCAGGCUUACUAGGAUUGGGAAACCCCAGUCCUGGUAA
AUUUGAGGUCAGGCCGGUUCUUUGCUCUGAACCCUGUUUUCUGUUAAGAUGUUUAUCAAGACAAUACAUGCACCGCUGAACAUAGACCC
UUAUCAGGAGUUUCUGAUUUUGCUCUGGUCCUGUUUCUUCAGAAGCAUGUCAUCUUUGCUCUGCCUUCUGCCCUUUGAAGCAUGUGAUC
UUUGUGACCUACUCCCUGUUCAUACACCCCUCCCCUUUUAAAAUCCCUAAUAAAAACUUGCUGGUUUUGUGGCUCAGGGGGGCAUCAUG
GACCUACCAAUACGUGAUGUCACCCCCGGUGGCCCAGCUGU
SEQ ID 1199
UUAUGUAUAUGCACAUCAAAAGCACAGCACUUUUUUCUUUACCUUGUUUAUGAUGCAGAGACAUUUGUUCACAUGUUUUCCUGCUGGCC
CUCUCCCCACUAUUACCCUAUUGUCCUGCCACAUCCCCCUCUCCGAGAUGGUAGAGAUAAUGAUCAAUAAAUACUGAGGGAACUCAGAG
ACCGGUGCGGCGCGGGUCCUCCAUAUGCUGAGCGCCGGUCCCCUGGGCCCACUUUUCUUUCUCUAUACUUUGUCUCUGUUGUCUUUCUU
UUCUCAAGUCUCUCGUUCCACCUGAGGAGAAAUGCCCACAGCUGUGGAGGCGCAGGCCACUCCAUCUGGUGCCCAACGUGGAUGCUUUU
CUCUAGGGUGAAGGGACUCUCGAGUGUGGUCAUUGAGGACAAGUCAACGAGAGAUUCCCGAGUACGUCUACAGUGAGCCUUGUGUCUCU
CAUCC
SEQ ID 1200
UUAUGUAUAUGCACAUCAAAAGCACAGCACUUUUUUCUUUACCUUGUUUAUGAUGCAGAGACAUUUGUUCACAUGUUUUCCUGCUGGCC
CUCUCCCCACUAUUACCCUAUUGUCCUGCCACAUCCCCCUCUCCGAGAUGGUAGAGAUAAUGAUCAAUAAAUACUGAGGGAACUCAGAG
ACCGGUGCGGCGCGGGUCCUCCAUAUGCUGAGCGCCGGUCCCCUGGGCCCACUUUUCUUUCUCUAUACUUUGUCUCUGUUGUCUUUCUU
UUCUCAAGUCUCUCGUUCCACCUGAGGAGAAAUGCCCACAGCUGUGGAGGCGCAGGCCACUCCAUCUGGUGCCCAACGUGGAUGCUUUU
CUCUAGGGUGAAGGGACUCUCGAGUGUGGUCAUUGAGGACAAGUCAACGAGAGAUUCCCGAGUACGUCUACAGUGAGCCUUGUGGGUGA
AGGUA
SEQ ID 1201
UUAUGUAUAUGCACAUCAAAAGCACAGCACUUUUUUCUUUACCUUGUUUAUGAUGCAGAGACAUUUGUUCACAUGUUUUCCUGCUGGCC
CUCUCCCCACUAUUACCCUAUUGUCCUGCCACAUCCCCCUCUCCGAGAUGGUAGAGAUAAUGAUCAAUAAAUACUGAGGGAACUCAGAG
ACCGGUGCGGCGCGGGUCCUCCAUAUGCUGAGCGCCGGUCCCCUGGGCCCACUUUUCUUUCUCUAUACUUUGUCUCUGUUGUCUUUCUU

UUCUCAAGUCUCUCGUUCCACCUGAGGAGAAAUGCCCACAGCUGUGGAGGCGCAGGCCACUCCAUCUGGUGCCCAACGUGGAUGCUUUU
CUCUAGGGUGAAGGGACUCUCGAGUGUGGUCAUUGAGGACAAGUCAACGAGAGAUUCCCGAGUACGUCUACAGUGAGCCUUGUG
SEQ ID 1202
AGAGAAAAGCCTCCACGTTGGGCACC
SEQ ID 1203
GTAGGGGTGGGTTGCCCC
SEQ ID 1204
AAACCGCCTTAGGGCTGGAGGTGGGAC
SEQ ID 1205
TGCGGGCAGCAATACTGC
SEQ ID 1206
TAAAGCACTGAGATGTTTATGTGTATGC
SEQ ID 1207
GCACAGCACTTAATCCTTTACATT
SEQ ID 1208
GTTTGTCTGCTGACCCTCTCCC

## Claims

1. A polypeptide comprising an amino acid sequence encoded by a human endogenous retrovirus (PCAV) located at megabase 20.428 on chromosome 22.

2. The polypeptide according to claim 1, wherein the polypeptide is selected from the group consisting of:
(a) a polypeptide comprising an amino acid sequence selected from the group consisting of SEQ IDs 54, 55, 56, 110, 1186 and 1188.
(b) a polypeptide comprising a fragment of at least 7 amino acids of one or more of SEQ IDs 54, 55, 56, 110, 1186 and 1188;
(c) a polypeptide comprising an amino acid sequence having at least 70% identity to one or more of SEQ IDs 54, 55, 56, 110, 1186 and 1188;
(d) a polypeptide comprising a T-cell or a B-cell epitope of SEQ ID 54, 55, 56, 110, 1186 and 1188.

3. The polypeptide according to claim 1, wherein the polypeptide is selected from the group consisting of:
(a) a polypeptide comprising an amino acid sequence selected from the group consisting of SEQ IDs 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, and 98;
(b) a polypeptide comprising a fragment of at least 7 amino acids of one or more of SEQ IDs 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, and 98 ;
(c) a polypeptide comprising an amino acid sequence having at least 70% identity to one or more of SEQ IDs 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, and 98;
(d) a polypeptide comprising a T-cell or a B-cell epitope of SEQ ID 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, and 98.

4. A polypeptide having formula NH2-XX-YY-ZZ-COOH, wherein:
XX is a polypeptide sequence consisting of xx amino acids;
ZZ is a polypeptide sequence consisting of zz amino acids;
YY is a polypeptide sequence consisting of a fragment of yy amino acids of an amino acid sequence selected from the group consisting of SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188;
said polypeptide NH2-XX-YY-ZZ-COOH is not a fragment of a polypeptide sequence selected from SEQ IDs 54, 55, 56, 59, 60, 61, 62, 64, 65, 66, 67, 68, 69, 70, 71, 72, 73, 74, 75, 76, 77, 78, 79, 80, 82, 83, 84, 85, 87, 92, 93, 94, 95, 96, 97, 98, 110, 1186 and 1188;
xx+zz is at least 1; and
xx+yy+zz is at most 100.

5. A pharmaceutical composition comprising a polypeptide according to any one of claims 1 to 4 and a pharmaceutically acceptable carrier.

6. The pharmaceutical composition of claim 5, wherein the composition is immunogenic.

7. The pharmaceutical composition of claim 6, wherein the composition is a vaccine composition.

8. The pharmaceutical composition of claim 7, further comprising a vaccine adjuvant.

9. A pharmaceutical composition according to any one of claims 5 to 8 for use in a method for raising an immune response in a patient, wherein the method comprises administering an immunogenic dose of the composition.

10. The polypeptide of any one of claims 1 to 4, for use in diagnosis.

11. A nucleic acid comprising a nucleotide sequence corresponding to or complementary to an mRNA transcript transcribed from a human endogenous retrovirus (PCAV) located at megabase 20.428 on chromosome 22 for use in diagnosis.

12. The nucleic acid according to claim 11, wherein the nucleic acid is selected from the group consisting of:
(a) nucleic acid comprising a nucleotide sequence with 90% or more sequence identity to SEQ ID 10, SEQ ID 1197 and/or SEQ ID 1198;
(b) nucleic acid comprising a nucleotide sequence -N1-N2-, where: N1 is a nucleotide sequence from (1) the 5' end of a mRNA transcribed from the first 5' LTR of a human endogenous retrovirus located at megabase 20.428 on chromosome 22, to (2) a first splice donor site downstream of the U5 region of said mRNA transcribed from the first 5' LTR; and N2 is a nucleotide sequence immediately downstream of a splice acceptor site located (1) downstream of said first splice donor site and (2) upstream of a second splice donor site, the second splice donor site being downstream of the second 5' LTR of said endogenous retrovirus;;
(c) nucleic acid comprising a nucleotide sequence with 70% or more sequence identity to SEQ ID 5, SEQ ID 6, SEQ ID 1199 or SEQ ID 1200;
(d) nucleic acid comprising a nucleotide sequence -N3-N4-, where: N₃ is a nucleotide sequence from the 3' end of the 5' fragment of the 3' LTR of a human endogenous retrovirus located at megabase 20.428 on chromosome 22, and N₄ is a nucleotide sequence from 5' end of the MER11a insertion in a human endogenous retrovirus located at megabase 20.428 on chromosome 22;
(e) nucleic acid comprising a nucleotide sequence with 70% or more sequence identity to SEQ ID 9;
(f) nucleic acid comprising a nucleotide sequence -N7-N8-, where: N₇ is a nucleotide sequence preceding the alu insertion within the env gene of a human endogenous retrovirus located at megabase 20.428 on chromosome 22, and N₈ is a nucleotide sequence beginning at the 5' end of said alu insertion;
(g) nucleic acid comprising a nucleotide sequence with 70% or more sequence identity to SEQ ID 38;
(h) nucleic acid comprising a nucleotide sequence -N9-N10-, where: N9 is a nucleotide sequence at the end of the alu insertion within the env gene of a human endogenous retrovirus located at megabase 20.428 on chromosome 22, and N10 is a nucleotide sequence immediately downstream of said alu insertion;
(i) nucleic acid comprising nucleotide sequence SEQ ID 42;
(j) nucleic acid comprising a nucleotide sequence with 70% or more sequence identity to SEQ ID 42;
(k) nucleic acid comprising nucleotide sequence SEQ ID 53;
(l) nucleic acid comprising a nucleotide sequence with 70% or more sequence identity to SEQ ID 53;
(m) nucleic acid comprising nucleotide sequence SEQ ID 111;
(n) nucleic acid comprising a nucleotide sequence with 70% or more sequence identity to SEQ ID 111;
(o) nucleic acid comprising nucleotide sequence SEQ ID 1191; or
(p) nucleic acid comprising one or more of SEQ IDs 120 to 1184.

13. A nucleic acid according to claim 11 or claim 12, wherein the nucleic acid comprises a sequence selected from the group consisting of one or more of SEQ IDs 5, 6, 9, 10, 38, 42, 53, 99, 100, 101, 102, 103, 104, 105, 106, 107, 108, 109, 111, 337-599, 600-1184, 1191, 1197 and 1198, 1199, 1200.

14. A nucleic acid probe, wherein the nucleic acid probe is specific to a human endogenous retrovirus (PCAV) located at megabase 20.428 on chromosome 22.

15. The nucleic acid probe according to claim 14, wherein the nucleic acid probe is selected from the group consisting of:
(a) a probe which can hybridize to sequence -N₁-N₂- (or the complement thereof) within a PCAV nucleic acid target, but which does not hybridize to sequences N₁ or N₂ alone (or to their complements alone), where: N₁ is a nucleotide sequence from (1) the 5' end of a mRNA transcribed from the first 5' LTR of a human endogenous retrovirus located at megabase 20.428 on chromosome 22, to (2) a first splice donor site downstream of the U₅ region of said mRNA transcribed from the first 5' LTR; and N₂ is a nucleotide sequence immediately downstream of a splice acceptor site located (1) downstream of said first splice donor site and (2) upstream of a second splice donor site, the second splice donor site being downstream of the second 5' LTR of said endogenous retrovirus;
(b) a probe which can hybridize to sequence -N₃-N₄- (or the complement thereof) within a PCAV nucleic acid target, but which does not hybridize to sequences N₃ or N₄ alone (or to their complements alone), where: N₃ is a nucleotide sequence from the 3' end of the 5' fragment of the 3' LTR of a human endogenous retrovirus located at megabase 20.428 on chromosome 22, and N₄ is a nucleotide sequence from 5' end of the MER11a insertion in a human endogenous retrovirus located at megabase 20.428 on chromosome 22;
(c) a probe which can hybridize to sequence -N₇-N₈- (or the complement thereof) within a PCAV nucleic acid target, but which does not hybridize to sequences N₇ or N₈ alone (or to their complements alone), where: N₇ is a nucleotide sequence preceding the alu insertion within the env gene of a human endogenous retrovirus located at megabase 20.428 on chromosome 22, and N₈ is a nucleotide sequence beginning at the 5' end of said alu insertion;
(d) a probe which can hybridize to sequence -N₉-N₁₀- (or the complement thereof) within a PCAV nucleic acid target, but which does not hybridize to sequences N₉ or N₁₀ alone (or to their complements alone), where: N₉ is a nucleotide sequence at the end of the alu insertion within the env gene of a human endogenous retrovirus located at megabase 20.428 on chromosome 22, and N₁₀ is a nucleotide sequence immediately downstream of said alu insertion;
(e) a probe comprising a nucleotide sequence with 70% or more sequence identity to a fragment of SEQ ID 10, SEQ ID 1197 or SEQ ID 1198, or to the complement of a fragment of SEQ ID 10, SEQ ID 1197 or SEQ ID 1198;
(f) a probe comprising a nucleotide sequence with 70% or more sequence identity to a fragment of SEQ ID 5 and/or SEQ ID 1199 or to the complement of a fragment of SEQ ID 5 and/or SEQ ID 1199;
(g) a probe comprising a nucleotide sequence with 70% or more sequence identity to a fragment of SEQ ID 6 and/or SEQ ID 1200 or to the complement of a fragment of SEQ ID 6 and/or SEQ ID 1200;
(h) a probe comprising a nucleotide sequence with 70% or more sequence identity to a fragment of SEQ ID 9 or to the complement of a fragment of SEQ ID 9;
(i) a probe comprising a nucleotide sequence with 70% or more sequence identity to a fragment of SEQ ID 53 or to the complement of a fragment of SEQ ID 53;
(j) a probe comprising a nucleotide sequence with 70% or more sequence identity to a fragment of SEQ ID 1191 or to the complement of a fragment of SEQ ID 1191;
(k) a probe comprising a fragment of at least 10 contiguous nucleotides of SEQ ID 10 and/or SEQ ID 1198 or of the complement of SEQ ID 10 and/or SEQ ID 1198;
(l) a probe comprising a fragment of at least 10 contiguous nucleotides of SEQ ID 47 or of the complement of SEQ ID 47;
(m)a probe comprising nucleotide sequence B1a-B2a (or its complement), wherein B1a comprises 6 or more nucleotides from the 3' end of SEQ ID 2 and B2a comprises 6 or more nucleotides from the 5' end of SEQ ID 46;
(n) a probe comprising a fragment of at least 10 contiguous nucleotides of SEQ ID 49 or of the complement of SEQ ID 49;
(o) a probe comprising nucleotide sequence Blb-B2b (or its complement), wherein B1b comprises 6 or more nucleotides from the 3' end of SEQ ID 2 and B2b comprises 6 or more nucleotides from the 5' end of SEQ ID 48;
(p) a probe comprising a fragment of at least 10 contiguous nucleotides of SEQ ID 9 or of the complement of SEQ ID 9;
(q) a probe comprising nucleotide sequence B3-B4 (or its complement), wherein B3 comprises 6 or more nucleotides from the 3' end of SEQ ID 7 and B4 comprises 6 or more nucleotides from the 5' end of SEQ ID 8;
(r) a probe comprising a fragment of at least 10 contiguous nucleotides of SEQ ID 38 or of the complement of SEQ ID 38;
(s) a probe comprising nucleotide sequence B7-B8 (or its complement), wherein B7 comprises 6 or more nucleotides from the 3' end of SEQ ID 37 and B4 comprises 6 or more nucleotides from the 5' end of SEQ ID 32;
(t) a probe comprising a fragment of at least 10 contiguous nucleotides of SEQ ID 43 or of the complement of SEQ ID 43;
(u) a probe comprising nucleotide sequence B9-B10 (or its complement) wherein B9 comprises 6 or more nucleotides from the 3' end of SEQ ID 32 and B10 comprises 6 or more nucleotides from the 5' end of SEQ ID 40;
(v) a probe comprising a fragment of at least 10 contiguous nucleotides of SEQ ID 53 or of the complement of SEQ ID 53;
(w)a probe comprising a fragment of at least 10 contiguous nucleotides of SEQ ID 111 or of the complement of SEQ ID 111;
(x) a probe comprising a fragment of at least 10 contiguous nucleotides of SEQ ID 112 or of the complement of SEQ ID 112; and
(y) a probe comprising a fragment of at least 10 contiguous nucleotides of SEQ ID 1191 or of the complement of SEQ ID 1191.

16. The probe of claim 15, comprising one or more of SEQ IDs 11, 12, 13, 36, 39, 44, 45, 50, 51, 52, (or their complements).

17. Nucleic acid of formula 5'-X-Y-Z-3', wherein:
-X- is a nucleotide sequence consisting of x nucleotides;
-Z- is a nucleotide sequence consisting of z nucleotides;
-Y- is a nucleotide sequence consisting of either (a) a fragment of y nucleotides of any of SEQ IDs 1-13, 20-53, 57, 58, 63, 81, 86, 88-91, 99-109, 111, or 112 or 1191, or (b) the complement of (a);
said nucleic acid 5'-X-Y-Z-3' is neither (i) a fragment of SEQ IDs 1-13, 20-53, 57, 58, 63, 81, 86, 88-91, 99-109, 111, or 112 or 1191 or (ii) the complement of (i);
the value of x+z is at least 1; and
the value of x+y+z is at least 8.

18. The nucleic acid of claim 17, wherein the -X- and/or -Z- moieties comprises a promoter sequence (or its complement).

19. A kit comprising primers for amplifying a template sequence contained within the endogenous retrovirus located at megabase 20.428 on human chromosome 22 (PCAV), the kit comprising a first primer and a second primer, wherein the first primer comprises a sequence substantially complementary to a portion of said template sequence and the second primer comprises a sequence substantially complementary to a portion of the complement of said template sequence, wherein the sequences within said primers which have substantial complementarity define the termini of the template sequence to be amplified.

20. The kit of claim 19, further comprising a probe which is substantially complementary to the template sequence and/or to its complement and which can hybridize thereto.

21. The kit of claim 19 or claim 20, wherein the template sequence is located within a transcript of a HERV-K located at megabase 20.428 of chromosome 22 (PCAV).

22. The kit of any one of claims 19 to 21, wherein the first and second primers are located in different exons of the template sequence.

23. The kit of any one of claims 19 to 22, wherein one of the primers comprises one or more of nucleotide sequence SEQ IDs 120 to 336 or their complements.

24. The kit of any one of claims 19 to 23, wherein:
(a) the first primer comprises a sequence which is substantially identical to a portion of N₁ and the second primer comprises a sequence which is substantially complementary to a portion of N₂;
(b) the first primer comprises a sequence which is substantially identical to a portion of the complement of N₁ and the second primer comprises a sequence which is substantially complementary to a portion of the complement of N₂;
(c) the first primer comprises a sequence which is substantially identical to a portion of N₁ and the second primer comprises a sequence which is substantially complementary to a portion of PCAV sequence downstream of a splice donor which is itself downstream of the splice acceptors near the 3' end of the second PCAV 5' LTR
(d) the first primer comprises a sequence which is substantially identical to a portion of the complement of N₁ and the second primer comprises a sequence which is substantially complementary to a portion of the complement of a PCAV sequence downstream of a splice donor which is itself downstream of the splice acceptors near the 3' end of the second PCAV 5' LTR;
(e) the first primer comprises a sequence which is substantially identical to the splice junction site in N₁-N₂ and the second primer comprises a sequence which is substantially complementary to a portion of a PCAV sequence upstream or downstream of the splice junction site;
(f) the first primer comprises a sequence which is substantially identical to the complement of the splice junction site in N₁-N₂ and the second primer comprises a sequence which is substantially complementary to a portion of a PCAV upstream or sequence downstream of the splice junction site;
(g) the first primer comprises a sequence which is substantially identical to a portion of N₃ and the second primer comprises a sequence which is substantially complementary to a portion of N₄;
(h) the first primer comprises a sequence which is substantially identical to a portion of the complement of N₃ and the second primer comprises a sequence which is substantially complementary to a portion of the complement of N₄;
(i) the first primer comprises a first sequence which is substantially identical to a portion of N₃ and a second sequence which is substantially identical to a portion of N₄, and the second primer comprises a sequence which is substantially complementary to a ortion of an upstream or downstream PCAV sequence;
(j) the first primer comprises a first sequence which is substantially identical to a portion of the complement of N₃ and a second sequence which is substantially identical to a portion of the complement of N₄, and the second primer comprises a sequence which is substantially complementary to a portion of the complement of an upstream or downstream PCAV sequence;
(k) the first primer comprises a sequence which is substantially identical to a portion of N₃ and the second primer comprises a sequence which is substantially complementary to a portion of a polyA tail;
(l) the first primer comprises a sequence which is substantially identical to a portion of the complement of N₃ and the second primer comprises a sequence which is substantially complementary to a portion of the complement of polyA tail;
(m)the first primer comprises a sequence which is substantially identical to a portion of N₇ and the second primer comprises a sequence which is substantially complementary to a portion of N₈;
(n) the first primer comprises a sequence which is substantially identical to a portion of the complement of N₇ and the second primer comprises a sequence which is substantially complementary to a portion of the complement of N₈;
(o) the first primer comprises a first sequence which is substantially identical to a portion of N₇ and a second sequence which is substantially identical to a portion of N₈, and the second primer comprises a sequence which is substantially complementary to a portion of an upstream or downstream PCAV sequence;
(p) the first primer comprises a first sequence which is substantially identical to a portion of the complement of N₇ and a second sequence which is substantially identical to a portion of the complement of N₈, and the second primer comprises a sequence which is substantially complementary to a portion of the complement of an upstream or downstream PCAV sequence;
(q) the first primer comprises a sequence which is substantially identical to a portion of Ng and the second primer comprises a sequence which is substantially complementary to a portion of N₁₀;
(r) the first primer comprises a sequence which is substantially identical to a portion of the complement of N₉ and the second primer comprises a sequence which is substantially complementary to a portion of the complement of N₁₀;
(s) the first primer comprises a first sequence which is substantially identical to a portion of N₉ and a second sequence which is substantially identical to a portion of N₁₀, and the second primer comprises a sequence which is substantially complementary to a portion of an upstream or downstream PCAV sequence;
(t) the first primer comprises a first sequence which is substantially identical to a portion of the complement of N₉ and a second sequence which is substantially identical to a portion of the complement of N₁₀, and the second primer comprises a sequence which is substantially complementary to the complement of an upstream or downstream PCAV sequence;
(u) the first primer comprises a sequence which is substantially identical to a first portion of SEQ ID 111, 112 or 53 and the second primer comprises a sequence which is substantially complementary to a second portion of SEQ ID 111, 112 or 53, such that the primer pair defines a template sequence within, consisting of or comprising SEQ ID 111, 112 or 53;
(v) the first primer comprises a sequence which is substantially identical to a first portion of the complement of SEQ ID 111, 112 or 53 and the second primer comprises a sequence which is substantially complementary to a second portion of the complement of SEQ ID 111, 112 or 53, such that the primer pair defines a template sequence within, consisting of or comprising SEQ ID 111, 112 or 53,
wherein N₁ to N₁₀ are as defined in claim 15, and wherein 'PCAV' is the endogenous retrovirus located at megabase 20.428 on human chromosome 22.

25. A pharmaceutical composition comprising the nucleic acid of any one of claims 11 I to 18, and a pharmaceutically acceptable carrier.

26. The pharmaceutical composition of claim 25, wherein the composition is immunogenic.

27. The pharmaceutical composition of claim 26, wherein the composition is a vaccine composition.

28. The pharmaceutical composition of claim 27, further comprising a vaccine adjuvant.

29. The pharmaceutical composition of claim 28, further comprising a vaccine adjuvant.

30. A pharmaceutical composition according to any one of claims 25 to 29 for use in a method for raising an immune response in a patient, wherein the method comprises administering an immunogenic dose of the composition.

31. An antibody that binds to a polypeptide of claim 1.

32. The antibody of claim 31, which recognizes a HERV-K gag protein.

33. The antibody of claim 32, which recognizes gag from the human endogenous retrovirus located at megabase 20.428 on chromosome 22, but not the gag from other HERVs.

34. A method of screening for compounds with activity against cancer, comprising: contacting a test compound with a tissue sample derived from a cell in which expression of the human endogenous retrovirus located at megabase 20.428 on chromosome 22 (PCAV) is up-regulated, or a cell line; and monitoring expression of the retrovirus in the sample, wherein a decrease in expression indicates anti-cancer efficacy of the test compound.

35. A method of screening for compounds with activity against prostate cancer, comprising: contacting a test compound with a nucleic acid or polypeptide according to any of claims 1 to 4 or 11 to 18; and detecting a binding interaction between the test compound and the nucleic acid or polypeptide, wherein a binding interaction indicates potential anti-cancer efficacy of the test compound.
